# FASCICULE DE BREVET EUROPEEN

(11) **EP 1 599 464 B1**
(45) Date de publication et mention de la délivrance du brevet: **22.09.2010**
(21) Numéro de dépôt: 04705838.3
(22) Date de dépôt: 28.01.2004
(51) Int. Cl.: C07D 401/06, A61K 31/14, A61K 31/41, C07D 409/14, C07D 401/14, C07D 401/04, C07D 417/14, C07D 521/00, C07D 413/06, C07D 417/06, C07D 405/06, C07D 403/06

(54) **DERIVES D'UREE CYCLIQUE, LEUR PREPARATION ET LEUR UTILISATION PHARMACEUTIQUE COMME INHIBITEURS DE KINASES**
CYCLISCHE HARNSTOFFDERIVATE, VERFAHREN ZU IHRER HERSTELLUNG UND IHRE PHARMAZEUTISCHE VERWENDUNG ALS KINASEINHIBITOREN
NOVEL CYCLIC UREA DERIVATIVES, PREPARATION METHOD THEREOF AND PHARMACEUTICAL USE OF SAME AS KINASE INHIBITORS

(30) Priorité: 31.01.2003 FR 0301098
(43) Date de publication de la demande: 30.11.2005
(73) Titulaire: Aventis Pharma S.A., 92160 Antony (FR)
(72) Inventeur: PATEK, Marcel, Tucson, AZ 85704 (US); NAIR, Anil, Tucson, AZ 85737 (US); HITTINGER, Augustin, F-91430 Igny (FR); NEMECEK, Conception, F-94320 Thiais (FR); BOND, Daniel, Tucson, AZ 85742 (US); HARLOW, Greg, Boulder, CO 80303 (US); BOUCHARD, Hervé, F-94320 Thiais (FR); MAUGER, Jacques, Tucson, AZ 85704 (US); MALLERON, Jean-Luc, F-91460 Marcoussis (FR); PALERMO, Mark, 07830 CALIFON (NJ) (US); AL-OBEIDI, Fahad, Tucson, AZ 85704 (US); FAITG, Thomas, Exton, PA 19341 (US); STROBEL, Hartmut, 65835 Liederbach (DE); RUF, Sven, 66484 DIETRICHINGEN (DE); RITTER, Kurt, 60594 Frankfurt am Main (DE); EL-AHMAD, Youssef, F-94000 Créteil (FR); LESUISSE, Dominique, F-93100 Montreuil (FR); BENARD, Didier, F-95560 MONTSOULT (FR)
(86) Numéro de dépôt international: PCT/FR2004/000188
(87) Numéro de publication internationale: WO 2004/070050

(56) Documents cités:
- EP-A- 0 584 694
- EP-A- 0 770 613
- WO-A-01/92253
- WO-A-98/39303
- US-A- 4 097 578
- US-A- 6 022 875

## Description

La présente invention concerne de nouveaux dérivés d'urée cyclique, leur procécé de préparation, leur application comme médicaments, les compositions pharmaceutiques les renfermant et l'utilisation pharmaceutique de tels dérivés pour la prévention et le traitement d'affections capables d'être modulées par l'inhibition de l'activité dé protéines kinases.

La présente invention concerne de nouveaux dérivés d'urée cyclique possédant des effets inhibiteurs pour des protéines kinases.

Les produits de la présente invention peuvent ainsi notamment être utilisés pour la prévention ou le traitement d'affections capables d'être modulées par l'inhibition de l'activité de protéines kinases.

L'inhibition et la régulation de protéines kinases constituent notamment un nouveau puissant mécanisme d'action pour le traitement d'un grand nombre de tumeurs solides.

De telles affections que peuvent traiter les produits de la présente demande sont donc tout particulièrement les tumeurs solides.

De telles protéines kinases appartiennent notamment au groupe suivant: EGFR, Fak, FLK-1, FGFR1, FGFR2, FGFR3, FGFR4, FGFR5, flt-1, IGF-1R, KDR, PLK,PDGFR, tie2, VEGFR, AKT, Raf.

On indique particulièrement la protéine kinase IGF1-R (Insulin Growth Factor-1 Receptor).

On indique également la protéine kinase FAK.

On indique également la protéine kinase AKT.

La présente invention concerne ainsi particulièrement de nouveaux inhibiteurs du récepteur IGF-1R qui peuvent être utilisés pour des traitements en oncologie.

La présente invention concerne également de nouveaux inhibiteurs du récepteur FAK qui peuvent être utilisés pour des traitements en oncologie.

La présente invention concerne également de nouveaux inhibiteurs du récepteur AKT qui peuvent être utilisés pour des traitements en oncologie.

Le cancer reste une maladie pour laquelle les traitements existants sont clairement insuffisants. Certaines protéines kinases dont notamment IGF-1R (Insulin Growth Factor 1 Receptor)jouent un rôle important dans de nombreux cancers. L'inhibition de telles protéines kinases est potentiellement importante dans la chimiothérapie de cancers notamment pour supprimer la croissance ou la survie de tumeurs. La présente invention concerne donc l'identification de nouveaux produits qui inhibent de telles protéines kinases.

Les protéines kinases participent aux évènements de signalisation qui contrôlent l'activation, la croissance et la différentiation des cellules en réponse, soit à des médiateurs extracellulaires, soit à des changements de l'environnement. En générale, ces kinases appartiennent à deux groupes: celles qui phosphorylent préférentiellement les résidus sérines et/ou thréonine et celles qui phosphorylent préférentiellement les résidus tyrosines [S.K.Hanks and T.Hunter, FASEB. J., 1995, 9, pages 576-596]. Les sérine/thréonine kinases sont par exemple, les isoformes des protéines kinases C [A.C.Newton, J. Biol. Chem., 1995, 270, pages 28495-28498] et un groupe de kinases dépendantes des cyclines, comme cdc2 [J.Pines, Trends in Biochemical Sciences, 1995, 18, pages 195-197]. Les tyrosine kinases comprennent les récepteurs aux facteurs de croissance comme le récepteur au facteur de croissance épidermal (EGF) [S.Iwashita and M.Kobayashi, Cellular Signalling, 1992, 4, pages 123-132], et des kinases cytosoliques comme p56tck, p59fYn, ZAP-70 et les kinases csk [C. Chan et. al., Ann. Rev. Immunol., 1994, 12, pages 555-592].

Des niveaux anormalement élevés d'activité protéine kinase ont été impliqués dans de nombreuses maladies, résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inapproprié de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome ...) et sa présence est souvent associée à un phénotype plus agressif.

De fortes concentrations d'IGF1 circulant sont fortement corrélées à un risque de cancer de la prostate, poumon et sein.

De plus, il a été largement documenté que IGF-I-R est nécessaire à l'établissement et au maintient du phénotype transformé in vitro comme in vivo[Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie-, radiation-, et l'apoptose induite par des cytokines. De plus, l'inhibition d'IGF-I-R endogène par un dominant négatif, la formation de triple hélice ou l'expression d'un antisens provoque une suppression de l'activité transformante *in vitro* et la diminution de la croissance de tumeurs dans les modèles animaux.

Parmi les kinases pour lesquelles une modulation de l'activité est recherchée, FAK (Focal Adhesion Kinase) est également une kinase préférée.

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33) : 23439-442 (1992)]. L'autophosphorylation sur la tyrosine 397 de FAK représente un site de liaison pour une autre tyrosine kinase, Src, via son domaine SH2 [Schaller et al. Mol. Cell. Biol. 14 : 1680-1688 1994 ; Xing et al. Mol. Cell. Biol. 5 : 413-421 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature ; 372 : 786-791 1994 ; Schlaepfer et al. Prog. Biophy. Mol. Biol. 71 : 435-478 1999 ; Schlaepfer and Hunter, J. Biol. Chem. 272 : 13189-13195 1997].

L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 : 1461-1469 1999]. Phosphatidylinositol-3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91 : 10148-10152 1994 ; Ling et al. J. Cell. Biochem. 73 : 533-544 1999]. Le complexe FAK/Src phosphoryle différents substrats comme la paxilline et p130CAS dans les fibroblastes [Vuori et al. Mol. Cell. Biol. 16 : 2606-2613 1996].

Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaire *in vitro*. Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143 : 1997-2008 1998]. D'autres auteurs ont montré que des cellules tumorales traitées avec des oligonucléotides anti-sens de FAK perdent leur adhésion et entrent en apoptose (Xu et al, Cell Growth Differ. 4 : 413-418 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112 : 2677-91 1999]. La surexpression du domaine C-terminal de FAK (FRNK) bloque l'étirement des cellules adhérentes et réduit la migration cellulaire *in vitro* [Richardson A. and Parsons J.T. Nature. 380 : 538-540 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109 : 1787-94 1996 ; Wang D et al. J. Cell Sci. 113 : 4221-4230 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342 (8878) : 1024-1025 1993 ; Owens et al. Cancer Research. 55 : 2752-2755 1995 ; Maung K. et al. Oncogene 18 : 6824-6828 1999 ; Wang D et al. J. Cell Sci. 113 : 4221-4230 2000].

La protéine kinase AKT (également connue sous le nom de PKB) et la phosphoinositide 3-kinase (PI3K) sont impliqués dans une voie de signalisation cellulaire qui transmet des signaux venant de facteurs de croissance activant des récepteurs membranaires.

Cette voie de transduction est impliquée dans de multiples fonctions cellulaires: régulation de l'apoptose, contrôle de la transcription et de la traduction, métabolisme du glucose, angiogénèse et intégrité mitochondriale. Identifié d'abord comme un acteur important dans les voies de signalisation insulino-dépendantes régulant des réponses métaboliques, la sérine/thréonine kinase AKT a ensuite été identifiée comme un médiateur jouant un rôle clé dans la survie induite par des facteurs de croissance. Il a été montré qu'AKT pouvait inhiber la mort par apoptose induite par des stimuli variés, dans un certain nombre de types cellulaires et de cellules tumorales. En accord avec ces constatations, il a été montré qu'AKT pouvait, par phosphorylation de résidus sérine donnés, inactiver BAD, GSK3β, caspase-9, le facteur de transcription Forkhead et activer IKKalpha et e-NOS. Il est intéressant de noter que la protéine BAD est retrouvée hyper-phosphorylée dans 11 lignées cellulaires humaines tumorales sur 41 étudiées. De plus, il a été montré que l'hypoxie modulait l'induction du VEGF dans des cellules transformées par Ha-ras en activant la voie PI3K/AKT et en impliquant la séquence de fixation du facteur de transcription HIF-1 (hypoxia indicible factor-1) appelé HRE pour « hypoxy-responsive-element ».

AKT joue un rôle très important dans les pathologies cancéreuses. L'amplification et/ou la surexpression d'AKT a été rapportée dans de nombreuses tumeurs humaines comme le carcinome gastrique (amplification d'AKT1), les carcinomes de l'ovaire, du sein ou du pancréas (amplification et surexpression d'AKT2) et les carcinomes du sein déficients en récepteurs aux oestrogènes ainsi que les carcinomes de la prostate indépendants des androgènes (surexpression d'AKT3). De plus, AKT est activée constitutivement dans toutes les tumeurs PTEN (-/-), la phosphatase PTEN étant délétée ou inactivée par des mutations dans de nombreux types de tumeurs comme les carcinomes de l'ovaire, de la prostate, de l'endomètre, les glioblastomes et les mélanomes. AKT est également impliqué dans l'activation oncogénique de bcr-abl (Références : Khawaja A., Nature 1999, 401, 33-34; Cardone et al. Nature 1998, 282, 1318-1321 ; Kitada S. et al., Am J Pathol 1998 Jan ; 152(1) : 51-61 ; Mazure NM et al. Blood 1997, 90, 3322-3331 ; Zhong H. et al. Cancer Res. 2000, 60, 1541-1545).

La présente invention a donc pour objet les produits de formule générale (I): 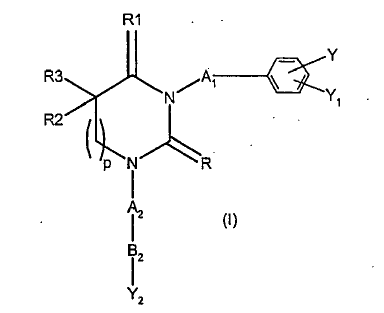 dans laquelle p représente un entier de 0 à 2,
R et R1 identiques ou différents représentent O ou NH,
R2 et R3, identiques ou différents représentent hydrogène, alkyle, alkényle, alkynyle, cycloalkyle, aryle et hétéroaryle éventuellement substitués ou bien R2 et R3 forment ensemble avec l'atome de carbone auxquels ils sont liés un radical carbocyclique ou hétérocyclique, ces radicaux étant constitués de 3 à 10 chaînons et le radical hétérocyclique renfermant un ou plusieurs hétéroatomes choisis parmi O, S, N et NR7, tous ces radicaux étant éventuellement substitués,
A1 représente une simple liaison, un radical alkyle, un radical alkényle ou alkynyle,
Y et Y1 identiques ou différents sont tels que l'un de Y et Y1 est choisi parmi OCF3, -O-CF2-CHF2, -O-CHF2, -O-CH2-CF3, S(O)nCF3, -S-CF2-CF2-CF3, -S(O)n-Alk, -S-Alk-O-Alk, -S-Alk-OH, -S-Alk-CN, -S-Alk-hétérocycloalkyle, - SO2CHF2, -S02CF2CF3, -S02NR5R6 et -SF5, avec Alk représente un radical alkyle renfermant de 1 à 4 atomes de carbone, et l'autre de Y et Y1 est choisi parmi les valeurs suivantes : hydrogène, halogène, nitro, NR5R6, carboxy libre ou estérifié et CONR5R6,
ou bien le radical phényle forme avec ses substituants Y et Y1 l'un des radicaux suivants : 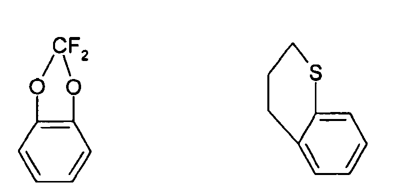 le radical ainsi formé étant éventuellement substitué par un ou plusieurs radicaux alkyle eux-mêmes éventuellement substitués,
R5 et R6 identiques ou différents sont choisis parmi hydrogène, alkyle, alkényle, alkynyle, cycloalkyle, cycloalkényle, hétérocycloalkyle, aryle et hétéroaryle éventuellement substitués ou bien R5 et R6 forment avec l'atome d'azote auxquels ils sont liés un radical hétérocyclique renfermant 3 à 10 chaînons renfermant un ou plusieurs hétéroatomes choisis parmi O, S, N et NR7 éventuellement substitué,
A2, identique ou différent de A1, représente les valeurs de A1 et CO et SO2,
B2 représente un radical hétérocyclique monocyclique ou bicyclique saturé ou insaturé renfermant 1 à plusieurs hétéroatomes identiques ou différents choisis parmi O, S, N et NR7, éventuellement substitués par un ou plusieurs substituants identiques ou différents choisis parmi les valeurs de Y2,
R7 représente un atome d'hydrogène, un radical alkyle, cycloalkyle, phényle, acyle, S(O)2Alk, S(O)2Aryle, S(O)2hétéroaryle et S(O)2NR5R6,
Y2 représente hydrogène, halogène, hydroxyle, cyano, alkyle, alcoxy, cycloalkyl, hétérocycloalkyle, aryle, hétéroaryle, -O-alkényle, -O-alkynyle, -O-cycloalkyle, - S(O)n-alkyle, -S(O)n-alkényle, -S(O)n-alkynyle, S(O)n-cycloalkyle, COOR13, -OCOR13, NR5R6, CONR5R6, S(O)n-NR5R6, -NR10-CO-R13,-NR10-SO2-R13, NH-SO2-NR5R6, -NR10-CO-NR5R6, -NR10-CS-NR5R6, -NR10-COOR13, tous ces radicaux étant éventuellement substitués,
tous les radicaux ci-dessus alkyle, alkényle, alkynyle, alcoxy, étant linéaires ou ramifiés et renfermant au plus 6 atomes de carbone,
tous les radicaux ci-dessus cycloalkyle, hétérocycloalkyle renfermant au plus 7 atomes de carbone, tous les radicaux ci-dessus aryle et hétéroaryle renfermant au plus 10 atomes de carbone,
tous les radicaux ci-dessus alkyle, alkényle, alkynyle, alcoxy, cycloalkyle, hétérocycloalkyle, aryle et hétéroaryle, carbocyclique et hétérocycliques ainsi que le cycle formé par R5 et R6 avec l'atome auxquels ils sont liés étant éventuellement substitués par un ou plusieurs radicaux identiques ou différents choisis parmi les atomes d'halogène et les radicaux cyano, hydroxy, alcoxy, CF3, nitro, aryle, hétéroaryle, -C(=O)-OR9, - C(=O)-R8, -NR11R12, -C(=O)-NR11R12, -N(R10)-C(=O)-R8, - N(R10)-C(=O)-OR9, N(R10)-C(=O)-NR11R12, -N(R10)-S(O)n-R8, -S(O)n-R8, -N(R10)-S(O)n-11R11R12 ou -S(O)n-NR11R12,
tous les radicaux aryle et hétéroaryle ci-dessus étant de plus éventuellement substitués par un ou plusieurs radicaux choisis parmi les radicaux alkyle, alcoxy et alkylènedioxy,
tous les radicaux cycliques ci-dessus ainsi que le cycle formé par R5 et R6 avec l'atome auxquels ils sont liés étant de plus éventuellement substitués par un ou plusieurs radicaux choisis parmi oxo et thioxo,
n représente un entier de 0 à 2,
R8 représente alkyle, alkényle, cycloalkyle, cycloalkylalkyle, hétérocycloalkyle, hétérocycloalkylalkyle, aryle, arylalkyle, hétéroaryle et hétéroarylalkyle,
R9 représente les valeurs de R8 et hydrogène,
R10 représente hydrogène ou alkyle,
R11 et R12, identiques ou différents, représentent hydrogène, C3-C6 cycloalkyle, C1-C4 alkyle et phényle éventuellement substitués par un ou plusieurs radicaux identiques ou différents choisis parmi les atomes d'halogène et les radicaux cyano, hydroxy, alcoxy, CF3, nitro, phényle et carboxy libre, salifié, estérifié ou amidifié,
ou bien R11 et R12 forment avec l'atome d'azote auxquels ils sont liés un radical cyclique renfermant 5 à 7 chaînons renfermant un ou plusieurs hétéroatomes choisis parmi O, S, N et NR7 et de préférence une amine cyclique, R13, identique ou différent de R5 ou R6, étant chois parmi les valeurs de R5 ou R6,
étant entendu que les produits de formule (I) sont tels que définis ci-après de a) à d) :
a) lorsque p représente l'entier 0, R représente Oxygène, R1 représente Oxygène, A1 représente une simple liaison ou alkyle, Y et Y1 identiques ou différents sont tels que l'un au moins représente OCF3 ou Salk, A2 représente simple liaison ou alkyle et B2 représente un radical hétérocycle éventuellement substitué, alors R2 et R3 ne représentent pas l'un hydrogène et l'autre imidazolylalkyle
b) lorsque p représente l'entier 0, R et R1 représentent oxygène, A1 représente une simple liaison ou alkyle, Y et Y1 identiques ou différents sont tels que l'un au moins représente OCF3, SOAlk, S(O)2alk ou SO2NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
c) lorsque p représente l'entier 0, R et R1 représentent Oxygène, A1 représente une simple liaison ou alkyle, Y et Y1 identiques ou différents sont tels que l'un au moins représente S(O)nAlk, A2 représente une simple liaison et B2 représente un radical hétérocycle aromatique renfermant 5 ou 6 chaînons éventuellement substitué, alors R2 et R3 ne sont pas choisis parmi hydrogène, alkyle, arylalkyle, aryle et hétéroaryle,
d) lorsque p représente un entier de 0 à 2, R et R1 représentent Oxygène, A1 représente une simple liaison, Y et Y1 identiques ou différents sont tels que l'un représente SO2Alk ou S02NH2 et l'autre représente NR5R6, A2 représente une simple liaison ou alkylène et B2 représente un radical hétérocycle renfermant 5 à 10 chaînons éventuellement substitué, alors R2 et R3 ne représentent pas tous deux hydrogène,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention a ainsi pour objet les produits de formule générale (I) telle que définie ci-dessus dans laquelle l'un de Y et Y1 représente un atome d'hydrogène et l'autre est choisi parmi OCF3, S(O)nCF3, S(O)nAlk, SO2CHF2, SO2CF2CF3 et SO2NR5R6 ,
les autres substituants desdits produits de formule (I) étant choisis parmi les valeurs définies ci-dessus étant entendu que :
a) lorsque p représente l'entier 0, R représente oxygène, R1 représente oxygène, A1 représente une simple liaison ou alkyle, Y et Y1 identiques ou différents sont tels que l'un représente hydrogène et l'autre représente OCF3 ou Salk, A2 représente simple liaison ou alkyle et B2 représente un radical hétérocycle éventuellement substitué, alors R2 et R3 ne représentent pas l'un hydrogène et l'autre imidazolylalkyle,
b) lorsque p représente l'entier 0, R et R1 représentent Oxygène, A1 représente une simple liaison ou alkyle, Y et Y1 identiques ou différents sont tels que l'un représente hydrogène et l'autre représente OCF3, SOAlk, S(O)2alk ou S02NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
c) lorsque p représente l'entier 0, R et R1 représentent Oxygène, A1 représente une simple liaison ou alkyle, Y et Y1 identiques ou différents sont tels que l'un représente hydrogène et l'autre représente S(O)nAlk, A2 représente une simple liaison et B2 représente un radical hétérocycle aromatique renfermant 5 ou 6 chaînons éventuellement substitué, alors R2 et R3 ne sont pas choisis parmi hydrogène, alkyle, arylalkyle, aryle et hétéroaryle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I). La présente invention a ainsi pour objet les produits de formule générale (I) telle que définie ci-dessus dans laquelle l'un de Y et Y1 représente un atome d'hydrogène et l'autre est choisi parmi S(O)nCF3, SOAlk, S(O)2Alk, SO2CHF2, S02CF2CF3 et SO2NR5R6,
les autres substituants desdits produits de formule (I) étant choisis parmi les valeurs définies ci-dessus et étant entendu que les produits de formule (I) sont tels que définis ci-après en a) et b) :
a) lorsque p représente l'entier 0, R et R1 représentent Oxygène, A1 représente une simple liaison ou alkyle, Y et Y1 identiques ou différents sont tels que l'un représente hydrogène et l'autre représente SOAlk, S(O)2alk ou S02NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
b) lorsque p représente l'entier 0, R et R1 représentent oxygène, A1 représente une simple liaison ou alkyle, Y et Y1 identiques ou différents sont tels que l'un représente hydrogène et l'autre représente SOAlk ou S(O)2Alk, A2 représente une simple liaison et B2 représente un radical hétérocycle aromatique renfermant 5 ou 6 chaînons éventuellement substitué, alors R2 et R3 ne sont pas choisis parmi hydrogène, alkyle, arylalkyle, aryle et hétéroaryle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention a ainsi pour objet les produits de formule générale (I) telle que définie ci-dessus dans laquelle l'un de Y et Y1 représente un atome d'hydrogène et l'autre est choisi parmi S(O)nCF3, S02CHF2, SO2CF2CF3 et SO2NR5R6,
les autres substituants desdits produits de formule (I) étant choisis parmi les valeurs définies ci-dessus et étant entendu que lorsque p représente l'entier 0, R et R1 représentent Oxygène, A1 représente une simple liaison ou alkyle, Y et Y1 identiques ou différents sont tels que l'un représente hydrogène et l'autre représente SO2NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

Dans les produits de formule (I) et dans ce qui suit, les termes indiqués ont les significations qui suivant :
- le terme 'Hal', "Halo" ou halogène désigne les atomes de fluor, de chlore, de brome ou d'iode.
- le terme radical alkyle, alk, Alk ou ALK, désigne un radical linéaire ou ramifié renfermant au plus 12 atomes de carbone choisi parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés.

On cite plus particulièrement les radicaux alkyle ayant au plus 6 atomes de carbone et notamment les radicaux méthyle, éthyle, propyle, isopropyle, n-butyle, isobutyle, terbutyle, pentyle linéaire ou ramifié, hexyle linéaires ou ramifiés.
- le terme radical alkényle désigne un radical linéaire ou ramifié renfermant au plus 12 atomes de carbone et préférentiellement 4 atomes de carbone choisi par exemple parmi les valeurs suivantes: éthényle ou vinyle, propényl ou alkyle, 1-propényle, n-butényle, i-butényle, 3-méthylbut-2-ényle, n-pentényle, hexényle, heptényle, octényle, cyclohexybutényle et décényle ainsi que leurs isomères de position linéaires ou ramifiés.

Parmi les valeurs alkényle, on cite plus particulièrement les valeurs allyle ou butényle.
- le terme radical alkynyle désigne un radical linéaire ou ramifié renfermant au plus 12 atomes de carbone et préférentiellement 4 atomes de carbone choisi par exemple parmi les valeurs suivantes: éthynyle, propynyle ou propargyle, butynyle, n-butynyle, i-butynyle, 3-méthylbut-2-ynyle, pentynyle ou hexynyle ainsi que leurs isomères de position linéaires ou ramifiés.

Parmi les valeurs alkynyle, on cite plus particulièrement la valeur propargyle.
- le terme radical alcoxy désigne un radical linéaire ou ramifié renfermant au plus 12 atomes de carbone et préférentiellement 6 atomes de carbone choisi par exemple parmi les radicaux méthoxy, éthoxy, propoxy, isopropoxy, butoxy linéaire, secondaire ou tertiaire, pentoxy, hexoxy et heptoxy ainsi que leurs isomères de position linéaires ou ramifiés,
- le terme radical alkoxycarbonyle ou alkyl-O-CO-désigne un radical linéaire ou ramifié renfermant au plus 12 atomes de carbone dans lequel le radical alkyle a la signification indiquée ci-dessus : on peut citer par exemple les radicaux méthoxy- et éthoxycarbonyle.
- le terme radical alkylènedioxy ou -O-alkylène-O-désigne un radical linéaire ou ramifié renfermant au plus 12 atomes de carbone dans lequel le radical alkylène a la signification indiquée ci-dessus : on peut citer par exemple les radicaux méthylènedioxy et éthylènedioxy.
- le terme alkylsulfinyle ou alkyl-SO- désigne un radical linéaire ou ramifié renfermant au plus 12 atomes de carbone dans lequel le radical alkyle a la signification indiquée ci-dessus et renferme de préférence 4 atomes de carbone.
- Le terme alkylsulfonyle ou alkyl-SO2- désigne un radical linéaire ou ramifié renfermant au plus 12 atomes de carbone dans lequel le radical alkyle a la signification indiquée ci-dessus et renferme de préférence 4 atomes de carbone.
- le terme alkylsulfonylcarbamoyle ou alkyl-SO2-NH-C(=O)- désigne un radical linéaire ou ramifié renfermant au plus 12 atomes de carbone dans lequel le radical alkyle a la signification indiquée ci-dessus et renferme de préférence 4 atomes de carbone.
- le terme alkylthio ou alkyl-S- désigne un radical linéaire ou ramifié renfermant au plus 12 atomes de carbone et représente notamment les radicaux méthylthio, éthylthio, isopropylthio et heptylthio.
- le terme radical cycloalkyle désigne un radical carbocyclique monocyclique ou bicyclique renfermant de 3 à 10 chaînons et désigne notamment les radicaux cyclopropyle, cyclobutyle, cyclopentyle et cyclohexyle,
- le terme radical -O-cycloalkyle désigne un radical dans lequel le radical cycloalkyle a la signification indiquée ci-dessus
- le terme radical cycloalkényle désigne un radical carbocyclique monocyclique ou bicyclique non aromatique contenant au moins une double liaison et renfermant de 3 à 10 chaînons et désigne notamment les radicaux cyclobutényle, cyclopentényle ou cyclohexényle.
- le terme radical cycloalkylalkyle désigne un radical dans lequel cycloalkyle et alkyle sont choisis parmi les valeurs indiquées ci-dessus : ce radical désigne ainsi par exemple les radicaux cyclopropylméthyle, cyclopentylméthyle, cyclohexylméthyle et cycloheptylméthyle.
- le terme radical acyle ou r-CO- désigne un radical linéaire ou ramifié renfermant au plus 12 atomes de carbone dans lequel le radical r représente un atome d'hydrogène, un radical alkyle, cycloalkyle, cycloalkényle, cycloalkyle, hétérocycloalkyle ou aryle, ces radicaux ayant les valeurs indiquées ci-dessus et étant éventuellement substitués comme indiqué : on cite par exemple les radicaux formyle, acétyle, propionyle, butyryle ou benzoyle, ou encore valéryle, hexanoyle, acryloyle, crotonoyle ou carbamoyle.
- par radical acyloxy, on entend les radicaux acyl-O- dans lesquels acyl a la signification indiquée ci-dessus : on cite par exemple les radicaux acétoxy ou propionyloxy.
- par radical acylamino, on entend les radicaux acyl-NH- dans lesquels acyl a la signification indiquée ci-dessus.
- le terme radical aryle désigne les radicaux insaturés, monocycliques ou constitués de cycles condensés, carbocycliques. Comme exemples de tel radical aryle, on peut citer les radicaux phényle ou naphtyle,

On cite plus particulièrement le radical phényle.
- par arylalkyle on entend les radicaux résultant de la combinaison des radicaux alkyle cités précédemment éventuellement substitués et les radicaux aryles également cités ci-dessus, éventuellement substitués : on cite par exemple les radicaux benzyle, phényléthyle, 2-phénéthyle, triphénylméthyle ou naphthlèneméthyle.
- le terme radical hétérocyclique désigne un radical carbocylique saturé (hétérocycloalkyle) ou insaturé (hétéroaryle) constitué au plus de 6 chaînons interrompus par un ou plusieurs hétéroatomes, identiques ou différents, choisis parmi les atomes d'oxygène, d'azote ou de soufre.

Comme radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridinyle, pyrrolidinyle, imidazolidinyle, pyrazolidinyle, morpholinyle ou encore tétrahydrofuryle, tétrahydrothiényle, chromanyle, dihydrobenzofuranyle, indolinyle, pipéridinyle, perhydropyranyle, pyrindolinyle, tétrahydroquinoléinyle, tétrahydroisoquinoléinyle ou thioazolidinyle, tous ces radicaux étant éventuellement substitués.

Parmi les radicaux hétérocycloalkyles, on peut citer notamment les radicaux pipérazinyle éventuellement substitué, pipéridinyle éventuellement substitué, pyrrolidinyle éventuellement substitué, imidazolidinyle, pyrazolidinyle, morpholinyle ou thioazolidinyle : on peut citer encore plus particulièrement les radicaux morpholinyle, pyrrolidinyle et pipérazinyle éventuellement substitués.
- par radical hétérocycloalkylalkyle, on entend les radicaux dans lesquels les restes hétérocycloalkyle et alkyle ont les significations précédentes
- parmi les radicaux hétéroaryles à 5 chaînons on peut citer les radicaux furyle tel que 2-furyle, thiényle tel que 2-thiényle et 3-thiényle, pyrrolyle, diazolyle, thiazolyle, thiadiazolyle, thiatriazolyle, isothiazolyle, oxazolyle oxadiazolyle, 3- ou 4-isoxazolyle, imidazolyle, pyrazolyle, isoxazolyle.

Parmi les radicaux hétéroaryles à 6 chaînons on peut citer notamment les radicaux pyridyle tel que 2-pyridyle, 3-pyridyle et 4-pyridyle, pyrimidyle, pyrimidinyle, pyridazinyle, pyrazinyle et tétrazolyle.
- Comme radicaux hétéroaryles condensés contenant au moins un hétéroatome choisi parmi le soufre, l'azote et l'oxygène, on peut citer par exemple benzothiényle tel que 3-benzothiényle, benzofuryle, benzofurannyle, benzopyrrolyle, benzimidazolyle, benzoxazolyle, thionaphtyle, indolyle, purinyle, quinoléinyle, isoquinoléinyle et naphtyridinyle.

Parmi les radicaux hétéroaryles condensés, on peut citer plus particulièrement les radicaux benzothiényle, benzofurannyle, indolyle ou quinoléinyle, benzimidazolyle, benzothiazolyle, furyle, imidazolyle, indolizinyle, isoxazolyle, isoquinolinyle, isothiazolyle, oxadiazolyle, pyrazinyle, pyridazinyle, pyrazolyle, pyridyle, pyrimidinyle, pyrrolyle, quinazolinyle, 1,3,4-thiadiazolyle, thiazolyle, thiényle et groupes triazolyle, ces radicaux étant éventuellement substitués comme indiqué pour les radicaux hétéroaryles.
- le terme amine cyclique désigne un radical cycloalkyle renfermant de 3 à 8 chaînons dans lequel un atome de carbone est remplacé par un atome d'azote, le radical cycloalkyle ayant la signification indiquée ci-dessus et pouvant renfermer aussi un ou plusieurs autres hétéroatomes choisi parmi O, S, SO2, N ou NR7 avec R7 tel que défini ci-dessus, comme exemples de telles amines cycliques, on peut citer par exemple les radicaux pyrrolidinyle, pipéridinyle, morpholinyle, pipérazinyle, indolinyle, pyrindolinyle ou tétrahydroquinoléinyle.

Le terme patient désigne les êtres humains mais aussi les autres mammifères.

Le terme "Prodrug" désigne un produit qui peut être transformé in vivo par des mécanismes métaboliques (tel que l'hydrolyse) en un produit de formule (I). Par exemple, un ester d'un produit de formule (I) contenant un groupe hydroxyle peut être converti par hydrolyse in vivo en sa molécule mère. Ou encore un ester d'un produit de formule (I) contenant un groupe carboxy peut être converti par hydrolyse in vivo en sa molécule mère.

On peut citer à titre d'exemples des esters de produits de formule (I) contenant un groupe hydroxyle tels que les acétates, citrates, lactates, tartrates, malonates, oxalates, salicylates, propionates, succinates, fumarates, maléates, méthylene-bis-b-hydroxynaphthoates, gentisates, iséthionates, di-p-toluoyltartrates, méthanesulfonates, éthanesulfonates, benzenesulfonates, p-toluènesulfonates, cyclohexylsulfamates et quinates.

Des esters de produits de formule (I) particulièrement utiles contenant un groupe hydroxyle peuvent être préparés à partir de restes acides tels que ceux décrits par Bundgaard et. al., J. Med. Chem., 1989, 32, page 2503-2507: ces esters incluent notamment des (aminométhyl)-benzoates substitués, dialkylamino-méthylbenzoates dans lesquels les deux groupements alkyle peuvent être liés ensemble ou peuvent être interrompus par un atome d'oxygène ou par un atome d'azote éventuellement substitué soit un atome d'azote alkylé ou encore des morpholino-méthyl)benzoates, e.g. 3- ou 4-(morpholinométhyl)-benzoates, et (4-alkylpiperazin-1-yl)benzoates, e.g. 3- ou 4-(4-alkylpiperazin-1-yl)benzoates.

Le ou les radicaux carboxy des produits de formule (I) peuvent être salifiés ou estérifiés par les groupements divers connus de l'homme du métier parmi lesquels on peut citer, à titre d'exemples non limitatifs, les composés suivants :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino, méthane, l'éthanolamine, la pyridine, la picoline, la dicyclohexylamine, la morpholine, la benzylamine, la prochaine, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxy-carbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxy-méthyle, propionyloxyméthyle, méthylthiométhyle, diméthyl-aminoéthyle, benzyle ou phénéthyle.

Par carboxy estérifié on entend par exemple les radicaux tels que les radiaux alkyloxycarbonyle par exemple méthoxycarbonyle, éthoxycarbonyle, propoxycarbonyle, butyl ou tert-butyloxycarbonyle, cyclobutyloxycarbonyle, cyclopentyloxycarbonyle ou cyclohexyloxycarbonyle.

On peut également citer des radicaux formés avec les restes esters facilement clivables tels que les radicaux méthoxyméthyle, éthoxyméthyle ; les radicaux acyloxy-alkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les radicaux du type -CONR5R6 tel que défini ci-dessus : on entend aussi les radicaux NCOR6R7 dans lesquels les radicaux R6 et R7 identiques ou différents représentent un atome d'hydrogène ou un radical alkyle ayant de 1 à 4 atomes de carbone tels que les radicaux méthyle, éthyle, propyle, isopropyle, butyle, isobutyle, sec-butyle ou tert-butyle et notamment les radicaux amino, alkylamino et dialkylamino.

Par radical alkylamino on entend les radicaux méthylamino, éthylamino, propylamino ou butylamino, linéaire ou ramifié. On préfère les radicaux alkyle ayant au plus 4 atomes de carbone, les radicaux alkyle peuvent être choisis parmi les radicaux alkyle cités ci-dessus.

Par radical dialkylamino on entend par exemple les radicaux diméthylamino, diéthylamino, méthyléthylamino. Comme précédemment on préfère les radicaux alkyle ayant au plus 4 atomes de carbone choisis dans la liste indiquée ci-dessus.

Les radicaux NR5R6 ou NR6R7 peuvent également représenter un hétérocycle qui peut ou non comporter un hétéroatome supplémentaire. On peut citer les radicaux pyrrolyle, imidazolyle, indolyle, pipéridinyle, morpholinyle et pipérazinyle. On préfère les radicaux pipéridinyle, morpholinyle ou pipérazinyle.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine. On préfère le sel de sodium.

Lorsque les produits de formule (I) comportent un radical amino salifiable par un acide il est bien entendu que ces sels d'acides font également partie de l'invention. On peut citer les sels fournis avec les acides chlorhydrique ou méthanesulfonique par exemple.

Les sels d'addition avec les acides minéraux ou organi-ques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthane-sulfonique, l'acide propanesulfonique, les acides alcoyldisulfoniques tels que par exemple l'acide méthanedisulfonique, l'acide α, β-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus.

L'invention a notamment pour objet les produits de formule (I) telle que définie ci-dessus dans laquelle l'un de Y et Y1 représente un atome d'hydrogène et l'autre est choisi parmi S(O)nCF3, S02CHF2 et SO2CF2CF3, les autres substituants desdits produits de formule (I) étant choisis parmi les valeurs définies ci-dessus, lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

L'invention a notamment pour objet les produits de formule (I) telle que définie ci-dessus telle que tous les radicaux alkyle, alkényle, alkynyle, cycloalkyle, hétérocycloalkyle, aryle ou hétéroaryle définis ci-dessus sont éventuellement substitués par un ou plusieurs radicaux identiques ou différents choisis halogène, cyano, hydroxy, alcoxy, CF3, nitro, phényle, carboxy libre, salifié, estérifié par un radical alkyle ou amidifié par un radical NR11aR12a, -C(=O)-R9a, -NR11aR12a, -C(=O)-NR11aR12a, -N(R10a)-C(=O)-R9a, -N(R10a)-C(=O)-OR8a, N(R10a)-C(=O)-NR11aR12a, -N(R10a)-S(O)n-R9a, -S(O)n-R9a, -N(R10a)-S(O)n-NR11aR12a ou -S(O)n-NR11aR12a,
tous les radicaux aryle et hétéroaryle ci-dessus étant de plus éventuellement substitués par un radical éthylènedioxy,
R8a représente hydrogène, alkyle, alkényle, phényle, phénylalkyle, hétéroaryle ou hétéroarylalkyle,
R9a représente alkyle, cycloalkyle, cycloalkylalkyle, hétérocycloalkyle, hétérocycloalkylalkyle, phényle, phénylalkyle, hétéroaryle ou hétéroarylalkyle,
R10a représente hydrogène ou alkyle,
R11a et R12a, identiques ou différents, représentent hydrogène, alkyle, cycloalkyle, cycloalkylalkyle, phényle, phénylalkyle éventuellement substitués par un ou plusieurs substituants identiques ou différents choisis parmi halogène, hydroxy, C1-C4alkyl ou C1-C4alkoxy ou bien R11a et R12a forment avec l'atome d'azote auxquels ils sont liés un radical cyclique choisi parmi pyrrolidinyle, pipéridinyle, pipérazinyle, morpholinyle, indolinyle, pyrindolinyle, tétrahydroquinoléinyle, thiazolidinyle et naphtyridyle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I). L'invention a notamment pour objet les produits de formule (I) telle que définie ci-dessus telle que p représente l'entier 0, les autres substituants desdits produits de formule (I) ayant chacun l'une quelconque des valeurs définies ci-dessus.

L'invention a notamment pour objet les produits de formule (I) telle que définie ci-dessus telle que p représente l'entier 1, les autres substituants desdits produits de formule (I) ayant l'une quelconque des valeurs définies ci-dessus.

L'invention a notamment pour objet les produits de formule (I) telle que définie ci-dessus telle que p représente l'entier 2, les autres substituants desdits produits de formule (I) ayant les valeurs définies dans la présente invention.

L'invention a notamment pour objet les produits de formule (I) telle que définie ci-dessus telle que R1 représente 0, les autres substituants desdits produits de formule (I) ayant l'une quelconque des valeurs définies ci-dessus.

L'invention a notamment pour objet les produits de formule (I) telle que définie ci-dessus telle que R représente 0, les autres substituants desdits produits de formule (I) ayant l'une quelconque des valeurs définies ci-dessus.

L'invention a notamment pour objet les produits de formule (I) telle que définie ci-dessus telle que R2 et R3, identiques ou différents représentent hydrogène, alkyle, alkényle, cycloalkyle, cycloalkylalkyle, phényle, phénylalkyle, hétérocycloalkyle, hétérocycloalkylalkyle, hétéroaryle et hétéroarylalkyle, éventuellement substitués ou bien R2 et R3 forment ensemble avec l'atome de carbone auxquels ils sont liés un radical carbocyclique ou hétérocyclique, ces radicaux étant constitués de 3 à 10 chaînons et le radical hétérocyclique renfermant un ou plusieurs hétéroatomes choisis parmi O, S, N et NR7b, tous ces radicaux étant éventuellement substitués, tous les radicaux ci-dessus étant éventuellement substitués par un ou plusieurs radicaux choisis parmi halogène, cyano, hydroxy, alkyle et alcoxy renfermant 1 à 4 atomes de carbone, CF3, nitro, phényle, carboxy libre, salifié, estérifié par un radical alkyle ou amidifié par un radical NR11bR12b, -C(=O)-R9b, -NR11bR12b et -C(=O)-NR11bR12b,
R7b représente un atome d'hydrogène, un radical alkyle ou un radical phényle,
R9 représente hydrogène, alkyle, cycloalkyle, cycloalkylalkyle et phényle,
R11b et R12b, identiques ou différents, représentent hydrogène, alkyle, cycloalkyle et phényle ou bien R11b et R12b forment avec l'atome d'azote auxquels ils sont liés un radical pipérazinyle éventuellement substitué,
les autres substituants desdits produits de formule (I) ayant l'une quelconque des valeurs définies ci-dessus.

L'invention a notamment pour objet les produits de formule (I) telle que définie ci-dessus telle que R2 et R3 identiques ou différents sont choisis parmi hydrogène, alkyle, phénylalkyle, pyridylalkyle et benzothiénylalkyle et thiénylbenzothiénylalkyle, éventuellement substitués par un ou plusieurs radicaux choisis parmi les atomes d'halogène et les radicaux hydroxy, alkyle et alcoxy renfermant un à 4 atomes de carbone ou bien R2 et R3 forment ensemble avec l'atome de carbone auxquels ils sont liés un radical cycloalkyle ou hétérocycloalkyle de 3 à 6 chaînons renfermant un atome d'azote, les autres substituants desdits produits de formule (I) ayant l'une quelconque des valeurs définies ci-dessus.

L'invention a notamment pour objet les produits de formule (I) telle que définie ci-dessus telle que R2 et R3 identiques ou différents sont choisis parmi hydrogène, alkyle, hydroxyalkyle, phénylalkyle, hydroxyphénylalkyle, pyridylalkyle, benzothiénylalkyle, thiénylbenzothiénylalkyle ou bien R2 et R3 forment ensemble avec l'atome de carbone auxquels ils sont liés un radical cycloalkyle renfermant de 3 à 6 atomes de carbone ou un radical azétidinyle, pyrrolidinyle et pipéridinyle,
les autres substituants desdits produits de formule (I) ayant l'une quelconque des valeurs définies ci-dessus.

L'invention a notamment pour objet les produits de formule (I) telle que définie ci-dessus telle que R2 et R3 identiques ou différents sont choisis parmi hydrogène, alkyle, hydroxyalkyle, phénylalkyle et hydroxyphénylalkyle, ou bien R2 et R3 forment ensemble avec l'atome de carbone auxquels ils sont liés un radical cycloalkyle renfermant de 3 à 6 atomes de carbone.

L'invention a notamment pour objet les produits de formule (I) telle que définie ci-dessus telle que l'un de R2 et R3 est choisi parmi hydrogène et alkyle, et l'autre de R2 et R3 est choisi parmi toutes les valeurs de R2 et R3 ou bien R2 et R3 forment ensemble avec l'atome de carbone auxquels ils sont liés un radical cycloalkyle renfermant de 3 à 6 atomes de carbone,
les autres substituants desdits produits de formule (I) ayant l'une quelconque des valeurs définies ci-dessus.

L'invention a notamment pour objet les produits de formule (I) telle que définie ci-dessus telle que R2 et R3 identiques ou différents représentent hydrogène et alkyle, ou bien R2 et R3 forment ensemble avec l'atome de carbone auxquels ils sont liés un radical cycloalkyle renfermant de 3 à 6 atomes de carbone,
les autres substituants desdits produits de formule (I) ayant l'une quelconque des valeurs définies ci-dessus.

L'invention a notamment pour objet les produits de formule (I) telle que définie ci-dessus telle que R2 et R3 identiques ou différents représentent hydrogène et CH3, ou bien R2 et R3 forment ensemble avec l'atome de carbone auxquels ils sont liés un radical cyclopropyle, les autres substituants desdits produits de formule (I) ayant l'une quelconque des valeurs définies ci-dessus.

L'invention a notamment pour objet les produits de formule (I) telle que définie ci-dessus telle que A1 représente une simple liaison et A2 est choisi parmi une simple liaison, un radical alkyle linéaire ou ramifié renfermant au plus 6 atomes de carbone et les radicaux allyle, propynyle, C=o et SO2, les autres substituants desdits produits de formule (I) ayant l'une quelconque des valeurs définies ci-dessus.

L'invention a notamment pour objet les produits de formule (I) telle que définie dans la présente invention telle que A1 représente une simple liaison et A2 est choisi parmi une simple liaison, les radicaux alkyle, allyle, propynyle, C=O et SO2, les autres substituants desdits produits de formule (I) ayant les valeurs définies ci-dessus.

L'invention a notamment pour objet les produits de formule (I) telle que définie ci-dessus telle que A1 représente une simple liaison et A2 est choisi parmi les radicaux alkyle, allyle, propynyle, C=o et SO2, les autres substituants desdits produits de formule (I) ayant l'une quelconque des valeurs définies ci-dessus.

L'invention a notamment pour objet les produits de formule (I) telle que définie ci-dessus telle que A1 représente une simple liaison et A2 représente un radical alkyle ou C=O, les autres substituants desdits produits de formule (I) ayant l'une quelconque des valeurs définies ci-dessus.

L'invention a notamment pour objet les produits de formule (I) telle que définie ci-dessus telle que A1 représente une simple liaison et A2 représente C=O, -CH2-CH2- ou -CH2, les autres substituants desdits produits de formule (I) ayant l'une quelconque des valeurs définies ci-dessus.

L'invention a notamment pour objet les produits de formule (I) telle que définie ci-dessus telle que A1 représente une simple liaison et A2 représente -CH2, les autres substituants desdits produits de formule (I) ayant l'une quelconque des valeurs définies ci-dessus.

L'invention a notamment pour objet les produits de formule (I) telle que définie ci-dessus dans laquelle Y et Y1 sont tels que l'un représente un atome d'hydrogène, un atome d'halogène ou un radical amino et l'autre est choisi parmi -OCF3, -O-CF2-CHF2, -O-CHF2, -O-CH2-CF3, -SF5, -S(O)n-CF3, -S(O)n-Alk, -SO2CHF2, SO2CF2CF3, -SO2NH2, -S-CF2-CF2-CF3, -S-Alk-O-Alk, -S-Alk-OH, -S-Alk-CN, -S-Alk-morpholino, -S-Alk-pyrrolidinyle et -S-Alk-pipérazinyle, les radicaux morpholino, pyrrolidinyle et pipérazinyle étant éventuellement substitué par Alk, avec Alk représente un radical alkyle renfermant de 1 à 4 atomes de carbone,
les autres substituants desdits produits de formule (I) étant choisis parmi les valeurs définies dans la présente invention.

L'invention a notamment pour objet les produits de formule (I) telle que définie ci-dessus telle que Y représente un atome d'hydrogène et Y1 est choisi parmi -OCF3, S(O)n-CF3, S(O)n-CH3, S02CHF2 et SO2NH2,
les autres substituants desdits produits de formule (I) ayant l'une quelconque des valeurs définies ci-dessus.

L'invention a notamment pour objet les produits de formule (I) telle que définie ci-dessus telle que Y représente un atome d'hydrogène et Y1 est choisi parmi -OCF3, S(O)n-CF3 et SO2CHF2,
les autres substituants desdits produits de formule (I) ayant l'une quelconque des valeurs définies ci-dessus.

L'invention a notamment pour objet les produits de formule (I) telle que définie ci-dessus telle que Y représente un atome d'hydrogène et Y1 est choisi parmi -OCF3 et S(O)n-CF3,
les autres substituants desdits produits de formule (I) ayant l'une quelconque des valeurs définies ci-dessus.

L'invention a notamment pour objet les produits de formule (I) telle que définie ci-dessus telle que Y représente un atome d'hydrogène et Y1 est choisi parmi -OCF3, S-CF3 et S(O)2-CF3,
les autres substituants desdits produits de formule (I) ayant l'une quelconque des valeurs définies ci-dessus.

L'invention a notamment pour objet les produits de formule (I) telle que définie ci-dessus dans laquelle B2 représente un radical hétéroaryle monocyclique ou bicyclique choisi parmi les radicaux pyridyle, pyrimidinyle, quinolyle, azaindolyle, 1H-pyrrolo[2,3-b]pyridinyle, quinazolyle, thiazolyle, imidazolyle, pyrazolyle, furazanyle, isoxazolyle, morpholinyle, pyrrolidinyle, furyle, pipéridyle, thiényle, chroményle, oxochroményle, indolyle, pyrrolyle, purinyle, benzoxazinyle, benzimidazolyle, benzofurannyle ces radicaux étant éventuellement substitués par un ou plusieurs radicaux choisis parmi les valeurs de Y2,
les autres substituants desdits produits de formule (I) ayant les valeurs définies pour les produits de formule (I).

L'invention a notamment pour objet les produits de formule (I) telle que définie ci-dessus telle que B2 représente un radical hétéroaryle choisi parmi les radicaux 3- ou 4- pyridyle, 3- ou 4- quinolyle, imidazolyle, thiazolyle, indolyle, pyrazolyle, pyrrolyle, pyrimidyle, purinyle, benzoxazinyle, benzimidazolyle, benzofurannyle ces radicaux étant éventuellement substitués par un ou plusieurs radicaux choisis parmi les valeurs de Y2,
les autres substituants desdits produits de formule (I) ayant l'une quelconque des valeurs définies ci-dessus.

L'invention a notamment pour objet les produits de formule (I) telle que définie ci-dessus telle que B2 représente un radical hétéroaryle choisi parmi les radicaux 4- pyridyle, 4- quinolyle, imidazolyle, thiazolyle, pyrazolyle, pyrrolyle, pyrimidyle et purinyle, ces radicaux étant éventuellement substitués par un ou plusieurs radicaux choisis parmi les valeurs de Y2,
les autres substituants desdits produits de formule (I) ayant l'une quelconque des valeurs définies ci-dessus.

Notamment dans les produits de formule (I), B2 représente un radical hétéroaryle choisi parmi les radicaux 3- ou 4-pyridyle, pyrimidinyle, 3- ou 4- quinolyle, azaindolyle, quinazolyle, thiazolyle, imidazolyle, pyrazolyle, furazanyle et isoxazolyle,
ces radicaux étant éventuellement substitués par un ou plusieurs radicaux choisis parmi les valeurs de Y2, les autres substituants desdits produits de formule (I) ayant les valeurs définies dans la présente invention.

Notamment dans les produits de formule (I), B2 représente un radical hétéroaryle choisi parmi les radicaux 3- ou 4-pyridyle, pyrimidinyle, 3- ou 4- quinolyle, azaindolyle et quinazolyle, ces radicaux étant éventuellement substitués par un ou plusieurs radicaux choisis parmi les valeurs de Y2,
les autres substituants desdits produits de formule (I) ayant les valeurs définies dans la présente invention.

La présente invention a pour objet les produits de formule (I) telle que définie dans la présente invention telle que B2 représente les radicaux 4- pyridyle et 4-quinolyle, 1H-Pyrrolo[2,3-b]pyridine-4-yl éventuellement substitués par un ou plusieurs radicaux choisis parmi les valeurs de Y2 définies dans la présente invention,
les autres substituants desdits produits de formule (I) ayant les valeurs définies dans la présente invention.

Notamment dans les produits de formule (I) de la présente invention, Y2 peut représenter le radical 2-aminopyridine-4-yl dans lequel le radical amino est éventuellement substitué comme indiqué pour le radical NR5R6 tel que défini ci-dessus ou ci-après et dans la partie expérimentale, les autres subtituants desdits produits de formule (I) pouvant prendre les valeurs telles que définis dans la présente invention pour lesdits substituants.

Notamment les produits de formule (I) de la présente invention sont tels que Y2 représente hydrogène, halogène, hydroxyle, cyano, alkyle, alcoxy, phényle, COOH, COOAlk, CONR5R6, NR5R6, -NR10-COOR6, -NR10-CO-R6, -NR10-CS-NR5R6, -NR10-CO-NR5R6 ou -NR10-SO2-R6, tous ces radicaux étant éventuellement substitués,
R5 et R6 identiques ou différents sont choisis parmi hydrogène, alkyle, cycloalkyl, phényle et les radicaux hétéroaryles renfermant 5 à 6 chaînons et contenant 1 à 3 hétéroatomes choisis parmi O, N et S, tous ces radicaux étant éventuellement substitués, ou bien R5 et R6 forment avec l'atome d'azote auxquels ils sont liés un radical pyrrolidinyle, pipéridyle, pipérazinyle, morpholinyle ou quinazolinyle éventuellement substitués
R10 représente hydrogène ou alkyle,
tous les radicaux ci-dessus alkyle, alcoxy, cycloalkyle et phényle ainsi que le cycle formé par R5 et R6 avec l'atome auxquels ils sont liés étant éventuellement substitués par un ou plusieurs radicaux identiques ou différents choisis parmi les atomes d'halogène et les radicaux cyano; hydroxyle ; alkyle ; alcoxy ; OCF3 ; CF3 ; S(O)n-CF3 ; nitro ; oxo ; thioxo ; OCOAlk ; phényle lui-même éventuellement substitué par un ou plusieurs radicaux choisis parmi les atomes d'halogène et les radicaux alkyle et alcoxy ; -OCOAlk ; NH2, NHAlk, N(Alk)2, N(alk)(phénylalkyle), N(Alk)(aminoalkyle) N(Alk)(alkylaminoalkyle)
N(Alk)(dialkylaminoalkyle ; carboxy libre ou estérifié par un radical alkyle
tous les radicaux ci-dessus phényle étant de plus éventuellement substitués par un radical alkylènedioxy,.. tous les radicaux ci-dessus alkyle étant de plus éventuellement substitués par un ou plusieurs radicaux hétérocycliques saturés ou partiellement insaturés renfermant 4 à 7 chaînons et contenant au moins un atome d'azote N et en plus 0 à 2 autres hétéroatomes choisi(s) parmi O, N et S,
tous les radicaux pyrrolidinyle et quinazolinyle ci-dessus étant de plus éventuellement substitués par oxo ou thioxo,
tous les radicaux ci-dessus alkyle et alcoxy, étant linéaires ou ramifiés et renfermant au plus 6 atomes de carbone,
tous les radicaux ci-dessus cycloalkyle renfermant au plus 7 atomes de carbone,
les autres substituants desdits produits de formule (I) ayant les valeurs définies ci-dessus.

Notamment, R5 et R6 peuvent représenter les radicaux hétéroaryles suivants : pyridinyle, pyrazinyle, pyrimidinyle, thiényle, thiazolyle et oxazolyle, tous ces radicaux étant éventuellement substitués,

Notamment dans les produits de formule (I) de la présente invention, les radicaux alkyle peuvent être substitués par des radicaux hétérocycliques choisis parmi les radicaux suivants : les radicaux thiomorpholine-4-yl, thiazolidine-3-yl, àzetidine-1-yl, pipérazinyle, imidazolyle, morpholinyle, pyrrolidinyle, pipéridyle et azépanyl, tous ces radicaux étant éventuellement substitués comme indiqué ci-dessus et notamment par un ou plusieurs radicaux choisis parmi alkyle, hydroxyalkyle, oxo, pyridyle et phényle éventuellement substitué par un ou plusieurs radicaux choisis parmi les atomes d'halogène et le radicaux alkyle, hydroxyle, alcoxy, CN, carboxy ou amino, eux-mêmes éventuellement substitués.

On peut citer par exemple les radicaux pipérazinyle éventuellement substitué par Alk, Alk-OH, pyridyle ou phényle lui-même éventuellement substitué par un ou plusieurs radicaux choisis parmi les atomes d'halogène et le radicaux alkyle, hydroxyle, alcoxy, CN, carboxy ou amino, eux-mêmes éventuellement substitués.; pipéridyle éventuellement substitué par un ou deux Alk ; azépanyl éventuellement substitué par oxo.

La présente invention a pour objet les produits de formule (I) telle que définie ci-dessus telle que Y2 représente hydrogène, halogène, hydroxyle, cyano, alkyle, alcoxy, phényle, CONR5R6, NR5R6, -NR10-COOH, -NR10-COOAlk, -NR10-CO-R6, -NR10-CS-NR5R6, -NR10-CO-NR5R6 ou -NR10-SO2-R6,
R5 et R6 identiques ou différents sont choisis parmi hydrogène ; alkyle ; cycloalkyle ; phényle i pyrimidinyle ; thiényle ; pyridyle ; quinolyle ; thiazolyle éventuellement substitué par un ou deux atomes d'halogène ; pyranne éventuellement substitué par un ou plusieurs OCOAlk ; phényle substitué par un ou plusieurs radicaux choisis parmi les atomes d'halogène et les radicaux alkyle, alcoxy, amino, alkylamino, dialkylamino et carboxy libre ou estérifié par un radical alkyle ; alkyle substitué par phényle lui-même éventuellement substitué par un ou plusieurs radicaux choisis parmi les atomes d'halogène, alkyle, alcoxy, amino, alkylamino, dialkylamino, carboxy libre ou estérifié par un radical alkyle ; alkyle substitué par pipérazinyle lui-même éventuellement substitué par un ou plusieurs radicaux choisis parmi Alk, Alk-OH et pyridyle ; alkyle substitué par imidazolyle ; alkyle substitué par un ou plusieurs radicaux choisis parmi NH2, NHAlk, N(Alk)2, N(alk)(phénylalkyle), N(Alk)(aminoalkyle) N(Alk) (alkylaminoalkyle)et N(Alk)(dialkylaminoalkyle) ; alkyle substitué par morpholinyle éventuellement substitué par un ou deux Alk ; alkyle substitué par pyrrolidinyle ; alkyle substitué par pipéridyle lui-même éventuellement substitué par un ou deux Alk ; alkyle substitué par thiomorpholinyle alkyle substitué par azetidinyle ; alkyle substitué par azépanyle éventuellement substitué par oxo,
ou bien R5 et R6 forment avec l'atome d'azote auxquels ils sont liés un radical pyrrolidinyle ; pipéridyle ; pipérazinyle ; morpholinyle ; ou quinazolinyle, tous ces radicaux étant éventuellement substitués par un ou plusieurs radicaux identiques ou différents choisis parmi les atomes d'halogène et les radicaux alkyle, hydroxyle, alcoxy et phényle lui-même éventuellement substitué par un ou plusieurs radicaux choisis parmi les atomes d'halogène et les radicaux alkyle et alcoxy,
les radicaux pyrrolidinyle et quinazolinyl étant de plus éventuellement substitué par oxo ou thioxo,
le radical pipérazinyle lui-même éventuellement substitué, par un ou plusieurs radicaux choisis parmi Alk, Alk-OH et pyridyle,
R10 représente hydrogène ou alkyle,
tous les radicaux ci-dessus alkyle ou Alk et alcoxy, étant linéaires ou ramifiés et renfermant au plus 6 atomes de carbone,
tous les radicaux ci-dessus cycloalkyle renfermant au plus 7 atomes de carbone,
tous les radicaux phényle étant de plus éventuellement substitués par un radical choisi parmi CF3, -OCF3, nitro et alkylènedioxy,
les autres substituants desdits produits de formule (I) ayant l'une quelconque des valeurs définies ci-dessus.

Parmi les structures préférées de la présente invention, on peut citer les produits de formule (I) dont les trois structures suivent, dans lesquelles les valeurs de NR14R15 sont choisies parmi les valeurs de NR5R6 et les valeurs des radicaux Alkyle, Aryl et Heteroaryl sont choisies parmi les valeurs des radicaux alkyle, aryle et hétéroaryle telles que définies ci-dessus et éventuellement substitués comme défini dans la présente invention.

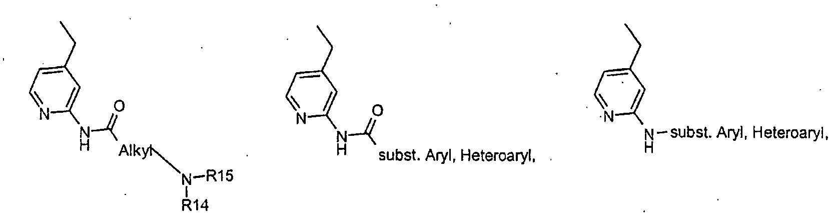

L'invention a notamment pour objet les produits de formule (I) telle que définie ci-dessus telle que B2 représente les radicaux 4- pyridyle et 4- quinolyle, éventuellement substitués par un ou plusieurs radicaux choisis parmi les valeurs de Y2, les autres substituants desdits produits de formule (I) ayant l'une quelconque des valeurs définies ci-dessus.

L'invention a notamment pour objet les produits de formule (I) telle que définie ci-dessus telle que Y2 représente V1, halogène, hydroxyle, -C(=NH)NH2, OV1, O-CO-V1, COOV1, COV1, CO-NV1V2, -NV1V2, -NH-CO-V1, -NH-COO-V1, -NH-NH-CO-V1, , -NV1-CO-NV1V2, -NV1-CQ-NHV1, -NH-CO-NHV1, -NH-SO2-NHV1 et -NH-SO2-V1,
dans lesquels V1 et V2 identiques ou différents représentent un atome d'hydrogène, un radical alkyle, cycloalkyle ou phényle ou un radical hétérocyclique tel que pyridinyle, pyrazolyle, imidazolyle, dihydroimidazolyle, tétrazolyle, morpholinyle, pipérazinyle, pipérazinylalkyle, alkylpipérazinyle, phénylpipérazinyle, thiényle, furannyle, pipéridinyle, méthylpipéridinyle, pyridyle, pyrrolidinyle et pyrrolidinylalkyle,
tous les radicaux alkyle, phényle et hétérocyclique étant éventuellement substitués par un ou plusieurs radicaux choisis parmi les atomes d'halogène, les radicaux hydroxyle, alkyle, alcoxy, CF3, NH2, NHalk, N(alk)2 et phényle lui-même éventuellement substitué par un ou plusieurs substituants choisis parmi les atomes d'halogène, les radicaux hydroxy et alcoxy,
tous les radicaux phényle et hétérocyclique ci-dessus étant de plus éventuellement substitués par un ou plusieurs radicaux alkyle,
les radicaux phényle étant de plus éventuellement substitués par NR5R6 avec R5 et R6 tels que définis ci-dessus,
les autres substituants desdits produits de formule (I) ayant l'une, quelconque des valeurs définies ci-dessus.

L'invention a notamment pour objet les produits de formule (I) telle que définie ci-dessus telle que Y2 représente hydrogène, halogène, alkyle, cycloalkyle, hydroxyle, alcoxy, carboxy libre ou estérifié par un radical alkyle ou phényle, NH2, NHalk, N(alk)2 et phényle,
tous les radicaux alkyle, alcoxy et phényle étant éventuellement substitués par un ou plusieurs radicaux choisis parmi les atomes d'halogène, les radicaux hydroxyle, C1-C4alkyle, C1-C4alcoxy, CF3, NH2, NHalk, N(alk)2 et phényle lui-même éventuellement substitué par un ou plusieurs substituants choisis parmi les atomes d'halogène, les radicaux hydroxy et alcoxy,
tous les radicaux phényle étant de plus éventuellement substitués par un ou plusieurs radicaux C1-C4alkyle et éventuellement substituées par NR5R6 avec R5 et R6 tels que définis ci-dessus,
les autres substituants desdits produits de formule (I) ayant l'une quelconque des valeurs définies ci-dessus.
L'invention a notamment pour objet les produits de formule (I) telle que définie ci-dessus telle que Y2 représente hydrogène, F, C1, CH3, CH2CH3, OH, OCH3, NH2, NHalk et phényle éventuellement substitué par NR5R6 avec R5 et R6 tels que définis ci-dessus,
les autres substituants desdits produits de formule (I) ayant l'une quelconque des valeurs définies ci-dessus.

L'invention a notamment pour objet les produits de formule (I) telle que définie ci-dessus telle que B2 représente les radicaux 4- pyridyl et 4- quinolyl substitués by un ou deux radicaux choisis parmi F, Cl, OH et OCH3,
les autres substituants desdits produits de formule (I) ayant l'une quelconque des valeurs définies ci-dessus.

Dans certains produits de formule (I) de la présente invention, le groupe suivant : 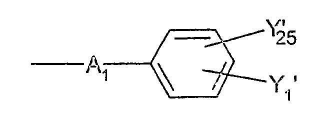 peut notamment représenter les radicaux suivants :

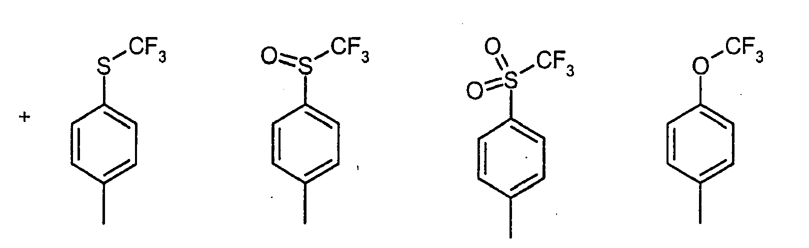

Dans certains produits de formule (I) de la présente invention, le groupe -A2-B2-Y2 peut notamment représenter les radicaux suivants :

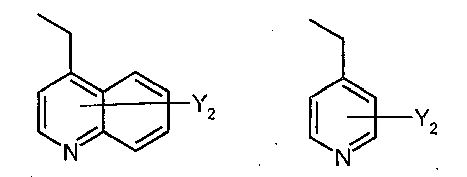

Dans les produits de formule (I) de la présente invention, R2 et R3 peuvent notamment former ensemble un radical cycloalkyle ou hétérocycloalkyle ou bien identiques ou différents peuvent représenter notamment les radicaux hydrogène et méthyle.

Produits de formule (I) telle que définie ci-dessus répondant à la formule (IC) _{:} 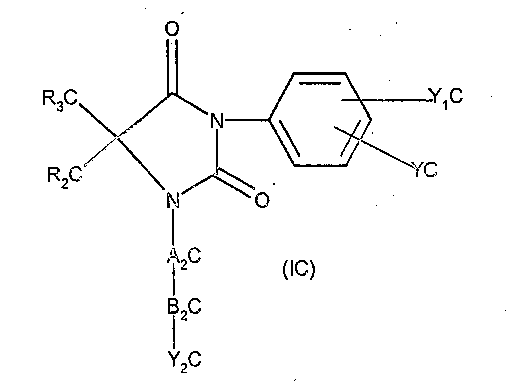 dans laquelle YC et Y1C sont tels que l'un représente un atome d'hydrogène, un atome d'halogène ou un radical amino et l'autre est choisi parmi -OCF3, -O-CF2-CHF2, -O-CHF2, -O-CH2-CF3, -SF5, -S(O)n-CF3, -S(O)n-Alk, -S02CHF2, SO2CF2CF3,-SO2NH2, -S-CF2-CF2-CF3, -S-Alk-O-Alk, -S-Alk-OH, -S-Alk-CN, -S-Alk-morpholino, -S-Alk-pyrrolidinyle et -S-Alk-pipérazinyle, les radicaux morpholino, pyrrolidinyle et pipérazinyle étant éventuellement substitué par Alk, avec Alk représente un radical alkyle renfermant de 1 à 4 atomes de carbone,
ou bien le radical phényle forme avec ses substituants YC et Y1C l'un des deux radicaux suivants : 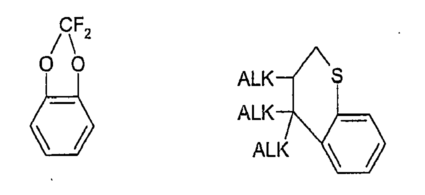 R2C et R3C identiques ou différents représentent hydrogène ou alkyle éventuellement substitué ou bien R2C et R3C forment ensemble avec l'atome de carbone auxquels ils sont liés un radical C3-C10 cycloalkyle ou hétérocycloalkyle,
A2C représente une simple liaison ou CH2,
B2C représente les radicaux pyridyle, pyrimidinyle, quinolyle, azaindolyle, quinazolyle, thiazolyle, imidazolyle, pyrazolyle, furazannyle, isoxazolyle, morpholinyle, pyrrolidinyl, furyle, pipéridyle, chroményle, oxochroményle, quinazolyle, thiényle, indolyle, pyrrolyle, purinyle, benzoxazinyle, benzimidazolyle, benzofurannyle éventuellement substitués par un ou plusieurs radicaux choisis parmi les valeurs de Y2A,
Y2CA représente hydrogène, halogène, hydroxyle, cyano, alkyle, alcoxy, phényle, COOH, COOAlk, CONR5R6, NR5R6, -NR10-COOH, -NR10-COOAlk, -NR10-CO-R6, -NR10-CS-NR5R6, -NR10-CO-NR5R6 ou -NR10-SO2-R6 tous ces radicaux étant éventuellement substitués,
R5 et R6 identiques ou différents sont choisis parmi hydrogène, alkyle, cycloalkyle, phényle, pyrimidinyle, thiényle, pyridyle, quinolyle, thiazolyle et pyranne, tous ces radicaux étant éventuellement substitués, ou bien R5 et R6 forment avec l'atome d'azote auxquels ils sont liés un radical pyrrolidinyle, pipéridyle, pipérazinyle, morpholinyle ou quinazolinyle éventuellement substitués,
R10 représente hydrogène ou alkyle,
tous les radicaux ci-dessus alkyle, Alk ou ALK, alcoxy, cycloalkyle et phényle ainsi que le cycle formé par R5 et R6 avec l'atome auxquels ils sont liés étant éventuellement substitués par un ou plusieurs radicaux identiques ou différents choisis parmi les atomes d'halogène et les radicaux cyano ; hydroxyle ; alkyle ; alcoxy ; OCF3 ; CF3 ; S(O)n-CF3 ; nitro ; oxo ; thioxo ; OCOAlk ; phényle lui-même éventuellement substitué par un ou plusieurs radicaux choisis parmi les atomes d'halogène et les radicaux alkyle et alcoxy ; -OCOAlk ; NH2, NHAlk, N(Alk)2, N(alk)(phénylalkyle), N(Alk)(aminoalkyle) N(Alk)(alkylaminoalkyle) N(Alk)(dialkylamino-alkyle) ; carboxy libre ou estérifié par un radical alkyle
tous les radicaux ci-dessus phényle étant de plus éventuellement substitués par un radical alkylènedioxy, tous les radicaux ci-dessus alkyle étant de plus éventuellement substitués par un ou plusieurs radicaux choisis parmi les radicaux pipérazinyle lui-même éventuellement substitué par Alk, Alk-OH et pyridyle ; imidazolyle ; morpholinyle ; pyrrolidinyle ; pipéridyle, lui-même éventuellement substitué par un ou deux àlk; azépanyle éventuellement substitué par oxo,
tous les radicaux pyrrolidinyle et quinazolinyle ci-dessus étant de plus éventuellement substitués par oxo ou thioxo,
tous les radicaux ci-dessus alkyle et alcoxy, étant linéaires ou ramifiés et renfermant au plus 6 atomes de carbone,
tous les radicaux ci-dessus cycloalkyle renfermant au plus 7 atomes de carbone,
n représente un entier de 0 à 2,
lesdits produits de formule (IC) étant sous toutes les formes isomères possibles racémiques, énantiomères et diastéréo-isomères, ainsi que les sels d'addition avec les acides minéraux et organiques ou avec les bases minérales et organiques desdits produits de formule (IC).

Notamment dans des produits de formule (I), le radical ci-après que peut former phényle avec ses substituants Y et Y1 _{:} 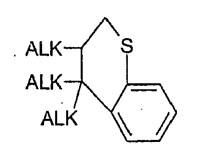 peut représenter le radical suivant :

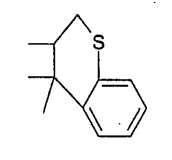

La présente invention a notamment pour objet les produits de formule (I) telle que définie ci-dessus et répondant à la formule (IA) _{:} 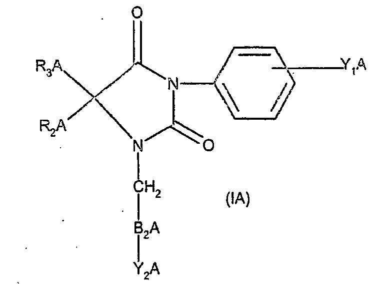 dans laquelle :
Y1A représente -OCF3, S(O)n-CF3 et SO2CHF2,
B2a représente les radicaux 4-quinolyl et 4-pyridyl éventuellement substitués par un ou plusieurs radicaux choisis parmi les valeurs de Y2A,
Y2A a la signification indiquée ci-dessus pour Y2,
R2A et R3A identiques ou différents représentent hydrogène ou alkyle éventuellement substitué ou bien R2A et R3A forment ensemble avec l'atome de carbone auxquels ils sont liés un radical C3-C10 cycloalkyle ou hétérocycloalkyle,
tous les radicaux alkyle et phényle étant éventuellement substitués par un ou plusieurs radicaux choisis parmi Halogène, OH, alk, Oalk, OCF3, S(O)n-CF3, CF3, NH2, NHAlk et N(alk)2,
n représente un entier de 0 à 2,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

La présente invention a notamment pour objet les produits de formule (IA) telle que définie ci-dessus dans laquelle Y1A, B2a, R2A et R3A ont les significations indiquées précédemment et Y2A représente les radicaux halogène, -OH, -alk, Oalk, -Oacyl, -NR5AR6A, -CO2H, -CO2alk, -CO-NR5AR6A, -S(O)n-CF3, -NH-S(O)n-CF3 ou phényle, alk représentant un radical alkyle linéaire ou ramifié renfermant au plus 6 atomes de carbone, tous les radicaux alkyle, alcoxy et phényle étant éventuellement substitués,
R5A et R6A identiques ou différents représentent hydrogène, alkyle, cycloalkyle, phényle, les radicaux alkyle et phényle étant éventuellement substitués ou bien R5A et R6A forment avec l'atome d'azote auxquels ils sont liés un radical cyclique choisi parmi les radicaux pyrrolidinyle, pipéridinyle, pipérazinyle, morpholinyle, indolinyle, pyrindolinyle, tétrahydroquinoline et azétidine,
tous les radicaux alkyle, alcoxy et phényle étant éventuellement substitués par un ou plusieurs radicaux choisis parmi Halogène, OH, alk, Oalk, OCF3, S(O)n-CF3, CF3, NH2, NHAlk et N(alk)2,
n représente un entier de 0 à 2,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

La présente invention a notamment pour objet les produits de formule (IA) telle que définie ci-dessus dans laquelle :
Y1A représente -OCF3, SCF3 ou S(O)2-CF3,
B2a représente un radical 4-quinolyle ou 4-pyridyle éventuellement substitués par un ou deux radicaux choisis parmi halogène, -OH, alk, -Oalk, -CO2H, -CO2alk, -NR5AR6A, -CF3, -OCF3 et phényle éventuellement substitué,
R5A et R6A identiques ou différents représentent hydrogène, alkyle, cycloalkyle, phényle, les radicaux alkyle et phényle étant éventuellement substitués,
ou bien R5A et R6A forment avec l'atome d'azote auxquels ils sont liés un radical cyclique choisi parmi les radicaux pyrrolidinyle, pipéridinyle, pipérazinyle, morpholinyle, pipérazinyle et azétidine,
R2A et R3A identiques ou différents représentent hydrogène ou alkyle éventuellement substitué ou bien R2A et R3A forment ensemble avec l'atome de carbone auxquels ils sont liés un radical C3-C6 cycloalkyle ou hétérocycloalkyle,
tous les radicaux alkyle et phényle étant éventuellement substitués par un ou plusieurs radicaux choisis parmi Halogène, OH, alk, Oalk, OCF3, S(O)n-CF3, CF3, NH2, NHalk et N(alk)2,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

La présente invention a notamment pour objet les produits de formule (IA) telle que définie ci-dessus répondant à la formule (IA) dans laquelle :
Y1A représente -OCF3, SCF3 ou S(O)2-CF3,
B2a représente un radical 4-quinolyle ou 4-pyridyle éventuellement substitués par un ou deux radicaux choisis parmi halogène, -OH, alk et -Oalk,
R2A et R3A identiques ou différents représentent hydrogène et alkyle linéaire ou ramifié renfermant au plus 4 atome de carbone éventuellement substitué par un radical hydroxyle ou bien R2A et R3A forment ensemble avec l'atome de carbone auxquels ils sont liés un radical C3-C6 cycloalkyle
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

La présente invention a notamment pour objet les produits de formule (IA) telle que définie ci-dessus répondant à la formule (IA) dans laquelle Y1a représente OCF3, SCF3 ou S(O)2CF3 et R2A et R3A identiques ou différents représentent hydrogène et CH3 ou bien R2A et R3A forment ensemble avec l'atome de carbone auxquels ils sont liés un radical cyclopropyle,
les autres substituants ayant l'une quelconque des valeurs définies ci-dessus,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

La présente invention a encore pour objet les produits de formule (I) telle que définie ci-dessus répondant à la formule (IB) : 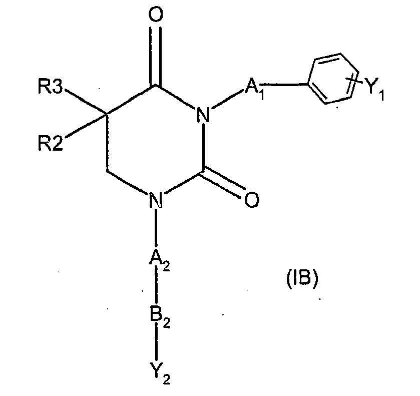 dans laquelle R2, R3, A1, Y, Y1, A2, B2 et Y2 ont les significations indiquées ci-dessus
lesdits produits de formule (IB) étant sous toutes les formes isomères possibles racémiques, énantiomères et diastéréo-isomères, ainsi que les sels d'addition avec les acides minéraux et organiques ou avec les bases minérales et organiques desdits produits de formule (IB).

La présente invention a encore pour objet les produits de formule (IB) telle que définie ci-dessus dans laquelle Y1 représente OCF3, SCF3 ou S(O)2CF3 et R2 et R3 identiques ou différents représentent hydrogène et CH3 ou bien R2 et R3 forment ensemble avec l'atome de carbone auxquels ils sont liés un radical cyclopropyle,
les autres substituants ayant l'une quelconque des valeurs définies ci-dessus,
lesdits produits de formule (IB) étant sous toutes les formes isomères possibles racémiques, énantiomères et diastéréo-isomères, ainsi que les sels d'addition avec les acides minéraux et organiques ou avec les bases minérales et organiques desdits produits de formule (IB).

Parmi les produits préférés de l'invention, on peut citer plus précisément les produits de formule (I) telle que définie ci-dessus dont les noms suivant :
- le trifluoroacétate de (S)-5-méthyl-1-quinolin-4-ylméthyl-3-(4-trifluorométhanesulfonyl-phényl)-imidazolidine-2,4-dione
- le trifluoroacétate de (S)-5-méthyl-1-pyridin-4-ylméthyl-3-(4-trifluorométhanesulfonyl-phényl)-imidazolidine-2,4-dione
- le trifluoroacétate de (S)-5-méthyl-1-quinolin-4-ylméthyl-3-(4-trifluorométhylsulfanyl-phényl)-imidazolidine-2,4-dione
- le trifluoroacétate de 5,5-diméthyl-1-quinolin-4-ylméthyl-3-(4-trifluorométhanesulfonyl-phényl)-imidazolidine-2,4-dione
- le trifluoroacétate de (R)-5-méthyl-1-quinolin-4-ylméthyl-3-(4-trifluorométhanesulfonyl-phênyl)-imidazolidine-2,4-dione
- le trifluoroacétate de (R)-5-méthyl-1-quinolin-4-ylméthyl-3-(4-trifluorométhylsulfanyl-phényl)-imidazolidine-2,4-dione
- le trifluoroacétate de (R)-5-méthyl-1-pyridin-4-ylméthyl-3-(4-trifluorométhanesulfonyl-phényl)-imidazolidine-2,4-dione
- le trifluoroacétate de (R)-5-méthyl-1-(3-méthyl-pyridin-4-yléthyl)-3-(4-trifluorométhanesulfonyl-phényl)-imidazolidine-2,4-dione
- le trifluoroacétate de (R)-4-méthyl-3-quinolin-4-ylméthyl-5-thioxo-1-(4-trifluorométhylsulfanyl-phényl)-imidazolidin-2-one
- le trifluoroacétate de (R)-5-isopropyl-1-quinolin-4-ylméthyl-3-(4-trifluorométhylsulfanyl-phényl)-imidazolidine-2,4-dione
- le trifluoroacétate de (R)-5-isopropyl-1-quinolin-4-ylméthyl-3-(4-trifluorométhanesulfonyl-phényl)-imidazolidine-2,4-dione
- le trifluoroacétate de (R)-5-(4-hydroxy-benzyl)-1-quinolin-4-ylméthyl-3-(4-trifluorométhanesulfonyl-phényl)-imidazolidine-2,4-dione
- le trifluoroacétate de (R)-5-(4-hydroxy-benzyl)-1-pyridin-4-ylméthyl-3-(4-trifluorométhanesulfonyl-phényl)-imidazolidine-2,4-dione
- le trifluoroacétate de (R)-5-(1-hydroxy-éthyl)-1-quinolin-4-ylméthyl-3-(4-trifluorométhanesulfonyl-phényl)-imidazolidine-2,4-dione
- le trifluoroacétate de, 4-quinolin-4-ylméthyl-6-(4-trifluorométhylsulfanyl-phényl)-4,6-diaza-spiro[2.4]heptane-5,7-dione
- le trifluoroacétate de 4-quinolin-4-ylméthyl-6-(4-trifluorométhanesulfonyl-phényl)-4,6-diaza-spiro[2.4]heptane-5,7-dione, trifluoroacétate
- le trifluoroacétate de 4-pyridin-4-ylméthyl-6-(4-trifluorométhylsulfanyl-phényl)-4,6-diaza-spiro[2.4]heptane-5,7-dione
- le trifluoroacétate de 4-pyridin-4-ylméthyl-6-(4-trifluorométhanesulfonyl-phényl)-4,6-diaza-spiro[2.4]heptane-5,7-dione
- le trifluoroacétate de (R)-1-(3-hydroxy-pyridin-4-ylméthyl)-5-méthyl-3-(4-trifluorométhylsulfanyl-phényl)-imidazolidine-2,4-dione
- le trifluoroacétate de 5,5-diméthyl-1-quinolin-4-ylméthyl-3-(4-trifluorométhooxy-phényl)-imidazolidine-2,4-dione,
- le trifluoroacétate de 5,5-diméthyl-1-quinolin-4-ylméthyl-3-(4-trifluorométhylsulfanyl-phényl)-imidazolidine-2,4-dione,
- le trifluoroacétate de 5,5-diméthyl-1-(3-méthyl-pyridin-4-ylméthyl)-3-(4-trifluorométhoxy-phényl)-imidazolidine-2,4-dione,
- le trifluoroacétate de 5,5-diméthyl-1-(3-méthyl-pyridin-4-ylméthyl)-3-(4-trifluorométhylsulfanyl-phényl)-imidazolidine-2,4-dione,
- le trifluoroacétate de 5,5-diméthyl-1-(3-méthyl-pyridin-4-ylméthyl)-3-(4-trifluorométhanesulfonyl-phényl)-imidazolidine-2,4-dione,
- le trifluoroacétate de 1-(3-hydroxy-pyridin-4-ylméthyl)-5,5-diméthyl-3-(4-trifluorométhoxy-phényl)-imidazolidine-2,4-dione,
- le trifluoroacétate de 1-(3-hydroxy-pyridin-4-ylméthyl)-5,5-diméthyl-3-(4-trifluorométhylsulfanyl-phényl)-imidazolidine-2,4-dione,
- le trifluoroacétate de 1-(3-hydroxy-pyridin-4-ylméthyl)-5,5-diméthyl-3-(4-trifluorométhanesulfonyl-phényl)-imidazolidine-2,4-dione,
- le trifluoroacétate de 4-quinolin-4-ylméthyl-6-(4-trifluorométhoxy-phényl)-4,6-diaza-spiro[2.4]heptane-5,7-dione,
- le trifluoroacétate de 4-(3-méthyl-pyridin-4-ylméthyl)-6-(4-trifluorométhylsulfanylphényl)-4,6-diaza-spiro[2.4]heptane-5,7-dione,
- le trifluoroacétate de 4-(3-hydroxy-pyridin-4-ylméthyl)-6-(4-trifluorométhoxy-phényl)-4,6-diaza-spiro[2.4]heptane-5,7-dione,
- le trifluoroacétate de 4-(3-Hydroxy-pyridin-4-ylméthyl)-6-(4-trifluorométhylsulfanyl-phényl)-4,6-diaza-spiro[2.4]heptane-5,7-dione,
- le trifluoroacétate de 4-(3-hydroxy-pyridin-4-ylméthyl)-6-(4-trifluorométhanesulfonyl-phényl)-4,6-diaza-spiro[2.4]heptane-5,7-dione,
lesdits produits de formule (I) étant sous toutes les formes isomères possibles racémiques, énantiomères et diastéréo-isomères, ainsi que les sels d'addition avec les acides minéraux et organiques ou avec les base minérales et organiques desdits produits de formule (I).

Parmi les produits préférés de l'invention, on peut citer tout particulièrement les produits de formule (I) telle que définie ci-dessus dont les noms suivent :
- le Trifluoroacétate de la {4-[5,5-diméthyl-2,4-dioxo-3-(4-trifluorométhylsulfanyl- phényl)-imidazolidin-1-ylméthyl]-pyridin-2-yl}-amide de l'acide cyclopropanecarboxylique ;
- le 5,5-diméthyl-1-[2-(pyridin-2-ylamino)-pyridin-4-yl-méthyl]-3-(4-trifluorométhylsulfanyl-phényl)-imidazoli-dine-2,4-dione ; composé avec de l'acide trifluoroacétique ;
- le N-{4-[5,5-diméthyl-2,4-dioxo-3-(4-trifluorométhyl-sulfanyl-phényl)-imidazolidin-1-ylméthyl]-pyridin-2-yl}-isobutyramide ; composé avec de l'acide trifluoroacétique ;
- le N-{4-[5,5-diméthyl-2,4-dioxo-3-(4-trifluorométhyl-sulfanyl-phényl)-imidazolidin-1-ylméthyl]-pyridin-2-yl}-propionamide ; composé avec de l'acide trifluoroacétique ;
- le chlorhydrate de 1-(2-amino-pyridin-4-ylméthyl)-5,5-diméthyl-3-(4-trifluorométhylsulfanyl-phényl)-imidazolidine-2,4-dione ;
- le trifluoroacétate de la {4-[5,5-diméthyl-2,4-dioxo-3-(4-trifluorométhylsulfanyl-phényl)-imidazolidin-1-ylmé-thyl]-pyridin-2-yl}-amide de l'acide pyridine-2-carboxy-lique ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-piperidin-1-yl-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-[4-(2-hydroxy-éthyl)-piperazin-1-yl]-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-morpholin-4-yl-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-pyrrolidin-1-yl-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-(4-méthyl-piperazin-1-yl)-propion-amide ;
- le 1-{4-[5,5-diméthyl-2,4-dioxo-3-(4-trifluorométhyl-sulfanyl-phényl)-imidazolidin-1-ylméthyl]-pyridin-2-yl}-3-phényl-urée
- le 1-[2-(6-éthyl-pyridin-2-ylamino)-pyridin-4-ylmé-thyl]-5,5-diméthyl-3-(4-trifluorométhylsulfanyl-phényl)-imidazolidine-2,4-dione ;
- le 5,5-diméthyl-1-[2-(4-méthyl-pyridin-2-ylamino)-pyri-din-4-ylméthyl]-3-(4-trifluorométhylsulfanyl-phényl)-imidazolidine-2,4-dione ;
- le 5,5-diméthyl-1-[2-(6-méthyl-pyridin-2-ylamino)-pyri-din-4-ylméthyl]-3-(4-trifluorométhylsulfanyl-phényl)-imi-dazolidine-2,4-dione ;
- le 1-[2-(4,6-diméthyl-pyridin-2-ylamino)-pyridin-4-yl-méthyl]-5,5-diméthyl-3-(4-trifluorométhylsulfanyl-phényl)-imidazolidine-2,4-dione ;
- le 1-[2-(3,5-dichloro-pyridin-2-ylamino)-pyridin-4-yl-méthyl]-5,5-diméthyl-3-(4-trifluorométhylsulfanyl-phényl)-imidazolidine-2,4-dione ;
- le 5,5-diméthyl-1-[2-(pyridin-4-ylamino)-pyridin-4-yl-méthyl]-3-(4-trifluorométhylsulfanyl-phényl)-imidazoli-dine-2,4-dione ;
- le 5,5-diméthyl-1-[2-(pyridin-3-ylamino)-pyridin-4-yl-méthyl]-3-(4-trifluorométhylsulfanyl-phényl)-imidazoli-dine-2,4-dione ;
- le N-{4-[5,5-diméthyl-2,4-dioxo-3-(4-trifluorométhyl-sulfanyl-phényl)-imidazolidin-1-ylméthyl]-pyridin-2-yl}-3-(2-oxo-azépan-1-yl)-propionamide ;
- le 3-(benzyl-méthyl-amino)-N-{4-[5,5-diméthyl-2,4-di-oxo-3-(4-trifluorométhylsulfanyl-phényl)-imidazolidin-1-ylméthyl]-pyridin-2-yl}-propionamide ;
- l'acide 4,5-diacétoxy-6-acétoxyméthyl-2-(3-{4-[5,5-diméthyl-2,4-dioxo-3-(4-trifluorométhylsulfanyl-phényl)-imidazolidin-1-ylméthyl]-pyridin-2-yl}-thiouréido acétique ;
- le 5,5-diméthyl-1-[2-(5-méthyl-pyridin-2-ylamino)-pyri-din-4-ylméthyl]-3-(4-trifluorométhylsulfanyl-phényl)-imidazolidine-2,4-dione ;
- le trifluoroacétate de N-{4-[5,5-diméthyl-2,4-dioxo-3-(4-trifluorométhyl-sulfanyl-phényl)-imidazolidin-1-ylmét-hyl]-pyridin-2-yl}-3,5-diméthoxy-benzamide ;
- le trifluoroacétate de 5,5-diméthyl-1-[2-(pyrazin-2-yl-amino)-pyridin-4-ylméthyl]-3-(4-trifluorométhylsulfanyl-phényl)-imidazolidine-2,4-dione
- le trifluoroacétate de N-{4-[5,5-diméthyl-2,4-dioxo-3-(4-trifluorométhylsulfanyl-phényl)-imidazolidin-1-ylmét-hyl]-pyridin-2-yl}-3-(3-méthyl-piperidin-1-yl)-propion-amide ;
- le trifluoroacétate de N-{4-[5,5-diméthyl-2,4-dioxo-3-(4-trifluorométhylsulfanyl-phényl)-imidazolidin-1-ylmét-hyl]-pyridin-2-yl}-3-(3,5-diméthy-1-piperidin-1-yl)-propionamide ;
- le trifluoroacétate de N-{4-[5,5-diméthyl-2,4-dioxo-3-(4-trifluorométhylsulfanyl-phényl)-imidazolidin-1-ylmét-hyl]-pyridin-2-yl}-3-méthoxy-benzamide ;
- le trifluoroacétate de la {4-[5,5-diméthyl-2,4-dioxo-3-(4-trifluorométhylsulfanyl-phényl)-imidazolidin-1-ylmé-thyl]-pyridin-2-yl}-amide de l'acide pyrazine-2-carboxy-lique ;
- le trifluoroacétate de la {4-[5,5-diméthyl-2,4-dioxo-3-(4-trifluorométhylsulfanyl-phényl)-imidazolidin-1-ylmé-thyl]-pyridin-2-yl}-amide de l'acide thiophène-2-carboxy-lique ;
- le N-{4-[5,5-diméthyl-2,4-dioxo-3-(4-trifluorométhyl-sulfanyl-phényl)-imidazolidin-1-ylméthyl]-pyridin-2-yl}-4-méthyl-benzamide ; composé avec de l'acide trifluoroacétique ;
- le 1-isoquinolin-5-yl-5,5-diméthyl-3-(4-trifluorométhylsulfanyl-phényl)-imidazolidine-2,4-dione ;
- le 3-(4-acétyl-piperazin-1-yl)-N-{4-[5,5-diméthyl-2,4-dioxo-3-(4-trifluorométhylsulfanyl- phényl)-imidazolidin-1-ylméthyl]-pyridin-2-yl}-propionamide ;
- le 3-[4-(2-diéthylamino-éthyl)-piperazin-1-yl]-N-{4-[5,5-diméthyl-2,4-dioxo-3-(4-trifluorométhylsulfanyl-phényl)-imidazolidin-1-ylméthyl]-pyridin-2-yl}-propionamide ;
- le N-{4-[5,5-diméthyl-2,4-dioxo-3-(4-trifluorométhyl-sulfanyl-phényl)-imidazolidin-1-ylméthyl]-pyridin-2-yl}-3-(2,6-diméthyl-morpholin-4-yl)-propionamide ;
- le N-{4-[5,5-diméthyl-2,4-dioxo-3-(4-trifluorométhyl-sulfanyl-phériyl)-imidazolidin-1-ylméthyl]-pyridin-2-yl}-3-(4-pyrrolidin-1-yl-piperidin-1-yl)-propionamide ;
- le N-{4-[5,5-diméthyl-2,4-dioxo-3-(4-trifluorométhyl-sulfanyl-phényl)-imidazolidin-1-ylméthyl]-pyridin-2-yl}-2-(4-pyrrolidin-1-yl-piperidin-1-yl)-acétamide ;
- le 5,5-diméthyl-1-[2-(4-méthyl-pyridin-3-ylamino)-pyri-din-4-ylméthyl]-3-(4-trifluorométhylsulfanyl-phényl)-imidazolidine-2,4-dione ;
- le 5,5-diméthyl-1-[2-(6-morpholin-4-yl-pyridin-3-yl-amino)-pyridin-4-ylméthyl]-3-(4-trifluorométhylsulfanyl-phényl)-imidazolidine-2,4-dione ;
- le 1-[2-(2,6-diméthyl-pyridin-3-ylamino)-pyridin-4-yl-méthyl]-5,5-diméthyl-3-(4-trifluorométhylsulfanyl-phényl)-imidazolidine-2,4-dione ;
- l'ester méthylique de l'acide 5-{4-[5,5-diméthyl-2,4-dioxo-3-(4-trifluorométhyl-sulfanyl-phényl)-imidazolidin-1-ylméthyl]-pyridin-2-ylamino}-pyridine-2-carboxylique ;
- le 1-[2-(2,6-diméthoxy-pyridin-3-ylamino)-pyridin-4-ylméthyl]-5,5-diméthyl-3-(4-trifluorométhylsulfanyl-phényl)-imidazolidine-2,4-dione ;
- le 1-[2-(6-fluoro-pyridin-3-ylamino)-pyridin-4-ylmé-thyl]-5,5-diméthyl-3-(4-trifluorométhylsulfanyl-phényl)-imidazolidine-2,4-dione ;
- le 1-[2-(6-méthoxy-pyridin-3-ylamino)-pyridin-4-ylmé-thyl]-5,5-diméthyl-3-(4-trifluorométhylsulfanyl-phényl)-imidazolidine-2,4-dione,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention a également pour objet un procédé de préparation des produits de formule générale (I) telle que définie ci-dessus, **caractérisé en ce que :** soit l'on fait agir en présence d'une base tertiaire un produit de formule (II) : 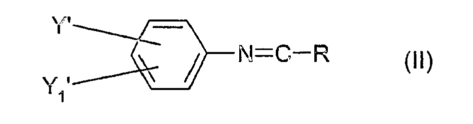 dans laquelle Y' et Y1' ont les significations indiquées ci-dessus respectivement pour Y et Y1 dans lesquels les éventuelles fonctions réactives sont éventuellement protégées et R a la signification indiquée ci-dessus, avec un produit de formule (III) : 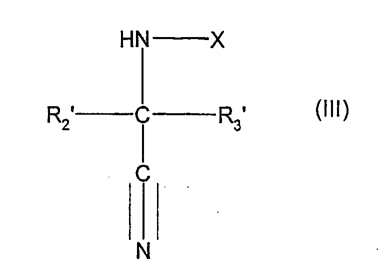 dans laquelle X représente -A2'-B2'-Y2' ou bien hydrogène, et A2', B2', Y2', R2' et R3' ont les significations indiquées ci-dessus, respectivement pour A2', B2', Y2', R2' et R3' dans lesquels les éventuelles fonctions réactives sont éventuellement protégées ci-dessus, pour obtenir un produit de formule (IV) : 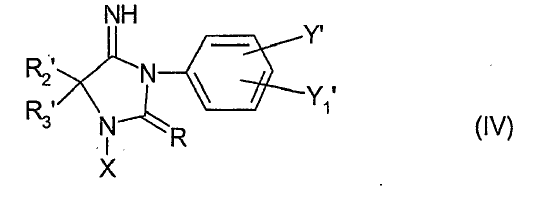 dans laquelle Y', Y1', X, R, R2' et R3' ont les significations indiquées ci-dessus,
produits de formule (IV) que, si nécessaire ou si désiré l'on soumet à l'une quelconque ou plusieurs des réactions suivantes, dans un ordre quelconque :
a) réaction d'élimination des éventuels groupements protecteurs que peuvent porter Y', Y1', R, R2' et R3' et X lorsque X représente -A2'-B2'-Y2';
b) réaction d'hydrolyse du groupement >C=NH en fonction cétone
c) action sur les produits de formule (IV) dans laquelle X représente un atome d'hydrogène, et après éventuelle hydrolyse au groupement >C=NH en fonction cétone d'un réactif de formule Hal-A2'-B2'-Y2' dans laquelle A2', B2' et Y2' ont les significations indiquées précédemment et Hal représente un atome d'halogène pour obtenir des produits de formule (Ii) : 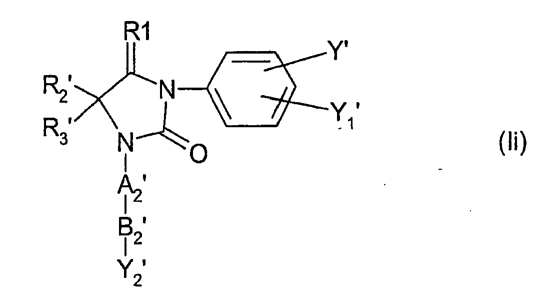
dans laquelle Y', Y1', R2', R3', A2',B2' et Y2' ont les significations indiquées précédemment puis, si désiré, action sur ces produits, d'un agent d'élimination des éventuels groupements protecteurs que peuvent porter Y', Y1', R2', R3', A2',B2' et Y2' ou le cas échéant, action d'un agent d'estérification, d'amidification ou de salification,
soit l'on fait agir en présence d'une base tertiaire le produit de formule (II) défini ci-dessus, avec un produit de formule (III') : 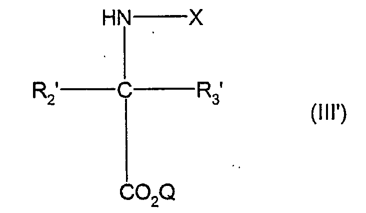 dans laquelle R2' et R3' ont les significations indiquées précédemment et Q représente soit un atome de métal alcalin par exemple le sodium ou un radical alkyle renfermant de 1 à 6 atomes de carbone, pour obtenir un produit de formule (IVa): 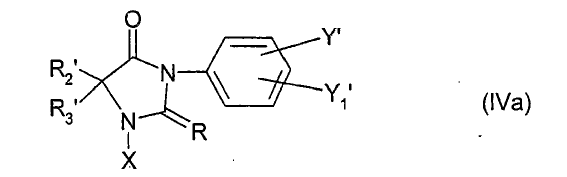 dans laquelle Y', Y1', R, R2', R3' et X, ont les significations indiquées précédemment que si désiré l'on soumet à l'une quelconque ou plusieurs des réactions suivantes, dans un ordre quelconque :
a) réaction d'élimination des éventuels groupements protecteurs que peut porter X ;
b) action sur les produits de formule (IVa) dans laquelle X représente un atome d'hydrogène, d'un réactif de formule Hal-A2'-B2'-Y2' dans laquelle A2', B2' et Y2' ont les significations indiquées précédemment et Hal représente un atome d'halogène pour obtenir des produits de formule (Iii) : 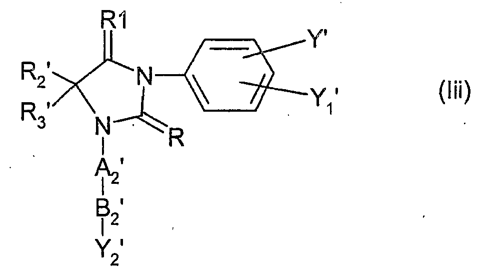
dans laquelle Y', Y1', R, R1, R2', R3' et A2', B2' et Y2' ont les significations indiquées précédemment, puis, si désiré, action sur ces produits, d'un agent d'élimination des éventuels groupements protecteurs que peut porter Y', Y1', R2', R3', A2', B2' et Y2' ou le cas échéant, action d'un agent d'estérification, d'amidification ou de salification,
soit l'on fait agir un réactif de formule Hal-A2'-B2'-Y2' dans laquelle A2', B2', Y2' et Hal ont les significations indiquées précédemment sur un produit de formule (IV') : 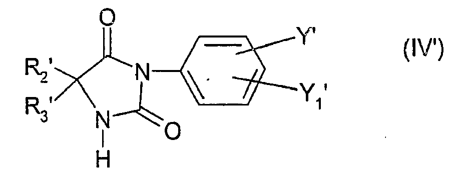 dans laquelle Y', Y1', R2' et R3' ont les significations indiquées précédemment,
pour obtenir un produit de formule (IV") : 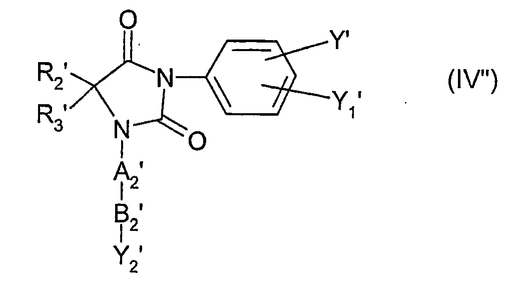 dans laquelle Y', Y1', R2', R3', A2', B2' et Y2' ont les significations indiquées précédemment,
produit de formule (IV") que, si nécessaire ou si désiré, l'on soumet à une réaction d'élimination des éventuels groupements protecteurs que peut porter -A2'-B2'-Y2' puis le cas échéant action d'un agent d'estérification, d'amidification ou de salification.

On peut noter que selon les valeurs de Y', Y1', R2', R3', A2', B2' et Y2', les produits de formules (IV), (Ii), (IVa), (Iii) et (IV") peuvent être des produits de formule (I) et que, pour obtenir des ou d'autres produits de formule (I), l'on peut soumettre ces produits, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction de réduction des composés nitrés en composés aminés,
j) une réaction d'élimination des groupements protecteurs que peuvent porter les fonctions réactives protégées,
k) une réaction de salification par un acide minéral ou organique ou par une base pour obtenir le sel correspondant,
l) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthyl-silyle, tert-butyldiméthylsilyle, méthoxyméthyle, tétrahydro-pyrannyle, benzyle ou acétyle,
- les groupements amino peuvent être protégés par exemple par les radicaux acétyle, trityle, benzyle, tert-butoxycarbonyle, benzyloxycarbonyle, phtalimido ou d'autres radicaux connus dans la chimie des peptides,

- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acides des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante,
- les fonctions acides peuvent être protégées par exemple sous forme d'esters formés avec les esters facilement clivables tels que les esters benzyliques ou terbutyliques ou des esters connus dans la chimie des peptides.

Ces réactions a) à k) indiquées ci-dessus peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus, dans lesquels le radical alkyle est éventuellement substitué par un ou plusieurs atomes d'halogène, notamment de fluor, peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide.
   L'obtention de la fonction sulfone peut être favorisée par un mélange du produit renfermant un groupement alkylthio avec un excès du réactif tel que notamment un peracide.
d) La réaction de transformation d'une fonction cétone en oxime peut être réalisée dans les conditions usuelles connues de l'homme de métier, telle que notamment une action en présence d'une hydroxylamine éventuellement O-substituée dans un alcool tel que par exemple l'éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhy-drique dans de l'eau ou de l'acide trifluoroacétique au reflux.
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit : J. Organometallic Chemistry., 33, 337 (1971) KOZIMA S.& coll.
   On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.
   Il est entendu que les réactions décrites ci-dessus peuvent être effectuées comme indiqué ou encore, le cas échéant, selon d'autres méthodes usuelles connues de l'homme du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protec-teurs utilisables par exemple dans le brevet BF 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier.
k) Les éventuelles formes optiquement actives des produits décrits ci-dessus peuvent être préparées par dédoublement des racémiques selon les méthodes usuelles connues de l'homme du métier.

L'action des produits de formule (II) avec les produits de formule (III) est effectuée de préférence dans un solvant organique tel que le tétrahydrofuranne ou le dichloroéthane mais on peut également utiliser l'éther éthylique ou l'éther isopropylique.

On opère éventuellement en présence d'une base tertiaire telle que la triéthylamine ou encore la pyridine ou la méthyléthylpyridine.

Les éventuelles fonctions réactives qui sont éventuellement protégées dans le produit de formule (III), (IVa) ou (IV") sont les fonctions hydroxy ou amino. On utilise pour protéger ces fonctions des groupements protecteurs usuels. On peut citer par exemples les groupements protecteurs suivants du radical amino : tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthylsilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français 2.499.995,dont le contenu est incorporé ici par référence.

Les réactions éventuelles d'élimination des groupements protecteurs sont effectuées comme indiqué dans ledit brevet 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.

La réaction éventuelle d'hydrolyse du groupement >C=NH en groupement cétone est également effectuée de préférence à l'aide d'un acide tel que l'acide chlorhydrique aqueux par exemple au reflux.

L'action sur les produits de formules (IV), (IVa) ou (IV') du réactif de formule Hal-A2'-B2'-Y2' est effectuée en présence d'une base forte telle que l'hydrure de sodium ou de potassium. On peut opérer par réaction de transfert de phase en présence de sels d'ammonium quaternaires tels que le tert-butyl ammonium.

Un exemple d'élimination du groupement terbutyldiméthylsilyle au moyen de l'acide chlorhydrique est donné ci-après dans les exemples.
- L'estérification éventuelle d'un radical OH libre est effectuée dans des conditions classiques. On peut utiliser par exemple un acide ou un dérivé fonctionnel, par exemple un anhydride tel que l'anhydride acétique en présence d'une base telle que la pyridine.

L'estérification ou la salification éventuelle d'un groupement COOH est effectuée dans les conditions classiques connues de l'homme du métier.
- L'amidification éventuelle d'un radical COOH est effectuée dans des conditions classiques. On peut utiliser une amine primaire ou secondaire sur un dérivé fonctionnel de l'acide par exemple un anhydride symétrique ou mixte.

La présente invention a également pour objet un procédé de préparation des produits de formule (I") : 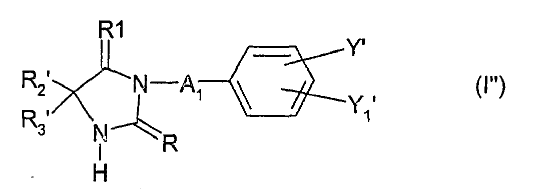 dans laquelle dans laquelle Y', Y1', A1, R, R1, R2' et R3' ont les significations indiquées ci-dessus, **caractérisé en ce que** l'on fait réagir un produit de formule (V) : 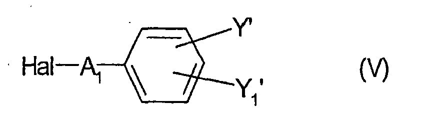 dans laquelle A1, Y1' et Y2' ont les significations précédentes et Hal représente un atome d'halogène avec un produit de formule (VI) : 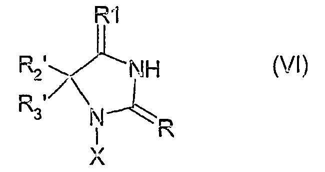 dans laquelle R, R1, R2', R3' et X ont les significations précédentes, la réaction s'effectuant en présence d'un catalyseur et éventuellement d'un solvant.

En ce qui concerne les produits de formule (V), le terme Hal désigne de préférence l'atome de chlore, mais peut aussi représenter un atome de brome ou d'iode.

L'invention a plus précisément pour objet un procédé tel que défini ci-dessus dans lequel le catalyseur est un métal sous forme native ou oxydée ou une base.

Le catalyseur utilisé peut être un métal sous forme native, sous forme d'oxyde métallique ou encore sous forme de sels métalliques. Le catalyseur peut également être une base. Quand le catalyseur utilisé est un métal, ce métal peut être du cuivre ou du nickel.

Les sels métalliques peuvent être un chlorure ou un acétate.

On peut noter que lorsque A1 représente une simple liaison, on peut utiliser un catalyseur. Lorsque A1 représente alkyle, il s'agit alors d'une alkylation que l'on peut réaliser notamment en présence d'un réactif tel qu'une base.

Quand le catalyseur est une base, cette base peut être par exemple la soude ou la potasse et on peut, si désiré, ajouter au milieu réactionnel du diméthylsulfoxyde.

L'invention a plus précisément pour objet un procédé tel que défini ci-dessus dans lequel le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

L'invention a particulièrement pour objet un procédé tel que défini ci-dessus dans lequel le catalyseur est l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

On peut noter que, notamment lorsque A1 représente une simple liaison, dans la réaction d'un produit de formule (V) avec un produit de formule (VI) tels que définis ci-dessus, on peut également utiliser comme catalyseur le palladium ou un de ses sels comme décrit par exemple dans les articles suivants ou un sel de cuivre avec un ligand comme un dérivé de 1,2-diamino-cyclohexane : Buchwald S.L., J. AM. CHEM. SOC., 2002, 6043 et Buchwald S.L., J. AM. CHEM. SOC., 2001, 7727.

L'invention a plus particulièrement pour objet un procédé tel que défini ci-dessus **caractérisé en ce que** l'on opère en présence d'un solvant de type éther tel que l'oxyde de phényle, le diglyme, le triglyme, le diméthylsulfoxyde, le toluène ou le dioxane.

L'invention a tout particulièrement pour objet un procédé tel que défini ci-dessus dans lequel le solvant utilisé est l'oxyde de phényle ou le triglyme.

Le procédé de préparation du produit recherché, défini ci-dessus peut être réalisé sous pression ou à la pression atmosphérique, à une température préférentiellement élevée.

L'invention a ainsi pour objet un procédé tel que défini ci-dessus **caractérisé en ce que** la réaction est réalisée à une température supérieure à 100°C et de préférence supérieure à 150°C.

L'invention a plus précisément pour objet un procédé tel que défini ci-dessus **caractérisé en ce que** la réaction est réalisée pendant plus de 2 heures.

L'invention a très précisément pour objet un procédé tel que défini ci-dessus **caractérisé en ce que** la réaction est réalisée en présence d'oxyde cuivreux, dans le triglyme, à une température supérieure ou égale à 200°C et pendant plus de 3 heures.

Les produits objet de la présente invention sont doués de propriétés pharmacologiques intéressantes: on a constaté qu'ils possédaient notamment des propriétés inhibitrices de protéines kinases.

Parmi ces protéines kinases, on cite notamment IGF1R.

On cite également FAK. On cite également AKT.

Des tests donnés dans la partie expérimentale ci-après illustrent l'activité inhibitrice de produits de la présente invention vis-à-vis de telles protéines kinases.

Ces propriétés rendent donc les produits de formule générale (I) de la présente invention utilisables comme médicaments pour le traitement de tumeurs malignes.

Les produits de formule (I) peuvent également être utilisés dans le domaine vétérinaire.

L'invention a donc pour objet l'application, à titre de médicaments, des produits de formule générale (I) pharmaceutiquement acceptables.

L'invention a particulièrement pour objet l'application à titre de médicaments, des produits dont les noms suivent :
- le trifluoroacétate de (S)-5-méthyl-1-quinolin-4-ylméthyl-3-(4-trifluorométhanesulfonyl-phényl)-imidazolidine-2,4-dione,
- le trifluoroacétate de (S)-5-méthyl-1-pyridin-4-ylméthyl-3-(4-trifluorométhanesulfonyl-phényl)-imidazolidine-2,4-dione,
- le trifluoroacétate de (S)-5-méthyl-1-quinolin-4-ylméthyl-3-(4-trifluorométhylsulfanyl-phényl)-imidazolidine-2,4-dione,
- le trifluoroacétate de 5,5-diméthyl-1-quinolin-4-ylméthyl-3-(4-trifluorométhanesulfonyl-phényl)-imidazolidine-2,4-dione,
- le trifluoroacétate de (R)-5-méthyl-1-quinolin-4-ylméthyl-3-(4-trifluorométhanesulfonyl-phényl)-imidazolidine-2,4-dione,
- le trifluoroacétate de (R)-5-méthyl-1-quinolin-4-ylméthyl-3-(4-trifluorométhylsulfanyl-phényl)-imidazolidine-2,4-dione,
- le trifluoroacétate de (R)-5-méthyl-1-pyridin-4-ylméthyl-3-(4-trifluorométhanesulfonyl-phényl)-imidazolidine-2,4-dione,
- le trifluoroacétate de (R)-5-méthyl-1-(3-méthyl-pyridin-4-ylméthyl)-3-(4-trifluorométhanesulfonyl-phényl)-imidazolidine-2,4-dione,
- le trifluoroacétate de (R)-4-méthyl-3-quinolin-4-ylméthyl-5-thioxo-1-(4-trifluorométhylsulfanyl-phényl)-imidazolidin-2-one
- le trifluoroacétate de (R)-5-isopropyl-1-quinolin-4-ylméthyl-3-(4-trifluorométhylsulfanyl-phényl)-imidazolidine-2,4-dione
- le trifluoroacétate de (R)-5-isopropyl-1-quinolin-4-ylméthyl-3-(4-trifluorométhanesulfonyl-phényl)-imidazolidine-2,4-dione
- le trifluoroacétate de (R)-5-(4-hydroxy-benzyl)-1-quinolin-4-ylméthyl-3-(4-trifluorométhanesulfonyl-phényl)-imidazolidine-2,4-dione
- le trifluoroacétate de (R)-5-(4-hydroxy-benzyl)-1-pyridin-4-ylméthyl-3-(4-trifluorométhanesulfonyl-phényl)-imidazolidine-2,4-dione
- le trifluoroacétate de (R)-5-(1-hydroxy-éthyl)-1-quinolin-4-ylméthyl-3-(4-trifluorométhanesulfonyl-phényl)-imidazolidine-2,4-dione
- le trifluoroacétate de, 4-quinolin-4-ylméthyl-6-(4-trifluorométhylsulfanyl-phényl)-4,6-diaza-spiro[2.4]heptane-5,7-dione
- le trifluoroacétate de 4-quinolin-4-ylméthyl-6-(4-trifluorométhanesulfonyl-phênyl)-4,6-diaza-spiro[2.4]heptane-5,7-dione, trifluoroacétate
- le trifluoroacétate de 4-pyridin-4-ylméthyl-6-(4-trifluorométhylsulfanyl-phényl)-4,6-diaza-spiro[2.4]heptane-5,7-dione,
- le trifluoroacétate de 4-pyridin-4-ylméthyl-6-(4-trifluorométhanesulfonyl-phényl)-4,6-diaza-spiro[2.4]heptane-5,7-dione
- le trifluoroacétate de (R)-1-(3-hydroxy-pyridin-4-ylméthyl)-5-méthyl-3-(4-trifluorométhylsulfanyl-phényl)-imidazolidine-2,4-dione
- le trifluoroacétate de 5,5-diméthyl-1-quinolin-4-ylmêthyl-3-(4-trifluorométhoxy-phényl)-imidazolidine-2,4-dione
- le trifluoroacétate de 5,5-diméthyl-1-quinolin-4-ylméthyl-3-(4-trifluorométhylsulfanyl-phényl)-imidazolidine-2,4-dione
- le trifluoroacétate de 5,5-diméthyl-1-(3-méthyl-pyridin-4-ylméthyl)-3-(4-trifluorométhoxy-phényl)-imidazolidine-2,4-dione
- le trifluoroacétate de 5,5-diméthyl-1-(3-méthyl-pyridin-4-ylméthyl)-3-(4-trifluorométhylsulfanyl-phényl)-imidazolidine-2,4-dione
- le trifluoroacétate de 5,5-diméthyl-1-(3-méthyl-pyridin-4-ylméthyl)-3-(4-trifluorométhanesulfonyl-phényl)-imidazolidine-2,4-dione
- le trifluoroacétate de 1-(3-hydroxy-pyridin-4-ylméthyl)-5,5-diméthyl-3-(4-trifluorométhoxy-phényl)-imidazolidine-2,4-dione
- le trifluoroacétate de 1-(3-hydroxy-pyridin-4-ylméthyl)-5,5-diméthyl-3-(4-trifluorométhylsulfanyl-phényl)-imidazolidine-2,4-dione
- le trifluoroacétate de 1-(3-hydroxy-pyridin-4-ylméthyl)-5,5-diméthyl-3-(4-trifluorométhanesulfonyl-phényl)-imidazolidine-2,4-dione
- le trifluoroacétate de 4-quinolin-4-ylméthyl-6-(4-trifluorométhoxy-phényl)-4,6-diaza-spiro[2.4]heptane-5,7-dione
- le trifluoroacétate de 4-(3-méthyl-pyridin-4-ylméthyl)-6-(4-trifluorométhylsulfanylphényl)-4,6-diaza-spiro[2.4]heptane-5,7-dione
- le trifluoroacétate de 4-(3-hydroxy-pyridin-4-ylméthyl)-6-(4-trifluorométhoxy-phényl)-4,6-diaza-spiro[2.4]heptane-5,7-dione
- le trifluoroacétate de 4-(3-Hydroxy-pyridin-4-ylméthyl)-6-(4-trifluorométhylsulfanyl-phényl)-4,6-diaza-spiro[2.4] heptane-5,7-dione
- le trifluoroacétate de 4-(3-hydroxy-pyridin-4-ylméthyl)-6-(4-trifluorométhanesulfonyl-phényl)-4,6-diaza-spiro[2.4]heptane-5,7-dione.
lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

L'invention a particulièrement pour objet l'application à titre de médicaments, des produits dont les noms suivent:
- le Trifluoroacétate de la {4-[5,5-diméthyl-2,4-dioxo-3-(4-trifluorométhylsulfanyl- phényl)-imidazolidin-1-ylméthyl]-pyridin-2-yl}-amide de l'acide cyclopropanecarboxylique ;
- le 5,5-diméthyl-1-[2-(pyridin-2-ylamino)-pyridin-4-yl-méthyl]-3-(4-trifluorométhylsulfanyl-phényl)-imidazolidine-2,4-dione ; composé avec de l'acide trifluoroacétique ;
- le N-{4-[5,5-diméthyl-2,4-dioxo-3-(4-trifluorométhylsulfanyl-phényl)-imidazolidin-1-ylméthyl]-pyridin-2-yl}-isobutyramide ; composé avec de l'acide trifluoroacétique ;
- le N-{4-[5,5-diméthyl-2,4-dioxo-3-(4-trifluorométhylsulfanyl-phényl)-imidazolidin-1-ylméthyl]-pyridin-2-yl}-propionamide ; composé avec de l'acide trifluoroacétique ;
- le chlorhydrate de 1-(2-amino-pyridin-4-ylméthyl)-5,5-diméthyl-3-(4-trifluorométhylsulfanyl-phényl)-imidazolidine-2,4-dione ;
- le trifluoroacétate de la {4-[5,5-diméthyl-2,4-dioxo-3-(4-trifluorométhylsulfanyl-phényl)-imidazolidin-1-ylmé-thyl]-pyridin-2-yl}-amide de l'acide pyridine-2-carboxy-lique ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-piperidin-1-yl-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-[4-(2-hydroxy-éthyl)-piperazin-1-yl]-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-morpholin-4-yl-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-pyrrolidin-1-yl-propionamide ;
- le trifluoroacétate de N-{4-[5,5-diméthyl-2,4-dioxo-3-(4-trifluorométhylsulfanyl-phényl)-imidazolidin-1-ylmé-thyl]-pyridin-2-yl}-3-(4-méthyl-piperazin-1-yl)-propionamide ;
- le 1-{4-[5,5-diméthyl-2,4-dioxo-3-(4-trifluorométhylsulfanyl-phényl)-imidazolidin-1-ylméthyl]-pyridin-2-yl}-3-phényl-urée ;
- le 1-[2-(6-éthyl-pyridin-2-ylamino)-pyridin-4-ylmé-thyl]-5,5-diméthyl-3-(4-trifluorométhylsulfanyl-phényl)-imidazolidine-2,4-dione ;
- le 5,5-diméthyl-1-[2-(4-méthyl-pyridin-2-ylamino)-pyri-din-4-ylméthyl]-3-(4-trifluorométhylsulfanyl-phényl)-imidazolidine-2,4-dione ;
- le 5,5-diméthyl-1-[2-(6-méthyl-pyridin-2-ylamino)-pyri-din-4-ylméthyl]-3-(4-trifluorométhylsulfanyl-phényl)-imi-dazolidine-2,4-dione ;
- le 1-[2-(4,6-diméthyl-pyridin-2-ylamino)-pyridin-4-yl-méthyl]-5,5-diméthyl-3-(4-trifluorométhylsulfanyl-phényl)-imidazolidine-2,4-dione ;
- le 1-[2-(3,5-dichloro-pyridin-2-ylamino)-pyridin-4-yl-méthyl] -5,5-diméthyl-3-(4-trifluorométhylsulfanyl-phényl)-imidazolidine-2,4-dione ;
- le 5,5-diméthyl-1-[2-(pyridin-4-ylamino)-pyridin-4-yl-méthyl]-3-(4-trifluorométhylsulfanyl-phényl)-imidazoli-dine-2,4-dione ;
- le 5,5-diméthyl-1-[2-(pyridin-3-ylamino)-pyridin-4-yl-méthyl]-3-(4-trifluorométhylsulfanyl-phényl)-imidazoli-dine-2,4-dione ;
- le N-{4-[5,5-diméthyl-2,4-dioxo-3-(4-trifluorométhylsulfanyl-phényl)-imidazolidin-1-ylméthyl]-pyridin-2-yl}-3-(2-oxo-azépan-1-yl)-propionamide ;
- le 3-(benzyl-méthyl-amino)-N-{4-[5,5-diméthyl-2,4-di-oxo-3-(4-trifluorométhylsulfanyl-phényl)-imidazolidin-1-ylméthyl]-pyridin-2-yl}-propionamide ;
- l' acide 4,5-diacétoxy-6-acétoxyméthyl-2-(3-{4-[5,5-diméthyl-2,4-dioxo-3-(4-trifluorométhylsulfanyl-phényl)-imidazolidin-1-ylméthyl]-pyridin-2-yl}-thiouréido acétique ;
- le 5,5-diméthyl-1-[2-(5-méthyl-pyridin-2-ylamino)-pyridin-4-ylméthyl]-3-(4-trifluorométhylsulfanyl-phényl)-imidazolidine-2,4-dione ;
- le trifluoroacétate de N-{4-[5,5-diméthyl-2,4-dioxo-3-(4-trifluorométhyl-sulfanyl-phényl)-imidazolidin-1-ylméthyl]-pyridin-2-yl}-3,5-diméthoxy-benzamide ;
- le trifluoroacétate de 5,5-diméthyl-1-[2-(pyrazin-2-yl-amino)-pyridin-4-ylméthyl]-3-(4-trifluorométhylsulfanyl-phényl)-imidazolidine-2,4-dione ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-(3-méthyl-piperidin-1-yl)-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-(3,5-diméthyl-piperidin-1-yl)-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-méthoxy-benzamide ;
- le trifluoroacétate de la {4-[5,5-diméthyl-2,4-dioxo-3-(4-trifluorométhylsulfanyl-phényl)-imidazolidin-1-ylméthyl]-pyridin-2-yl}-amide de l'acide pyrazine-2-carboxylique ;
- le trifluoroacétate de la {4-[5,5-diméthyl-2,4-dioxo-3-(4-trifluorométhylsulfanyl-phényl)-imidazolidin-1-ylméthyl]-pyridin-2-yl}-amide de l'acide thiophène-2-carboxylique ;
- le N-{4-[5,5-diméthyl-2,4-dioxo-3-(4-trifluorométhylsulfanyl-phényl)-imidazolidin-1-ylméthyl]-pyridin-2-yl}-4-méthyl-benzamide ; composé avec de l'acide trifluoroacétique ;
- le 1-isoquinolin-5-yl-5,5-diméthyl-3-(4-trifluorométhylsulfanyl-phényl)-imidazolidine-2,4-dione ;
- le 3-(4-acétyl-piperazin-1-yl)-N-{4-[5,5-diméthyl-2,4-dioxo-3-(4-trifluorométhylsulfanyl- phényl)-imidazolidin-1-ylméthyl]-pyridin-2-yl}-propionamide ;
- le 3-[4-(2-diéthylamino-éthyl)-pigerazin-1-yl]-N-{4-[5,5-diméthyl-2,4-dioxo-3-(4-trifluorométhylsulfanyl-phényl)-imidazolidin-1-ylméthyl]-pyridin-2-yl}-propionamide ;
- le N-{4-[5,5-diméthyl-2,4-dioxo-3-(4-trifluorométhylsulfanyl-phényl)-imidazolidin-1-ylméthyl]-pyridin-2-yl}-3-(2,6-diméthyl-morpholin-4-yl)-propionamide ;
- le N-{4-[5,5-diméthyl-2,4-dioxo-3-(4-trifluorométhylsulfanyl-phényl)-imidazolidin-1-ylméthyl]-pyridin-2-yl}-3-(4-pyrrolidin-1-yl-piperidin-1-yl)-propionamide ;
- le N-{4-[5,5-diméthyl-2,4-dioxo-3-(4-trifluorométhylsulfanyl-phényl)-imidazolidin-1-ylméthyl]-pyridin-2-yl}-2-(4-pyrrolidin-1-yl-piperidin-1-yl)-acétamide ;
- le 5,5-diméthyl-1-[2-(4-méthyl-pyridin-3-ylamino)-pyridin-4-ylméthyl]-3-(4-trifluorométhylsulfanyl-phényl)-imidazolidine-2,4-dione ;
- le 5,5-diméthyl-1-[2-(6-morpholin-4-yl-pyridin-3-yl-amino)-pyridin-4-ylméthyl]-3-(4-trifluorométhylsulfanyl-phényl)-imidazolidine-2,4-dione ;
- le 1-[2-(2,6-diméthyl-pyridin-3-ylamino)-pyridin-4-yl-méthyl]-5,5-diméthyl-3-(4-trifluorométhylsulfanyl-phényl)-imidazolidine-2,4-dione ;
- l'ester méthylique de l'acide 5-{4-[5,5-diméthyl-2,4-dioxo-3-(4-trifluorométhyl-sulfanyl-phényl)-imidazolidin-1-ylméthyl]-pyridin-2-ylamino}-pyridine-2-carboxylique ;
- le 1-[2-(2,6-diméthoxy-pyridin-3-ylamino)-pyridin-4-ylméthyl]-5,5-diméthyl-3-(4-trifluorométhylsulfanyl-phényl)-imidazolidine-2,4-dione ;
- le 1-[2-(6-fluoro-pyridin-3-ylamino)-pyridin-4-ylméthyl]-5,5-diméthyl-3-(4-trifluorométhylsulfanyl-phényl)-imidazolidine-2,4-dione ;
- le 1-[2-(6-méthoxy-pyridin-3-ylamino)-pyridin-4-ylméthyl]-5,5-diméthyl-3-(4-trifluorométhylsulfanyl-phényl)-imidazolidine-2,4-dione,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

Les produits peuvent être administrés par voie parentérale, buccale, perlinguale, rectale ou topique.

L'invention a aussi pour objet les compositions pharmaceutiques, **caractérisées en ce qu**'elles renferment, à titre de principe actif, un au moins des médicaments de formule générale (I).

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

La présente invention concerne ainsi l'utilisation de produits de formule (I) tels que définis ci -dessus ou de sels pharmaceutiquement acceptables desdits produits de formule (I) pour la préparation de médicaments destinés à inhiber l'activité de protéines kinases et notamment d'une protéine kinase.

La présente invention concerne ainsi 1' utilisation de produits de formule (I) telle que définie ci-dessus ou de sels pharmaceutiquement acceptables desdits produits de formule (I) dans laquelle la protéine kinase est une protéine tyrosine kinase.

La présente invention concerne ainsi l'utilisation de produits de formule (I) telle que définie ci-dessus ou de sels pharmaceutiquement acceptables desdits produits de formule (I) dans laquelle la protéine kinase est choisie dans le groupe suivant: EGFR, Fak, FLK-1, FGFR1, FGFR2, FGFR3, FGFR4, FGFR5, flt-1, IGF-1R, KDR, PDGFR, tie2, VEGFR, AKT, Raf.

La présente invention concerne ainsi particulièrement l'utilisation de produits de formule (I) telle que définie ci-dessus ou de sels pharmaceutiquement acceptables desdits produits de formule (I) dans laquelle la protéine kinase est IGF1R.

La présente invention concerne également l'utilisation de produits de formule (I) telle que définie ci-dessus ou de sels pharmaceutiquement acceptables desdits produits de formule (I) dans laquelle la protéine kinase est FAK.

La présente invention concerne également l'utilisation de produits de formule (I) telle que définie ci-dessus ou de sels pharmaceutiquement acceptables desdits produits de formule (I) dans laquelle la protéine kinase est AKT.

La présente invention concerne également l'utilisation de produits de formule (I) telle que définie ci-dessus ou de sels pharmaceutiquement acceptables desdits produits de formule (I) dans laquelle la protéine kinase est dans une culture cellulaire et également cette utilisation dans un mammifère.

La présente invention concerne ainsi l'utilisation de produits de formule (I) telle que définie ci-dessus ou de sels pharmaceutiquement acceptables desdits produits de formule (I) pour la préparation d'un médicament destiné à prévenir ou traiter une maladie caractérisée par le dérèglement de l'activité d'une protéine kinase et notamment une telle maladie chez un mammifère.

La présente invention concerne l'utilisation de produits de formule (I) telle que définie ci-dessus ou de sels pharmaceutiquement acceptables desdits produits de formule (I) pour la préparation d'un médicament destiné à prévenir ou traiter une maladie appartenant au groupe suivant: désordres de la prolifération de vaisseaux sanguins, désordres fibrotiques, désordres de la prolifération de cellules mésangiales, désordres métaboliques, allergies, asthme, thromboses, maladies du système nerveux, rétinopathies, psoriasis, arthrite rhumatoïde, diabètes, dégénération musculaire, maladies en oncologie, cancers.

La présente invention concerne ainsi l'utilisation de produits de formule (I) telle que définie ci-dessus ou de sels pharmaceutiquement acceptables desdits produits de formule (I) pour la préparation d'un médicament destiné à traiter des maladies en oncologie.

La présente invention concerne particulièrement l'utilisation de produits de formule (I) telle que définie ci-dessus ou de sels pharmaceutiquement acceptables desdits produits de formule (I) pour la préparation d'un médicament destiné à traiter des cancers.

Parmi ces cancers, la présente invention s'intéresse tout particulièrement au traitement des tumeurs solides et au traitement de cancers résistants aux agents cytotoxiques

Parmi ces cancers, la présente invention concerne tout particulièrement le traitement du cancer du sein, de l'estomac, du colon, des poumons, des ovaires, de l'utérus, du cerveau, du rein, du larynx, du système lymphatique, de la thyroïde, du tractus uro-génital, du tractus incluant vésicule et prostate, du cancer des os, du pancréas, les mélanomes.

La présente invention s'intéresse encore plus particulièrement au traitement du cancer du sein, du colon et des poumons.

La présente invention concerne aussi l'utilisation de produits de formule (I) telle que définie ci-dessus ou de sels pharmaceutiquement acceptables desdits produits de formule (I) pour la préparation d'un médicament destiné à la chimiothérapie de cancers.

A titre de médicaments selon la présente invention destinés à la chimiothérapie de cancers, les produits de formule (I) selon la présente invention peuvent être utilisés seuls ou en association avec chimiothérapie ou radiothérapie ou alternativement en association avec d'autres agents thérapeutiques.

La présente invention concerne ainsi notamment les compositions pharmaceutiques telles que définies ci-dessus contenant en plus, des principes actifs d'autres médicaments de chimiothérapie contre le cancer.

De tels agents thérapeutiques peuvent être des agents anti-tumoraux utilisés communément.

Comme exemples d'inhibiteurs connus de protéines kinases, on peut citer notamment la butyrolactone, le flavopiridol, la 2-(2-hydroxyéthylamino)-6-benzylamino-9-méthylpurine, l'olomucine, le Glivec ainsi que l'Iressa.

Les produits de formule (I) selon la présente invention peuvent ainsi également être avantageusement utilisées en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisomérase I, les inhibiteurs de topoisomérase II, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR, les antimétabolites antinéoplastique, les composés du platine, les composés faisant décroître l'activité des protéines kinases et également les composés anti-angiogéniques, les agonistes de la gonadoréline, les anti-androgènes, les bengamides, les biphophonates et le trastuzumab.

On peut citer ainsi à titre d'exemples, des agents anti-microtubules comme les taxoides, vinka-alkaloides, des agents d'alkylation tels que cyclophosphamide, des agents DNA-intercalant comme le cis-platinum, des agents interactifs sur topoisomérase comme la camptothécine et dérivés, les anthracyclines comme l'adriamycine, des antimétabolites comme le 5-fluorouracile et dérivés et analogues.

La présente invention concerne donc des produits de formule (I) comme inhibiteurs de protéines kinases, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I) ainsi que leurs prodrugs.

La présente invention concerne particulièrement des produits de formule (I) tels que définis ci-dessus comme inhibiteurs de IGF1R.

La présente invention concerne aussi des produits de formule (I) tels que définis ci-dessus comme inhibiteurs FAK.

La présente invention concerne aussi des produits de formule (I) tels que définis ci-dessus comme inhibiteurs AKT.

La présente invention concerne plus particulièrement les produits de formule (IA) tels que définis ci-dessus comme inhibiteurs de IGF1R.

Les produits de formule (I) selon la présente invention peuvent être préparés par l'application ou l'adaptation de méthodes connues et notamment des méthodes décrites dans la littérature comme par exemple celles décrites par R.C.Larock dans : Comprehensive Organic Transformations, VCH publishers, 1989.

Dans les réactions décrites ci-après, il peut être nécessaire de protéger des groupes fonctionnels réactifs tels que par exemple des groupements hydroxy, amino, imino, thio ou carboxy, quand ceux-ci sont désirés dans le produit final mais quand leur participation n'est pas souhaitée dans les réactions de synthèse des produits de formule (I). On peut utiliser des groupes protecteurs conventionnels en accord avec les pratiques usuelles standard comme ceux décrits par exemple par T.W. Greene and P.G.M.Wuts dans "Protective Groups in Organic Chemistry" John Wiley and Sons, 1991.

Les produits de formule (II) utilisés au départ de l'invention peuvent être obtenus par action du phosgène lorsque X représente un atome d'oxygène ou du thiophosgène lorsque X représente un atome de soufre sur l'amine correspondante de formule (A) soit le dérivé aminophényl portant les substituants Y et Y1' comme définis ci-dessus

Un produit de ce type est décrit également dans le brevet français 2.329.276.

Les produits de formule (III) ou (III') sont connus ou peuvent être préparés à partir de la cyanhydrine correspondante selon le procédé décrit dans la publication : J. Am. Chem. Soc. (1953), 75, 4841.

Les produits de formule (III) peuvent être obtenus par action d'un produit de formule Y2-B2-A2-Hal sur le 2-cyano 2-amino propane dans les conditions énoncées ci-dessus pour l'action de Y2-B2-A2-Hal sur les produits de formule (IV). Un exemple de préparation de ce type est décrit dans la référence :
- Jilek et Coll. Collect. Czech. Chem. Comm. 54(8) 2248 (1989).

Les produits de formule (IV') sont décrits dans le brevet français 2.329.276.

Les produits de départ de formules (V) et (VI), sur lesquels s'exerce un procédé, objet de l'invention, pour l'obtention des produits de formule (I), sont connus et disponibles dans le commerce ou peuvent être préparés selon des méthodes connues de l'homme de métier.

La préparation de produits de formule (VI) est décrite notamment dans les publications suivantes :
- Zhur. Préklad. Khim. 28, 969-75 (1955) (CA 50, 4881a, 1956)
- Tétrahédron 43, 1753 (1987)
- J. Org. Chem. 52, 2407 (1987)
- Zh. Org. Khim. 21, 2006 (1985)
- J. Fluor. Chem. 17, 345 (1981)
ou dans les brevets :
- allemand DRP 637.318 (1935)
- européen EP 0.130.875
- japonais JP 81.121.524.

Les produits de formule (VI) qui sont des dérivés de l'hydantoïne sont largement utilisés et cités dans la littérature comme par exemple dans les articles suivants :
- J. Pharm. Pharmacol., 67, Vol. 19(4), p. 209-16 (1967)
- Khim. Farm. Zh., 67, Vol. 1 (5) p. 51-2
- Brevet allemand 2.217.914
- Brevet européen 0.091.596
- J. Chem. Soc. Perkin. Trans. 1, p. 219-21 (1974).

Les produits de formule (I) de la présente demande telle que définie ci-dessus, pour lesquels p représente l'entier 0 et qui constituent donc des dérivés de l'hydantoine, peuvent être synthétisés selon le procédé indiqué ci-dessus et notamment suivant le schéma général ci-après qui décrit cette synthèse sur support solide. Le protocole qui suit ce schéma donne les conditions opératoires d'une telle synthèse sur support solide des produits de formule (I) de la présente demande.

La partie expérimentale ci-après donne plus particulièrement une illustration d'une telle synthèse sur support solide suivant le protocole ci-dessus avec la préparation des exemples 1 à 56 de la présente demande. Une telle synthèse peut être réalisée suivant le protocole général suivant.

La résine Rink, protégée par un groupement Fmoc est déprotégée par une solution à 20% de pipéridine dans le DMF. La résine amine résultante est couplée à un acide aminé protégé par un groupement Fmoc, en présence de diisopropylaminecarbodiimide (DIC) et d'hydroxybenzotriazole (HOBt). L'acide aminé N-Fmoc, supporté, est ensuite déprotégé par une solution de pipéridine à 20 % dans le DMF. L'amine libre est mise en réaction avec un aldéhyde en solution dans un mélange 50/50 de THF et de triéthylorthormiate (TEOF) pour donner une base de Schiff qui est réduite par le cyanoborohydrure de sodium. L'amine résultante est condensée avec un isocyanate ou un isothiocyanate pour donner l'urée ou la thiourée correspondante. Quand l'isocyanate n'est pas commercial, il peut être préparé à partir de l'amine correspondante par réaction avec 1/3 d'équivalent de triphosgène en présence de 2 équivalents de pyridine. Le produit est ensuite clivé avec un mélange à 95 % d'acide trifluororacétique/eau. L'urée ainsi libérée cyclise pour donner l'hydantoïnine attendue. Dans certain cas, la solution de clivage doit être chauffée à 80°C pour obtenir une cyclisation complète.

Les produits de formule (I) de la présente demande telle que définie ci-dessus, pour lesquels p représente l'entier 1 et qui constituent donc des dérivés de dihydrouraciles, peuvent être synthétisés selon le procédé indiqué ci-dessus et notamment suivant le schéma général ci-après qui décrit cette synthèse sur support solide. Le protocole qui suit ce schéma donne les conditions opératoires d'une telle synthèse sur support solide des produits de formule (I) de la présente demande.

La partie expérimentale ci-après donne plus particulièrement une illustration d'une telle synthèse sur support solide suivant le protocole ci-dessus avec la préparation de l'exemple 5 de la présente demande.

Pour la synthèse des dihydrouraciles sur support solide, on peut utiliser le protocole qui suit.

On utilise par exemple la résine polystyrène Wang (1,7 mmole/g) que l'on traite par un mélange d'acide β-aminé, de chlorure de l'acide 2,6-dichlorobenzoïque et de pyridine dans le DMF. Après lavage, la résine est traitée avec une solution à 10 % de pipéridine dans le DMF. L'amine libre résultante est mise en réaction avec un aldéhyde dans un mélange de THF/ triméthyl orthoformate (TMOF). La base de Schiff résultante est réduite par le cyanoborohydrure de sodium dans un mélange de méthanol, THF et acide acétique. L'amine secondaire obtenue est acylée par le phosgène et le chlorure de carbamoyle résultant est traité par une amine primaire pour donner l'urée correspondante. La cyclisation en dihydouracile ainsi que le clivage du produit final est effectué par le traitement d'une base forte telle que le diazabicycloundécène (DBU).

Les produits de formule (I) de la présente demande peuvent donc être synthétisés sur support solide comme décrit ci-dessus ou en phase liquide suivant le procédé indiqué ci-dessous : la partie expérimentale de la présente demande donne une illustration d'une telle synthèse en phase liquide avec la préparation des exemples 57 à 62.

Pour ce procédé de synthèse en phase liquide, on peut procéder suivant deux voies A et B chacune en deux étapes.

### Voie A :

étape a : l'alkylation de l'aminoester peut s'effectuer par amination réductrice avec un aldéhyde aromatique ou hétérocyclique suivant le procédé général décrit dans Advanced Organic Reaction, March, third edition, page 798-800. En particulier, la formation de la base de Schiff (intermédiaire) peut se faire à partir d'un aminoester éventuellement sous forme de sel, un aldéhyde et éventuellement un agent de déshydratation (par exemple le sulfate de magnésium) dans un solvant comme le dichlorométhane ou le dichloroéthane à une température comprise entre 0°C et celle du reflux du solvant. L'imine formée peut être isolée. L'imine formée est réduite par un hydrure métallique comme le borohydrure de sodium dans un solvant comme un alcool (par exemple l'éthanol ou le méthanol) à une température comprise entre 0°C et celle du reflux du solvant.
étape b : l'aminoester obtenu est condensé avec un isocyanate dans un solvant comme le THF ou le dichlorométhane avec ou sans la présence d'une base (par exemple de la triéthylamine) ou un acide (par exemple de l'acide trifluoroacétique) à une température comprise entre 0°C et celle du reflux du solvant. Quand les isocyanates ne sont pas commercialisés, ils sont préparés à partir des amines correspondantes et de triphosgène ou de diphosgène ou de phosgène en présence d'une base (par exemple la pyridine ou la triéthylamine) d'après le procédé général décrit dans Advanced Organic Reaction, March, third edition, page 370.

### Voie B :

étape a : la formation de l'isocyanate peut se faire par condensation d'une amine aromatique ou hétérocyclique avec du diphosgène en présence de noir végétal activé dans un solvant comme le toluène à une température comprise entre -40°C et celle du reflux du solvant. L'isocyanate formé n'est pas isolé et peut réagir avec aminoester ou son sel dans le même solvant en présence d'une base comme la triéthylamine à une température comprise entre 0°C et celle du reflux du solvant pour conduire au dérivé de 3-aryl-imidazolidine-2,4-dione.
étape b : la condensation de ce dérivé avec un halogénure d'alkyle s'effectue en présence d'une base comme le tert-butylate de potassium ou d'hydrure de sodium dans un solvant comme le THF ou le DMF à une température comprise entre 0°C et celle du reflux du solvant.

Les produits de formule (I) de la présente demande qui constituent les exemples 201 à 207 de la présente demande ont été préparés comme indiqué ci-après dans la partie expérimentale et comme indiqué sur les schémas qui suivent: dans ces schémas, Exemple I représente l'exemple 201, Exemple II représente l'exemple 202, Exemple III représente l'exemple 203, Exemple IV représente l'exemple 204, Exemple V représente l'exemple 205, Exemple VI représente l'exemple 206 et Exemple VII représente l'exemple 207.

### EXEMPLES I et II

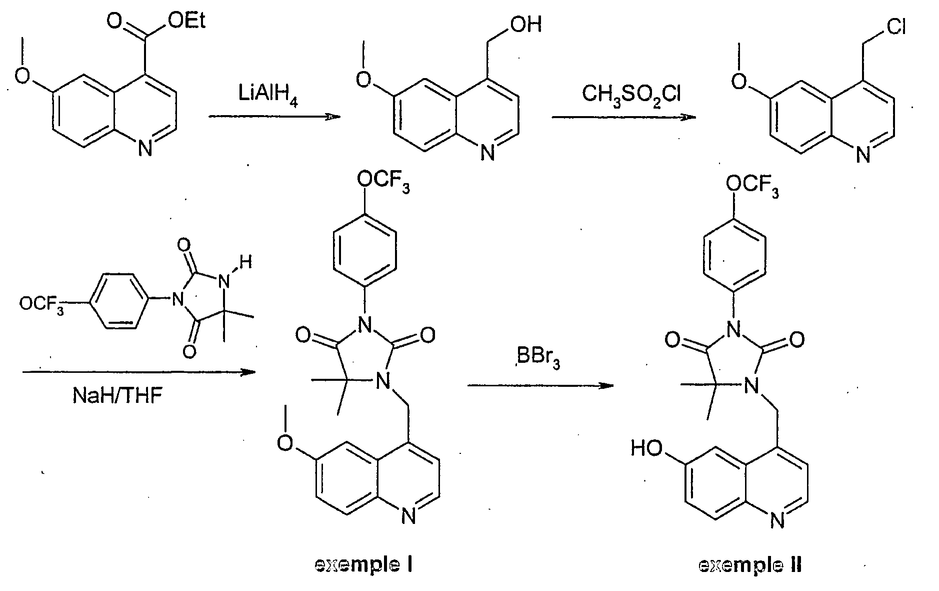

### EXEMPLES III et IV

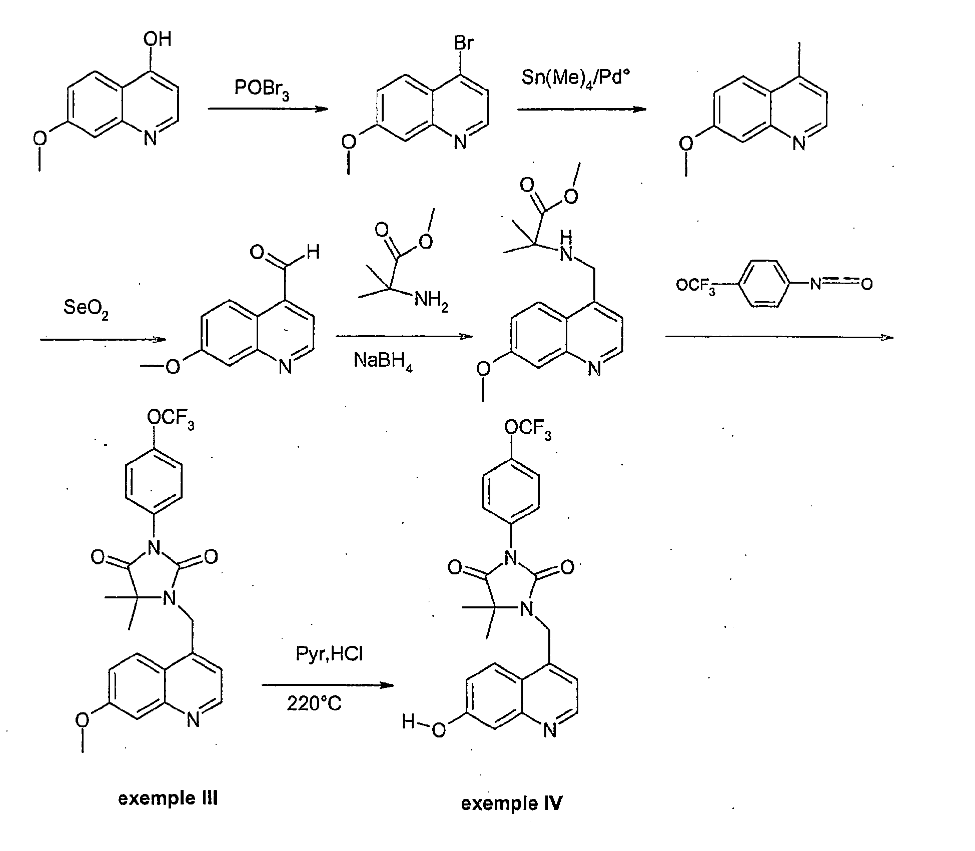

### EXEMPLES V, VI et VII

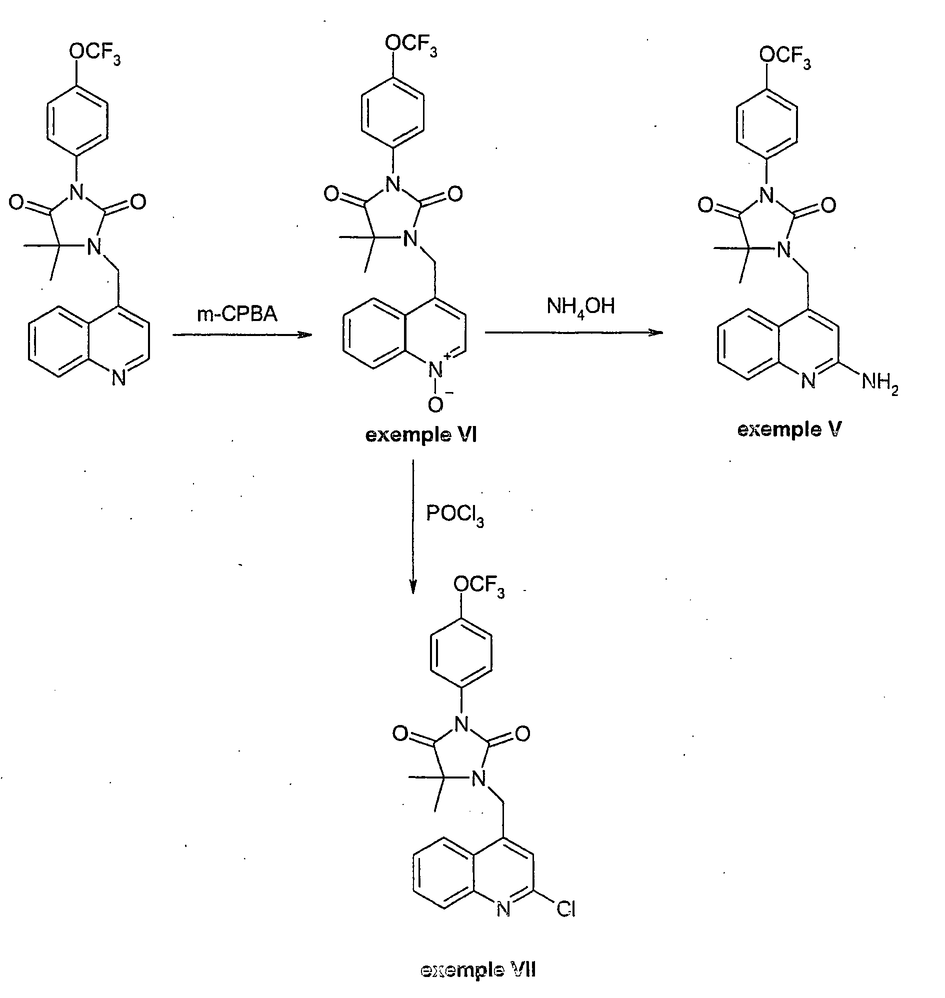

La synthèse des produits de formule (I) de la présente demande qui constituent les produits des exemples 208 à 243 a été réalisée en utilisant la voie B. L'halogénure d'alkyle peut être préparé à partir des acides carboxyliques correspondants.

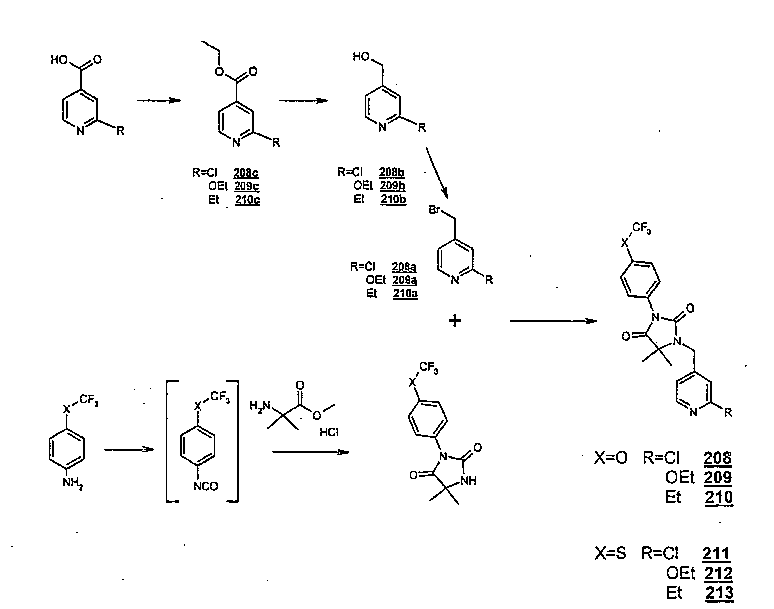

Les carboxylates d'éthyle ont été préparés par estérification des acides ,carboxyliques dans l'éthanol en présence d'acide sulfurique en reprenant les conditions décrites dans Synthesis 2000, 1138.

La réduction des carboxylates d'éthyle en alcool a été effectuée dans l'éthanol en présence de borohydrure de sodium en reprenant les conditions décrites dans Synthesis 2000, 1665.

La transformation des alcools ainsi obtenus en halogénures d'alkyle a été réalisée en utilisant le dibromotriphénylphosphorane comme agent d'halogénation en reprenant les conditions décrites dans J. Heterocyclic Chem., 30, 631 (1993)

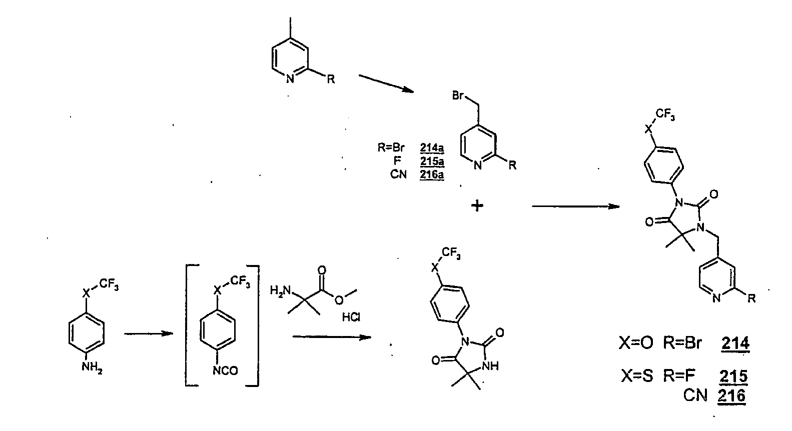

Les halogénures d'alkyle peuvent également être préparés par bromation radicalaire des méthylènes correspondants en présence de N-bromosuccinimide et de peroxyde de benzoyle dans le tétrachlorure de carbone en reprenant les conditions décrites dans J. Heterocyclic Chem., 30, 631 (1993)

C'est ainsi que nous avons fonctionnalisé le noyau pyridine en position-2 par atome de brome, de fluor ou encore par un groupement nitrile.

Ce dernier composé va également nous permettre de préparer différents composés carbonylés.

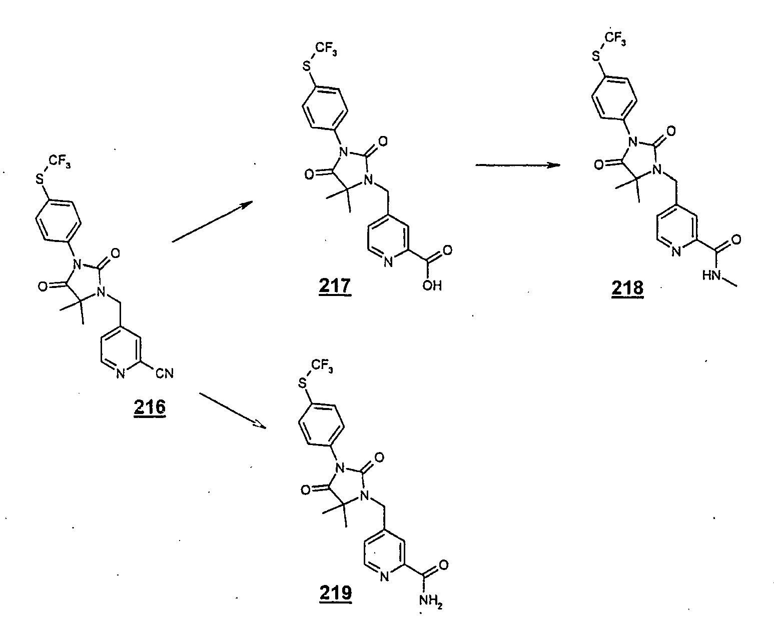

L'hydrolyse acide du nitrile en présence d'acide sulfurique en reprenant les conditions décrites dans J. Med. Chem. 1991, 34, 281-290, nous a conduit au carboxamide sous forme de dimère. L'hydrolyse du nitrile dans des conditions plus douces nous a permis d'obtenir le carboxamide attendu.

L'hydrolyse acide du nitrile en présence d'acide chlorhydrique 5N en reprenant les conditions décrites dans J. Heterocyclic Chem., 30, 631 (1993), nous a conduit à l'acide carboxylique correspondant.

L'amide a pu être préparé à partir de l'acide carboxylique en utilisant le chlorhydrate de 1- (3-diméthylaminopropyl)-3-éthylcarbodiimide comme agent de couplage dans le dichlorométhane en reprenant les conditions décrites dans J. Am. Chem. Soc., 95, 875, (1973).

Différents analogues aminés ont été obtenus par substitution nucléophile de l'intermédiaire chloré par des amines sous irradiation dans un four à micro-onde en s'inspirant des conditions décrites dans Tetrahedron 2002, 58, 1125.

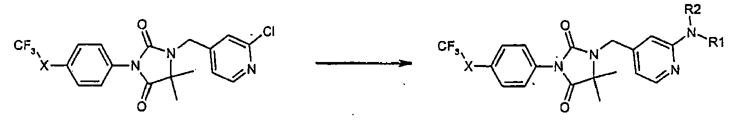

| X | NR1R2 | | NR1R2 | | NR1R2 | |
|---|---|---|---|---|---|---|
| O | 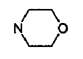 | **220** | | | | |
| S | 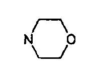 | **221** | 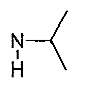 | **225** | 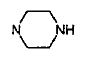 | **229** |
| | 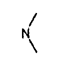 | **222** | 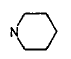 | **226** | 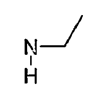 | **230** |
| | 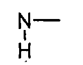 | **223** | 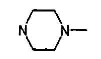 | **227** | 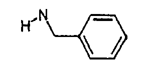 | **231** |
| | 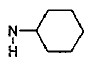 | **224** | 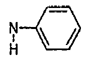 | **228** | 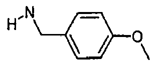 | **232** |

L'analogue amino a été obtenu par déprotéction du groupement *p*méthoxybenzylamine en présence d'acide trifluoroacétique en s'inspirant des conditions décrites dans J. Chem. Soc., Perkin Trans. 1, 2002, 428-433.

Ce dérivé amino nous a permis d'accéder à d'autres fonctions chimiques comme la fonction amide, la fonction carbamate ou encore la fonction sulfonamide.

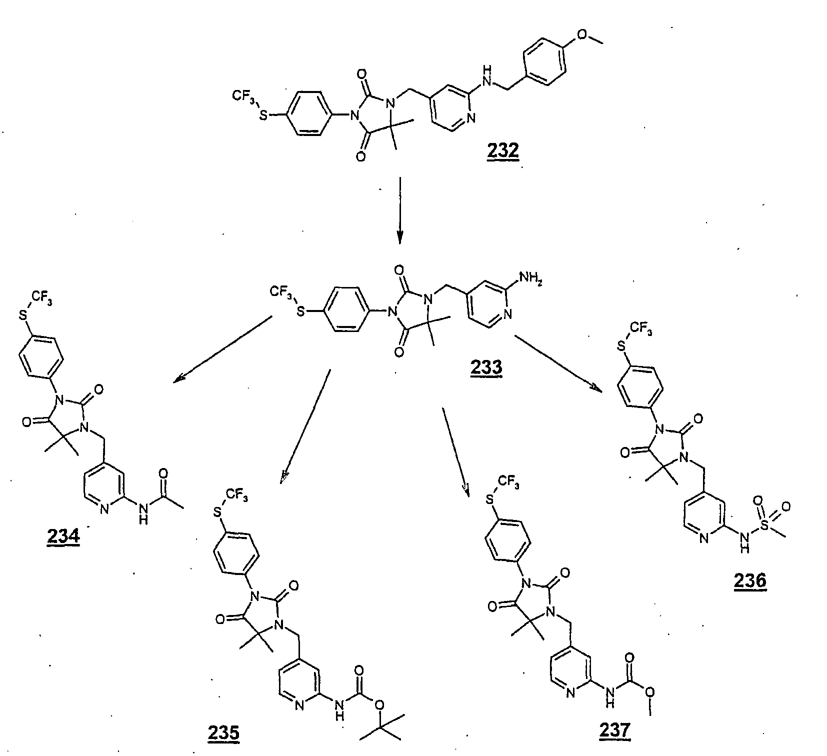

Le dérivé acétamido a été obtenu par acylation du dérivé aminé en présence de anhydride acétique en s'inspirant des conditions décrites dans Tetrahedron Lett. 2002, 43, 3121. Nous avons également préparé les carbamates de méthyle et de ter-buthyle en s'inspirant des conditions décrites dans J. Heterocyclic Chem., 22, 313 (1985) et dans J. Org. Chem. 2002, 67, 4965. Le dérivé aminé a également été sulfonylé par le chlorure de mésyle en s'inspirant des conditions décrites dans J. Med. Chem., 1985, 28, 824.

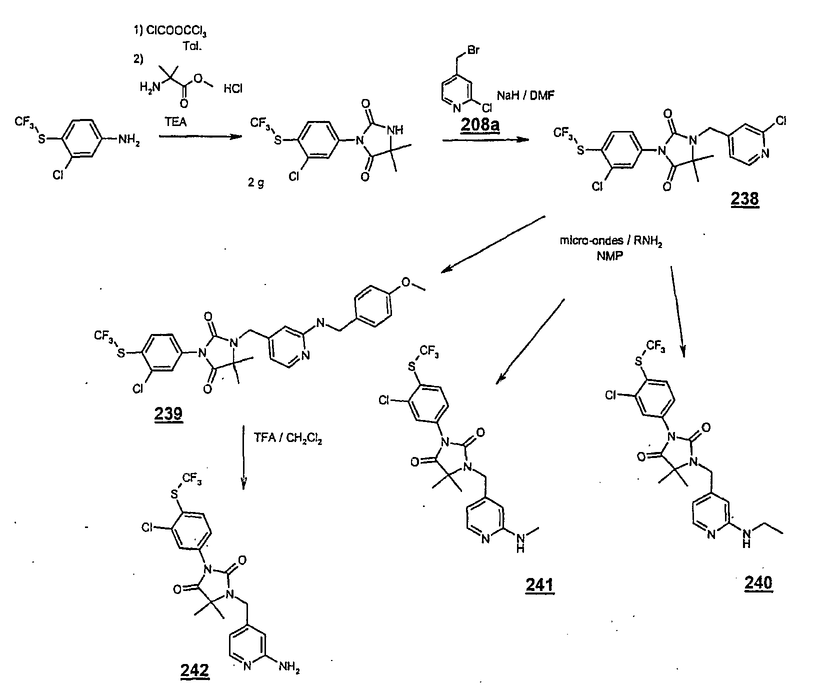

Les conditions précédentes ont également été utilisées pour la synthèse des composés possédants un groupement phényl disubstitué.

L'hydantoïne possédant un noyau pyridine disubstitué en position -2,6 par un atome de brome a été préparée en utilisant les conditions précédentes à partir de la 2,6-dibromo-4-(hydroxyméthyl)pyridine décrite dans Synthesis 2000, 1665.

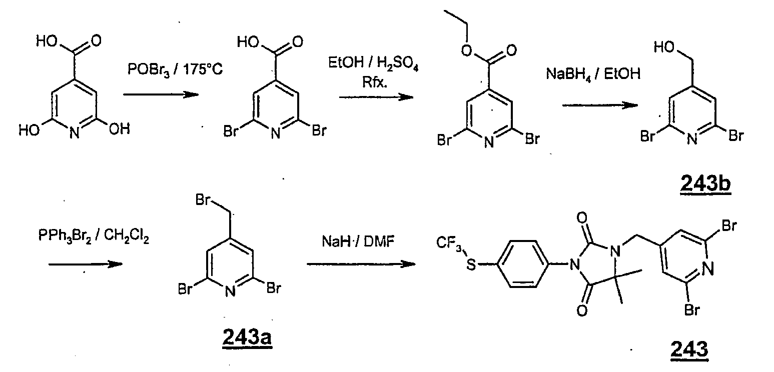

Les produits peuvent être purifiés comme suit:

### Purification par LC/MS

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système était controlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau/acétonitrile 95/5 (v/v) contenant 0,07 % (v/v) d'acide trifluoroacétique, pendant que l'autre colonne était en cours de séparation. L'élution des colonnes a été effectuée en utilisant un gradient linéaire de 5 à 95% d'acétonitrile contenant 0,07 % (v/v) d'acide trifluoroacétique dans l'eau contenant 0,07 % (v/v) d'acide trifluoroacétique, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90 % d'acétonitrile contenant 0,05 % (v/v) d'acide trifluoroacétique (TFA) dans l'eau contenant 0,05% (v/v) TFA en 3,5 mn à un débit de 1 mL/mn. Le temps total d'analyse, incluant la période de rééquilibration de la colonne, est de 7 minutes.

Les produits des exemples 244 à 255 de la présente invention ont été préparés comme indiqué dans la partie expérimentale et suivant la voie de synthèse générale du schéma ci-après :

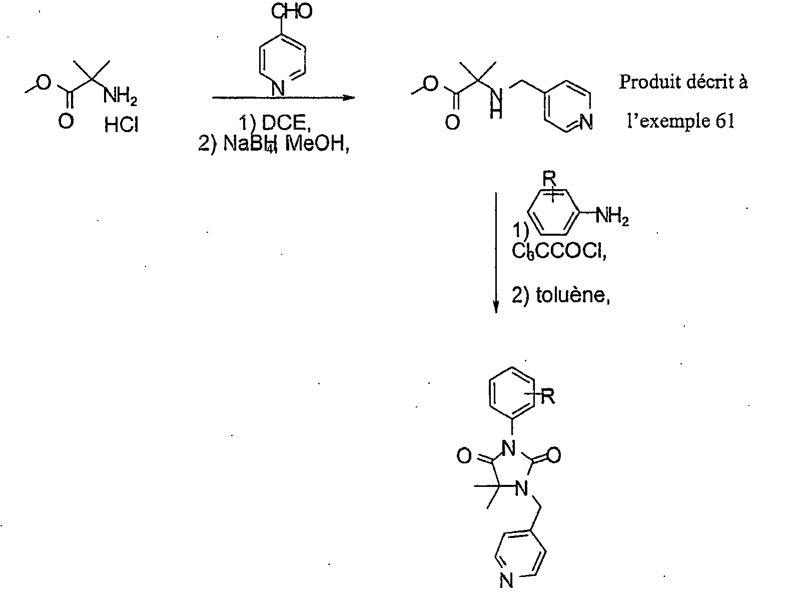

Les produits des exemples 256 à 263 de la présente invention ont été préparés selon les schémas réactionnels 1 et 2 indiqués ci-dessous dans lesquels les chiffres 1 à 8 correspondent respectivement aux exemples 256 à 263 : les produits des exemples 256 à 261 (soit produits 1 à 6) ont été préparés selon le schéma 1 et les deux composés thiohydantoine des exemples 262 et 263 (soit produits 7 et 8) ont été préparés selon le schéma 2.

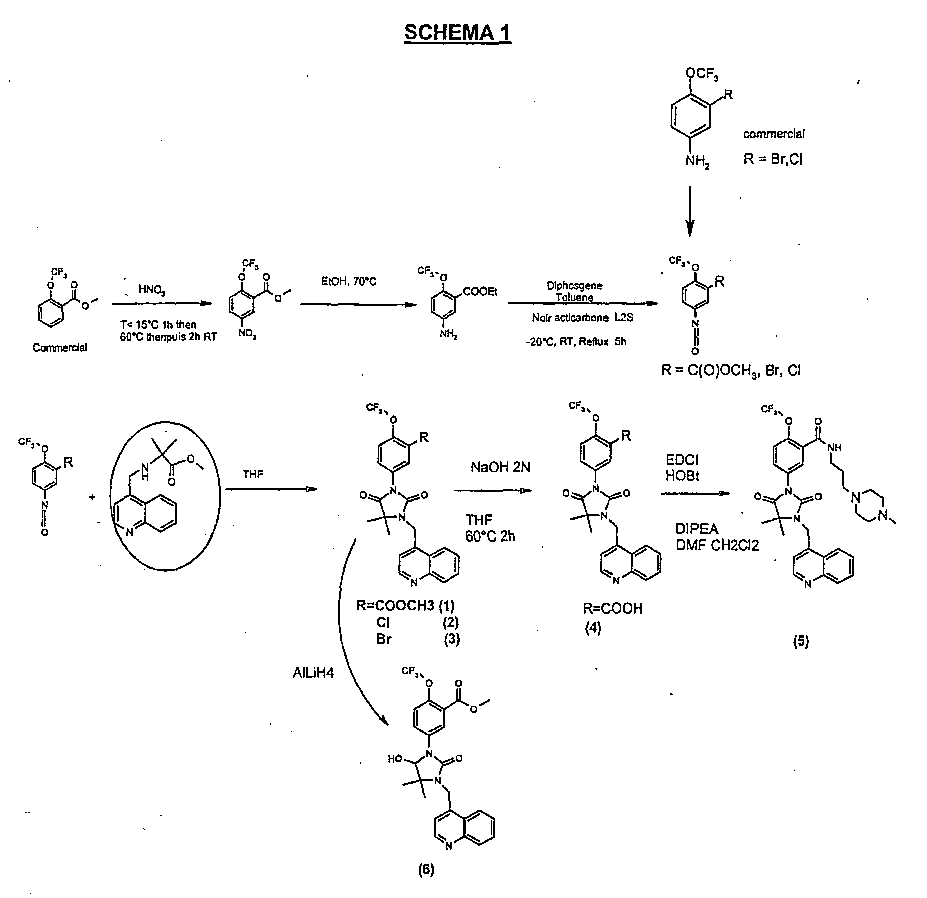

Le composé nitré est préparé par nitration du 2-trifluromethoxy-benzoic acid methyl ester par nitration (acide nitrique fumant) en contrôlant la température. Selon les conditions décrites dans le brevet PCT Int. Appl. (2000), 564 : WO 0069810.
L'amine correspondante est préparée par réduction de la fonction nitrée en présence SnCl₂ dans l'éthanol selon le même brevet.
L'isocyanate est préparé par réaction du diphosgene en solution dans le toluène à -20°C dans les conditions usuelles connues. L'isocyanate est mis en réaction avec le dérivé quinoléine préparé selon les méthodes connues afin de préparer l'hydantoine voulue.
L'acide est obtenu par saponification en utilisant de la soude 2N dans le THF à 60°C.
L'amide est préparé par couplage de l'amine voulue en utilisant EDCI comme agent de couplage (conditions classiques de couplage.
L'alcool est obtenu par réduction de l'ester dans le THF en présence AlLiH₄.
Les dérivés halogénés R= Cl et R= Br sont préparés à partir des anilines commerciales selon le même schéma de synthèse.

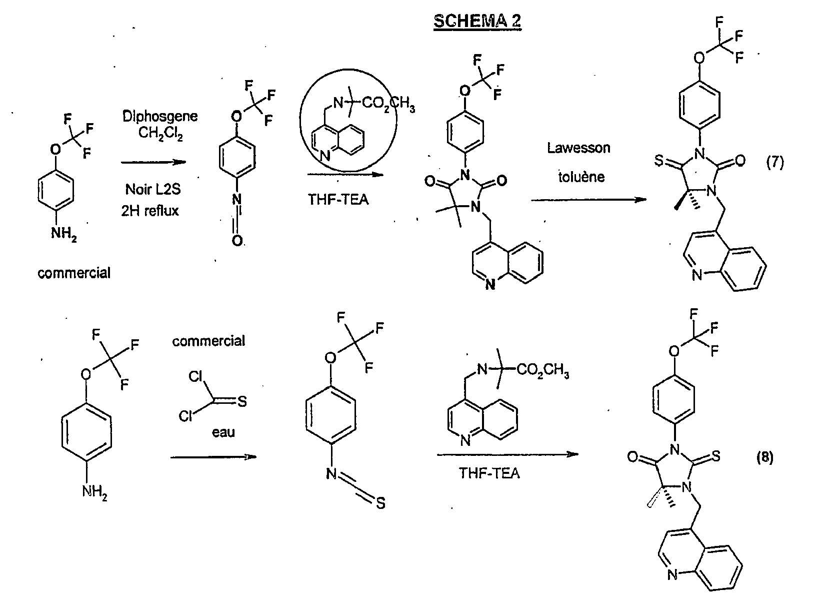

Les exemples dont la préparation suit illustrent la présente invention sans toutefois la limiter.

### Exemple 1 : Préparation du trifluoroacétate de (S)-5-méthyl-1-quinolin-4-ylméthyl-3-(4-trifluorométhanesulfonyl-phényl)-imidazolidine-2,4-dione

Dans une seringue de 50 ml munie d'un fritté, est mise en suspension 2 g (1,02 mmoles) de Polystyrène AM RAM (résine Rink) (0,51 mmol/g) dans 20 ml de DMF. Après 10 minutes d'agitation le DMF est filtré et remplacé par 10 ml de solution à 20 % de pipéridine dans le DMF. Après une heure d'agitation à température ambiante, la solution est filtrée et la résine lavée successivement par 3 X 10 ml de DMF, 2 X 10 ml de méthanol, et 3 X 10 ml de DMF. Une solution de 0,94 g de Fmoc-Ala(OH) (3 mmoles), 0,41 g de HOBt (3 mmoles) et 0,48 ml de DIC (3 mmoles) dans 10 ml de DMF est additionnée à la résine. La seringue est laissée une nuit sous agitation à température ambiante, puis la résine est lavée successivement par 5 X 10 ml de DMF, 3 X 10 ml de MeOH et 5 X 10 ml de DCM. Ensuite, 10 ml de solution de pipéridine à 20 % dans le DMF sont introduits dans la seringue. Après 1 heure d'agitation, la solution est filtrée et la résine lavée par 5 X 10 ml de DMF, 2 X 10 ml de MeOH, 3 X 10 ml de DCM et 3 X 10 ml de THF. Puis une solution de 0,79 g de quinoline 4-carboxaldéhyde (5,1 mmoles) dans 10 ml d'un mélange 50/50 de THF/TEOF est additionnée à la résine. Après une nuit d'agitation a température ambiante, la solution est filtrée et la résine lavée avec 10 X 10 ml de THF. A la résine est ensuite additionnée 0,63 g de cyanoborohydrure de sodium dans un mélange de 1,5 ml de MeOH, 3,5 ml de dichloroéthane et 0,1 ml d'acide acétique. La résine est laissée une nuit sous agitation, puis après filtration, lavée par 10 X 10 ml de DCM, 3 X 10 ml de MeOH et 5 X 10 ml de DCM.

Parallèlement, une solution de 0,563 g de 4-(trifluorométhanesulfonyl)aniline (2,5 mmoles) est traitée avec 0,25 g de triphosgène (0,83 mmole) suivi par 0,23 ml de pyridine (2,5 mmoles) à 0°C sous azote. Après que la température est progressivement remontée à TA, la réaction est laissée 2 heures sous agitation, puis 0,23 ml de pyridine dans 1 ml de DMF est de nouveau additionné au mélange. La solution obtenue est transférée dans la seringue qui est laissée sous agitation 2 heures. La solution est ensuite filtrée et la résine lavée par 5 X 10 ml de DCM, 3 X 10 ml de MeOH et 5 X 10 ml de DCM. Finalement la résine est traitée avec 5 ml de solution à 95 % d'acide trifluoroacétique dans l'eau. Le mélange est maintenu 2 heures sous agitation puis filtré. La résine est lavée avec 2 ml de MeOH, suivis de 2 ml de DCM. Les filtrats rassemblés sont évaporés sous vide. 280 mg de produit brut sont ainsi obtenus. Après purification par LC-MS préparative, 240 mg (rendement global = 41 %) de produit attendu sont isolés sous forme de solide blanc.
EIMS ([M+H]+ : 464
Temps de rétention (TR) = 3,12 min (colonne YMC Basic S5 ; gradient ACN/H2O 2-85 % en 7 min)
RMN-H1 (300MHz) (CDC13): 1,59(d, 3H); 4,11(t, 1H); 5,01 et 5, 55 (AB, 2H) ; 7,70 (d, 1H); 7,89(m, 1H) ; 7,99(m, 3H) ; 8, 15 (m, 2H); 8,30(d, 1H), 8, 50 (d, 1H) ; 9,22(d, 1H) .

### Exemple 2 : Préparation du trifluoroacétate de (S)-4-méthyl-3-quinolin-4-ylméthyl-5-thioxo-1-(4-trifluorométhanesulfonyl-phényl)-imidazolidine-2-one

La résine 3, 0,036 mmole, préparée selon l'exemple 1, est utilisée pour la préparation du composé. 33 mg de thiocarbonyldiimidazole (0,18 mmole) sont additionnés à une solution de 41 mg de 4-(trifluorométhane-sulfonyl)aniline (0,18 mmole) dans 3 ml de DCM. Le mélange réactionnel est maintenu 2 heures sous agitation à température ambiante, puis directement ajouté à la résine. Après 2 heures d'agitation, la solution est filtrée puis la résine est lavée avec 5 X 2 ml de DCM, 3 X 5 ml de MeOH et 5 X 2 ml de DCM.
Enfin, 2 ml de solution à 95 % de TFA dans l'eau sont additionnés à la résine. Après 2 heures d'agitation, le mélange est filtré, la résine lavée avec 1 ml de MeOH et 1 ml de DCM. Les filtrats rassemblés sont chauffés à 60°C pendant 2 heures, puis concentrés sous vide. Après purification par LC-MS préparative, on isole 1,8 mg de produit attendu.
EIMS [(M+H]+) : 480
TR= 4,72 min (colonne YMC Basic S5 ; gradient ACN/H2O 2-85 % en 7 min)

### Exemple 3 : Préparation du trifluoroacétate de (S)-5-méthyl-1-pyridin-4-ylméthyl-3-(4-trifluorométhane sulfonyl-phényl)-imidazolidine-2,4-dioae

Le composé est préparé à partir de 0,025 mmole de résine, 0,075 mmole de N-Fmoc-L-Ala(OH), 0,125 mmole de 4-pyridinecarboxaldéhyde, et 0,0625 mmole de 4-(trifluorométhanesulfonyl)aniline, de la même façon que l'exemple 1. Après purification par LC-MS préparative, on obtient 5,6 mg de produit attendu
EIMS ([M+H]+) : 414
TR= 2,72 min

### Exemple 4 : Préparation du trifluoroacétate de (S)-5-méthyl-1-pyridin-4-ylméthyl-3-(4-trifluorométhylsulfanylphényl)-imidazolidine-2,4-dione

Le composé est préparé à partir de 0,025 mmole de résine, 0,075 mmole de N-Fmoc-L-Ala(OH), 0,125 mmole de 4-pyridinecarboxaldéhyde, et 0,0625 mmole de 4-(trifluorométhanéthio)aniline, de la même façon que l'exemple 1. Après purification par LC-MS préparative, on obtient 1,3 mg de produit attendu
EIMS ([M+H]+) : 382
TR= 2,83 min

### Exempte 5 : Préparation du trifluoroacétate de (S)-5-méthyl-1-quiaolin-4-ylméthyl-3-(4-trifluorométhylsulfanyl-phényl)-imidazolidine-2,4-dione

Le composé est préparé à partir de 0,025 mmole de résine, 0,075 mmole de N-Fmoc-L-Ala(OH), 0,125 mmole de 4-quinolinecarboxaldéhyde, et 0,0625 mmole de 4-(trifluorométhanéthio)aniline, de la même façon que l'exemple 1. Après purification par LC-MS préparative, on obtient 0,6 mg de produit attendu.
EIMS ([M+H]+) : 432
TR= 3,14 min

### Exemple 6 : Préparation du trifluoroacétate de 1-quinolin-4-ylméthyl-3-(4-trifluorométhanesulfonylphényl)-imidazolidine-2,4-dione

Le composé est préparé à partir de 4 mmoles de résine, 12 mmoles de N-Fmoc-Gly(OH), 20 mmoles de 4-quinolinecarboxaldéhyde, et 10 mmoles de 4-(trifluorométhanesulfonyl) aniline, de la même façon que l'exemple 1. Après purification par LC-MS préparative, on obtient 1 g de produit attendu
EIMS ([M+H]+) : 450
TR= 3,20 min

### Exemple 7 : Préparation du trifluoroacétate de 5,5-diméthyl-1-quinolin-4-ylméthyl-3-(4-trifluorométhanesulfonyl-phényl)-imidazolidine-2,4-dione

Le composé est préparé à partir de 0,25 mmole de résine, 0,75 mmole de Fmoc-AIB-(OH), 1,25 mmole de 4-quinolinecarboxaldéhyde, et 0,625 mmole de 4-(trifluorométhanesulfonyl)aniline, de la même façon que l'exemple 1. Après purification par LC-MS préparative, on obtient 22 mg de produit attendu
EIMS ([M+H] +) : 478
TR= 4,26 min

### Exemple 8 : Préparation du trifluoroacétate de (R)-5-méthyl-1-quinolin-4-ylméthyl-3-(4-trifluorométhanesulfonyl-phényl)-imidazolidine-2,4-dione

Le composé est préparé à partir de 0,04 mmole de résine, 0,12 mmole de N-Fmoc-D-Ala(OH), 0,20 mmole de 4-quinolinecarboxaldéhyde, et 0,10 mmole de 4-(trifluorométhanesulfonyl) aniline, de la même façon que l'exemple 1. Après purification par LC-MS préparative, on obtient
10 mg de produit attendu
EIMS ([M+H]+) : 464
TR= 4,36 min

### Exemple 9 : Préparation du trifluoroacétate de (R)-5-méthyl-1-quinolin-4-ylméthyl-3-(4-trifluorométhylsulfanyl-phényl)-imidazolidine-2,4-dione

Le composé est préparé à partir de 0,04 mmole de résine, 0,12 mmole de N-Fmoc-D-Ala(OH), 0,20 mmole de 4-quinolinecarboxaldéhyde, et 0,10 mmole de 4-(trifluoro-méthanéthio) aniline, de la même façon que l'exemple 1. Après purification par LC-MS préparative, on obtient 11 mg de produit attendu
EIMS ([M+H]+) : 432
TR= 4,50 min

### Exemple 10 : Préparation du trifluoroacétate de (R)-5-méthyl-1-pyridin-4-ylméthyl-3-(4-trifluorométhanesulfonyl-phényl)-imidazolidine-2,4-dione

Le composé est préparé à partir de 0,28 mmole de résine, 0,84 mmole de N-Fmoc-D-Ala(OH), 1,4 mmole de 4-pyridinecarboxaldéhyde, et 0,70 mmole de 4-(trifluorométhanesulfonyl) aniline, de la même façon que l'exemple 1. Après purification par LC-MS préparative, on obtient 105 mg de produit attendu
EIMS ([M+H]+) : 414
TR= 2,40 min

### Exempta 11 : Préparation du trifluoroacétate de (R)-5-méthyl-1-pyridin-4-ylméthyl-3-(4-trifluorométhylsulfanyl-phényl)-imidazolidine-2,4-dione

Le composé est préparé à partir de 0,28 mmole de résine, 0,84 mmole de N-Fmoc-D-Ala(OH), 1,4 mmole de 4-pyridinecarboxaldéhyde, et 0,70 mmole de 4-(trifluorométhanethio) aniline, de la même façon que l'exemple 1. Après purification par LC-MS préparative, on obtient 91 mg de produit attendu
EIMS ([M+H]+) : 382
TR= 2,52 min

### Exemple 12 : Préparation du trifluoroacétate de (S)-5-méthyl-1-(3-méthyl-pyridin-4-ylméthyl)-3-(4-trifluorométhylsulfanyl-phényl)-imidazolidine-2,4-dione

Le composé est préparé à partir de 0,04 mmole de résine, 0,12 mmole de N-Fmoc-L-Ala(OH), 0,20 mmole de 3-méthyl-4-pyridinecarboxaldéhyde, et 0,10 mmole de 4-(trifluorométhanethio)aniline, de la même façon que l'exemple 1. Après purification par LC-MS préparative, on obtient 17 mg de produit attendu
EIMS ([M+H]+) : 396
TR= 4,20 min

### Exemple 13 : Préparation du trifluoroacétate (S)-5-méthyl-1-(3-méthyl-pyridin-4-ylméthyl)-3-(4-trifluorométhanesulfonyl-phényl)-imidazolidiae-2,4-dione

Le composé est préparé à partir de 0,04 mmole de résine, 0,12 mmole de N-Fmoc-L-Ala(OH), 0,20 mmole de 3-méthyl-4-pyridinecarboxaldéhyde, et 0,10 mmole de 4-(trifluorométhanesulfonyl)aniline, de la même façon que l'exemple 1. Après purification par LC-MS préparative, on obtient 16 mg de produit attendu.
EIMS ([M+H]+) : 428
TR= 4,07 min

### Exemple 14 : Préparation du trifluoroacétate de (S)-4-méthyl-3-pyridin-4-ylméthyl-5-thioxo-1-(4-trifluorométhylsulfanyl-phényl)-imidazolidin-2-one

Le composé est préparé à partir de 0,04 mmole de résine, 0,12 mmole de N-Fmoc-L-Ala(OH), 0,20 mmole de 4-pyridihecarboxaldéhyde, et 0,10 mmole de 4-(trifluorométhanethio) aniline, de la même façon que l'exemple 2. Après purification par LC-MS préparative, on obtient 1,7 mg de produit attendu
EIMS ([M+H]+) : 398
TR= 4,51 min

### Exemple 15 : Préparation du trifluoroacétate de (S)-4-méthyl-3-pyridia-4-ylméthyl-5-thioxo-1-(4-trifluorométhanesulfonyl-phényl)-imidazolidin-2-one

Le composé est préparé à partir de 0,04 mmole de résine, 0,12 mmole de N-Fmoc-L-Ala(OH), 0,20mmole de 4-pyridinecarboxaldéhyde, et 0,10 mmole de 4-(trifluorométhanesulfonyl) aniline, de la même façon que l'exemple 2. Après purification par LC-MS préparative, on obtient 2,2 mg de produit attendu
EIMS ([M+H]+) : 430
TR= 4,34 min

### Exemple 16 : Préparation du trifluoroacétate de (R)-4-méthyl-3-(3-méthyl-pyridin-4-ylméthyl)-5-thioxo-1-(4-trifluorométhylsulfanyl-phényl)-imidazolidin-2-one

Le composé est préparé à partir de 0,04 mmole de résine, 0,12 mmole de N-Fmoc-D-Ala(OH), 0,20 mmole de 3-méthyl-4-pyridinecarboxaldéhyde, et 0,10 mmole de 4-(trifluorométhanethio)aniline, de la même façon que l'exemple 2. Après purification par LC-MS préparative, on obtient 1,9 mg de produit attendu
EIMS ([M+H]+) : 412
TR= 4,60 min

### Exemple 17 : Préparation du trifluoroacétate de (R)-4-méthyl-3-(3-méthyl-pyridin-4-ylméthyl)-5-thioxo-1-(4-trifluorométhylsulfonyl-phényl)-imidazolidin-2-one

Le composé est préparé à partir de 0,04 mmole de résine, 0,12 mmole de N-Fmoc-D-Ala(OH), 0,20 mmole de 3-méthyl-4-pyridinecarboxaldéhyde, et 0,10 mmole de 4-(trifluorométhanesulfonyl)aniline, de la même façon que l'exemple 2. Après purification par LC-MS préparative, on obtient 4,5 mg de produit attendu
EIMS ([M+H]+) : 444
TR= 4,41 min

### Exemple 18 : Préparation du trifluoroacétate (R)-5-méthyl-1-(3-méthyl-pyridin-4-ylméthyl)-3-(4-trifluorométhylsulfanyl-phényl)-imidazolidine-2,4-dione

Le composé est préparé à partir de 0,04 mmole de résine, 0,12 mmole de N-Fmoc-D-Ala(OH), 0,20 mmole de 3-méthyl-4-pyridinecarboxaldéhyde, et 0,10 mmole de 4-(trifluorométhanethio)aniline, de la même façon que l'exemple 1. Après purification par LC-MS préparative, on obtient 14 mg de produit attendu
EIMS ([M+H]+) : 396
TR= 4,22 min

### Exemple 19 : Préparation du trifluoroacétate de (R)-5-méthyl-1-(3-méthyl-pyridin-4-ylméthyl)-3-(4-trifluorométhanesulfonyl-phényl)-imidazolidine-2,4-dione

Le composé est préparé à partir de 0,04 mmole de résine, 0,12 mmole de N-Fmoc-D-Ala(OH), 0,20 mmole de 3-méthyl-4-pyridinecarboxaldéhyde, et 0,10 mmole de 4-(trifluorométhanesulfonyl)aniline, de la même façon que l'exemple 1. Après purification par LC-MS préparative, on obtient 6,3 mg de produit attendu
EIMS ([M+H]+) : 428
TR= 4,10 min

### Exemple 20 : Préparation du trifluoroacétate de (R)-4-méthyl-3-quinolin-4-ylméthyl-5-thioxo-1-(4-trifluorométhylsulfanyl-phényl)-imidazolidin-2-one

Le composé est préparé à partir de 0,04 mmole de résine, 0,12 mmole de N-Fmoc-D-Ala(OH), 0,20 mmole de 4-quinolinecarboxaldéhyde, et 0,10 mmole de 4-(trifluorométhanethio) aniline, de la même façon que l'exemple 2. Après purification par LC-MS préparative, on obtient 0,4 mg de produit attendu
EIMS ([M+H]+) : 448
TR= 4,89 min

### Exemple 21 : Préparation du trifluoroacétate de (R)-5-isopropyl-1-quinolin-4-ylméthyl-3-(4-trifluorométhylsulfanyl-phényl)-imidazolidine-2,4-dione

Le composé est préparé à partir de 0,05 mmole de résine, 0,15 mmole de N-Fmoc-D-Val(OH), 0,25 mmole de 4-quinolinecarboxaldéhyde, et 0,125 mmole de 4-(trifluorométhanethio) aniline, de la même façon que l'exemple 1. Après purification par LC-MS préparative, on obtient 0,3 mg de produit attendu
TR= 4,01 min
EIMS ([M+H]+) : 460

### Exemple 22 : Préparation du trifluoroacétate de (R)-5-isopropyl-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Val(OH), 0,25 mmole de 4-quinolinecarboxaldéhyde, et 0,125 mmole de 4-(trifluorométhanesulfonyl) aniline, de la même façon que l'exemple 1. Après purification par LC-MS préparative, on obtient 1 mg de produit attendu
EIMS ([M+H]+) : 492
TR= 3,91 min

### Exemple 23 : Préparation du trifluoroacétate de (R)-5-Benzyl-1-quinolin-4-ylméthyl-3-(4-trifluorométhylsulfanyl-phényl)-imidazolidine-2,4-dione

Le composé est préparé à partir de 0,05 mmole de résine, 0,15 mmole de N-Fmoc-D-Phe(OH), 0,25 mmole de 4-quinolinecarboxaldéhyde, et 0,125 mmole de 4-(trifluorométhanethio)aniline, de la même façon que l'exemple 1. Après purification par LC-MS préparative, on obtient 8,9 mg de produit attendu
EIMS ([M+H]+) : 508
TR= 4,11 min

### Exemple 24 : Préparation du trifluoroacétate de (R)-5-Benzyl-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Phe(OH), 0,25 mmole de 4-quinolinecarboxaldéhyde, et 0,125 mmole de 4-(trifluorométhanesulfonyl)aniline, de la même façon que l'exemple 1. Après purification par LC-MS préparative, on obtient 5,9 mg de produit attendu
EIMS ([M+H]+) : 540
TR= 4,01 min

### Exemple 25 : Préparation du trifluoroacétate de (R)-5-Benzyl-1-pyridin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Phe(OH), 0,25 mmole de 4-pyridinecarboxaldéhyde, et 0,125 mmole de 4-(trifluorométhanesulfonyl)aniline, de la même façon que l'exemple 1. Après purification par LC-MS préparative, on obtient 11,1 mg de produit attendu
EIMS ([M+H]+) : 490
TR= 3,76 min

### Exemple 26 : Préparation du trifluoroacétate de (R)-5-isobutyl-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Leu(OH), 0,25 mmole de 4-quinolinecarboxaldéhyde, et 0,125 mmole de 4-(trifluorométhanesulfonyl)aniline, de la même façon que l'exemple 1. Après purification par LC-MS préparative, on obtient 5,9 mg de produit attendu
TR= 4,02 min
EIMS ([M+H]+) : 506

### Exemple 27 : Préparation du trifluoroacétate de (R)-5-(4-hydroxy-benzyl)-1-quinolin-4-ylméthyl-3-(4-trifluorométhylsulfanyl-phényl)-imidazolidine-2,4-dione

Le composé est préparé à partir de 0, 05 mmole de résine, 0,15 mmole de N-Fmoc-D-Tyr(OH), 0,25 mmole de 4-quinolinecarboxaldéhyde, et 0,125 mmole de 4-(trifluorométhanethio) aniline, de la même façon que l'exemple 1. Après purification par LC-MS préparative, on obtient 5,1 mg de produit attendu
EIMS ([M+H]+) : 524
TR= 3,75 min

### Exemple 28 : Préparation du trifluoroacétate de (R)-5-(4-hydroxy-benzyl)-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Tyr(OH), 0,25 mmole de 4-quinolinecarboxaldéhyde, et 0,125mmole de 4-(trifluorométhanesulfonyl) aniline, de la même façon que l'exemple 1. Après purification par LC-MS préparative, on obtient 1.8 mg de produit attendu
EIMS ([M+H]+) : 556
TR= 3,66 min

### Exemple 29 : Préparation du trifluoroacétate de (R)-5-(4-hydroxy-benzyl)-1-pyridin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Tyr(OH), 0,25 mmole de 4-pyridinecarboxaldéhyde, et 0,125 mmole de 4-(trifluorométhanesulfonyl) aniline, de la même façon que l'exemple 1. Après purification par LC-MS préparative, on obtient 0,7 mg de produit attendu
EIMS ([M+H]+): 506
TR= 3,48 min

### Exemple 30 : Préparation du trifluoroacétate de (R)-5-(1-hydroxy-éthyl)-1-quinolin-4-ylméthyl-3-(4-trifluorométhylsulfanyl-phényl)-imidazolidine-2,4-dione

Le composé est préparé à partir de 0,05 mmole de résine, 0,15 mmole de N-Fmoc-D-Thr(OH), 0,25 mmole de 4-quinolinecarboxaldéhyde, et 0,125 mmole de 4-(trifluorométhanethio) aniline, de la même façon que l'exemple 1. Après purification par LC-MS préparative, on obtient 4,2 mg de produit attendu
EIMS ([M+H]+) : 462
TR= 3,52 min

### Exemple 31 : Préparation du trifluoroacétate de (R)-5-(1-hydroxy-éthyl)-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-Thr(OH), 0,25 mmole de 4-quinolinecarboxaldéhyde, et 0,125 mmole de 4-(trifluorométhanesulfonyl)aniline, de la même façon que l'exemple 1. Après purification par LC-MS préparative, on obtient 3,4 mg de produit attendu
EIMS ([M+H]+) : 494
TR= 3,43 min

### Exemple 32 : Préparation du trifluoroacétate de, 4-quinolin-4-ylméthyl-6-(4-trifluorométhylsulfanylphényl)-4,6-diaza-spiro[2.4]heptane-5,7-dione

Le composé est préparé à partir de 0,05 mmole de résine, 0,15 mmole de Fmoc-ACPC-(OH), 0,25 mmole de 4-quinolinecarboxaldéhyde, et 0,125 mmole de 4-(trifluorométhanethio) aniline, de la même façon que l'exemple 1. Après purification par LC-MS préparative, on obtient 7,5 mg de produit attendu
EIMS ([M+H]+) : 444
TR= 3,68 min

### Exemple 33 : Préparation du trifluoroacétate de 4-quinolin-4-ylméthyl-6-(4-trifluorométhanesulfonylphényl)-4,6-diaza-spiro[2.4]heptane-5,7-dione, trifluoroacétate

Le composé est préparé à partir de 0,05 mmole de résine, 0,15 mmole de Fmoc-ACPC-(OH), 0,25 mmole de 4-quinolinecarboxaldéhyde, et 0,125 mmole de 4-(trifluorométhanesulfonyl)aniline, de la même façon que l'exemple 1. Après purification par LC-MS préparative, on obtient 3,8 mg de produit attendu
EIMS ([M+H]+) : 476
TR= 3,60 min

### Exemple 34 : Préparation du trifluoroacétate de 4-pyridin-4-ylméthyl-6-(4-trifluorométhylsulfanylphényl)-4,6-diaza-spiro[2.4]heptane-5,7-dione

Le composé est préparé à partir de 0,05 mmole de résine, 0,15 mmole de Fmoc-ACPC-(OH), 0,25 mmole de 4-pyridinecarboxaldéhyde, et 0,125 mmole de 4-(trifluorométhanethio) aniline, de la même façon que l'exemple 1. Après purification par LC-MS préparative, on obtient 1,7 mg de produit attendu
EIMS ([M+H]+) : 394
TR= 3,43 min

### Exemple 35 : Préparation du trifluoroacétate de 4-pyridin-4-ylméthyl-6-(4-trifluorométhanesulfonyl-phényl)-4,6-diaza-spiro[2.4]heptane-5,7-dione

Le composé est préparé à partir de 0,05 mmole de résine, 0,15 mmole de Fmoc-ACPC-(OH), 0,25 mmole de 4-pyridinecarboxaldéhyde, et 0,125 mmole de 4-(trifluorométhanesulfonyl)aniline, de la même façon que l'exemple 1. Après purification par LC-MS préparative, on obtient 1,4 mg de produit attendu
EIMS ([M+H]+) : 426
TR= 3,35 min

### Exemple 36 : Préparation du trifluoroacétate de, (R)-5-Benzo[b]thiophen-3-ylméthyl-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-benzôthiénylAla(OH), 0,25 mmole de 4-quinolinecarboxaldéhyde, et 0,125mmole de 4-(trifluorométhanesulfonyl)aniline, de la même façon que l'exemple 1. Après purification par LC-MS préparative, on obtient 5 mg de produit attendu
EIMS ([M+H]+) : 596
TR= 4,12 min

### Exemple 37 : Préparation du trifluoroacétate de, (R)-5-Benzo[b]thioplien-3-ylméthyl-1-pyridin-4-ylméthyl-3-(4-trifluorométhylsulfanyl-phényl)-imidazolidine-2,4-dione

Le composé est préparé à partir de 0,05 mmole de résine, 0,15 mmole de N-Fmoc-D-benzothiénylAla(OH), 0,25 mmole de 4-pyridinecarboxaldéhyde, et 0,125 mmole de 4-(trifluorométhanethio)aniline, de la même façon que l'exemple 1. Après purification par LC-MS préparative, on obtient 1,4 mg de produit attendu
EIMS ([M+H]+) : 514
TR= 3,35 min

### Exemple 38 : Préparation du trifluoroacétate de (R)-5-Benzo[b]thiophen-3-ylméthyl-1-pyridin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-benzothienylAla(OH), 0,25 mmole de 4-pyridinecarboxaldéhyde, et 0,125 mmole de 4-(trifluorométhanesulfonyl)aniline, de la même façon que l'exemple 1. Après purification par LC-MS préparative, on obtient 8,5 mg de produit attendu
EIMS ([M+H]+) : 546
TR= 3,90 min

### Exemple 39 : Préparation du trifluoroacétate de (S)-5-Pyridin-2-ylméthyl-1-quinolin-4-ylméthyl-3-(4-trifluorométhylsulfanyl-phényl)-imidazolidine-2,4-dione

Le composé est préparé à partir de 0,05 mmole de résine, 0,15 mmole de N-Fmoc-D-2-pyridine-Ala(OH), 0,25 mmole de 4-quinolinecarboxaldéhyde, et 0,125 mmole de 4-(trifluorométhanethio)aniline, de la même façon que l'exemple 1. Après purification par LC-MS préparative, on obtient 7,5 mg de produit attendu
EIMS ([M+H]+): 509
TR= 3,47 min

### Exemple 40 : Préparation du trifluoroacétate de (S)-5-pyridin-2-ylméthyl-1-quinolin-4-ylméthyl-3-(4-trifluorométhanesulfonyl-phényl)-imidazolidine-2,4-dione

Le composé est préparé à partir de 0,05 mmole de résine, 0,15 mmole de N-Fmoc-D-2-pyridine-Ala(OH), 0,25 mmole de 4-quinolinecarboxaldéhyde, et 0,125 mmole de 4-(trifluorométhanesulfonyl)aniline, de la même façon que l'exemple 1. Après purification par LC-MS préparative, on obtient 1,6 mg de produit attendu
EIMS ([M+H]+) : 541
TR= 3,41 min

### Exemple 41 : Préparation du trifluoroacétate de (R)-1-(3-hydroxy-pyridin-4-ylméthyl)-5-méthyl-3-(4-trifluorométhylsulfanyl-phényl)-imidazolidine-2,4-dione

Le composé est préparé à partir de 0,05 mmole de résine, 0,15 mmole de N-Fmoc-D-Ala(OH), 0,25 mmole de 3-hydroxy-4-pyridinecarboxaldéhyde, et 0,125 mmole de 4-(trifluorométhanethio)aniline, de la même façon que l'exemple 1. Après purification par LC-MS préparative, on obtient 8,3 mg de produit attendu
EIMS ([M+H]+) : 398
TR= 4,26 min

### Exemple 42 : Préparation du trifluoroacétate de 5,5-diméthyl-1-quinolin-4-ylméthyl-3-(4-trifluorométhoxy-phényl)-imidazolidine-2,4-dione

Le composé est préparé à partir de 0,05 mmole de résine, 0,15 mmole de Fmoc-AIB-(OH), 0,25 mmole de 4-quinolinecarboxaldéhyde, et 0,125 mmole de 4-(trifluorométhoxy) aniline, de la même façon que l'exemple 1. Après purification par LC-MS préparative, on obtient 5,5 mg de produit attendu
EIMS ([M+H]+) : 430
TR= 4,33

### Exemple 43 : Préparation du trifluoroacétate de 5,5-diméthyl-1-quinolin-4-ylméthyl-3-(4-trifluorométhylsulfanyl-phényl)-imidazolidine-2,4-dione

Le composé est préparé à partir de 0,05 mmole de résine, 0,15 mmole de Fmoc-AIB-(OH), 0,25 mmole de 4-quinolinecarboxaldéhyde, et 0,125 mmole de 4-(trifluorométhanethio) aniline, de la même façon que l'exemple 1. Après purification par LC-MS préparative, on obtient 9,4 mg de produit attendu
EIMS ([M+H]+) : 446
TR= 4,58 min

### Exemple 44 : Préparation du trifluoroacétate de 5,5-diméthyl-1-(3-méthyl-pyridin-4-ylméthyl)-3-(4-trifluorométhoxy-phényl)-imidazolidine-2,4-dione

Le composé est préparé à partir de 0,05 mmole de résine, 0,15 mmole de Fmoc-AIB-(OH), 0,25 mmole de 3-méthyl-4-pyridinecarboxaldéhyde, et 0,125 mmole de 4-(trifluorométhoxy)aniline, de la même façon que l'exemple 1. Après purification par LC-MS préparative, on obtient 5,5 mg de produit attendu
EIMS ([M+H]+) : 394
TR= 4,06 min

### Exemple 45 : Préparation du trifluoroacétate de 5,5-diméthyl-1-(3-méthyl-pyridin-4-ylméthyl)-3-(4-trifluorométhylsulfanyl-phényl)-imidazolidine-2,4-dione

Le composé est préparé à partir de 0,05 mmole de résine, 0,15 mmole de Fmoc-AIB-(OH), 0,25 mmole de 3-méthyl-4-pyridinecarboxaldéhyde, et 0,125 mmole de 4-(trifluorométhanethio)aniline, de la même façon que l'exemple 1. Après purification par LC-MS préparative, on obtient 13,1 mg de produit attendu
EIMS ([M+H]+) : 410
TR= 4,30 min

### Exemple 46 : Préparation du trifluoroacétate de 5,5-diméthyl-1-(3-méthyl-pyridin-4-ylméthyl)-3-(4-trifluorométhanesulfonyl-phényl)-imidazolidine-2,4-dione

Le composé est préparé à partir de 0,05 mmole de résine, 0,15 mmole de Fmoc-AIB-(OH), 0,25 mmole de 3-méthyl-4-pyridinecarboxaldéhyde, et 0,125 mmole de 4-(trifluorométhanesulfonyl)aniline, de la même façon que l'exemple 1. Après purification par LC-MS préparative, on obtient 9,6 mg de produit attendu
EIMS ([M+H]+) : 442
TR= 4,18 min

### Exemple 47 : Préparation du trifluoroacétate de 1-(3-hydroxy-pyridin-4-ylméthyl)-5,5-diméthyl-3-(4-trifluorométhoxy-phényl)-imidazolidine-2,4-dione

Le composé est préparé à partir de 0,05 mmole de résine, 0,15 mmole de Fmac-AIB-(OH), 0,25 mmole de 3-hydroxy-4-pyridinecarboxaldéhyde, et 0,125 mmole de 4-(trifluorométhoxy)aniline, de la même façon que l'exemple 1. Après purification par LC-MS préparative, on obtient 6,9 mg de produit attendu
EIMS ([M+H]+) : 394
TR= 4,15 min

### Exemple 48 : Préparation du trifluoroacétate de 1-(3-hydroxy-pyridin-4-ylméthyl)-5,5-diméthyl-3-(4-trifluorométhylsulfanyl-phényl)-imidazolidine-2,4-dione

Le composé est préparé à partir de 0,05 mmole de résine, 0,15 mmole de Fmoc-AIB-(OH), 0,25 mmole de 3-hydroxy-4-pyridinecarboxaldéhyde, et 0,125 mmole de 4-(trifluorométhanethio)aniline, de la même façon que l'exemple 1. Après purification par LC-MS préparative, on obtient 9,7 mg de produit attendu
EIMS ([M+H]+) : 412
TR= 4,39 min

### Exemple 49 : Préparation du trifluoroacétate de 1-(3-hydroxy-pyridin-4-ylméthyl)-5,5-diméthyl-3-(4-trifluorométhanesulfonyl-phényl)-imidazolidine-2,4-dione

Le composé est préparé à partir de 0,05 mmole de résine, 0,15 mmole de Fmoc-AIB-(OH), 0,25 mmole de 3-hydroxy-4-pyridinecarboxaldéhyde, et 0,125 mmole de 4-(trifluorométhanesulfonyl)aniline, de la même façon que l'exemple 1. Après purification par LC-MS préparative, on obtient 15,2 mg de produit attendu
EIMS ([M+H]+) : 444
TR= 4,30 min

### Exemple 50 : Préparation du trifluoroacétate de 4-quinolin-4-ylméthyl-6-(4-trifluorométhoxy-phényl)-4,6-diaza-spiro[2.4] heptane-5,7-dione

Le composé est préparé à partir de 0,05 mmole de résine, 0,15 mmole Fmoc-ACPC-(OH), 0,25 mmole de 4-quinolinecarboxaldéhyde, et 0,125 mmole de 4-(trifluorométhoxy) aniline, de la même façon que l'exemple 1. Après purification par LC-MS préparative, on obtient 4,1 mg de produit attendu
EIMS ([M+H]+) : 428
TR= 4,24 min

### Exemple 51 : Préparation du trifluoroacétate de 4-(3-méthyl-pyridin-4-ylméthyl)-6-(4-trifluorométhoxy-phényl)-4,6-diaza-spiro[2.4]heptane-5,7-dione

Le composé est préparé à partir de 0,05 mmole de résine, 0,15 mmole de Fmoc-ACPC-(OH), 0,25 mmole de 4-pyridinecarboxaldéhyde, et 0,125 mmole de 4-(trifluorométhoxy) aniline, de la même façon que l'exemple 1. Après purification par LC-MS préparative, on obtient 3 mg de produit attendu.
EIMS ([M+H]+) : 392
TR= 3,95 min

### Exemple 52 : Préparation du trifluoroacétate de 4-(3-méthyl-pyridin-4-ylméthyl)-6-(4-trifluorométhylsulfanylphényl)-4,6-diaza-spiro[2.4]heptane-5,7-dione

Le composé est préparé à partir de 0,05 mmole de résine, 0,15 mmole de Fmoc-ACPC-(OH), 0,25 mmole de 3-méthyl-4-pyridinecarboxaldéhyde, et 0,125 mmole de 4-(trifluorométhanethio)aniline, de la même façon que l'exemple 1. Après purification par LC-MS préparative, on obtient 5 mg de produit attendu.
EIMS ([M+H]+) : 408
TR= 4,24 min

### Exemple 53 : Préparation du trifluoroacétate de 4-(3-méthyl-pyridin-4-ylméthyl)-6-(4-trifluorométhanesulfonyl-phényl)-4,6-diaza-spiro[2.4]heptane-5,7-dione

Le composé est préparé à partir de 0,05 mmole de résine, 0,15 mmole de Fmoc-ACPC-(OH), 0,25 mmole de 3-méthyl-4-pyridinecarboxaldéhyde, et 0,125 mmole de 4-(trifluorométhanesulfonyl)aniline, de la même façon que l'exemple 1. Après purification par LC-MS préparative , on obtient 1,9 mg de produit attendu.
EIMS ([M+H]+) : 440
TR= 4,11 min

### Exemple 54 : Préparation du trifluoroacétate de 4-(3-hydroxy-pyridin-4-ylméthyl)-6-(4-trifluorométhoxy-phényl)-4,6-diaza-spiro[2.4]heptane-5,7-dione

Le composé est préparé à partir de 0, 05 mmole de résine, 0,15 mmole de Fmoc-ACPC-(OH), 0,25 mmole de 3-hydroxy-4-pyridinecarboxaldéhyde, et 0,125 mmole de 4-(trifluorométhoxy)aniline, de la même façon que l'exemple 1. Après purification par LC-MS préparative, on obtient 5,5 mg de produit attendu.
EIMS ([M+H]+) : 394
TR= 4,04 min

### Exemple 55 : Préparation du trifluoroacétate de 4-(3-Hydroxy-pyridin-4-ylméthyl)-6-(4-trifluorométhylsulfanylphényl)-4,6-diaza-spiro[2.4]heptane-5,7-dione

Le composé est préparé à partir de 0,05 mmole de résine, 0,15 mmole de Fmoc-ACPC-(OH), 0,25 mmole de 3-hydroxy-4-pyridinecarboxaldéhyde, et 0,125 mmole de 4-(trifluorométhanethio)aniline, de la même façon que l'exemple 1. Après purification par LC-MS préparative, on obtient 0,7 mg de produit attendu.
EIMS ([M+H]+) : 410
TR= 4,34 min

### Exemple 56 : Préparation du trifluoroacétate de 4-(3-hydroxy-pyridin-4-ylméthyl)-6-(4-trifluorométhanesulfonyl-phényl)-4,6-diaza-spiro[2.4]heptane-5,7-dione

Le composé est préparé à partir de 0,05 mmole de résine, 0,15 mmole de Fmoc-ACPC-(OH), 0,25 mmole de 3-hydroxy-4-pyridinecarboxaldéhyde, et 0,125 mmole de 4-(trifluorométhanesulfonyl)aniline, de la même façon que l'exemple 1. Après purification par LC-MS préparative, on obtient 2,5 mg de produit attendu
EIMS ([M+H]+) : 442
TR= 4,17 min

### Exemple 57 : Préparation de la 5-méthyl-1-quinolin-4-ylméthyl-3-(4-trifluorométhanesulfonyl-phényl)-dihydro-pyrimidine-2,4-dione

588 mg (1 mmol) de résine Wang polystyrène (1,7 mmol/g), sont lavés par 2X5ml de DMF, 1 X 5 ml de DCM puis traités par une solution de 0,49 g d'acide N-Fmoc-3-amino-2-(R,S)-méthyl-propionique (1,5 mmole), 0,24 g de pyridine (3 mmoles) dans 5 ml de DMF immédiatement suivi de 0,31 g de chlorure de l'acide 2,6-dichlorobenzoique (addition goutte à goutte pour contrôler l'exothermie). Le mélange réactionnel est laissé une nuit sous agitation à température ambiante. Le mélange est filtré puis la résine lavée avec 1 X 5 ml de DMF, 1 X 5 ml de DCM et 2 X 5 ml de DMF et ensuite traitée par 5 ml de solution à 10 % de pipéridine dans le DMF. La résine est ensuite lavée avec 2 x 5 ml de DMF, 1 x 5 ml de DCM, 1 x 5 ml de DMF, 1 x 5 ml de DMF, 4 x 5 ml de DCM, 4 x 5 ml de MeOH et séchée sous vide. 0,94 g de 4-quinolinecarboxaldéhyde (6 moles) dans 16 ml de mélange 50/50 de THF/TMOF est additionné à la résine qui est maintenue sous agitation une nuit. La résine est ensuite lavée 3 fois par 5 ml du même mélange THF/TMOF puis traitée par 12 ml de solution 1M de cyanobo-rohydrure de sodium dans le THF (12 mmoles) en présence de 1,2 ml de MeOH et 0,12 ml d'acide acétique. Après une nuit d'agitation à température ambiante, la résine est lavée avec 1 X 5 ml de THF, 4 X 5 ml de solution à 30 % d'acide acétique dans le DMF, 1 X 5 ml de MeOH, 1 X 5 ml de THF, 1 X 5 ml de DMF, 1 X 5 ml de THF, 1 X 5 ml de MeOH et séché sous vide.

Parallèlement, 0,121 g de triphosgène (0,41 mmole) dans 1 ml de DCM est additionné goutte à goutte à une solution de 0,281 g de 4-(trifluorométhane-sulfonyl)aniline (1,25 mmole) et 0,1 g de pyridine(1,25 mmole) dans 2 ml de DCM. Après 15 minutes d'agitation à température ambiante, la même quantité de pyridine est additionnée suivi par la résine précédemment préparée. Le mélange est agité une nuit puis filtré. La résine est lavée avec 1 X 5 ml de MeOH, 1 X 5 ml de THF, 1 X 5 ml de MeOH, 1 X 5 ml de DMF, 1 X 5 ml de THF, 1 X 5 ml de MeOH et 3 X 5 ml de THF et séchée sous vide. La résine est ensuite traitée par 154 mg de DBU(1 mmole) dans 5 ml de DCM et laissée une nuit sous agitation. Finalement la dihydrouracile est obtenue par traitement de la resine avec 5 ml de solution à 2 % d'acide acétique dans le THF. Après purification par HPLC préparative, 90 mg de produit attendu est isolé.
EIMS ([M+H]+) : 477
TR= 1,83 min (gradient ACN/H2O 20-100 % en 5 min)

### Exemple 58 : (S)-5-méthyl-1-quinolin-4-ylméthyl-3-(4-trifluorométhanesulfanyl-phényl)-imidazolidine-2,4-dione

Cet exemple décrit une nouvelle préparation de l'exemple 5 ci-dessus.
Un mélange de 0,71 g d'ester éthylique de l'acide (S)-2-[(quinolin-4-ylméthyl)-aminol-propanoique et de 1,42 g de 4-(trifluorométhanesulfanyl-phényl)isocyanate dans 15 ml de THF est agité 15 heures à température ambiante sous atmosphère d'argon. Après évaporation du solvant sous pression réduite, 20 ml de dichlorométhane sont ajoutés. Le précipité est filtré. Le filtrat est concentré sous pression réduite et le résidu est purifié par flash-chromatographie (SiO2, CH2C12 puis CH2Cl2/MeOH, 95/5 en volume comme éluant, Ar). 0,69 g de (S)-5-méthyl-1-quinolin-4-ylméthyl-3-(4-trifluorométhanesulfanyl-phényl)-imidazolidine-2,4-dione sont isolés sous forme d'une poudre blanche.
[a]D= -33.1° +/- 0,8° (MeOH)
Masse: EI m/z = 431 M+. pic de base
m/z = 143 [C10H9N]+.

Spectre de R.M.N. 1H (400 MHz, (CD3)2SO d6, δ en ppm) : 1,42 (d, J = 5, 5 Hz : 3H) ; 4.35 (q, J = 5, 5 Hz: 1H) ; 5,08 (d, J = 17 Hz : 1H) ; 5,25 (d, J = 17 Hz : 1H) ; 7,65 (d, J = 5 Ha : 1H) ; de 7,65 à 7,75 (mt : 1H) ; 7,70 (d, J = 8,5 Hz : 2H) ; 7,83 (t large, J = 8 Hz : 1H) ; 7,89 (d, J = 8,5 Hz : 2H) ; 8,10 (d large, J = 8,5 Ha : 1H) ; 8,25 (d large, J = 8,5 Hz : 1H) ; 8,90 (d, J = 5Hz : 1H).

### Ester éthylique de l'acide (S)-2-[(quinolin-4-ylméthyl)-amino]-propanoique (P-31397-073-1)

Un mélange de 2 g d'ester éthylique de L-alanine sous forme de chlorhydrate et de 1,83 ml of triéthylamine dans 30 ml de dichlorométhane est agité à température ambiante pendant 10 minutes. Puis 2,05 g de quinoline-4-carbaldéhyde sont ajoutés. Le milieu réactionnel est agité à température ambiante pendant 15 heures puis concentré sous pression réduite. 35 ml d'éthanol sont ensuite ajoutés ; la solution est refroidie à 0°C puis 0,49 g de borohydrure de sodium sont ajoutés par portion. L'agitation est maintenue pendant 15 heures à température ambiante. Le précipité formé est filtré ; le filtrat est concentré sous pression réduite. Le résidu est purifié par flash-chromatographie sur colonne (SiO2, CH2Cl2/MeOH, 95/5 en volumes comme éluant, Ar). 0,71 g d'ester éthylique de l'acide (S)-2-[(quinolin-4-ylméthyl)-amino]-propanoique sont obtenus sous forme d'une huile rose.
Masse : EI m/z = 258 M+.
m/z = 185 [M - COOCH2CH3]+ pic de base
m/z = 142 [C10H8N]+

Spectre de R.M.N. 1H (300 MHz, (CD3)2SO d6, δ en ppm) : 1, 22 (t, J = 7 Hz : 3H) ; 1, 28 (d, J = 7 Hz : 3H) ; 2, 72 (mf : 1H) ; 3,42 (mt : 1H) ; de 4,00 à 4,20 (mt : 1H) ; 4,13 (q, J = 7 Hz : 2H) ; 4,27 (d large, J =
16 Hz : 1H) ; 7,55 (d large, J = 5 Hz : 1H) ; 7,64 (ddd, J = 8,5 - 7,5 et 1 Hz : 1H) ; 7,77 (ddd, J = 8,5 - 7,5 et 1 Hz : 1H) ; 8,04 (d large, J = 8,5 Hz : 1H) ; 8,22 (d large, J = 8,5 Hz : 1H) ; 8,86 (d, J = 5 Hz : 1H).

### Exemple 59 : 5,5-diméthyl-1-quinolin-4-ylméthyl-3-(4-trifluorométhoxy-phényl)-,imidazolidine-2,4-dione

Cet exemple décrit une nouvelle préparation de l'exemple 42 ci-dessus.

Le produit est préparé suivant le mode opératoire décrit dans l'exemple 58 avec 600 mg d'ester méhylique de l'acide 2-méthyl-2-[(quinolin-4-ylméthyl)-amino]-propanoique au lieu de l'ester éthylique de l'acide (S)-2-[(quinolin-4-ylmêthyl)-amino]-propanoïque utilisé à l'exemple 58 et de 1,114 g de 4-(trifluorométhoxy-phényl)isocyanate au lieu du 4-(trifluorométhanesulfanyl-phényl)isocyanate utilisé à l'exemple 58. Après purification par flash-chromatographie sur colonnne (SiO2, CH2Cl2 comme éluant, Ar) puis une seconde purification par flash-chromatographie sur colonne (SiO2, cyclohexane/ AcOEt 60/ 40 en volumes comme éluant, Ar), 710 mg du produit recherché sont obtenus.
Masse : EI m/z = 429 M+. pic de base
m/z = 414 [M - CH3] +
m/z = 359 [M - C4H60] +.

Spectre de R.M.N. 1H (300 MHz, (CD3)2SO d6, δ en ppm) : 1,46 (s : 6H) ; 5,16 (s : 2H) ; 7,55 (d large, J = 8,5 Hz : 2H) ; 7,65 (d, J = 5 Hz : 1H) ; 7,69 (d, J = 8,5 Hz : 2H) ; 7,70 (mt : 1H) ; 7,83 (ddd, J = 8 - 7,5 et 1,5 Ha : 1H) ; 8,09 (d large, J = 8,5 Hz : 1H) ; 8,27 (d large, J = 8,5 Hz : 1H) ; 8,88 (d, J = 5 Hz : 1H).

### Préparation de l'ester méthylique de l'acide -2-((quinolin-4-ylméthyl)-amino)-propanoïque

Un mélange de 1,5 g de chlorhydrate de l'ester méthylique de l'acide α-aminoisobutyrique et de 1,4 ml de triéthylamine dans 30 ml de dichlorométhane est agité à 0°C pendant 20 minutes. Ensuite 1 g de sulfate de magnésium et 1,5 g de quinoline-4-carbaldéhyde sont ajoutés. L'agitation est maintenue 15 heures à température ambiante puis le mélange est concentré sous pression réduite. Le résidu est repris par 35 ml de méthanol, la solution obtenue refroidie à 0°C. 0,4 g de borohydrure de sodium sont ajoutés par portion et l'agitation est maintenue à température ambiante pendant 15 heures. Le précipité formé est filtré, le filtrat est concentré sous pression réduite. Le résidu obtenu est purifié par recristallisation dans de l'éther diisopropylique. On obtient 600 mg du produit attendu sous forme d'une huile rose.
Masse : DCI m/z = 259 [M+H]+
m/z = 199 [M+H- HCOOCH3]+

Spectre de R.M.N. 1H (300 MHz, (CD3)2SO d6, δ en ppm) : 1,36 (s : 6H) ; 2,68 (t large, J = 7 Hz : 1H) ; 3,69 (s : 3H) ; 4,11 (d, J = 7 Ha : 2H) ; 7,60 (d large, J = 5 Ha : 1H) ; 7,63 (ddd, J = 9 - 8,5 et 1 Hz : 1H) ; 7,76 (ddd, J = 9 - 8,5 et 1 Ha : 1H) ; 8,03 (d large, J = 8,5 Hz : 1H) ; 8,20 (d large, J = 8,5 Hz : 1H) ; 8,85 (d, J = 5 Ha : 1H).

### Exemple 60 : 5,5-diméthyl-1-(3-chloro-6-méthoxy-quinolin-4-ylméthyl)-3-(4-trifluoromëthaxy-phényl)-imidaaalidine-2,4-dione

Le produit est préparé en suivant le mode opératoire décrit à l'exemple 59 à partir de 180 mg de l'ester méthylique de l'acide 2-méthyl-2-[(3-chloro-6-méthoxy-quinolin-4-ylméthyl)-amino]-p-ropanoique au lieu de l'ester méthylique de l'acide 2-méthyl-2-[(quinolin-4-ylméthyl)-aminol-propanoique utilisé à l'exemple 59 et de 267 mg de 4-(trifluorométhoxy-phényl)isocyanate. Après purification par flash-chromatographie sur colonne (SiO2, cyclohexane/ AcOEt 80/ 20 en volumes comme éluant, Ar), on obtient 137 mg du produit attendu.
Masse : EI m/z = 493 M+.massif isotopique du pic
monochloré m/z = 458 [M - Cl]+ pic de base

Spectre de R.M.N. 1H (300 MHz, (CD3)2SO d6, δ en ppm) : 1,27 (s : 6H) ; 3,89 (s : 3H) ; 5,27 (s : 2H) ; 7,48 (dd, J = 9 et 3 Hz : 1H) ; 7,56 (d large, J = 8, 5 Hz : 2H) ; 7,68 (dt, J = 8,5 et 2 Ha : 2H) ; 7,79 (d, J = 3 Hz : 1H) ; 8,01 (d, J = 9 Hz : 1H) ; 8,80 (s : 1H).

### Préparation de l'ester méthylique de l'acide 2-méthyl-2-[(3-chloro-6-méthoxy-quinolin-4-ylméthyl)-amino]-propanoique (P-31397-099-1)

Le produit est préparé en suivant le mode opératoire décrit à l'exemple 59 à partir de 1 g du chlorhydrate de l'ester méthylique de l'acide α-aminoisobutyrique, 1,25 g de (3-chloro-6-méthoxy-quinolin)-4-carbaldéhyde au lieu du quinolin-4-carbaldehyde utilisé à l'exemple 59, 0,66 g de triéthylamine et 250 mg de borohydrure de sodium. Après purification par flash-chromatographie (SiO2, cyclohexane/ AcOEt 70/30 volumes comme éluant, Ar), on obtient 180 mg du produit attendu.
Masse : EI m/z = 322 M+. massif isotopique du pic monochloré m/z = 263[M-COOCH3]+ pic de base, massif isotopique du pic monochloré m/z = 206 [M-C5H1002N]+ massif isotopique du pic monochloré

Spectre de R.M.N. 1H (300 MHz, (CD3)2SO d6, δ en ppm) : 1,39 (s : 6H) ; 2, 47 (t large, J = 7,5 Hz : 1H) ; 3,74 (s : 3H) ; 3,98 (s : 3H) ; 4,04 (d, J = 7,5 Hz : 2H) ; 7,46 (dd, J = 9 et 3 Hz : 1H) ; 7,66 (d, J = 3 Hz : 1H) ; 7,97 (d, J = 9 Hz : 1H) ; 8,71 (s : 1H).

### Préparation de la (3-chloro-6-mêthoxy-quinolin)-4-carbaldéhyde (P-31397-097-1)

Une solution de 2 g de 4-bromo-3-chloro-6-méthoxy-quinoléine dans 50 ml de THF est refroidie à -78°C. 6,9 ml d'une solution 1,6 M de nBuLi dans le dioxane sont ajoutés. La solution est agitée pendant 2 heures à cette température puis 1, 7 ml de DMF sont ajoutés. Le mélange est agité à -60°C pendant 2 heures 30 minutes, puis on laisse le milieu réactionnel revenir à température ambiante. 200 ml d'eau sont ensuite ajoutés. La phase organique est extraite par 200 ml d'acétate d'éthyle, lavée par 5 x 200 ml d'eau, séchée sur du sulfate de magnésium et concentrée sous pression réduite. Le résidu obtenu est purifié par flash-chromatographie sur colonne (SiO2, cyclohexane/AcOEt 80/20 en volumes comme éluant, Ar). On obtient 1,2 g du produit attendu sous forme d'une poudre jaune.
Masse : EI m/z = 221 M+. pic de base, massif isotopique
du monochloré
m/z = 193 [M-CO]+.massif isotopique du pic monochloré
m/z = 150[M-C3H302]+ massif isotopique du pic monochloré

Spectre de R.M.N. 1H (300 MHz, (CD3)2SO d6, δ en ppm) : 3,96 (s : 3H) ; 7,56 (dd, J = 9 et 3 Ha : 1H) ; 8, 07 (d, J = 9 Hz : 1H) ; 8,22 (d, J = 3 Hz : 1H) ; 8,93 (s : 1H) ; 10,77 (s : 1H).

La préparation de la 4-bromo-3-chloro-6-méthoxy-quinoléine est décrite dans le brevet français FR 2 816 618 à l'exemple 1.

### Exemple 61 : 5,5-diméthyl-1-yridin-4-ylméthyl-3-(4-trifluorométhanesulfanyl-phényl)-imidaolidine,2,4-dione

A une solution de 0,726 g d'ester méthylique de l'acide -2-méthyl-2-[(pyridin-4-ylméthyl)-amino]-propanoique dans 10 ml de tétrahydrofurane est ajouté 0,764 g de 4-(trifluorométhanesulfanyl-phényl)isocyanate. Le milieu réactionnel est agité sous atmosphère d'argon pendant environ 3 jours à une température voisine de 20°C. Le mélange réactionnel est repris par de l'acétate d'éthyle, lavé successivement par de l'eau puis par une solution saturée de chlorure de sodium. La phase organique est séchée sur sulfate de magnésium, puis concentrée sous pression réduite. Le résidu ainsi obtenu est purifié par flash chromatographie sur cartouche AIT de référence FC-50SI chargée de 50 g de silice conditionnée et éluée au dichlorométhane à un débit de 10 ml par minute. Les fractions comprises entre 100 et 280 ml sont concentrées sous pression réduite et le résidu obtenu a été repris à l'éther éthylique, l'insoluble obtenu a été filtré. On obtient ainsi 700 mg de 5,5-diméthyl-1-pyridin-4-ylméthyl-3-(4-trifluorométhanesulfanyl-phényl)-imidazolidine-2,4-dione sous forme de poudre blanche dont les caractéristiques sont les suivantes :

Spectre de R.M.N. 1H (300 MHz, (CD3)2SO d6, δ en ppm) : 1,43 (s : 6H) ; 4,66 (s : 2H) ; 7,44 (d large, J = 6 Hz : 2H) ; 7,69 (d t, J = 8,5 et 2,5 Hz : 2H) ; 7,87 (d large, J = 8,5 Ha : 2H) ; 8,55 (dd, J = 6 et 1,5 Hz : 2H).
Masse IE m/z=395 M+. pic de base
m/z=380 (M-CH3)+
m/z=219 C8H4NOSF3+.
m/z=92 C6H6N+

### Préparation de l'ester méthylique de l'acide-2-méthyl-2-[(pyridin-4-ylméthyl)-aminol-propanoique (P-31402-151-1)

A une solution de 0,945 g de chlorhydrate de l'ester méthylique de l'acide α-aminoisobutyrique dans 28 ml de dichloroéthane on charge successivement 1,04 ml de triéthylamine puis 0,659 g de pyridine-4-carbaldéhyde. Le mélange réactionnel est agité pour la nuit à une température voisine de 20°C. Le mélange est purifié par filtration sur silice greffée aminopropyl Lichroprep Merck. Le filtrat est concentré sous pression réduite et le résidu ainsi obtenu est repris par 25 ml de méthanol, 0,372 g de borohydrure de sodium sont ajoutés. Le mélange réactionnel est agité pendant 48 heures à une température voisine de 20°C, puis versé dans un mélange de solution normale d'hydroxyde de sodium/glace. Le mélange obtenu est extrait trois fois par de l'acétate d'éthyle. La phase organique est séchée sur sulfate de magnésium, puis concentrée sous pression réduite. On obtient ainsi 0,726 g d'ester méthylique de l'acide-2-méthyl-2-[(pyridin-4-ylméthyl)-amino]-propanoique sous forme d'huile dont les caractéristiques sont les suivante

Spectre de R.M.N. 1H (300 MHz, (CD3)2SO d6, δ en ppm) : 1,27 (s : 6H) ; 2,69 (t large, J = 5 Hz : 1H) ; 3,64 (s : 3H) ; 3,65 (d, J = 5 Hz: 2H) ; 7,35 (d large, J = 6 Ha : 2H) ; 8,47 (dd, J = 6 et 1,5 Hz : 2H).
Masse IC m/z=209 MH+ pic de base
m/z=149 (M-C2H4O2)+

### Exemple 62 : 1-Pyridin-4-ylméthyl-3-(4-trifluorométhane-sulfanyl-phényl)-imidazolidine-2,4-dione

A une solution de 0,300 g de 3-(4-trifluorométhyl-sulfanyl-phényl)-imidazolidine-2,4-dione dans 6 ml de diméthylformamide anhydre, sous atmosphère inerte d'argon à une température voisine de 20°C, est ajoutés 0,087 g d'hydrure de sodium, l'agitation est maintenue à cette température pendant 30 minutes, est ajouté successivement 0,152 ml de triéthylamine, 0,274 g de bromhydrate de 4-(bromométhyl) pyridine puis de l'eau glacée après 10 minutes. Le mélange réactionnel est déposé sur une cartouche de diamètre 37 mm garnie avec 50 g de silice greffée octadécyl Amicon 50 µm de réf. conditionnée successivement par le mélange (eau/acétonitrile) (5/95), (v/v) puis par le mélange (eau/acétonitrile) (95/5), (v/v). L'élution a été effectuée par un mélange (eau/acétonitrile) (95/5) (v/v) en 20 minutes, suivi d'un gradient linéaire de 5 à 95 % d'acétonitrile en 60 minutes, à un débit de 10 ml/minute. Les fractions comprises entre 580 et 630 ml sont concentrées sous pression réduite. On obtient ainsi 0,220 g de 1-pyridin-4-ylméthyl-3-(4-trifluorométhylsulfanyl-phényl)-imidazolidine-2,4-dione sous forme de poudre blanche dont les caractéristiques sont les suivantes :

Spectre de R.M.N. 1H (300 MHz, (CD3)2SO d6, δ en ppm) : 4,16 (s : 2H) ; 4,65 (s : 2H) ; 7,42 (d large, J = 6 Hz : 2H) ; 7,64 (d large, J = 8,5 Hz : 2H) ; 7,87 (d large, J = 8,5 Hz : 2H) ; 8,57 (d large, J = 6 Hz : 2H).
Masse IE m/z=367 M+. pic de base
m/z=219 C8H4NOSF3+.
m/z=92 C6H6N+

Le composé 3-(4-trifluorométhylsulfanyl-phényl)-imidazolidine-2,4-dione est décrit dans le brevet US4496575

### Exemple 63 : 5,5-diméthyl-1-pyridin-4-ylméthyl-3-(4-trifluorométhoxy-phényl)-imidazolidine-2,4-dione

A une solution de 0,150 g de 5,5-diméthyl-3-(4-trifluorométhoxy-phényl)-imidazolidine-2,4-dione dans 3 ml de diméthylformamide anhydre, sous atmosphère inerte d'argon à une température voisine de 20°C, est ajouté 0,042 g d'hydrure de sodium, l'agitation est maintenue à cette température pendant 30 minutes, est ajouté successivement 0,094 ml de triéthylamine, 0,132 g de bromhydratede 4-(bromométhyl) pyridine puis de l'eau glacée après 10 minutes. Le mélange réactionnel est déposé sur une cartouche de diamètre 27 mm garnie avec 30 g de silice greffée octadécyl Amicon 50 µm conditionnée successivement par le mélange (eau/acétonitrile) (5/95) (v/v) puis par le mélange (eau/acétonitrile) (95/5), (v/v). L'élution a été effectuée par un mélange (eau/acétonitrile) (95/5) (v/v) en 20 minutes, suivi d'un gradient linéaire de 5 à 95 % d'acétonitrile en 60 minutes, à un débit de 10 ml/minute. Les fractions comprises entre 300 et 450 ml sont concentrées sous pression réduite. On obtient ainsi 0,1 g de mélange qu'on purifie à nouveau sur une cartouche de diamètre 37 mm garnie avec 50 g de silice greffée octadécyl Amicon 50 µm de réf. conditionnée successivement par le mélange (eau/acétonitrile) (5/95), (v/v) puis par le mélange (eau/acétonitrile) (95/5), (v/v). L'élution a été effectuée par un mélange (eau/acétonitrile) (95/5) (v/v) en 20 minutes, suivi d'un gradient linéaire de 5 à 95 % d'acétonitrile en 60 minutes, à un débit de 10 ml/minute. Les fractions comprises entre 550 et 750 ml sont concentrées sous pression réduite. On obtient ainsi 0,060 g de 5,5-diméthyl-1-pyridin-4-ylméthyl-3-(4-trifluorométhoxy-phényl)-imidazolidine-2,4-dione dont les caractéristiques sont les suivante

Spectre de R.M.N. 1H (300 MHz, (CD3)2SO d6, δ en ppm) : 1,42 (s : 6H) ; 4,65 (s : 2H) ; 7,44 (d large, J = 6 Hz : 2H) ; 7,53 (d large, J = 8,5 Ha : 2H) ; 7,64 (dt, J = 8,5 et 2,5 Hz : 2H) ; 8,54 (d large, J = 6 Hz : 2H).
Masse IE m/z=379 M+. pic de base
m/z=364 (M-CH3)+
m/z=203 C8H4NO2F3+.
m/z=92 C6H6N+

### Préparation du 5,5-diméthyl-3-(4-trifluorométhoxy-phényl)-imidazolidine-2,4-dione

A une suspension de 8,7 g diphosgène et de 1 g de noir végétal dans 100 ml de toluène, à une température voisine de -20°C, est ajouté en 15 minutes, une solution de 7,08 g de 4-trifluorométhoxy-aniline dans 50 ml de toluène. Le mélange est agité jusqu'à une température voisine de 20°C, puis chauffé au reflux pendant 3 heures. Le mélange est refroidi à une température voisine de 20°C, puis filtré sur célite, 5 g de chlorhydrate de l'ester méthylique de l'acide α-aminoisobutyrique, 50 ml de toluène et 10 ml de triéthylamine sont ajoutés au filtrat. Le mélange ainsi obtenu est porté au reflux pendant 16 heures puis refroidi à une température voisine de 20°C. Le précipité est filtré et le filtrat est concentré sous pression réduite, le résidu obtenu est purifié par flash-chromatographie sur une colonne garnie de silice, conditionnée puis éluée par le mélange (cyclohexane/acétate d'éthyle), (50/50), (v/v). Les fractions contenant le produit attendu sont concentrées sous pression réduite et l'on obtient ainsi 3,4 g de 5,5-diméthyl-3-(4-trifluorométhoxy-phényl)-imidazolidine-2,4-dione dont les caractéristiques sont les suivantes :
Masse IE m/z=288 M+. pic de base
m/z=273 (M-CH3)+
m/z=203 C8H4NO2F3+.

Spectre de R.M.N. 1H (300 MHz, (CD3)2SO d6, δ en ppm) : 1,42 (s : 6H) ; 7,49 (d, J = 9 Hz : 2H) ; 7,55 (d, J = 9 Ha : 2H) ; 8,63 (mf : 1H)).

### Exemple 64 : Préparation de trifluoroacétate de 3-[4-(pentafluorothio)-phényl]-5,5-diméthyl-1-quinolin-4-ylméthyl-imidazolidine-2,4-dione.

Le composé est préparé à partir de 0,16 mmol de résine, 0,48 mmol de Fmoc-AIB-(OH), 1,12 mmol de 4-quinolinecarboxaldéhyde, et 0,4 mmol de 4-(pentafluorothio) aniline, de la même manière que dans l'exemple 1. Après purification par chromatographie LC-MS préparative, 5 mg du produit espéré est obtenu.
EIMS ([M+H]⁺) : 472

### Exemple 65 : Préparation de trifluoroacétate de 3-[4-(pentafluorothio)-phényl]-5,5-diméthyl-1-pyridin-4-ylméthyl-imidazolidine-2,4-dione

Le composé est préparé à partir de 0,16 mmol de résine, 0,48 mmol de Fmoc-AIB-(OH), 1,12 mmol de 4-pyridinecarboxaldéhyde, et 0,4 mmol de 4-(pentafluorothio) aniline, de la même manière que dans l'exemple 1. Après purification par chromatographie LC-MS préparative, 13,2 mg du produit espéré est obtenu.
EIMS ([M+H]⁺) : 422

### Exemple 66 : Préparation de trifluoroacétate de 3-[4-(pentafluorothio)-phényl]-1-quinolin-4-ylméthyl-imidazolidine-2,4-dione

Le composé est préparé à partir de 0,1 mmol de résine, 0,3 mmol de N-Fmoc-Gly-(OH), 0,5 mmol de 4-quinolinecarboxaldéhyde, et 0,25 mmol de 4-(pentafluorothio) aniline, de la même manière que danse l'exemple 1. Après purification par chromatographie LC-MS préparative, 18 mg du produit espéré est obtenu.
EIMS ([M+H]⁺) : 444

### Exemple 67 : Préparation de trifluoroacétate de 3-[4-(pentafluorothio)-phényl]-1-pyridin-4-ylméthyl-imidazolidine-2,4-dione

Le composé est préparé à partir de 0,1 mmol de résine, 0,3 mmol de N-Fmoc-Gly-(OH), 0,5 mmol de 4-quinolinecarboxaldéhyde, et 0,25 mmol de 4-(pentafluorothio) aniline, de la même manière que dans l'exemple 1. Après purification par chromatographie LC-MS préparative, 18 mg du produit espéré est obtenu.
EIMS ([M+H]⁺) : 394

### Exemple 68 : Préparation da trifluoroacétate de 3-[4-(pentafluorothio)-phényl]-1-pyridin-2-ylméthyl-imidazolidine-2,4-dione

Le composé est préparé à partir de 0,1 mmol de résine, 0,3 mmol de N-Fmoc-Gly-(OH), 0,5 mmol de 4-quinolinecarboxaldéhyde, et 0,25 mmol de 4-(pentafluorothio) aniline, de la même manière que dans l'exemple 1. Après purification par chromatographie LC-MS préparative, 9 mg du produit espéré est obtenu.
EIMS ([M+H]⁺) : 394

### Exemple 69 : Préparation de trifluoroacétate de 3-[4-(pentafluorothio)-phényl]-1-pyridin-3-ylméthyl-imidazolidine-2,4-dione

Le composé est préparé à partir de 0,2 mmol de résine, 0,6 mmol de N-Fmoc-Gly-(OH), 1 mmol de 4-quinolinecarboxaldéhyde, et 0,5 mmol de 4-(pentafluorothio) aniline, de la même manière que dans l'exemple 1. Après purification par chromatographie LC-MS préparative, 42 mg du produit espéré est obtenu.
EIMS ([M+H]⁺) : 394

Les 6 schémas réactionnels ci-après décrivent la préparation de produits de formule (I) selon la présente invention, notamment parmi les produits des exemples 70 à 178 qui suivent.

Le schéma 1 décrit la préparation de dérivés de l'hydantoïne avec des substituants amino aux deux positions du cycle pyridine (B2).

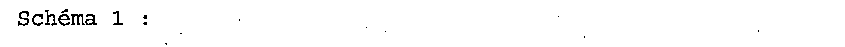

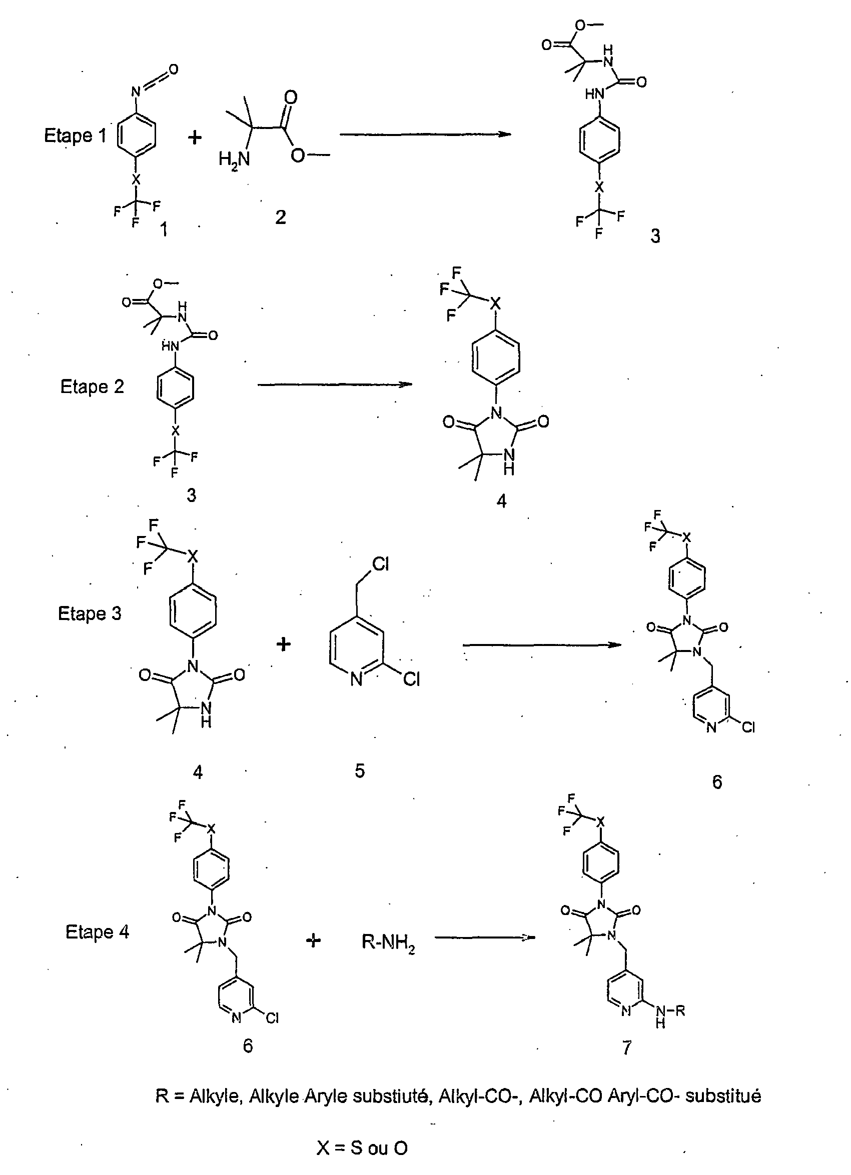

### Modes opératoires du schéma 1

### Etape 1 :

### (Synthèses décrites pour X = S, schéma analogue pour X = O).

Du 1-isocyanato-4-trifluorométhylsulfanyl-benzène (14,27 g, 65 mmol) est dissous dans 30 ml de CH₂Cl₂ sec et est refroidi jusqu'à 0°C. 7,5 g (65 mmol) de N-éthyl-morpholine est ajouté et ensuite 10 g (65 mmol) d'ester méthylique de l'acide 2-amino-2-méthyl-propionique. On laisse la réaction atteindre 25°C sur une période de temps de 6 h, on lave avec de l'eau et après élimination du solvant 21,8 g du produit ester méthylique de l'acide 2-méthyl-2-[3-(4-trifluorométhylsulfanyl-phényl)-uréido]-propionique 3 sont isolés.

### Etape 2 :

30 g d'ester méthylique de l'acide 2-méthyl-2-[3-(4-trifluorométhylsulfanyl-phényl)-uréido]-propioniqué-3 sont dissous dans un mélange de 225 ml de HCI 3N et de 230 ml de dioxane, chauffés à reflux pendant 6 h et après refroidissement à 4°C le produit 5,5-diméthyl-3-(4-trifluorométhylsulfanyl-phényl)-imidazolidine-2,4-dione est isolé sous forme de cristaux blancs (24, 14 g).

### Etape 3 :

De la 5,5-diméthyl-3-(4-trifluorométhylsulfanyl-phényl)-imidazolidine-2,4-dione 3 (1 g, 3,29 mmol) est dissoute dans 10 ml de DMF, 1,36 g de K₂CO₃ (3 Eq, 9,87 mmol) et 909 mg de 2-chloro-4-chlorométhyl-pyridine (3,95 mmol, 1,2 Eq) sont ajoutés et le mélange est chauffé à reflux pendant 20 heures. Le solvant est éliminé sous vide, le résidu est dissous dans du CH₂Cl₂ et traité trois fois avec du charbon actif. Après élimination du solvant le produit 1-(2-chloro-pyridin-4-ylméthyl)-5,5-diméthyl-3-(4-trifluorométhylsulfanyl-phényl)-imidazoli-dine-2,4-dione est obtenu (3,2 g ; 56 %).

Données analytiques :
MS (LC-MS) : 429,05 ; Temps de rétention : 2,49 min.
RMN : 1,4 : s, 6H ; 4,7 : s, 2H ; 7,45 : m, 1H ; 7,6 : s, 1H ; 7,7 : d, 2H ; 7,9 : d, 2H ; 8,4 : m, 1H.

### Préparation de 2-Chloro-4-chlorométhyl-pyridine

10 g de 2-chloro-4-méthyl-pyridine sont dissous dans 30 ml de CH₃CN et un mélange de AIBN (3 g) et de NCS (30 g) est ajouté. Le mélange résultant est chauffé à reflux pendant 4 h. Après élimination du solvant le produit brut est davantage purifié par distillation (Point d'ébullition : : 70°C, 20 mtorr).

### Etape 4 : procédure générale pour l'amination catalysé par le palladium du système cyclique pyridine dans la 1-(2-chloro-pyridin-4-ylméthyl)-5,5-diméthyl-3-(4-trifluorométhylsulfanyl-phényl)-imidazolidine-2,4-dione

100 mg (0,23 mmol) de 1-(2-chloro-pyridin-4-ylméthyl)-5,5-diméthyl-3-(4-trifluorométhylsulfanyl-phényl)-imidazolidine-2,4-dione, 20 mg de Pd(OAc)₂, 60 mg de XANTPHOS et 300 mg de Cs₂CO₃ sont transférés dans un tube de réaction avec un bouchon à vis équipé d'un joint en caoutchouc et une atmosphère d'argon est générée dans le tube. 1,5 équivalents (0,35 mmol) de l'amine ou de l'amide approprié sont dissous dans 10 ml de toluène, la solution est transférée dans le tube de réaction mentionné ci-dessus et le mélange résultant est chauffé à 95°C pendant 6 à 10 heures en fonction de la progression de la réaction contrôlée par LCMS.

Après filtration le solvant est éliminé sous vide et le produit brut est davantage purifié par chromatographie sur un système HPLC.

Le schéma 2 décrit la préparation de dérivés d'urée et de thiourée.

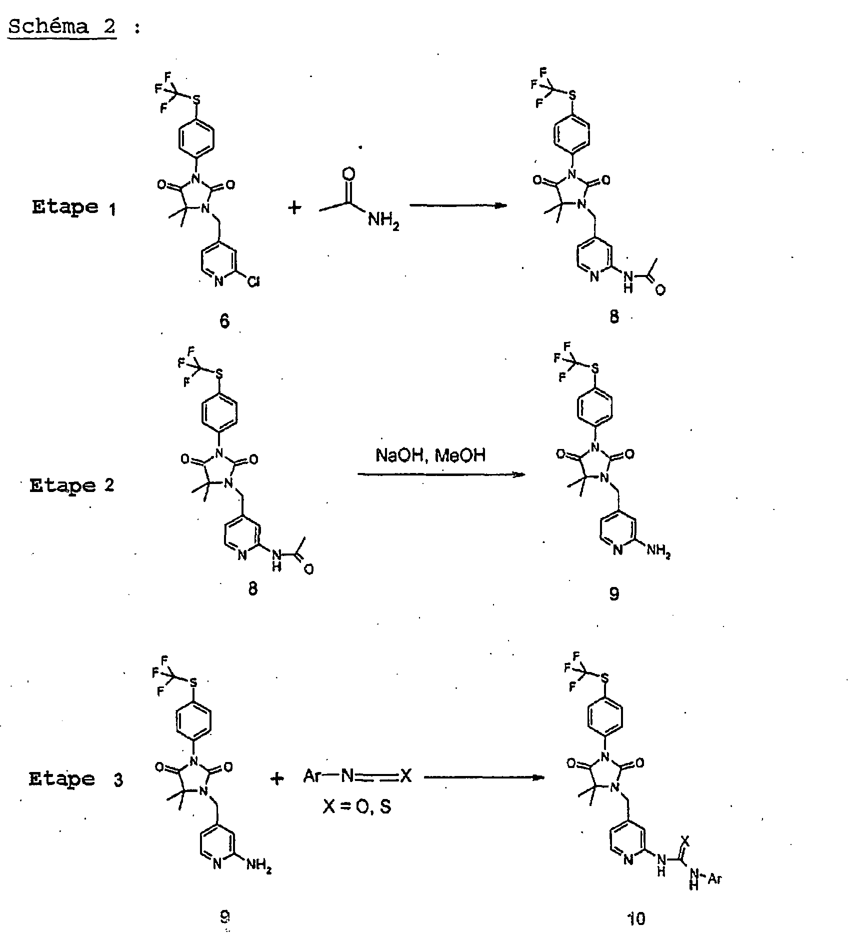

### Modes opératoires du schéma 2 :

### Etape 1 :

Cette étape est identique à l'étape 4 du schéma 1 avec de l'acétamide comme réactif dans la réaction catalytique.

Données analytiques pour le N-{4-[5,S-diméthyl-2,4-dioxo-3-(4-trifluorométhylsulfanyl-phényl)-imidazolidin-1-ylméthyl]-pyridin-2-yl}-acétamide :
MS (LC-MS) : 452,11 ; Temps de rétention : 1,82 min.
RNIN : 1,4 : s, 6H ; 2,05 : s, 3H ; 4,65 : s, 2H ; 7,1 : m, 1H ; 7,65 : d, 2H ; 7,85 : d, 2H ; 8,15 : m, 1H ; 8,25 : m, 1H.

### Etape 2 :

500 mg (1,11 mmol) de N-{4-[5,5-diméthyl-2,4-dioxo-3-(4-trifluorométhylsulfanyl-phényl)-imidazolidin-1-yl-méthyl]-pyridin-2-yl}-acétamide est dissous dans du MeOH, 1,5 mmol de NaOMe sont ajoutés et le mélange résultant est chauffé à reflux pendant 4 heures. Le solvant est éliminé, le résidu est repris dans du CH₂Cl₂, lavé deux fois avec une solution de MaHCO₃ à 10 % et de l'eau et la phase organique est évaporée. 340 mg (75 %) de 1-(2-amino-pyridin-4-ylméthyl)-5,5-diméthyl-3-(4-trifluorométhyl-sulfanyl-phényl)-imidazolidine-2,4-dione sont isolés de cette façon.
MS (LC-MS) : 410,10 ; Temps de rétention : 1,57.
RMN : 1,4 : s, 6H ; 4,4 : s, 2H ; 6,35 : s, 2H ; 6,4 : m, 1H ; 6,5 : m, 1H ; 7,65 : d, 2H ; 7,9 : m, 3H.

### Etape 3 : Procédure générale, tous les dérivés d'urée et de thiourée sont préparés de cette façon

100 mg de 1-(2-amino-pyridin-4-ylméthyl)-5,5-diméthyl-3-(4-trifluorométhylsulfanyl-phényl)-imidazolidine-2,4-dione sont dissous dans 5 ml de dioxane et 1,5 équivalents de l'isocyanate ou de l'isothiocyanate correspondant sont ajoutés. La réaction est agitée à température légèrement élevée jusqu'à la fin, qui est contrôlée par LCMS. Le solvant est éliminé, une purification supplémentaire est obtenue par chromatographie sur un système HPLC.

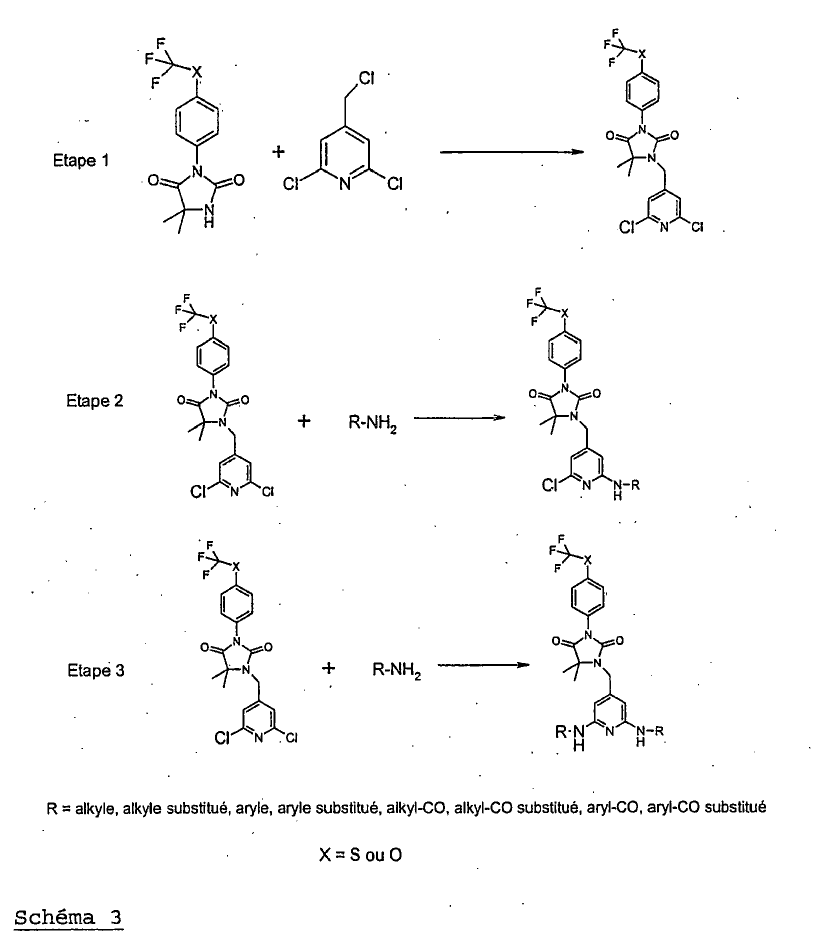

### Etape 1 :

### (synthèses décrites pour X = S, schéma analogue pour X =0)

De la 5,5-diméthyl-3-(4-trifluorométhylsulfanyl-phényl)-imidazolidine-2,4-dione (1 000 mg/3,29 mmol) est dissoute dans 20 ml de DMF, du Cs₂CO₃ (3,21 g/9,9 mmol) et de la 2,6-dichloro-4-chlorométhyl-pyridine (774 g/3,9 mmol) sont ajoutés et le mélange résultant est chauffé à 80°C pendant 6 heures. Le solvant est éliminé, le résidu est dissous dans du CH₂Cl₂ et lavé trois fois dans de l'eau. Après évaporation du solvant le matériau brut est davantage purifié par chromatographie sur un système HPLC.

### Etape 2 : Procédure générale de mono-amination catalysée par le Pd de la 1-(2,6-dichloro-pyridin-4-ylméthyl)-5,5-diméthyl-3-(4-trifluorométhylsulfanyl-phényl)-imidazolidine-2,4-dione

Cette étape est identique à l'étape 4 du schémas 1, mais seulement 1 équivalent de l'amine ou de l'amide correspondant est utilisé.

### Etape 3 : Procédure générale pour la bis-amination catalysée par le Pd de la 1-(2,6-dichloro-pyridin-4-ylméthyl)-5,5-diméthyl-3-(4-trifluorométhylsulfanyl-phényl)-imidazolidine-2,4-dione

Cette étape est identique à l'étape 4 du schéma 1, mais à présent 2,2, équivalents de l'amine ou de l'amide correspondant sont utilisés.

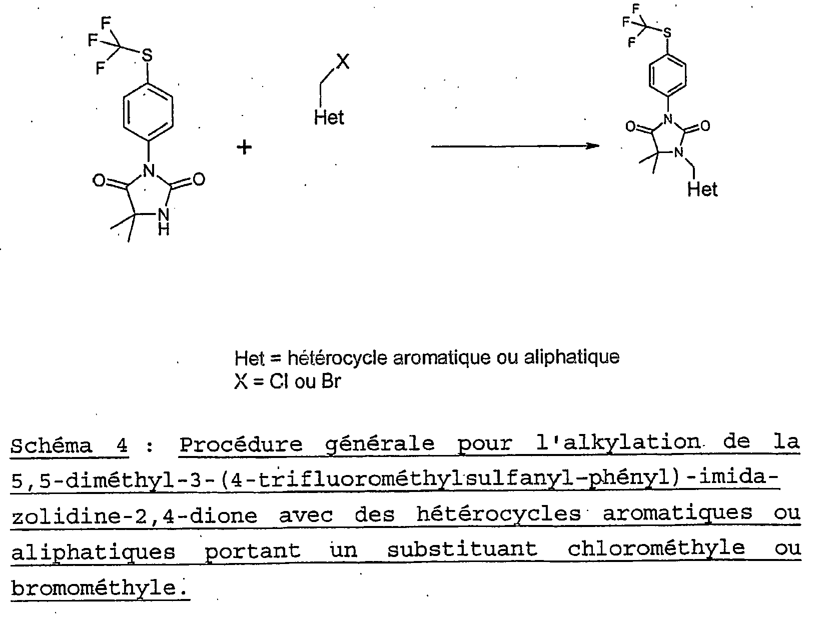

De la 5,5-diméthyl-3-(4-trifluorométhylsulfanyl-phényl)-imidazolidine-2,4-dione (100 mg/0,33 mmol) est dissoute dans 10 ml de DMF, du Cs₂CO₃ (321 mg/0,99 mmol) et 0,49 mmol (1,5 équivalents) de l'hétérocycle aromatique ou aliphatique substitué par un groupe chlorométhyle ou bromométhyle correspondant sont ajoutés et le mélange résultant est chauffé à 80°C pendant 6 heures. Le solvant est éliminé et le matériau brut obtenu est davantage purifié par chromatographie sur un système HPLC.

### Synthèses des exemples 175, 176 et 177

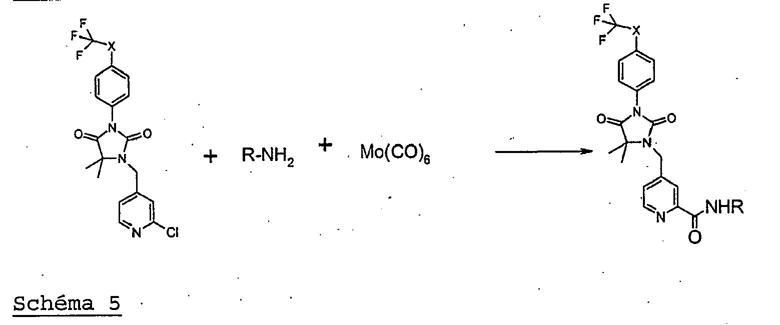

100 mg (0,23 mmol) de 1-(2-chloro-pyridin-4-ylméthyl)-5,5-diméthyl-3-(4-trifluorométhylsulfanyl-phényl)-imidazolidine-2,4-dione, 20 mg de Pd(OAc)₂, 60 mg de XANTPHOS, 300 mg de Cs₂CO₃, 124 mg de Mo(CO)₆ (2 Eq) sont transférés dans un tube réactionnel avec un bouchon à vis équipé avec d'un joint en caoutchouc et une atmosphère d'argon est générée dans le tube.

1,5 équivalents (0,35 mmol) de l'amine appropriée et 212 mg de DBU sont dissous dans 10 ml de toluène, cette solution est transférée dans le tube réactionnel mentionné ci-dessus et le mélange résultant est chauffé à 95°C pendant 6 à 10 heures en fonction de la progression de la réaction contrôlée par LCMS.

Après filtration le solvant est éliminé sous vide et le produit brut est davantage purifié par chromatographie sur un système HPLC.

### Synthèse de l'exemple 178

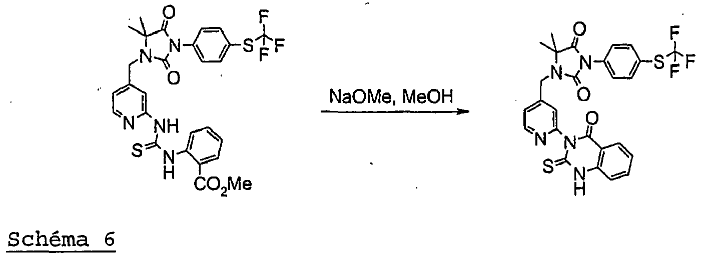

30 mg (0,05 mmol) d'ester méthylique de l'acide 2-(3-{4-[5,5-diméthyl-2,4-dioxo-3-(4-trifluorométhylsulfanyl-phényl)-imidazolidin-1-ylméthyl]-pyridin-2-yl}-thiouréido)-benzoîque sont dissous dans 2 ml de MeOH, 0,1 mmol de NaOMe est ajouté et le mélange résultant est agité à température ambiante toute une nuit. Après évaporation du solvant le matériau brut de 5,5-diméthyl-1-[2-(4-oxo-2-thioxo-1,4-dihydro-2H-quinazolin-3-yl)-pyridin-4-ylméthyl]-3-(4-trifluorométhylsulfanyl-phényl)-imidazolidine-2,4-dione est davantage purifié par chromatographie sur un système HPLC.

### Exemple 70 : N-{4-[5,5-diméthyl-2,4-dioxo-3-(4-trifluorométhyl-sulfanyl-phényl)-imidazolidin-1-ylméthyl]-pyridin-2-yl}-propionamide ; composé avec de l'acide trifluoroacétique

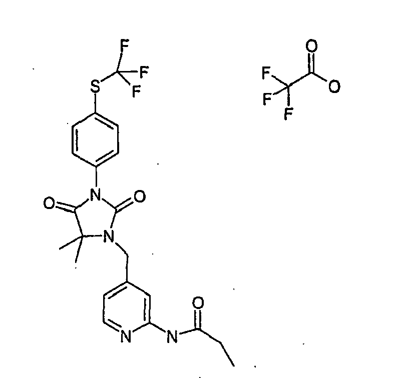

MS (LC-MS) : 466,13, Temps de rétention : 1,83 min.
RMN : 1,05 : t, 3H ; 1,40 : s, 6H ; 2,35 : q, 2H ; 4,65 : s, 2H ; 7,1 : d, 1H ; 7,65 : d, 2H ; 7,85 : d, 2H ; 8,15 : s, 1H ; 8,25 : d, 1H.

La synthèse est décrite dans le schéma 1.

### Exemple 71 : N-{4-[5,5-diméthyl-2,4-dioxo-3-(4-trifluorométhyl-sulfanyl-phényl)-imidazolidin-1-ylméthyl]-pyridin-2-yl}-isobutyramide; composé avec de l'acide trifluoroacétique

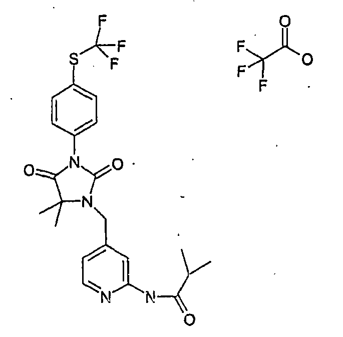

MS (LC-MS) : 480,14, Temps de rétention : 1,93 min.
RMN : 1,10 : d, 6H ; 1,40 : s, 6H ; 2,75 : s, 1H ; 4,65 : s, 2H ; 7,15 : d, 1H ; 7,65 : d, 2H ; 7,85 : d, 2H ; 8,15 : s, 1H ; 8,25 : d, 1H.

La synthèse est décrite dans le schéma 1.

### Exemple 72 : N-{4-[5,5-diméthyl-2,4-dioxo-3-(4-trifluorométhyl-sulfanyl-phényl)-imidazolidin-1-ylméthyl]-pyridin-2-yl}-3-morpholin-4-yl-propionamide ; composé avec de l'acide trifluoroacétique

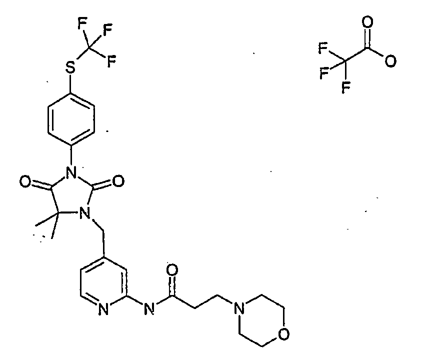

MS (LC-MS) : 551,18, Temps de rétention : 1,54 min.
RMN : 1,40 : s, 6H ; 2,85 : m, 2H ; 3,1 : m, 2H ; 3,4 : m, 2H ; 3,95 : m, 2H ; 4,65 : s, 2H ; 7,15 : d, 1H ; 7,65 : d, 2H ; 7,85 : d, 2H ; 8,15 : s, 1H ; 8,25 : d, 1H.

La synthèse est décrite dans le schéma 1.

### Exemple 73 : N-{4-[5,5-diméthyl-2,-4-dioxo-3-(4-trifluorométhyl-sulfanyl-phényl)-imidazolidin-1-ylméthyl]-pyridin-2-yl}-3-[4-(2-hydroxy-éthyl)-piperazin-1-yl]-propionamide

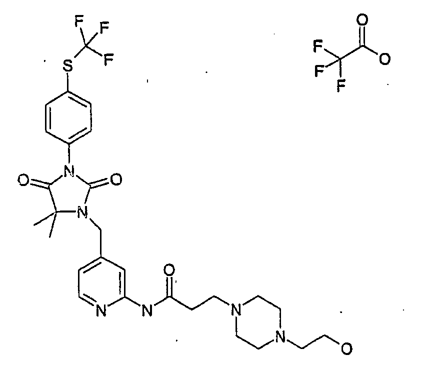

MS (LC-MS) : 594,22, Temps de rétention : 1,40 min.
RMN : 1,40 : s, 6H ; 2,55 à 3,50 : m, 12H ; 4,65 : s, 2H ; 7,15 : m, 1H ; 7,65 : d, 2H ; 7,85 : d, 2H ; 8,15 : s, 1H ; 8,25 : d, 1H.

La synthèse est décrite dans le schéma 1.

### Exemple 74 : N-{4-[5,5-diméthyl-2,4-dioxo-3-(4-trifluorométhyl-sulfanyl-phényl)-imidazolidin-1-ylméthyl]-pyridin-2-yl}-3-(4-méthyl-piperazin-1-yl)-propionamide

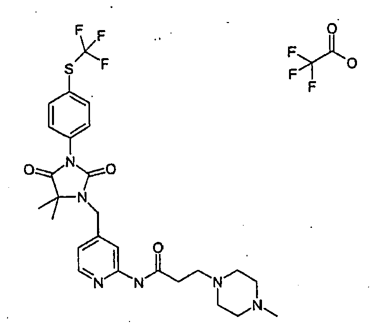

MS (LC-MS) : 564,21, Temps de rétention : 1,41 min.
RMN : 1,40 : s, 6H ; 2,55 à 3,50 : m, 12H ; 2,75 : s, 3H ; 4,65 : s, 2H ; 7,15 : m, 1H ; 7,65 : d, 2H ; i 7,85 : d, 2H ; 8,15 : s, 1H ; 8,25 : d, 1H.
La synthèse est décrite dans le schéma 1.

### Exemple 75 : {4-[5,5-diméthyl-2,4-dioxo-3-(4-trifluorométhyl-sulfanyl-phényl)-imidazolidin-1-ylmethyl]-pyridin-2-yl}-amide de l'acide cyclopropanecarboxylique

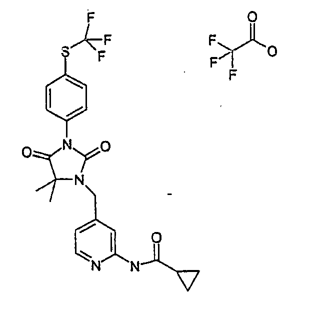

MS (LC-MS) : 478,13, Temps de rétention : 1,99 min.
RMN : 0,80 : m, 4H ; 1,15 : t, 1H ; 1,40 : s, 6H ; 4,65 : s, 2H ; 7,15 : m, 1H ; 7,65 : d, 2H ; 7,85 : d, 2H ; 8,15 : s, 1H ; 8,25 : d, 1H.
La synthèse est décrite dans le schéma 1.

### Exemple 76 : 5,5-diméthyl-1-[2-(pyridin-2-ylamino)-pyridin-4-ylméthyl]-3-(4-trifluorométhylsulfanyl-phényl)-imidazo-lidine-2,4-dione

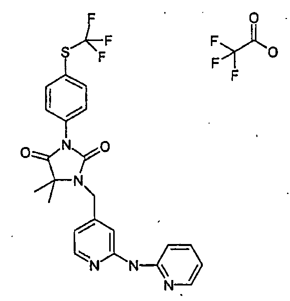

MS (LC=MS) : 487,13, Temps de rétention : 1,85 min.
RMN : 1,40 : s, 6H ; 4,65 : s, 2H ; 7,3 : m, 4H ; 7,65 : d, 2H ; 7,85 : d, 2H ; 8,05 : s, 1H ; 8,35 : m, 1H.
La synthèse est décrite dans le schéma 1.

### Exemple 77 : N-{4-[5,5-diméthyl-2,4-dioxo-3-(4-trifluorométhyl-sulfanyl-phényl)-imidazolidin-1-ylméthyl]-pyridin-2-yl}-3-pyrrolidin-1-yl-propionamide

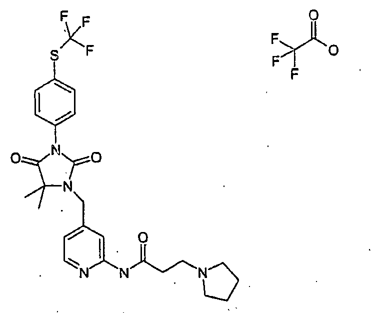

MS (LC-MS) : 535,19, Temps de rétention : 1,52 min.
RMN : 1,40 : s, 6H ; 1,8 : m, 2H ; 2,0 : m, 2H ; 2,85 : m, 2H ; 3,0 : m, 2H ; 3,4 : m, 2H ; 3,5 : m, 2H ; 4,65 : s, 2H ; 7,2 : m, 1H ; 7,65 : d, 2H ; 7,85 : d, 2H ; 8,15 : s, 1H ; 8,40 : m, 1H.

La synthèse est décrite dans le schéma 1.

### Exemple 78 : 5,5-diméthyl-1-{2-[3-(4-méthyl-piperazin-1-yl)-propylamino]-pyridin-4-ylméthyl}-3-(4-trifluorométhyl-sulfanyl-phényl)-imidazolidine-2,4-dione

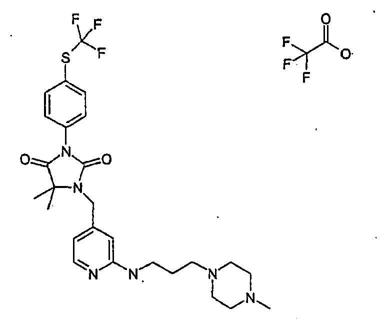

MS (LC-MS) : 550,23, Temps de rétention : 1,52 min.
RMN : 1,40 : s, 6H ; 2,6 à 3,5 : m, 14H ; 2,75 : s, 3H ; 4,65 : s, 2H ; 6,85 : m, 1H ; 6,95 : m, 1H ; 7,65 : d, 2H ; 7,9 : m, 3H.
La synthèse est décrite dans le schéma 1.

### Exemple 79 : 5,5-diméthyl-1-{2-[3-(4-éthyl-piperazin-1-yl)-propylamino]-pyridin-4-ylméthyl}-3-(4-trifluoromethyl-sulfanyl-phényl)-imidazolidine-2,4-dione

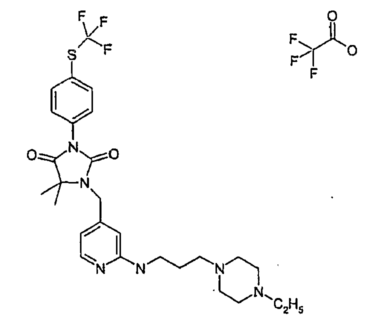

La synthèse est décrite dans le schéma 1.

### Exemple 80 : 1-[2-(3-méthoxy-phénylamino)-pyridin-4-ylméthyl]-5,5-diméthyl-3-(4-trifluorométhylsulfanyl-phényl)-imida-zolidine-2,4-dione

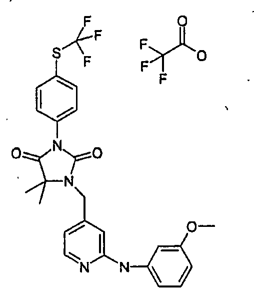

MS (LC-MS) : 516,14, Temps de rétention : 1,90 min.
RMN : 1,40 : s, 6H ; 3,7 : s, 3H ; 4,65 : s, 2H ; 6,6 : m, 1H ; 6,9 : m, 2H ; 7,15 : m, 1H ; 7,2 : m, 1H ; 7,25 : m, 1H ; 7,65 : d, 2H ; 7,85 : d, 2H ; 8,05 : s, 1H.

La synthèse est décrite dans le schéma 1.

### Exemple 81 : N-{4-[5,5-diméthyl-2,4-dioxo-3-(4-trifluorométhyl-sulfanyl-phényl)-imidazolidin-1-ylméthyl]-pyridin-2-yl}-3-(4-éthyl-piperazin-1-yl)-propionamide

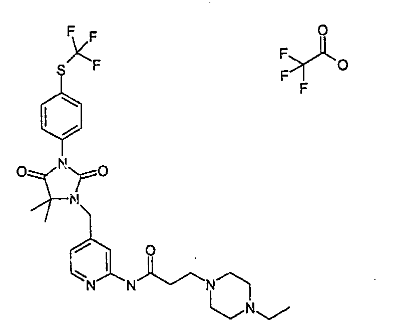

MS (LC-MS) : 516,14, Temps de rétention : 1,90 min.
RMN : 1,15 : t, 3H ; 1,40 : s, 6H ; 2,5 à 3,7 : m, 14H ; 4,65 : s, 2H ; 7,15 : m, 1H ; 7,65 : d, 2H ; 7,85 : d, 2H ; 8,15 : s, 1H ; 8,25 : m, 1H.

La synthèse est décrite dans le schéma 1.

### Exemple 82 : 5,5-diméthyl-1-[2-(3-méthyl-2-oxo-pyrrolidin-1-yl)-pyridin-4-ylméthyl]-3-(4-trifluorométhylsulfanyl-phényl)-imidazolidine-2,4-dione ; composé avec de l'acide trifluoroacétique

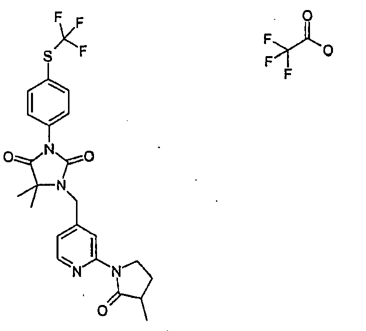

MS (LC-MS) : 492,14 ; Temps de rétention : 2,23 min.
RMN : 1,15 : d, 3H ; 1,40 s, 6H ; 1,60 : m, 1H ; 2,30 : m, 1H ; 2,70 : m, 1H ; 3,80 : m, 1H ; 4,0 : m, 1H ; 4,65 : s, 2H ; 7,15 : m, 1H ; 7,65 : d, 2H ; 7,85 d, 2H ; 8,35 : m, 2H.

La synthèse est décrite dans le schéma 1.

### Exemple 83

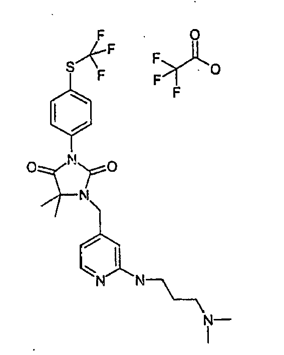

MS (LC-MS) : 495,19 ; Temps de rétention : 1,43 min.

La synthèse est décrite dans le schéma 1.

### Exemple 84 : 5,5-diméthyl-1-[2-(4-pyridin-2-yl-piperazin-1-yl)-pyridin-4-ylméthyl]-3-(4-trifluorométhylsulfanylphényl)-imidazolidine-2,4-dione ; composé avec de l'acide trifluoroacétique

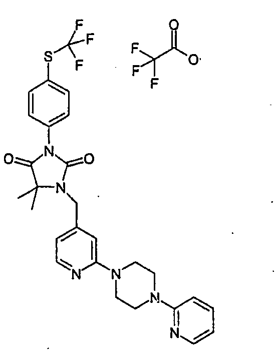

MS (LC-MS) : 556,19 ; Temps de rétention : 1,56 min. La synthèse est décrite dans le schéma 1.

### Exemple 85 : N-{4-[5,5-diméthyl-2,4-dioxo-3-(4-trifluorométhyl-sulfanyl-phényl)-imidazolidin-1-ylméthyl]-pyridin-2-yl}-3-piperidin-1-yl-propionamide ; composé avec de l'acide trifluoroacétique

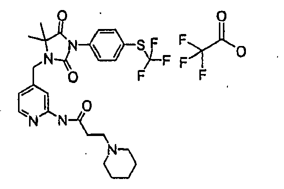

MS (LC-MS) : 549,22 ; Temps de rétention : 1,62 min.
RMN : 1,45 : s, 6H ; 1,55 : m, 3H ; 1,80 : m, 2H ; 2,80 : m, 4H ; 3,3 à 3,5 : m, 7H ; 4,65 : s, 2H ; 7,15 : m, 1H ; 7,65 : d, 2H ; 7,85 _{:} m, 2H ; 8,15 : m, 1H ; i 8,3 : m, 1H ; 9,0 : m, 1H.

La synthèse est décrite dans le schéma 1.

### Exemple 86 : 1-[2-(3-imidazol-1-yl-propylamino)-pyridin-4-ylméth-yl]-5,5-diméthyl-3-(4-trifluorométhylsulfanylphényl)-imidazolidine-2,4-dione ; composé avec de l'acide trifluoroacétique

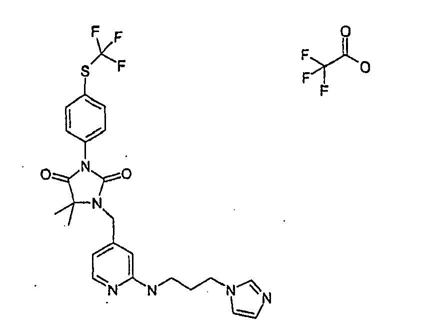

MS (LC-MS) : 518,17 ; Temps de rétention : 1,57 min. La synthèse est décrite dans le schéma 1.

### Exemple 87 : 1-[2-(4-éthyl-pyridin-2-ylamino)-pyridin-4-yl-méthyl]-5,5-diméthyl-3-(4-trifluorométhylsulfanylphényl)-imidazolidine-2,4-dione

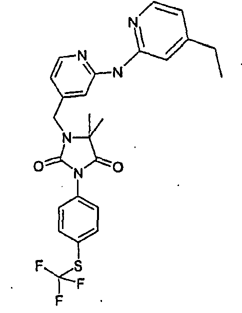

MS (LC-MS) : 515,16 ; Temps de rétention : 1,85 min.
RMN : 1,15 : t, 3H ; 1,45 : s, 6H ; 2,7 : q, 2H ; 4,75 : s, 2H ; 7,1 : s large, 2H ; 7,3 : s large 2HM ; 7,65 : d, 2H ; 7,85 : d, 2H ; 8,25 : s, 1H ; 8,45 : s, 1H.

La synthèse est décrite dans le schéma 1.

### Exemple 88 : 1-[2-(6-éthyl-pyridin-2-ylamino)-pyridin-4-yl-méthyl]-5,5-diméthyl-3-(4-trifluorométhylsulfanylphényl)-imidazolidine-2,4-dione

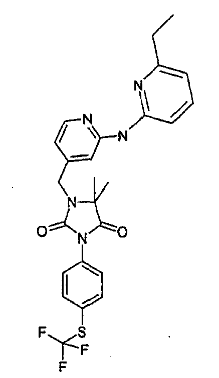

MS (LC-MS) : 515,16 ; Temps de rétention : 1,95 min.
RMN : 1,15 : t, 3H ; 1,45 : s, 6H ; 2,7 : q, 2H ; 4,75 : s, 2H ; 7,1 : s large, 2H ; 7,3 : s large 2HM ; 7,65 d, 2H ; 7,85 : d, 2H ; 8,25 : s, 1H ; 8,45 : s, 1H.

La synthèse est décrite dans le schéma 1.

### Exemple 89 : 5,5-diméthyl-1-[2-(quinolin-2-ylamino)-pyridin-4-ylméthyl]-3-(4-trifluorométhylsulfanyl-phényl)-imidazoli-dine-2,4-dione

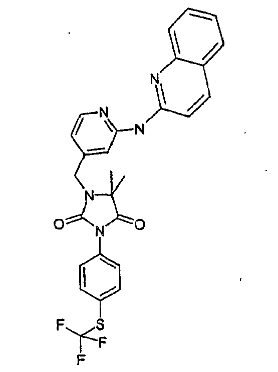

MS (LC-MS) : 537,14 ; Temps de rétention : 1,95 min.
RMN : 1,45 : s, 6H ; 4,75 : s, 2H ; 7,4 à 7,6 : m, 6H ; 7,65 : d, 2H ; 7,85 : d, 2H ; 8,00 : s large, 1H ; 8,50 : s large, 2H.

La synthèse est décrite dans le schéma 1.

### Exemple 90 : 5,5-diméthyl-1-[2-(4-méthyl-pyridin-2-ylamino)-pyridin-4-ylméthyl]-3-(4-trifluorométhylsulfanyl-phényl)-imidazolidine-2,4-dione

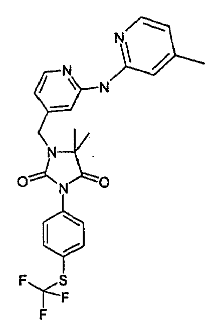

MS (LC-MS) : 501,14 ; Temps de rétention : 1,80 min.
RMN : 1,45 : s, 6H ; 2,4 : s, 3H ; 4,75 : s, 2H ; 7,1 : m, 2H ; 7,25 : m, 2H ; 7,65 : d, 2H ; 7,85 : d, 2H ; 8,20 : s large, 1H ; 8,45 : s large, 1H.

La synthèse est décrite dans le schéma 1.

### Exemple 91 : 5,5-diméthyl-1-[2-(6-méthyl-pyridin-2-ylamino)-pyridin-4-ylméthyl]-3-(4-trifluorométhylsulfanyl-phényl)-imidazolidine-2,4-dione

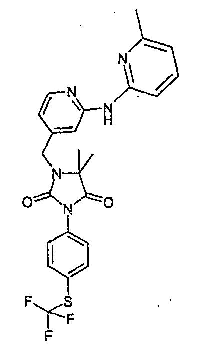

MS (LC-MS) : 501,14 ; Temps de rétention : 1,85 min.
RMN : 1,45 : s, 6H ; 2,55 : s, 3H ; 4,75 : s, 2H ; 7,1 : m, 1H ; 7,25 : m, 2H ; 7,4 : s, 1H ; 7,65 : d, 2H ; 7,85 : m, 3H ; 8,45 : s large, 1H.

La synthèse est décrite dans le schéma 1.

### Exemple 92 : 1-[2-(3,5-dichloro-pyridin-2-ylamino)-pyridin-4-yl-méthyl]-5,5-diméthyl-3-(4-trifluorométhylsulfanyl-phényl)-imidazolidine-2,4-dione

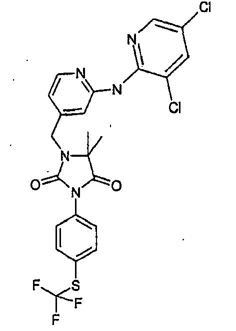

MS (LC-MS) : 555,05 ; Temps de rétention : 1,96 min.
RMN : 1,45 : s, 6H ; 4,70 : s, 2H ; 7,1 m, 1H ; 7,20 : m, 1H ; 7,65 : d, 2H ; 7,85 : m, 3H ; 8,30 : m, 3H.

La synthèse est décrite dans le schéma 1.

### Exemple 93 : 1-[2-(4,6-diméthyl-pyridin-2-ylamino)-pyridin-4-yl-méthyl]-5,5-diméthyl-3-(4-trifluorométhylsulfanyl-phényl)-imidazolidine-2,4-dione

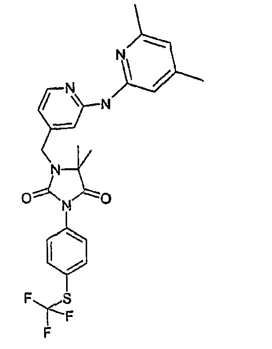

MS (LC-MS) : 515,16 ; Temps de rétention : 2,00 min.
RMN : 1,45 : s ; 2,35 : s, 3H ; 2,6 : s, 3H ; 6H ; 4,75 : s, 2H ; 7,0 : m, 2H ; 7,35 : m, 2H ; 7,70 : d, 2H ; 7,9 : d, 2H ; 8,40 : s large, 1H.

La synthèse est décrite dans le schéma 1.

### Exemple 94 : 5,5-diméthyl-1-[2-(méthyl-pyridin-2-yl-amino)-pyridin-4-ylméthyl]-3-(4-trifluorométhylsulfanylphényl)-imidazolidine-2,4-dione

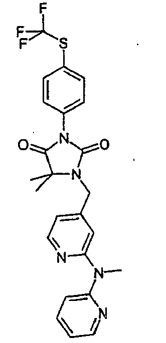

MS (LC-MS) : 501,14 ; Temps de rétention : 1,74 min. La synthèse est décrite dans le schéma 1.

### Exemple 95 : 5,5-diméthyl-1-[2-(pyridin-4-ylamino)-pyridin-4-yl-méthyl]-3-(4-trifluorométhylsulfanylphényl)-imidazoli-dine-2,4-dione

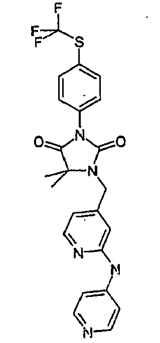

MS (LC-MS) : 487,13 ; Temps de rétention : 1,69 min.
RMN : 1,45 : s ; 4,70 : s, 2H ; 7,15 : m, 1H ; 7,25 : m, 1H ; 7,65 : d, 2H ; 7,9 : d, 2H ; 8,10 : s large, 2H ; 8,40 : s, 1H ; 8,50 : m, 2H.

La synthèse est décrite dans le schéma 1.

### Exemple 96 : 5,5-diméthyl-1-[2-(pyridin-3-ylamino)-pyridin-4-yl-méthyl]-3-(4-trifluorométhylsulfanylphényl)-imidazoli-dine-2,4-dione

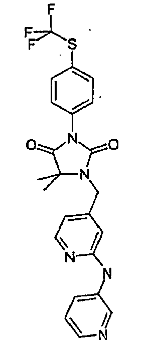

MS (LC-MS) : 487,13 ; Temps de rétention : 1,69 min.
RMN : 1,45 : s ; 4,60 : s, 2H ; 6,95 : s, 1H ; 7,05 : s, 1H ; 7,7: d, 2H ; 7,8 : m, 1H ; 7,85 : d, 2H ; 8,25 : s, 1H ; 8,35 : s, 1H ; 8,45 : m, 1H ; 9,4 : s, 1H.

La synthèse est décrite dans le schéma 1.

### Exemple 97 : N-{4-[5,5-diméthyl-2,4-dioxo-3-(4-trifluorométhyl-sulfanyl-phényl)-imidazolidin-1-ylméthyl]-pyridia-2-yl}-3-(2-oxo-azépan-1-yl)-propionamide.

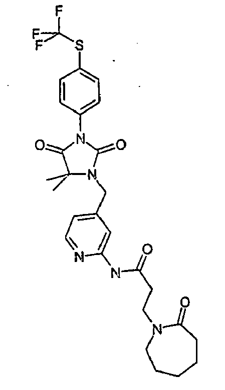

MS (LC-MS) : 577,20 ; Temps de rétention : 2,00 min.
RMN : 1,45 : s, 6H ; 1,5 : m, 4H ; 1,55 : m, 2H ; 2,35 : m, 2H ; 2,6 : m, 4H ; 3,3. m, 2H ; 4,65 : s, 2H ; 7,15 : s, 1H ; 7,65 : d, 2H ; 7,85 : d, 2H ; 8,1 : s, 1H ; 8,35 : s, 1H.

La synthèse est décrite dans le schéma 1.

### Exemple 98 : 3-(benzyl-méthylamino)-N-{4-[5,5-diméthyl-2,4-dioxo-3-(4-trifluorométhylsulfanyl-phényl)-imidazolidin-1-yl-méthyl]-pyridin-2-yl}-propionamide

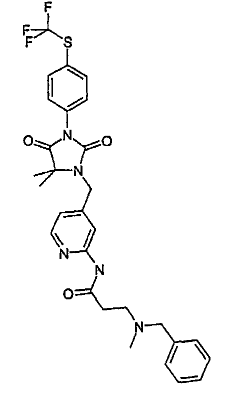

MS (LC-MS) : 585,20, Temps de rétention : 1,73 min.
RMN : 1,45 : s, 6H ; 2,2 : s, 3H ; 2,95 : m, 2H ; 3,3 : m, 1H ; 4,3 : m, 1H ; 4,4 : m, 1H ; 4,65 : s, 2H ; 7,2 : s, 1H ; 7,45 : m, 2H ; 7,50 : m, 2H ; 7,65 : d, 2H ; 7,85 : d, 2H ; 8,1 : m, 1H ; 8,3 : m, 1H.

La synthèse est décrite dans le schéma 1.

### Exemple 99 : N-4-[5,5-diméthyl-2,4-dioxo-3-(4-trifluorométhyl-sulfanyl-phényl)-imidazolidin-1-ylméthyl]-pyridin-2-yl}-2-pyrrolidin-1-yl-acétamide

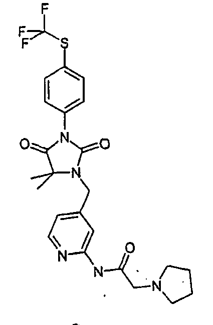

MS (LC-MS) : 521,17, Temps de rétention : 1,68 min.
RMN : 1,45 : s, 6H ; 1,85 : m, 2H ; 2,00 : m, 2H ; 3,1 : m, 2H ; 4,25 : m, 2H ; 4,7 : s, 2H ; 7,25 : m, 1H ; 7,65 : d, 2H ; 7,85 : d, 2H ; 8,1 : s large, 1H ; 8,3 : s, 1H.

La synthèse est décrite dans le schéma 1.

### Exemple 100 : N-{4-[5,5-diméthyl-2,4-dioxo-3-(4-trifluorométhyl-sulfanyl-phényl)-imidazolidin-1-ylméthyl]-pyridin-2-yl}-2-(4-pyridin-2-yl-piperazin-1-yl)-acétamide

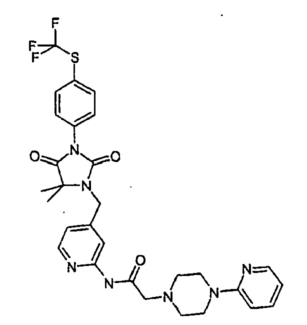

MS (LC-MS) : 613,21, Temps de rétention : 1,60 min.
RMN : 1,45 : s, 6H ; 3,3 : m large, 4H ; 4,35 : m large, 4H ; 4,65 : s, 2H ; 6,7 : m, 1H ; 6,9 : m, 1H ; 7,25 : s, 1H ; 7,65, m + d : 3H ; 7,85 : d, 2H ; 8,15 : m, 2H ; 8,35 : s, 1H.

La synthèse est décrite dans le schéma 1.

### Exemple 101 : 2-[(2-diméthylamino-éthyl)-méthylamino]-N-{4-[5,5-diméthyl-2,4-dioxo-3-(4-trifluorométhylsulfanylphényl)-imidazolidin-1-ylméthyl]-pyridin-2-yl}-acétamide

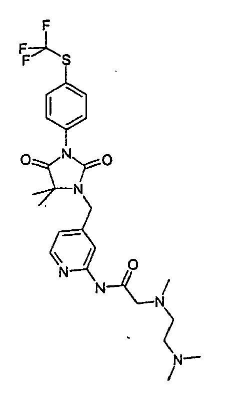

MS (LC-MS) : 552,21, Temps de rétention : 1,52 min.
RMN : 1,45 : s, 6H ; 2,75 : s, 6H ; 4,65 : s, 2H ; 7,20 : m, 1H ; 7,65 : d, 2H ; 7,85 : d, 2H ; 8,15 : s, 1H ; 8,30 : m, 1H.

La synthèse est décrite dans le schéma 1.

### Exemple 102 : 3-[(2-diméthylamino-éthyl)-méthylamino]-N-{4-[5,5-diméthyl-2,4-dioxo-3-(4-trifluorométhylsulfanylphényl)-imidazolidin-1-ylméthyl]-pyridin-2-yl}-propionamide

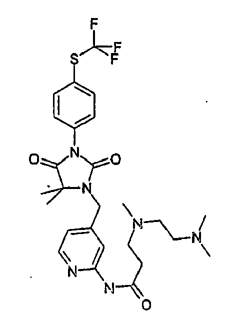

MS (LC-MS) : 566,23, Temps de rétention : 1,37 min.
RMN : 1,45 : s, 6H ; 2,75 : s, 6H ; 2,8 à 3,4 : m large, 8H ; 7,20 : m, 1H ; 7,65 : d, 2H ; 7,85 : d, 2H ; 8,15 : s, 1H ; 8,30 : m, 1H.

La synthèse est décrite dans le schéma 1.

### Exemple 103 : 5,5-diméthyl-1-[2-(5-méthyl-pyridin-2-ylamino)-pyridin-4-ylméthyl]-3-(4-trifluorométhylsulfanyl-phényl)-imidazolidine-2,4-dione

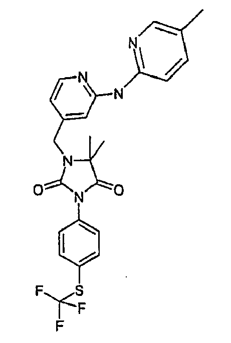

MS (LC-MS) : 501,14, Temps de rétention : 1,83 min.
RMN : 1,45 : s, 6H ; 2,3 : s, 3H ; 4,7 : s, 2H ; 7,3 : m, 3H ; 7,7 : d, 2H ; 7,9 : d, 2H ; 8,2 : m, 1H ; 8,35 : m, 1H.

La synthèse est décrite dans le schéma 1.

### Exemple 104 : N-{4-[5,5-diméthyl-2,4-dioxo-3-(4-trifluorométhyl-sulfanyl-phényl)-imidazolidin-1-ylméthyl]-pyridin-2-yl}-3,5-diméthoxy-benzamide ; composé avec de l'acide trifluoroacétique

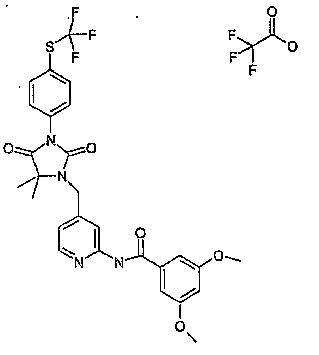

MS (LC-MS) : 574,78, Temps de rétention : 2,34 min.
RMN : 1,45 : s, 6H ; 2,3 : s, 3H ; 4,7 : s, 2H ; 6,7 : s, 2H ; 7,2 : m, 2H ; 7,25 : m, 1H ; 7,7 : d, 2H ; 7,9 : d, 2H ; 8,2 : m, 1H ; 8,3 : m, 1H.

La synthèse est décrite dans le schéma 1.

### Exemple 105 : 2-(benzyl-méthylamino)-N-{4-[5,5-diméthyl-2,4-dioxo-3-(4-trifluorométhylsulfanyl-phényl)-imidazolidin-1-yl-méthyl]-pyridin-2-yl}-acétamide ; composé avec de l'acide trifluoroacétique

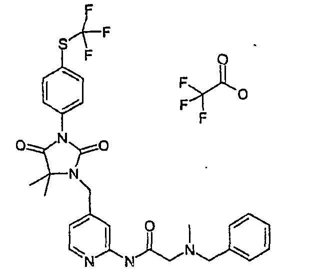

MS (LC-MS) : 574,78, Temps de rétention : 2,34 min.
RMN : 1,45 : s, 6H ; 2,8 : s, 4,0 à 4,4 : m, 4H ; 4,7 : s, 2H ; 7,2 : m, 1H ; 7,4 : m, 2H ; 7,5 : 1 m, 2H ; 7,7 : d, 2H ; 7,9 : d, 2H ; 8,1 : m, 1H ; 8,3 : m, 1H.

La synthèse est décrite dans le schéma 1.

### Exemple 106 : 5,5-diméthyl-1-[2-(pyrazin-2-ylamino)-pyridin-4-yl-méthyl]-3-(4-trifluorométhylsulfanylphényl)-imidazoli-dine-2,4-dione ; composé avec de l'acide trifluoroacétique

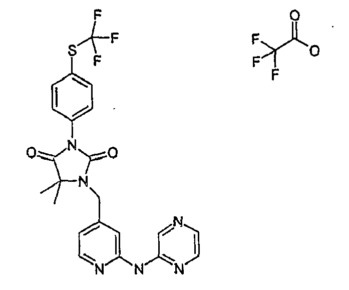

MS (LC-MS) : 488,12, Temps de rétention : 1,63.
RMN : 1,45 : s, 6H ; 4,7 : s, 2H ; 7,1 : m, 1H ; 7,6 : m, 1H ; 7,65 : d, 2H ; 7,9 : d, 2H ; 8,2 : s, 1H ; i 8,3 : m, 2H ; 9,0 : s, 1H.

La synthèse est décrite dans le schéma 1.

### Exemple 107 : N-{4-[5,5-diméthyl-2,4-dioxo-3-(4-trifluorométhyl-sulfanyl-phényl)-imidazolidin-1-ylméthyl]-pyridin-2-yl}-2-phényl-butyramide ; composé avec de l'acide trifluoroacétique

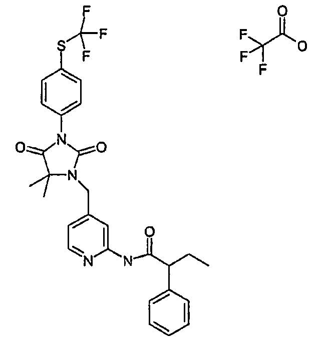

MS (LC-MS) : 566,18, Temps de rétention : 2,43.
RMN : 0,7 : t, 3H ; 1,4 : s, 6H ; 1,7 : m, 1H ; 2,05 : m, 1H ; 4,6 : s, 2H ; 7,1 : s, 1H ; 7,25 : m, 1H ; 7,3 : m, 1H ; 7,4 : m, 2H ; 7,65 : d, 2H ; 7,85 : d, 2H ; 8,15 : s, 1H ; 8,25 : m, 1H.

La synthèse est décrite dans le schéma 1.

### Exemple 108 : N-{4-[5,5-diméthyl-2,4-dioxo-3-(4-trifluorométhyl-sulfanyl-phényl)-imidazolidin-1-ylméthyl]-pyridin-2-yl}-3-(3-méthyl-piperidin-1-yl)-propionamide ; composé avec de l'acide trifluoroacétique

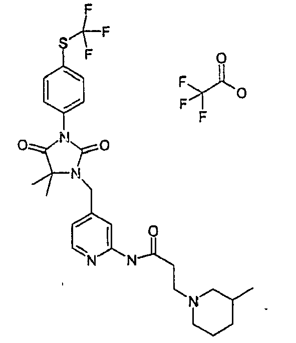

MS (LC-MS) : 563,22, Temps de rétention : 1,62.

La synthèse est décrite dans le schéma 1.

### Exemple 109 : 1-[2-(4-méthoxy-phénylamino)-pyridin-4-ylméthyl]-5,5-diméthyl-3-(4-trifluorométhylsulfanyl-phényl)-imida-zolidine-2,4-dione

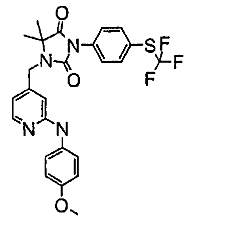

MS (LC-MS) : 517,1 ; Temps de rétention : 1,74.
RMN : 1,4 : s, 6H ; 4,6 : s, 2H ; 6,85 : s large, 1H ; 7,0 : m, 1H ; 7,4 : m, 2H ; 7,6 : d, 2H ; 7,9 : d, 2H ; 8,95 : s, 1H.

La synthèse est décrite dans le schéma 1.

### Exemple 110 : 5,5-diméthyl-1-[2-(2-oxo-pyrrolidin-1-yl)-pyridin-4-ylméthyl]-3-(4-trifluorométhylsulfanyl-phényl)-imidazoli-dine-2,4-dione

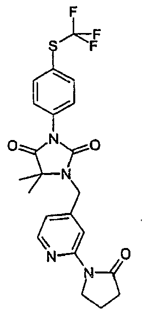

MS (LC-MS) : 478,13 ; Temps de rétention : 1,83.

La synthèse est décrite dans le schéma 1.

### Exemple 111 : {4-[5,5-diméthyl-2,4-dioxo-3-(4-trifluorométhyl-sulfanyl-phényl)-imidazolidin-1-ylméthyl]-pyridin-2-yl}-amide de l'acide pyrazine-2-carboxylique ; composé avec de l'acide trifluoroacétique

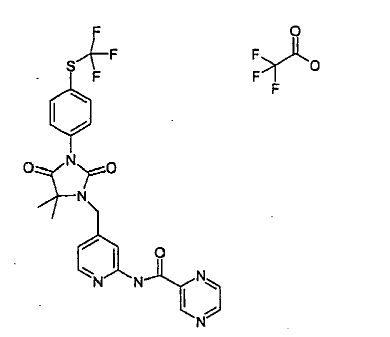

MS (LC-MS) : 516,12 ; Temps de rétention : 2,42.
RMN : 1,4 : s, 6H ; 4,7 : s, 2H ; 7,3 : m, 1H ; 7,65 : d, 2H ; 7,95 : d, 2H ; 8,3 : s, 1H ; 8,4 : s, 1H ; 8,7 : s, 1H ; 9,0 : s, 1H ; 9,4 : s, 1H.

La synthèse est décrite dans le schéma 1.

### Exemple 112 : N-{4-[5,5-diméthyl-2,4-dioxo-3-(4-trifluorométhyl-sulfanyl-phényl)-imidazolidin-1-ylméthyl]-pyridin-2-yl}-2,2-diméthyl-propionamide ; composé avec de l'acide trifluoroacétique

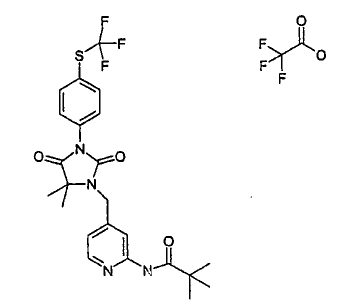

MS (LC-MS) : 494,16 ; Temps de rétention : 2,48.
RMN : 1,2 : s, 9H ; 1,4 : s, 6H ; 4,7 : s, 2H ; 7,2 : m, 1H ; 7,65 : d, 2H ; 7,90 : d, 2H ; 8,1 : s, 1H ; 8,3 : s, 1H.

La synthèse est décrite dans le schéma 1.

### Exemple 113 : {4-[5,5-diméthyl-2,4-dioxo-3-(4-trifluorométhyl-sulfanyl-phényl)-imidazolidin-1-ylméthyl]-pyridin-2-yl}-amide de l'acide thiophène-2-carboxylique ; composé avec de l'acide trifluoroacétique

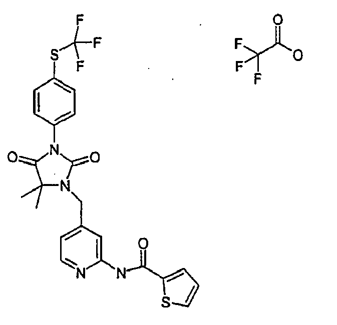

MS (LC-MS) : 520,09 ; Temps de rétention : 2,14.
RMN : 1,4 : s, 6H ; 4,7 : s, 2H ; 7,2 : m, 2H ; 7,65 : d, 2H ; 7,8 : m, 3H ; 8,2 : s, 1H ; 8,25 : s, 1H ; 8,4 : m, 1H.

La synthèse est décrite dans le schéma 1.

### Exemple 114 : N-{4-[5,5-diméthyl-2,4-dioxo-3-(4-trifluorométhyl-sulfanyl-phényl)-imidazolidin-1-ylméthyl]-pyridin-2-yl}-4-méthyl-benzamide ; composé avec de l'acide trifluoroacétique

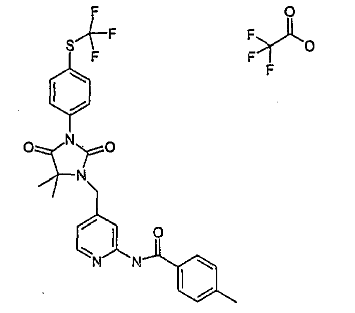

MS (LC-MS) : 528,16 ; Temps de rétention : 2,23.
RMN : 1,4 : s, 6H ; 4,7 : s, 2H ; 7,2 : m, 1H ; 7,3 : d, 2H ; 7,7 : d, 2H ; 7,85 : d, 2H ; 7,95 : d, 2H ; 8,2 : s, 1H ; 8,3 : s, 1H.

La synthèse est décrite dans le schéma 1.

### Exemple 115 : N-{4-[5,5-diméthyl-2,4-dioxo-3-(4-trifluorométhyl-sulfanyl-phényl)-imidazolidin-1-ylméthyl]-pyridin-2-yl}-3-(3,5-diméthyl-piperidin-1-yl)-propionamide ; compose avec de l'acide trifluoroacétique

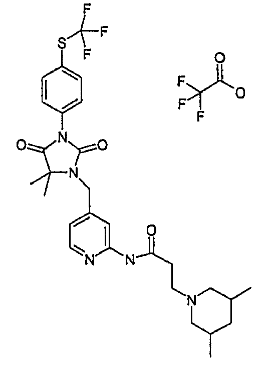

MS (LC-MS) : 577,23 ; Temps de rétention : 1,72.

La synthèse est décrite dans le schéma 1.

### Exemple 116 : N-{4-[5,5-diméthyl-2,4-dioxo-3-(4-trifluorométhyl-sulfanyl-phényl)-imidazolidin-1-ylméthyl]-pyridin-2-yl}-3-(4-pyridin-2-yl-piperazin-1-yl)-propionamide ; composé avec de l'acide trifluoroacétique

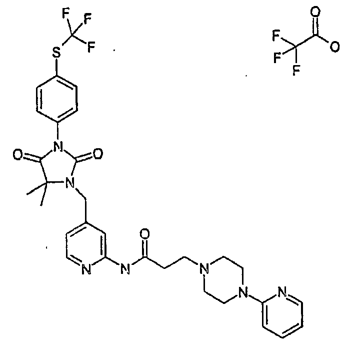

MS (LC-MS) : 627,22 ; Temps de rétention : 1,50.
RMN : 1,4 : s, 6H ; 2,9 : m, 2H ; 3,1 : m, 2H ; 3,4 : m, 2H ; 3,6 : m, 2H ; 4,4 : m, 2H ; 4,65 : s, 2H ; 6,7 : m, 1H ; 6,95 : d, 1H ; 7,2 : m, 1H ; 7,7 : d, 2H ; 7,9 : d, 2H ; 8,15 : m, 2H ; 8,3 : m, 1H.

La synthèse est décrite dans le schéma 1.

### Exemple 117 : 5,5-diméthyl-1-[2-(5-trifluorométhylpyridin-2-yl-amino)-pyridin-4-ylméthyl]-3-(4-trifluorométhyl-sulfanyl-phényl)-imidazolidine-2,4-dione

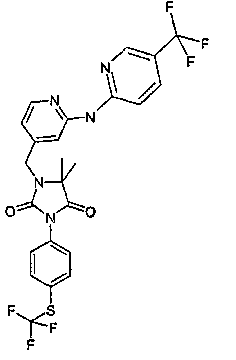

MS (LC-MS) : 555,51 ; Temps de rétention : 1,78.

La synthèse est décrite dans le schéma 1.

### Exemple 118 : N-{4-[5,5-diméthyl-2,4-dioxo-3-(4-trifluorométhyl-sulfanyl-phényl)-imidazolidin-1-ylméthyl]-pyridin-2-yl}-3-méthoxy-benzamide ; composé avec de l'acide trifluoroacétique

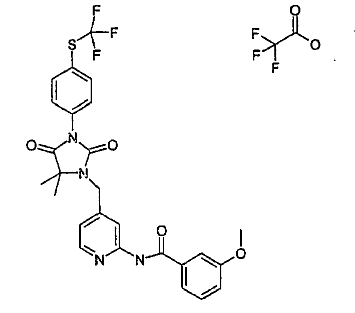

MS (LC-MS) : 544,14 ; Temps de rétention : 2,15.

La synthèse est décrite dans le schéma 1.

### Exemple 119 : 1-(3,5-dichloro-phényl)-3-{4-[5,5-dimithyl-2,4-dioxo-3-(4-trifluorométhylsulfanyl-phényl)-imidazolidin-1-ylméthyl]-pyridin-2-yl}-urée

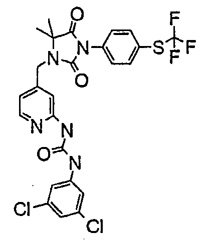

MS (LC-MS) : 597,06 ; Temps de rétention : 2,15 min.

La synthèse est décrite dans le schéma 2.

### Exemple 120 : Ester méthylique de l'acide 3-(3-{4-[5,5-diméthyl-2,4-dioxo-3-(4-trifluorométhylsulfanyl-phényl)-imidazo-lidin-1-ylméthyl]-pyridin-2-yl}-thiouréido)-benzoïque

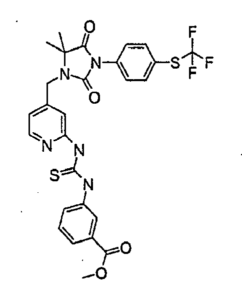

MS (LC-MS) : 603,12 ; Temps de rétention : 2,81 min.

La synthèse est décrite dans le schéma 2.

### Exemple 121 : 1-{4-[5,5-diméthyl-2,4-dioxo-3-(4-trifluorométhyl-sulfanyl-phényl)-imidazolidin-1-ylméthyl]-pyridin-2-yl}-3-phényl-urée

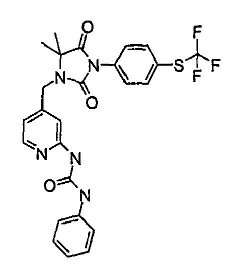

MS (LC-MS) : 529,14 ; Temps de rétention : 2,33 min.
RMN : 1,50 : s, 6H ; 4,65 : s, 1H ; 7,0 : m, 2H ; 7,30 : m, 2H ; 7,5 : m, 3H ; 7,65 : d, 2H ; 7,85 : d, 2H ; 8,25 : d, 1H ; 9,4 : s, 1H.

La synthèse est décrite dans le schéma 2.

### Exemple 122 : 1-(2,4-dichloro-phényl)-3-{4-[5,5-diméthyl-2,4-dioxo-3-(4-trifluorométhylsulfanyl-phényl)-imidazolidin-1-ylméthyl]-pyridin-2-yl}-urée

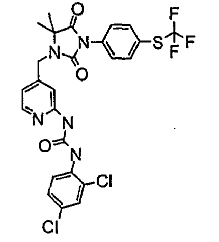

MS (LC-MS) : 597,06 ; Temps de rétention : 3,00 min.

La synthèse est décrite dans le schéma 2.

### Exemple 123 : 1-(3-chloro-phényl)-3-{4-[5,5-diméthyl-2,4-dioxo-3- (4-trifluorméthylsulfanyl-phényl)-imidazolidin-1-yl-méthyl]-pyridin-2-yl}-thiourée

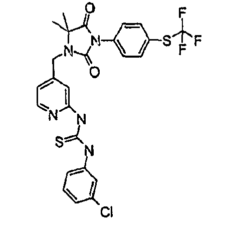

MS (LC-MS) : 579,08 ; Temps de rétention : 3,05 min.

La synthèse est décrite dans le schéma 2.

### Exemple 124 : Ester méthylique de l'acide 2-(3-{4-[5,5-diméthyl-2,4-dioxo-3-(4-trifluorométhylsulfanyl-phényl)-imidazo-lidin-1-ylméthyl]-pyridin-2-yl}-thiouréido)-benzoïque

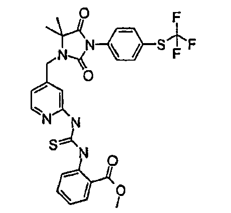

MS (LC-MS) : 603,12 ; Temps de rétention : 2,85 min.

La synthèse est décrite dans le schéma 2.

### Exemple 125 : Ester 3,5-diacétoxy-2-acétoxyméthyl-6-(3-{4-[5,5-diméthyl-2,4-dioxo-3-(4-trifluorométhylsulfanyl-phényl)-imidazolidin-1-ylméthyl]-pyridin-2-yl}-thiouréido)-tétrahydro-pyran-4-ylique de l'acide acétique

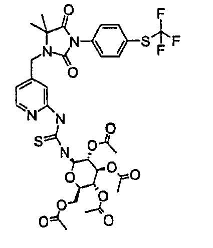

MS (LC-MS) : 799,18, Temps de rétention : 2,60 min.

La synthèse est décrite dans le schéma 2.

### Exemple 126 : 1-(4-diméthylamino-phényl)-3-{4-[5,5-diméthyl-2,4-dioxo-3-(4-trifluorométhylsulfanyl-phényl)-imidazolidin-1-ylméthyl]-pyridin-2-yl}-thiourée

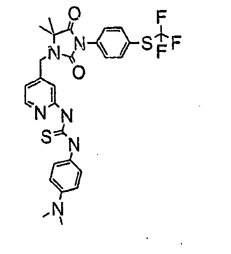

MS (LC-MS) : 588,16 ; Temps de rétention : 1,99.
RMN : 1,4 : s, 6H ; 2,9 : s, 6H ; 4,7 : s, 2H ; 6,7 : d, 2H ; 7,1 : m, 1H ; 7,25 : s, 1H ; 7,4 : d, 2H ; 7,7 : d, 2H ; 7,9 : d, 2H ; 8,25 : m, 1H.

La synthèse est décrite dans le schéma 2.

### Exemple 127 : 1-(2,4-diméthoxy-phényl)-3-{4-[5,5-diméthyl-2,4-dioxo-3-(4-trifluorométhylsulfanyl-phényl)-imidazolidin-1-ylméthyl]-pyridin-2-yl}-urée

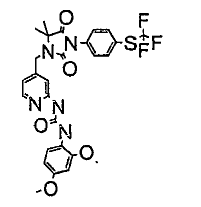

MS (LC-MS) : 589,60 ; Temps de rétention : 1,98.

La synthèse est décrite dans le schéma 2.

### Exemple 128 : Acide 3-(3-{4-[5,5-diméthyl-2,4-dioxo-3-(4-tri-fluorométhylsulfanyl-phényl)-imidazolidin-1-ylméthyl]-pyridin-2-yl}-thiouréido )-benzoïque

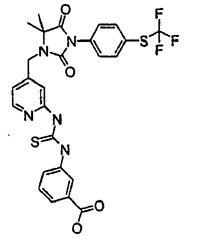

MS (LC-MS) : 589,11 ; Temps dé rétention : 2,30.

La synthèse est décrite dans le schéma 2.

### Exemple 129 : 1-{4-[5,5-diméthyl-2,4-dioxo-3-(4-trifluorométhyl-sulfanyl-phényl)-imidazolidin-1-ylméthyl]-pyridin-2-yl}-3-(2-méthoxy-phényl)-urée

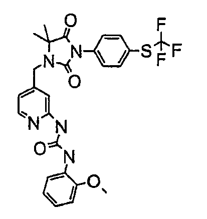

MS (LC-MS) : 559,15 ; Temps de rétention : 1,88.

La synthèse est décrite dans le schéma 2.

### Exemple 130 : 1-(2-amino-pyridin-4-ylméthyl)-5,5-diméthyl-3-(4-trifluorométhylsulfanyl-phényl)-imidazolidine-2,4-dione

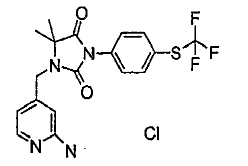

MS (LC-MS) : 410,10 ; Temps de rétention : 1,57.
RMN : 1,4 : s, 6H ; 4,6 : s, 2H ; 6,9 : d, 1H ; 7,0 : s, 1H ; 7,7 : d, 2H ; 7,85 : d, 2H ; 7,95 : d, 1H ; 8,0 : s large, 1H.

La synthèse est décrite dans le schéma 2.

### Exemple 131 : 1-(2,6-dichloro-pyridin-4-yl)-3-{4-[5,5-diméthyl-2,4-dioxo-3-(4-trifluorométhylsulfanyl-phényl)-imida-zolidin-1-ylméthyl]-pyridin-2-yl}-urée

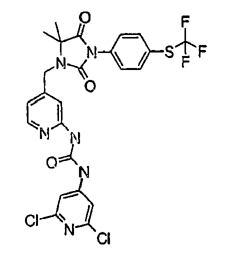

MS (LC-MS) : 599,42 ; Temps de rétention : 2,73 min.
RMN : 1,40, s, 6H ; 4,65 s, 2H ; 7,15 : m, 1H ; 7,65 : d, 2H ; 7,85 : d, 2H ; 8,15 : s, 1H ; 8,25 : m, 1H.

La synthèse est décrite dans le schéma 2.

### Exemple 132 : 1-(2,6-dichloro-phényl)-3-{4-[5,5-diméthyl-2,4-dioxo-3-(4-trifluorométhylsulfanyl-phényl)-imidazolidin-1-ylméthyl]-pyridin-2-yl}-urée

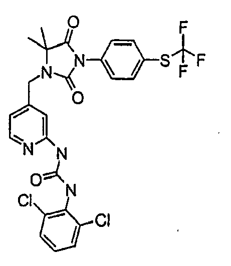

MS (LC-MS) : 597,06 ; Temps de rétention : 2,44 min.
La synthèse est décrite dans le schéma 2.

### Exemple 133 : 1-(2,3-dichloro-phényl)-3-{4-[5,5-diméthyl-2,4-dioxo-3-(4-trifluorométhylsulfanyl-phényl)-imidazolidin-1-ylméthyl]-pyridin-2-yl}-urée

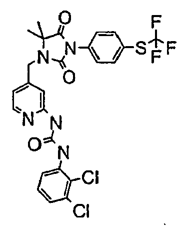

MS (LC-MS) : 597,06 ; Temps de rétention : 2,08 min.

La synthèse est décrite dans le schéma 2.

### Exemple 134 _{:} 1-{4-[5,5-diméthyl-2,4-dioxo-3-(4-trifluorométhyl-sulfanyl-phényl)-imidazolidin-1-ylméthyl]-pyridin-2-yl}-3-pyridin-3-yl-thiourée

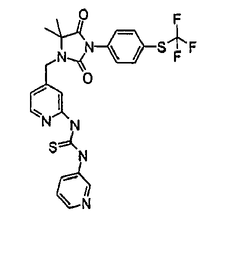

MS (LC-MS) : 546,11 ; Temps de rétention : 1,89 min.

La synthèse est décrite dans le schéma 2.

### Exemple 135 : 1-[2-chloro-6-(4-méthyl-thiazol-2-ylamino)-pyridin-4-ylméthyl]-5,5-diméthyl-3-(4-trifluorométhylsulfanyl-phényl)-imidazolidine-2,4-dione

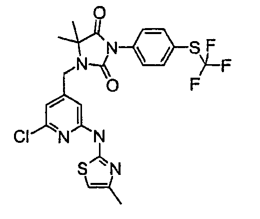

MS (LC-MS) : 541,06 ; Temps de rétention : 2,49 min.

La synthèse est décrite dans le schéma 3.

### Exemple 136 : N-{6-chloro-4-[5,5-diméthyl-2,4-dioxo-3-(4-trifluo-rométhylsulfanyl-phényl)-imidazolidin-1-ylméthyl]-pyridin-2-yl}-2-phényl-butyramide

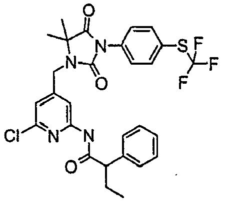

MS (LC-MS) : 541,06 ; Temps de rétention : 2,49 min.

La synthèse est décrite dans le schéma 3.

### Exemple 137 : N-{6-acétylamino-4-[5,5-diméthyl-2,4-dioxo-3-(4-trifluorométhylsulfanyl-phényl)-imidasolidin-1-ylméthyl]-pyridin-2-yl}-acétamide

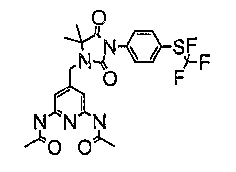

MS (LC-MS) : 509,13 ; Temps de rétention : 1,73 min.

La synthèse est décrite dans le schéma 3.

### Exemple 138 : 1-[2-chloro-6-(4-méthyl-pyridin-2-ylamino)-pyridin-4-ylméthyl]-5,5-diméthyl-3-(4-trifluorométhylsulfanyl-phényl)-imidazolidine-2,4-dione

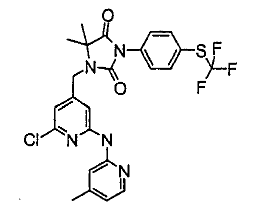

MS (LC-MS) : 535,11 ; Temps de rétention : 1,90 min.

La synthèse est décrite dans le schéma 3.

### Exemple 139 : 1-[2,6-bis-(3-méthoxy-phénylamino)-pyridin-4-yl-méthyl]-5,3-dimethyl-3-(4-trifluorométhoxy-phényl)-imida-zolidine-2,4-dione ; composé avec de l'acide trifluoro-acétique

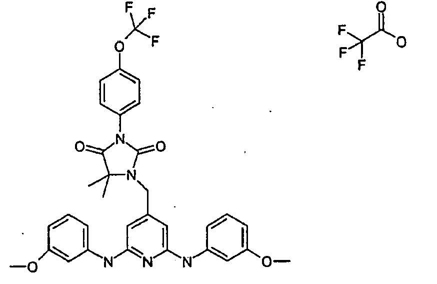

MS (LC-MS) : 621,22 ; Temps de rétention : 1,95 min.

La synthèse est décrite dans le schéma 3.

### Exemple 140 : 1-[2,6-bis-(2,4-diméthoxy-phénylamino)-pyridin-4-ylméthyl]-5,5-diméthyl-3-(4-trifluorométhoxy-phényl)-imidazolidine-2,4-dione ; composé avec de l'acide trifluoroacétique

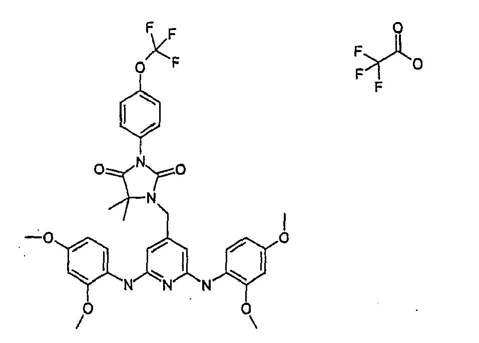

MS (LC-MS) : 681,24 ; Temps de rétention : 1,83 min.

La synthèse est décrite dans le schéma 3.

### Exemple 141 : 1-[2,6-bis-(4-méthoxy-phénylamino)-pyridin-4-yl-méthyl]-5,5-diméthyl-3-(4-trifluorométhoxy-phényl)-imida-zolidine-2,4-dione ; composé avec de l'acide trifluoroacétique

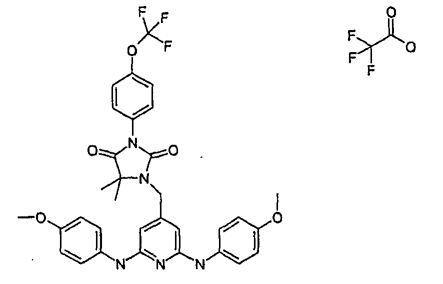

MS (LC-MS) : 621,22 ; Temps de rétention : 1,63 min.

La synthèse est décrite dans le schéma 3.

### Exemple 142 : N-{4-[5,5-diméthyl-2,4-dioxo-3-(4-trifluorométhoxy-phényl)-imidazolidin-1-ylméthyl]-6-estobutyrylamino-pyridin-2-yl}-isobutyramide ; composé avec de l'acide trifluoroacétique

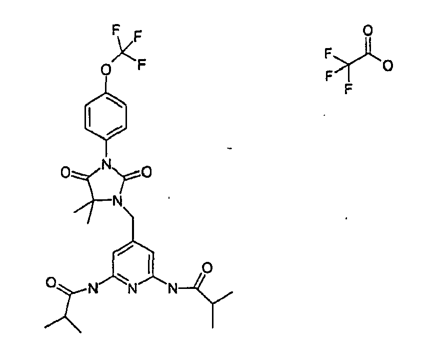

MS (LC-MS) : 549,22 ; Temps de rétention : 1,83 min.

La synthèse est décrite dans le schéma 3.

### Exemple 143 : 1-[2-chloro-6-(pyridin-4-ylamino)-pyridin-4-yl-méthy1]-5,5-diméthyl-3-(4-trifluorométhoxy-phényl)-imida-zolidine-2,4-dione ; composé avec de l'acide trifluoroacétique

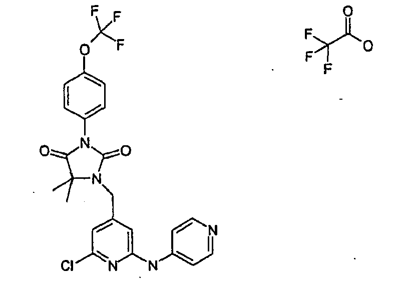

MS (LC-MS) : 563,19 ; Temps de rétention : 2,61 min.

La synthèse est décrite dans le schéma 3.

### Exemple 144 : 1-[2-chloro-6-(pyridin-4-ylamino)-pyridin-4-yl-méthyl]-5,5-diméthyl-3-(4-trifluorométhylsulfanyl-phényl)-imidazolidine-2,4-dione

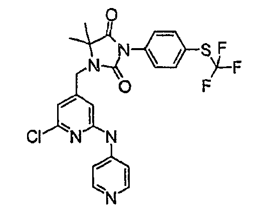

MS (LC-MS) : 521,09 ; Temps de rétention : 1,91 min.

La synthèse est décrite dans le schéma 3.

### Exemple 145 : N-{6-chloro-4-[5,5-diméthyl-2,4-dioxo-3-(4-trifluo-rométhylsulfanyl-phényl)-imidazolidin-1-ylméthyl]-pyr-idin-2-yl}-propionamide

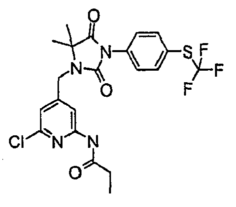

MS (LC-MS) : 500,09 ; Temps de rétention : 2,46.

La synthèse est décrite dans le schéma 3.

### Exemple 146 : N-{6-chloro-4-[5,5-diméthyl-2,4-dioxo-3-(4-trifluo-rométhylsulfanyl-phényl)-imidazolidin-1-ylméthyl]-pyridin-2-yl}-acétamide

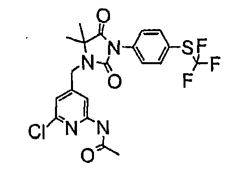

MS (LC-MS) : 486,90 ; Temps de rétention : 2,15.

La synthèse est décrite dans le schéma 3.

### Exemple 147 : 1-[2-chloro-6-(pyridin-3-ylamino)-pyridin-4-yl-méthyl]-5,5-diméthyl-3-(4-trifluorométhoxy-phényl)-imida-zolidine-2,4-dione ; composé avec de l'acide trifluoroacétique

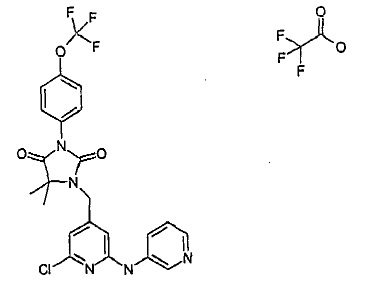

MS (LC-MS) : 505,11 ; Temps de rétention : 2,27.

La synthèse est décrite dans le schéma 3.

### Exemple 148 : {6-chloro-4-[5,5-diméthyl-2,4-dioxo-3-(4-trifluorométhylsulfanyl-phényl)-imidazolidin-1-ylméthyl]-pyridin-2-yl}-amide de l'acide cyclopropanecarboxylique

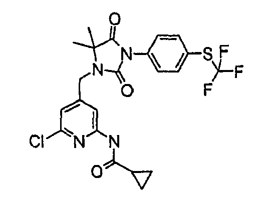

MS (LC-MS) : 512,09 ; Temps de rétention : 2,32.

La synthèse est décrite dans le schéma 3.

### Exemple 149 : N-{6-chloro-4-[5,5-diméthyl-2,4-dioxo-3-(4-tri-fluorométhylsulfanyl-phényl)-imidazolidin-1-ylméthyl]-pyridin-2-yl}-isobutyramide

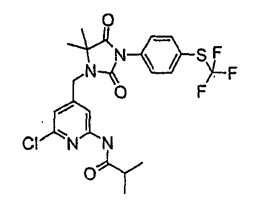

MS (LC-MS) : 514,11 ; Temps de rétention : 1,63.

La synthèse est décrite dans le schéma 3.

### Exemple 150 : 1-[2,6-bis-(pyridin-2-ylamino)-pyridin-4-ylmethyl]-5,5-diméthyl-3-(4-trifluorométhoxy-phényl)-imidazolidine-2,4-dione ; composé avec de l'acide trifluoroacétique

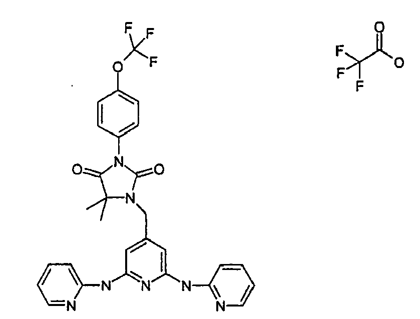

MS (LC-MS) : 563,15 ; Temps de rétention : 1,70 min.

La synthèse est décrite dans le schéma 3.

### Exemple 151 : 1-[2-chloro-6-(pyridin-2-ylamino)-pyridin-4-yl-méthyl]-5,5-diméthyl-3-(4-trifluorométhylsulfanyl-phényl)-imidazolidine-2,4-dione

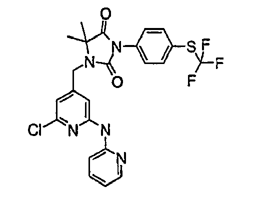

MS (LC-MS) : 521,09 ; Temps de rétention : 1,78 min.

La synthèse est décrite dans le schéma 3.

### Exemple 152 : 5,5-diméthyl-1-(5-phényl-[1,2,4]oxadiazol-3-yl-méthyl)-3-(4-trifluorométhylsulfanyl-phényl)-imidazo-lidine-2,4-dione

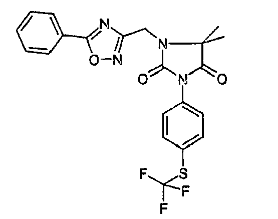

MS (LC-MS) : 462,10 ; Temps de rétention : 2,77 min.

La synthèse est décrite dans le schéma 4.

### Exemple 153 : 1-(2-imidazol-1-yl-éthyl)-5,5-diméthyl-3-(4-tri-fluorométhylsulfanyl-phényl)-imidazolidine-2,4-dione

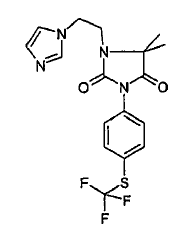

MS (LC-MS) : 398,10 ; Temps de rétention : 1,49 min.

La synthèse est décrite dans le schéma 4.

### Exemple 154 : 1-[2-(4-chloro-phényl)-oxazol-5-ylméthyl]-5,5-di-méthyl-3-(4-trifluorométhylsulfanyl-phényl)-imidazo-lidine-2,4-dione

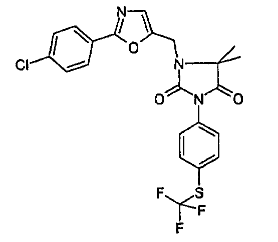

MS (LC-MS) : 495,06 ; Temps de rétention : 2,99 min.

La synthèse est décrite dans le schéma 4.

### Exemple 155 : Préparation de 1-isoquinolin-5-yl-5,5-diméthyl-3-(4-trifluorométhylsulfanyl-phényl)-imidazolidine-2,4-dione

500 mg (2,4 mmol) de 2-(isoquinolin-5-ylamino)-2-méthyl-propionitrile, 1,6 mg (0,013 mmol) d'acide benzoïque et 526 mg (2,4 mmol) de 4-(trifluorométhylthio)-phénylisocyanate dans 4 ml de chlorobenzène ont été chauffés à reflux pendant 2 jours. Le mélange résultant a été filtré, et le filtrat a été évaporé à siccité. L'imine intermédiaire a été séparée par chromatographie HPLC préparative (RP 18, acétonitrile, eau, 0,01 % de TFA). 60 mg du produit résultant ont été agités pendant 1 heure à 40°C avec de l'acide chlorhydrique 1N. Le solvant a été évaporé et le résidu repris dans une solution d'hydrogénocarbonate de sodium et extrait avec de l'acétate d'éthyle. Les phases organiques combinées ont été séchées et le résidu restant après évaporation a été purifié par chromatographie flash (SiO₂, chlorure de méthylène:méthanol = 98:2) conduisant à 38 mg du produit souhaité.
M+H⁺ = 432.
LC/MS Temps de rétention = 1,245.

### Exemple 156 : 5,5-diméthyl-1-(5-phényl-oxazol-4-ylméthyl)-3-(4-trifluorométhylsulfanyl-phényl)-imidazolidine-2,4-dione

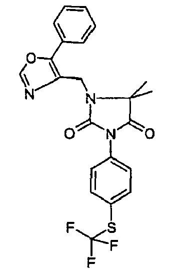

MS (LC-MS) : 461,10, Temps de rétention : 2,63 min.

La synthèse est décrite dans le schéma 4.

### Exemple 157 : 5,5-diméthyl-1-(1-méthyl-piperidin-3-ylméthyl)-3-(4-trifluorométhylsulfanyl-phényl)-imidazoldine-2,4-dione

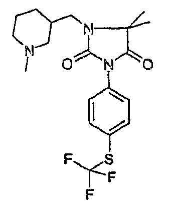

MS (LC-MS) : 415,15 ; Temps de rétention : 1,63.

La synthèse est décrite dans le schéma 4.

### Exemple 158 : 5,5-diméthyl-1-(1-méthyl-piperidin-3-ylméthyl)-3-(4-trifluorométhoxy-phényl)-imidazolidine-2,4-dione

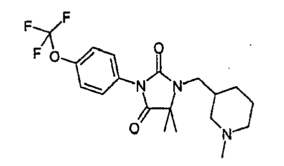

MS (LC-MS) : 499,18 ; Temps de rétention : 1,39.

La synthèse est décrite dans le schéma 4.

### Exemple 159 : 1-[2-(4-chloro-phényl)-thiazol-4-ylméthyl]-5,5-diméthyl-3-(4-trifluorométhylsulfanyl-phényl)-imidazolidine-2,4-dione

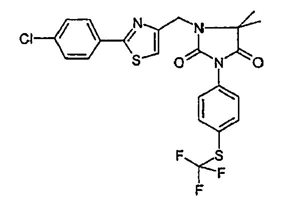

MS (LC-MS) : 511,04 ; Temps de rétention : 3,12.

La synthèse est décrite dans le schéma 4.

### Exemple 160 : 5,5-diméthyl-1-(1-méthyl-1H-imidazol-2-ylméthyl)-3-(4-trifluorométhylsulfanyl-phényl)-imidazolidine-2,4-dione

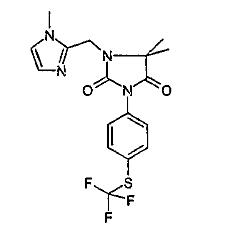

MS (LC-MS) : 398,10 ; Temps de rétention : 1,53.

La synthèse est décrite dans le schéma 4.

### Exemple 161 : 1-(7-méthoxy-2-oxo-2H-chromen-4-ylméthyl)-5,5-di-méthyl-3-(4-trifluorométhylsulfanyl-phényl)-imidazoli-dine-2,4-dione

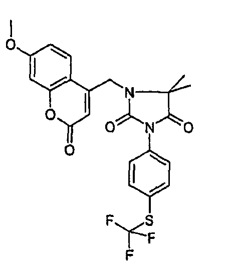

MS (LC-MS) : 492,48 ; Temps de rétention : 1,84.

La synthèse est décrite dans le schéma 4.

### Exemple 162 : 5,5-diméthyl-1-(5-méthyl-estoxazol-3-ylméthyl)-3-(4-trifluorométhylsulfanyl-phényl)-imidazolidine-2,4-dione

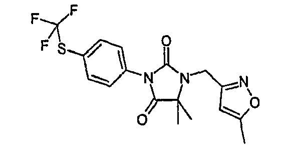

MS (LC-MS) : 500,09 ; Temps de rétention : 2,52.

La synthèse est décrite dans le schéma 4.

### Exemple 163 : 1-[5-(4-méthoxy-phényl)-[1,2,4]oxadiazol-3-yl-méthyl]-5,5-diméthyl-3-(4-trifluorométhylsulfanyl-phényl)-imidazolidine-2,4-dione

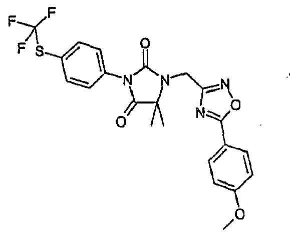

MS (LC-MS) : 492,11 ; Temps de rétention : 1,87.

La synthèse est décrite dans le schéma 4.

### Exemple 164 : 1-[5-(4-méthoxy-phényl)-[1,2,4]oxadiazol-3-yl-méthyl]-5,5-diméthyl-3-(4-trifluorométhoxy-phényl)-imida-zolidine-2,4-dione

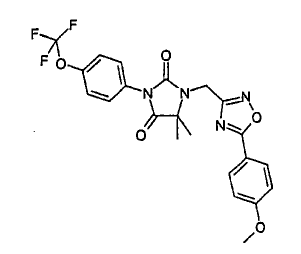

MS (LC-MS) : 476,13 ; Temps de rétention : 2,03.

La synthèse est décrite dans le schéma 4.

### Exemple 165 : Ester méthylique de l'acide 5-[5,5-diméthyl-2,4-dioxo-3-(4-trifluorométhylsulfanyl-phényl)-imidasolidin-1-ylméthyl]-furan-2-carboxylique

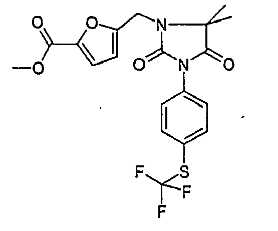

MS (LC-MS) : 442,42 ; Temps de rétention : 1,74.

La synthèse est décrite dans le schéma 4.

### Exemple 166 : 1-(1-benzyl-1H-imidazol-2-ylméthyl)-5,5-diméthyl-3-(4-trifluorométhylsulfanyl-phényl)-imidazolidine-2,4-dione

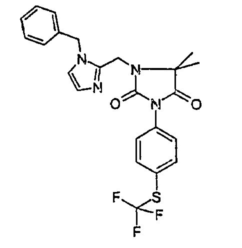

MS (LC-MS) : 474,13 ; Temps de rétention : 2,02.

La synthèse est décrite dans le schéma 4.

### Exemple 167 : 1-(4-diméthylamino-pyrimidin-2-ylméthyl)-5,5-di-méthyl-3-(4-trifluorométhylsulfanyl-phényl)-imidazo-lidine-2,4-dione

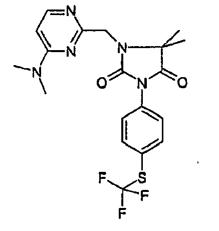

MS (LC-MS) : 439,46 ; Temps de rétention : 1,91.

La synthèse est décrite dans le schéma 4.

### Exemple 168 : 1-[1-(4-méthoxy-benzyl)-1H-imidazol-2-ylméthyl]-5,5-diméthyl-3-(4-trifluorométhylsulfanyl-phényl)-imidazoli-dine-2,4-dione

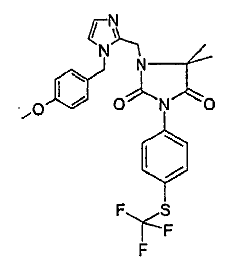

MS (LC-MS) : 504,14 ; Temps de rétention : 2,24.

La synthèse est décrite dans le schéma 4.

### Exemple 169 : 5,5-diméthyl-1-[2-(1-méthyl-pyrrolidin-2-yl)-éthyl]-3-(4-trifluoromethylsulfanyl-phényl)-imidanzolidine-2,4-dione

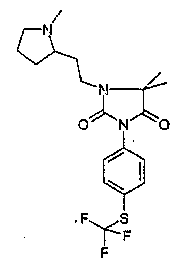

MS (LC-MS) : 415,15 ; Temps de rétention : 1,66 min.

La synthèse est décrite dans le schéma 4.

### Exemple 170 : 5,5-diméthyl-1-(2-morpholin-4-yl-éthyl)-3-(4-trifluorométhylsulfanyl-phinyl)-imidazolidine-2,4-dione

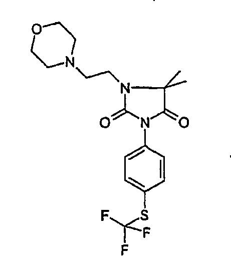

MS (LC-MS) : 417,13 ; Temps de rétention : 1,62 min.

La synthèse est décrite dans le schéma 4.

### Exemple 171 : 1-[5-(2-méthoxy-phényl)-[1,2,4]oxadiazol-3-yl-méthyl]-5,5-diméthyl-3-(4-trifluorométhylsulfanyl-phényl)-imidazolidine-2,4-dione

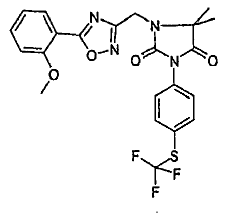

MS (LC-MS) : 492,11 ; Temps de rétention : 2,67 min.

La synthèse est décrite dans le schéma 4.

### Example 172 : 5,5-diméthyl-1-(5-méthyl-estoxazol-3-ylméthyl)-3-(4-trifluoromethoxy-phényl)-imidazolidine-2,4-dione

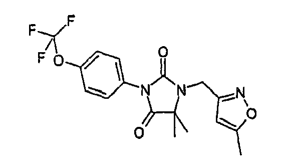

MS (LC-MS) : 383,33 ; Temps de rétention : 1,95 min.

La synthèse est décrite dans le schéma 4.

### Exemple 173 : 1-(5-tert-butyl-[1,2,4]-oxadiazol-3-ylméthyl)-5,5-diméthyl-3-(4-trifluorométhylsulfanyl-phényl)-imidazo-lidine-2,4-dione

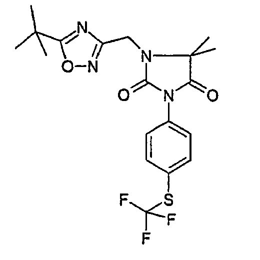

MS (LC-MS) : 442,16 ; Temps de rétention : 2,64 min.

La synthèse est décrite dans le schéma 4.

### Exemple 174 : 4-méthyl-benzylamide de l'acide 4-[5,5-diméthyl-2,4-dioxo-3-(4-trifluorométhyl-sulfanyl-phényl)-imidazolidin-1-ylméthyl]-pyridine-2-carboxylique ; composé avec de l'acide trifluoroacétique

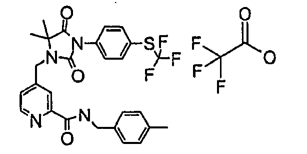

MS (LC-MS) : 542,16 ; Temps de rétention : 2,80 min.

La synthèse est décrite dans le schéma 5.

### Exemple 175 : (2-p-tolyl-éthyl)-amide de l'acide 4-[5,5-diméthyl-2,4-dioxo-3-(4-trifluorométhyl-sulfanyl-phényl)-imidazo-lidin-1-ylméthyl]-pyridine-2-carboxylique

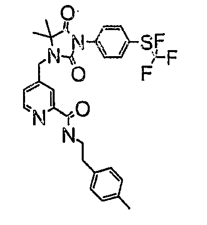

MS (LC-MS) : 556,18 ; Temps de rétention : 2,31.

La synthèse est décrite dans le schéma 5.

### Exemple 176 : 4-[5,5-diméthyl-2,4-dioxo-3-(4-trifluorométhyl-sulfanyl-phényl)-imidazolidin-1-ylméthyl]-pyridine-2-carboxylique acid 3,4-diméthoxy-benzylamide

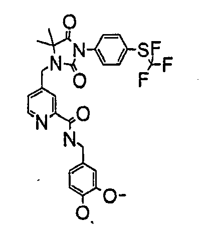

MS (LC-MS) : 588,16 ; Temps de rétention : 1,58.

La synthèse est décrite dans le schéma 5.

### Exemple 177 : Benzylamide de l'acide 4-[5,5-diméthyl-2,4-dioxo-3-(4-trifluorométhyl-sulfanyl-phényl)-imidazolidin-1-yl-méthyl]-pyridine-2-carboxylique

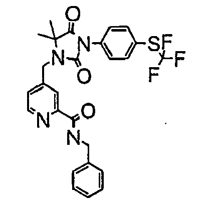

MS (LC-MS) : 528,14 ; Temps de rétention : 1,77.

La synthèse est décrite dans le schéma 5.

### Exemple 178 : 5,5-diméthyl-1-[2-(4-oxo-2-thioxo-1,4-dihydro-2H-quinazolin-3-yl)-pyridin-4-ylméthyl]-3-(4-trfiluoro-méthylsulfanyl-phényl)-imdazolidine-2,4-dione

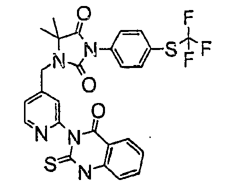

MS (LC-MS) : 571,10 ; Temps de rétention : 2,3.

La synthèse est décrite dans le schéma 6

Les deux schémas réactionnels ci-après décrivent la synthèse de produits de formule (I) selon la présente invention, notamment parmi les produits des exemples 178 à 200 ci-après.

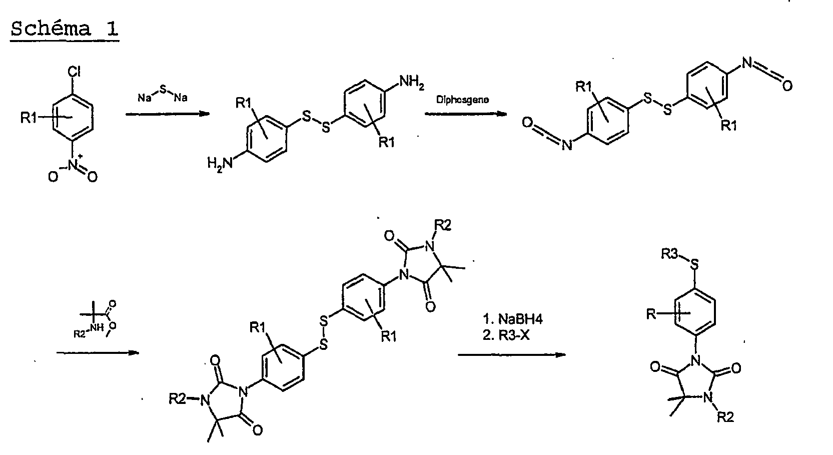

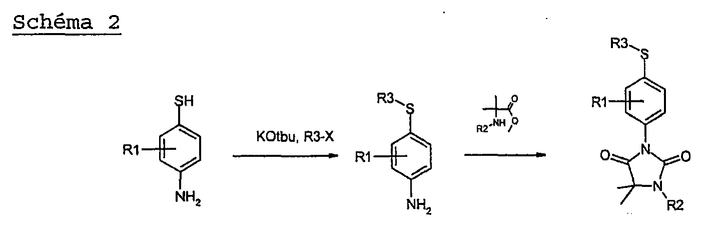

### Bis-(4-amino-2-chlorophényl)-disulfide

76,8 g (0,4 mol) de 1,2-dichoro-4-nitrobenzène, mis en suspension dans 120 ml d'eau ont été chauffés à 90°C et traités avec une solution de 62,4 g (0,8 mol) de sulfure de sodium et de 12,8 g (0,4 mol) de soufre dans 200 ml d'eau.

Après avoir chauffé le mélange à reflux pendant 5 heures, 6 g de dioxyde de carbone a été fait buller à travers la solution, suivi d'un courant d'air régulier. Le pH a été ajusté à 5,5, le mélange a été refroidi à température ambiante et le précipité résultant a été collecté par filtration.

Le matériau brut a été recristallisé dans de l'isopropanol, pour conduire à 45 g (71 %) du produit souhaité.
M+H⁺ = 318.
LC/MS Temps de rétention = 1,526.

### Bis-[(2-Chloro-4-(4,4-diméthyl-2,5-dioxo-3-pyridin-4-yl-méthyl-imidazolidin-1-yl)]-phényldisulfide

Une solution de 13,7 g (69,5 mmol) de diphosgène dans 100 ml de toluène a été refroidie à -20°C et traitée avec une solution de 5,0 g (15,8 mmol) de bis-(4-amino-2-chlorophényl)-disulfide. Le mélange résultant a été agité pendant 30 minutes à température ambiante, chauffé à reflux pendant 1 heure et ensuite évaporé à siccité.

2,53 g (ca. 6,86 mmol) de ce matériau ont été dissous dans du THF et traités avec 2,0 g (9,5 mmol) d'ester méthylique de l'acide 2-méthyl-2-[(pyridin-4-ylméthyl)-amino]-propionique. Le mélange a été agité pendant 3 heures à température ambiante, évaporé à siccité et le solide restant a été purifié par chromatographie flash (Si0₂, chlorure de méthylène: méthanol = 97:3) pour conduire à 1,8 g (37 %) du produit souhaité.
M+H⁺ = 722.
LC/MS Temps de rétention = 1,176.

### Exemple 179 : Préparation de 3-(4-tert-butylsulfanyl-3-chloro-phényl)-5,5-diméthyl-1-pyridin-4-ylméthyl-imidazolidine-2,4-dione trifluoroacétate

200 mg (0,28 mol) de bis-[(2-chloro-4-(4,4-diméthyl-2,5-dioxo-3-pyridin-4-ylméthyl-imidazolidin-1-yl)]-phényldisulfide ont été dissous dans 10 ml de méthanol et traités avec 22 mg (0,56 mmol) de borohydrure de sodium. Après 30 minutes d'agitation à température ambiante, le mélange a été évaporé à siccité. Le résidu a été dissous dans 10 ml d'acide sulfurique (75 %) et ajouté dans 20 ml d'acide sulfurique (75 %) qui ont été saturés avec de l'isobutylène. Le mélange a été agité pendant 20 minutes à température ambiante et ensuite ajouté avec précautions à une solution refroidie d'hydroxyde de sodium dans l'eau. La phase aqueuse alcaline a été extraite trois fois avec de l'acétate d'éthyle, les phases organiques combinées ont été séchées avec du sulfate de sodium et le matériau restant après évaporation a été filtré sur gel de silice (chlorure de méthylène: méthanol = 95:5). Le produit brut résultant a été purifié par chromatographie HPLC préparative (RP 18, acétonitrile, eau, 0,01 % de TFA) conduisant à 120 mg (81 %) du produit souhaité.
M+H⁺ = 418.
LC/MS Temps de rétention = 1,249.

### Procédure générale 1A : Préparation de 3-(3-Chloro-4-alkylsulfanyl-phényl)-5,5-diméthyl-1-pyridin-4-ylméthyl-imidazolidine- 2,4-diones

445 mg (0,62 mol) de bis-[(2-chloro-4-(4,4-diméthyl-2,5-dioxo-3-pyridin-4-ylméthyl-imidazolidin-1-yl)]-phényldisulfide ont été dissous dans 15 ml de méthanol et traités avec 49 mg (1,24 mmol) de borohydrure de sodium. Après 30 minutes d'agitation à température ambiante, 1,24 mmol de l'halogénure d'alkyle respectif ont été ajoutés et le mélange résultant a été chauffé à reflux pendant 1 heure. Le solvant a été éliminé par évaporation et les matériaux bruts restants ont été purifiés par chromatographie HPLC préparative (RP 18, acétonitrile, eau, 0,01 % de TFA)

### Exemple 180 : Préparation de 3-(3-Chloro-4-isopropyl-sulfanyl-phényl)-5,5-diméthyl-1-pyridin-4-ylmithyl-imidazolidine- 2,4-dione

Synthétisé selon la procédure générale 1A.
M+H⁺ = 404.
LC/MS Temps de rétention = 1,196.

### Exemple 181 : Préparation de 3-(3-chloro-4-isobutyl-sulfanyl-phényl)-5,5-diméthyl-1-pyridin-4-ylméthyl-imidazolidine- 2,4-dione trifluoroacétate

Préparé selon la procédure générale 1A.
M+H⁺ = 418.
LC/MS Temps de rétention = 1,272.

### Exemple 182 : Préparation de 3-(3-chloro-4-méthylsulfanyl-phényl)-5,5-diméthyl-1-pyridin-4-ylméthyl-imidazolidine-2,4-dione

Préparé selon la procédure générale 1A.
M+H⁺ = 376.
LC/MS Temps de rétention = 1,054.

### Exemple 183 : Préparation de trifluoroacétate de 3-[3-chloro-4-(3-méthoxy-propylsulfanyl)-phényl]-5,5-diméthyl-1-pyridin-4-yl-méthyl-imidazolidine-2,4-dione

Préparé selon la procédure générale 1A.
M+H⁺ = 434.
LC/MS Temps de rétention = 1,096.

### Exemple 184 : Préparation de trifluoroacétate de 3-[3-chloro-4-(2-morpholin-4-yl-éthylsulfanyl)-phényl]-5,5-diméthyl-1-pyridin-4-ylméthyl- imidazolidine-2,4-dione

Préparé selon la procédure générale 1A.
M+H⁺ = 475.
LC/MS Temps de rétention = 0,792

### Exemple 185 : Préparation de trifluoroacétate de 3-{3-chloro-4-[2-(1-méthyl-pyrrolidin-2-yl)-éthylsulfanyl]-phényl}-5,5-diméthyl-1-pyridin- 4-ylméthyl-imidazolidine-2,4-dione

Préparé selon la procédure générale 1A.
M+H⁺ = 473.
LC/MS Temps de rétention = 0,865.

### Exemple 186 : Préparation de trifluoroacétate de 3-{3-chloro-4-[3-(4-méthyl-piperazin-1-yl)-propylsulfanyl]-phényl}-5,5-diméthyl-1-pyridin- 4-ylméthyl-imidazolidine-2,4-dione

Préparé selon la procédure générale 1A.
M+H⁺ = 502.
LC/MS Temps de rétention = 0,795.

### Exemple 187 : Préparation de trifluoroacétate de 3-[3-chloro-4-(3-hydroxy-propylsulfanyl)-phényl]-5,5-diméthyl-1-pyridin-4-ylméthyl- imidazolidine-2,4-dione

Préparé selon la procédure générale 1A.
M+H⁺ = 420.
LC/MS Temps de rétention = 0,968.

### Exemple 188 : Préparation de trifluoroacétate de 3-[3-chloro-4-(2-hydroxy-éthylsulfanyl)-phinyl]-5,5-diméthyl-1-pyridin-4-ylméthyl- imidazolidine-2,4-dione

Préparé selon la procédure générale 1A.
M+H⁺ = 406.
LC/MS Temps de rétention = 0,921.

### Exemple 189 : Préparation de trifluoroacétate de [2-chloro-4-(4,4-diméthyl-2,5-dioxo-3-pyridin-4-ylméthyl-imidazolidin-1-yl)-phénylsulfanyl]-acétonitrile

Préparé selon la procédure générale 1A.
M+H⁺ = 401.
LC/MS Temps de rétention = 0,999.

### 3-nitro-4-trifluorométhoxy-aniline

20 g (112,9 mmol) de 4-trifluorométhoxy-aniline ont été dissous dans 50 ml d'acide sulfurique concentré à une température de 0 à 5°C traité avec 30 ml d'un mélange d'acide sulfurique et d'acide nitrique a 4:1. Le mélange a été agité pendant 5 heures à 0°C, et ensuite versé sur un mélange glace-eau et rendu alcalin avec 200 ml de solution d'ammoniaque concentrée. L'extraction avec EE, le séchage avec du sulfate de sodium, l'évaporation à siccité et la recristallisation dans de l'acétate d'éthyle/heptane a conduit à 15,5 g (63 %) du produit souhaité.
M+H⁺ = 223.
LC/MS Temps de rétention = 1,378.

### Exemple 190 : Préparation de l'acide trifluoroacétique ; 5,5-diméthyl-3-(3-nitro-4-trifluorométhoxy-phényl)-1-pyridin-4-ylméthyl-imidazolidine-2,4-dione

2,27 g (11,5 mmol) de diphosgène dans du 1,2-dichloroéthane ont été ajoutés à -20°C dans 1,0 g (4,5 mmol) de 3-nitro-4-trifluorométhoxy-aniline, dissous dans 20 ml de 1,2-dichloroéthane. Le mélange a été agité pendant 1 heure tout en étant laissé atteindre la température ambiante et est ensuite chauffé à 50°C pendant 2 heures. Après avoir reposé toute une nuit, le solvant a été évaporé, le résidu a été repris avec du THF sec et traité avec 937 mg (4,5 mmol) d'ester méthylique de l'acide 2-méthyl-2-[(pyridin-4-ylméthyl)-amino]-propionique. Le mélange a été agité pendant 2 heures à température ambiante et pendant. 1 heure à 40°C, puis évaporé à siccité et le résidu restant a été purifié (RP 18, acétonitrile, eau, 0,01 % de TFA) pour conduire à 1,15 g (61 %) du produit souhaité.
M+H⁺ = 425.
LC/MS Temps de rétention = 1,324.

### Exemple 191 : Préparation de 3-(3-amino-4-trifluorométhoxy-phényl)-5,5-diméthyl-1-pyridin-4-ylméthyl-imidazolidine-2,4-dione

9,63 g (22,7 mmol) de trifluoroacétate de 5,5-diméthyl-3-(3-nitro-4-trifluorométhoxy-phényl)-1-pyridin-4-ylméthyl-imidazoli-dine-2,4-dione ont été dissous dans de l'acide chlorhydrique semi-concentré, chauffé à reflux et traité avec 30 g de poussière de zinc. Après 1,5 heures le mélange a été refroidi, filtré et extrait deux fois avec de l'éther méthyl-tert-butylique. La phase aqueuse a été rendue alcaline avec de l'hydroxyde de sodium 6N, extraite avec de l'êther méthyl-tert-butylique et les phases organiques combinées ont été séchées avec du sulfate de sodium. L'évaporation du solvant a conduit à 5,0 g (56 %) du produit souhaité.
M+H⁺ = 395.
LC/MS Temps de rétention = 0,948.

### Exemple 192 : Préparation de 5,5-Diméthyl-1-pyridin-4-yl-méthyl-3-[4-(2,2,2-trifluoro-ethoxy)-phényl]-imidazolidine-2,4-dione

688 mg (3,6 mmol) de 2,2,2-trifluoro-éthoxy-aniline dans 30 ml de chlorure de méthylène ont été ajoutés à 0°C 356 mg (3,6 mmol) de triéthylamine et une solution de 356 mg (1,2 mmol) de triphosgène dans 30 ml de chlorure de méthylène. Le mélange a été agité toute une nuit tout en étant laissé atteindre la température ambiante. Par la suite, le solvant a été évaporé, le résidu a été repris dans 15 ml de THF et traité avec 100 mg (0,48 mmol) d'ester méthylique de l'acide 2-méthyl-2-[(pyridin-4-ylméthyl)-amino]-propionique et 48,5 mg (0,48 mmol) de triéthylamine. Le mélange a été agité 4 heures à température ambiante et 1 heure à 50°C, puis évaporé à siccité et le résidu restant a été purifié par chromatographie HPLC préparative (RP 18, acétonitrile, eau, 0,01 % de TFA) pour conduire à 67 g (36 %) du produit souhaité.
M+H⁺ = 394.
LC/MS Temps de rétention = 1,032.

### Exemple 193 : Préparation de 3-(8-Chloro-3,4,4-triméthyl-thiochroman-6-yl)-5,5-diméthyl-1-pyridin-4-ylméthyl-imidazolidine-2,4-dione

200 mg (0,28 mol) de bis-[(2-chloro-4-(4,4-diméthyl-2,5-dioxo-3-pyridin-4-ylméthyl-imidazolidin-1-yl)]-phényldisulfide ont été dissous dans 10 ml de méthanol et traités avec 22 mg (0,56 mmol) de borohydrure de sodium. Après 30 minutes d'agitation à température ambiante, le mélange a été évaporé à siccité. Le résidu a été dissous dans 10 ml d'acide sulfurique (75 %) et donné dans 20 ml d'acide sulfurique (75 %) qui ont été saturés avec de l'isobutylène. Le mélange a été agité pendant 30 minutes à 40°C pendant qu'un courant d'air régulier d'isobutylène a été fait buller au travers de la solution. Après avoir reposé toute une nuit, le mélange a été ajouté avec précaution à une solution refroidie d'hydroxyde de sodium dans l'eau. La phase aqueuse alcaline a été extraite trois fois avec de l'acétate d'éthyle, les phases organiques combinées ont été séchées avec du sulfate de sodium et le matériau restant après évaporation a été filtré sur gel de silice (chlorure de méthylène: méthanol = 95:5). Le produit brut résultant a été purifié par chromatographie HPLC préparative (RP 18, acétonitrile, eau, 0,01 % de TFA) conduisant à 70 mg (56 %) du produit souhaité.
M+H⁺ = 444.
LC/MS Temps de rétention = 1,240.

### Exemple 194 : Préparation de trifluoroaoétate de 1-(2-chloro-thiazol-5-yl-méthyl)-5,5-diméthyl-3-(4-trifluorométhylsulfanyl-phényl)-imidazolidine-2,4-dione

200 mg (0,65 mmol) de 5,5-diméthyl-3-(4-trifluorométhylsulfanyl-phényl)-imidazolidine-2,4-dione; 115 mg (0,82 mmol) de carbonate de potassium et 276 mg (1,64 mmol) de 2-chloro-5-chlorométhyl-thiazole ont été dissous dans 2 ml de DMF et agités à température ambiante pendant 2 jours. Le mélange a été versé sur de l'eau, extrait trois fois avec de l'acétate d'éthyle, les phases organiques combinées ont été séchées avec du sulfate de sodium et le matériau restant après évaporation a été purifié par chromatographie HPLC préparative (RP 18, acétonitrile, eau, 0,01% de TFA) conduisant à 143 mg (50 %) du produit souhaité.
M+H⁺ = 436.
LC/MS Temps de rétention = 1,784.

### Exemple 195 : Préparation de 3-[3-chloro-4-(propane-2-sulfonyl)-phényl]-5,5-diméthyl-1-pyridin-4-ylméthyl-imidazolidine-2,4-dione

40 mg (0,1 mmol) de 3-(3-chloro-4-isopropylsulfanylphényl)-5,5-diméthyl-1-pyridin-4-ylméthyl-imidazolidine-2,4-dione ont été dissous dans 2 ml de chlorure de méthylène et traités avec une solution de 37 mg (0,15 mmol) d'acide m-chloroperbenzoïque dans 2 ml de chlorure de méthylène. La réaction a été contrôlée par TLC et stoppée quand le sulfoxyde et le sulfone ont été formés en quantités égales. Le mélange a été évaporé à siccité et purifié par chromatographie flash (Si0₂, chlorure de méthylène:méthanol = 98:2) conduisant à 4 mg 3-[3-chloro-4-(propane-2-sulfonyl)-phényl]-5,5-diméthyl-1-pyridin-4-ylméthyl-imidazolidine-2,4-dione et 7 mg de 3-[3-chloro-4-(propane-2-sulfinyl)-phényl]-5,5-diméthyl-1-pyridin-4-ylméthyl-imidazolidine-2,4-dione
M+H⁺ = 436.
LC/MS Temps de rétention = 0,970.

### Exemple 196 : Préparation de 3-[3-chloro-4-(propane-2-sulfinyl)-phényl]-5,5-diméthyl-1-pyridin-4-ylméthyl-imidazolidine-2,4-dione

Préparation comme décrite ci-dessus.
M+H⁺ = 420.
LC/MS Temps de rétention = 0,932.

### 2-(isoquinolin-5-ylamino)-2-mêthyl-propionitrile :

5,0 g (34,7 mmol) de 5-aminoisoquinoline, 5,1 ml (69,4 mmol) d'acétone, et 945 mg (6,94 mmol) de chlorure de zinc ont été dissous dans 100 ml d'acétonitrile et traités à 0°C avec 6,9 g (69,4 mmol) de cyanure de triméthylsilyl. Le mélange a été chauffé à reflux pendant 3 heures, puis le solvant a été évaporé, et le résidu repris dans 200 ml d'hydrogénocarbonate de sodium et extraits 3 fois avec de l'acétate d'éthyle. Les phases organiques combinées ont été séchées et le résidu restant après évaporation a été purifié par chromatographie flash (Si0₂, chlorure de méthylène:méthanol = 95:5) conduisant à 6,0 g (82 %) du produit souhaité.
M+H⁺ = 212.
LC/MS Temps de rétention = 0,696.

### Procédure générale 2 : synthèse d'alkylsulfanylanilines.

1,1 g (8 mmol) de 4-aminothiophénol et 896 mg (8 mmol) de tert-butylate de potassium ont été dissous dans 10 ml de DMF et agités pendant 45 minutes sous une atmosphère d'argon. Ensuite 8,8 mmol du bromure d'alkyle correspondant ont été ajoutés, le mélange a été agité pendant 3 heures à température ambiante, versé sur de l'eau et extrait avec de l'acétate d'éthyle. Les phases organiques combinées ont été séchées et évaporées. Le produit résiduel a été essentiellement pur et a pu être utilisé sans purification supplémentaire.

### 4-(3-méthoxy-propylsulfanyl)-phénylamine

Préparé selon la procédure générale 2.
M+H⁺ = 197.
LC/MS Temps de rétention = 0,777.

### 4-isopropylsulfanyl-phénylamine

Préparé selon la procédure générale 2.
M+H⁺ = 266.
LC/MS Temps de rétention = 0,173.

### 4-[3-(4-méthyl-piperazin-1-yl)-propylsulfanyl]-phényl-amine

Préparé selon la procédure générale 2.
M+H⁺ = 168.
LC/MS Temps de rétention = 0,894.

### Procédure générale 3 : Préparation de 3-(3-chloro-4-alkylsulfanyl-phényl)-5,5-diméthyl-1-pyridin-4-ylméthyl-imidazolidine- 2,4-diones.

371 mg (2,3 mmol) de carbonyldiimidazole, 41 mg (0,5 mmol) d'imidazolè et 1,9 mmol de l'alkylsulfanylaniline respective ont été dissous à 0°C dans 10 ml de THF et agités pendant 1 heure. Ensuite 280 mg (1,35 mmol) d'ester méthylique de l'acide 2-méthyl-2-[(pyridin-4-ylmêthyl)-amino]-propionique dissous dans 5 ml de THF a été ajouté et le mélange a été chauffé à reflux pendant 5 heures. Par la suite, le solvant a été évaporé et le matériau restant après évaporation a été purifié par chromatographie HPLC préparative (RP 18, acétonitrile, eau, 0,01 % de TFA).

### Exemple 197 : Préparation de trifluoroacétate de 5,5-diméthyl-3-(4-méthylsulfanyl-phényl)-1-pyridin-4-yl-méthyl-imidazolidine-2,4-dione

Préparé selon la procédure générale 3.
M+H⁺ = 342.
LC/MS Temps de rétention = 0,942.

### Exemple 198 : Préparation de trifluoroacétate de 3-[4-(3-méthoxy-propylsulfanyl)-phényl]-5,5-diméthyl-1-pyridin-4-ylméthyl-imidazolidine-2,4-dione

Préparé selon la procédure générale 3.
M+H⁺ = 400.
LC/MS Temps de rétention = 1,007.

### Exemple 199 : Préparation de trifluoroacétate de 3-(4-isopropylsulfanyl-phényl)-5,5-diméthyl-1-pyridin-4-yl-méthyl-imidazolidine-2,4-dione

Préparé selon la procédure générale 3.
M+H⁺ = 370.
LC/MS Temps de rétention =1,096.

### Exemple 200 : Préparation de trifluoroacétate de 5,5-diméthyl-3-{4-[3-(4-méthyl-piperazin-1-yl)-propylsulfanyl]-phényl}-1-pyridin-4-ylméthyl-imidazolidine-2,4-dione

Préparé selon la procédure générale 3.
M+H⁺ = 468.
LC/MS Temps de rétention = 0,689

### Exemple 201 : 5,5-diméthyl-1-(6-méthoxy-quinolin-4-ylméthyl)-3-(4-trifluorométhoxy-phényl)-imidazolidine-2,4-dione

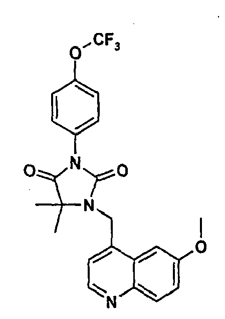

A une solution de 0,65 g de 5,5-diméthyl-3-(4-trifluorométhoxy-phényl)-imidazolidine-2,4-dione dans 40 ml de THF anhydre, sous atmosphère inerte d'argon à une température voisine de 20°C, est ajouté 0,1 g d'hydrure de sodium (à 60 %). L'agitation est maintenue à cette température pendant 30 minutes. On ajoute 0,47 g de 4-chlorométhyl-6-méthoxyquinoléine solubilisé dans 10 ml de THF. Le milieu réactionnel est chauffé à reflux pendant 16 heures. Après refroidissement, 100 ml d'eau et 75 ml d'acétate d'éthyle sont additionnés. Après décantation, la phase organique est séchée sur sulfate de sodium, filtrée puis concentrée sous pression réduite. L'huile brune obtenue est purifiée par flash-chromatographie (SiO2, AcOEt/cyclohexane, 50/50 en volume comme éluant, Ar). Les fractions contenant le produit sont concentrées sous pression réduite. On obtient ainsi 0,36 g de 5,5-diméthyl-1-(6-méthoxy-quinolin-4-ylméthyl)-3-(4-trifluorométhoxy-phényl)-imidazolidine-2,4-dione dont les caractéristiques sont les suivantes :
Spectre de R.M.N. ¹H (300 MHz, (CD₃)₂SO d6, δ en ppm) : 1,45 (s : 6H) ; 3,96 (s : 3H) ; 5,10 (s large : 2H) ; 7,46 (dd, J = 9 et 3 Hz : 1H) ; 7,54 (d, J = 3 Hz : 1H) ; 7,56 (d large, J = 9 Hz : 2H) ; 7,61 (d, J = 4,5 Hz : 1H) ; 7,68 (d large, J = 9 Ha : 2H) ; 8,00 (d, J = 9 Hz : 1H) ; 8,72 (d, J = 4,5 Hz : 1H) .

| | |
|---|---|
| Masse IE m/z=459 M+. pic de base | |
| m/z=213 | C₁₂H₉N₂O₂⁺ |
| m/z=172 | C₁₁H₁₀NO⁺ |

Le composé 5,5-diméthyl-3-(4-trifluorométhoxy-phényl)-imidazolidine-2,4-dione est préparé selon le procédé décrit dans l'exemple 63.

### Préparation de 4-chlorométhyl-6-méthoxyquinoléine

A une solution de 1,2 g de 4-hydroxyméthyl-6-méthoxyquinoléine dans 45 ml de dichlorométhane, sous atmosphère inerte d'argon à une température voisine de 20°C, est ajouté successivement, 1,16 ml de triéthylamine et 0.64 ml de chlorure de méthanesulphonyle. L'agitation est maintenue à cette température pendant 3 heures. Le milieu réactionnel est concentré sous pression réduite pour donner un résidu brun. Le produit est utilisé tel quel dans l'étape suivante.

Spectre R.M.N. ¹H (300 MHz, (CD₃)₂SO d6, δ en ppm) : 3,97 (s : 3H) ; 5,30 (s : 2H) ; 7,48 (dd, J = 9 et 3 Hz : 1H) ; 7,52 (d, J = 3 Hz : 1H) ; 7,62 (d, J = 4,5 Hz : 1H) ; 8,00 (d, J = 9 Hz : 1H) ; 8,75 (d, J = 4,5 Hz : 1H).

| | | |
|---|---|---|
| Masse IE m/z=207 | M⁺ | pic de base |
| m/z=172 | (M - Cl)⁺ | |
| m/z=157 | (m/z=172 - CH₃)⁺ | |
| m/z=129 | (m/z=157 - CO)⁺ | |

### Préparation de 4-hydroxyméthyl-6-méthoxyquinoléine

A une solution de 4 g de 4-éthoxycarbonyl-6-méthoxyquinoléine dans 100 ml de THF anhydre, sous atmosphère inerte d'argon à une température voisine de 5°C, est ajouté goutte à goutte, 17 ml d'une solution 1M de LiAlH4 dans le THF. Le milieu réactionnel est maintenu sous agitation pendant 16 heures à une température voisine de 20°C. 50 ml d'eau et 50 ml d'acétate d'éthyle sont additionnés. Après décantation, la phase organique est séchée sur sulfate de sodium, filtrée puis concentrée sous pression réduite. Le meringue obtenu est purifiée par flash-chromatographie (Si02, AcOEt comme éluant, Ar). Les fractions contenant le produit sont concentrées sous pression réduite. On obtient ainsi 0,86 g de 4-hydroxyméthyl-6-méthoxyquinoléine dont les caractéristiques sont les suivantes :
Spectre de R.M.N. : ¹H (300 MHz, (CD₃)₂SO d6, δ en ppm) : 3,93 (s : 3H) ; 5,02 (d, J = 5,5 Ha : 2H) ; 5,57 (t, J = 5,5 Ha : 1H) ; 7,30 (d, J = 3 Ha : 1H) ; 7,38 (dd, J = 9 et 3 Hz : 1H) ; 7,50 (d, J = 4,5 Ha : 1H) ; 7,92 (d, J = 9 Hz : 1H) ; 8,69 (d, J = 4,5 Hz : 1H).

| | | |
|---|---|---|
| Masse IE, | m/z=189 | M⁺ pic de base |
| m/z=174 | (M CH-₃)⁺ | |
| m/z=160 | (M - CHO)⁺ | |
| m/z=146 | (m/z=174 - CO)⁺ | |
| m/z=117 | (m/z=146 - CHO)⁺ | |

### Exemple 202 : 5,5-diméthyl-1-(6-hydroxy-quinolin-4-ylméthyl)-3-(4-trifluorométhoxy-phényl)-imidazolidine-2,4-dione

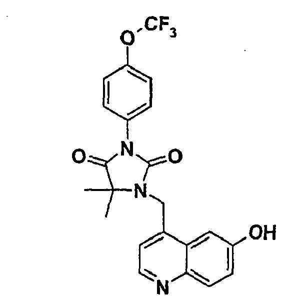

A une solution de 0,26 g de 5,5-diméthyl-1-(6-méthoxy-quinolin-4-ylméthyl)-3-(4-trifluorométhoxy-phényl)-imidazolidine-2,4-dione dans 40 ml de dichlorométhane, sous atmosphère inerte d'argon à une température voisine de 0°C, est ajouté 6 ml d'une solution 1M de tribromure de bore dans le CH2C12. Le milieu réactionnel est maintenu sous agitation à une température voisine de 20°C pendant 16 heures. 5 ml de méthanol sont ajoutés goutte à goutte. Après 30 minutes d'agitation à cette température, 50 ml d'eau, 30 ml de CH2C12 et 10 ml d'une solution saturée de NaHC03 sont additionnés. Après décantation, la phase organique est séchée sur sulfate de sodium, filtrée puis concentrée sous pression réduite. Le meringue beige obtenu est purifié par flash-chromatographie (SiO2, AcOEt comme éluant, Ar). Les fractions contenant le produit sont concentrées sous pression réduite. On obtient ainsi 0,17 g de 5,5-diméthyl-1-(6-hydroxy-quinolin-4-ylméthyl)-3-(4-trifluorométhoxy-phényl)-imidazolidine-2,4-dione dont les caractéristiques sont les suivantes :
Spectre R.M.N. : ¹H (300 MHz, (CD₃)₂SO d6, δ en ppm) : 1,46 (s : 6H) ; 5,00 (s large : 2H) ; de 7,30 à 7,40 (mt : 2H) ; 7,51 (d, J = 4,5 Hz : 1H) ; 7,55 (d large, J = 8,5 Hz : 2H) ; 7,69 (d, J = 8,5 Hz : 2H) ; 7,94 (d, J = 9 Hz : 1H) ; 8,64 (d, J = 4,5 Hz : 1H) ; 10,12 (mf : 1H).

| | | |
|---|---|---|
| Masse IE | m/z=445 M⁺ | |
| m/z=199 | C₁₁H₇N₂O₂⁺ | |
| m/z=158 | C₁₀H₈NO⁺ | pic de base |

### Exemple 203 : 5,5-diméthyl-1-(7-méthoxy-quinolin-4-ylméthyl)-3-(4-trifluorométhoxy-phényl)-imidazolidine-2,4-dione

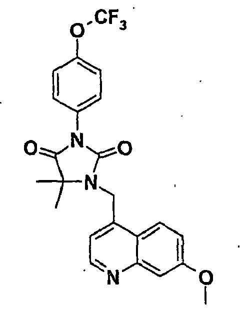

Le produit est préparé en suivant le mode opératoire décrit à l'exemple 59 à partir de 0,94 g de l'ester méthylique de l'acide 2-méthyl-2-[(7-méthoxy-quinolin-4-ylméthyl)-amino]-propanoique au lieu de l'ester méthylique de l'acide 2-méthyl-2-[(quinolin-4-ylméthyl)-amino]-propanoique utilisé à l'exemple 59 et de 1,7 g de 4-(trifluorométhoxy-phényl)isocyanate. Après purification par flash-chromatographie sur colonne (SiO2, cyclohexane/AcOEt 70/ 30 en volumes puis CH2Cl2/MeOH 90/10 en volumes comme éluants, Ar), on obtient 1,45 g du produit attendu.

Spectre de R.M.N. ¹H (300 MHz, (CD₃)₂SO d6, δ en ppm) : 1,44 (s : 6H) ; 3,96 (s : 3H) ; 5,12 (s large : 2H) ; 7,35 (dd, J = 9 et 3 Hz : 1H) ; 7,47 (d, J = 3 Hz : 1H) ; 7,49 (d, J = 4,5 Hz : 1H) ; 7,55 (d large, J = 9 Hz : 2H) ; 7,68 (d large, J = 9 Hz : 2H) ; 8,19 (d, J = 9 Hz : 1H) ; 8,80 (d, J = 4,5 Hz : 1H).

| | | |
|---|---|---|
| Masse IE | m/z=459 M⁺ | pic de base |
| m/z=444 | (M - CH₃)⁺ | |
| m/z=213 | C₁₂H₉N₂O₂⁺ | |
| m/z=172 | C₁₁H₁₀NO⁺ | |

### Préparation de l'ester méthylique de l'acide -2-((7-méthoxyquinolin-4-ylméthyl)-amino)-propanoïque

Un mélange de 1,23 g de chlorhydrate de l'ester méthylique de l'acide α-aminoisobutyrique et de 1,12 ml de triéthylamine dans 30 ml de dichlorométhane est agité à 0°C pendant 20 minutes. Ensuite 1 g de sulfate de magnésium et 1,5 g de 7-méthoxyquinoline-4-carbaldéhyde sont ajoutés. L'agitation est maintenue 15 heures à température ambiante puis le mélange est concentré sous pression réduite. Le résidu est repris par 35 ml de méthanol, la solution obtenue est refroidie à 0°C, puis 0,31 g de borohydrure de sodium sont ajoutés par portion. Le milieu réactionnel est agité à une température voisine de 20°C pendant 15. heures. Le milieu réactionnel est concentré sous pression réduite. Le résidu obtenu est repris avec 100 ml d'AcOEt. Le précipité formé est filtré et le filtrat est concentré sous pression réduite puis purifié par flash-chromatographie sur colonne (SiO2, AcOEt comme éluant, Ar), on obtient 0,95 g du produit attendu.

Spectre de R.M.N. ¹H (300 MHz, (CD₃)₂SO d6, δ en ppm) : 1,35 (s : 6H) ; 2,63 (t, J = 7,5 Hz : 1H) ; 3,70 (s : 3H) ; 3,93 (s : 3H) ; 4,05 (d, J = 7,5 Ha : 2H) ; 7,28 (dd, J = 9 et 3 Ha : 1H) ; 7,41 (d, J = 3 Ha : 1H) ; 7,43 (d, J = 4,5 Hz :1H) ; 8,12 (d, J = 9 Hz : 1H) ; 8,76 (d, J = 4,5 Hz : 1H).

| | | |
|---|---|---|
| Masse IC | m/z=289 MH⁺ | pic de base |
| | m/z=229 (M - C₂H₄O₂)⁺ | |

### Préparation du 7-méthoxyquinolin-4-carbaldéhyde

Un mélange de 1,9 g d'oxyde de sélénium en solution dans 35 ml de dioxane est ajouté goutte à goutte à une solution de 2,7 g de 4-méthyl-7-méthoxyquinoléine dans 35 ml de dioxane préalablement chauffée à 65°C. A la fin de l'addition, la suspension marron est chauffé à une température voisine de 80°C pendant 5 heures. Le milieu réactionnel est agité pendant 16 heures à une température voisine de 20°C. La suspension verdâtre est essorée puis lavée avec l'AcOEt. Le filtrat est concentré sous pression réduite. La suspension obtenu est cristallisé dans l'oxyde isopropylique pour donner 1,54 g du 7-méthoxyquinoline-4-carbaldéhyde.

Spectre de R.M.N. ¹H (300 MHz, (CD₃)₂SO d6, δ en ppm) : 3,99 (s : 3H) ; 7,48 (dd, J = 9 et 3 Hz : 1H) ; 7,57 (d, J = 3 Hz : 1H) ; 7,90 (d, J = 4,5 Hz : 1H) ; 8,89 (d, J = 9 Hz : 1H) ; 9,19 (d, J = 4,5 Hz : 1H) ; 10, 52 (s : 1H).
Masse IC m/z=188 MH⁺ pic de base

### Préparation du 4-méthyl-7-méthoxyquinoléine

A une solution de 6 g de 4-bromo-7-méthoxyquinoléine dans 100 ml de DMF, sous atmosphère inerte d'argon à une température voisine de 20°C, est ajouté 4 g de triphénylphosphine, 5,3 g de chlorure de lithium, 14 ml de tétraméthylétain et 2,1 g du chlorure de bis(triphénylphosphine)palladium(II). Le milieu réactionnel est chauffé à une température voisine de 120°C pendant 16 heures. Après refroidissement, l'insoluble est filtré. Le filtrat est concentré sous pression réduite. Le résidu obtenu est repris avec 300 ml d'AcOEt et 300 ml d'eau. Après décantation, la phase organique est séchée sur sulfate de magnésium, filtrée puis concentrée sous pression réduite. L'huile obtenue est repris avec 300 ml d'AcOEt et 300 ml d'eau puis acidifiée avec l'acide chlorhydrique jusqu'au PH=1. La phase acqueuse est alcalinisée avec la soude jusqu'au PH=10 puis extraite avec 300 ml d'AcOEt. Après décantation, la phase organique est séchée sur sulfate de magnésium, filtrée puis concentrée sous pression réduite pour donner 2,7 g du 4-méthyl-7-méthoxyquinoléine dont les caractéristiques sont les suivantes :
Spectre de R.M.N. ¹H (300 MHz, (CD₃)₂SO d6, δ en ppm) : 2,65 (s : 3H) ; 3,94 (s : 3H) ; 7,23 (d large, J = 4,5 Hz : 1H) ; 7,28 (dd, J = 9 et 3 Hz : 1H) ; 7,40 (d, J = 3 Hz : 1H) ; 8,01 (d, J = 9 Hz : 1H) ; 8,58 (d, J = 4,5 Hz : 1H).

| | | |
|---|---|---|
| Masse | IE m/z=173 M⁺ | pic de base |
| | m/z=158 (M - CH₃)⁺ | |
| | m/z=143 (M - CH₂O)⁺ | |
| | m/z=130 (m/z=158 - CO)⁺ | |

### Préparation du 4-bromo-7-méthoxyquinoléine

22,74 g de 4-hydroxy-7-méthoxyquinoléine sont ajoutés sur 200 g d'oxybromure de phosphore préalablement chauffés à une température voisine de 110°C. Le milieu réactionnel est chauffé à cette même température pendant 3 heures. Le milieu réactionnel est versé chaud sur un mélange de 500 ml d'AcOEt et 500 ml d'eau glacée. Le milieu est neutralisée à l'aide du carbonate de potassium jusqu'au PH=7. Après décantation, la phase organique est séchée sur sulfate de magnésium, évaporée sous pression réduite, puis purifié par chromatographie sur colonne (Sio2, AcOEt/cyclohexane 50/50 en volume comme éluants, Ar), on obtient 14,6 g du produit attendu.

Spectre de R.M.N. ¹H (300 MHz, (CD₃)₂SO d6, δ en ppm) : 3,97 (s : 3H) ; 7,43 (dd, J = 9 et 3 Hz : 1H) ; 7,49 (d, J = 3 Hz : 1H) ; 7,79 (d, J = 4,5 Hz : 1H) ; 8,06 (d, J = 9 Hz : 1H) ; 8,67 (d, J = 4,5 Hz : 1H).
Masse IE m/z=237 M⁺ pic de base

La 4-hydroxy-7-méthoxyquinoléine est préparée selon le procédé décrit dans : J. Am. Chem. Soc., 68, 1268, 1946.

### Exemple 204 : 5,5-diméthyl-1-(7-hydroxy-quinlin-4-ylméthyl)-3-(4-trifluorométhoxy-phényl)-imidasolidine-2,4-dione

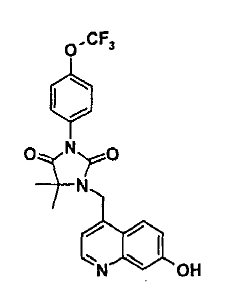

Le mélange de 0,89 g de 5,5-diméthyl-1-(7-méthoxy-quinolin-4-ylméthyl)-3-(4-trifluorométhoxy-phényl)-imidazolidine-2,4-dione et 5,5 g de chlorhydrate de pyridine est chauffé à une température voisine de 220°C pendant 4 heures. Après refroidissement, 200 ml d'eau et 100 ml de CH2C12 sont ajoutés. Après décantation, la phase organique est séchée sur sulfate de sodium, filtrée puis concentrée sous pression réduite. Le solide obtenu est purifié par flash-chromatographie (SiO2, AcOEt/CH2Cl2 40/60 en volumes comme éluant, Ar). Les fractions contenant le produit sont concentrées sous pression réduite. On obtient ainsi 85 mg de 5,5-diméthyl-1-(7-hydroxy-quinolin-4-ylméthyl)-3-(4-trifluorométhoxy-phényl)-imidazolidine-2,4-dione dont les caractéristiques sont les suivantes :
Spectre de R.M.N. ¹H (300 MHz, (CD₃)₂SO d6, δ en ppm) : 1,45 (s : 6H) ; 5,08 (s large : 2H) ; 7,24 (dd, J = 9 et 3 Hz : 1H) ; 7,30 (d, J = 3 Hz : 1H) ; 7,38 (d, J = 4,5 Hz : 1H) ; 7,55 (d large, J = 9 Hz : 2H) ; 7,68 (d large, J = 9 Hz : 2H) ; 8,10 (d, J = 9 Hz : 1H) ; 8,71 (d, J = 4,5 Hz : 1H) ; de 9,90 à 10,50 (mf étalé : 1H).

| | | |
|---|---|---|
| Masse IE | m/z=445 M⁺ | pic de base |
| | m/z=430 (M - CH₃)⁺ | |
| | m/z=199 C₁₁H₇N₂O₂⁺ | |
| | m/z=158 C₁₀H₈NO⁺ | |

### Exemple 205 : 5,5-diméthyl-1-(2-amino-quinolin-4-ylméthyl)-3-(4-trifluorométhoxy-phényl)-imidazolidine-2,4-dione

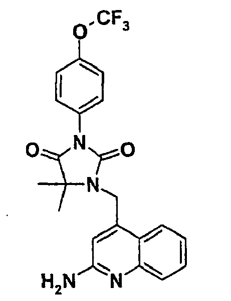

A une solution de 0,8 g de 5,5-diméthyl-1-(N-oxyquinolin-4-ylméthyl)-3-(4-trifluorométhoxy-phényl)-imidazolidine-2,4-dione dans 10 ml de chloroforme, sous atmosphère inerte d'argon à une température voisine de 5°C, est ajouté 0,44 g de chlorure de tosyle. Après 30 minutes d'agitation à cette même température, 1.5 ml d'ammoniaque à 32 % sont ajoutés. On laisse remonter la température à 20°C. Le milieu réactionnel est agité à une température voisine de 20°C pendant 16 heures. 100 ml d'eau sont ajoutés. Après décantation, la phase organique est séchée sur sulfate de sodium, filtrée puis concentrée sous pression réduite. Le solide obtenu est purifié par flash-chromatographie (Si02, AcOEt comme éluant, Ar). Les fractions contenant le produit sont concentrées sous pression réduite. On obtient ainsi 200 mg de 5,5-diméthyl-1-(2-amino-quinolin-4-ylinéthyl)-3-(4-trifluorométhoxy-phényl)-imidazolidine-2,4-dione dont les caractéristiques sont les suivantes :
Spectre de R.M.N. ¹H (300 MHz, (CD₃)₂SO d6, δ en ppm) : 1,49 (s : 6H) ; 4,96 (s large : 2H) ; 6,36 (s large : 2H) ; 6,81 (s : 1H) ; 7,23 (mt : 1H) ; 7,51 (mt : 2H) ; 7,58 (d large, J = 9 Hz : 2H) ; 7,68 (d large, J = 9 Hz : 2H) ; 7,91 (d, J = 8,5 Hz : 1H) .

| | | |
|---|---|---|
| Masse IE | m/z=444M⁺ | pic de base |
| | m/z=429 (M - CH₃)⁺ | |
| | m/z=198 C₁₁H₈N₃O⁺ | |
| | m/z=158 C₁₀H₁₀N₂⁺ | |

### Exemple 206 : 5,5-diméthyl-1-(N-oxy-quinolin-4-ylméthyl)-3-(4-trifluorométhoxy-phényl)-imidazolidine-2,4-dione

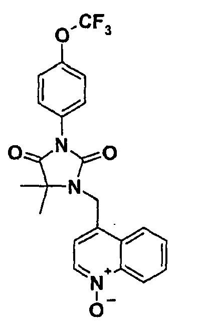

A une solution de 1,95 g de 5,5-diméthyl-1-(quinolin-4-ylméthyl)-3-(4-trifluorométhoxy-phényl)-imidazolidine-2,4-dione dans 200 ml de chloroforme et 10 ml de méthanol, sous atmosphère inerte d'argon à une température voisine de 20°C, est ajouté 0,78 g d'acide m-chloroperbenzoique. Le milieu réactionnel est agité à une température voisine de 20°C pendant 4 heures. 0,8 g supplémentaire d'acide m-chloroperbenzoique est ajouté. Le milieu réactionnel est concentré sous pression réduite. Le produit est cristallisé dans l'oxyde isopropylique pour donner 1,76 g de 5,5-dimêthyl-1-(N-oxyde-quinolin-4-ylméthyl)-3-(4-trifluorométhoxy-phényl)-imidazolidine-2,4-dione dont les caractéristiques sont les suivantes :
Spectre de R.M.N. ¹H (300 MHz, (CD₃)₂SO d6, δ en ppm) : 1,45 (s : 6H) ; 5,09 (s large : 2H) ; 7,55 (d large, J = 8,5 Hz : 2H) ; 7,63 (d, J = 4,5 Hz : 1H) ; 7,68 (d large, J = 8,5 Hz : 2H) ; 7,85 (t large, J = 8,5 Hz : 1H) ; 7,91 (t large, J = 8,5 Hz : 1H) ; 8,35 (d large, J = 8,5 Hz : 1H) ; 8,56 (d, J = 4,5 Hz : 1H) ; 8,64 (d large, J = 8,5 Hz : 1H).
Masse ES m/z=446 MH⁺ pic de base

### Exemple 207 : 5,5-diméthyl-1-(2-chloro-quinolin-4-ylméthyl)-3-(4-trifluorométhoxy-phényl)-imidazolidine-2,4-dione

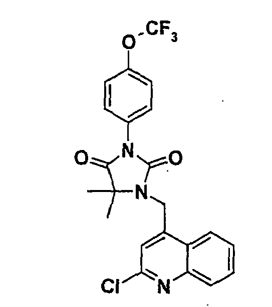

A une solution de 4 ml de DMF et 2 ml de toluène, sous atmosphère inerte d'argon à une température voisine de 5°C, est ajouté 0,17 ml d'oxychlorure de phosphore. Après 30 minutes d'agitation à cette même température, 0,4 g de 5,5-diméthyl-1-(N-oxyde-quinolin-4-ylméthyl)-3-(4-trifluorométhoxy-phényl)-imidazolidine-2,4-dione dans 10 ml de toluène, sont ajoutés. Le milieu réactionnel est maintenu sous agitation à une température voisine de 5°C pendant 2 heures et demie. On laisse remonter la température à 20°C. Le milieu réactionnel est concentré sous pression réduite. Le résidu obtenu est repris avec 50 ml d'AcOEt et lavé avec une solution saturée de NaHCO3. Après décantation, la phase organique est séchée sur sulfate de sodium, filtrée puis concentrée sous pression réduite. Le produit est cristallisé dans l'oxyde isopropylique pour donner 0,37 g de 5,5-diméthyl-1-(2-chloro-quinolin-4-ylméthyl)-3-(4-trifluorométhoxy-phényl)-imidazolidine-2,4-dione dont les caractéristiques sont les suivantes :
Spectre de R.M.N. ¹H (300 MHz, (CD₃)₂SO d6, δ en ppm) : 1,50 (s : 6H) ; 5,17 (s large : 2H) ; 7,56 (d large, J = 8,5 Ha : 2H) ; 7,71 (d large, J = 8,5 Hz : 2H) ; 7,76 (s : 1H) ; 7,76 (t dédoublé, J = 8 et 1,5 Hz : 1H) ; 7,89 (t dédoublé, J = 8 et 1,5 Hz : 1H) ; 8,03 (d large, J = 8 Hz : 1H) ; 8,31 (d large, J = 8 Hz : 1H).

| | | |
|---|---|---|
| Masse IE | m/z=463 M^{+.} | pic de base |
| | m/z=448 (M - CH₃)⁺ | |
| | m/z=428 (M - Cl)⁺ | |
| | m/z=358 (m/z=428 - C₄H₆O)⁺ | |
| | m/z=217 C₁₁H₆N₂OCl⁺ | |
| | m/z=176 C₁₀H₇NCl⁺ | |

### Exemple 208 : 5,5-Diméthyl-1-(2-chloro-pyridin-4-ylméthyl)-3-(4-trifluorométhoxy-phényl)-imidazolidine-2,4-dione

A une solution de 0,1 g de 5,5-Diméthyl-3-(4-trifluorométhoxy-phényl)-imidazolidine-2,4-dione dans 2 ml de diméthylformamide anhydre, sous atmosphère inerte d'argon à une température voisine de 20°C, est ajouté 0,028 g d'hydrure de sodium, l'agitation est maintenue à cette température pendant 30 minutes, est ajouté une solution de 0,1 g de 2-chloro-4-(bromométhyl)pyridine dans 2 ml de diméthylformamide puis de l'eau glacée après 10 minutes de réaction. Le mélange réactionnel est déposé sur une cartouche de diamètre 20 mm garnie avec 17 g de silice greffée octadécyl 50 µm conditionnée successivement à l'acétonitrile puis à l'eau. L'élution a été effectuée par gradient en utilisant le mélange (eau/acétonitrile) de 0 à 100 % d'acétonitrile. Les fractions contenant l'attendu sont concentrées sous pression réduite. On obtient ainsi 0,130 g brut qui sera purifié par une double chromatographie en utilisant une cartouche garnie avec 10 g de silice 20-40 µm conditionnée, puis éluée par un mélange (cyclohexane/acétate d'éthyle), (8/2), (v/v) avec un débit de 5 ml/minute. Les fractions comprises entre 30 et 75 ml sont concentrées sous pression réduite. On obtient ainsi 0,1 g de 5,5-Diméthyl-1-(2-chloro-pyridin-4-ylméthyl)-3-(4-trifluorométhoxy-phényl)-imidazolidine-2,4-dione sous forme de poudre blanche dont les caractéristiques sont les suivantes :
PF 134°C

| | | |
|---|---|---|
| Masse IE | m/z=413 M^{+.} | pic de base |
| | m/z=398 | (M - CH₃)⁺ |
| | m/z=203 | C₈H₄NO₂F₃^{+.} |
| | m/z=167 | C₇H₄N₂OCl⁺ |
| | m/z=126 | C₆H₅NCl⁺ |

Spectre de R.M.N. ¹H (300 MHz, (CD₃)₂SO d6, δ en ppm) : 1,44 (s : 6H) ; 4,67 (s : 2H) ; 7,50 (d large, J = 5,5 Ha : 1H) ; 7,54 (d large, J = 9 Hz : 2H) ; 7,61 (s large : 1H) ; 7,66 (d large, J = 9 Ha : 2H) ; 8,40 (d, J = 5,5 Hz : 1H).

Le composé 5,5-diméthyl-3-(4-trifluorométhoxy-phényl)-imidazolidine-2,4-dione est décrit dans l'exemple 63.

### Exemple 209 : 5,5-Diméthyl-1-(2-éthoxy-pyridin-4-ylméthyl)-3-(4-trifluorométhoxy-phényl)-imidazolidine-2,4-dione

A une solution de 0,175 g de 5,5-Diméthyl-3-(4-trifluorométhoxy-phényl)-imidazolidine-2,4-dione dans 2 ml de diméthylformamide anhydre, sous atmosphère inerte d'argon à une température voisine de 20°C, est ajouté 0,015 g d'hydrure de sodium, l'agitation est maintenue à cette température pendant 30 minutes, est ajouté une solution de 0,185 g de 2-éthoxy-4-(bromométhyl)pyridine dans 2 ml de diméthylformamide anhydre puis de l'eau glacée après 10 minutes de réaction. Le mélange réactionnel est déposé sur une cartouche de diamètre 37 mm garnie avec 65 g de silice greffée octadécyl 40-60 µm conditionnée successivement par le mélange (eau/acétonitrile) (5/95), (v/v) puis par le mélange (eau/acétonitrile) (95/5), (v/v). L'élution a été effectuée par un mélange (eau/acétonitrile), (95/5), (v/v) en 20 minutes, suivi d'un gradient linéaire de 5 à 95 % d'acétonitrile en 60 minutes, à un débit de 10 ml/minute. Les fractions comprises entre 700 et 760 ml sont concentrées sous pression réduite. On obtient ainsi 0,145 g de 5,5-diméthyl-1-(2-éthoxy-pyridin-4-ylméthyl)-3-(4-trifluorométhoxy-phényl)-imidazolidine-2,4-dione dont les caractéristiques sont les suivantes :

| | | |
|---|---|---|
| Masse IE | m/z=423 | M⁺ |
| | m/z=408 | (M - CH₃)⁺ pic de base |
| | m/z=395 | (M - C₂H₄)^{+.} |
| | m/z=203 | C₈H₄NO₂F₃⁺ |

Spectre de R.M.N. ¹H (300 MHz, (CD₃)₂SO d6, δ en ppm) : 1,32 (t, J = 7 Hz : 3H) ; 1,42 (s : 6H) ; 4,30 (q, J = 7 Hz : 2H) ; 4,58 (s large : 2H) ; 6,85 (s large : 1H) ; 7,01 (d large, J = 5,5 Hz : 1H) ; 7,52 (d large, J = 8,5 Hz : 2H) ; 7,63 (d large, J = 8,5 Hz : 2H) ; 8,10 (d, J = 5,5 Hz : 1H).

Le composé 5,5-Diméthyl-3-(4-trifluorométhoxy-phényl)-imidazolidine-2,4-dione est décrit dans l'exemple 63.

### Exemple 210 : 5,5-diméthyl-1-(2-éthyl-pyridin-4-ylméthyl)-3-(4-trifluorométhoxy-phényl)-imidazolidine-2,4-dione

A une solution de 0,064 g de 5,5-diméthyl-3-(4-trifluorométhoxy-phényl)-imidazolidine-2,4-dione dans 1 ml de diméthylformamide anhydre, sous atmosphère inerte d'argon à une température voisine de 20°C, est ajouté 0,009 g d'hydrure de sodium, l'agitation est maintenue à cette température pendant 30 minutes, est ajouté une solution de 0,062 g de 2-éthyl-4-(bromométhyl)pyridine dans 0,5 ml de diméthylformamide anhydre puis de l'eau glacée après 10 minutes de réaction. Le mélange réactionnel est déposé sur une cartouche de diamètre 27 mm garnie avec 25 g de silice greffée octadécyl 40-60 µm conditionnée successivement par le mélange (eau/acétonitrile) (5/95), (v/v) puis par le mélange (eau/acêtonitrile) (95/5), (v/v). L'élution a été effectuée par un mélange (eau/acétonitrile) (95/5) (v/v) en 20 minutes, suivi d'un gradient linéaire de 5 à 95% d'acétonitrile en 60 minutes, à un débit de 10 ml/minute. Les fractions comprises entre 750 et 790 ml sont concentrées sous pression réduite. On obtient ainsi 0,06 g de 5,5-Diméthyl-l-(2-éthyl-pyridin-4-ylmé-thyl)-3-(4-trifluorométhoxy-phényl)-imidazolidine-2,4-dione dont les caractéristiques sont les suivantes :

| | | |
|---|---|---|
| Masse IE | m/z=407 | M⁺ pic de base |
| | m/z=392 | (M - CH₃)⁺ |
| | m/z=203 | C₈H₄NO₂F₃^{+.} |
| | m/z=120 | C₈H₁₀N⁺ |

Spectre de R.M.N. ¹H (400 MHz, (CD₃)₂SO d6, δ en ppm) : 1,27 (t, J = 7,5 Hz : 3H) ; 1,44 (s : 6H) ; 2,75 (q, J = 7,5 Hz : 2H) ; 4,54 (s : 2H) ; 7,10 (d large, J = 5,5 Hz : 1H) ; 7,15 (s large : 1H) ; 7,39 (d large, J = 8,5 Hz : 2H) ; 7,49 (d, J = 8,5 Hz : 2H) ; 8,26 (d, J =5,5 Hz : 1H).

Le composé 5,5-diméthyl-3-(4-trifluorométhoxy-phényl)-imidazolidine-2,4-dione est décrit dans l'exemple 63.

### Exemple 211 : 5,5-diméthyl-1-(2-chloro-pyridin-4-ylméthyl)-3-(4-trifluorométhanesulfanyl-phényl)-imidazolidine-2,4-dione

A une solution de 0,175 g de 5,5-diméthyl-3-(4-trifluorométhylsulfanyl-phényl)-imidazolidine-2,4-dione dans 3 ml de diméthylformamide anhydre, sous atmosphère inerte d'argon à une température voisine de 20°C, est ajouté 0,046 g d'hydrure de sodium, l'agitation est maintenue à cette température pendant 30 minutes, est ajouté une solution de 0,166 g de 2-chloro-4-(bromométhyl)pyridine dans 2 ml de diméthylformamide anhydre puis de l'eau glacée après 10 minutes de réaction. Le mélange réactionnel est déposé sur une cartouche de diamètre 37 mm garnie avec 65 g de silice greffée octadécyl 40-60 µm conditionnée successivement par le mélange (eau/acétonitrile) (5/95), (v/v) puis par le mélange (eau/acétonitrile) (95/5), (v/v). L'élution a été effectuée par un mélange (eau/acétonitrile) (95/5), (v/v) en 20 minutes, suivi d'un gradient linéaire de 5 à 95 % d'acétonitrile en 60 minutes, à un débit de 10 ml/minute. Les fractions comprises entre 740 et 780 ml sont concentrées sous pression réduite. On obtient ainsi 0,03 g de 5,5-Diméthyl-1-(2-chloro-pyridin-4-ylméthyl)-3-(4-trifluorométhanesulfanyl-phényl)-imidazolidine-2,4-dione sous forme de poudre blanche dont les caractéristiques sont les suivantes :
PF 111°C

| | | |
|---|---|---|
| Masse | IC | |
| | m/z=447 MMH₄⁺ | |
| | m/z=430 MH⁺ | pic de base |

Spectre de R.M.N*.* ¹H (300 MHz, (CD₃)₂SO d6, δ en ppm) : 1,44 (s : 6H) ; 4,67 (s large : 2H) ; 7,49 (d large, J= 5,5 Hz : 1H) ; 7,61 (s large : 1H) ; 7,70 (d large, J = 8,5 Hz : 2H) ; 7,88 (d large, J = 8,5 Hz : 2H) ; 8,38 (d, J = 5,5 Hz : 1H).

### Préparation du 5,5-diméthyl-3-(4-trifluorométhylsulfanyl-phényl)-imidazolidine-2,4-dione

A une solution de 4 g de 4-trifluorométhylsulfanyl-phényl isocyanate dans 40 ml de toluène, sous atmosphère inerte d'argon à une température voisine de 20°C, est ajouté 5,12 ml de triéthylamine et 2,8 g de chlorhydrate de l'ester méthylique de l'acide α-aminoisobutyrique. Le mélange ainsi obtenu est porté au reflux pendant 24 heures puis refroidi à une température voisine de 20°C. Le mélange réactionnel est concentré à sec sous pression réduite, le résidu obtenu est repris par de l'éther éthylique, filtré. On obtient ainsi 5,3 g de 5,5-diméthyl-3-(4-trifluorométhylsulfanyl-phényl)-imidazolidine-2,4-dione dont les caractéristiques sont les suivantes :

| | | |
|---|---|---|
| Masse IC | | |
| m/z=322 | MNH₄⁺ | |
| m/z=102 | triethylamineH⁺ | pic de base |

Spectre R.M.N. ¹H (300 MHz, (CD₃)₂SO d6, δ en ppm) : 1,44 (s : 6H) ; 7,62 (d large, J = 8,5 Hz : 2H) ; 7,85 (d large, J = 8,5 Hz : 2H) ; 8,72 (mf : 1H).

L'insoluble ainsi obtenu est repris par du dichlorométhane puis lavé à l'eau. On obtient ainsi 2,76 g de 5,5-diméthyl-3-(4-trifluorométhylsulfanyl-phényl)-imidazolidine-2,4-dione qui sera utilisé pour la suite de la synthèse.

### Exemple 212 : 5,5-diméthyl-1-(2-éthoxy-pyridin-4-ylméthyl)-3-(4-trifluorométhanesulfanyl-phényl)-imidazolidine-2,4-dione

A une solution de 0,185 g de 5,5-diméthyl-3-(4-trifluorométhylsulfanyl-phényl)-imidazolidine-2,4-dione dans 3,2 ml de diméthylformamide anhydre, sous atmosphère inerte d'argon à une température voisine de 20°C, est ajouté 0,049 g d'hydrure de sodium, l'agitation est maintenue à cette température pendant 30 minutes, est ajouté une solution de 0,184 g de 2-éthoxy-4-(bromométhyl)pyridine dans 2 ml de diméthylformamide puis de l'eau glacée après 10 minutes de réaction. Le mélange réactionnel est déposé sur une cartouche de diamètre 37 mm garnie avec 65 g de silice greffée octadécyl 40-60 µm conditionnée successivement par le mélange (eau/acétonitrile) (5/95), (v/v) puis par le mélange (eau/acétonitrile) (95/5), (v/v). L'élution a été effectuée par un mélange (eau/acétonitrile) (95/5) (v/v) en 20 minutes, suivi d'un gradient linéaire de 5 à 95 % d'acétonitrile en 60 minutes, à un débit de 10 ml/minute. Les fractions comprises entre 520 et 700 ml sont concentrées sous pression réduite. Le résidu ainsi obtenu sera purifié par flash chromatographie en utilisant une cartouche garnie avec 2 g de silice 15-35 µm conditionnée, puis éluée par un mélange (cyclohexane/acétate d'éthyle) (9/1), (v/v). Les fractions contenant l'attendu sont concentrées sous pression réduite. On obtient ainsi 0,01 g de 5,5-diméthyl-1-(2-éthoxy-pyridin-4-ylméthyl)-3-(4-trifluorométhanesulfanyl-phényl)-imidazolidine-2,4-dione dont les caractéristiques sont les suivantes :

| | | |
|---|---|---|
| Masse IE | | |
| m/z=439 | M^{+.} | |
| m/z=424 | (M - CH₃)⁺ | pic de base |
| m/z=411 | (M - C₂H₄)^{+.} | |
| m/z=219 | C₈H₄NOSF₃^{+.} | |

Spectre de R.M.N. ¹H (300 MHz, (CD₃)₂SO d6, δ en ppm) : 1,32 (t, J = 7 Hz : 3H) ; 1,42 (s : 6H) ; 4,30 (q, J = 7 Hz : 2H) ; 4,58 (s large : 2H) ; 6,86 (s large : 1H) ; 7,01 (d large, J = 5,5 Hz : 1H) ; 7,69 (d large, J = 8,5 Hz : 2H) ; 7,88 (d large, J = 8,5 Hz : 2H) ; 8,10 (d large, J = 5,5 Hz : 1H).

Le composé 5,5-diméthyl-3-(4-trifluorométhylsulfanyl-phényl)-imidazolidine-2,4-dione est décrit dans l'exemple 211.

### Exemple 213 : 5,5-diméthyl-1-(2-éthyl-pyridin-4-ylméthyl)-3-(4-trifluorométhanesulfanyl-phényl)-imidazolidine-2,4-dione

A une solution de 0,135 g de 5,5-diméthyl-3-(4-trifluorométhylsulfanyl-phényl)-imidazolidine-2,4-dione dans 2 ml de diméthylformamide anhydre, sous atmosphère inerte d'argon à une température voisine de 20°C, est ajouté 0,021 g d'hydrure de sodium, l'agitation est maintenue à cette température pendant 30 minutes, est ajouté une solution de 0,126 g de 2-éthyl-4-(bromométhyl)pyridine dans 1 ml de diméthylformamide anhydre puis de l'eau glacée après 10 minutes de réaction. Le mélange réactionnel est déposé sur une cartouche de diamètre 37 mm garnie avec 65 g de silice greffée octadécyl 40-60 µm conditionnée successivement par le mélange (eau/acétonitrile) (5/95), (v/v) puis par le mélange (eau/acétanitrile) (95/5), (v/v). L'élution a été effectuée par un mélange (eau/acétonitrile) (95/5), (v/v) en 20 minutes, suivi d'un gradient linéaire de 5 à 95 % d'acétonitrile en 60 minutes et d'une élution à l'acétonitrile à 100 % pendant 10 minutes, à un débit de 10 ml/minute. Les fractions comprises entre 800 et 880 ml sont concentrées sous pression réduite. On obtient ainsi 0,120 g de 5,5-diméthyl-1-(2-éthyl-pyridin-4-ylméthyl)-3-(4-trifluorométhanesulfanyl-phényl)-imidazolidine-2,4-dione dont les caractéristiques sont les suivantes :

| | | |
|---|---|---|
| Masse IE | | |
| m/z=423 | M^{+.} | pic de base |
| m/z=408 | (M - CH₃)⁺ | |
| m/z=219 | C₈H₄NOSF₃^{+.} | |
| m/z=120 | C₈H₁₀N⁺ | |

Spectre de R.M.N. ¹H (400 MHz, (CD₃)₂SO d6, δ en ppm) : 1,23 (t, J = 7,5 Hz : 3H) ; 1,42 (s : 6H) ; 2,75 (q, J = 7,5 Hz : 2H) ; 4,62 (s large : 2H) ; 7,25 (d large, J = 5,5 Hz : 1H) ; 7,30 (s large : 1H) ; 7,70 (d, J = 8,5 Hz : 2H) ; 7,88 (d, J = 8,5 Hz : 2H) ; 8,43 (d, J = 5,5 Hz : 1H) .

Le composé 5,5-diméthyl-3-(4-trifluorométhylsulfanyl-phényl)-imidazolidine-2,4-dione est décrit dans l'exemple 211.

### Exemple 214 : 5,5-diméthyl-1-(2-bromo-pyridin-4-ylméthyl)-3-(4-trifluorométhoxy-phényl)-imidazolidine-2,4-dione

A une solution de 0,081 g de 5,5-diméthyl-3-(4-trifluorométhoxy-phényl)-imidazolidine-2,4-dione dans 2 ml de diméthylformamide anhydre, sous atmosphère inerte d'argon à une température voisine de 20°C, est ajouté 0,023 g d'hydrure de sodium, l'agitation est maintenue à cette température pendant 35 minutes, est ajouté une solution de 0,071 g de 2-bromo-4-(bromométhyl)pyridine dans 1 ml de diméthylformamide anhydre puis de l'eau glacée après 15 minutes de réaction. Le mélange réactionnel est déposé sur une cartouche de diamètre 16 mm garnie avec 5 g de silice greffée octadécyl 40-60 µm conditionnée successivement par le mélange (eau/acétonitrile) (5/95), (v/v) puis par le mélange (eau/acétonitrile) (95/5), (v/v). L'élution a été effectuée par un gradient linéaire de 5 à 95 % d'acétonitrile en 30 minutes puis par un mélange (eau/acétonitrile), (5/95), (v/v) en 10 minutes, à un débit de 5 ml/minute. Les fractions comprises entre 90 et 100 ml sont concentrées sous pression réduite. On obtient ainsi 0,015 g de 5,5-diméthyl-1-(2-bromo-pyridin-4-ylméthyl)-3-(4-trifluorométhoxy-phényl)-imidazolidine-2,4-dione sous forme de meringue blanche dont les caractéristiques sont les suivantes :

| | | |
|---|---|---|
| Masse IE | | |
| m/z=457 | M^{+.} | pic de base |
| m/z=442 | (M - CH₃)⁺ | |
| m/z=211 | C₇H₄N₂OBr⁺ | |
| m/z=203 | C₈H₄NO₂F₃^{+.} | |
| m/z=170 | C₆H₅NBr⁺ | |

Spectre de R.M.N. ¹H (300 MHz, (CD₃)₂SO d6, δ en ppm) : 1,44 (s : 6H) ; 4,67 (s large : 2H) ; 7,52 (d large, J = 5,5 Hz : 1H) ; 7,54 (d large, J = 9 Hz : 2H) ; 7,66 (d large, J = 9 Hz : 2H) ; 7,75 (s large : 1H) ; 8,37 (d, J = 5,5 Hz : 1H).

Le composé 5,5-Diméthyl-3-(4-trifluorométhoxy-phényl)-imidazolidine-2,4-dione est décrit dans l'exemple 63.

### Exemple 215 : 5,5-Diméthyl-1-(2-fluoro-pyridin-4-ylméthyl)-3-(4-trifluorométhanesulfanyl-phényl)-imidazolidine-2,4-dione

A une solution de 0,096 g de 5,5-diméthyl-3-(4-trifluorométhylsulfanyl-phényl)-imidazolidine-2,4-dione dans 2 ml de diméthylformamide anhydre, sous atmosphère inerte d'argon à une température voisine de 20°C, est ajouté 0,025 g d'hydrure de sodium, l'agitation est maintenue à cette température pendant 25 minutes, est ajouté une solution de 0,060 g de 2-fluoro-4-(bromométhyl)pyridine dans 1 ml de diméthylformamide anhydre puis de l'eau glacée après 15 minutes de réaction. Le mélange réactionnel est déposé sur une cartouche de diamètre 16 mm garnie avec 5 g de silice greffée octadécyl 40-60 µm conditionnée successivement par le mélange (eau/acétonitrile), (5/95), (v/v) puis par le mélange (eau/acétonitrile) (95/5), (v/v). L'élution a été effectuée par un gradient linéaire de 5 à 95 % d'acétonitrile en 30 minutes puis par un mélange (eau/acétonitrile), (5/95), (v/v) en 10 minutes, à un débit de 5 ml/minute. Les fractions comprises entre 105 et 125 ml sont concentrées sous pression réduite. On obtient ainsi 0,069 g de 5,5-diméthyl-1-(2-fluoro-pyridin-4-ylméthyl)-3-(4-trifluorométhanesulfanyl-phényl)-imidazolidine-2,4-dione sous forme de solide blanc dont les caractéristiques sont les suivantes :
PF : 82°C

| | | |
|---|---|---|
| Masse IE | | |
| m/z=413 | M^{+.} | pic de base |
| m/z=393 | (M - CH₃)⁺ | |
| m/z=219 | C₈H₄NOSF₃^{+.} | |
| m/z=151 | C₇H₄N₂OF⁺ | |
| m/z=110 | C₆H₅NF⁺ | |

Spectre de R.M.N. ¹H (300 MHz, (CD₃)₂SO d6, δ en ppm) : 1,45 (s : 6H) ; 4,72 (s large : 2H) ; 7,29 (s large : 1H) ; 7,43 (d large, J = 5,5 Hz : 1H) ; 7,71 (d large, J = 8,5 Hz : 2H) ; 7,89 (d large, J = 8,5 Hz : 2H) ; 8,23 (d, J = 5,5 Hz : 1H).

Le composé 5,5-diméthyl-3-(4-trifluorométhylsulfanyl-phényl)-imidazolidine-2,4-dione est décrit dans l'exemple 211.

### Exemple 216 : 5,5-Diméthyl-1-(2-cyano-pyridin-4-ylméthyl)-3-(4-trifluorométhanesulfanyl-phényl)-imidazolidine-2,4-dione

A une solution de 0,139 g de 5,5-diméthyl-3-(4-trifluorométhylsulfanyl-phényl)-imidazolidine-2,4-dione dans 4 ml de diméthylformamide anhydre, sous atmosphère inerte d'argon à une température voisine de 20°C, est ajouté 0,037 g d'hydrure de sodium, l'agitation est maintenue à cette température pendant 30 minutes, est ajouté une solution de 0,090 g de 2-cyano-4-(bromométhyl)pyridine dans 1 ml de diméthylformamide anhydre puis de l'eau glacée après 15 minutes de réaction. Le mélange réactionnel est déposé sur une cartouche de diamètre 20 mm garnie avec 10 g de silice greffée octadécyl 40-60 µm conditionnée successivement par le mélange (eau/acétonitrile), (5/95), (v/v) puis par le mélange (eau/acétonitrile) (95/5), (v/v). L'élution a été effectuée par un gradient linéaire de 5 à 95 % d'acétonitrile en 30 minutes puis par un mélange (eau/acétonitrile), (5/95), (v/v) pendant 10 minutes, à un débit de 5 ml/minute. Les fractions comprises entre 210 et 230 ml sont concentrées sous pression réduite. On obtient ainsi 0,1 g de 5,5-Diméthyl-1-(2-cyano-pyridin-4-ylméthyl)-3-(4-trifluorométhanesulfanyl-phényl)-imidazolidine-2,4-dione sous forme de solide dont les caractéristiques sont les suivantes :
PF : 148°C

| | | |
|---|---|---|
| Masse IE | | |
| m/z=420 | M^{+.} | pic de base |
| m/z=405 | (M - CH₃)⁺ | |
| m/z=219 | C₈H₄NOSF₃^{+.} | |
| m/z=158 | C₈H₄N₃O⁺ | |
| m/z=117 | C₇H₅N₂⁺ | |

Spectre de R.M.N. ¹H (300 MHz, (CD₃)₂SO d6, δ en ppm) : 1,44 (s : 6H) ; 4,74 (s large : 2H) ; 7,72 (d large, J = 9 Hz : 2H) ; 7,81 (dd, J = 5,5 et 2 Hz : 1H) ; 7,89 (d large, J = 9 Hz : 2H) ; 8,15 (s large : 1H) ; 8,72 (d large, J = 5,5 Hz : 1H).

Le composé 5,5-diméthyl-3-(4-trifluorométhylsulfanyl-phényl)-imidazolidine-2,4-dione est décrit dans l'exemple 211.

### Exemple 217 : 5,5-diméthyl-1-(2-hydroxycarbonyl-pyridin-4-ylméthyl)-3-(4-rifluorométhanesulfanyl-pényl)-imidazolidine-2,4-dione

Une solution de 0,09 g de 5,5-diméthyl-1-(2-cyano-pyridin-4-ylméthyl)-3-(4-trifluorométhanesulfanyl-phényl)-imidazolidine-2,4-dione dans 5 ml d'acide chlorhydrique 5N est portée au reflux pendant environ 16 heures. Le mélange réactionnel est concentré sous pression réduite et le brut ainsi obtenu est purifié par flash chromatographie en utilisant une cartouche de diamètre 20 mm garnie avec 10 g de silice 20-40 µm conditionnée, puis éluée par un mélange (dichlorométhane/méthanol), (9/1), (v/v) à un débit de 8 ml par minutes. Les fractions comprises entre 40 et 200 ml sont concentrées sous pression réduite. On obtient ainsi 0,06 g de 5,5-diméthyl-1-(2-hydroxycarbonyl-pyridin-4-ylméthyl)-3-(4-trifluorométhanesulfanyl-phényl)-imidazolidine-2,4-dione dont les caractéristiques sont les suivantes :

| | | |
|---|---|---|
| Masse IE | | |
| m/z=439 | M^{+.} | |
| m/z=395 | (M - CONH₂)⁺ | pic de base |
| m/z=219 | C₈H₄NOSF₃^{+.} | |
| m/z=185 | C₇H₉N₂O₂S⁺ | |

Spectre de R.M.N. ¹H (300 MHz, (CD₃)₂SO d6, δ en ppm) : 1,45 (s : 6H) ; 4,76 (s large : 2H) ; 7,70 (d, J = 8,5 Hz : 2H) ; 7,72 (mt : 1H) ; 7,89 (d, J = 8,5 Hz : 2H) ; 8,12 (s large : 1H) ; 8,68 (d, J = 5,5 Hz : 1H).

### Exemple 218 : 5,5-diméthyl-1-[2-(méthylamino)carbonyl-pyridin-4-ylméthyl]-3-(4-trifluorométhanesulfanyl-phényl)-imidazolidine-2,4-dione

A une solution de 0,055 g de 5,5-diméthyl-1-(2-hydroxycarbonyl-pyridin-4-ylméthyl)-3-(4-trifluorométhanesulfanyl-phényl)-imidazolidine-2,4-dione dans 2 ml de dichlorométhane, sous atmosphère inerte d'argon à une température voisine de 20°C, est ajouté successivement 0,025 g de chlorhydrate de méthylamine, 0,005 g d'hydrate d'hydroxybenzotriazole, 0,105 ml de triéthylamine et 72 mg de chlorhydrate de 1-(3-diméthylaminopropyl)-3-éthylcarbodiimide, l'agitation est maintenue à cette température pendant environ 16 heures. Le mélange réactionnel est lavé à l'eau, la phase organique est séchée sur sulfate de magnésium, filtrée puis concentrée sous pression réduite. Le brut ainsi obtenu est purifié par flash chromatographie en utilisant une cartouche de diamètre 16 mm garnie avec 5 g de silice 20-40 µm conditionnée, puis éluée par un mélange (dichlorométhane/méthanol), (95/05), (v/v) à un débit de 10 ml par minutes. Les fractions contenant l'attendu sont concentrées sous pression réduite. On obtient ainsi 0,013 g de 5,5-Diméthyl-1-[2-(méthylamino)carbonyl-pyridin-4-ylméthyl]-3-(4-trifluorométhanesulfanyl-phényl)-imidazolidine-2,4-dione sous forme de poudre blanche dont les caractéristiques sont les suivantes :

| | | |
|---|---|---|
| Masse IE | | |
| m/z=452 | M^{+.} | |
| m/z=395 | (M - C₂H₃ON)⁺ | pic de base |
| m/z=219 | C₈H₄NOSF₃^{+.} | |
| m/z=148 | C₈H₈N₂O⁺ | |

Spectre de R.M.N. ¹H (300 MHz, (CD₃)₂SO d6, δ en ppm) : 1,44 (s : 6H) ; 2,85 (d, J = 5 Hz : 3H) ; 4,76 (s large : 2H) ; 7,67 (dd, J = 5 et 2 Hz : 1H) ; 7,70 (d large, J = 8,5 Hz : 2H) ; 7,89 (d large, J = 8,5 Hz : 2H) ; 8,10 (s large : 1H) ; 8,60 (d, J = 5 Hz : 1H) ; 8,75 (q large, J = 5 Hz : 1H).

### Exemple 219 : 5,5-diméthyl-1-(2-aminocarbonyl-pyridin-4-ylméthyl)-3-(4-trifluorométhanesulfanyl-phényl)-imidazolidine-2,4-dione

Une solution de 0,04 g de 5,5-diméthyl-1-(2-cyano-pyridin-4-ylméthyl)-3-(4-trifluorométhanesulfanyl-phényl)-imidazolidine-2,4-dione dans 1 ml d'acide sulfurique à 98 % est portée à 40°C pendant 30 minutes. Le mélange est repris par 15 ml d'eau glacée puis neutralisé par une solution normale de soude. La solution ainsi obtenue est extraite par 100 ml d'acétate d'éthyle. La phase organique est séchée sur sulfate de magnésium, filtrée, puis concentrée sous pression réduite, le brut ainsi obtenu est purifié par flash chromatographie en utilisant une cartouche de diamètre 16 mm garnie avec 5 g de silice 20-40 µm conditionnée, puis éluée au dichlorométhane à un débit de 10 ml par minute. Les fractions contenant l'attendu sont concentrées sous pression réduite. On obtient ainsi 0,017 g de 5,5-diméthyl-1-(2-aminocarbonyl-pyridin-4-ylméthyl)-3-(4-trifluorométhanesulfanyl-phényl)-imidazolidine-2,4-dione sous forme de poudre amorphe blanche dont les caractéristiques sont les suivantes :

| | | |
|---|---|---|
| Masse IE | | |
| m/z=438 | M^{+.} | pic de base |
| m/z=423 | (M - CH₃)⁺ | |
| m/z=393 | (M - CH₃NO)⁺ | |
| m/z=219 | C₈H₄NOSF₃^{+.} | |
| m/z=135 | C₇H₇N₂O⁺ | |

Spectre de R.M.N. ¹H (300 MHz, (CD₃)₂SO d6, δ en ppm) : 1,44 (s : 6H) ; 4,76 (s large : 2H) ; de 7,60 à 7,70 (mt : 2H) ; 7,71 (d large, J = 8,5 Hz : 2H) ; 7,89 (d large, J = 8,5 Hz : 2H) ; 8,11 (s large : 2H) ; 8,60 (d, J = 5,5 Hz : 1H).

### Exemple 220 : 5,5-diméthyl-1-(2-morpholino-pyridin-4-ylméthyl)-3-(4-trifluorométhoxy-phényl)-imidazolidine-2,4-dione

Un tube scellé de 2,5 ml contenant 0,05 g de 5,5-diméthyl-1-(2-chloro-pyridin-4-ylméthyl)-3-(4-trifluorométhoxy-phényl)-imidazolidine-2,4-dione, 0,5 ml de diméthylformamide, 0,034g de carbonate de potassium et 0,021 ml de morpholine, est placé dans un four à micro-ondes Personal Chemistry Emrys Optimiser. Le mélange est irradié sous agitation magnétique à 210°C pendant environ 50 minutes. Le mélange réactionnel est déposé sur une cartouche BOND ELUT VARIAN de référence 1225-6053 contenant 2 g de phase SCX conditionnée au diméthylformamide. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée par un mélange (cyclohexane/acétate d'éthyle), (8/2), (v/v) avec un débit de 10 ml/minute. Les fractions comprises entre 160 et 260 ml sont concentrées à sec sous pression réduite. On obtient ainsi 0,01 g de 5,5-diméthyl-1-(2-morpholino-pyridin-4-ylméthyl)-3-(4-trifluorométhoxy-phényl)-imidazolidine-2,4-dione sous forme de poudre dont les caractéristiques sont les suivantes :

| | | |
|---|---|---|
| Masse IE | | |
| m/z=464 | M^{+.} | |
| m/z=433 | (M - CH₃O)⁺ | pic de base |
| m/z=419 | (M - C₂H₅O)⁺ | |
| m/z=407 | (M - C₃H₅O)⁺ | |
| m/z=379 | (M - C₄H₅NO)⁺ | |
| m/z=203 | C₈H₄NO₂F₃^{+.} | |
| m/z=176 | C₁₀H₁₂N₂O⁺ | |

Spectre de R.M.N. ¹H (300 MHz, (CD₃)₂SO d6, δ en ppm) : 1,42 (s : 6H) ; 3,44 (t large, J = 5 Hz : 4H) ; 3,72 (t large, J = 5 Hz : 4H) ; 4,53 (s large : 2H) ; 6,75 (d large, J = 5,5 Hz : 1H) ; 6,85 (s large : 1H) ; 7,54 (d large, J = 8,5 Hz : 2H) ; 7,64 (d, J = 8,5 Hz : 2H) ; 8,10 (d, J = 5,5 Hz : 1H).

### Exemple 221 : 5,5-diméthyl-1-(2-morpholino-pyridin-4-ylméthyl)-3-(4-trifluorométhanesulfanyl-phényl)-imidazolidine-2,4-dione

Un tube scellé de 2,5 ml contenant 0,05 g de 5,5-diméthyl-1-(2-chloro-pyridin-4-ylméthyl)-3-(4-trifluorométhanesulfanyl-phényl)-imidazolidine-2,4-dione, 0,5 ml de morpholine, est placé dans un four à micro-ondes Personal Chemistry Emrys Optimiser. Le mélange est irradié sous agitation magnétique à 160°C pendant environ 70 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARAN de référence 1225-6053 contenant 2 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,03 g de 5,5-diméthyl-1-(2-morpholino-pyridin-4-ylméthyl)-3-(4-trifluorométhanesulfanyl-phényl)-imidazolidine-2,4-dione sous forme de poudre dont les caractéristiques sont les suivantes :

| | | |
|---|---|---|
| Masse IE | | |
| m/z=480 | M^{+.} | |
| m/z=449 | (M - CH₃O)⁺ | pic de base |
| m/z=435 | (M - C₂H₅O)⁺ | |
| m/z=423 | (M - C₃H₅O)⁺ | |
| m/z=219 | C₈H₄NOSF₃^{+.} | |
| m/z=176 | C₁₀H₁₂N₂O^{+.} | |

Spectre de R.M.N. ¹H (300 MHz, (CD₃)₂SO d6, δ en ppm) : 1,42 (s : 6H) ; 3,45 (t large, J = 5 Hz : 4H) ; 3,71 (t large, J = 5 Hz : 4H) ; 4,54 (s large : 2H) ; 6,75 (d large, J = 5,5 Hz : 1H) ; 6,84 (s large : 1H) ; 7,68 (d large, J = 8,5 Hz : 2H) ; 7,88 (d, J = 8,5 Hz : 2H) ; 8,09 (d, J = 5,5 Hz : 1H).

### Exemple 222 : 5,5-diméthyl-1-(2-diméthylamino-pyridin-4-ylméthyl)-3-(4-trifluorométhanesulfanyl-phényl)-imidazolidine-2,4-dione

Un tube scellé de 2,5 ml contenant 0,05 g de 5,5-diméthyl-1-(2-chloro-pyridin-4-ylméthyl)-3-(4-trifluorométhanesulfanyl-phényl)-imidazolidine-2,4-dione, 0,4 ml de d'isopropylamine, 0,1 ml de diméthylformamide, est placé dans un four à micro-ondes Personal Chemistry Emrys Optimiser. Le mélange est irradié sous agitation magnétique à 140°C pendant environ 130 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6053 contenant 2 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,013 g de 5,5-diméthyl-1-(2-diméthylamino-pyridin-4-ylméthyl)-3-(4-trifluorométhanesulfanyl-phényl)-imidazolidine-2,4-dione sous forme de poudre dont les caractéristiques sont les suivantes :

| | |
|---|---|
| Masse IC | |
| m/z=439 MH⁺ | pic de base |

Spectre de R.M.N. ¹H (300 MHz, (CD₃)₂SO d6, δ en ppm) : 1,42 (s : 6H) ; 3,03 (s : 6H) ; 4,53 (s large : 2H) ; 6,62 (d large, J = 5,5 Hz : 1H) ; 6,65 (s large : 1H) ; 7,68 (d large, J = 8,5 Hz : 2H) ; 7,87 (d, J = 8,5 Hz : 2H) ; 8,03 (d, J = 5,5 Hz : 1H).

### Exemple 223 : 5,5-diméthyl-1-(2-méthylamino-pyridin-4-ylméthyl)-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,5 ml de N-méthylpyrrolidone-2, 0,016 mg de chlorhydrate de méthylamine, 0,064 ml de triéthylamine, est placé dans un four à micro-ondes Personal Chemistry Emrys Optimiser. Le mélange est irradié sous agitation magnétique à 180°C pendant environ 80 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6054 contenant 3 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions contenant l'attendu sont concentrées à sec sous pression réduite. On obtient ainsi 0,021 g de 5,5-diméthyl-1-(2-méthylamino-pyridin-4-ylméthyl)-3-(4-trifluorométhanesulfanyl-phényl)-imidazolidine-2,4-dione sous forme de poudre blanche amorphe dont les caractéristiques sont les suivantes

| | | |
|---|---|---|
| Masse IE | | |
| m/z=424 | M^{+.} | pic de base |
| m/z=396 | (M - CH₂N)⁺ | |
| m/z=219 | C₈H₄NOSF₃^{+.} | |
| m/z=120 | C₇H₈N₂^{+.} | |

Spectre de R.M.N. ¹H (300 MHz, (CD₃)₂SO d6, δ en ppm) : 1,42 (s : 6H) ; 2,77 (d, J = 5 Hz : 3H) ; 4,48 (s large : 2H) ; 6,42 (mt : 1H) ; 6,46 (s large : 1H) ; 6,52 (d large, J = 5, 5 Hz : 1H) ; 7,69 (d large, J = 8,5 Hz : 2H) ; 7,89 (d large, J = 8,5 Hz : 2H) ; 7,94 (d, J = 5,5 Hz : 1H).

### Exemple 224: 5,5-diméthyl-1-(2-cyclohéxylamino-pyridin-4-ylméthyl)-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,2 ml de N-méthylpyrrolidone-2, 0,4 ml de cyclohéxylamine, est placé dans un four à micro-ondes Personal Chemistry Emrys Optimiser. Le mélange est irradié sous agitation magnétique à 200°C pendant environ 50 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6054 contenant 3 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 15 et 45 ml sont concentrées à sec sous pression réduite. On obtient ainsi 0,017 g de 5,5-Diméthyl-1-(2-cyclohéxylamino-pyridin-4-ylméthyl)-3-(4-trifluorométhanesulfanyl-phényl)-imidazolidine-2,4-dione sous forme de poudre amorphe dont les caractéristiques sont les suivantes :

| | | |
|---|---|---|
| Masse IE | | |
| m/z=492 | M^{+.} | |
| m/z=449 | (M - C₃H₇)⁺ | |
| m/z=435 | (M - C₃H₇)⁺ | |
| m/z=410 | (M - C₆H₁₀)^{+.} | pic de base |
| m/z=219 | C₈H₄NOSF₃^{+.} | |
| m/z=175 | C₁₁H₁₅N₂⁺ | |
| m/z=98 | C₆H₁₂N⁺ | |

Spectre de R.M.N. ¹H (400 MHz, (CD₃)₂SO d6, δ en ppm) : de 1,10 à 1,40 (mt : 5H) ; 1,43 (s : 6H) ; de 1,55 à 2,00 (mt : 5H) ; 3,67 (mt : 1H) ; 4,45 (s : 2H) ; 6,28 (d, J = 8 Hz : 1H) ; 6,44 (s large : 1H) ; 6,46 (d large, J = 5,5 Hz : 1H) ; 7,68 (d large, J = 9 Hz : 2H) ; de 7,85 à 7,95 (mt : 3H).

### Exemple 225 : 5,5-diméthyl-1-(2-isopropylamino-pyridin-4-ylméthyl)-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,2 ml de N-Méthylpyrrolidone-2, 0,4 ml d'isopropylamine, est placé dans un four à micro-ondes Personal Chemistry Emrys Optimiser. Le mélange est irradié sous agitation magnétique à 200°C pendant environ 70 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 10 et 35 ml sont concentrées à sec sous pression réduite. On obtient ainsi 0,008 g de 5,5-diméthyl-1-(2-isopropylamino-pyridin-4-ylméthyl)-3-(4-trifluorométhanesulfanyl-phényl)-imidazolidine-2,4-dione sous forme de poudre amorphe dont les caractéristiques sont les suivantes :

| | | |
|---|---|---|
| Masse IE | | |
| m/z=452 | M^{+.} | |
| m/z=437 | (M - CH₃)⁺ | pic de base |
| m/z=410 | (M - C₃H₆)^{+.} | |
| m/z=219 | C₈H₄NOSF₃^{+.} | |
| m/z=134 | C₈H₁₀N₂^{+.} | |
| m/z=58 | C₃H₈N⁺ | |

Spectre de R.M.N. ¹H (300 MHz, (CD₃)₂SO d6, δ en ppm) : 1,13 (d, J = 6,5 Hz : 6H) ; 1,42 (s : 6H) ; 3,98 (mt : 1H) ; 4,45 (s large : 2H) ; 6,27 (d large, J = 7,5 Hz : 1H) ; 6,42 (s large : 1H); 6,46 (d large, J = 5,5 Hz : 1H) ; 7,68 (d large, J = 8 Hz : 2H) ; 7,88 (d large, J = 8 Hz : 2H) ; 7,90 (d, J = 5,5 Hz : 1H).

### Exemple 226 : 5,5-diméthyl-1-(2-pipéridino-pyridin-4-ylméthyl)-3-(4-trifluorométhanesulfanyl-phényl) - imidazolidine-2,4-dione

Un tube scellé de 2,5 ml contenant 0,1 g de 5,5-Diméthyl-1-(2-chloro-pyridin-4-ylméthyl)-3-(4-trifluorométhanesulfanyl-phényl)-imidazolidine-2,4-dione, 0,2 ml de N-méthylpyrrolidone-2, 0,4 ml de pipéridine, est placé dans un four à micro-ondes Personal Chemistry Emrys Optimiser. Le mélange est irradié sous agitation magnétique à 200°C pendant environ 30 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 10 et 30 ml sont concentrées à sec sous pression réduite. On obtient ainsi 0,07 g de 5,5-Diméthyl-1-(2-pipéridino-pyridin-4-ylméthyl)-3-(4-trifluorométhanesulfanyl-phényl)-imidazolidine-2,4-dione sous forme de poudre amorphe dont les caractéristiques sont les suivantes :

| | | |
|---|---|---|
| Masse IE | | |
| m/z=478 | M^{+.} | pic de base |
| m/z=449 | (M - C₂H₅)⁺ | |
| m/z=422 | (M - C₄H₈)^{+.} | |
| m/z=395 | (M - C₅H₉N)⁺ | |
| m/z=219 | C₈H₄NOSF₃^{+.} | |
| m/z=161 | C₁₀H₁₃N₂⁺ | |
| m/z=84 | C₅H₁₀N⁺ | |

Spectre de R.M.N. : ¹H (300 MHz, (CD₃)₂SO d6, δ en ppm) : 1,42 (s : 6H) ; de 1,45 à 1,65 (mt : 6H) ; 3,52 (t large, J = 5 Hz : 4H) ; 4,52 (s large : 2H) ; 6,64 (d large, J = 5,5 Hz : 1H) ; 6,83 (s large : 1H) ; 7,68 (d, J = 8,5 Hz : 2H) ; 7,88 (d, J = 8,5 Hz : 2H) ; 8,04 (d, J = 5,5 Hz : 1H).

### Exemple 227 : 5,5-diméthyl-1-[2-(4-méthylpipérazino)-pyridin-4-ylméthyl]-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,2 ml de N-méthylpyrrolidone-2, 0,4 ml de N-Méthylpipérazine, est placé dans un four à micro-ondes Personal Chemistry Emrys Optimiser. Le mélange est irradié sous agitation magnétique à 200°C pendant environ 30 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 105 et 135 ml sont concentrées à sec sous pression réduite. On obtient ainsi 0,028 g de 5,5-Diméthyl-1-[2-(4-méthylpipérazino)-pyridin-4-ylméthyl]-3-(4-trifluorométhanesulfanyl-phényl)-imidazolidine-2,4-dione sous forme de poudre amorphe dont les caractéristiques sont les suivantes :

| | | |
|---|---|---|
| Masse IE | | |
| m/z=493 | M^{+.} | |
| m/z=423 | (M - C₄H₈N)^{+.} | pic de base |
| m/z=219 | C₈H₄NOSF₃^{+.} | |
| m/z=176 | C₁₀H₁₄N₃⁺ | |

Spectre de R.N.N. : ¹H (300 MHz, (CD₃)₂SO d6, δ en ppm) : 1,42 (s : 6H) ; 2,23 (s : 3H) ; 2,40 (t large, J = 5 Ha : 4H) ; 3,50 (t large, J = 5 Hz : 4H) ; 4,53 (s large : 2H) ; 6,70 (d large, J = 5, 5 Hz : 1H) ; 6,85 (s large : 1H) ; 7,69 (d, J = 8,5 Hz : 2 H) ; 7,88 (d, J = 8,5 Hz : 2H) ; 8,07 (d, J = 5,5 Hz 1H).

### Exemple 228 : 5,5-diméthyl-1-(2-phénylamino-pyridin-4-ylméthyl)-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,2 ml de N-méthylpyrrolidone-2, 0,4 ml d'aniline, est placé dans un four à micro-ondes Personal Chemistry Emrys Optimiser. Le mélange est irradié sous agitation magnétique à 200°C pendant environ 30 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 40 et 85 ml sont concentrées à sec sous pression réduite. On obtient ainsi 0,082g de 5,5-Diméthyl-1-(2-phénylamino-pyridin-4-ylméthyl)-3-(4-trifluorométhanesulfanyl-phényl)-imidazolidine-2,4-dione sous forme de poudre amorphe dont les caractéristiques sont les suivantes :

| | | |
|---|---|---|
| Masse IE | | |
| m/z=486 | M^{+.} | |
| m/z=485 | (M - H)⁺ | pic de base |
| m/z=417 | (M - CF₃)⁺ | |
| m/z=182 | C₁₂H₁₀N₂⁺. | |

Spectre de R.M.N. : ¹H (300 MHz, (CD₃)₂SO d6, δ en ppm) : 1,46 (s : 6H) ; 4,55 (s large : 2H) ; 6,80 (d large, J = 5,5 Hz : 1H) ; 6,84 (s large : 1H) ; 6,88 (t large, J = 7,5 Hz : 1H) ; 7,25 (dd, J = 8 et 7,5 Hz : 2H) ; 7,67 (d large, J = 8 Hz : 2H) ; 7,69 (d, J = 8,5 Hz : 2H) ; 7,88 (d large, J = 8,5 Hz : 2H) ; 8,01 (d, J = 5,5 Hz : 1H) ; 9,01 (s large : 1H).

### Exemple 229 : 5,5-diméthyl-1-[2-(4-pipérazino)-pyridin-4-ylméthyl]-3-(4-trifluorométhanesulfanyl-phényl)-imidazolidine-2,4-dione

Un tube scellé de 2,5 ml contenant 0,1 g de 5,5-Diméthyl-1-(2-chloro-pyridin-4-ylméthyl)-3-(4-trifluorométhanesulfanyl-phényl)-imidazolidine-2,4-dione, 0,6 ml de N-méthylpyrrolidone-2, 0,123 g de pipérazine, est placé dans un four à micro-ondes Personal Chemistry Emrys Optimiser. Le mélange est irradié sous agitation magnétique à 200°C pendant environ 30 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 45 et 110 ml sont concentrées à sec sous pression réduite. On obtient ainsi 0,042 g de 5,5-Diméthyl-1-[2-(4-pipérazino)-pyridin-4-ylméthyl]-3-(4-trifluorométhanesulfanyl-phényl)-imidazolidine-2,4-dione sous forme de poudre amorphe dont les caractéristiques sont les suivantes :

| | | |
|---|---|---|
| Masse IC | | |
| m/z=480 | MH⁺ | pic de base |

Spectre de R.M.N. : ¹H (300 MHz, (CD₃)₂SO d6, δ en ppm) : 1,43 (s : 6H) ; 2,79 (t large, J = 5 Hz : 4H) ; 3,41(t large, J = 5 Hz : 4H) ; 4,52 (s large : 2H) ; 6,69 (d large, J = 5,5 Hz : 1H) ; 6,82 (s large : 1H) ; 7,69 (d large, J = 8,5 Hz : 2H) ; 7,88 (d, J = 8,5 Hz : 2H) ; 8,06 (d, J = 5,5 Hz : 1H).

### Exemple 230 : 5,5-diméthyl-1-(2-éthylamino-pyridin-4-ylméthyl)-3-(4-trifluorométhanesulfanyl-phényl)-imidazolidine-2,4-dione

Un tube scellé de 2,5 ml contenant 0,1 g de 5,5-Diméthyl-1-(2-chloro-pyridin-4-ylméthyl)-3-(4-trifluorométhanesulfanyl-phényl)-imidazolidine-2,4-dione, 0,5 ml de N-méthylpyrrolidone-2, 0,019 g de chlorhydrate d'éthylamine, 0,064 ml de triéthylamine est placé dans un four à micro-ondes Personal Chemistry Emrys Optimiser. Le mélange est irradié sous agitation magnétique à 200°C pendant environ 120 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 280 et 300 ml sont concentrées à sec sous pression réduite. On obtient ainsi 0,033 g de 5,5-Diméthyl-1-(2-éthylamino-pyridin-4-ylméthyl)-3-(4-trifluorométhanesulfanyl-phényl)-imidazolidine-2,4-dione sous forme de poudre amorphe dont les caractéristiques sont les suivantes :
PF = 136°C

| | | |
|---|---|---|
| Masse IE | | |
| m/z=438 | M^{+.} | |
| m/z=423 | (M - CH₃)⁺ | pic de base |
| m/z=395 | (M - C₂H₅N)⁺ | |
| m/z=369 | (M - CF₃)⁺ | |
| m/z=121 | C₇H₉N₂^{+.} | |
| m/z=44 | C₂H₆N⁺ | |

Spectre de R.M.N. ¹H (300 MHz, (CD₃)₂SO d6, δ en ppm) : 1,14 (t, J = 7 Hz : 3H) ; 1,44 (s : 6H) ; 3,26 (mt : 2H) ; 4,47 (s large : 2H) ; 6,40 (t large, J = 5.5 Hz : 1H) ; 6,45 (s large : 1H) ; 6,50 (d large, J = 5,5 Hz : 1H) ; 7,69 (d large, J = 8,5 Hz : 2H) ; 7,89 (d large, J = 8,5 Hz : 2H) ; 7,92 (d, J = 5,5 Hz : 1H).

### Exemple 231 : 5,5-diméthyl-1-(2-benzylamino-pyridin-4-ylméthyl)-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,2 ml de N-méthylpyrrolidone-2, 0,4 ml de benzylamine est placé dans un four à micro-ondes Personal Chemistry Emrys Optimiser. Le mélange est irradié sous agitation magnétique à 200°C pendant environ 30 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 10 et 50 ml sont concentrées à sec sous pression réduite. On obtient ainsi 0,035 g de 5,5-Diméthyl-1-(2-benzylamino-pyridin-4-ylméthyl)-3-(4-trifluoromêthanesulfanyl-phényl)-imidazolidine-2,4-dione sous forme de poudre amorphe dont les caractéristiques sont les suivantes :
PF 144°C

| | | |
|---|---|---|
| Masse IE | | |
| m/z=500 | M^{+.} | pic de base |
| m/z=431 | (M - CF₃)⁺ | |
| m/z=196 | C₁₃H₁₂N₂^{+.} | |
| m/z=106 | C₇H₈N⁺ | |
| m/z=91 | C₇H₇⁺ | |

Spectre de R.M.N. ¹H (300 MHz, (CD₃)₂SO d6, δ en ppm) : 1,39 (s : 6H) ; 4,47 (s large : 2H) ; 4,48 (d, J = 6 Hz : 2H) ; 6,51 (s large : 1H) ; 6,54 (d large, J = 5,5 Hz : 1H) ; 7,02 (t large, J = 6 Hz : 1H) ; de 7,15 à 7,35 (mt : 5H) ; 7,68 (d large, J = 8,5 Hz : 2H) ; 7,89 (d, J = 8,5 Hz : 2H) ; 7,92 (d, J = 5,5 Hz : 1H).

### Exemple 232 : 5,5-diméthyl-1-[2-(4-méthoxybenzylamino)-pyridin-4-ylméthyl]-3-(4-trifluorométhanesulfanyl-phényl)-imidazolidine-2,4-dione

Un tube scellé de 2,5 ml contenant 0,1 g de 5,5-diméthyl-1-(2-chloro-pyridin-4-ylméthyl)-3-(4-trifluorométhanesulfanyl-phényl)-imidazolidine-2,4-dione, 0,2 ml de N-méthylpyrrolidone-2, 0,4 ml de para méthoxybenzylamine est placé dans un four à micro-ondes Personal Chemistry Emrys Optimiser. Le mélange est irradié sous agitation magnétique à 200°C pendant environ 30 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 16 mm garnie avec 2 g de silice 15-35 µm conditionnée, puis éluée au dichlorométhane avec un débit de 5 ml/minute. Les fractions comprises entre 5 et 55 ml sont concentrées à sec sous pression réduite. On obtient ainsi 0,035 g de 5,5-diméthyl-1-[2-(4-méthoxybenzyl-amino)-pyridin-4-ylméthyl]-3-(4-trifluorométhanesulfanyl-phényl)-imidazolidine-2,4-dione sous forme de poudre amorphe dont les caractéristiques sont les suivantes :

| | | |
|---|---|---|
| Masse IE | | |
| m/z=530 | M^{+.} | |
| m/z=461 | (M - CF₃)⁺ | |
| m/z=136 | C₈H₁₀NO⁺ | |
| m/z=121 | C₈H₉O⁺ | pic de base |

Spectre de R.M.N. : ¹H (300 MHz, (CD₃)₂SO d6, δ en ppm) : 1,39 (s : 6H) ; 3,72 (s : 3H) ; 4,39 (d, J = 5,5 Hz : 2H) ; 4,46 (s large : 2H) ; 6,48 (s large : 1H) ; 6,53 (d large, J = 5,5 Hz : 1H) ; 6,87 (d large, J = 8,5 Hz : 2H) ; 6,93 (t, J = 5,5 Hz : 1H) ; 7,25 (d large, J = 8,5 Hz : 2H) ; 7,68 (d large, J = 8,5 Hz : 2H) ; 7,89 (d large, J = 8,5 Hz : 2H) ; 7,92 (d, J = 5,5 Hz : 1H).

### Exemple 233 : 5,5-diméthyl-1-(2-amino-pyridin-4-ylméthyl)-3-(4-trifluorométhanesulfanyl-phényl)-imidazolidine-2,4-dione.

A une solution de 0,035 g de 5,5-diméthyl-1-[2-(4-méthoxybenzylamino)-pyridin-4-ylméthyl]-3-(4-trifluorométhanesulfanyl-phényl)-imidazolidine-2,4-dione dans 0,5 ml de dichlorométhane, à une température voisine de 20°C, est ajoutés 0,5 ml d'acide trifluoroacétique, l'agitation est maintenue à cette température pendant 5 heures. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,015 g de 5,5-Diméthyl-1-(2-amino-pyridin-4-ylméthyl)-3-(4-trifluorométhanesulfanyl-phényl)-imidazolidine-2,4-dione sous forme de poudre dont les caractéristiques sont les suivantes :
PF : 161°C

| | | |
|---|---|---|
| Masse IE | | |
| m/z=410 | M^{+.} | pic de base |
| m/z=395 | (M - CH₃)⁺ | |
| m/z=219 | C₈H₄NOSF₃^{+.} | |
| m/z=148 | C₇H₆N₃O⁺ | |
| m/z=107 | C₆H₇N₂⁺ | |

Spectre de R.M.N. ¹H (300 MHz, (CD₃)₂SO d6, δ en ppm) : 1,43 (s : 6H) ; 4,46 (s large : 2H) ; 5,86 (s large : 2H) ; 6,46 (s large : 1H) ; 6,53 (d large, J = 5,5 Hz : 1H) ; 7,69 (d large, J = 8,5 Hz : 2H) ; 7,84 (d, J = 5,5 Hz : 1H) ; 7,89 (d large, J = 8,5 Hz : 2H).

### Exemple 234 : 5,5-diméthyl-1-(2-acétamido-pyridin-4-ylméthyl)-3-(4-trifluorométhanesulfanyl-phényl)-imidazolidine-2,4-dione

Une solution de 0,050 g de 5,5-diméthyl-1-(2-amino-pyridin-4-ylméthyl)-3-(4-trifluorométhanesulfanyl-phényl)-imidazolidine-2,4-dione dans 5 ml d'anhydride acétique, est agitée sous atmosphère inerte d'argon pendant 24 heures à une température voisine de 80°C. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,006 g de 5,5-Diméthyl-1-(2-acétamido-pyridin-4-ylméthyl)-3-(4-trifluorométhanesulfanyl-phényl)-imidazolidine-2,4-dione sous forme de poudre dont les caractéristiques sont les suivantes :

| | | |
|---|---|---|
| Masse IE | | |
| m/z=452 | M^{+.} | |
| m/z=410 | (M - C₂H₂O)^{+.} | pic de base |
| m/z=395 | (m/z=410 - CH₃)⁺ | |
| m/z=219 | C₈H₄NOSF₃^{+.} | |
| m/z=150 | C₈H₁₀N₂O^{+.} | |
| m/z=107 | C₆H₇N₂⁺ | |
| m/z=43 | C₂H₃O⁺ | |

Spectre de R.M.N. : ¹H (300 MHz, (CD₃)₂SO d6, δ en ppm) : 1,44 (s large : 6H) ; 2,11 (s : 3H) ; 4,65 (s large : 2H) ; 7,15 (d large, J = 5,5 Hz : 1H) ; 7,68 (d large, J = 8,5 Hz : 2H) ; 7,89 (d large, J = 8,5 Hz : 2H) ; 8,14 (s large : 1H) ; 8,27 (d, J = 5,5 Hz : 1H) ; 10,49 (mf : 1H).

### Exemple 235 : 5,5-diméthyl-1-(2-ter-butoxycarbonylamino-pyridin-4-ylméthyl)-3-(4-trifluorométhanesulfanyl-phényl)-imidazolidine-2,4-dione

A une solution de 0,029 g de dicarbonate de di-*ter*-butyl dans 0,65 ml d'alcool *ter*-butylique, sous atmosphère inerte d'argon à une température voisine de 20°C, est ajoutés par petites fractions 0,05g de 5,5-diméthyl-1-(2-amino-pyridin-4-ylméthyl)-3-(4-trifluorométhanesulfanyl-phényl)-imidazolidine-2,4-dione, l'agitation est maintenue à cette température pendant 24 heures. Le mélange réactionnel est concentré à sec sous pression réduite. Le résidu ainsi obtenu est purifié par flash chromatographie en utilisant une cartouche de diamètre 16 mm garnie avec 5 g de silice 20-40 µm conditionnée, puis éluée par un mélange (cyclohexane/acétate d'éthyle), (8/2), (v/v). Les fractions comprises entre 260 et 400 ml sont concentrées sous pression réduite. On obtient ainsi 0,035 g 5,5-diméthyl-1-(2-*ter*-butoxycarbonylamino-pyridin-4-ylméthyl)-3-(4-trifluorométhanesulfanyl-phényl)-imidazolidine-2,4-dione dont les caractéristiques sont les suivantes :

| | | |
|---|---|---|
| Masse IC | | |
| m/z=511 | MH⁺ | pic de base |

Spectre de R.M.N. ¹H (300 MHz, (CD₃))₂SO d6, δ en ppm) : 1,43 (s : 6H) ; 1,49 (s : 9H) ; 4,63 (s large : 2H) ; 7,08 (dd large, J = 5,5 et 1,5 Hz : 1H) ; 7,68 (d large, J = 9 Hz : 2H) ; 7,84 (s large : 1H) ; 7,87 (d large, J = 9 Hz : 2H) ; 8,19 (d, J = 5,5 Hz : 1H) 9,73(s large : 1H).

### Exemple 236 : 5,5-diméthyl-1-(2-méthylsulfonylamino-pyridin-4-ylméthyl)-3-(4-trifluorométhanesulfanyl-phényl)-imidazolidine-2,4-dione

A une solution de 0,050 g de 5,5-diméthyl-1-(2-amino-pyridin-4-ylméthyl)-3-(4-trifluorométhanesulfanyl-phényl)-imidazolidine-2,4-dione dans 0,4 ml de pyridine, sous atmosphère inerte d'argon à une température voisine de 20°C, est ajoutés 0,010 ml de chlorure de méthanesulfonyl, l'agitation est maintenue à cette température pendant 2 heures. Le mélange réactionnel est versé dans 50 ml d'une solution saturée de bicarbonate de sodium puis extrait par 2x50 ml d'acétate d'éthyle. La phase organique est séchée sur sulfate de magnésium, filtrée puis concentrée à sec sous pression réduite. On obtient ainsi 0,020, g de 5,5-diméthyl-1-(2-méthyl-sulfonylamino-pyridin-4-ylméthyl)-3-(4-trifluorométhanesulfanyl-phényl)-imidazolidine-2,4-dione sous forme de poudre blanche dont les caractéristiques sont les suivantes :
PF : 208°C

| | | |
|---|---|---|
| Masse IE | | |
| m/z=488 | M⁺ | pic de base |
| m/z=473 | (M - CH₃)⁺ | |
| m/z=409 | (M - SO₂CH₃)⁺ | |
| m/z=219 | C₈H₄NOSF₃^{+.} | |
| m/z=185 | C₇H₉N₂O₂S⁺ | |

Spectre de R.M.N. ¹H (300 MHz, (CD₃)₂SO d6, δ en ppm) : 1,45 (s : 6H) ; 3,27 (mf : 3H) ; 4,63 (s large : 2H) ; de 6,95 à 7, 10 (mf : 2H) ; 7,68 (d large, J = 8,5 Hz : 2H) ; 7,89 (d large, J = 8,5 Hz : 2H) ; 8,17 (mf : 1H).

### Exemple 237 : 5,5-diméthyl-1-(2-méthoxycarbonylamino-pyridin-4-ylméthyl)-3-(4-trifluorométhanesulfanyl-phényl)-imidazolidine-2,4-dione

A une solution de 0,100 g de 5,5-Diméthyl-1-(2-amino-pyridin-4-ylméthyl)-3-(4-trifluorométhanesulfanyl-phényl)-imidazolidine-2,4-dione dans 1,5 mL de pyridine, sous atmosphère inerte d'argon à une température voisine de 20°C, est ajoutés 0,056 mL de chloroformiate de méthyle, l'agitation est maintenue à cette température pendant 20 heures, est ajouté à nouveau 0, 56 ml chloroformiate de méthyle. L'agitation est maintenue à cette température pendant 20 heures. Le mélange réactionnel est repris par 5 ml d'acétate d'éthyle, puis lavé successivement par 5 ml d'eau, 5 ml d'une solution saturée en chlorure de sodium, puis 5 ml d'eau. La phase organique est séchée sur sulfate de magnésium, filtrée puis concentrée à sec sous pression réduite. Le résidu obtenu est purifié sur une colonne KROMASIL C18, 5 µm (100 x 20 mm), en éluant par un gradient linéaire de 5 à 95 % d'acétonitrile contenant 0,07% (v/v) d'acide trifluoroacétique (TFA) dans l'eau contenant 0,07 % (v/v) TFA à un débit de 10 mL/mn. Les fractions contenant l'attendu sont concentrées à sec sous pression réduite. Le résidu obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,024 g de 5,5-diméthyl-1-(2-méthoxycarbonylamino-pyridin-4-ylméthyl)-3-(4-trifluorométhanesulfanyl-phényl)-imidazolidine-2,4-dione sous forme de solide blanc dont les caractéristiques sont les suivantes :

| | | |
|---|---|---|
| Masse IE | | |
| m/z=468 | M⁺ | pic de base |
| m/z=453 | (M - CH₃)⁺ | |
| m/z=410 | (M - C₂H₂O₂)⁺ | |
| m/z=219 | C₈H₄NOSF₃^{+.} | |
| m/z=166 | C₈H₁₀N₂O₂^{+.} | |

Spectre de R.M.N. ¹H (300 MHz, (CD₃)₂SO d6, δ en ppm) : 1,44 (s : 6H) ; 3,70 (s : 3H) ; 4,65 (s large : 2H) ; 7,12 (dd, J = 5,5 et 2 Hz : 1H) ; 7,69 (d large, J = 9 Hz : 2H) ; 7,89 (d large, J = 9 Hz : 2H) ; 7,90 (s large : 1H) ; 8,23 (d, J = 5,5 Hz : 1H) ; 10,18 (s large : 1H).

### Exemple 238 : 5,5-diméthyl-1-(2-chloro-pyridin-4-ylméthyl)-3-(3-chloro-4-trifluorométhanesulfanyl-phényl)-imidazolidine-2,4-dione

A une solution de 0,417 g de 5,5-diméthyl-3(3-chloro-4-trifluorométhylsulfanyl-phényl)-imidazolidine-2,4-dione dans 20 ml de diméthylformamide anhydre, sous atmosphère inerte d'argon à une température voisine de 0°C, est ajouté 0,1 g d'hydrure de sodium, l'agitation est maintenue à cette température pendant 30 minutes, est ajouté une solution de 0,380 g de 2-chloro-4-(bromométhyl)pyridine dans 20 ml de diméthylformamide anhydre puis de l'eau glacée après 10 minutes de réaction. Le mélange réactionnel est repris avec 400 ml d'acétate d'éthyle puis lavé par 400 mL d'eau. La phase organique est séchée sur sulfate de magnésium, filtrée puis concentrées à sec sous pression réduite. Le résidu ainsi obtenu est purifié par flash chromatographie en utilisant une cartouche de diamètre 27 mm garnie avec 25 g de silice 20-40 µm conditionnée, puis éluée par un mélange (cyclohexane/acétate d'éthyle), (8/2), (v/v). Les fractions comprises entre 150 et 350 ml sont concentrées à sec sous pression réduite. On obtient ainsi 0,3 g 5,5-diméthyl-1-(2-chloro-pyridin-4-ylméthyl)-3-(3-chloro-4-trifluorométhanesulfanyl-phényl)-imidazolidine-2,4-dione dont les caractéristiques sont les suivantes :

| | | |
|---|---|---|
| Masse IE | | |
| m/z=463 | M⁺ | pic de base |
| m/z=448 | (M - CH₃)⁺ | |
| m/z=253 | C₈H₃NOSClF₃⁺ | |
| m/z=167 | C₇H₄N₂OCl⁺ | |
| m/z=126 | C₆H₅NCl⁺ | |

Spectre de R.M.N. ¹H (300 MHz, (CD₃)₂SO d6, δ en ppm) : 1,43 (s : 6H) ; 4,68 (s large : 2H) ; 7,49 (d large, J = 5,5 Hz : 1H) ; 7,63 (s large : 1H) ; 7,71 (dd, J = 9 et 3 Hz : 1H) ; 7,97 (d, J = 3 Hz : 1H) ; 8,05 (d, J = 9 Hz : 1H) ; 8,38 (d, J = 5,5 Hz : 1H).

### Préparation du 5,5-diméthyl-3(3-chloro-4-trifluorométhylsulfanyl-phényl)-imidasolidine-2,4-dione

A une suspension de 1,97 g diphosgène et de 0,03 g de noir végétal dans 22 ml de toluène, à une température voisine de -20°C, est ajouté en 30 minutes, une solution de 2 g de 3-chloro-4-trifluorométhylsulfanyl-aniline dans 22 ml de toluène. Le mélange est agité jusqu'à une température voisine de 20 °C, puis chauffé au reflux pendant 3 heures. Le mélange est refroidi à une température voisine de 20°C, puis filtré sur célite, 1,35 g de chlorhydrate de l'ester méthylique de l'acide α-aminoisobutyrique, 15 ml de toluène et 2,23 ml de triéthylamine sont ajoutés au filtrat. Le mélange ainsi obtenu est porté au reflux pendant 24 heures puis refroidi à une température voisine de 20°C. Le mélange réactionnel est concentré sous pression réduite, le résidu obtenu est purifié par flash-chromatographie sur une cartouche de diamètre 37 mm garnie de silice 20-40 µm, conditionnée puis éluée par le mélange (cyclohexane/acétate d'éthyle), (5/5), (v/v). Les fractions comprises entre 110 et 250 ml sont concentrées sous pression réduite et l'on obtient ainsi 2 g de 5,5-diméthyl-3(3-chloro-4-trifluorométhylsulfanyl-phényl)-imidazolidine-2,4-dione dont les caractéristiques sont les suivantes :

| | | |
|---|---|---|
| Masse IE | | |
| m/z=338 | M⁺ | pic de base |
| m/z=253 | C₈H₃NOSClF₃⁺ | |
| m/z=184 | (m/z=253 - CF₃)⁺ | |
| m/z=84 | C₄H₆NO⁺ | |

Spectre R.M.N. : ¹H (300 MHz, (CD₃)₂SO d6, δ en ppm) : 1,42 (s : 6H) ; 7,63 (dd, J = 8,5 et 2,5 Hz : 1H) ; 7,90 (d, J = 2,5 Hz : 1H) ; 8,02 (d, J = 8,5 Hz : 1H) ; 8,75 (mf : 1H).

### Exemple 239 : 5,5-Diméthyl-1-[2-(4-méthoxybenzylamino)-pyridin-4-ylméthyl]-3-(3-chloro-4-trifluorométhane-sulfanyl-phényl)--imidazsolidine-2,4-dione

Un tube scellé de 2,5 ml contenant 0,3 g de 5,5-diméthyl-1-(2-chloro-pyridin-4-ylméthyl)-3-(3-chloro-4-trifluorométhanesulfanyl-phényl)-imidazolidine-2,4-dione, 0,2 ml de N-méthylpyrrolidone-2, 0,4 ml de para méthoxybenzylamine est placé dans un four à micro-ondes Personal Chemistry Emrys Optimiser. Le mélange est irradié sous agitation magnétique à 200°C pendant environ 70 minutes. Le mélange réactionnel est purifié par flash chromatographie en utilisant une cartouche de diamètre 37 mm garnie avec 50 g de silice 20-40 µm conditionnée, puis éluée par un mélange (cyclohexane/acétate d'éthyle), (8/2), (v/v). Les fractions comprises entre 470 et 640 ml sont concentrées à sec sous pression réduite. On obtient ainsi 0,091 g 5,5-Diméthyl-1-[2-(4-mêthoxybenzylamino)-pyridin-4-ylméthyl]-3-(3-chloro-4-trifluorométhanesulfanyl-phényl)-imidazolidine-2,4-dione dont les caractéristiques sont les suivantes :

| | | |
|---|---|---|
| Masse IE | | |
| m/z=564 | M⁺ | |
| m/z=495 | (M - CF₃)⁺ | |
| m/z=136 | C₈H₁₀NO⁺ | |
| m/z=121 | C₈H₉O⁺ | pic de base |

Spectre de R.M.N. : ¹H (300 MHz, (CD₃)₂SO d6, δ en ppm) : 1,38 (s : 6H) ; 3,71 (s : 3H) ; 4,39 (d, J = 5,5 Hz : 2H) ; 4,45 (s large : 2H) ; 6,48 (s large : 1H) ; 6,53 (d large, J = 5,5 Hz : 1H) ; 6,85 (d, J = 8,5 Hz : 2H) ; 6,89 (t, J = 5,5 Hz : 1H) ; 7,24 (d, J = 8,5 Hz : 2H) ; 7,69 (dd, J = 8,5 et 2,5 Hz : 1H) ; 7,92 (d, J = 5,5 Hz : 1H) ; 7,95 (d, J = 2,5 Hz : 1H) ; 8,06 (d, J = 8,5 Hz : 1H).

### Exemple 240 : 5,5-diméthyl-1-(2-éthylamino-pyridin-4-ylméthyl)-3-(3-chloro-4-trifluorométhanesulfanyl-phényl)-imidazolidine-2-4-dione

Un tube scellé de 2,5 ml contenant 0,1 g de 5,5-diméthyl-1-(2-chloro-pyridin-4-ylméthyl)-3-(3-chloro-4-trifluorométhanesulfanyl-phényl)-imidazolidine-2,4-dione, 0,4 ml de N-méthylpyrrolidone-2, 0,044 g de chlorhydrate d'éthylamine, 0,120 ml de triéthylamine est placé dans un four à micro-ondes Personal Chemistry Emrys Optimiser. Le mélange est irradié sous agitation magnétique à 200°C pendant environ 70 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,029 g de 5,5-Diméthyl-1-(2-éthylamino-pyridin-4-ylméthyl)-3-(3-chloro-4-trifluorométhanesulfanyl-phényl)-imidazolidine-2,4-dione dont les caractéristiques sont les suivantes :

| | | |
|---|---|---|
| Masse IE | | |
| m/z=472 | M^{+.} | |
| m/z=457 | (M - CH₃)⁺ | pic de base |
| m/z=429 | (M - C₂H₅N)⁺ | |
| m/z=403 | (M - CF₃)⁺ | |
| m/z=121 | C₇H₉N₂^{+·} | |
| m/z=44 | C₂H₆N⁺ | |

Spectre de R.M.N. ¹H (300 MHz, (CD₃)₂SO d6, δ en ppm) : 1,14 (t, J = 7 Hz : 3H) ; 1,43 (s : 6H) ; 3,27 (mt : 2H) ; 4,46 (s large : 2H) ; 6,38 (t large, J = 5,5 Hz : 1H) ; 6,45 (s large : 1H) ; 6,51 (d large, J = 5,5 Hz : 1H) ; 7,70 (dd, J = 8,5 et 2,5 Hz : 1H) ; 7,92 (d, J = 5,5 Hz : 1H) ; 7,96 (d, J = 2,5 Hz : 1H) ; 8,06 (d, J = 8,5 Hz : 1H).

### Exemple 241 : 5,5-diméthyl-1-(2-méthylamino-pyridin-4 ylméthyl)-3-(3-chloro-4-trifluorométhanesulfanyl-phényl)-imidazolidine-2,4-dione

Un tube scellé de 2,5 ml contenant 0,1 g de 5,5-Diméthyl-1-(2-chloro-pyridin-4-ylméthyl)-3-(3-chloro-4-trifluorométhanesulfanyl-phényl)-imidazolidine-2,4-dione, 0,5 ml de N-méthylpyrrolidone-2, 0,030 g de chlorhydrate de méthylamine, 0,120 ml de triéthylamine, est placé dans un four à micro-ondes Personal Chemistry Emrys Optimiser. Le mélange est irradié sous agitation magnétique à 200°C pendant environ 70 minutes. Le mélange réactionnel est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. Le résidu ainsi obtenu est purifié par flash chromatographie en utilisant une cartouche de diamètre 20 mm garnie avec 10 g de silice 20-40 µm conditionnée, puis éluée au dichlorométhane avec un débit de 8 ml/minute Les fractions comprises entre 70 et 100 ml sont concentrées à sec sous pression réduite. On obtient ainsi 0,015 g de 5,5-Diméthyl-1-(2-méthylamino-pyridin-4-ylméthyl)-3-(3-chloro-4-trifluorométhanesulfanyl-phényl)-imidazolidine-2,4-dione sous forme de poudre blanche amorphe dont les caractéristiques sont les suivantes :
Spectre R.M.N. : ¹H (300. MHz, (CD₃)₂SO d6, δ en ppm) : 1,43 (s : 6H) ; 2,77 (d, J = 5 Hz : 3H) ; 4,48 (s large : 2H) ; 6,40 (q large, J = 5 Hz : 1H) ; 6,46 (s large : 1H) ; 6,54 (d large, J = 5,5 Hz : 1H) ; 7,70 (dd, J = 8,5 et 3 Hz : 1H) ; 7,94 (d, J = 5,5 Hz : 1H) ; 7,97 (d, J = 3 Hz : 1H) ; 8,06 (d, J = 8,5 Hz : 1H).

### Exemple 242 : 5,5-diméthyl-1-(2-amino-pyridin-4-ylméthyl)-3-(3-chloro-4-trifluorméthanesulfanyl-phényl)-imidazolidine-2,4-dione

A une solution de 0,080 g de 5,5-diméthyl-1-[2-(4-méthoxybenzylamino)-pyridin-4-ylméthyl]-3-(3-chloro-4-trifluorométhanesulfanyl-phényl)-imidazolidine-2,4-dione dans 1 ml de dichlorométhane, à une température voisine de 20°C, est ajoutés 3 ml d'acide trifluoroacétique, l'agitation est maintenue à cette température pendant environ 16 heures. Le mélange réactionnel est concentré à sec sous pression réduite, le résidu obtenu est purifié par LC/MS préparative. Les fractions contenant l'attendu sont concentrées à sec sous pression réduite. Le résidu ainsi obtenu est repris dans du méthanol puis déposé sur une cartouche BOND ELUT VARIAN de référence 1225-6027 contenant 5 g de phase SCX conditionnée au méthanol. On procède par un lavage au méthanol suivi d'une élution au méthanol ammoniacal 2M. Les fractions ammoniacales sont concentrées à sec sous pression réduite. On obtient ainsi 0,027 g de 5,5-Diméthyl-1-(2-amino-pyridin-4-ylméthyl)-3-(3-chloro-4-trifluorométhanesulfanyl-phényl)-imidazolidine-2,4-dione sous forme de poudre dont les caractéristiques sont les suivantes :

| | | |
|---|---|---|
| Masse IE | | |
| m/z=444 | M^{+·} | pic de base |
| m/z=429 | (M - CH₃)⁺ | |
| m/z=148 | C₇H₆N₃O⁺ | |
| m/z=107 | C₆H₇N₂⁺ | |

Spectre de R.M.N. : ¹H (300 MHz, (CD₃)₂SO d6, δ en ppm) : 1,43 (s : 6H) ; 4,46 (s large : 2H) ; 5,86 (s large : 2H) ; 6,46 (s large : 1H) ; 6,54 (dd large, J = 5,5 et 1,5 Hz : 1H) ; 7,70 (dd, J = 8,5 et 2,5 Hz : 1H) ; 7,86 (d, J = 5,5 Ha : 1H) ; 7,97 (d, J = 2,5 Hz : 1H) ; 8,06 (d, J = 8,5 Hz : 1H).

### Exemple 243 : 5,5-diméthyl-1-(2,6-dibrimo-pyridin-4-ylméthyl)-3-(4-trifluorométhoxy-phényl)-imidazolidine-2,4-dione

A une solution de 0,230 g de 5,5-diméthyl-3-(4-trifluorométhylsulfanyl-phényl)-imidazolidine-2,4-dione dans 2 ml de diméthylformamide anhydre, sous atmosphère inerte d'argon à une température voisine de 20°C, est ajouté 0,036 g d'hydrure de sodium, l'agitation est maintenue à cette température pendant 30 minutes, est ajouté une solution de 0,349 g de 2,6-dibromo-4-(bromométhyl)pyridine dans 3 ml de diméthylformamide anhydre puis de l'eau glacée après 20 minutes de réaction. Le mélange réactionnel est repris par de l'acétate d'éthyle puis lavé à l'eau. La phase organique est séchée sur sulfate de magnésium, filtrée puis concentrées à sec sous pression réduite. Le résidu ainsi obtenu est purifié par flash chromatographie en utilisant une cartouche de diamètre 27 mm garnie avec 25 g de silice 20-40 µm conditionnée, puis éluée au dichlorométhane. Les fractions comprises entre 65 et 135 ml sont concentrées sous pression réduite. On obtient ainsi 0,25 g de 5,5-diméthyl-1-(2,6-dibrimo-pyridin-4-ylméthyl)-3-(4-trifluorométhoxy-phényl)-imidazolidine-2,4-dione dont les caractéristiques sont les suivante

| | | |
|---|---|---|
| Masse IC | | |
| m/z=569 | MNH₄⁺ | pic de base |
| m/z=552 | MH⁺ | |
| m/z=474 | (M - Br + 2H)⁺ | |
| m/z=396 | (m/z=474 - Br + H)⁺ | |

Spectre de R.M.N. : ¹H (300 MHz, (CD₃)₂SO d6, δ en ppm) : 1,45 (s : 6H) ; 4,67 (s large : 2H) ; 7,72 (d large, J = 8 Hz : 2H) ; 7,83 (s : 2H) ; 7,89 (d large, J = 8 Hz : 2H).

Le composé 5,5-diméthyl-3-(4-trifluorométhylsulfanyl-phényl)-imidazolidine-2,4-dione est décrit dans l'exemple 211.

### Synthèse des réactifs

### Référence Exemple 203a : 2-chloro-4-(bromométhyl)pyridine

A une solution de 0,706 g de dibromotriphénylphosphorane dans 9,5 ml de dichlorométhane sous atmosphère inerte d'argon à une température voisine de 20°C, est ajouté goutte à goutte une solution de 0,2 g de 2-chloro-4-(hydroxyméthyl)pyridine dans 2,5 ml de dichlorométhane, l'agitation est maintenue à cette température pendant environ 1 heure. Le milieu réactionnel est repris par 50 ml de dichlorométhane puis lavé par 3x50 ml d'eau. La phase organique est séchée sur sulfate de magnésium, filtrée puis concentrées à sec sous pression réduite. Le résidu ainsi obtenu est purifié par flash chromatographie en utilisant une cartouche de diamètre 20 mm garnie avec 10 g de silice 20-40 µm conditionnée, puis éluée par un mélange (cyclohexane/acétate d'éthyle), (8/2), (v/v) avec un débit de 8 ml/minute. Les fractions comprises entre 8 et 24 mal sont concentrées sous pression réduite. On obtient ainsi 0,1 g de 2-chloro-4-(bromométhyl)pyridine.

### Référence Exemple 208b : 2-chloro-4-(hydroxyméthyl) pyridine

A une solution de 1,7 g de 2-chloropyridine-4-carboxylate d'éthyle dans 20 ml d'éthanol, sous atmosphère inerte d'argon à une température voisine de 0°C, est ajouté en plusieurs fois 2,04 g de borohydrure de sodium. Le mélange réactionnel est porté au reflux sous agitation pendant 3 heures puis concentré à sec sous pression réduite. Le résidu ainsi obtenu est repris par du dichlorométhane puis lavé à l'eau. La phase organique est séchée sur sulfate de magnésium, filtrée puis concentrée à sec sous pression réduite. On obtient ainsi 1 g de 2-chloro-4-(hydroxyméthyl)pyridine.

### Référence Exemple 208c : 2-chloropyridine-4-carboxylate d'éthyle

A une solution de 2,2 g d'acide 2-chloropyridine-4-carboxylique dans 30 ml d'éthanol, est ajouté 1 ml d'acide sulfurique concentré. Le mélange réactionnel est porté au reflux sous agitation pendant 16 heures puis concentré à sec sous pression réduite. Le résidu ainsi obtenu est repris par 30 ml d'eau puis extrait par 3x30 ml de dichlorométhane. La phase organique est séchée sur sulfate de magnésium, filtrée puis concentrées à sec sous pression réduite. L'huile ainsi obtenue est purifiée par filtration sur 13 g de silice en éluant au dichlorométhane. Les fractions contenant l'attendu sont concentrées à sec sous pression réduite. On obtient ainsi 2,1 g de 2-chloropyridine-4-carboxylate d'éthyle.

### Référence Exemple 209a : 2-éthoxy-4-(bromométhyl)pyridine

A une solution de 1,025 g de dibromotriphénylphosphorane dans 12 ml de dichlorométhane sous atmosphère inerte d'argon à une température voisine de 20°C, est ajouté goutte à goutte une solution de 0,31 g de 2-éthoxy-4-(hydroxyméthyl)pyridine dans 6 ml de dichlorométhane, l'agitation est maintenue à cette température pendant environ 1 heure. Le milieu réactionnel est repris par 100 ml de dichlorométhane puis lavé par 3x100 ml d'eau. La phase organique est séchée sur sulfate de magnésium, filtrée puis concentrées à sec sous pression réduite. Le résidu ainsi obtenu est purifié par flash chromatographie en utilisant une cartouche de diamètre 27 mm garnie avec 20 g de silice 20-40 µm conditionnée, puis éluée par un mélange (cyclohexane/acétate d'éthyle), (9/1), (v/v) avec un débit de 8 ml/minute Les fractions comprises entre 80 et 180 ml sont concentrées sous pression réduite. On obtient ainsi 0,37 g de 2-éthoxy-4-(bromométhyl)pyridine.

### Référence Exemple 209b : 2-éthoxy-4-(hydroxyméthyl) pyridine

A une solution de 0,524 g de 2-éthoxypyridine-4-carboxylate d'éthyle dans 5,2 ml d'éthanol, sous atmosphère inerte d'argon à une température voisine de 0°C, est ajouté en plusieurs fois 0,508 g de borohydrure de sodium. Le mélange réactionnel est porté au reflux sous agitation pendant 3 heures puis concentré à sec sous pression réduite. Le résidu ainsi obtenu est repris par 50 ml de dichlorométhane puis lavé par 50 ml d'eau. La phase organique est séchée sur sulfate de magnésium, filtrée puis concentrée à sec sous pression réduite. On obtient ainsi 0,310 g de 2-éthoxy-4-(hydroxyméthyl) pyridine.

### Référence Exemple 209c : 2-éthoxypyridine-4-carboxylate d'éthyle

A une solution de 1 g d'acide 2-fluoropyridine-4-carboxylique dans 15 ml d'éthanol, est ajouté 0,5 ml d'acide sulfurique concentré. Le mélange réactionnel est porté au reflux sous agitation pendant 48 heures puis concentré à sec sous pression réduite. Le résidu ainsi obtenu est repris par 100 ml d'eau puis extrait par 3x100 ml de dichlorométhane. La phase organique est séchée sur sulfate de magnésium, filtrée puis concentrée à sec sous pression réduite. Le brut ainsi obtenu est purifié par flash chromatographie en utilisant en utilisant une cartouche de diamètre 37 mm garnie avec 50 g de silice 20-40 µm conditionnée, puis éluée par un mélange (dichlorométhane/méthanol), (90/10), (v/v) à un débit de 8 ml par minute. Les fractions comprises entre 350 ml et 390 ml sont concentrées sous pression réduite. On obtient ainsi 0,524 g de 2-éthoxypyridine-4-carboxylate d'éthyle sous forme d'huile.

### Référence Exemple 210a : 2-éthyl-4-(bromométhyl)pyridine

A une solution de 1,25 g de dibromotriphénylphosphorane dans 4 ml de dichlorométhane sous atmosphère inerte d'argon à une température voisine de 0°C, est ajouté goutte à goutte une solution de 0,3 g de 2-éthyl-4-(hydroxyméthyl)pyridine dans 3,5 ml de dichlorométhane. Le mélange est agité jusqu'à une température voisine de 20°C pendant environ 1 heure. Le milieu réactionnel est repris par 50 ml de dichlorométhane puis lavé par 3x50 ml d'eau. La phase organique est séchée sur sulfate de magnésium, filtrée puis concentrées à sec sous pression réduite. Le résidu ainsi obtenu est purifié par flash chromatographie en utilisant une cartouche de diamètre 20 mm garnie avec 10 g de silice 20-40 µm conditionnée, puis éluée par un mélange (cyclohexane/acétate d'éthyle), (9/1), (v/v) avec un débit de 8 ml/minute. Les fractions comprises entre 80 et 120 ml sont concentrées sous pression réduite. On obtient ainsi 0,062 g de 2-étyhl-4-(bromométhyl)pyridine.

### Référence Exemple 210b : 2-éthyl-4-(hydroxyméthyl) pyridine

A une solution de 5,08 g de 2-éthylpyridine-4-carboxylate d'éthyle dans 53 ml d'éthanol, sous atmosphère inerte d'argon à une température voisine de 0°C, est ajouté en plusieurs fois 5,36 g de borohydrure de sodium. Le mélange réactionnel est porté au reflux sous agitation pendant 3 heures puis concentré à sec sous pression réduite. Le résidu ainsi obtenu est repris par 500 ml de dichlorométhane puis lavé par 500 ml d'eau. La phase organique est séchée sur sulfate de magnésium, filtrée puis concentrée à sec sous pression réduite. On obtent ainsi 3,16 g de 2-éthyl-4-(hydroxyméthyl)pyridine.

### Référence Exemple 210c : 2-éthylpyridine-4-carboxylate d'éthyle

A une solution de 5 g d'acide 2-éthylpyridine-4-carboxylique dans 75 ml d'éthanol, est ajouté 2,35 ml d'acide sulfurique concentré. Le mélange réactionnel est porté au reflux sous agitation pendant environ 64 heures puis concentré à sec sous pression réduite. Le résidu ainsi obtenu est repris par 500 ml d'eau puis extrait par 500 ml de dichlorométhane. La phase organique est séchée sur sulfate de magnésium, filtrée puis concentrée à sec sous pression réduite. On obtient ainsi 5,08 g de 2-éthylpyridine-4-carboxylate d'éthyle sous forme d'huile.

### Référence Exemple 214a : 2-bromo-4-(bromométhyl)pyridine

A une solution de 0,172 g de 2-bromo-4-méthylpyridine dans 2,5 ml de tétrachlorure de carbone, sous atmosphère inerte d'argon à une température voisine de 20°C, est ajouté 0,073 ml d'acide acétique, le mélange est porté sous agitation à une température de 50°C, à cette température est ajouté successivement 0,356 g de N-bromosuccinimide et 0,048 g de peroxyde de benzoyle. Le mélange est porté à 80°C pendant environ 18 heures. Le milieu réactionnel est déposé après refroidissement sur une cartouche de diamètre 20 mm garnie avec 10 g de silice 20-40 µm non conditionnée au préalable puis éluée au dichlorométhane avec un débit de 5 ml/minute. Les fractions comprises entre 30 et 40 ml sont concentrées à sec sous pression réduite. On obtient ainsi 0,071 g de 2-bromo-4-(bromométhyl)pyridine.

### Référence Exemple 215a : 2-fluoro-4-(bromométhyl)pyridine

A une solution de 0,111 g de 2-fluoro-4-méthylpyridine dans 2,5 ml de tétrachlorure de carbone, sous atmosphère inerte d'argon à une température voisine de 20°C, est ajouté 0,073 ml d'acide acétique, le mélange est porté sous agitation à une température de 50°C, à cette température est ajouté successivement 0,356 g de N-bromosuccinimide et 0,048 g de peroxyde de benzoyle. Le mélange est porté à 80°C pendant environ 16 heures. Le milieu réactionnel est déposé après refroidissement sur une cartouche de diamètre 20 mm garnie avec 10 g de silice 20-40 µm non conditionnée au préalable puis éluée au dichlorométhane avec un débit de 5 ml/minute. Les fractions comprises entre 28 et 38 ml sont concentrées à sec sous pression réduite. On obtient ainsi 0,063 g de 2-fluoro-4-(bromométhyl)pyridine.

### Référence Exemple 216a : 2-cyano-4-(bromométhyl)pyridine

A une solution de 0,236 g de 2-cyano-4-méthylpyridine dans 5 ml de tétrachlorure de carbone, sous atmosphère inerte d'argon à une température voisine de 20°C, est ajouté 0,146 ml d'acide acétique, le mélange est porté sous agitation à une température de 50°C, à cette température est ajouté successivement 0,712 g de N-bromosuccinimide et 0,096 g de peroxyde de benzoyle. Le mélange est porté à 80°C pendant environ 18 heures. Le milieu réactionnel est déposé après refroidissement sur une cartouche de diamètre 27 mm garnie avec 25 g de silice 20-40 µm non conditionnée au préalable puis éluée au dichlorométhane avec un débit de 10 ml/minute. Les fractions comprises entre 140 et 175 ml sont concentrées à sec sous pression réduite. On obtient ainsi 0,097 g de 2-cyano-4-(bromométhyl)pyridine.

### Référence Exemple 243a : 2,6-dibromo-4-(bromométhyl) pyridine

A une solution de 0,95 g de dibromotriphénylphosphorane dans 5 ml de dichlorométhane sous atmosphère inerte d'argon à une température voisine de 0°C, est ajouté goutte à goutte une solution de 0,5 g de 2,6-dibromo-4-(hydroxyméthyl)pyridine dans 7 ml de dichlorométhane. Le mélange est agité jusqu'à une température voisine de 20°C pendant environ 2 heures. Le milieu réactionnel est repris par 100 ml de dichlorométhane puis lavé par 100 ml d'eau. La phase organique est séchée sur sulfate de magnésium, filtrée puis concentrées à sec sous pression réduite. Le résidu ainsi obtenu est purifié par flash chromatographie en utilisant une cartouche de diamètre 37 mm garnie avec 50 g de silice 20-40 µm conditionnée, puis éluée par un mélange (cyclohexane/acétate d'éthyle), (9/1), (v/v) avec un débit de 8 ml/minute. Les fractions comprises entre 135 et 200 ml sont concentrées sous pression réduite. On obtient ainsi 0,349 de 2,6-dibromo-4-(bromométhyl)pyridine.

### Mode opératoire général des produits des exemples 244 à 255

A une suspension de 0,064 ml de diphosgène (0,53 mM) et de 20 mg de noir végétal dans 1 ml de toluène, à une température voisine de -20°C, est ajouté une solution de 0,481 mM de dérivé anilino dans 0,6 ml de toluène. Le mélange est agité jusqu'à une température voisine de 20°C, puis chauffé au reflux pendant 3 heures. Le mélange est refroidi à une température voisine de 20°C, puis filtré sur célite. Au filtrat est ajoutée une solution de 100 mg de l'ester méthylique de l'acide-2-méthyl-2-[(pyridin-4-ylméthyl)-amino]-propanoique (0,48 mM) dans 0,6 ml de toluène. Le mélange ainsi obtenu est porté au reflux pendant 16 heures puis refroidi à une température voisine de 20°C. Le précipité est filtré et le filtrat est concentré sous pression réduite. Le résidu obtenu est purifié par LC/MS préparative.

**Tableau récapitulatif des dérivés anilino de départ et des produits préparés**

| **Exemple N°** | **Nom du dérivé anilino de départ** | **Quantité du dérivé anilino utilisée (mg)** | **Structure du produit obtenu** | **Nom du produit obtenu** | **Formule Brut du produit obtenu** | **Poids Moléculaire du produit obtenu** | **Quantité obtenue (mg)** |
|---|---|---|---|---|---|---|---|
| 244 | 2-NITRO-4-(TRIFLUORO-METHOXY)ANILINE | 106,7 | 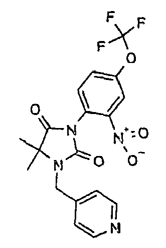 | 5,5-Diméthyl-3-(2-nitro-4-trifluorométhoxy-phenyl)-1-pyridin-4-ylméthyl-imidazolidine-2,4-dione 2 | C18 H15 F3 N4 05 | 424,34 | 54,2 |
| 245 | 4-(1,1,2,2-TETRAFLUORO-ETHOXY)ANILINE | 100,4 | 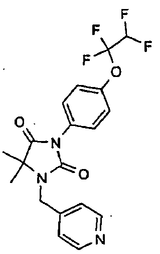 | 5,5-Diméthyl-1-pyfldin4 ylméthyl-3-[4-(1,1,2,2-tetrafluoro-éthoxy)-phenyl]-imidazolidine-2,4-dione 2 | C19H17 F4 N3 03 | 411,36 | 32,1 |
| 246 | 4-(DIFLUOROMETHOXY) ANILINE | 76,4 | 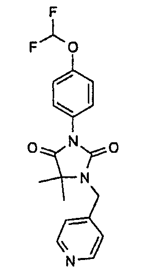 | 3-(4-Difluorométhoxy-phenyl)-5,5-diméthyl-1-pyridin-4-ylméthyl-imidazofidine-2,4-dione 2 | C18 H17 F2 N3 03 | 361,35 | 3,9 |
| 247 | 3-CHLORO-4-(TRIFLUORO-METHOXY)ANILINE | 101,6 | 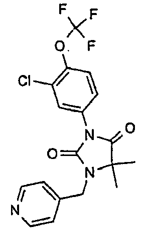 | 3-(3-Chloro-4-trifluorométhoxy-phenyl)-5,5-diméthyl-1-pyridin-4-ylméthyl-imidazolidine-2,4-dione 2 | C18 H15 ClF3N3 03 | 413,79 | 46,2 |
| 248 | 3-BROMO-4-(TRIFLUORO-METHOXY)ANILINE | 122,9 | 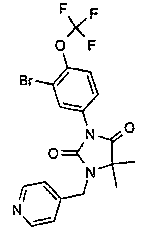 | 3-(3-Bromo-4-trifluorométhoxy-phenyl)-5,5-diméthyl-1-pyridin-4-ylméthyl-imidazolidine-2,4-dione 2 | C18 H15 BrF3N3 03 | 458,24 | 43,2 |
| 249 | 2,6-DICHLORO-4-(TRIFLUORO-METHOXY)ANILINE | 118,1 | 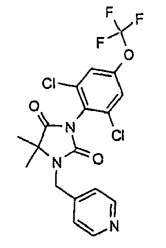 | 3-(2,6-Dichloro-4-trifluorométhoxy-phenyl)-5,5-diméthyl-1-pyridin-4-ylméthyl-imidazolidine-2,4-dione 2 | C18H14 Cl2F3 N3 03 | 448,23 | 19,9 |
| 250 | 3-CHLORO-4-(TRI FLUORO-METHYLTHIO)ANILINE | 109,3 | 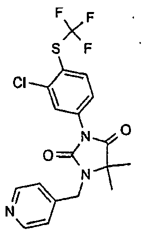 | 3-(3-Chloro-4-trifluorométhylsulfanyl-phenyl)-5,5-diméthyl-1-pyridin-4-ylméthyl-imidazotidine-2,4-dione 2 | C18H15 Cl F3 N3 O2 S | 429,85 | 33 |
| 251 | 2,2-DIFLUORO-5-AMINOBENZODIOXOLE | 83,1 | 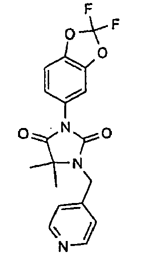 | 3-(2,2-Difluoro-1,3-benzodioxol-5-yl)-5,5-diméthyl-1-pyridin-4-ylméthyl-imidazolidine-2,4-dione 2 | C18H15 F2 N3 04 | 375,33 | 7,4 |
| 252 | 2-BROMO-4-(TRIFLUORO-METHOXY)ANILINE | 122,9 | 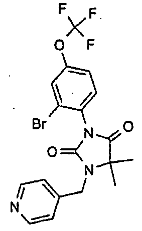 | 3-(2-Bromo-4-fifluorométhoxy-phenyl)-5,5-diméthyl-1-pyrldin4 ylméthyl-imidazolidine-2,4-dione 2 | C18 H15 Br F3 N3 03 | 458,24 | 21,9 |
| 253 | 4-(HEPTAFLUORO-PROPYLTHIO)ANILINE | 140,8 | 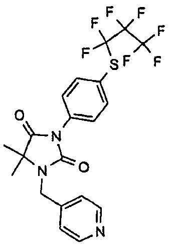 | 3-(4-Heptafluoropropylsulfanyl-phenyl)-5,5-diméthyl-1-pyridin-4-ylméthyl-imidazolidine-2,4-dione 2 | C20H16 F7 N3 O2 S | 495,42 | 38,5 |
| 254 | 2-METHYL-4-(TRIFLUORO-METHOXY)ANILINE | 91,8 | 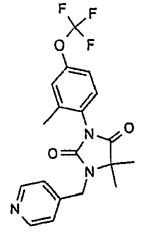 | 5,5-Diméthyl-3-(2-méthyl-4-trifluorométhoxy-phenyl)-1-pyridin-4-ylméthyl-imidazolidine-2,4-dione 2 | C19H18 F3 N3 03 | 393,37 | 68,8 |
| 255 | 2-CHLORO-4-(TRIFLUORO-METHOXY)ANILINE | 101,6 | 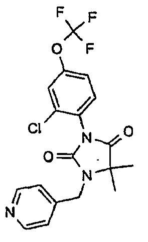 | 3-(2-Chloro-4-trifluorométhoxy-phenyl)-5,5-diméthyl-1-pyridin-4-ylméthyl-imidazolidine-2,4-dione 2 | C18 H15 Cl F3 N3 03 | 413,79 | 77,6 |

**Tableau récapitulatif des caractéristiques physico-chimiques des produits obtenus**

| **Exemple N°** | **Nom du produit obtenu** | **Formule Brut du produit obtenu** | **Poids Moléculaire du produit obtenu** | **Description du spectre de masse (Impact électronique)** | **Description du spectre de masse (LC/MS analytique) temps de rétention, m/z** | **Description du spectre de RMN (Spectre de R.M.N. 1H (300 MHz, (CD3)2SO d6, δ en ppm)** |
|---|---|---|---|---|---|---|
| 244 | 5,5-Diméthyl-3-(2-nitro-4-trifluorométhoxy-phenyl)-1-pyridin-4-ylméthyl-imidazolidine-2,4-dione 2 | C18 H15 F3 N4 O5 | 424,34 | m/z=424 M+. m/z=409 (M -CH3)+ m/z=407 (M -OH)+ m/z=378 (M -NO2)+. m/z=147 C9H11N2+ m/z=33 C7H5N2O+ m/z=92 C6H6N+ pic de base | | 1,44 (s large : 6H) ; 4,69 (s large : 2H); 7,41 (d large, J = 5,5 Hz : 2H) ;7,98 (d, J = 8,5 Hz : 1H) ; 8,07 (d large, J = 8,5 Hz : 1H) ; 8,29 (d, J =2,5 Hz: 1H) ; 8,56 (d large, J = 5,5 Hz : 2H) |
| 245 | 5,5-Diméthyl-1-pyridin-4-ylméthyl-3-[4-(1,1,2,2-tetrafluoro-éthoxy)-phenyl]-imidazolidine-2,4-dione 2 | C19 H17 F4 N3 03 | 411,36 | m/z=411 M+. pic de base m/z=396 (M -CH3)+ m/z=294 (m/z=396 -C2H2F4)+ m/z=235 C9H5N02F4+. m/z=147 C9H11N2+ m/z=133 C7H5N2O+ m/z=92 C6H6N+ | | 1,43 (s : 6H) ; 4,65 (s : 2H) ; 6,85 (tt, J = 51 et 3Hz:1H); 7,46 (mt : 4H) ; 7,62 (dt, J = 8,5 et 2,5 Hz: 2H) ; 8,56 (dd, J = 6 et 1,5 Hz : 2H) |
| 246 | 3-(4-Difluorométhoxy-phenyl)-5,5-diméthyl-1-pyridin-4-ylméthyl-imidazolidine-2,4-dione 2 | C18 H17 F2 N3 03 | 361,35 | | t = 2,64min m/z = 362 (M+1) | |
| 247 | 3-(3-Chloro-4-trifluorométhoxy-phenyl)-5,5-diméthyl-1-pyridin-4-ylméthyl-imidazolidine-2,4-dione 2 | C18 H15 Cl F3 N3 03 | 413, 79 | m/z=413 M+. m/z=398 (M -CH3)+ m/z=237 C8H3NO2ClF3+. m/z=147 C9H11N2+ m/z=133 C7H5N20+ m/z=92 C6H6N+ pic de base | | 1,43(s : 6H) ; 4,66(s : 2H) ; 7,46 (d, J = 6 Hz : 2H) ; 7,66 (dd, J = 8,5 et 2,5 Hz: 1H) ; 7,77 (dd large, J = 8,5 et 1 Hz : 1H) ; 7,93 (d, J = 2,5 Hz : 1H) ; 8,55(d large, J = 6 Hz : 2H) |
| 248 | 3-(3-Bromo-4-trifluorométhoxy-phenyl)-5,5-diméthyl-1-pyridin-4-ylméthyl-imidazolidine-2,4-dione 2 | C18 H15 Br F3 N3 03 | 458,24 | m/z=457 M+. m/z=442 (M -CH3)+ m/z=281 C8H3NO2BrF3+. m/z=147 C9H11N2+ m/z=133 C7H5N2O+ m/z=92 C6H6N+ pic de base | | 1,43 (s : 6H) ; 4,65 (s : 2H) ; 7,46 (d large, J = 6 Hz : 2H) ; 7,68 (dd, J = 8,5 et 2,5 Hz:1H); 7,74 (d large, J = 8,5 Hz: 1H) ; 8,05 (d, J = 2,5 Hz : 1H) ; 8,55 (dd, J = 6 et 1,5Hz:2H) |
| 249 | 3-(2,6-Dichloro-4-trifluorométhoxy-phenyl)-5,5-diméthyl-1-pyridin-4-ylméthyl-imidazolidine-2,4-dione 2 | C18 H14 Cl2 F3 N3 03 | 448,23 | m/z=447 M+. m/z=432 (M -CH3)+ m/z=412(M -Cl)+ m/z=271 C8H2NO2Cl2F3+. m/z=147 C9H11N2+ m/z=133 C7H5N2O+ m/z=92 C6H6N+ pic de base | | 1,47 (s : 6H) ; 4,73 (s : 2H) ; 7,47 (d large, J = 6 Hz : 2H) ; 8,00 (s large : 2H) ; 8,58 (dd, J =6et1,5 Hz : 2H) |
| 250 | 3-(3-Chloro-4-trifluorométhylsulfanyl-phenyl)-5,5-diméthyl-1-pyridin-4-ylméthyl-imidazolidine-2,4-dione 2 | C18 H15 Cl F3 N3 02 S | 429,85 | m/z=429 M+. pic de base m/z=414 (M -CH3)+ m/z=253 C8H3NOSClF3+. m/z=147 C9H11N2+ m/z=133 C7H5N2O+ m/z=92 C6H6N+ | | 1,43 (s : 6H) ; 4,66 (s : 2H) ; 7,46 (d large, J = 6 Hz : 2H) ; 7,70 (dd, J = 8,5 et 2 Hz:1H); 7,97 (d, J = 2 Hz: 1H); 8,05 (d, J = 8,5 Hz: 1H) ; 8,54 (dd, J = 6 et 1,5 Hz : 2H) |
| 251 | 3-(2,2-Difluoro-1,3-benzodioxol-5-yl)-5,5-diméthyl-1-pyridin-4-ylméthyl-imidazolidine-2,4-dione 2 | C18 H15 F2 N3 04 | 375,33 | | t = 2,80 min m/z = 376 (M+1) | |
| 252 | 3-(2-Bromo-4-trifluorométhoxy-phenyl)-5,5-diméthyl-1-pyridin-4-ylméthyl-imidazolidine-2,4-dione 2 | C18 H15 Br F3 N3 O3 | 458,24 | m/z=457 M+. m/z=414 (M -CH3)+ m/z=378 (M -Br)+ pic de base m/z=350 (m/z=378 - CO)+ m/z=281 C8H3NO2BrF3+. m/z=244 (m/z=378 -C7H6N2O)+ m/z=147 C9H11N2+ m/z=133 C7H5N2O+ m/z=92 C6H6N+ | | 1,43 (s : 3H) ; 1,48 (s : 3H) ; 4,68 (AB limite, J =17Hz: 2H) ; 7,42 (d large, J = 6 Hz : 2H) ; 7,66 (dd large, J = 8,5 et 2 Hz : 1H) ; 7,83 (d, J = 8,5 Hz : 1H) ; 8,07 (d large, J = 2 Hz : 1H) ; 8,56 (dd. J =6et1,5 Hz:2H) |
| 253 | 3-(4-Heptafluoropropylsulfanyl-phenyl)-5,5-diméthyl-1-pyridin-4-ylméthyl-imidazolidine-2,4-dione 2 | C20 H16 F7 N3 02 S | 495,42 | m/z=495 M+. pic de base m/z=480 (M -CH3)+ m/z=39 C10H4NOSF7+. m/z=150 (m/z=319 -C3F7)+ m/z=147 C9H11N2+ m/z=133 C7H5N2O+ m/z=92 C6H6N+ | | 1,43 (s : 6H) ; 4,66 (s : 2H) ; 7,46 (d large, J = 6 Hz : 2H) ; 7,71 (d large, J = 8,5 Hz : 2H) ; 7,89 (d large, J = 8,5 Hz : 2H) ; 8,55 (dd, J = 6 et 1,5 Hz : 2H) |
| 254 | 5,5-Diméthyl-3-(2-méthyl-4-trifluorométhoxy-phenyl)-1-pyridin-4-ylméthyl-imidazolidine-2,4-dione 2 | C19 H18 F3 N3 03 | 393,37 | m/z=393 M+. pic de base m/z=378 (M -CH3)+ m/z=217 C9H6NO2F3+. m/z=147 C9H11N2+ m/z=133 C7H5N2O+ m/z=92 C6H6N+ | | 1,43 et 1,44 (2 s : 6H en totalité) ; 2,21 (s : 3H) ; 4,65 (s large : 2H) ; 7,36 (d large, J = 8,5 Hz : 1H) ; 7,42 (d large, J = 6 Hz : 2H) ; 7,45 (s large : 1H) ; 7,55 (d, J = 8,5 Hz : 1H) ; 8,55 (dd, J = 6 et 1,5 Hz:2H) |
| 255 | 3-(2-Chloro-4-trifluorométhoxy-phenyl)-5,5-diméthyl-1-pyridin-4-ytméthyl-imidazotidine-2,4-dione 2 | C18 H15 Cl F3 N3 03 | 413,79 | m/z=413 M+. m/z=398 (M -CH3)+ m/z=378 (M -Cl)+ pic de base m/z=350 (m/z=378 - CO)+ m/z=237 C8H3NO2ClF3+. m/z=147 C9H11N2+ m/z=133 C7H5N20+ m/z=92 C6H6N+ | | 1,43 (s: 3H) ; 1,48 (s : 3H) ; 4,67 (s large : 2H) ; 7,42 (d large, J = 6 Hz : 2H) ; 7,62 (ddd, J =8,5-2,5 et 1Hz : 1H) ; 7,85 (d, J = 8,5 Hz : 1H) ; 7,88 (d large, J = 2,5 Hz: 1H) ; 8,57 (dd, J = 6 et 1,5 Hz : 2H) |

Les produits des exemples 256 à 263 ont été obtenus selon le mode opératoire décrit ci-dessus.

### Exemple 256

### 5-(5-hydroxy-4,4-dimethyl-2-oxo-3-quinolin-4-ylmethyl-imidazolidin-1-yl)-2-trifluoromethoxy-benzoic acid methyl ester

RMN 1H (DMSO)
- (P-31800-112-1): 1.47 ppm (s, 6H) ; 3.89 ppm (s, 3H) ; 5.15 ppm (s, 2H) ; de 7.65 à 7.73 ppm (m, 3H) ; 7.82 ppm tl, J= 8.5 Hz, 1H) ; 7.92 ppm (dd, J= 2.5-8.5 Hz, 1H) ; 8.07 ppm (dl, J= 8.5 Hz, 1H) ; 8.17 ppm (d, J= 2.5 Hz, 1H) ; 8.26 ppm (dl, J= 8.5 Hz, 1H) ; 8.86 ppm (d, J= 4.5 Hz, 1H)
LC/MS: MH⁺ = 488

### Exemple 257

### 3-(3-bromo-4-trifluoromethoxy-phenyl)-5,5-dimethyl-1-quinolin-4-ylmethyl-imidazolidine-2,4-dione

RMN 1H (DMSO)
- (P-31800-101-1): 1.45 ppm (s, 6H) ; 5.15 ppm (s, 2H) ; de 7.62 à 7.72 ppm (m, 4H) ; 7.80 ppm (tl, J= 8.5 Hz, 1H) ; 8.08 ppm (m, 2H) ; 8.26 ppm (dl, J= 8.5 Hz, 1H) ; 8.86 ppm (d, J= 4.5 Hz).
LC/SM: MH+ = 508

### Exemple 258 :

### 3-(3-chloro-4-trifluoromethoxy-phenyl)-5,5-dimethyl-1-quinolin-4-ylmethyl-imidazoline-2,4-dione

RMN 1H (DMSO):
- P-31800-105-1 RMN n° 6354R : 1.44 ppm (s, 6H) ; 5.14 ppm (s, 2H) ; de 7.61 à 7.84 ppm (m, 5H) ; 7.94 ppm (d, J= 2.5 Hz, 1H) ; 8.07 ppm (dl, J= 8.5 Hz, 1H) ; 8.24 ppm (dl, J= 8.5 Hz, 1H) ; 8.83 ppm (d, J= 4.5 Hz, 1H) .
LC/MS: MH+ = 464

### Exemple 259

### 5-(4,4-dimethyl-2,5-dioxo-3-quinolin-4-ylmethyl-imidazolidin-1-yl)-2-trifluoromethyl-benzoic acid

RMN 1H (DMSO)
- (P-29798-111-1): 1.47 ppm (s, 6H) ; 5.15 ppm (s, 2H) ; de 7.62 à 7.72 ppm (m, 3H) ; 7.81 ppm (tl, J= 8.5 Hz, 1H) ; 7.88 ppm (dd, J= 2.5-8.5, 1H) ; 8.08 ppm (tl, J= 8.5 Hz, 1H) ; 8.15 ppm (d, J= 2.5 Hz, 1H) ; 8.26 ppm (dl, J= 8.5 Hz, 1H) ; 8.87 ppm (d, J= 4.5 Hz, 1H) ; 13.5 ppm (sl, 1H) .
LC/MS: MH⁺ = 474

### Exemple 260

### 5,5-dimethyl-3-{3-[4-(4-methyl-piperazin-1-yl)-butyryl]-4-trifluoromethoxy-phenyl}-1-quinolin-4-ylmethyl-imidazolidine-2,4-dione

RMN 1H(DMSO)
- (P-29798-112-1): 1.51 ppm (s, 6H) ; 1.97 ppm (m, 2H) ; 2.82 ppm ( s, 3H) ; de 3.0 à 4.0 ppm (massif étalé, 12 H) ; 5.35 ppm (s, 2H) ; 7.63 ppm (qd, J= 1.5-8.5 Hz, 1H) ; 7.75 ppm (dd, J= 2.5-8.5 Hz, 1H) ; 7.82 ppm (d, J= 2.5 Hz, 1H) ; 7.94 ppm (tl, J= 8.5 Hz, 1H) ; 8.10 ppm (m, 2H) ; 8.39 ppm (dl, J= 8.5 Hz, 1H) ; 8.51 ppm (dl, J= 8.5 Hz, 1H) ; 8.72 ppm (tl, J= 5.5 Hz, 1H) ; 9.15 ppm (d, J= 5Hz, 1H) ; 11.97 ppm (sl, 1H).
MS/EI : M+ = 612+

### Exemple 261

### 5-(5-hydroxy-4,4-dimethyl-2-oxo-3-quinolin-4-ylmethyl-imidazolidin-1-yl)-2-trifluoromethoxy-benzoic acid

Dans un ballon de 30 ml muni d'une agitation magnétique, d'un septum surmonté d'une arrivée d'azote, on introduit :
- 200 mg (0.41 mmol) de d'ester dans 2 ml d'éther
- 8 mg (0.205 mmol) de LiALH4 soit 8 ml de solution 1 M dans 2 ml de THF.
Le milieu réactionnel est agité à température ambiante pendant 24 heures.
En c.c.m ;CH2Cl2 / AcOEt 8/2 : un produit plus polaire que le produit de départ, se forme mais il reste du produit de départ.
On additionne à nouveau 0.5 équivalent de Li AlH4 et on agite pendant 5 heures. Il reste encore un peu de produit de départ, mais on traite.
On hydrolyse, en versant de l'eau sur le milieu réactionnel et on extrait avec 3 fois 10 ml d'acétate d'éthyle. Les extraits organiques sont regroupés, séchés sur sulfate de magnésium et on évapore l'AcOEt.
Le brut réactionnel est purifié en utilisant le mélange éluant CH2Cl2/AcOEt (9/1).
On obtient ainsi 60 mg d'alcool.

RMN 1H (DMSO) : 1.19 ppm (s, 3H) ; 1.24 ppm (s, 3H) ; 3.86 ppm (s, 3H) ; 4.92 ppm (système AB, J=18 Hz, 2H) ; 5.36 ppm (s, 1H) ; 7.51 ppm (dm, J= 9.0 Hz, 1H) ; 7.58 ppm (d, J= 4.5 Hz, 1H) ; 7.66 (m, 1H) ; 7.80 ppm (m, 1H) ; 8.00 ppm (dd, J= 2.5-9.0 Hz, 1H) ; 8.06 ppm (dd, J=1.5-8.5 Hz, 1H) ; 8.28 ppm (dd, J=1.5-8.5 Hz, 1H) ; 8.33 ppm (d, J=2.5 Hz, 1H) ; 8.84 ppm (df, J=4.5 Hz, 1H). LC/MS : MH+ = 489

### Exemple 262

### 4,4-dimethyl-3-quinolin-4-ylmethyl-5-thioxo-1-(4-trifluoromethoxy-phenyl)-imidazolidin-2-one

On porte à reflux pendant 9 heures le mélange réactionnel suivant :
- 317 mg (0.739 10⁻³ mole) d'hydantoïne SB 31051-139
- 78 mg (0.19 ⁻³mole ) de réactif de Lawesson
- 1 cm³de toluène
puis refroidit, filtre et concentre sous pression réduite. On récupère une mousse jaune que l'on purifie par chromatographie plusieurs fois sur silice, éluant chloroforme/acétone (97/3). On obtient 231 mg de résine jaune pure à Rf 0.38 Th= 329 mg Rdt= 70 %

RMN 1H (DMSO) ppm
- (P-31051-146-2): 1.57 ppm (s, 6H) ; 5.24 ppm (s, 2H) ; 7.57 ppm (m, 2H) ; 7.63 ppm (d, J= 4.5 Hz, 1H) ; 7.70 ppm ( m, 3H) ; 7.81 ppm (tl, J= 8.5 Hz, 1H) ; 8.08 ppm (dd, J= 1.5-8.5 Hz, 1H) ; 8.26 ppm ( dd, J= 1.5-8.5 Hz, 1H) ; 8.86 ppm ( d, J= 4.5 Hz, 1H) .
LC/SM: MH₊ = 446

### Exemple 263

### 5,5-dimethyl-1-quinolin-4-ylmethyl-2-thioxo-3-(4-trifluoromethoxy-phenyl)-imidazolidin-4-one

On agite à température ambiante pendant 20 heures le mélange réactionnel suivant :
- 0.6 g ( mmol)de dérivé quinoléine
- 0.918 g (mmol)de thio-isocyanate
- 12 cm³de THF anhydre
- 0.2 cm³ de TEA

On concentre sous pression réduite puis purifie sur colonne AIT de 25g, éluant DMC/Acétone 95/5.
On récupère le produit de Rf = 0.12 soit 266 mg
Rdt = 26 %

RMN 1H (DMSO)
(P-31051-160-2): 1.55 ppm (s, 6H) ; 5.56 ppm (s, 2H) ; 7.55 ppm (m, 3H) ; 7.69 ppm (m, 3H) ; 7.82 ppm (tl, J= 8.5 Hz, 1H) ; 8.08 ppm (dd, J= 1.5-8.5, 1H) ; 8.28 ppm (dd, J= 1.5-8.5, 1H) ; 8.83 ppm (d, J= 4.5 Hz, 1H).
SM: M⁺ = 445⁺

Les produits des exemples 264 à 332 sont décrits dans le tableau 1 ci-après : ces produits sont préparés comme indiqué dans la partie expérimentale de la présente demande et notamment comme indiqué pour les produits des exemples 70 à 200 et constituent ainsi les exemples 264 à 332 de la présente invention.

### EXEMPLE 333 : COMPOSITION PHARMACEOTIQUE

On a préparé des comprimés répondant à la formule suivante :

| | |
|---|---|
| Produit de l'exemple 9 | 0,2 g |
| Excipient pour un comprimé terminé à | 1 g |

(détail de l'excipient : lactose, talc, amidon, stéarate de magnésium).

### EXEMPLE 334 : COMPOSITION PHARMACEUTIQUE

On a préparé des comprimés répondant à la formule suivante :

| | |
|---|---|
| Produit de l'exemple 52 | 0,2 g |
| Excipient pour un comprimé terminé à | 1 g |

(détail de l'excipient : lactose, talc, amidon, stéarate de magnésium).

Les exemples de compositions pharmaceutiques 333 et 334 ci-dessus illustrent la présente invention, étant entendu que les mêmes préparations peuvent être réalisées avec d'autres produits préférés de la présente invention et font partie de la présente invention.

La présente invention comprend également encore à titre d'exemples les produits de formule (I) suivants: la liste suivante donne les noms de produits de formule (I) telle que définie ci-dessus pour lesquels B2 représente un radical pyridyle ou un radical quinoléinyle : ces produits font partie de la présente invention et peuvent être synthétisés selon les procédés décrits ci-dessus pour l'obtention des produits de formule (I) et notamment suivant les conditions opératoires décrites pour la préparation des exemples 61 à 63 :
- la 5,5-diméthyl-1-(2-méthyl-pyridin-4-ylméthyl)-3-(4-trifluorométhoxy-phényl)-imidazolidine-2,4-dione
- la 5,5-diméthyl-1-(2-éthyl-pyridin-4-ylméthyl)-3-(4-trifluorométhoxy-phényl)-imidazolidine-2,4-dione
- la 5,5-diméthyl-1-(2-isopropyl-pyridin-4-ylméthyl)-3-(4-trifluorométhoxy-phényl)-imidazolidine-2,4-dione
- la 5,5-diméthyl-1-(2-ter-butyl-pyridin-4-ylméthyl)-3-(4-trifluorométhoxy-phényl)-imidazolidine-2,4-dione
- la 5,5-diméthyl-1-(2-isobutyl-pyridin-4-ylméthyl)-3-(4-trifluorométhoxy-phényl)-imidazolidine-2,4-dione
- la 5,5-diméthyl-1-(2-cyclohéxyl-pyridin-4-ylméthyl)-3-(4-trifluorométhoxy-phényl)-imidazolidine-2,4-dione
- la 5,5-diméthyl-1-(2-phényl-pyridin-4-ylméthyl)-3-(4-trifluorométhoxy-phényl)-imidazolidine-2,4-dione
- la 5,5-diméthyl-1-[2-(4-chlorophényl)-pyridin-4-ylméthyl]-3-(4-trifluorométhoxy-phényl)-imidazolidine-2,4-dione
- la 5,5-diméthyl-1-[2-(3-chlorophényl)-pyridin-4-ylméthyl]-3-(4-trifluorométhoxy-phényl)-imidazolidine-2,4-dione
- la 5,5-diméthyl-1-[2-(2-chlorophényl)-pyridin-4-ylméthyl]-3-(4-trifluorométhoxy-phényl)-imidazolidine-2,4-dione
- la 5,5-diméthyl-1-[2-(4-fluorophényl)-pyridin-4-ylméthyl]-3-(4-trifluorométhoxy-phényl)-imidazolidine-2,4-dione
- la 5,5-diméthyl-1-[2-(4-méthoxyphényl)-pyridin-4-ylméthyl]-3-(4-trifluorométhoxy-phényl)-imidazolidine-2,4-dione
- la 5,5-diméthyl-1-(2-benzyl-pyridin-4-ylméthyl)-3-(4-trifluorométhoxy-phényl)-imidazolidine-2,4-dione
- la 5,5-diméthyl-1-(2,2'bipyridin-4-ylméthyl)-3-(4-trifluorométhoxy-phényl)-imidazolidine-2,4-dione
- la 5,5-diméthyl-1-(2-hydroxyméthyl-pyridin-4-ylméthyl)-3-(4-trifluorométhoxy-phényl)-imidazolidine-2,4-dione
- la 5,5-diméthyl-1-(2-chloro-pyridin-4-ylméthyl)-3-(4-trifluorométhoxy-phényl)-imidazolidine-2,4-dione
- la 5,5-diméthyl-1-(2-fluoro-pyridin-4-ylméthyl)-3-(4-trifluorométhoxy-phényl)-imidazolidine-2,4-dione
- la 5,5-diméthyl-1-(2-b-romo-pyridin-4-ylméthyl)-3-(4-trifluorométhoxy-phényl)-imidazolidine-2,4-dione
- la 5,5-diméthyl-1-(2-amino-pyridin-4-ylméthyl)-3-(4-trifluorométhoxy-phényl)-imidazolidine-2,4-dione
- la 5,5-diméthyl-1-(2-méthylamino-pyridin-4-ylméthyl)-3-(4-trifluorométhoxy-phényl)-imidazolidine-2,4-dione
- la 5,5-diméthyl-1-(2-éthylamino-pyridin-4-ylméthyl)-3-(4-trifluorométhoxy-phényl)-imidazolidine-2,4-dione
- la 5,5-diméthyl-1-(2-isopropylamino-pyridin-4-ylméthyl)-3-(4-trifluorométhoxy-phényl)-imidazolidine-2,4-dione
- la 5,5-diméthyl-1-(2-phénylamino-pyridin-4-ylméthyl)-3-(4-trifluorométhoxy-phényl)-imidazolidine-2,4-dione
- la 5,5-diméthyl-1-(2-cyclohéxylamino-pyridin-4-ylméthyl)-3-(4-trifluorométhoxy-phényl)-imidazolidine-2,4-dione
- la 5,5-diméthyl-1-(2-diéthylamino-pyridin-4-ylméthyl)-3-(4-trifluorométhoxy-phényl)-imidazolidine-2,4-dione
- la 5,5-diméthyl-1-(2-N-cyclohéxyl-N-méthylamino-pyridin-4-ylméthyl)-3-(4-trifluorométhoxy-phényl)-imidazolidine-2,4-dione
- la 5,5-diméthyl-1-(2-pipéridino-pyridin-4-ylméthyl)-3-(4-trifluorométhoxy-phényl)-imidazolidine-2,4-dione
- la 5,5-diméthyl-1-(2-morpholino-pyridin-4-ylméthyl)-3-(4-trifluorométhoxy-phényl)-imidazolidine-2,4-dione
- la 5,5-diméthyl-1-[2-(4-méthylpipérazino)-pyridin-4-ylméthyl]-3-(4-trifluorométhoxy-phényl)-imidazolidine-2,4-dione
- la 5,5-diméthyl-1-[2-(1H-pyrazol-lyl)-pyridin-4-ylméthyl]-3-(4-trifluorométhoxy-phényl)-imidazolidine-2,4-dione
- la 5,5-diméthyl-1-[2-(2-benzoylhydrazino)-pyridin-4-ylméthyl]-3-(4-trifluorométhoxy-phényl)-imidazolidine-2,4-dione
- la 5,5-diméthyl-1-(2-benzoylamino-pyridin-4-ylméthyl)-3-(4-trifluorométhoxy-phényl)-imidazolidine-2,4-dione
- la 5,5-diméthy-1-1-[2-(4-chlorobenzoylamino)-pyridin-4-ylméthyl]-3-(4-trifluorométhoxy-phényl)-imidazolidine-2,4-dione
- la 5,5-diméthyl-1-[2-(3-chlorobenzoylamino)-pyridin-4-ylméthyl]-3-(4-trifluorométhoxy-phényl)-imidazolidine-2,4-dione
- la 5,5-diméthyl-1-[2-(2-chlorobenzoylamino)-pyridin-4-ylméthyl]-3-(4-trifluorométhoxy-phényl)-imidazolidine-2,4-dione
- la 5,5-diméthyl-1-[2-(4-fluorobenzoylamino)-pyridin-4-ylméthyl]-3-(4-trifluorométhoxy-phényl)-imidazolidine-2,4-dione
- la 5,5-diméthyl-1-[2-(4-méthoxybenzoylamino)-pyridin-4-yl méthyl]-3-(4-trifluorométhoxy-phényl)-imidazolidine-2,4-dione
- la 5,5-diméthyl-1-(2-acétamido-pyridin-4-ylméthyl)-3-(4-trifluorométhoxy-phényl)-imidazolidine-2,4-dione
- la 5,5-diméthyl-1-(2-phénylacétamido-pyridin-4-ylméthyl)-3-(4-trifluorométhoxy-phényl)-imidazolidine-2,4-dione
- la 5,5-diméthyl-1-[2- (4-méthylpipérazino)acétamido-pyridin-4-ylméthyl]-3-(4-trifluorométhoxy-phényl)-imidazolidine-2,4-dione
- la 5,5-diméthyl-1-(2-cyclohéxanylcarboxamido-pyridin-4-ylméthyl)-3-(4-trifluorométhoxy-phényl)-imidazolidine-2,4-dione
- la 5,5-diméthyl-1-[2-(1-méthyl-4-pipéridinyl)carbonylamino-pyridin-4-ylméthyl]-3-(4-trifluorométhoxy-phényl)-imidazolidine-2,4-dione
- la 5,5-diméthyl-1-[2-(2-furanylcarbonyl)amino-pyridin-4-ylméthyl]-3-(4-trifluorométhoxy-phényl)-imidazolidine-2,4-dione
- la 5,5-diméthyl-1-(2-éthoxycarbonylamino-pyridin-4-ylméthyl)-3-(4-trifluorométhoxy-phényl)-imidazolidine-2,4-dione
- la 5,5-diméthyl-1-(2-isopropoxycarbonylamino-pyridin-4-ylméthyl)-3-(4-trifluorométhoxy-phényl)-imidazolidine-2,4-dione
- la 5,5-diméthyl-1-(2-butoxycarbonylamino-pyridin-4-ylméthyl)-3-(4-trifluorométhoxy-phényl)-imidazolidine-2,4-dione
- la 5,5-diméthyl-1-[2-(2-diéthylaminoéthoxycarbonylamino)-pyridin-4-ylméthyl]-3-(4-trifluorométhoxy-phényl)-imidazolidine-2,4-dione
- la 5,5-diméthyl-1-(2-phénoxycarbonylamino-pyridin-4-ylméthyl)-3-(4-trifluorométhoxy-phényl)-imidazolidine-2,4-dione
- la 5,5-diméthyl-1-[2-(4-chloroanilino)carbonylamino-pyridin-4-ylméthyl]-3-(4-trifluorométhoxy-phényl)-imidazolidine-2,4-dione
- la 5,5-diméthyl-1-(2-anilinocarbonylamino-pyridin-4-ylméthyl)-3-(4-trifluorométhoxy-phényl)-imidazolïdine-2,4-dione
- la 5,5-diméthyl-1-(2-benzylaminocarbonylamino-pyridin-4-ylméthyl)-3-(4-trifluorométhoxy-phényl)-imidazolidine-2,4-dione
- la 5,5-diméthyl-1-[2-(2-pyridylamino)carbonylamino-pyridin-4-ylméthyl]-3-(4-trifluorométhoxy-phényl)-imidazolidine-2,4-dione
- la 5,5-diméthyl-1-(2-uréido-pyridin-4-ylméthyl)-3-(4-trifluorométhoxy-phényl)-imidazolidine-2,4-dione
- la 5,5-diméthyl-1-(2-benzylaminosulfonylamino-pyridin-4-ylméthyl)-3-(4-trifluorométhoxy-phényl)-imidazolidine-2,4-dione
- la 5,5-diméthyl-1-(2-méthylaminosulfonylamino-pyridin-4-ylméthyl)-3-(4-trifluorométhoxy-phényl)-imidazolidine-2,4-dione
- la 5,5-diméthyl-1-(2-aminosulfonylamino-pyridin-4-ylméthyl)-3-(4-trifluorométhoxy-phényl)-imidazolidine-2,4-dione
- la 5,5-diméthyl-1-(2-morpholinosulfonylamino-pyridin-4-ylméthyl)-3-(4-trifluorométhoxy-phényl)-imidazolidine-2,4-dione
- la 5,5-diméthyl-1-(2-diméthylaminosulfonylamino-pyridin-4-ylméthyl)-3-(4-trifluorométhoxy-phényl)-imidazolidine-2,4-dione
- la 5,5-diméthyl-1-(2-anilinosulfonylamino-pyridin-4-ylméthyl)-3-(4-trifluorométhoxy-phényl)-imidazolidine-2,4-dione
- la 5,5-diméthyl-1-(2-phénylsulfonylamino-pyridin-4-ylméthyl)-3-(4-trifluorométhoxy-phényl)-imidazolidine-2,4-dione
- la 5,5-diméthyl-1-[2-(2-thiényl)sulfonylamino-pyridin-4-ylméthyl]-3-(4-trifluorométhoxy-phényl)-imidazolidine-dione
- la 5,5-diméthyl-1-(2-méthylsulfonylamino-pyridin-4-ylméthyl)-3-(4-trifluorométhoxy-phényl)-imidazolidine-2,4-dione
- la 5,5-diméthyl-1-[2-(4-chlorophényl)sulfonylamino-pyridin-4-ylméthyl]-3-(4-trifluorométhoxy-phényl)-imidazolidine-2,4-dione
- la 5,5-diméthyl-1-(2-benzylsulfonylamino-pyridin-4-ylméthyl)-3-(4-trifluorométhoxy-phényl)-imidazolidine-2,4-dione
- la 5,5-diméthyl-1-(2-hydroxycarbonyl-pyridin-4-ylméthyl)-3-(4-trifluorométhoxy-phényl)-imidazolidine-2,4-dione
- la 5,5-diméthyl-1-(2-éthoxycarbonyl-pyridin-4-ylméthyl)-3-(4-trifluorométhoxy-phényl)-imidazolidine-2,4-dione
- la 5,5-diméthyl-1-(2-ter-butoxycarbonyl-pyridin-4-ylméthyl)-3-(4-trifluorométhoxy-phényl)-imidazolidine-2,4-dione
- la 5,5-diméthyl-1-(2-phénoxycarbonyl-pyridin-4-ylméthyl)-3-(4-trifluorométhoxy-phényl)-imidazolidine-2,4-dione
- la 5,5-diméthyl-1-[2- (4-méthylpipérazinocarbonyl)-pyridin-4-ylméthy]-3-(4-trifluorométhoxy-phényl)-imidazolidine-2,4-dione
- la 5,5-diméthyl-1-[2-(méthylamino)carbonyl-pyridin-4-ylméthyl]-3-(4-trifluorométhoxy-phényl)-imidazolidine-_{.} 2,4-dione
- la 5,5-diméthyl-1-[2-(diméthylamino)carbonyl-pyridin-4-ylméthyl]-3-(4-trifluorométhoxy-phényl)-imidazolidine-2,4-dione
- la 5,5-diméthyl-1-(2-pipéridinocarbonyl-pyridin-4-ylméthyl)-3-(4-trifluorométhoxy-phényl)-imidazolidine-2,4-dione
- la 5,5-diméthyl-1-(2-morpholinocarbonyl-pyridin-4-ylméthyl)-3-(4-trifluorométhoxy-phényl)-imidazolidine-2,4-dione
- la 5,5-diméthyl-1-[2-(4-phénylpipérazinocarbonyl)-pyridin-4-ylméthyl]-3-(4-trifluorométhoxy-phényl)-imidazolidine-2,4-dione
- la 5,5-diméthyl-1-(2-phénylaminocarbonyl-pyridin-4-ylméthyl)-3-(4-trifluorométhoxy-phényl)-imidazolidine-2,4-dione
- la 5,5-diméthyl-1-[2-(méthylphénylamino)carbonyl-pyridin-4-ylméthyl]-3-(4-trifluorométhoxy-phényl)-imidazolidine-2,4-dione
- la 5,5-diméthyl-1-[2-(4-méthoxyphényl)méthylaminocarbonyl-pyridin-4-ylméthyl]-3-(4-trifluorométhoxy-phényl)-imidazolidine-2,4-dione
- la 5,5-diméthyl-1-[2-(4-chlorophényl)méthylaminocarbonyl-pyridin-4-ylméthyl]-3-(4-trifluorométhoxy-phényl)-imidazolidine-2,4-dione
- la 5,5-diméthyl-1-[2-(4-morpholinylamino)carbonyl-pyridin-4-ylméthyl]-3-(4-trifluorométhoxy-phényl)-imidazolidine-2,4-dione
- la 5,5-diméthyl-1-(2-aminocarbonyl-pyridin-4-ylméthyl)-3-(4-trifluorométhoxy-phényl)-imidazolidine-2,4-dione
- la 5,5-diméthyl-1-(2-phénoxy-pyridin-4-ylméthyl)-3-(4-trifluorométhoxy-phényl)-imidazolidine-2,4-dione
- la 5,5-diméthyl-1-(2-méthoxy-pyridin-4-ylméthyl)-3-(4-trifluorométhoxy-phényl)-imidazolidine-2,4-dione
- la 5,5-diméthyl-1-(2-éthoxy-pyridin-4-ylméthyl)-3-(4-trifluorométhoxy-phényl)-imidazolidine-2,4-dione
- la 5,5-diméthyl-1-[2-(1-pyrrolidinyl)éthoxy-pyridin-4-ylméthyl]-3-(4-trifluorométhoxy-phényl)-imidazolidine-2,4-dione
- la 5,5-diméthyl-1-(2-benzyloxy-pyridin-4-ylméthyl)-3-(4-trifluorométhoxy-phényl)-imidazolidine-2,4-dione
- la 5,5-diméthyl-1-(2-benzoyloxy-pyridin-4-ylméthyl)-3-(4-trifluorométhoxy-phényl)-imidazolidine-2,4-dione
- la 5,5-diméthyl-1-[2-(4,5-dihydro-1H-imidazol-2-yl)-pyridin-4-ylméthyl]-3-(4-trifluorométhoxy-phényl)-imidazolidine-2,4-dione
- la 5,5-diméthyl-1-[2-(1H-imidazol-2-yl)-pyridin-4-ylméthyl]-3-(4-trifluorométhoxy-phényl)-imidazolidine-2,4-dione
- la 5,5-diméthyl-1-[2-(1H-tétrazol-5-yl)-pyridin-4-ylméthyl]-3-(4-trifluorométhoxy-phényl)-imidazolidine-2,4-dione
- la 5,5-diméthyl-1-(2-amidino-pyridin-4-ylméthyl)-3-(4-trifluorométhoxy-phényl)-imidazolidine-2,4-dione
- la 5,5-diméthyl-1-(2-diméthylaminométhyl-pyridin-4-ylméthyl)-3-(4-trifluorométhoxy-phényl)-imidazolidine-2,4-dione
- la 5,5-diméthyl-1-(2-aminoméhyl-pyridin-4-ylméthyl)-3-(4-trifluorométhoxy-phényl)-imidazolidine-2,4-dione
- la 5,5-diméthyl-1-(3-méthyl-pyridin-4-ylméthyl)-3-(4-trifluorométhoxy-phényl)-imidazolidine-2,4-dione
- la 5,5-diméthyl-1-(3-éthyl-pyridin-4-ylméthyl)-3-(4-trifluorométhoxy-phényl)-imidazolidine-2,4-dione
- la 5,5-diméthyl-1-(3-isopropyl-pyridin-4-ylméthyl)-3-(4-trifluorométhoxy-phényl)-imidazolidine-2,4-dione
- la 5,5-diméthyl-1-(3-ter-butyl-pyridin-4-ylméthyl)-3-(4-trifluorométhoxy-phényl)-imidazolidine-2,4-dione
- la 5,5-diméthyl-1-(3-isobutyl-pyridin-4-ylméthyl)-3-(4-trifluorométhoxy-phényl)-imidazolidine-2,4-dione
- la 5,5-diméthyl-1-(3-cyclohéxyl-pyridin-4-ylméthyl)-3-(4-trifluorométhoxy-phényl)-imidazolidine-2,4-dione
- la 5,5-diméthyl-1-(3-phényl-pyridin-4-ylméthyl)-3-(4-trifluorométhoxy-phényl)-imidazolidine-2,4-dione
- la 5,5-diméthyl-1-[3-(4-chlorophényl)-pyridin-4-ylméthyl]-3-(4-trifluorométhoxy-phényl)-imidazolidine-2,4-dione
- la 5,5-diméthyl-1-[3-(3-chlorophényl)-pyridin-4-ylméthyl]-3-(4-trifluorométhoxy-phényl)-imidazolidine-2,4-dione
- la 5,5-diméthyl-1-[3-(2-chlorophényl)-pyridin-4-ylméthyl]-3-(4-trifluorométhoxy-phényl)-imidazolidine-2,4-dione
- la 5,5-diméthyl-1-[3-(4-fluorophényl)-pyridin-4-ylméthyl]-3-(4-trifluorométhoxy-phényl)-imidazolidine-2,4-dione
- la 5,5-diméthyl-1-[3-(4-méthoxyphényl)-pyridin-4-ylméthyl]-3-(4-trifluorométhoxy-phényl)-imidazolidine-2,4-dione
- la 5,5-diméthyl-1-(3-benzyl-pyridin-4-ylméthyl)-3-(4-trifluorométhoxy-phényl)-imidazolidine-2,4-dione
- la 5,5-diméthyl-1-(3,2'bipyridin-4-ylméthyl)-3-(4-trifluorométhoxy-phényl)-imidazolidine-2,4-dione
- la 5,5-diméthyl-1-(3-hydroxyméthyl-pyridin-4-ylméthyl)-3-(4-trifluorométhoxy-phényl)-imidazolidine-2,4-dione
- la 5,5-diméthyl-1-(3-chloro-pyridin-4-ylméthyl)-3-(4-trifluorométhoxy-phényl)-imidazolidine-2,4-dione
- la 5,5-diméthyl-1-(3-fluoro-pyridin-4-ylméthyl)-3-(4-trifluorométhoxy-phényl)-imidazolidine-2,4-dione
- la 5,5-diméthyl-1-(3-bromo-pyridin-4-ylméthyl)-3-(4-trifluorométhoxy-phényl)-imidazolidine-2,4-dione
- la 5,5-diméthyl-1-(3-amino-pyridin-4-ylméthyl)-3-(4-trifluorométhoxy-phényl)-imidazolidine-2,4-dione
- la 5,5-diméthyl-1-(3-méthylamino-pyridin-4-ylméthyl)-3-(4-trifluorométhoxy-phényl)-imidazoliaine-2,4-dione
- la 5,5-diméthyl-1-(3-éthylamino-pyridin-4-ylméthyl)-3-(4-trifluorométhoxy-phényl)-imidazolidine-2,4-dione
- la 5,5-diméthyl-1-(3-isopropylamino-pyridin-4-ylméthyl)-3-(4-trifluorométhoxy-phényl)-imidazolidine-2,4-dione
- la 5,5-diméthyl-1-(3-phénylamino-pyridin-4-ylméthyl)-3-(4-trifluorométhoxy-phényl)-imidazolidine-2,4-dione
- la 5,5-diméthyl-1-(3-cyclohéxylamino-pyridin-4-ylméthyl)-3-(4-trifluorométhoxy-phényl)-imidazolidine-2,4-dione
- la 5,5-diméthyl-1-(3-diéthylamino-pyridin-4-ylméthyl)-3-(4-trifluorométhoxy-phényl)-imidazolidine-2,4-dione
- la 5,5-diméthyl-1-(3-N-cyclohéxyl-N-méthylamino-pyridin-4-ylméthyl)-3-(4-trifluorométhoxy-phényl)-imidazolidine-2,4-dione
- la 5,5-diméthyl-1-(3-pipéridino-pyridin-4-ylméthyl)-3-(4-trifluorométhoxy-phényl)-imidazolidine-2,4-dione
- la 5,5-diméthyl-1-(3-morpholino-pyridin-4-ylméthyl)-3-(4-trifluorométhoxy-phényl)-imidazolidine-2,4-dione
- la 5,5-diméthyl-1-[3-(4-méthylpipérazino)-pyridin-4-ylméthyl]-3-(4-trifluorométhoxy-phényl)-imidazolidine-2,4-dione
- la 5,5-diméthyl-1-[3-(1H-pyrazol-1yl)-pyridin-4-ylméthyl]-3-(4-trifluorométhoxy-phényl)-imidazolidine-2,4-diode
- la 5,5-diméthyl-1-[3-(2-benzoylhydrazino)-pyridin-4-ylméthyl]-3-(4-trifluorométhoxy-phényl)-imidazolidine-2,4-dione
- la 5,5-diméthyl-1-(3-benzoylamino-pyridin-4-ylméthyl)-3-(4-trifluorométhoxy-phényl)-imidazolidine-2,4-dione
- la 5,5-diméthyl-1-[3-(4-chlorobenzoylamino)-pyridin-4-ylméthyl]-3-(4-trifluorométhoxy-phényl)-imidazolidine-2,4-dione
- la 5,5-diméthyl-1-[3-(3-chlorobenzoylamino)-pyridin-4-ylméthyl]-3-(4-trifluorométhoxy-phényl)-imidazolidine-2,4-dione
- la 5,5-diméthyl-1-[3-(2-chlorobenzoylamino)-pyridin-4-ylméthyl]-3-(4-trifluorométhoxy-phényl)-imidazolidine-2,4-dione
- la 5,5-diméthyl-1-[3-(4-fluorobenzoylamino)-pyridin-4-ylméthyl]-3-(4-trifluorométhoxy-phényl)-imidazolidine-2,4-dione
- la 5,5-diméthyl-1-[3-(4-méthoxybenzoylamino)-pyridin-4-ylméthyl]-3-(4-trifluorométhoxy-phényl)-imidazolidine-2,4-dione
- la 5,5-diméthyl-1-(3-acétamido-pyridin-4-ylméthyl)-3-(4-trifluorométhoxy-phényl)-imidazolidine-2,4-dione
- la 5,5-diméthyl-1-(3-phénylacétamido-pyridin-4-ylméthyl)-3-(4-trifluorométhoxy-phényl)-imidazolidine-2,4-dione
- la 5,5-diméthyl-1-[3-(4-méthylpipérazino)acétamido-pyridin-4-ylméthyl]-3-(4-trifluorométhoxy-phényl)-imidazolidine-2,4-dione
- la 5,5-diméthyl-1-(3-cyclohéxanylcarboxamido-pyridin-4-ylméthyl)-3-(4-trifluorométhoxy-phényl)-imidazolidine-2,4-dione
- la 5,5-diméthyl-1-[3-(1-méthyl-4-pipéridinyl)carbonylamino-pyridin-4-ylméthyl]-3-(4-trifluorométhoxy-phényl)-imidazolidine-2,4-dione
- la 5,5-diméthyl-1-[3-(2-furanylcarbonyl)amino-pyridin-4-ylméthyl]-3-(4-trifluorométhoxy-phényl)-imidazolidine-2,4-dione
- la 5,5-diméthyl-1-(3-éthoxycarbonylamino-pyridin-4-ylméthyl)-3-(4-trifluorométhoxy-phényl)-imidazolidine-2,4-dione
- la 5,5-diméthyl-1-(3-isopropoxycarbonylamino-pyridin-4-ylméthyl)-3-(4-trifluorométhoxy-phényl)-imidazolidine-2,4-dione
- la 5,5-diméthyl-1-(3-butoxycarbonylamino-pyridin-4-ylméthyl)-3-(4-trifluorométhoxy-phényl)-imidazolidine-2,4-dione
- la 5,5-diméthyl-1-[3-(2-diéthylaminoéthoxycarbonylamino)-pyridin-4-ylméthyl]-3-(4-trifluorométhoxy-phényl)-imidazolidine-2,4-dione
- la 5,5-diméthyl-1-(3-phénoxycarbonylamino-pyridin-4-ylméthyl)-3-(4-trifluorométhoxy-phényl)-imidazolidine-2,4-dione
- la 5,5-diméthyl-1-[3-(4-chloroanilino)carbonylamino-pyridin-4-ylméthyl]-3-(4-trifluorométhoxy-phényl)-imidazolidine-2,4-dione
- la 5,5-diméthyl-1-(3-anilinocarbonylamino-pyridin-4-ylméthyl)-3-(4-trifluorométhoxy-phényl)-imidazolidine-2,4-dione
- la 5,5-diméthyl-1-(3-benzylaminocarbonylamino-pyridin-4-ylméthyl)-3-(4-trifluorométhoxy-phényl)-imidazolidine-2,4-dione
- la 5,5-diméthyl-1-[3-(2-pyridylamino)carbonylamino-pyridin-4-ylméthyl]-3-(4-trifluorométhoxy-phényl)-imidazolidine-2,4-dione
- la 5,5-diméthyl-1-(3-uréido-pyridin-4-ylméthyl)-3-(4-trifluorométhoxy-phényl)-imidazolidine-2,4-dione
- la 5,5-diméthyl-1-(3-benzylaminosulfonylamino-pyridin-4-ylméthyl)-3-(4-trifluorométhoxy-phényl)-imidazolidine-2,4-dione
- la 5,5-diméthyl-1-(3-méthylaminosulfonylamino-pyridin-4-ylméthyl)-3-(4-trifluorométhoxy-phényl)-imidazolidine-2,4-diode
- la 5,5-diméthyl-1-(3-aminosulfonylamino-pyridin-4-ylméthyl)-3-(4-trifluorométhoxy-phényl)-imidazolidine-2,4-dione
- la 5,5-diméthyl-1-(3-morpholinosulfonylamino-pyridin-4-ylméthyl)-3-(4-trifluorométhoxy-phényl)-imidazolidine-2,4-dione
- la 5,5-diméthyl-1-(3-diméthylaminosulfonylamino-pyridin-4-ylméthyl)-3-(4-trifluorométhoxy-phényl)-imidazolidine-2,4-dione
- la 5,5-diméthyl-1-(3-anilinosulfonylamino-pyridin-4-ylméthyl)-3-(4-trifluorométhoxy-phényl)-imidazolidine-2,4-dione
- la 5,5-diméthyl-1-(3-phénylsulfonylamino-pyridin-4-ylméthyl)-3-(4-trifluorométhoxy-phényl)-imidazolidine-2,4-dione
- la 5,5-dimêthyl-1-[3-(2-thiényl)sulfonylamino-pyridin-4-ylméthyl]-3-(4-trifluorométhoxy-phényl)-imidazolidine-2,4-dione
- la 5,5-diméthyl-1-(3-méthylsulfonylamino-pyridin-4-ylméthyl)-3-(4-trifluorométhoxy-phényl)-imidazolidine-2,4-dione
- la 5,5-diméthyl-1-[3-(4-chlorophényl)sulfonylamino-pyridin-4-ylméthyl]-3-(4-trifluorométhoxy-phényl)-imidazolidine-2,4-dione
- la 5,5-diméthyl-1-(3-benzylsulfonylamino-pyridin-4-ylméthyl)-3-(4-trifluorométhoxy-phényl)-imidazolidine-2,4-dione
- la 5,5-diméthyl-1-(3-hydroxycarbonyl-pyridin-4-ylméthyl)-3-(4-trifluorométhoxy-phényl)-imidazolidine-2,4-dione
- la 5,5-diméthyl-1-(3-éthoxycarbonyl-pyridin-4-ylméthyl)-3-(4-trifluorométhoxy-phényl)-imidazolidine-2,4-dione
- la 5,5-diméthyl-1-(3-ter-butoxycarbonyl-pyridin-4-ylméthyl)-3-(4-trifluorométhoxy-phényl)-imidazolidine-2,4-dione
- la 5,5-diméthyl-1-(3-phénoxycarbonyl-pyridin-4-ylméthyl)-3-(4-trifluorométhoxy-phényl)-imidazolidine-2,4-dione
- la 5,5-diméthyl-1-[3-(4-méthylpipérazinocarbonyl)-pyridin-4-ylméthyl]-3-(4-trifluorométhoxy-phényl)-imidazolidine-2,4-dione
- la 5,5-diméthyl-1-[3-(méthylamino)carbonyl-pyridin-4-ylméthyl]-3-(4-trifluorométhoxy-phényl)-imidazolidine-2,4-dione
- la 5,5-diméthyl-1-[3-(diméthylamino)carbonyl-pyridin-4-ylméthyl]-3-(4-trifluorométhoxy-phényl)-imidazolidine-2,4-dione
- la 5,5-diméthyl-1-(3-pipéridinocarbonyl-pyridin-4-ylméthyl)-3-(4-trifluoromthony-phényl)-imidazolidine-2,4-dione
- la 5,5-diméthyl-1-(3-morpholinocarbonyl-pyridin-4-ylméthyl)-3-(4-trifluorométhoxy-phényl)-imidazolidine-2,4-dione
- la 5,5-diméthyl-1-[3-(4-phénylpiprazinocarbonyl)-pyridin-4-ylméthyl]-3-(4-trifluorométhoxy-phényl)-imidazolidine-2,4-dione
- la 5,5-diméthyl-1-(3-phénylaminocarbonyl-pyridin-4-ylméthyl)-3-(4-trifluorométhoxy-phényl)-imidazolidine-2,4-dione
- la 5,5-diméthyl-1-[3-(méthylphénylamino)carbonyl-pyridin-4-ylméthyl]-3-(4-trifluorométhoxy-phényl)-imidazolidine-2,4-dione
- la 5,5-diméthyl-1-[3-(4-méthoxyphényl)méthylaminocarbonyl-pyridin-4-ylméthyl]-3-(4-trifluorométhoxy-phényl)-imidazolidine-2,4-dione
- la 5,5-diméthyl-1-[3-(4-chlorophényl)méthylaminocarbonyl-pyridin-4-ylméthyl]-3-(4-trifluorométhoxy-phényl)-imidazolidine-2,4-dione
- la 5,5-diméthyl-1-[3-(4-morpholinylamino)carbonyl-pyridin-4-ylméthyl]-3-(4-trifluorométhoxy-phényl)-imidazolidine-2,4-dione
- la 5,5-diméthyl-1-(3-aminocarbonyl-pyridin-4-ylméthyl)-3-(4-tr-ifluorométhoxy-phényl)-imidazolidine-2,4-dione
- la 5,5-diméthyl-1-(3-phénoxy-pyridin-4-ylméthyl)-3-(4-trifluorométhoxy-phényl)-imidazolidine-2,4-dione
- la 5,5-diméthyl-1-(3-méthoxy-pyridin-4-ylméthyl)-3-(4-trifluorométhoxy-phényl)-imidazolidine-2,4-dione
- la 5,5-diméthyl-l-(3-éthoxy-pyridin-4-ylméthyl)-3-(4-trifluorométhoxy-phényl)-imidazolidine-2,4-dione
- la 5,5-diméthyl-1-[3-(1-pyrrolidinyl)éthoxy-pyridin-4-ylméthyl]-3-(4-trifluorométhoxy-phényl)-imidazolidine-2,4-dione
- la 5,5-diméthyl-1-(3-benzyloxy-pyridin-4-ylméthyl)-3-(4-trifluorométhoxy-phényl)-imidazolidine-2,4-dione
- la 5,5-diméthyl-1-(3-benGoyloxy-pyridin-4-ylméthyl)-3-(4-trifluorométhoxy-phényl)-imidazolidine-2,4-dione
- la 5,5-diméthyl-1-[3-(4,5-dihydro-1H-imidazol-2-yl)-pyridin-4-ylméthyl]-3-(4-trifluorométhoxy-phényl)-imidazolidine-2,4-dione
- la 5,5-diméthyl-1-[3-(1H-imidazol-2-yl)-pyridin-4-ylméthyl]-3-(4-trifluorométhoxy-phényl)-imidazolidine-2,4-dione
- la 5,5-diméthyl-1-[3-(1H-tétrazol-5-yl)-pyridin-4-ylméthyl]-3-(4-trifluorométhoxy-phényl)-imidazolidine-2,4-dione
- la 5,5-diméthyl-1-(3-amidino-pyridin-4-ylméthyl)-3-(4-trifluorométhoxy-phényl)-imidazolidine-2,4-dione
- la 5,5-diméthyl-1-(3-diméthylaminométhyl-pyridin-4-ylméthyl)3-(4-trifluorométhoxy-phényl)-imidazolidine-2,4-dione
- la 5,5-diméthyl-1-(3-aminométhyl-pyridin-4-ylméthyl)-3-(4-trifluorométhoxy-phényl)-imidazolidine-2,4-dione
- la 4-(2-méthyl-pyridin)-4-ylméthyl-6-(4-trifluorométhoxy-phényl)-4,6-diaza-spiro[2.4]heptane-5,7-dione
- la 4-(2-éthyl-pyridin)-4-ylméthyl-6-(4-trifluorométhoxy-phényl)-4,6-diaza-spiro[2.4]heptane-5,7-dione
- la 4-(2-isopropyl-pyridin)-4-ylméthyl-6-(4-trifluorométhoxy-phényl)-4,6-diaza-spiro[2.4]heptane-5,7-dione
- la 4-(2-ter-butyl-pyridin)-4-ylméthyl-6-(4-trifluorométhoxy-phényl)-4,6-diaza-spiro[2.4]heptane-5,7-dione
- la 4-(2-isobutyl-pyridin)-4-ylméthyl-6-(4-trifluorométhoxy-phényl)-4,6-diaza-spiro[2.4]heptane-5,7-dione
- la 4-(2-cyclohéxyl-pyridin)-4-ylméthyl-6-(4-trifluorométhoxy-phényl)-4,6-diaza-spiro[2.4]heptane-5,7-dione
- la 4-(2-phényl-pyridin)-4-ylméthyl-6-(4-trifluorométhoxy-phényl)-4,6-diaza-spiro[2.4]heptane-5,7-dione
- la 4-[2-(4-chlorophényl)-pyridin]-4-ylméthyl-6-(4-trifluorométhoxy-phényl)-4,6-diaza-spiro[2.4]heptane-5,7-dione
- la 4-[2-(3-chlorophényl)-pyridin]-4-ylméthyl-6-(4-trifluorométhoxy-phényl)-4,6-diaza-spiro[2.4]heptane-5,7-dione
- la 4-[2-(2-chlorophényl)-pyridin]-4-ylméthyl-6-(4-trifluorométhoxy-phényl)-4,6-diaza-spiro[2.4]heptane-5,7-dione
- la 4-[2-(4-fluorophényl)-pyridin]-4-ylméthyl-6-(4-trifluorométhoxy-phényl)-4,6-diaza-spiro[2.4]heptane-5,7-dione
- la 4-[2-(4-méthoxyphényl)-pyridin]-4-ylméthyl-6-(4-trifluorométhoxy-phényl)-4,6-diaza-spiro[2.4]heptane-5,7-dione
- la 4-(2-benzyl-pyridin)-4-ylméthyl-6-(4-trifluorométhoxy-phényl)-4,6-diaza-spiro[2.4]heptane-5,7-dione
- la 4-(2,2'bipyridin)-4-ylméthyl-6-(4-trifluorométhoxy-phényl)-4,6-diaza-spiro[2.4]heptane-5,7-dione
- la 4-(2-hydroxyméthyl-pyridin)-4-ylméthyl-6-(4-trifluorométhoxy-phényl)-4,6-diaza-spiro[2.4]heptane-5,7-dione
- la 4-(2-chloro-pyridin)-4-ylméthyl-6-(4-trifluorométhoxy-phényl)-4,6-diaza-spiro[2.4]heptane-5,7-dione
- la 4-(2-fluoro-pyridin)-4-ylméthyl-6-(4-trifluorométhoxy-phényl)-4,6-diaza-spiro[2.4]heptane-5,7-dione
- la 4-(2-bromo-pyridin)-4-ylméthyl-6-(4-trifluorométhoxy-phényl)-4,6-diaza-spiro[2.4]heptane-5,7-dione
- la 4-(2-amino-pyridin)-4-ylméthyl-6-(4-trifluorométhoxy-phényl)-4,6-diaza-spiro[2.4]heptane-5,7-dione
- la 4-(2-méthylamino-pyridin)-4-ylméthyl-6-(4-trifluorométhoxy-phényl)-4,6-diaza-spiro[2.4]heptane-5,7-dione
- la 4-(2-éthylamino-pyridin)-4-ylméthyl-6-(4-trifluorométhoxy-phényl)-4,6-diaza-spiro[2.4]heptane-5,7-dione
- la 4-(2-isopropylamino-pyridin)-4-ylméthyl-6-(4-trifluorométhoxy-phényl)-4,6-diaza-spiro[2.4]heptane-5,7-dione
- la 4-(2-phénylamino-pyridin)-4-ylméthyl-6-(4-trifluorométhoxy-phényl)-4,6-diaza-spiro[2.4]heptane-5,7-dione
- la 4-(2-cyclohéxylamino-pyridin)-4-ylméthyl-6-(4-trifluorométhoxy-phényl)-4,6-diaza-spiro[2.4]heptane-5,7-dione
- la 4-(2-diéthylamino-pyridin)-4-ylméthyl-6-(4-trifluorométhoxy-phényl)-4,6-diaza-spiro[2.4]heptane-5,7-dione
- la 4-(2-N-cyclohéxyl-N-méthylamino-pyridin)-4-ylméthyl-6-(4-trifluorométhoxy-phényl)-4,6-diaza-spiro[2.4]heptane-5,7-dione
- la 4-(2-pipéridino-pyridin)-4-ylméthyl-6-(4-trifluorométhoxy-phényl)-4,6-diaza-spiro[2.4]heptane-5,7-dione
- la 4-(2-morpholino-pyridin)-4-ylméthyl-6-(4-trifluorométhoxy-phényl)-4,6-diaza-spiro[2.4]heptane-5,7-dione
- la 4-[2-(4-méthylpipérazino)-pyridin]-4-ylméthyl-6-(4-trifluoraméthoxy-phényl)-4,6-diaza-spiro[2.4]heptane-5,7-diode
- la 4-[2-(1H-pyrazol-lyl)-pyridin]-4-ylméthyl-6-(4-trifluorométhoxy-phényl)-4,6-diaza-spiro[2.4]heptane-5,7-dione
- la 4-[2-(2-benzoylhydrasino)-pyridin]-4-ylméthyl-6-(4-trifluorométhoxy-phényl)-4,6-diaza-spiro[2.4]heptane-5,7-dione
- la 4-(2-benzoylamino-pyridin)-4-ylméthyl-6-(4-trifluorométhoxy-phényl)-4,6-diaza-spiro[2.4]heptane-5,7-dione
- la 4-[2-(4-chlorobenzoylamino)-pyridin]-4-ylméthyl-6-(4-trifluorométhoxy-phényl)-4,6-diaza-spiro[2.4]heptane-5,7-dione,
- la 4-[2-(3-chlorobenzoylamino)-pyridin]-4-ylméthyl-6-(4-trifluorométhoxy-phényl)-4,6-diaza-spiro[2.4]heptane-5,7-dione
- la 4-[2-(2-chlorobenzoylamino)-pyridin]-4-ylméthyl-6-(4-trifluorométhoxy-phényl)-4,6-diaza-spiro[2.4]heptane-5,7-dione
- la 4-[2-(4-fluorobenzoylamino)-pyridin]-4-ylméthyl-6-(4-trifluorométhoxy-phényl)-4,6-diaza-spiro[2.4]heptane-5,7-dione
- la 4-[2-(4-méthoxybenzoylamino)-pyridin]-4-ylméthyl-6-(4-trifluorométhoxy-phényl)-4,6-diaza-spiro[2.4]heptane-5,7-dione
- la 4-(2-acétamido-pyridin)-4-ylméthyl-6-(4-trifluorométhoxy-phényl)-4,6-diaza-spiro[2.4]heptane-5,7-dione
- la 4-(2-phénylacétamido-pyridin)-4-ylméthyl-6-(4-trifluorométhoxy-phényl)-4,6-diaza-spiro[2.4]heptane-5,7-dione
- la 4-[2-(4-méthylpipérazino)acétamido-pyridin]-4-ylméthyl-6-(4-trifluorométhoxy-phényl)-4,6-diaza-spiro[2.4]heptane-5,7-dione
- la 4-(2-cyclohéxanylcarboxamido-pyridin)-4-ylméthyl-6-(4-trifluorométhoxy-phényl)-4,6-diaza-spiro[2.4]heptane-5,7-dione
- la 4-[2-(1-méthyl-4-pipéridinyl)carbonylamino-pyridin]-4-ylméthyl-6-(4-trifluorométhoxyl-phényl)-4,6-diaza-spiro[2.4]heptane-5,7-dione
- la 4-[2-(2-furanylcarbonyl)amino-pyridin]-4-ylméthyl-6-(4-trifluorométhoxy-phényl)-4,6-diaza-spiro[2.4]heptane-5,7-dione
- la 4-(2-éthoxycarbonylamino-pyridin)-4-ylméthyl-6-(4-trifluorométhoxy-phényl)-4,6-diaza-spiro[2.4]heptane-5,7-dione
- la 4-(2-isopropoxycarbonylamino-pyridin)-4-ylméthyl-6-(4-trifluorométhoxy-phényl)-4,6-diaza-spiro[2.4]heptane-5,7-dione
- la 4-(2-butoxycarbonylamino-pyridin)-4-ylméthyl-6-(4-trifluorométhoxy-phényl)-4,6-diaza-spiro[2.4]heptane-5,7-dione
- la 4-[2-(2-diéthylaminoéthoxycarbonylamino)-pyridin]-4-ylméthyl-6-(4-trifluorométhoxy-phényl)-4,6-diaza-spiro[2.4]heptane-5,7-dione
- la 4-(2-phénoxycarbonylamino-pyridin)-4-ylméthyl-6-(4-trifluorométhoxy-phényl)-4,6-diaza-spiro[2.4]heptane-5,7-dione
- la 4-[2-(4-chloroanilino)carbonylamino-pyridin]-4-ylméthyl)-6-(4-trifluorométhoxy-phényl)-4,6-diaza-spiro[2.4]heptane-5,7-dione
- la 4-(2-anilinocarbonylamino-pyridin)-4-ylméthyl-6-(4-trifluorométhoxy-phényl)-4,6-diaza-spiro[2.4]heptane-5,7-dione
- la 4-(2-benzylaminocarbonylamino-pyridin)-4-ylméthyl-6-(4-trifluorométhoxy-phényl)-4,6-diaza-spiro[2.4]heptane-5,7-dione
- la 4-[2-(2-pyridylamino)carbonylamino-pyridin]-4-ylméthyl-6-(4-trifluorométhoxy-phényl)-4,6-diaza-spiro[2.4]heptane-5,7-dione
- la 4-(2-uréido-pyridin)-4-ylméthyl-6-(4-trifluorométhoxy-phényl)-4,6-diaza-spiro[2.4]heptane-5,7-dione
- la 4-(2-benzylaminosulfonylamino-pyridin)-4-ylméthyl-6-(4-trifluorométhoxy-phényl)-4,6-diaza-spiro[2.4]heptane-5,7-dione
- la 4-(2-méthylaminosulfonylamino-pyridin)-4-ylméthyl-6-(4-trifluorométhoxy-phényl)-4,6-diaza-spiro[2.4]heptane-5,7-dione
- la 4-(2-aminosulfonylamino-pyridin)-4-ylméthyl-6-(4-trifluorométhoxy-phényl)-4,6-diaza-spiro[2.4]heptane-5,7-dione
- la 4-(2-morpholinosulfonylamino-pyridin)-4-ylméthyl-6-(4-trifluorométhoxy-phényl)-4,6-diaza-spiro[2.4]heptane-5,7-dione
- la 4-(2-diméthylaminosulfonylamino-pyridin)-4-ylméthyl-6-(4-trifluorométhoxy-phényl)-4,6-diaza-spiro[2.4] heptane-5,7-dione
- la 4-(2-anilinosulfonylamino-pyridin)-4-ylméthyl-6-(4-trifluorométhoxy-phényl)-4,6-diaza-spiro[2.4]heptane-5,7-dione
- la 4-(2-phénylsulfonylamino-pyridin)-4-ylméthyl-6-(4-trifluorométhoxy-phényl)-4,6-diaza-spiro[2.4]heptane-5,7-dione
- la 4-[2-(2-thiényl)sulfonylamino-pyridin]-4-ylméthyl-6-(4-trifluorométhoxy-phényl)-4,6-diaza-spiro[2.4]heptane-5,7-dione
- la 4-(2-méthylsulfonylamino-pyridin)-4-ylméthyl-6-(4-trifluorométhoxy-phényl)-4,6-diaza-spiro[2.4]heptane-5,7-dione
- la 4-[2-(4-chlorophényl)sulfonylamino-pyridin]-4-ylméthyl-6-(4-trifluorométhoxy-phényl)-4,6-diaza-spiro[2.4]heptane-5,7-dione
- la 4-(2-benzylsulfonylamino-pyridin)-4-ylméthyl-6-(4-trifluorométhoxy-phényl)-4,6-diaza-spiro[2.4]heptane-5,7-dione
- la 4-(2-hydroxycarbonyl-pyridin)-4-ylméthyl-6-(4-trifluorométhoxy-phényl)-4,6-diaza-spiro[2.4]heptane-5,7-dione
- la 4-(2-éthoxycarbonyl-pyridin)-4-ylméthyl-6-(4-trifluorométhoxy-phényl)-4,6-diaza-spiro[2.4]heptane-5,7-dione
- la 4-(2-ter-butoxycarbonyl-pyridin)-4-ylméthyl-6-(4-trifluorométhoxy-phényl)-4,6-diaza-spiro[2.4]heptane-5,7-dione
- la 4-(2-phénoxycarbonyl-pyridin)-4-ylméthyl-6-(4-trifluorométhoxy-phényl)-4,6-diaza-spiro[2.4]heptane-5,7-dione
- la 4-[2-(4-méthylpipérazinocarbonyl)-pyridin]-4-ylméthyl-6-(4-trifluorométhoxy-phényl)-4,6-diaza-spiro[2.4]heptane-5,7-dione
- la 4-[2-(méthylamino)carbonyl-pyridin]-4-ylméthyl-6-(4-trifluorométhoxy-phényl)-4,6-diaza-spiro[2.4]heptane-5,7-dione
- la 4-[2-(diméthylamino)carbonyl-pyridin]-4-ylméthyl-6-(4-trifluorométhoxy-phényl)-4,6-diaza-spiro[2.4]heptane-5,7-dione
- la 4-(2-pipéridinocarbonyl-pyridin)-4-ylméthyl-6-(4-trifluorométhoxy-phényl)-4,6-diaza-spiro[2.4]heptane-5,7-dione
- la 4-(2-morpholinocarbonyl-pyridin)-4-ylméthyl-6-(4-trifluorométhoxy-phényl)-4,6-diaza-spiro[2.4]heptane-5,7-dione
- la 4-[2-(4-phénylpipérazinocarbonyl)-pyridin]-4-ylméthyl-6-(4-trifluorométhoxy-phényl)-4,6-diaza-spiro[2.4]heptane-5,7-dione
- la 4-(2-phénylaminocarbonyl-pyridin)-4-ylméthyl-6-(4-trifluorométhoxy-phényl)-4,6-diaza-spiro[2.4]heptane-5,7-dione
- la 4-[2-(méthylphénylamino)carbonyl-pyridin]-4-ylméthyl-6-(4-trifluorométhoxy-phényl)-4,6-diaza-spiro[2.4]heptane-5,7-dione
- la 4-[2-(4-méthoxyphényl)méthylaminocarbonyl-pyridin]-4-ylméthyl-6-(4-trifluorométhoxy-phényl)-4,6-diaza-spiro[2.4]heptane-5,7-dione
- la 4-[2-(4-chlorophényl)méthylaminocarbonyl-pyridin]-4-ylméthyl-6-(4-trifluorométhoxy-phényl)-4,6-diaza-spiro[2.4]heptane-5,7-dione
- la 4-[2-(4-morpholinylamino)carbonyl-pyridin]-4-ylméthyl-6-(4-trifluorométhoxy-phényl)-4,6-diaza-spiro[2.4]heptane-5,7-dione
- la 4-(2-aminocarbonyl-pyridin)-4-ylméthyl-6-(4-trifluorométhoxy-phényl)-4,6-diaza-spiro[2.4]heptane-5,7-dione
- la 4-(2-phénoxy-pyridin)-4-ylméthyl-6-(4-trifluorométhoxy-phényl)-4,6-diaza-spiro[2.4]heptane-5,7-dione
- la 4-(2-méthoxy-pyridin)-4-ylméthyl-6-(4-trifluoxométhoxy-phényl)-4,6-diaza-spiro[2.4]heptane-5,7-dione
- la 4-(2-éthoxy-pyridin)-4-ylméthyl-6-(4-trifluorométhoky-phényl) -4,6-diaza-spiro [2.4] heptane-5, 7-dione
- la 4-[2-(1-pyrrolidinyl)éthoxy-pyridin]-4-ylméthyl-6-(4-trifluorométhoxy-phényl)-4,6-diaza-spiro[2.4]heptane-5,7-dione
- la 4-(2-benzyloxy-pyridin)-4-ylméthyl-6-(4-trifluorométhoxy-phényl)-4,6-diaza-spiro[2.4]heptane-5,7-dione
- la 4-(2-benzoyloxy-pyridin)-4-ylméthyl-6-(4-trifluorométhoxy-phényl)-4,6-diaza-spiro[2.4]heptane-5,7-dione
- la 4-[2-(4,5-dihydro-1H-imidazol-2-yl)-pyridin]-4-ylméthyl-6-(4-trifluorométhoxy-phényl)-4,6-diaza-spiro[2.4]heptane-5,7-dione
- la 4-[2-(1H-imidazol-2-yl)-pyridin]-4-ylméthyl-6-(4-trifluorométhoxy-phényl)-4,6-diaza-spiro[2.4]heptane-5,7-dione
- la 4-[2-(1H-tétrazol-5-yl)-pyridin]-4-ylméthyl-6-(4-trifluorométhoxy-phényl)-4,6-diaza-spiro[2.4]heptane-5,7-dione
- la 4-(2-amidino-pyridin)-4-ylméthyl-6-(4-trifluorométhoxy-phényl)-4,6-diaza-spiro[2.4]heptane-5,7-dione
- la 4-(2-diméthylaminométhyl-pyridin)-4-ylméthyl-6-(4-trifluorométhoxy-phényl)-4,6-diaza-spiro[2.4]heptane-5,7-dione
- la 4-(2-aminométhyl-pyridin)-4-ylméthyl-6-(4-trifluorométhoxy-phényl)-4,6-diaza-spiro[2.4]heptane-5,7-dione
- la 4-(3-méthyl-pyridin)-4-ylméthyl-6-(4-trifluorométhoxy-phényl)-4,6-diaza-spiro[2.4]heptane-5,7-dione
- la 4-(3-éthyl-pyridin)-4-ylméthyl-6-(4-trifluorométhoxy-phényl)-4,6-diaza-spiro[2.4]heptane-5,7-dione
- la 4-(3-isopropyl-pyridin)-4-ylméthyl-6-(4-trifluorométhoxy-phényl)-4,6-diaza-spiro[2.4]heptane-5,7-dione
- la 4-(3-ter-butyl-pyridin)-4-ylméthyl-6-(4-trifluorométhoxy-phényl)-4,6-diaza-spiro[2.4]heptane-5,7-dione
- la 4-(3-isobutyl-pyridin)-4-ylméthyl-6-(4-trifluorométhoxy-phényl)-4,6-diaza-spiro[2.4]heptane-5,7-dione
- la 4-(3-cyclohéxyl-pyridin)-4-ylméthyl-6-(4-trifluorométhoxy-phényl)-4,6-diaza-spiro[2.4]heptane-5,7-dione
- la 4-(3-phényl-pyridin)-4-ylméthyl-6-(4-trifluorométhoxy-phényl)-4,6-diaza-spiro[2.4]heptane-5,7-dione
- la 4-[3-(4-chlorophényl)-pyridin]-4-ylméthyl-6-(4-trifluorométhoxy-phényl)-4,6-diaza-spiro[2.4]heptane-5,7-dione
- la 4-[3-(3-chlorophényl)-pyridin]-4-ylméthyl-6-(4-trifluorométhoxy-phényl)-4,6-diaza-spiro[2.4]heptane-5,7-dione
- la 4-[3-(2-chlorophényl)-pyridin]-4-ylméthyl-6-(4-trifluorométhoxy-phényl)-4,6-diaza-spiro[2.4]heptane-5,7-dione
- la 4-[3-(4-fluorophényl)-pyridin]-4-ylméthyl-6-(4-trifluorométhoxy-phényl)-4,6-diaza-spiro[2.4]heptane-5,7-dione
- la 4-[3-(4-méthoxyphényl)-pyridin]-4-ylméthyl-6-(4-trifluorométhoxy-phényl)-4,6-diaza-spiro[2.4]heptane-5,7-dione
- la 4-(3-benzyl-pyridin)-4-ylméthyl-6-(4-trifluorométhoxy-phényl)-4,6-diaza-spiro[2.4]heptane-5,7-dione
- la 4-(3,2'bipyridin)-4-ylméthyl-6-(4-trifluorométhoxy-phényl)-4,6-diaza-spiro[2.4]heptane-5,7-dione
- la 4-(3-hydroxyméthyl-pyridin)-4-ylméthyl-6-(4-trifluorométhoxy-phényl)-4,6-diaza-spiro[2.4]heptane-5,7-dione
- la 4-(3-chloro-pyridin)-4-ylméthyl-6-(4-trifluorométhoxy-phényl)-4,6-diaza-spiro[2.4]heptane-5,7-dione
- la 4-(3-fluoro-pyridin)-4-ylméthyl-6-(4-trifluorométhoxy-phényl)-4,6-diaza-spiro[2.4]heptane-5,7-dione
- la 4-(3-bromo-pyridin)-4-ylméthyl-6-(4-trifluorométhoxy-phényl)-4,6-diaza-spiro[2.4]heptane-5,7-dione
- la 4-(3-amino-pyridin)-4-ylméthyl-6-(4-trifluorométhoxy-phényl)-4,6-diaza-spiro[2.4]heptane-5,7-dione
- la 4-(3-méthylamino-pyridin)-4-ylméthyl-6-(4-trifluorométhoxy-phényl)-4,6-diaza-spiro[2.4]heptane-5,7-dione
- la 4-(3-éthylamino-pyridin)-4-ylméthyl-6-(4-trifluorométhoxy-phényl)-4,6-diaza-spiro[2.4]heptane-5,7-dione
- la 4-(3-isopropylamino-pyridin)-4-ylméthyl-6-(4-trifluorométhoxy-phényl)-4,6-diaza-spiro[2.4]heptane-5,7-dione
- la 4-(3-phénylamino-pyridin)-4-ylméthyl-6-(4-trifluorométhoxy-phényl)-4,6-diaza-spiro[2.4]heptane-5,7-dione
- la 4-(3-cyclohéxylamino-pyridin)-4-ylméthyl-6-(4-trifluorométhoxy-phényl)-4,6-diaza-spiro[2.4]heptane-5,7-dione
- la 4-(3-diéthylamino-pyridin)-4-ylméthyl-6-(4-trifluorométhoxy-phényl)-4,6-diaza-spiro[2.4]heptane-5,7-dione
- la 4-(3-N-cyclohéxyl-N-méthylamino-pyridin)-4-ylméthyl-6-(4-trifluorométhoxy-phényl)-4,6-diaza-spiro[2.4]heptane-5,7-dione
- la 4-(3-pipéridino-pyridin)-4-ylméthyl-6-(4-trifluorométhoxy-phényl)-4,6-diaza-spiro[2.4]heptane-5,7-dione
- la 4-(3-morpholino-pyridin)-4-ylméthyl-6-(4-trifluorométhoxy-phényl)-4,6-diaza-spiro[2.4]heptane-5,7-dione
- la 4-[3-(4-méthylpipérazino)-pyridin]-4-ylméthyl-6-(4-trifluorométhoxy-phényl)-4,6-diaza-spiro[2.4]heptane-5,7-dione
- la 4-[3-(1H-pyrazol-lyl)-pyridin]-4-ylméthyl-6-(4-trifluorométhoxy-phényl)-4,6-diaza-spiro[2.4]heptane-5,7-diode
- la 4-[3-(2-benzoylhydrazino)-pyridin]-4-ylméthyl-6-(4-trifluorométhoxy-phényl)-4,6-diaza-spiro[2.4]heptane-5,7-dione
- la 4-(3-benzoylamino-pyridin)-4-ylméthyl-6-(4-trifluorométhoxy-phényl)-4,6-diaza-spiro[2.4]heptane-5,7-dione
- la 4-[3-(4-chlorobenzoylamino)-pyridin]-4-ylméthyl-6-(4-trifluorométhoxy-phényl)-4,6-diaza-spiro[2.4]heptane-5,7-dione
- la 4-[3-(3-chlorobenzoylamino)-pyridin]-4-ylméthyl-6-(4-trifluorométhoxy-phényl)-4,6-diaza-spiro[2.4]heptane-5,7-dione
- la 4-[3-(2-chlorobenzoylamino)-pyridin]-4-ylméthyl-6-(4-trifluorométhoxy-phényl)-4,6-diaza-spiro[2.4]heptane-5,7-dione
- la 4-[3-(4-fluorobenzoylamino)-pyridin]-4-ylméthyl-6-(4-trifluorométhoxy-phényl)-4,6-diaza-spiro[2.4]heptane-5,7-dione
- la 4-[3-(4-méthoxybenzoylamino)-pyridin]-4-ylméthyl-6-(4-trifluorométhoxy-phényl)-4,6-diaza-spiro[2.4]heptane-5,7-dione
- la 4-(3-acétamido-pyridin)-4-ylméthyl-6-(4-trifluorométhoxy-phényl)-4,6-diaza-spiro[2.4]heptane-5,7-dione

- la 4-(3-phénylacétamido-pyridin)-4-ylméthyl-6-(4-trifluorométhoxy-phényl)-4,6-diaza-spiro[2.4]heptane-5,7-dione
- la 4-[3-(4-méthylpipérazino)acétamido-pyridin]-4-ylméthyl-6-(4-trifluorométhoxy-phényl)-4,6-diaza-spiro[2.4]heptane-5,7-dione
- la 4-(3-cyclohéxanylcarboxamido-pyridin)-4-ylméthyl-6-(4-trifluorométhoxy-phényl)-4,6-diaza-spiro[2.4]heptane-5,7-dione
- la 4-[3-(1-méthyl-4-pipéridinyl)carbonylamino-pyridin]-4-ylméthyl-6-(4-trifluorométhoxy-phényl)-4,6-diaza-spiro[2.4]heptane-5,7-dione
- la 4-[3-(2-furanylcarbonyl)amino-pyridin]-4-ylméthyl-6-(4-trifluorométhoxy-phényl)-4,6-diaza-spiro[2.4]heptane-5,7-dione
- la 4-(3-éthoxycarbonylamino-pyridin)-4-ylméthyl-6-(4-trifluorométhoxy-phényl)-4,6-diaza-spiro[2.4]heptane-5,7-dione
- la 4-(3-isopropoxycarbonylamino-pyridin)-4-ylméthyl-6-(4-trifluorométhoxy-phényl)-4,6-diaza-spiro[2.4]heptane-5,7-dione
- la 4-(3-butoxycarbonylamino-pyridin)-4-ylméthyl-6-(4-trifluorométhoxy-phényl)-4,6-diaza-spiro[2.4]heptane-5,7-dione
- la 4-[3-(2-diéthylaminoéthoxycarbonylamino)-pyridin]-4-ylméthyl-6-(4-trifluorométhoxy-phényl)-4,6-diaza-spiro[2.4]heptane-5,7-dione
- la 4-(3-phénoxycarbonylamino-pyridin)-4-ylméthyl-6-(4-trifluorométhoxy-phényl)-4,6-diaza-spiro[2.4]heptane-5,7-dione
- la 4-[3-(4-chloroanilino)carbonylamino-pyridin]-4-ylméthyl)-6-(4-trifluorométhoxy-phényl)-4,6-diaza-spiro[2.4]heptane-5,7-dione
- la 4-(3-anilinocarbonylamino-pyridin)-4-ylméthyl-6-(4-trifluorométhoxy-phényl)-4,6-diaza-spiro[2.4]heptane-5,7-dione
- la 4-(3-benzylaminocarbonylamino-pyridin)-4-ylméthyl-6-(4-trifluorométhoxy-phényl)-4,6-diaza-spiro[2.4]heptane-5,7-dione
- la 4-[3-(2-pyridylamino)carbonylamino-pyridin]-4-ylméthyl-6-(4-trifluorométhoxy-phényl)-4,6-diaza-spiro[2.4]heptane-5,7-dione
- la 4-(3-uréido-pyridin)-4-ylméthyl-6-(4-trifluorométhoxy-phényl)-4,6-diaza-spiro[2.4]heptane-5,7-dione
- la 4-(3-benzylaminosulfonylamino-pyridin)-4-ylméthyl-6-(4-trifluorométhoxy-phényl)-4,6-diaza-spiro[2.4]heptane-5,7-dione
- la 4-(3-méthylaminosulfonylamino-pyridin)-4-ylméthyl-6-(4-trifluorométhoxy-phényl)-4,6-diaza-spiro[2.4]heptane-5,7-dione
- la 4-(3-aminosulfonylamino-pyridin)-4-ylméthyl-6-(4-trifluorométhoxy-phényl)-4,6-diaza-spiro[2.4]heptane-5,7-dione
- la 4-(3-morpholinosulfonylamino-pyridin)-4-ylméthyl-6-(4-trifluorométhoxy-phényl)-4,6-diaza-spiro[2.4]heptane-5,7-dione
- la 4-(3-diméthylaminosulfonylamino-pyridin)-4-ylméthyl-6-(4-trifluorométhoxy-phényl)-4,6-diaza-spiro[2.4]heptane-5,7-dione
- la 4-(3-anilinosulfonylamino-pyridin)-4-ylméthyl-6-(4-trifluorométhoxy-phényl)-4,6-diaza-spiro[2.4]heptane-5,7-dione
- la 4-(3-phénylsulfonylamino-pyridin)-4-ylméthyl-6-(4-trifluorométhoxy-phényl)-4,6-diaza-spiro[2.4]heptane-5,7-dione
- la 4-[3-(2-thiényl)sulfonylamino-pyridin]-4-ylméthyl-6-(4-trifluorométhoxy-phényl)-4,6-diaza-spiro[2.4]heptane-5,7-dione
- la 4-(3-méthylsulfonylamino-pyridin)-4-ylméthyl-6-(4-trifluorométhoxy-phényl)-4,6-diaza-spiro[2.4]heptane-5,7-dione
- la 4-[3-(4-chlorophényl)sulfonylamino-pyridin]-4-ylméthyl-6-(4-trifluorométhoxy-phényl)-4,6-diaza-spiro[2.4]heptane-5,7-dione
- la 4-(3-benzylsulfonylamino-pyridin)-4-ylméthyl-6-(4-trifluorométhoxy-phényl)-4,6-diaza-spiro[2.4]heptane-5,7-dione
- la 4-(3-hydroxycarbonyl-pyridin)-4-ylméthyl-6-(4-trifluorométhoxy-phényl)-4,6-diaza-spiro[2.4]heptane-5,7-dione
- la 4-(3-éthoxycarbonyl-pyridin)-4-ylméthyl-6-(4-trifluorométhoxy-phényl)-4,6-diaza-spiro[2.4]heptane-5,7-diode
- la 4-(3-ter-butoxycarbonyl-pyridin)-4-ylméthyl-6-(4-trifluorométhoxy-phényl)-4,6-diaza-spiro[2.4]heptane-5,7-dione
- la 4-(3-phénoxycarbonyl-pyridin)-4-ylméthyl-6-(4-trifluorométhoxy-phényl)-4,6-diaza-spiro[2.4]heptane-5,7-dione
- la 4-[3-(4-méthylpipérazinocarbonyl)-pyridin]-4-ylméthyl-6-(4-trifluorométhoxy-phényl)-4,6-diaza-spiro[2.4]heptane-5,7-dione
- la 4-[3-(méthylamino)carbonyl-pyridin]-4-ylméthyl-6-(4-trifluorométhoxy-phényl)-4,6-diaza-spiro[2.4]heptane-5,7-dione
- la 4-[3-(diméthylamino)carbonyl-pyridin]-4-ylméthyl-6-(4-trifluorométhoxy-phényl)-4,6-diaza-spiro[2.4]heptane-5,7-dione
- la 4-(3-pipéridinocarbonyl-pyridin)-4-ylméthyl-6-(4-trifluorométhoxy-phényl)-4,6-diaza-spiro[2.4]heptane-5,7-dione
- la 4-(3-morpholinocarbonyl-pyridin)-4-ylméthyl-6-(4-trifluorométhoxy-phényl)-4,6-diaza-spiro[2.4]heptane-5,7-dione
- la 4-[3-(4-phénylpipérazinocarbonyl)-pyridin]-4-ylméthyl-6-(4-trifluorométhoxy-phényl)-4,6-diaza-spiro[2.4]heptane-5,7-dione
- la 4-(3-phénylaminocarbonyl-pyridin)-4-ylméthyl-6-(4-trifluorométhoxy-phényl)-4,6-diaza-spiro[2.4]heptane-5,7-dione
- la 4-[3-(méthylphénylamino)carbonyl-pyridin]-4-ylméthyl-6-(4-trifluorométhoxy-phényl)-4,6-diaza-spiro[2.4]heptane-5,7-dione
- la 4-[3-(4-méthoxyphényl)méthylaminocarbonyl-pyridin]-4-ylméthyl-6-(4-trifluorométhoxy-phényl)-4,6-diaza-spiro[2.4]heptane-5,7-dione
- la 4-[3-(4-chlorophényl)méthylaminocarbonyl-pyridin]-4-ylméthyl-6-(4-trifluorométhoxy-phényl)-4,6-diaza-spiro[2.4]heptane-5,7-dione
- la 4-[3-(4-morpholinylamino)carbonyl-pyridin]-4-ylméthyl-6-(4-trifluorométhoxy-phényl)-4,6-diaza-spiro[2.4]heptane-5,7-dione
- la 4-(3-aminocarbonyl-pyridin)-4-ylméthyl-6-(4-trifluorométhoxy-phényl)-4,6-diaza-spiro[2.4]heptane-5,7-dione
- la 4-(3-phénoxy-pyridin)-4-ylméthyl-6-(4-trifluorométhoxy-phényl)-4,6-diaza-spiro[2.4]heptane-5,7-dione
- la 4-(3-méthoxy-pyridin)-4-ylméthyl-6-(4-trifluorométhoxy-phényl)-4,6-diaza-spiro[2.4]heptane-5,7-dione
- la 4-(3-éthoxy-pyridin)-4-ylméthyl-6-(4-trifluorométhoxy-phényl)-4,6-diaza-spiro[2.4]heptane-5,7-dione
- la 4-[3-(1-pyrrolidinyl)éthoxy-pyridin]-4-ylméthyl-6-(4-trifluorométhoxy-phényl)-4,6-diaza-spiro[2.4]heptane-5,7-dione
- la 4-(3-benzyloxy-pyridin)-4-ylméthyl-6-(4-trifluorométhoxy-phényl)-4,6-diaza-spiro[2.4]heptane-5,7-dione
- la 4-(3-benzoyloxy-pyridin)-4-ylméthyl-6-(4-trifluorométhoxy-phényl)-4,6-diaza-spiro[2.4]heptane-5,7-dione
- la 4-[3-(4,5-dihydro-1H-imidazol-2-yl)-pyridin]-4-ylméthyl-6-(4-trifluorométhoxy-phényl)-4,6-diaza-spiro[2.4]heptane-5,7-dione
- la 4-[3-(1H-imidazol-2-yl)-pyridin]-4-ylméthyl-6-(4-trifluorométhoxy-phényl)-4,6-diaza-spiro[2.4]heptane-5,7-dione
- la 4-[3-(1H-tétrazol-5-yl)-pyridin]-4-ylméthyl-6-(4-trifluorométhoxy-phényl)-4,6-diaza-spiro[2.4]heptane-5,7-dione
- la 4-(3-amidino-pyridin)-4-ylméthyl-6-(4-trifluorométhoxy-phényl)-4,6-diaza-spiro[2.4]heptane-5,7-dione
- la 4-(3-diméthylaminométhyl-pyridin)-4-ylméthyl-6-(4-trifluorométhoxy-phényl)-4,6-diaza-spiro[2.4]heptane-5,7-dione
- la 4-(3-aminométhyl-pyridin)-4-ylméthyl-6-(4-trifluorométhoxy-phényl)-4,6-diaza-spiro[2.4]heptane-5,7-dione
- la 5-méthyl-1-(2-méthyl-pyridin-4-ylméthyl)-3-(4-trifluorométhoxy-phényl)-imidazolidine-2,4-dione
- la 5-méthyl-1-(2-éthyl-pyridin-4-ylméthyl)-3-(4-trifluorométhoxy-phényl)-imidazolidine-2,4-dione
- la 5-méthyl-1-(2-isopropyl-pyridin-4-ylméthyl)-3-(4-trifluorométhoxy-phényl)-imidazolidine-2,4-dione
- la 5-méthyl-1-(2-ter-butyl-pyridin-4-ylméthyl)-3-(4-trifluorométhoxy-phényl)-imidazolidine-2,4-dione
- la 5-méthyl-1-(2-isobutyl-pyridin-4-ylméthyl)-3-(4-trifluorométhoxy-phényl)-imidazolidine-2,4-dione
- la 5-méthyl-1-(2-cyclohéxyl-pyridin-4-ylméthyl)-3-(4-trifluorométhoxy-phényl)-imidazolidine-2,4-dione
- la 5-méthyl-1-(2-phényl-pyridin-4-ylméthyl)-3-(4-trifluorométhoxy-phényl)-imidazolidine-2,4-dione
- la 5-méthyl-1-[2-(4-chlorophényl)-pyridin-4-ylméthyl]-3-(4-trifluorométhoxy-phényl)-imidazolidine-2,4-dione
- la 5-méthyl-1-[2-(3-chlorophényl)-pyridin-4-ylméthyl]-3-(4-trifluorométhoxy-phényl)-imidazolidine-2,4-dione
- la 5-méthyl-1-[2-(2-chlorophényl)-pyridin-4-ylméthyl]-3-(4-trifluorométhoxy-phényl)-imidazolidine-2,4-dione
- la 5-méthyl-1-[2-(4-fluorophényl)-pyridin-4-ylméthyl]-3-(4-trifluorométhoxy-phényl)-imidazolidine-2,4-dione
- la 5-méthyl-1-[2-(4-méthoxyphényl)-pyridin-4-ylméthyl]-3-(4-trifluorométhoxy-phényl)-imidazolidine-2,4-dione
- la 5-méthyl-1-(2-benzyl-pyridin-4-ylméthyl)-3-(4-trifluorométhoxy-phényl)-imidazolidine-2,4-dione
- la 5-méthyl-1-(2,2'bipyridin-4-ylméthyl)-3-(4-trifluorométhoxy-phényl)-imidazolidine-2,4-dione
- la 5-méthyl-1-(2-hydroxyméthyl-pyridin-4-ylméthyl)-3-(4-trifluorométhoxy-phényl)-imidazolidine-2,4-dione
- la 5-méthyl-1-(2-chloro-pyridin-4-ylméthyl)-3-(4-trifluorométhoxy-phényl)-imidazolidine-2,4-dione
- la 5-méthyl-1-(2-fluoro-pyridin-4-ylméthyl)-3-(4-trifluorométhoxy-phényl)-imidazolidine-2,4-dione
- la 5-méthyl-1-(2-bromo-pyridin-4-ylméthyl)-3-(4-trifluorométhoxy-phényl)-imidazolidine-2,4-dione
- la 5-méthyl-1-(2-amino-pyridin-4-ylméthyl)-3-(4-trifluorométhoxy-phényl)-imidazolidine-2,4-dione
- la 5-méthyl-1-(2-méthylamino-pyridin-4-ylméthyl)-3-(4-trifluorométhoxy-phényl)-imidazolidine-2,4-dione
- la 5-méthyl-1-(2-éthylamino-pyridin-4-ylméthyl)-3-(4-trifluo-rométhoxy-phényl)-imidazolidine-2,4-dione
- la 5-méthyl-1-(2-isopropylamino-pyridin-4-ylméthyl)-3-(4-trifluorométhoxy-phényl)-imidazolidine-2,4-dione
- la 5-méthyl-1-(2-phénylamino-pyridin-4-ylméthyl)-3-(4-trifluorométhoxy-phényl)-imidazolidine-2,4-dione
- la 5-méthyl-1-(2-cyclohéxylamino-pyridin-4-ylméthyl)-3-(4-trifluorométhoxy-phényl)-imidazolidine-2,4-dione
- la 5-méthyl-1-(2-diéthylamino-pyridin-4-ylméthyl)-3-(4-trifluorométhoxy-phényl)-imidazolidine-2,4-dione
- la 5-méthyl-1-(2-N-cyclohéxyl-N-méthylamino-pyridin-4-ylméthyl)-3-(4-trifluorométhoxy-phényl)-imidazolidine-2,4-dione
- la 5-méthyl-1-(2-pipéridino-pyridin-4-ylméthyl)-3-(4-trifluorométhoxy-phényl)-imidazolidine-2,4-dione
- la 5-méthyl-1-(2-morpholino-pyridin-4-ylméthyl)-3-(4-trifluorométhoxy-phényl)-imidazolidine-2,4-dione
- la 5-méthyl-1-[2-(4-méthylpipérazino)-pyridin-4-ylméthyl]-3-(4-trifluorométhoxy-phényl)-imidazolidine-2,4-dione
- la 5-méthyl-1-[2-(1H-pyrazol-1yl)-pyridin-4-ylméthyl]-3-(4-trifluorométhoxy-phényl)-imidazolidine-2,4-dione
- la 5-méthyl-1-[2-(2-benzoylhydrazino)-pyridin-4-ylméthyl]-3-(4-trifluorométhoxy-phényl)-imidazolidine-2,4-dione
- la 5-méthyl-1-(2-benzoylamino-pyridin-4-ylméthyl)-3-(4-trifluoroinéthoxy-phényl)-imidazolidine-2,4-dione
- la 5-méthyl-1-[2-(4-chlorobenzoylamino)-pyridin-4-ylméthyl]-3-(4-trifluorométhoxy-phényl)-imidazolidine-2,4-dione
- la 5-méthyl-1-[2-(3-chlorobenzoylamino)-pyridin-4-ylméthyl]-3-(4-trifluorométhoxy-phényl)-imidazolidine-2,4-dione
- la 5-méthyl-1-[2-(2-chlorobenzoylamino)-pyridin-4-ylméthyl]-3-(4-trifluorométhoxy-phényl)-imidazolidine-2,4-dione
- la 5-méthyl-1-[2-(4-fluorobenzoylamino)-pyridin-4-ylméthyl]-3-(4-trifluorométhoxy-phényl)-imidazolidine-2,4-dione
- la 5-méthyl-1-[2-(4-méthoxybensoylamino)-pyridin-4-ylméthyl]-3-(4-trifluorométhoxy-phényl)-imidasolidine-2,4-dione
- la 5-méthyl-1-(2-acétamido-pyridin-4-ylméthyl)-3-(4-trifluorométhoxy-phényl)-imidazolidine-2,4-dione
- la 5-méthyl-1-(2-phénylacétamido-pyridin-4-ylméthyl)-3-(4-trifluorométhoxy-phényl)-imidazolidine-2,4-dione
- la 5-méthyl-1-[2-(4-méthylpipérazino)acétamido-pyridin-4-ylméthyl]-3-(4-trifluorométhoxy-phényl)-imidazolidine-2,4-dione
- la 5-méthyl-1-(2-cyclohéxanylcarboxamido-pyridin-4-ylméthyl)-3-(4-trifluorométhoxy-phényl)-imidazolidine-2,4-dione
- la 5-méthyl-1-[2-(1-méthyl-4-pipéridinyl)carbonylamino-pyridin-4-ylméthyl]-3-(4-trifluorométhoxy-phényl)-imidazolidine-2,4-dione
- la 5-méthyl-1-[2-(2-furanylcarbonyl)amino-pyridin-4-ylméthyl]-3-(4-trifluorométhoxy-phényl)-imidazolidine-2,4-dione
- la 5-méthyl-1-(2-éthoxycarbonylamino-pyridin-4-ylméthyl)-3-(4-trifluorométhoxy-phényl)-imidazolidine-2,4-dione
- la 5-méthyl-1-(2-isopropoxycarbonylamino-pyridin-4-ylméthyl)-3-(4-trifluorométhoxy-phényl)-imidazolidine-2,4-dione
- la 5-méthyl-1-(2-butoxycarbonylamino-pyridin-4-ylméthyl)-3-(4-trifluorométhoxy-phényl)-imidazolidine-2,4-dione
- la 5-méthyl-1-[2-(2-diéthylaminoéthoxycarbonylamino)-pyridin-4-ylméthyl]-3-(4-trifluorométhoxy-phényl)-imidazolidine-2,4-dione
- la 5-méthyl-1-(2-phénoxycarbonylamino-pyridin-4-ylméthyl)-3-(4-trifluorométhoxy-phényl)-imidazolidine-2,4-dione
- la 5-méthyl-1-[2-(4-chloroanilino)carbonylamino-pyridin-4-ylméthyl]-3-(4-trifluorométhoxy-phényl)-imidazolidine-2,4-dione
- la 5-méthyl-1-(2-anilinocarbonylamino-pyridin-4-ylméthyl)-3-(4-trifluorométhoxy-phényl)-imidazolidine-2,4-dione.
- la 5-méthyl-1-(2-benzylaminocarbonylamino-pyridin-4-ylméthyl)-3-(4-trifluorométhoxy-phényl)-imidazolidine-2,4-dione
- la 5-méthyl-1-[2-(2-pyridylamino)carbonylamino-pyridin-4-ylméthyl]-3-(4-trifluorométhoxy-phényl)-imidazolidine-2,4-dione
- la 5-méthyl-1-(2-uréido-pyridin-4-ylméthyl)-3-(4-trifluorométhoxy-phényl)-imidazolidine-2,4-dione
- la 5-méthyl-1-(2-benzylaminosulfonylamino-pyridin-4-ylméthyl)-3-(4-trifluorométhoxy-phényl)-imidazolidine-2,4-dione
- la 5-méthyl-1-(2-méthylaminosulfonylamino-pyridin-4-ylméthyl)-3-(4-trifluorométhoxy-phényl)-imidazolidine-2,4-dione
- la 5-méthyl-1-(2-aminosulfonylamino-pyridin-4-ylméthyl)-3-(4-trifluorométhoxy-phényl)-imidazolidine-2,4-dione
- la 5-méthyl-1-(2-morpholinosulfonylamino-pyridin-4-ylméthyl)-3-(4-trifluorométhoxy-phényl)-imidazolidine-2,4-dione
- la 5-méthyl-1-(2-diméthylaminosulfonylamino-pyridin-4-ylméthyl)-3-(4-trifluorométhoxy-phényl)-imidazolidine-2,4-dione
- la 5-méthyl-1-(2-anilinosulfonylamino-pyridin-4-ylméthyl)-3-(4-trifluorométhoxy-phényl)-imidazolidine-2,4-dione
- la 5-méthyl-1-(2-phénylsulfonylamino-pyridin-4-ylméthyl)-3-(4-trifluorométhoxy-phényl)-imidazolidine-2,4-dione
- la 5-méthyl-1-[2-(2-thiényl)sulfonylamino-pyridin-4-ylméthyl]-3-(4-trifluorométhoxy-phényl)-imidasolidine-2,4-dione
- la 5-méthyl-1-(2-méthyilsulfonylamino-pyridin-4-ylméthyl)-3-(4-trifluorométhoxy-phényl)-imidazolidine-2,4-dione
- la 5-méthyl-1-[2-(4-chlorophényl)sulfonylamino-pyridin-4-ylméthyl]-3-(4-trifluorométhoxy-phényl)-imidazolidine-2,4-dione
- la 5-méthyl-1-(2-benzylsulfonylamino-pyridin-4-ylméthyl)-3-(4-trifluorométhoxy-phényl)-imidazolidine-2,4-dione
- la 5-méthyl-1-(2-hydroxycarbonyl-pyridin-4-ylmêthyl)-3-(4-trifluorométhoxy-phênyl)-imidazolidine-2,4-dione
- la 5-méthyl-1-(2-éthoxycarbonyl-pyridin-4-ylmëthyl)-3-(4-trifluorométhoxy-phényl)-imidazolidine-2,4-dione
- la 5-méthyl-1-(2-ter-butoxycarbonyl-pyridin-4-ylméthyl)-3-(4-trifluorométhoxy-phényl)-imidazolidine-2,4-dione
- la 5-mêthyl-1-(2-phénoxycarbonyl-pyridin-4-ylméthyl)-3-(4-trifluoromêthoxy-phényl)-imidazolidine-2,4-dione
- la 5-mêthyl-1-[2-(4-méthylpipérazinocarbonyl)-pyridin-4-ylméthyl]-3-(4-trifluorométhoxy-phényl)-imidazolidine-2,4-dione
- la 5-méthyl-1-[2-(méthylamino)carbonyl-pyridin-4-ylméthyl]-3-(4-trifluorométhoxy-phényl)-imidazolidine-2,4-dione
- la 5-méthyl-1-[2-(diméthylamino)carbonyl-pyridin-4-ylméthyl]-3-(4-trifluorométhoxy-phényl)-imidazolidine-2,4-dione
- la 5-méthyl-1-(2-pipéridinocarbonyl-pyridin-4-ylméthyl)-3-(4-trifluorométhoxy-phényl)-imidazolidine-2,4-dione
- la 5-méthyl-1-(2-morpholinocarbonyl-pyridin-4-ylméthyl)-3-(4-trifluoroinéthoxy-phényl)-imidazolidine-2,4-dione
- la 5-méthyl-1-[2-(4-phénylpipérazinocarbonyl)-pyridin-4-ylméthyl]-3-(4-trifluorométhoxy-phényl)-imidazolidine-2,4-dione
- la 5-méthyl-1-(2-phénylaminocarbonyl-pyridin-4-ylméthyl)-3-(4-trifluorométhoxy-phényl)-imidazolidine-2,4-dione
- la 5-méthyl-1-[2-(méthylphénylamino)carbonyl-pyridin-4-ylmêthyl]-3-(4-trifluorométhoxy-phényl)-imidazolidine-2,4-dione
- la 5-méthyl-1-[2-(4-méthoxyphényl)méthylaminocarbonyl-pyridin-4-ylméthyl]-3-(4-trifluorométhoxy-phényl)-imidazolidine-2,4-dione
- la 5-méthyl-1-[2-(4-chlorophényl)méthylaminocarbonyl-pyridin-4-ylméthyl]-3-(4-trifluorométhoxy-phényl)-imidazolidine-2,4-dione
- la 5-méthyl-1-[2-(4-morpholinylamino)carbonyl-pyridin-4-ylmëthyl]-3-(4-trifluoromëthoxy-phényl)-imidazolidine-2,4-diode
- la 5-mêthyl-1-(2-aminocarbonyl-pyridin-4-ylméthyl)-3-(4-trifluorométhoxy-phényl)-imidazolidine-2,4-dione
- la 5-méthyl-1-(2-phénoxy-pyridin-4-ylméthyl)-3-(4-trifluoromêthoxy-phényl)-imidazolidine-2,4-dione
- la 5-méthyl-1-(2-méthoxy-pyridin-4-ylmëthyl)-3-(4-trifluoromêthoxy-phényl)-imidazolidine-2,4-dione
- la 5-méthyl-1-(2-éthoxy-pyridin-4-ylméthyl)-3-(4-triflùorométhoxy-phényl)-imidazolidine-2,4-dione
- la 5-méthyl-1-[2-(1-pyrrolidinyl)éthoxy-pyridin-4-ylméthyl]-3-(4-trifluorométhoxy-phênyl)-imidazolidine-2,4-dione
- la 5-mëthyl-1-(2-benzyloxy-pyridin-4-ylmêthyl)-3-(4-trifluorométhoxy-phényl)-imidazolidine-2,4-dione
- la 5-méthyl-1-(2-benzoyloxy-pyridin-4-ylméthyl)-3-(4-trifluorométhoxy-phényl)-imidazolidine-2,4-dione
- la 5-méthyl-1-[2-(4,5-dihydro-1H-imidazol-2-yl)-pyridin-4-ylméthyl]-3-(4-trifluorométhoxy-phényl)-imidazolidine-2,4-dione
- la, 5-méthyl-1-[2-(1H-imidazol-2-yl)-pyridin-4-ylméthyl]-3-(4-trifluorométhoxy-phényl)-imidazolidine-2,4-dione
- la 5-méthyl-1-[2-(1H-tétrazol-5-yl)-pyridin-4-ylméthyl]-3-(4-trifluorométhoxy-phényl)-imidazolidine-2,4-dione
- la 5-méthyl-1-(2-amidino-pyridin-4-ylméthyl)-3-(4-trifluorométhoxy-phényl)-imidazolidine-2,4-dione
- la 5-méthyl-1-(2-diméthylaminométhyl-pyridin-4-ylméthyl)-3-(4-trifluorométhoxy-phényl)-imidazolidine-2,4-dione
- la 5-méthyl-1-(2-aminométhyl-pyridin-4-ylméthyl)-3-(4-trifluorométhoxy-phényl)-imidazolidine-2,4-dione
- la 5-méthyl-1-(3-méthyl-pyridin-4-ylméthyl)-3-(4-trifluorométhoxy-phényl)-imidazolidine-2,4-dione
- la 5-méthyl-1-(3-éthyl-pyridin-4-ylméthyl)-3-(4-trifluorométhoxy-phényl)-imidazolidine-2,4-dione
- la 5-méthyl-1-(3-isopropyl-pyridin-4-ylméthyl)-3-(4-trifluorométhoxy-phényl)-imidazolidiné-2,4-dione
- la 5-méthyl-1-(3-ter-butyl-pyridin-4-ylméthyl)-3-(4-trifluorométhoxy-phényl)-imidazolidine-2,4-dione
- la 5-méthyl-1-(3-isobutyl-pyridin-4-ylméthyl)-3-(4-trifluorométhoxy-phényl)-imidazolidine-2,4-dione
- la 5-méthyl-1-(3-cyclohéxyl-pyridin-4-ylméthyl)-3-(4-trifluorométhoxy-phényl)-imidazolidine-2,4-dione
- la 5-méthyl-1-(3-phényl-pyridin-4-ylméthyl)-3-(4-trifluorométhoxy-phényl)-imidazolidine-2,4-dione
- la 5-méthyl-1-[3-(4-chlorophényl)-pyridin-4-ylméthyl]-3-(4-trifluorométhoxy-phényl)-imidazolidine-2,4-dione
- la 5-méthyl-1-[3-(3-chlorophényl)-pyridin-4-ylméthyl]-3-(4-trifluorométhoxy-phényl)-imidazolidine-2,4-dione
- la 5-méthyl-1-[3-(2-chlorophényl)-pyridin-4-ylméthyl]-3-(4-trifluorométhoxy-phényl)-imidazolidine-2,4-dione
- la 5-méthyl-1-[3-(4-fluorophényl)-pyridin-4-ylméthyl]-3-(4-trifluorométhoxy-phényl)-imidazolidine-2,4-dione
- la 5-méthyl-1-[3-(4-méthoxyphényl)-pyridin-4-ylméthyl]-3-(4-trifluorométhoxy-phényl)-imidazolidine-2,4-dione
- la 5-méthyl-1-(3-benzyl-pyridin-4-ylméthyl)-3-(4-trifluorométhoxy-phényl)-imidazolidine-2,4-dione
- la 5-méthyl-1-(3,2-bipyridin-4-ylméthyl)-3-(4-trifluorométhoxy-phényl)-imidazolidine-2,4-dione
- la 5-méthyl-1-(3-hydroxyméthyl-pyridin-4-ylméthyl)-3-(4-trifluorométhoxy-phényl)-imidazolidine-2,4-dione
- la 5-méthyl-1-(3-chloro-pyridin-4-ylméthyl)-3-(4-trifluorométhoxy-phényl)-imidazolidine-2,4-dione
- la 5-méthyl-1-(3-fluoro-pyridin-4-ylméthyl)-3-(4-trifluorométhoxy-phényl)-imidazolidine-2,4-dione
- la 5-méthyl-1-(3-bromo-pyridin-4-ylméthyl)-3-(4-trifluorométhoxy-phényl)-imidazolidine-2,4-dione
- la 5-méthyl-1-(3-amino-pyridin-4-ylméthyl)-3-(4-trifluorométhoxy-phényl)-imidazolidine-2,4-dione
- la 5-méthyl-1-(3-méthylamino-pyridin-4-ylméthyl)-3-(4-trifluorométhoxy-phényl)-imidazolidine-2,4-dione
- la 5-méthyl-1-(3-éthylamino-pyridin-4-ylméthyl)-3-(4-trifluorométhoxy-phényl)-imidazolidine-2,4-dione
- la 5-méthyl-1-(3-isopropylamino-pyridin-4-ylméthyl)-3-(4-trifluorométhoxy-phényl)-imidazolidine-2,4-dione
- la 5-méthyl-1-(3-phénylamino-pyridin-4-ylméthyl)-3-(4-trifluorométhoxy-phényl)-imidazolidine-2,4-dione
- la 5-méthyl-1-(3-cyclohéxylamino-pyridin-4-ylméthyl)-3-(4-trifluorométhoxy-phényl)-imidazolidine-2,4-dione
- la 5-méthyl-1-(3-diéthylamino-pyridin-4-ylméthyl)-3-(4-trifluorométhoxy-phényl)-imidazolidine-2,4-dione
- la 5-méthyl-1-(3-N-cyclohéxyl-N-méthylamino-pyridin-4-ylméthyl)-3-(4-trifluorométhoxy-phényl)-imidazolidine-2,4-dione
- la 5-méthyl-1-(3-pipéridino-pyridin-4-ylméthyl)-3-(4-trifluorométhoxy-phényl)-imidazolidine-2,4-dione
- la 5-méthyl-1-(3-morpholino-pyridin-4-ylméthyl)-3-(4-trifluorométhoxy-phényl)-imidazolidine-2,4-dione
- la 5-méthyl-1-[3-(4-méthylpipérazino)-pyridin-4-ylméthyl]-3-(4-trifluorométhoxy-phényl)-imidazolidine-2,4-dione
- la 5-méthyl-1-[3-(1H-pyrazol-lyl)-pyridin-4-ylméthyl]-3-(4-trifluorométhoxyy-phényl)-imidazolidine-2,4-dione
- la 5-méthyl-1-[3-(2-benzoylhydrazino)-pyridin-4-ylméthyl]-3-(4-trifluo-rométhoxy-phényl)-imidazolidine-2,4-dione
- la 5-méthyl-1-(3-benzoylamino-pyridin-4-ylméthyl)-3-(4-trifluorométhoxy-phényl)-imidazolidine-2,4-dione
- la 5-méthyl-1-[3-(4-chlorobenzoylamino)-pyridin-4-ylméthyl]-3-(4-trifluorométhoxy-phényl)-imidazolidine-2,4-dione
- la 5-méthyl-1-[3-(3-chlorobenzoylamino)-pyridin-4-ylméthyl]-3-(4-trifluorométhoxy-phényl)-imidazolidine-2,4-dione
- la 5-méthyl-1-[3-(2-chlorobenzoylamino)-pyridin-4-ylméthyl]-3-(4-trifluorométhoxy-phényl)-imidazolidine-2,4-dione
- la 5-méthyl-1-[3-(4-fluorobenzoylamino)-pyridin-4-ylméthyl]-3-(4-trifluorométhoxy-phényl)-imidazolidine-2,4-dione
- la 5-méthyl-1-[3-(4-méthoxybenzoylamino)-pyridin-4-ylméthyl]-3-(4-trifluorométhoxy-phényl)-imidazolidine-2,4-dione
- la 5-méthyl-1-(3-acétamido-pyridin-4-ylméthyl)-3-(4-trifluorométhoxy-phényl)-imidazolidine-2,4-dione
- la 5-méthyl-1-(3-phénylacétamido-pyridin-4-ylméthyl)-3-(4-trifluorométhoxy-phényl)-imidazolidine-2,4-dione
- la 5-méthyl-1-[3-(4-mé-thylpipérazino)acétamido-pyridin-4-ylméthyl]-3-(4-trifluorométhoxy-phényl)-imidazolidine-2,4-dione
- la 5-méthyl-1-(3-cyclohéxanylcarboxamido-pyridin-4-ylméthyl)-3-(4-trifluorométhoxy-phényl)-imidazolidine-2,4-dione
- la 5-méthyl-1-[3-(1-méthyl-4-pipéridinyl)carbonylamino-pyridin-4-ylméthyl]-3-(4-trifluorométhoxy-phényl)-imidazolidine-2,4-dione
- la 5-méthyl-1-[3-(2-furanylcarbonyl)amino-pyridin-4-ylméthyl]-3-(4-trifluorométhoxy-phényl)-imidazolidine-2,4-dione
- la 5-méthyl-1-(3-éthoxycarbonylamino-pyridin-4-ylméthyl)-3-(4-trifluorométhoxy-phényl)-imidazolidine-2,4-dione
- la 5-méthyl-1-(3-isopropoxycarbonylamino-pyridin-4-ylméthyl)-3-(4-trifluorométhoxy-phényl)-imidazolidine-2,4-dione
- la 5-méthyl-1-(3-butoxycarbonylamino-pyridin-4-ylméthyl)-3-(4-trifluorométhoxy-phényl)-imidazolidine-2,4-dione
- la 5-méthyl-1-[3-(2-diéthylaminoéthoxycarbonylamino)-pyridin-4-ylméthyl]-3-(4-trifluorométhoxy-phényl)-imidazolidine-2,4-dione
- la 5-méthyl-1-(3-phénoxycarbonylamino-pyridin-4-ylméthyl)-3-(4-trifluorométhoxy-phényl)-imidazolidine-2,4-dione
- la 5-méthyl-1-[3-(4-chloroanilino)carbonylamino-pyridin-4-ylméthyl]-3-(4-trifluorométhoxy-phényl)-imidazolidine-2,4-dione
- la 5-méthyl-1-(3-anilinocarbonylamino-pyridin-4-ylméthyl)-3-(4-trifluorométhoxy-phényl)-imidazolidine-2,4-dione
- la 5-méthyl-1-(3-benzylaminocarbonylamino-pyridin-4-ylméthyl)-3-(4-trifluorométhoxy-phényl)-imidazolidine-2,4-dione
- la 5-méthyl-1-[3-(2-pyridylamino)carbonylamino-pyridin-4-ylméthyl]-3-(4-trifluorométhoxy-phényl)-imidazolidine-2,4-dione
- la 5-méthyl-1-(3-uréido-pyridin-4-ylméthyl)-3-(4-trifluorométhoxy-phényl)-imidazolidine-2,4-dione
- la 5-méthyl-1-(3-benzylaminosulfonylamino-pyridin-4-ylméthyl)-3-(4-trifluorométhoxy-phényl)-imidazolidine-2,4-dione
- la 5-méthyl-1-(3-méthylaminosulfonylamino-pyridin-4-ylméthyl)-3-(4-trifluorométhoxy-phényl)-imidazolidine-2,4-dione
- la 5-méthyl-1-(3-aminosulfonylamino-pyridin-4-ylméthyl)-3-(4-trifluorométhoxy-phényl)-imidazolidine-2,4-dione
- la 5-méthyl-1-(3-morpholinosulfonylamino-pyridin-4-ylméthyl)-3-(4-trifluorométhoxy-phényl)-imidazolidine-2,4-dione
- la 5-méthyl-1-(3-diméthylaminosulfonylamino-pyridin-4-ylméthyl)-3-(4-trifluorométhoxy-phényl)-imidazolidine-2,4-dione
- la 5-méthyl-1-(3-anilinosulfonylamino-pyridin-4-ylméthyl)-3-(4-trifluorométhoxy-phényl)-imidazolidine-2,4-dione
- la 5-méthyl-1-(3-phénylsulfonylamino-pyridin-4-ylméthyl)-3-(4-trifluorométhoxy-phényl)-imidazolidine-2,4-dione
- la 5-méthyl-1-[3-(2-thiényl)sulfonylamino-pyridin-4-ylméthyl]-3-(4-trifluorométhoxy-phényl)-imidazolidine-2,4-dione
- la 5-méthyl-1-(3-méthylsulfonylamino-pyridin-4-ylméthyl)-3-(4-trifluorométhoxy-phényl)-imidazolidine-2,4-dione
- la 5-méthyl-1-[3-(4-chlorophényl)sulfonylamino-pyridin-4-ylméthyl]-3-(4-trifluorométhoxy-phényl)-imidazolidine-2,4-dione
- la 5-méthyl-1-(3-benzylsulfonylamino-pyridin-4-ylméthyl)-3-(4-trifluorométhoxy-phényl)-imidazolidine-2,4-dione
- la 5-méthyl-1-(3-hydroxycarbonyl-pyridin-4-ylméthyl)-3-(4-trifluorométhoxy-phényl)-imidazolidine-2,4-dione
- la 5-méthyl-1-(3-éthoxycarbonyl-pyridin-4-ylméthyl)-3-(4-trifluorométhoxy-phényl)-imidazolidine-2,4-dione
- la 5-méthyl-1-(3-ter-butoxycarbonyl-pyridin-4-ylméthyl)-3-(4-trifluorométhoxy-phényl)-imidazolidine-2,4-dione
- la 5-méthyl-1-(3-phénoxycarbonyl-pyridin-4-ylméthyl)-3-(4-trifluorométhoxy-phényl)-imidazolidine-2,4-dione
- la 5-méthyl-1-[3-(4-méthylpipérazinocarbonyl)-pyridin-4-ylméthyl]-3-(4-trifluorométhoxy-phényl)-imidazolidine-2,4-dione
- la 5-méthyl-1-[3-(méthylamino)carbonyl-pyridin-4-ylméthyl]-3-(4-trifluorométhoxy-phényl)-imidazolidine-2,4-dione
- la 5-méthyl-1-[3-(diméthylamino)carbonyl-pyridin-4-ylméthyl]-3-(4-trifluorométhoxy-phényl)-imidazolidine-2,4-dione
- la 5-méthyl-1-(3-pipéridinocarbonyl-pyridin-4-ylméthyl)-3-(4-trifluorométhoxy-phényl)-imidazolidine-2,4-dione
- la 5-méthyl-1-(3-morpholinocarbonyl-pyridin-4-ylméthyl)-3-(4-trifluorométhoxy-phényl)-imidazolidine-2,4-dione
- la 5-méthyl-1-[3-(4-phénylpipérazinocarbonyl)-pyridin-4-ylméthyl]-3-(4-trifluorométhoxy-phényl)-imidazolidine-2,4-dione
- la 5-méthyl-1-(3-phénylaminocarbonyl-pyridin-4-ylméthyl)-3-(4-trifluorométhoxy-phényl)-imidazolidine-2,4-dione
- la 5-méthyl-1-[3-(méthylphénylamino)carbonyl-pyridin-4-ylméthyl]-3-(4-trifluorométhoxy-phényl)-imidazolidine-2,4-dione
- la 5-méthyl-1-[3-(4-méthoxyphényl)méthylaminocarbonyl-pyridin-4-ylméthyl]-3-(4-trifluorométhoxy-phényl)-imidazolidine-2,4-dione
- la 5-méthyl-1-[3-(4-chlorophényl)méthylaminocarbonyl-pyridin-4-ylméthyl]-3-(4-trifluorométhoxy-phényl)-imidazolidine-2,4-dione
- la 5-méthyl-1-[3-(4-morpholinylamino)carbonyl-pyridin-4-ylméthyl]-3-(4-trifluorométhoxy-phényl)-imidazolidine-2,4-dione
- la 5-méthyl-1-(3-aminocarbonyl-pyridin-4-ylméthyl)-3-(4-trifluorométhoxy-phényl)-imidazolidine-2,4-dione
- la 5-méthyl-1-(3-phénoxy-pyridin-4-ylméthyl)-3-(4-trifluorométhoxy-phényl)-imidazolidine-2,4-dione
- la 5-méthyl-1-(3-méthoxy-pyridin-4-ylméthyl)-3-(4-trifluorométhoxy-phényl)-imidazolidine-2,4-dione
- la 5-méthyl-1-(3-éthoxy-pyridin-4-ylméthyl)-3-(4-trifluorométhoxy-phényl)-imidazolidine-2,4-dione
- la 5-méthyl-1-[3-(1-pyrrolidinyl)éthoxy-pyridin-4-ylméthyl]-3-(4-trifluorométhoxy-phényl)-imidazolidine-2,4-dione
- la 5-méthyl-1-(3-benzyloxy-pyridin-4-ylméthyl)-3-(4-trifluorométhoxy-phényl)-imidazolidine-2,4-dione
- la 5-méthyl-1-(3-benzoyloxy-pyridin-4-ylméthyl)-3-(4-trifluorométhoxy-phényl)-imidazolidine-2,4-dione
- la 5-méthyl-1-[3-(4,5-dihydro-1H-imidazol-2-yl)-pyridin-4-ylméthyl]-3-(4-trifluorométhoxy-phényl)-imidazolidine-2,4-dione
- la 5-méthyl-1-[3-(1H-imidazol-2-yl)-pyridin-4-ylméthyl]-3-(4-trifluorométhoxy-phényl)-imidazolidine-2,4-dione
- la 5-méthyl-1-[3-(1H-tétrazol-5-yl)-pyridin-4-ylméthyl]-3-(4-trifluorométhoxy-phényl)-imidazolidine-2,4-dione
- la 5-méthyl-1-(3-amidino-pyridin-4-ylméthyl)-3-(4-trifluorométhoxy-phényl)-imidazolidine-2,4-dione
- la 5-méthyl-1-(3-diméthylaminométhyl-pyridin-4-ylméthyl)-3-(4-trifluorométhoxy-phényl)-imidazolidine-2,4-dione
- la 5-méthyl-1-(3-aminométhyl-pyridin-4-ylméthyl)-3-(4-trifluorométhoxy-phényl)-imidazolidine-2,4-dione
- la 1-(2-méthyl-pyridin-4-ylméthyl)-3-(4-trifluorométhoxy-phényl)-imidazolidine-2,4-dione
- la 1-(2-éthyl-pyridin-4-ylméthyl)-3-(4-trifluorométhoxy-phényl)-imidazolidine-2,4-dione
- la 1-(2-isopropyl-pyridin-4-ylméthyl)-3-(4-trifluorométhoxy-phényl)-imidazolidine-2,4-dione
- la 1-(2-ter-butyl-pyridin-4-ylméthyl)-3-(4-trifluorométhoxy-phényl)-imidazolidine-2,4-dione
- la 1-(2-isobutyl-pyridin-4-ylméthyl)-3-(4-trifluorométhoxy-phényl)-imidazolidine-2,4-dione
- la 1-(2-cyclohéxyl-pyridin-4-ylméthyl)-3-(4-trifluorométhoxy-phényl)-imidazolidine-2,4-dione
- la 1-(2-phényl-pyridin-4-ylméthyl)-3-(4-trifluorométhoxy-phényl)-imidazolidine-2,4-dione
- la 1-[2-(4-chlorophényl)-pyridin-4-ylméthyl]-3-(4-trifluorométhoxy-phényl)-imidazolidine-2,4-dione
- la 1-[2-(3-chlorophényl)-pyridin-4-ylméthyl]-3-(4-trifluorométhoxy-phényl)-imidazolidine-2,4-dione
- la 1-[2-(2-chlorophényl)-pyridin-4-ylméthyl]-3-(4-trifluorométhoxy-phényl)-imidazolidine-2,4-dione
- la 1-[2-(4-fluorophényl)-pyridin-4-ylméthyl]-3-(4-trifluorométhoxy-phényl)-imidazolidine-2,4-dione
- la 1-[2-(4-méthoxyphényl)-pyridin-4-ylméthyl]-3-(4-trifluorométhoxy-phényl)-imidazolidine-2,4-dione
- la 1-(2-benzyl-pyridin-4-ylméthyl)-3-(4-trifluorométhoxy-phényl)-imidazolidine-2,4-dione
- la 1-(2,2'bipyridin-4-ylméthyl)-3-(4-trifluorométhoxy-phényl)-imidazolidine-2,4-dione
- la 1-(2-hydroxyméthyl-pyridin-4-ylméthyl)-3-(4-trifluorométhoxy-phényl)-imidazolidine-2,4-dione
- la 1-(2-chloro-pyridin-4-ylméthyl)-3-(4-trifluorométhoxy-phényl)-imidazolidine-2,4-dione
- la 1-(2-fluoro-pyridin-4-ylméthyl)-3-(4-trifluorométhoxy-phényl)-imidazolidine-2,4-dione
- la 1-(2-bromo-pyridin-4-ylméthyl)-3-(4-trifluorométhoxy-phényl)-imidazolidine-2,4-dione
- la 1-(2-amino-pyridin-4-ylméthyl)-3-(4-trifluorométhoxy-phényl)-imidazolidine-2,4-dione
- la 1-(2-méthylamino-pyridin-4-ylméthyl)-3-(4-trifluorométhoxy-phényl)-imidazolidine-2,4-dione
- la 1-(2-éthylamino-pyridin-4-ylméthyl)-3-(4-trifluorométhoxy-phényl)-imidazolidine-2,4-dione
- la 1-(2-isopropylamino-pyridin-4-ylméthyl)-3-(4-trifluorométhoxy-phényl)-imidazolidine-2,4-dione
- la 1-(2-phénylamino-pyridin-4-ylméthyl)-3-(4-trifluorométhoxy-phényl)-imidazolidine-2,4-dione
- la 1-(2-cyclohéxylamino-pyridin-4-ylméthyl)-3-(4-trifluorométhoxy-phényl)-imidazolidine-2,4-dione
- la 1-(2-diéthylamino-pyridin-4-ylméthyl)-3-(4-trifluorométhoxy-phényl)-imidazolidine-2,4-dione
- la 1-(2-N-cyclohéxyl-N-méthylamino-pyridin-4-ylméthyl)-3-(4-trifluorométhoxy-phényl)-imidazolidine-2,4-dione
- la 1-(2-pipéridino-pyridin-4-ylméthyl)-3-(4-trifluorométhoxy-phényl)-imidazolidine-2,4-dione
- la 1-(2-morpholino-pyridin-4-ylméthyl)-3-(4-trifluorométhoxy-phényl)-imidazolidine-2,4-dione
- la 1-[2-(4-méthylpipérazino)-pyridin-4-ylméthyl]-3-(4-trifluorométhoxy-phényl)-imidazolidine-2,4-dione
- la 1-[2-(1H-pyrazol-1yl)-pyridin-4-ylméthyl]-3-(4-trifluorométhoxy-phényl)-imidazolidine-2,4-dione
- la 1-[2-(2-benzoylhydrazino)-pyridin-4-ylméthyl]-3-(4-trifluorométhoxy-phényl)-imidazolidine-2,4-dione
- la 1-(2-benzoylamino-pyridin-4-ylméthyl)-3-(4-trifluorométhoxy-phényl)-imidazolidine-2,4-dione
- la 1-[2-(4-chlorobenzoylamino)-pyridin-4-ylméthyl]-3-(4-trifluorométhoxy-phényl)-imidazolidine-2,4-dione
- la 1-[2-(3-chlorobenzoylamino)-pyridin-4-ylméthyl]-3-(4-trifluorométhoxy-phényl)-imidazolidine-2,4-dione
- la 1-[2-(2-chlorobenzoylamino)-pyridin-4-ylméthyl]-3-(4-trifluorométhoxy-phényl)-imidazolidine-2,4-dione
- la 1-[2-(4-fluorobenzoylamino)-pyridin-4-ylméthyl]-3-(4-trifluorométhoxy-phényl)-imidazolidine-2,4-dione
- la 1-[2-(4-méthoxybenzoylamino)-pyridin-4-ylméthyl]-3-(4-trifluorométhoxy-phényl)-imidazolidine-2,4-dione
- la 1-(2-acétamido-pyridin-4-ylméthyl)-3-(4-trifluorométhoxy-phényl)-imidazolidine-2,4-dione
- la 1-(2-phénylacétamido-pyridin-4-ylméthyl)-3-(4-trifluorométhoxy-phényl)-imidazolidine-2,4-dione
- la 1-[2-(4-méthylpipérazino)acétamido-pyridin-4-ylméthyl]-3-(4-trifluorométhoxy-phényl)-imidazolidine-2,4-dione
- la 1-(2-cyclohéxanylcarboxamido-pyridin-4-ylméthyl)-3-(4-trifluorométhoxy-phényl)-imidazolidine-2,4-dione
- la 1-[2-(1-méthyl-4-pipéridinyl)carbonylamino-pyridin-4-ylméthyl]-3-(4-trifluorométhoxy-phényl)-imidazolidine-2,4-dione
- la 1-[2-(2-furanylcarbonyl)amino-pyridin-4-ylméthyl]-3-(4-trifluorométhoxy-phényl)-imidazolidine-2,4-dione
- la 1-(2-éthoxycarbonylamino-pyridin-4-ylméthyl)-3-(4-trifluorométhoxy-phényl)-imidazolidine-2,4-dione
- la 1-(2-isopropoxycarbonylamino-pyridin-4-ylméthyl)-3-(4-trifluorométhoxy-phényl)-imidazolidine-2,4-dione
- la 1-(2-butoxycarbonylamino-pyridin-4-ylméthyl)-3-(4-trifluorométhoxy-phényl)-imidazolidine-2,4-dione
- la 1-[2-(2-diéthylaminoéthoxycarbonylamino)-pyridin-4-ylméthyl]-3-(4-trifluorométhoxy-phényl)-imidazolidine-2,4-dione
- la 1-(2-phénoxycarbonylamino-pyridin-4-ylméthyl)-3-(4-trifluorométhoxy-phényl)-imidazolidine-2,4-dione
- la 1-[2-(4-chloroanilino)carbonylamino-pyridin-4-ylméthyl]-3-(4-trifluorométhoxy-phényl)-imidazolidine-2,4-dione
- la 1-(2-anilinocarbonylamino-pyridin-4-ylméthyl)-3-(4-trifluorométhoxy-phényl)-imidazolidine-2,4-dione
- la 1-(2-benzylaminocarbonylamino-pyridin-4-ylméthyl)-3-(4-trifluorométhoxy-phényl)-imidazolidine-2,4-dione
- la 1-[2-(2-pyridylamino)carbonylamino-pyridin-4-ylméthyl]-3-(4-trifluorométhoxy-phényl)-imidazolidine-2,4-dione
- la 1-(2-uréido-pyridin-4-ylméthyl)-3-(4-trifluorométhoxy-phényl)-imidazolidine-2,4-dione
- la 1-(2-benzylaminosulfonylamino-pyridin-4-ylméthyl)-3-(4-trifluorométhoxy-phényl)-imidazolidine-2,4-dione
- la 1-(2-méthylaminosulfonylamino-pyridin-4-ylméthyl)-3-(4-trifluorométhoxy-phényl)-imidazolidine-2,4-dione
- la 1-(2-aminosulfonylamino-pyridin-4-ylméthyl)-3-(4-trifluorométhoxy-phényl)-imidazolidine-2,4-dione
- la 1-(2-morpholinosulfonylamino-pyridin-4-ylméthyl)-3-(4-trifluorométhoxy-phényl)-imidazolidine-2,4-dione
- la 1-(2-diméthylaminosulfonylamino-pyridin-4-ylméthyl)-3-(4-trifluorométhoxy-phényl)-imidazolidine-2,4-dione
- la 1-(2-anilinosulfonylamino-pyridin-4-ylméthyl)-3-(4-trifluorométhoxy-phényl)-imidazolidine-2,4-dione
- la 1-(2-phénylsulfonylamino-pyridin-4-ylméthyl)-3-(4-trifluorométhoxy-phényl)-imidazolidine-2,4-dione
- la 1-[2-(2-thiényl)sulfonylamino-pyridin-4-ylméthyl]-3-(4-trifluorométhoxy-phényl)-imidazolidine-2,4-dione
- la 1-(2-méthylsulfonylamino-pyridin-4-ylméthyl)-3-(4-trifluorométhoxy-phényl)-imidazolidine-2,4-dione
- la 1-[2-(4-chlorophényl)sulfonylamino-pyridin-4-ylméthyl]-3-(4-trifluorométhoxy-phényl)-imidazolidine-2,4-dione
- la 1-(2-benzylsulfonylamino-pyridin-4-ylméthyl)-3-(4-trifluorométhoxy-phényl)-imidazolidine-2,4-dione
- la 1-(2-hydroxycarbonyl-pyridin-4-ylméthyl)-3-(4-trifluorométhoxy-phényl)-imidazolidine-2,4-dione
- la 1-(2-éthoxycarbonyl-pyridin-4-ylméthyl)-3-(4-trifluorométhoxy-phényl)-imidazolidine-2,4-dione
- la 1-(2-ter-butoxycarbonyl-pyridin-4-ylméthyl)-3-(4-trifluorométhoxy-phényl)-imidazolidine-2,4-dione
- la 1-(2-phénoxycarbonyl-pyridin-4-ylméthyl)-3-(4-trifluorométhoxy-phényl)-imidazolidine-2,4-dione
- la 1-[2-(4-méthylpipérazinocarbonyl)-pyridin-4-ylméthyl]-3-(4-trifluorométhoxy-phényl)-imidazolidine-2,4-dione
- la 1-[2-(méthylamino)carbonyl-pyridin-4-ylméthyl]-3-(4-trifluorométhoxy-phényl)-imidazolidine-2,4-dione
- la 1-[2-(diméthylamino)carbonyl-pyridin-4-ylméthyl]-3-(4-trifluorométhoxy-phényl)-imidazolidine-2,4-dione
- la 1-(2-pipéridinocarbonyl-pyridin-4-ylméthyl)-3-(4-trifluorométhoxy-phényl)-imidazolidine-2,4-dione
- la 1-(2-morpholinocarbonyl-pyridin-4-ylméthyl)-3-(4-trifluorométhoxy-phényl)-imidazolidine-2,4-dione
- la 1-[2-(4-phénylpipérazinocarbonyl)-pyridin-4-ylméthyl]-3-(4-trilluorométhoxy-phényl)-imidazolidine-2,4-dione
- la 1-(2-phénylaminocarbonyl-pyridin-4-ylméthyl)-3-(4-trifluorométhoxy-phényl)-imidazolidine-2,4-dione
- la 1-[2-(méthylphénylamino)carbonyl-pyridin-4-ylméthyl]-3-(4-trifluorométhoxy-phényl)-imidazolidine-2,4-dione
- la 1-[2-(4-méthoxyphényl)méthylaminocarbonyl-pyridin-4-ylméthyl]-3-(4-trifluorométhoxy-phényl)-imidazolidine-2,4-dione
- la 1-[2-(4-chlorophényl)méthylaminocarbonyl-pyridin-4-ylméthyl]-3-(4-trifluorométhoxy-phényl)-imidazolidine-2,4-dione
- la 1-[2-(4-morpholinylamino)carbonyl-pyridin-4-ylméthyl]-3-(4-trifluorométhoxy-phényl)-imidazolidine-2,4-dione
- la 1-(2-aminocarbonyl-pyridin-4-ylméthyl)-3-(4-trifluorométhoxy-phényl)-imidazolidine-2,4-dione
- la 1-(2-phénoxy-pyridin-4-ylméthyl)-3-(4-trifluorométhoxy-phényl)-imidazolidine-2,4-dione
- la 1-(2-méthoxy-pyridin-4-ylméthyl)-3-(4-triflùorométhoxy-phényl)-imidazolidine-2,4-dione
- la 1-(2-éthoxy-pyridin-4-ylméthyl)-3-(4-trifluorométhoxy-phényl)-imidazolidine-2,4-dione
- la 1-[2-(1-pyrrolidinyl)éthoxy-pyridin-4-ylméthyl]-3-(4-trifluorométhoxy-phényl)-imidazolidine-2,4-dione
- la 1-(2-benzyloxy-pyridin-4-ylméthyl)-3-(4-trifluorométhoxy-phényl)-imidazolidine-2,4-dione
- la 1-(2-benzoyloxy-pyridin-4-ylméthyl)-3-(4-trifluorométhoxy-phényl)-imidazolidine-2,4-dione
- la 1-[2-(4,5-dihydro-1H-imidazol-2-yl)-pyridin-4-ylméthyl]-3-(4-trifluorométhoxy-phényl)-imidazolidine-2,4-dione
- la 1-[2-(1H-imidazol-2-yl)-pyridin-4-ylméthyl]-3-(4-trifluorométhoxy-phényl)-imidazolidine-2,4-dione
- la 1-[2-(1H-tétrazol-5-yl)-pyridin-4-ylméthyl]-3-(4-trifluorométhoxy-phényl)-imidazolidine-2,4-dione
- la 1-(2-amidino-pyridin-4-ylméthyl)-3-(4-trifluorométhoxy-phényl)-imidazolidine-2,4-dione
- la 1-(2-diméthylaminométhyl-pyridin-4-ylméthyl)-3-(4-trifluorométhoxy-phényl)-imidazolidine-2,4-dione
- la 1-(2-aminométhyl-pyridin-4-ylméthyl)-3-(4-trifluorométhoxy-phényl)-imidazolidine-2,4-dione
- la 1-(3-méthyl-pyridin-4-ylméthyl)-3-(4-trifluorométhoxy-phényl)-imidazolidine-2,4-dione
- la 1-(3-éthyl-pyridin-4-ylméthyl)-3-(4-trifluorométhoxy-phényl)-imidazolidine-2,4-dione
- la 1-(3-isopropyl-pyridin-4-ylméthyl)-3-(4-trifluorométhoxy-phényl)-imidazolidine-2,4-dione
- la 1-(3-ter-butyl-pyridin-4-ylméthyl)-3-(4-trifluorométhoxy-phényl)-imidazolidine-2,4-dione
- la 1-(3-isobutyl-pyridin-4-ylméthyl)-3-(4-trifluorométhoxy-phényl)-imidazolidine-2,4-dione
- la 1-(3-cyclohéxyl-pyridin-4-ylméthyl)-3-(4-trifluorométhoxy-phényl)-imidazolidine-2,4-dione
- la 1-(3-phényl-pyridin-4-ylméthyl)-3-(4-trifluorométhoxy-phényl)-imidazolidine-2,4-dione
- la 1-[3-(4-chlorophényl)-pyridin-4-ylméthyl]-3-(4-trifluorométhoxy-phényl)-imidazolidine-2,4-dione
- la 1-[3-(3-chlorophényl)-pyridin-4-ylméthyl]-3-(4-trifluorométhoxy-phényl)-imidazolidine-2,4-dione
- la 1-[3-(2-chlorophényl)-pyridin-4-ylméthyl]-3-(4-trifluorométhoxy-phényl)-imidazolidine-2,4-dione
- la 1-[3-(4-fluorophényl)-pyridin-4-ylméthyl]-3-(4-trifluorométhoxy-phényl)-imidazolidine-2,4-dione
- la 1-[3-(4-méthoxyphényl)-pyridin-4-ylméthyl]-3-(4-trifluorométhoxy-phényl)-imidazolidine-2,4-dione
- la 1-(3-benzyl-pyridin-4-ylméthyl)-3-(4-trifluorométhoxy-phényl)-imidazolidine-2,4-dione 1-(3,2'bipyridin-4-ylméthyl)-3-(4-trifluoiométhoxy-phényl)-imidazolidine-2,4-dione
- la 1-(3-hydroxyméthyl-pyridin-4-ylméthyl)-3-(4-trifluorométhoxy-phényl)-imidazolidine-2,4-dione
- la 1-(3-chloro-pyridin-4-ylméthyl)-3-(4-trifluorométhoxy-phényl)-imidazolidine-2,4-dione
- la 1-(3-fluoro-pyridin-4-ylméthyl)-3-(4-trifluorométhoxy-phényl)-imidazolidine-2,4-dione
- la 1-(3-bromo-pyridin-4-ylméthyl)-3-(4-trifluorométhoxy-phényl)-imidazolidine-2,4-dione
- la 1-(3-amino-pyridin-4-ylméthyl)-3-(4-trifluorométhoxy-phényl)-imidazolidine-2,4-dione
- la 1-(3-méthylamino-pyridin-4-ylméthyl)-3-(4-trifluorométhoxy-phényl)-imidazolidine-2,4-dione
- la 1-(3-éthylamino-pyridin-4-ylméthyl)-3-(4-trifluorométhoxy-phényl)-imidazolidine-2,4-dione
- la 1-(3-isopropylamino-pyridin-4-ylméthyl)-3-(4-trifluorométhoxy-phényl)-imidazolidine-2,4-dione
- la 1-(3-phénylamino-pyridin-4-ylméthyl)-3-(4-trifluorométhoxy-phényl)-imidazolidine-2,4-dione
- la 1-(3-cyclohéxylamino-pyridin-4-ylméthyl)-3-(4-trifluorométhoxy-phényl)-imidazolidine-2,4-dione
- la 1-(3-diéthylamino-pyridin-4-ylméthyl)-3-(4-trifluorométhoxy-phényl)-imidazolidine-2,4-dione
- la 1-(3-N-cyclohéxyl-N-méthylamino-pyridin-4-ylméthyl)-3-(4-trifluorométhoxy-phényl)-imidazolidine-2,4-dione
- la 1-(3-pipéridino-pyridin-4-ylméthyl)-3-(4-trifluorométhoxy-phényl)-imidazolidine-2,4-dione
- la 1-(3-morpholino-pyridin-4-ylméthyl)-3-(4-trifluorométhoxy-phényl)-imidazolidine-2,4-dione
- la 1-[3-(4-méthylpipérazino)-pyridin-4-ylméthyl]-3-(4-trifluorométhoxy-phényl)-imidazolidine-2,4-dione
- la 1- [3-(1H-pyrazol-1yl) -pyridin-4-ylméthyl] -3- (4-trifluorométhoxy-phényl)-imidazolidine-2,4-dione
- la 1-[3-(2-benzoylhydrazino)-pyridin-4-ylméthyl]-3-(4-trifluorométhoxy-phényl)-imidazolidine-2,4-dione
- la 1-(3-benzoylamino-pyridin-4-ylméthyl)-3-(4-trifluorométhoxy-phényl)-imidazolidine-2,4-dione
- la 1-[3-(4-chlorobenzoylamino)-pyridin-4-ylméthyl]-3-(4-trifluorométhoxy-phényl)-imidazolidine-2,4-dione
- la 1-[3-(3-chlorobenzoylamino)-pyridin-4-ylméthyl]-3-(4-trifluorométhoxy-phényl)-imidazolidine-2,4-dione
- la 1-[3-(2-chlorobenzoylamino)-pyridin-4-ylméthyl]-3-(4-trifluorométhoxy-phényl)-imidazolidine-2,4-dione
- la 1-[3-(4-fluorobenzoylamino)-pyridin-4-ylméthyl]-3-(4-trifluorométhoxy-phényl)-imidazolidine-2,4-dione
- la 1-[3-(4-méthoxybenzoylamino)-pyridin-4-ylméthyl]-3-(4-trifluorométhoxy-phényl)-imidazolidine-2,4-dione
- la 1-(3-acétamido-pyridin-4-ylméthyl)-3-(4-trifluorométhoxy-phényl)-imidazolidine-2,4-dione
- la 1-(3-phénylacétamido-pyridin-4-ylméthyl)-3-(4-trifluorométhoxy-phényl)-imidazolidine-2,4-dione
- la 1-[3-(4-méthylpipérazino)acétamido-pyridin-4-ylméthyl]-3-(4-trifluorométhoxy-phényl)-imidazolidine-2,4-dione
- la 1-(3-cyclohéxanylcarboxamido-pyridin-4-ylméthyl)-3-(4-trifluorométhoxy-phényl)-imidazolidine-2,4-dione
- la 1-[3-(1-méthyl-4-pipéridinyl)carbonylamino-pyridin-4-ylméthyl]-3-(4-trifluorométhoxy-phényl)-imidazolidine-2,4-dione
- la 1-[3-(2-furanylcarbonyl)amino-pyridin-4-ylméthyl]-3-(4-trifluorométhoxy-phényl)-imidazolidine-2,4-dione
- la 1-(3-éthoxycarbonylamino-pyridin-4-ylméthyl)-3-(4-trifluorométhoxy-phényl)-imidazolidine-2,4-dione
- la 1-(3-isopropoxycarbonylamino-pyridin-4-ylméthyl)-3-(4-trifluorométhoxy-phényl)-imidazolidine-2,4-dione
- la 1-(3-butoxycarbonylamino-pyridin-4-ylméthyl)-3-(4-trifluorométhoxy-phényl)-imidazolidine-2,4-dione
- la 1-[3-(2-diéthylaminoéthoxycarbonylamino)-pyridin-4-ylméthyl]-3-(4-trifluorométhoxy-phényl)-imidazolidine-2,4-dione
- la 1-(3-phénoxycarbonylamino-pyridin-4-ylméthyl)-3-(4-trifluorométhoxy-phényl)-imidazolidine-2,4-dione
- la 1-[3-(4-chloroanilino)carbonylamino-pyridin-4-ylméthyl]-3-(4-trifluorométhoxy-phényl)-imidazolidine-2,4-dione
- la 1-(3-anilinocarbonylamino-pyridin-4-ylméthyl)-3-(4-trifluorométhoxy-phényl)-imidazolidine-2,4-dione
- la 1-(3-benzylaminocarbonylamino-pyridin-4-ylméthyl)-3-(4-trifluorométhoxy-phényl)-imidazolidine-2,4-dione
- la 1-[3-(2-pyridylamino)carbonylamino-pyridin-4-ylméthyl]-3-(4-trifluorométhoxy-phényl)-imidazolidine-2,4-dione

- la 1-(3-uréido-pyridin-4-ylméthyl)-3-(4-trifluorométhoxy-phényl)-imidazolidine-2,4-dione
- la 1-(3-benzylaminosulfonylamino-pyridin-4-ylméthyl)-3-(4-trifluorométhoxy-phényl)-imidazolidine-2,4-dione
- la 1-(3-méthylaminosulfonylamino-pyridin-4-ylméthyl)-3-(4-trifluorométhoxy-phényl)-imidazolidine-2,4-dione
- la 1-(3-aminosulfonylamino-pyridin-4-ylméthyl)-3-(4-trifluorométhoxy-phényl)-imidazolidine-2,4-dione
- la 1-(3-morpholinosulfonylamino-pyridin-4-ylméthyl)-3-(4-trifluorométhoxy-phényl)-imidazolidine-2,4-dione
- la 1-(3-diméthylaminosulfonylamino-pyridin-4-ylméthyl)-3-(4-trifluorométhoxy-phényl)-imidazolidine-2,4-dione
- la 1-(3-anilinosulfonylamino-pyridin-4-ylméthyl)-3-(4-trifluorométhoxy-phényl)-imidazolidine-2,4-dione
- la 1-(3-phénylsulfonylamino-pyridin-4-ylméthyl)-3-(4-trifluorométhoxy-phényl)-imidazolidine-2,4-dione
- la 1-[3-(2-thiényl)sulfonylamino-pyridin-4-ylméthyl]-3-(4-trifluorométhoxy-phényl)-imidazolidine-2,4-dione
- la 1-(3-méthylsulfonylamino-pyridin-4-ylméthyl)-3-(4-trifluorométhoxy-phényl)-imidazolidine-2,4-dione
- la 1-[3-(4-chlorophényl)sulfonylamino-pyridin-4-ylméthyl]-3-(4-trifluorométhoxy-phényl)-imidazolidine-2,4-dione
- la 1-(3-benzylsulfonylamino-pyridin-4-ylméthyl)-3-(4-trifluorométhoxy-phényl)-imidazolidine-2,4-dione
- la 1-(3-hydroxycarbonyl-pyridin-4-ylméthyl)-3-(4-trifluorométhoxy-phényl)-imidazolidine-2,4-dione
- la 1-(3-éthoxycarbonyl-pyridin-4-ylméthyl)-3-(4-trifluorométhoxy-phényl)-imidazolidine-2,4-dione
- la 1-(3-ter-butoxycarbonyl-pyridin-4-ylméthyl)-3-(4-trifluorométhoxy-phényl)-imidazolidine-2,4-dione
- la 1-(3-phénoxycarbonyl-pyridin-4-ylméthyl)-3-(4-trifluorométhoxy-phényl)-imidazolidine-2,4-dione
- la 1-[3-(4-méthylpipérazinocarbonyl)-pyridin-4-ylméthyl]-3-(4-trifluorométhoxy-phényl)-imidazolidine-2,4-dione
- la 1-[3-(méthylamino)carbonyl-pyridin-4-ylméthyl]-3-(4-trifluorométhoxy-phényl)-imidazolidine-2,4-dione
- la 1-[3-(diméthylamino)carbonyl-pyridin-4-ylméthyl]-3-(4-trifluorométhoxy-phényl)-imidazolidine-2,4-dione
- la 1-(3-pipéridinocarbonyl-pyridin-4-ylméthyl)-3-(4-trifluorométhoxy-phényl)-imidazolidine-2,4-dione
- la 1-(3-morpholinocarbonyl-pyridin-4-ylméthyl)-3-(4-trifluorométhoxy-phényl)-imidazolidine-2,4-dione
- la 1-[3-(4-phénylpipérazinocarbonyl)-pyridin-4-ylméthyl]-3-(4-trifluorométhoxy-phényl)-imidazolidine-2,4-dione
- la 1-(3-phénylaminocarbonyl-pyridin-4-ylméthyl)-3-(4-trifluorométhoxy-phényl)-imidazolidine-2,4-dione
- la 1-[3-(méthylphénylamino)carbonyl-pyridin-4-ylméthyl]-3-(4-trifluorométhoxy-phényl)-imidazolidine-2,4-dione
- la 1-[3-(4-méthoxyphényl)méthylaminocarbonyl-pyridin-4-ylméthyl]-3-(4-trifluorométhoxy-phényl)-imidazolidine-2,4-dione
- la 1-[3-(4-chlorophényl)méthylaminocarbonyl-pyridin-4-ylméthyl]-3-(4-trifluorométhoxy-phényl)-imidazolidine-2,4-dione
- la 1-[3-(4-morpholinylamino)carbonyl-pyridin-4-ylméthyl]-3-(4-trifluorométhoxy-phényl)-imidazolidine-2,4-dione
- la 1-(3-aminocarbony-1-pyridin-4-ylméthyl)-3-(4-trifluorométhoxy-phényl)-imidazolidine-2,4-dione
- la 1-(3-phénoxy-pyridin-4-ylméthyl)-3-(4-trifluorométhoxy-phényl)-imidazolidine-2,4-dione
- la 1-(3-méthoxy-pyridin-4-ylméthyl)-3-(4-trifluorométhoxy-phényl)-imidazolidine-2,4-dione
- la 1-(3-éthoxy-pyridin-4-ylméthyl)-3-(4-trifluorométhoxy-phényl)-imidazolidine-2,4-dione
- la 1-[3-(1-pyrrolidinyl)éthoxy-pyridin-4-ylméthyl]-3-(4-trifluorométhoxy-phényl)-imidazolidine-2,4-dione
- la 1-(3-benzyloxy-pyridin-4-ylméthyl)-3-(4-trifluorométhoxy-phényl)-imidazolidine-2,4-dione
- la 1-(3-benzoyloxy-pyridin-4-ylméthyl)-3-(4-trifluorométhoxy-phényl)-imidazolidine-2,4-dione
- la 1-[3-(4,5-dihydro-1H-imidazol-2-yl)-pyridin-4-ylméthyl]-3-(4-trifluorométhoxy-phényl)-imidazolidine-2,4-dione
- la 1-[3-(1H-imidazol-2-yl)-pyridin-4-ylméthyl]-3-(4-trifluorométhoxy-phényl)-imidazolidine-2,4-dione
- la 1-[3-(1H-tétrazol-5-yl)-pyridin-4-ylméthyl]-3-(4-trifluorométhoxy-phényl)-imidazolidine-2,4-dione
- la 1-(3-amidino-pyridin-4-ylméthyl)-3-(4-trifluorométhoxy-phényl)-imidazolidine-2,4-dione
- la 1-(3-diméthylaminométhyl-pyridin)-4-ylméthyl]-3-(4-trifluorométhoxy-phényl)-imidazolidine-2,4-dione
- la 1-(3-aminométhyl-pyridin-4-ylméthyl)-3-(4-trifluorométhoxy-phényl)-imidazolidine-2,4-dione
- la 5,5-diméthyl-1-(2-méthyl-pyridin-4-ylméthyl)-3-(4-trifluorométhanesulfanyl-phényl)-imidazolidine-2,4-dioné
- la 5,5-diméthyl-1-(2-éthyl-pyridin-4-ylméthyl)-3-(4-trifluorométhanesulfanyl-phényl)-imidazolidine-2,4-dione
- la 5,5-diméthyl-1-(2-isopropyl-pyridin-4-ylméthyl)-3-(4-trifluorométhanesulfanyl-phényl)-imidazolidine-2,4-dione
- la 5,5-diméthyl-1-(2-ter-butyl-pyridin-4-ylméthyl)-3-(4-trifluorométhanesulfanyl-phényl)-imidazolidine-2,4-dione
- la 5,5-diméthyl-1-(2-isobutyl-pyridin-4-ylméthyl)-3-(4-trifluorométhanesulfanyl-phényl)-imidazolidine-2,4-dione
- la 5,5-diméthyl-1-(2-cyclohéxyl-pyridin-4-ylméthyl)-3-(4-trifluorométhanesulfanyl-phényl)-imidazolidine-2,4-dione
- la 5,5-diméthyl-1-(2-phényl-pyridin-4-ylméthyl)-3-(4-trifluorométhanesulfanyl-phényl)-imidazolidine-2,4-dione
- la 5,5-diméthyl-1-[2-(4-chlorophényl)-pyridin-4-ylméthyl]-3-(4-trifluorométhanesulfanyl-phényl)-imidazolidine-2,4-dione
- la 5,5-diméthyl-1-[2-(3-chlorophényl)-pyridin-4-ylméthyl]-3-(4-trifluorométhanesulfanyl-phényl)-imidazolidine-2,4-dione
- la 5,5-diméthyl-1-[2-(2-chlorophényl)-pyridin-4-ylméthyl]-3-(4-trifluorométhanesulfanyl-phényl)-imidazolidine-2,4-dione
- la 5,5-diméthyl-1-[2-(4-fluorophényl)-pyridin-4-ylméthyl]-3-(4-trifluorométhanesulfanyl-phényl)-imidazolidine-2,4-dione
- la 5,5-diméthyl-1-[2-(4-méthoxyphényl)-pyridin-4-ylméthyl]-3-(4-trifluorométhanesulfanyl-phényl)-imidazolidine-2,4-dione
- la 5,5-diméthyl-1-(2-benzyl-pyridin-4-ylméthyl)-3-(4-trifluorométhanesulfanyl-phényl)-imidazolidine-2,4-dione
- la 5,5-dimé-1-(2,2'bipyridin-4-ylméthyl)-3-(4-trifluorométhanesulfanyl-phényl)-imidazolidine-2,4-dione
- la 5,5-diméthyl-1-(2-hydroxyméthyl-pyridin-4-ylméthyl)-3-(4-trifluorométhanesulfanyl-phényl)-imidazolidine-2,4-dione
- la 5,5-diméthyl-1-(2-chloro-pyridin-4-ylméthyl)-3-(4-trifluorométhanesulfanyl-phényl)-imidazolidine-2,4-dione
- la 5,5-diméthyl-1-(2-fluoro-pyridin-4-ylméthyl)-3-(4-trifluorométhanesulfanyl-phényl)-imidazolidine-2,4-dione
- la 5,5-diméthyl-1-(2-bromo-pyridin-4-ylméthyl)-3-(4-trifluorométhanesulfanyl-phényl)-imidazolidine-2,4-dione
- la 5,5-diméthyl-1-(2-amino-pyridin-4-ylméthyl)-3-(4-trifluorométhanesulfanyl-phényl)-imidazolidine-2,4-dione
- la 5,5-diméthyl-1-(2-méthylamino-pyridin-4-ylméthyl)-3-(4-trifluorométhanesulfanyl-phényl)-imidazolidine-2,4-dione
- la 5,5-diméthyl-1-(2-éthylamino-pyridin-4-ylméthyl)-3-(4-trifluorométhanesulfanyl-phényl)-imidazolidine-2,4-dione
- la 5,5-diméthyl-1-(2-isopropylamino-pyridin-4-ylméthyl)-3-(4-trifluorométhanesulfanyl-phényl)-imidazolidine-2,4-dione
- la 5,5-diméthyl-1-(2-phénylamino-pyridin-4-ylméthyl)-3-(4-trifluorométhanesulfanyl-phényl)-imidazolidine-2,4-dione
- la 5,5-diméthyl-1-(2-cyclohéxylamino-pyridin-4-ylméthyl)-3-(4-trifluorométhanesulfanyl-phényl)-imidazolidine-2,4-dione
- la 5,5-diméthyl-1-(2-diéthylamino-pyridin-4-ylméthyl)-3-(4-trifluorométhanesulfanyl-phényl)-imidazolidine-2,4-dione
- la 5,5-diméthyl-1-(2-N-cyclohéxyl-N-méthylamino-pyridin-4-ylméthyl)-3-(4-trifluorométhanesulfanyl-phényl)-imidazolidine-2,4-dione
- la 5,5-diméthyl-1-(2-pipéridino-pyridin-4-ylméthyl)-3-(4-trifluorométhanesulfanyl-phényl)-imidazolidine-2,4-dione
- la 5,5-diméthyl-1-(2-morpholino-pyridin-4-ylméthyl)-3-(4-trifluorométhanesulfanyl-phényl)-imidazolidine-2,4-dione
- la 5,5-diméthyl-1-[2-(4-méthylpipérazino)-pyridin-4-ylméthyl]-3-(4-trifluorométhanesulfanyl-phényl)-imidazolidine-2,4-dione
- la 5,5-diméthyl-1-[2-(1H-pyrazol-1yl)-pyridin-4-ylméthyl]-3-(4-trifluorométhanesulfanyl-phényl)-imidazolidine-2,4-dione
- la 5,5-diméthyl-1-[2-(2-benzoylhydrazino)-pyridin-4-ylméthyl]-3-(4-trifluorométhanesulfanyl-phényl)-imidazolidine-2,4-dione
- la 5,5-diméthyl-1-(2-benzoylamino-pyridin-4-ylméthyl)-3-(4-trifluorométhanesulfanyl-phényl)-imidazolidine-2,4-dione
- la 5,5-diméthyl-1-[2-(4-chlorobenzoylamino)-pyridin-4-ylméthyl]-3-(4-trifluorométhanesulfanyl-phényl)-imidazolidine-2,4-dione
- la 5,5-diméthyl-1-[2-(3-chlorobenzoylamino)-pyridin-4-ylméthyl]-3-(4-trifluorométhanesulfanyl-phényl)-imidazolidine-2,4-dione
- la 5,5-diméthyl-1-[2-(2-chlorobenzoylamino)-pyridin-4-ylméthyl]-3-(4-trifluorométhanesulfanyl-phényl)-imidazolidine-2,4-dione
- la 5,5-diméthyl-1-[2-(4-fluorobenzoylamino)-pyridin-4-ylméthyl]-3-(4-trifluorométhanesulfanyl-phényl)-imidazolidine-2,4-dione
- la 5,5-diméthyl-1-[2-(4-méthoxybenzoylamino)-pyridin-4-ylméthyl]-3-(4-trifluorométhanesulfanyl-phényl)-imidazolidine-2,4-dione
- la 5,5-diméthyl-1-(2-acétamido-pyridin-4-ylméthyl)-3-(4-trifluorométhanesulfanyl-phényl)-imidazolidine-2,4-dione
- la 5,5-diméthyl-1-(2-phénylacétamido-pyridin-4-ylméthyl)-3-(4-trifluorométhanesulfanyl-phényl)-imidazolidine-2,4-dione
- la 5,5-diméthyl-1-[2-(4-méthylpipérazino)acétamido-pyridin-4-ylméthyl]-3-(4-trifluorométhanésulfanyl-phényl)-imidazolidine-2,4-dione
- la 5,5-diméthyl-1-(2-cyclohéxanylcarboxamido-pyridin-4-ylméthyl)-3-(4-trifluorométhanesulfanyl-phényl)-imidazolidine-2,4-dione
- la 5,5-diméthyl-1-[2-(1-méthyl-4-pipéridinyl)carbonylamino-pyridin-4-ylméthyl]-3-(4-trifluorométhanesulfanyl-phényl)-imidazolidine-2,4-dione
- la 5,5-diméthyl-1-[2-(2-furanylcarbonyl)amino-pyridin-4-ylméthyl]-3-(4-trifluorométhanesulfanyl-phényl)-imidazolidine-2,4-dione
- la 5,5-diméthyl-1-(2-éthoxycarbonylamino-pyridin-4-ylméthyl)-3-(4-trifluorométhanesulfanyl-phényl)-imidazolidine-2,4-dione
- la 5,5-diméthyl-1-(2-isopropoxycarbonylamino-pyridin-4-ylméthyl)-3-(4-trifluorométhanesulfanyl-phényl)-imidazolidine-2,4-dione
- la 5,5-diméthyl-1-(2-butoxycarbonylamino-pyridin-4-ylméthyl)-3-(4-trifluorométhanesulfanyl-phényl)-imidazolidine-2,4-dione
- la 5,5-diméthyl-1-[2-(2-diéthylaminoéthoxycarbonylamino)-pyridin-4-ylméthyl]-3-(4-trifluorométhanesulfanyl-phényl)-imidazolidine-2,4-dione
- la 5,5-diméthyl-1-(2-phénoxycarbonylamino-pyridin-4-ylméthyl)-3-(4-trifluorométhanesulfanyl-phényl)-imidazolidine-2,4-dione
- la 5,5-diméthyl-1-[2-(4-chloroanilino)carbonylamino-pyridin-4-ylméthyl]-3-(4-trifluorométhanesulfanyl-phényl)-imidazolidine-2,4-dione
- la 5,5-diméthyl-1-(2-anilinocarbonylamino-pyridin-4-ylméthyl)-3-(4-trifluorométhanesulfanyl-phényl)-imidazolidine-2,4-dione
- la 5,5-diméthyl-1-(2-benzylaminocarbonylamino-pyridin-4-ylméthyl)-3-(4-trifluorométhanesulfanyl-phényl)-imidazolidine-2,4-dione
- la 5,5-diméthyl-1-[2-(2-pyridylamino)carbonylamino-pyridin-4-ylméthyl]-3-(4-trifluorométhanesulfanyl-phényl)-imidazolidine-2,4-dione
- la 5,5-diméthyl-1-(2-uréido-pyridin-4-ylméthyl)-3-(4-trifluorométhanesulfanyl-phényl)-imidazolidine-2,4-dione
- la 5,5-diméthyl-1-(2-benzylaminosulfonylamino-pyridin-4-ylméthyl)-3-(4-trifluorométhanesulfanyl-phényl)-imidazolidine-2,4-dione
- la 5,5-diméthyl-1-(2-méthylaminosulfonylamino-pyridin-4-ylméthyl)-3-(4-trifluo-rométhanesulfanyl-phényl)-imidazolidine-2,4-dione
- la 5,5-diméthyl-1-(2-aminosulfonylamino-pyridin-4-ylméthyl)-3-(4-trifluorométhanesulfanyl-phényl)-imidazolidine-2,4-dione
- la 5,5-diméthyl-1-(2-morpholinosulfonylamino-pyridin-4-ylméthyl)-3-(4-trifluorométhanesulfanyl-phényl)-imidazolidine-2,4-dione
- la 5,5-diméthyl-1-(2-diméthylaminosulfonylamino-pyridin-4-ylméthyl)-3-(4-trifluorométhanesulfanyl-phényl)-imidazolidine-2,4-dione
- la 5,5-diméthyl-1-(2-anilinosulfonylamino-pyridin-4-ylméthyl)-3-(4-trifluorométhanesulfanyl-phényl)-imidazolidine-2,4-dione
- la 5,5-diméthyl-1-(2-phénylsulfonylamino-pyridin-4-ylméthyl)-3-(4-trifluorométhanesulfanyl-phényl)-imidazolidine-2,4-dione
- la 5,5-diméthyl-1-[2-(2-thiényl)sulfonylamino-pyridin-4-ylméthyl]-3-(4-trifluorométhanesulfanyl-phényl)-imidazolidine-2,4-dione
- la 5,5-diméthyl-1-(2-méthylsulfonylamino-pyridin-4-ylméthyl)-3-(4-trifluorométhanesulfanyl-phényl)-imidazolidine-2,4-dione
- la 5,5-diméthyl-1-[2-(4-chlorophényl)sulfonylamino-pyridin-4-ylméthyl]-3-(4-trifluorométhanesulfanyl-phényl)-imidazolidine-2,4-dione
- la 5,5-diméthyl-1-(2-benzylsulfonylamino-pyridin-4-ylméthyl)-3-(4-trifluorométhanesulfanyl-phényl)-imidazolidine-2,4-dione
- la 5,5-diméthyl-1-(2-hydroxycarbonyl-pyridin-4-ylméthyl)-3-(4-trifluorométhanesulfanyl-phényl)-imidazolidine-2,4-dione
- la 5,5-diméthyl-1-(2-éthoxycarbonyl-pyridin-4-ylméthyl)-3-(4-trifluorométhanesulfanyl-phényl)-imidazolidine-2,4-dione
- la 5,5-diméthyl-1-(2-ter-butoxycarbonyl-pyridin-4-ylméthyl)-3-(4-trifluorométhanesulfanyl-phényl)-imidazolidine-2,4-dione
- la 5,5-diméthyl-1-(2-phénoxycarbonyl-pyridin-4-ylméthyl)-3-(4-trifluorométhanesulfanyl-phényl)-imidazolidine-2,4-dione
- la 5,5-diméthyl-1-[2-(4-méthylpipérazinocarbonyl)-pyridin-4-ylméthyl]-3-(4-trifluorométhanesulfanyl-phényl)-imidazolidine-2,4-dione
- la 5,5-diméthyl-1-[2-(méthylamino)carbonyl-pyridin-4-ylméthyl]-3-(4-trifluorométhanesulfanyl-phényl)-imidazolidine-2,4-dione
- la 5,5-diméthyl-1-[2-(diméthylamino)carbonyl-pyridin-4-ylméthyl]-3-(4-trifluorométhanesulfanyl-phényl)-imidazolidine-2,4-dione
- la 5,5-diméthyl-1-(2-pipéridinocarbonyl-pyridin-4-ylméthyl)-3-(4-trifluorométhanesulfanyl-phényl)-imidazolidine-2,4-dione
- la 5,5-diméthyl-1-(2-morpholinocarbonyl-pyridin-4-ylméthyl)-3-(4-trifluorométhanesulfanyl-phényl)-imidazolidine-2,4-dione
- la 5,5-diméthyl-1-[2-(4-phénylpipérazinocarbonyl)-pyridin-4-ylméthyl]-3-(4-trifluorométhanesulfanyl-phényl)-imidazolidine-2,4-dione
- la 5,5-diméthyl-1-(2-phénylaminocarbonyl-pyridin-4-ylméthyl)-3-(4-trifluorométhanesulfanyl-phényl)-imidazolidine-2,4-dione
- la 5,5-diméthyl-1-[2-(méthylphénylamino)carbonyl-pyridin-4-ylméthyl]-3-(4-trifluorométhanesulfanyl-phényl)-imidazolidine-2,4-dione
- la 5,5-diméthyl-1-[2-(4-méthoxyphényl)méthylaminocarbonyl-pyridin-4-ylméthyl]-3-(4-trifluorométhanesulfanyl-phényl)-imidazolidine-2,4-dione
- la 5,5-diméthyl-1-[2-(4-chlorophényl)méthylaminocarbonyl-pyridin-4-ylméthyl]-3-(4-trifluorométhanesulfanyl-phényl)-imidazolidine-2,4-dione
- la 5,5-diméthyl-1-[2-(4-morpholinylamino)carbonyl-pyridin-4-ylméthyl]-3-(4-trifluorométhanesulfanyl-phényl)-imidazolidine-2,4-dione
- la 5,5-diméthyl-1-(2-aminocarbonyl-pyridin-4-ylméthyl)-3-(4-trifluorométhanesulfanyl-phényl)-imidazolidine-2,4-dione
- la 5,5-diméthyl-1-(2-phénoxy-pyridin-4-ylméthyl)-3-(4-trifluorométhanesulfanyl-phényl)-imidazolidine-2,4-dione
- la 5,5-diméthyl-1-(2-méthoxy-pyridin-4-ylméthyl)-3-(4-trifluorométhanesulfanyl-phényl)-imidazolidine-2,4-dione
- la 5,5-diméthyl-1-(2-éthoxy-pyridin-4-ylméthyl)-3-(4-trifluorométhanesulfanyl-phényl)-imidazolidine-2,4-dione
- la 5,5-diméthyl-1-[2-(1-pyrrolidinyl)éthoxy-pyridin-4-ylméthyl]-3-(4-trifluorométhanesulfanyl-phényl)-imidazolidine-2,4-dione
- la 5,5-diméthyl-1-(2-benzyloxy-pyridin-4-ylméthyl)-3-(4-trifluorométhanesulfanyl-phényl)-imidazolidine-2,4-dione
- la 5,5-diméthyl-1-(2-benzoyloxy-pyridin-4-ylméthyl)-3-(4-trifluorométhanesulfanyl-phényl)-imidazolidine-2,4-dione
- la 5,5-diméthyl-1-[2-(4,5-dihydro-1H-imidazol-2-yl)-pyridin-4-ylméthyl]-3-(4-trifluorométhanesulfanyl-phényl)-imidazolidine-2,4-dione
- la 5,5-diméthyl-1-[2-(1H-imidazol-2-yl)-pyridin-4-ylméthyl]-3-(4-trifluorométhanesulfanyl-phényl)-imidazolidine-2,4-dione
- la 5,5-diméthyl-1-[2-(1H-tétrazol-5-yl)-pyridin-4-ylméthyl]-3-(4-trifluorométhanesulfanyl-phényl)-imidazolidine-2,4-dione
- la 5,5-diméthyl-1-(2-amidino-pyridin-4-ylméthyl)-3-(4-trifluorométhanesulfanyl-phényl)-imidazolidine-2,4-dione
- la 5,5-diméthyl-1-(2-diméthylaminométhyl-pyridin-4-ylméthyl)-3-(4-trifluorométhanesulfanyl-phényl)-imidazolidine-2,4-dione
- la 5,5-diméthyl-1-(2-aminoméhyl-pyridin-4-ylméthyl)-3-(4-trifluorométhanesulfanyl-phényl)-imidazolidine-2,4-dione
- la 5,5-diméthyl-1-(3-méthyl-pyridin-4-ylméthyl)-3-(4-trifluorométhanesulfanyl-phényl)-imidazolidine-2,4-dione
- la 5,5-diméthyl-1-(3-éthyl-pyridin-4-ylméthyl)-3-(4-trifluorométhanesulfanyl-phényl)-imidazolidine-2,4-dione
- la 5,5-diméthyl-1-(3-isopropyl-pyridin-4-ylméthyl)-3-(4-trifluorométhanesulfanyl-phényl)-imidazolidine-2,4-dione
- la 5,5-diméthyl-1-(3-ter-butyl-pyridin-4-ylméthyl)-3-(4-trifluorométhanesulfanyl-phényl)-imidazolidine-2,4-dione
- la 5,5-diméthyl-1-(3-isobutyl-pyridin-4-ylméthyl)-3-(4-trifluorométhanesulfanyl-phényl)-imidazolidine-2,4-dione
- la 5,5-diméthyl-1-(3-cyclohéxyl-pyridin-4-ylméthyl)-3-(4-trifluorométhanesulfanyl-phényl)-imidazolidine-2,4-dione
- la 5,5-diméthyl-1-(3-phényl-pyridin-4-ylméthyl)-3-(4-trifluorométhanesulfanyl-phényl)-imidazolidine-2,4-dione
- la 5,5-diméthyl-1-[3-(4-chlorophényl)-pyridin-4-ylméthyl]-3-(4-trifluorométhanesulfanyl-phényl)-imidazolidine-2,4-dione
- la 5,5-diméthyl-1-[3-(3-chlorophényl)-pyridin-4-ylméthyl]-3-(4-trifluorométhanesulfanyl-phényl)-imidazolidine-2,4-dione
- la 5,5-diméthyl-1-[3-(2-chlorophényl)-pyridin-4-ylméthyl]-3-(4-trifluorométhanesulfanyl-phényl)-imidazolidine-2,4-dione
- la 5,5-diméthyl-1-[3-(4-fluorophényl)-pyridin-4-ylméthyl]-3-(4-trifluorométhanesulfanyl-phényl)-imidazolidine-2,4-dione
- la 5,5-diméthyl-1-[3-(4-méthoxyphényl)-pyridin-4-ylméthyl]-3-(4-trifluorométhanesulfanyl-phényl)-imidazolidine-2,4-dione
- la 5,5-diméthyl-1-(3-benzyl-pyridin-4-ylméthyl)-3-(4-trifluorométhanesulfanyl-phényl)-imidazolidine-2,4-dione
- la 5,5-diméthyl-1-(3,2'bipyridin-4-ylméthyl)-3-(4-trifluorométhanesulfanyl-phényl)-imidazolidine-2,4-dione
- la 5,5-diméthyl-1-(3-hydroxyméthyl-pyridin-4-ylméthyl)-3-(4-trifluorométhanesulfanyl-phényl)-imidazolidine-2,4-dione
- la 5,5-diméthyl-1-(3-chloro-pyridin-4-ylméthyl)-3-(4-trifluorométhanesulfanyl-phényl)-imidazolidine-2,4-dione
- la 5,5-diméthyl-1-(3-fluoro-pyridin-4-ylméthyl)-3-(4-trifluorométhanesulfanyl-phényl)-imidazolidine-2,4-dione
- la 5,5-diméthyl-1-(3-bromo-pyridin-4-ylméthyl)-3-(4-trifluorométhanesulfanyl-phényl)-imidazolidine-2,4-dione
- la 5,5-diméthyl-1-(3-amino-pyridin-4-ylméthyl)-3-(4-trifluorométhanesulfanyl-phényl)-imidazolidine-2,4-dione
- la 5,5-diméthyl-1-(3-méthylamino-pyridin-4-ylméthyl)-3-(4-trifluorométhanesulfanyl-phényl)-imidazolidine-2,4-dione
- la 5,5-diméthyl-1-(3-éthylamino-pyridin-4-ylméthyl)-3-(4-trifluorométhanesulfanyl-phényl)-imidazolidine-2,4-dione
- la 5,5-diméthyl-1-(3-isopropylamino-pyridin-4-ylméthyl)-3-(4-trifluorométhanesulfanyl-phényl)-imidazolidine-2,4-dione
- la 5,5-diméthyl-1-(3-phénylamino-pyridin-4-ylméthyl)-3-(4-trifluorométhanesulfanyl-phényl)-imidazolidine-2,4-dione
- la 5,5-diméthyl-1-(3-cyclohéxylamino-pyridin-4-ylméthyl)-3-(4-trifluorométhanesulfanyl-phényl)-imidazolidine-2,4-dione
- la 5,5-diméthyl-1-(3-diéthylamino-pyridin-4-ylméthyl)-3-(4-trifluorométhanesulfanyl-phényl)-imidazolidine-2,4-dione
- la 5,5-diméthyl-1-(3-N-cyclohéxyl-N-méthylamino-pyridin-4-ylméthyl)-3-(4-trifluorométhanesulfanyl-phényl)-imidazolidine-2,4-dione
- la 5,5-diméthyl-1-(3-pipéridino-pyridin-4-ylméthyl)-3-(4-trifluorométhanesulfanyl-phényl)-imidazolidine-2,4-dione
- la 5,5-diméthyl-1-(3-morpholino-pyridin-4-ylméthyl)-3-(4-trifluorométhanesulfanyl-phényl)-imidazolidine-2,4-dione
- la 5,5-diméthyl-1-[3-(4-méthylpipérazino)-pyridin-4-ylméthyl]-3-(4-trifluorométhanesulfanyl-phényl)-imidazolidine-2,4-dione
- la 5,5-diméthyl-1-[3-(1H-pyrazol-1yl)-pyridin-4-ylméthyl]-3-(4-trifluorométhanesulfanyl-phényl)-imidazolidine-2,4-dione
- la 5,5-diméthyl-1-[3-(2-benzoylhydrazino)-pyridin-4-ylméthyl]-3-(4-trifluorométhanesulfanyl-phényl)-imidazolidine-2,4-dione
- la 5,5-diméthyl-1-(3-benzoylamino-pyridin-4-ylméthyl)-3-(4-trifluorométhanesulfanyl-phényl)-imidazolidine-2,4-dione
- la 5,5-diméthyl-1-[3-(4-chlorobenzoylamino)-pyridin-4-ylméthyl]-3-(4-trifluorométhanesulfanyl-phényl)-imidazolidine-2,4-dione
- la 5,5-diméthyl-1-[3-(3-chlorobenzoylamino)-pyridin-4-ylméthyl]-3-(4-trifluorométhanesulfanyl-phényl)-imidazolidine-2,4-dione
- la 5,5-diméthyl-1-[3-(2-chlorobenzoylamino)-pyridin-4-ylméthyl]-3-(4-trifluorométhanesulfanyl-phényl)-imidazolidine-2,4-dione
- la 5,5-diméthyl-1-[3-(4-fluorobenzoylamino)-pyridin-4-ylméthyl]-3-(4-trifluorométhanesulfanyl-phényl)-imidazolidine-2,4-dione
- la 5,5-diméthyl-1-[3-(4-méthoxybenzoylamino)-pyridin-4-ylméthyl]-3-(4-trifluorométhanesulfanyl-phényl)-imidazolidine-2,4-dione
- la 5,5-diméthyl-1-(3-acétamido-pyridin-4-ylméthyl)-3-(4-trifluorométhanesulfanyl-phényl)-imidazolidine-2,4-dione
- la 5,5-diméthyl-1-(3-phénylacétamido-pyridin-4-ylméthyl)-3-(4-trifluorométhanesulfanyl-phényl)-imidazolidine-2,4-dione
- la 5,5-diméthyl-1-[3-(4-méthylpipérazino)acétamido-pyridin-4-ylméthyl]-3-(4-trifluorométhanesulfanyl-phényl)-imidazolidine-2,4-dione
- la 5,5-diméthyl-1-(3-cyclohéxanylcarboxamido-pyridin-4-ylméthyl)-3-(4-trifluorométhanesulfanyl-phényl)-imidazolidine-2,4-dione
- la 5,5-diméthyl-1-[3-(1-méthyl-4-pipéridinyl)carbonylamino-pyridin-4-ylméthyl]-3-(4-trifluorométhanesulfanyl-phényl)-imidazolidine-2,4-dione
- la 5,5-diméthyl-1-[3-(2-furanylcarbonyl)amino-pyridin-4-ylméthyl]-3-(4-trifluorométhanesulfanyl-phényl)-imidazolidine-2,4-dione
- la 5,5-diméthyl-1-(3-éthoxycarbonylamino-pyridin-4-ylméthyl)-3-(4-trifluorométhanesulfanyl-phényl)-imidazolidine-2,4-dione
- la 5,5-diméthyl-1-(3-isopropoxycarbonylamino-pyridin-4-ylméthyl)-3-(4-trifluorométhanesulfanyl-phényl)-imidazolidine-2,4-dione
- la 5,5-diméthyl-1-(3-butoxycarbonylamino-pyridin-4-ylméthyl)-3-(4-trifluorométhanesulfanyl-phényl)-imidazolidine-2,4-dione
- la 5,5-diméthyl-1-[3-(2-diéthylaminoéthoxycarbonylamino)-pyridin-4-ylméthyl]-3-. (4-trifluorométhanesulfanyl-phényl)-imidazolidine-2,4-dione
- la 5,5-diméthyl-1(3-phénoxycarbonylamino-pyridin-4-ylméthyl)-3-(4-trifluorométhanesulfanyl-phényl)-imidazolidine-2,4-dione
- la 5,5-diméthyl-1-[3-(4-chloroanilino)carbonylamino-pyridin-4-ylméthyl]-3-(4-trifluorométhanesulfanyl-phényl)-imidazolidine-2,4-dione
- la 5,5-diméthyl-1-(3-anilinocarbonylamino-pyridin-4-ylméthyl)-3-(4-trifluorométhanesulfanyl-phényl)-imidazolidine-2,4-dione
- la 5,5-diméthyl-1-(3-benzylaminocarbonylamino-pyridin-4-ylméthyl)-3-(4-t-rifluorométhanesulfanyl-phényl)-imidazolidine-2,4-dione
- la 5,5-diméthyl-1-[3-(2-pyridylamino)carbonylamino-pyridin-4-ylméthyl]-3-(4-trifluorométhanesulfanyl-phényl)-imidazolidine-2,4-dione
- la 5,5-diméthyl-1-(3-uréido-pyridin-4-ylméthyl)-3-(4-trifluorométhanesulfanyl-phényl)-imidazolidine-2,4-dione
- la 5,5-diméthyl-1-(3-benzylaminosulfonylamino-pyridin-4-ylméthyl)-3-(4-trifluorométhanesulfanyl-phényl)-imidazolidine-2,4-dione
- la 5,5-diméthyl-1-(3-méthylaminosulfonylamino-pyridin-4-ylméthyl)-3-(4-trifluorométhanesulfanyl-phényl)-imidazolidine-2,4-dione
- la 5,5-diméthyl-1-(3-aminosulfonylamino-pyridin-4-ylméthyl)-3-(4-trifluorométhanesulfanyl-phényl)-imidazolidine-2,4-dione
- la 5,5-diméthyl-1-(3-morpholinosulfonylamino-pyridin-4-ylméthyl)-3-(4-trifluorométhanesulfanyl-phényl)-imidazolidine-2,4-dione
- la 5,5-diméthyl-1-(3-diméthylaminosulfonylamino-pyridin-4-ylméthyl)-3-(4-trifluorométhanesulfanyl-phényl)-imidazolidine-2,4-dione
- la 5,5-diméthyl-1-(3-anilinosulfonylamino-pyridin-4-ylméthyl)-3-(4-trifluorométhanesulfanyl-phényl)-imidazolidine-2,4-dione
- la 5,5-diméthyl-1-(3-phénylsulfonylamino-pyridin-4-ylméthyl)-3-(4-trifluorométhanesulfanyl-phényl)-imidazolidine-2,4-dione
- la 5,5-diméthyl-1-[3-(2-thiényl)sulfonylamino-pyridin-4-ylméthyl]-3-(4-trifluorométhanesulfanyl-phényl)-imidazolidine-2,4-dione
- la 5,5-diméthyl-1-(3-méthylsulfonylamino-pyridin-4-ylméthyl)-3-(4-trifluorométhanesulfanyl-phényl)-imidazolidine-2,4-dione
- la 5,5-diméthyl-1-[3-(4-chlorophényl)sulfonylamino-pyridin-4-ylméthyl]-3-(4-trifluorométhanesulfanyl-phényl)-imidazolidine-2,4-dione
- la 5,5-diméthyl-1-(3-benzylsulfonylamino-pyridin-4-ylméthyl)-3-(4-trifluorométhanesulfanyl-phényl)-imidazolidine-2,4-dione
- la 5,5-diméthyl-1-(3-hydroxycarbonyl-pyridin-4-ylméthyl)-3-(4-trifluorométhanesulfanyl-phényl)-imidazolidine-2,4-dione
- la 5,5-diméthyl-1-(3-éthoxycarbonyl-pyridin-4-ylméthyl)-3-(4-trifluorométhanesulfanyl-phényl)-imidazolidine-2,4-dione
- la 5,5-diméthyl-1-(3-ter-butoxycarbonyl-pyridin-4-ylméthyl)-3-(4-trifluorométhanesulfanyl-phényl)-imidazolidine-2,4-dione
- la 5,5-diméthyl-1-(3-phénoxycarbonyl-pyridin-4-ylméthyl)-3-(4-trifluorométhanesulfanyl-phényl)-imidazolidine-2,4-dione
- la 5,5-diméthyl-1-[3-(4-méthylpipérazinocarbonyl)-pyridin-4-ylméthyl]-3-(4-trifluorométhanesulfanyl-phényl)-imidazolidine-2,4-dione
- la 5,5-diméthyl-1-[3-(méthylamino)carbonyl-pyridin-4-ylméthyl]-3-(4-trifluorométhanesulfanyl-phényl)-imidazolidine-2,4-dione
- la 5,5-diméthyl-1-[3-(diméthylamino) carbonyl-pyridin-4-ylméthyl]-3-(4-trifluorométhanesulfanyl-phényl)-imidazolidine-2,4-dione
- la 5,5-diméthyl-1-(3-pipéridinocarbonyl-pyridin-4-ylméthyl)-3-(4-trifluorométhanesulfanyl-phényl)-imidazolidine-2,4-dione
- la 5,5-diméthyl-1-(3-morpholinocarbonyl-pyridin-4-ylméthyl)-3-(4-trifluorométhanesulfanyl-phényl)-imidazolidine-2,4-dione
- la 5,5-diméthyl-1-[3-(4-phénylpipérazinocarbonyl)-pyridin-4-ylméthyl]-3-(4-trifluorométhanesulfanyl-phényl)-imidazolidine-2,4-dione
- la 5,5-diméthyl-1-(3-phénylaminocarbonyl-pyridin-4-ylméthyl)-3-(4-trifluorométhanesulfanyl-phényl)-imidazolidine-2,4-dione
- la 5,5-diméthyl-1-[3-(méthylphénylamino)carbonyl-pyridin-4-ylméthyl]-3-(4-trifluorométhanesulfanyl-phényl)-imidazolidine-2,4-dione
- la 5,5-diméthyl-1-[3-(4-méthoxyphényl)méthylaminocarbonyl-pyridin-4-ylméthyl]-3-(4-trifluorométhanesulfanyl-phényl)-imidazolidine-2,4-dione
- la 5,5-diméthyl-1-[3-(4-chlorophényl)méthylamino-carbonyl-pyridin-4-ylméthyl]-3-(4-trifluorométhanesulfanyl-phényl)-imidazolidine-2,4-dione
- la 5,5-diméthyl-1-[3-(4-morpholinylamino)carbonyl-pyridin-4-ylméthyl]-3-(4-trifluorométhanesulfanyl-phényl)-imidazolidine-2,4-dione
- la 5,5-diméthyl-1-(3-aminocarbonyl-pyridin-4-ylméthyl)-3-(4-trifluorométhanesulfanyl-phényl)-imidazolidine-2,4-dione
- la 5,5-diméthyl-1-(3-phénoxy-pyridin-4-ylméthyl)-3-(4-trifluorométhanesulfanyl-phényl)-imidazolidine-2,4-dione
- la 5,5-diméthyl-1-(3-méthoxy-pyridin-4-ylméthyl)-3-(4-trifluorométhanesulfanyl-phényl)-imidazolidine-2,4-dione
- la 5,5-diméthyl-1-(3-éthoxy-pyridin-4-ylméthyl)-3-(4-trifluorométhanesulfanyl-phényl)-imidazolidine-2,4-dione
- la 5,5-diméthyl-1-[3-(1-pyrrolidinyl)éthoxy-pyridin-4-ylméthyl]-3-(4-trifluorométhanesulfanyl-phényl)-imidazolidine-2,4-dione
- la 5,5-diméthyl-1-(3-benzyloxy-pyridin-4-ylméthyl)-3-(4-trifluorométhanesulfanyl-phényi)-imidazolidine-2,4-dione
- la 5,5-diméthyl-1-(3-benzoyloxy-pyridin-4-ylméthyl)-3-(4-trifluorométhanesulfany1-phényl)-imidazolidine-2,4-dione
- la 5,5-diméthyl-1-[3-(4,5-dihydro-1H-imidazol-2-yl)-pyridin-4-ylméthyl]-3-(4-trifluorométhanesulfanyl-phényl)-imidazolidine-2,4-dione
- la 5,5-diméthyl-1-[3-(1H-imidazol-2-yl)-pyridin-4-ylméthyl]-3-(4-trifluoromé-thanesulfanyl-phényl)-imidazolidine-2,4-dione
- la 5,5-diméthyl-1-[3-(1H-tétrazol-5-yl)-pyridin-4-ylméthy1]-3-(4trifluorométhanesulfanyl-phényl)-imidazolidine-2,4-dione
- la 5,5-diméthyl-1-(3-amidino-pyridin-4-ylméthyl)-3-(4-trifluorométhanesulfanyl-phényl)-imidazolidine-2,4-dione
- la 5,5-diméthyl-1-(3-diméthylaminométhyl-pyridin-4-ylméthyl)3-(4-trifluorométhanesulfanyl-phényl)-imidazolidine-2,4-dione
- la 5,5-diméthyl-1-(3-aminométhyl-pyridin-4-ylméthyl)-3-(4-trifluorométhanesulfanyl-phényl)-imidazolidine-2,4-dione
- la 4-(2-méthyl-pyridin)-4-ylméthyl-6-(4-trifluorométhanesulfanyl-phényl)-4,6-diaza-spiro[2.4]heptane-5,7-dione
- la 4-(2-éthyl-pyridin)-4-ylméthyl-6-(4-trifluorométhanesulfanyl-phényl)-4,6-diaza-spiro[2.4]heptane-5,7-dione
- la 4-(2-isopropyl-pyridin)-4-ylméthyl-6-(4-trifluorométhanesulfanyl-phényl)-4,6-diaza-spiro[2.4]heptane-5,7-dione
- la 4-(2-ter-butyl-pyridin)-4-ylméthyl-6-(4-trifluorométhane sulfanyl-phényl)-4,6-diaza-spiro[2.4,]heptane-5,7-dione
- la 4-(2-isobutyl-pyridin)-4-ylméthyl-6-(4-trifluorométhanesulfanyl-phényl)-4,6-diaza-spiro[2.4]heptane-5,7-dione
- la 4-(2-cyclohéxyl-pyridin)-4-ylméthyl-6-(4-trifluorométhanesulfanyl-phényl)-4,6-diaza-spiro[2.4]heptane-5,7-dione
- la 4-(2-phényl-pyridin)-4-ylméthyl-6-(4-trifluorométhanesulfanyl-phényl)-4,6-diaza-spiro[2.4]heptane-5,7-dione
- la 4-[2-(4-chlorophényl)-pyridin]-4-ylméthyl-6-(4-trifluorométhanesulfanyl-phényl)-4,6-diaza-spiro[2.4]heptane-5,7-dione
- la 4-[2-(3-chlorophényl)-pyridin]-4-ylméthyl-6-(4-trifluorométhanesulfanyl-phênyl)-4,6-diaza-spiro[2.4]heptane-5,7-dione
- la 4-[2-(2-chlorophényl)-pyridin]-4-ylméthyl-6-(4-trifluorométhanesulfanyl-phényl)-4,6-diaza-spiro[2.4]heptane-5,7-dione
- la 4-[2-(4-fluorophényl)-pyridin]-4-ylméthyl-6-(4-trifluorométhanesulfanyl-phényl)-4,6-diaza-spiro[2.4]heptane-5,7-dione
- la 4-[2-(4-méthoxyphényl)-pyridin]-4-ylméthyl-6-(4-trifluorométhanesulfanyl-phényl)-4,6-diaza-spiro[2.4]heptane-5,7-dione
- la 4-(2-benzyl-pyridin)-4-ylméthyl-6-(4-trifluorométhanesulfanyl-phényl)-4,6-diaza-spiro[2.4]heptane-5,7-dione
- la 4-(2,2'bipyridin)-4-ylméthyl-6-(4-trifluorométhanesulfanyl-phényl)-4,6-diaza-spiro[2.4]heptane-5,7-dione
- la 4-(2-hydroxyméthyl-pyridin)-4-ylméthyl-6-(4-trifluorométhanesulfanyl-phényl)-4,6-diaza-spiro[2.4]heptane-5,7-dione
- la 4-(2-chloro-pyridin)-4-ylméthyl-6-(4-trifluorométhanesulfanyl-phényl)-4,6-diaza-spiro[2.4]heptané-5,7-dione
- la 4-(2-fluoro-pyridin)-4-ylméthyl-6-(4-trifluorométhanesulfanyl-phényl)-4,6-diaza-spiro[2.4]heptane-5,7-dione
- la 4-(2-bromo-pyridin)-4-ylméthyl-6-(4-trifluorométhanesulfanyl-phényl)-4,6-diaza-spiro[2.4]heptane-5,7-dione
- la 4-(2-amino-pyridin)-4-ylméthyl-6-(4-trifluorométhanesulfanyl-phényl)-4,6-diaza-spiro[2.4]heptane-5,7-dione
- la 4-(2-méthylamino-pyridin)-4-ylméthyl-6-(4-trifluorométhanesulfanyl-phényl)-4,6-dia.za-spiro[2.4]heptane-5,7-dione
- la 4-(2-éthylamino-pyridin)-4-ylméthyl-6-(4-trifluorométhanesulfanyl-phényl)-4,6-diaza-spiro[2.4]heptane-5,7-dione
- la 4-(2-isopropylamino-pyridin)-4-ylméthyl-6-(4-trifluorométhanesulfanyl-phényl)-4,6-diaza-spiro[2.4]heptane-5,7-dione
- la 4-(2-phénylamino-pyridin)-4-ylméthyl-6-(4-trifluorométhanesulfanyl-phényl)-4,6-diaza-spiro[2.4]heptane-5,7-dione
- la 4-(2-cyclohéxylamino-pyridin)-4-ylméthyl-6-(4-trifluorométhanesulfanyl-phényl)-4,6-diaza-spiro[2.4]heptane-5,7-dione
- la 4-(2-diéthylamino-pyridin)-4-ylméthyl-6-(4-trifluorométhanesulfanyl-phényl)-4,6-diaza-spiro[2.4]heptane-5,7-dione
- la 4-(2-N-cyclohéxyl-N-méthylamino-pyridin)-4-ylméthyl-6-(4-trifluorométhanesulfanyl-phényl)-4,6-diaza-spiro[2.4]heptane-5,7-dione
- la 4-(2-pipéridino-pyridin)-4-ylméthyl-6-(4-trifluorométhanesulfanyl-phényl)-4,6-diaza-spiro[2.4]heptane-5,7-dione
- la 4-(2-morpholino-pyridin)-4-ylméthyl-6-(4-trifluorométhanesulfanyl-phényl)-4,6-diaza-spiro[2.4]heptane-5,7-dione
- la 4-[2-(4-méthylpipérazino)-pyridin]-4-ylméthyl-6-(4-trifluorométhanesulfanyl-phényl)-4,6-diaza-spiro[2.4]heptane-5,7-dione
- la 4-[2-(1H-pyrazol-1yl)-pyridin]-4-ylméthyl-6-(4-trifluorométhanesulfanyl-phényl)-4,6-diaza-spiro[2.4]heptane-5,7-dione
- la 4-[2-(2-benzoylhydrazino)-pyridin]-4-ylméthyl-6-(4-trifluorométhanesulfanyl-phényl)-4,6-diaza-spiro[2.4]heptane-5,7-dione
- la 4-(2-benzoylamino-pyridin)-4-ylméthyl-6-(4-trifluorométhanesulfanyl-phényl)-4,6-diaza-spiro[2.4]heptane-5,7-dione
- la 4-[2-(4-chlorobenzoylamino)-pyridin]-4-ylméthyl-6-(4-trifluorométhanesulfanyl-phényl)-4,6-diaza-spiro[2.4]heptane-5,7-dione
- la 4-[2-(3-chlorobenzoylamino)-pyridin]-4-ylméthyl-6-(4-trifluorométhanesulfanyl-phényl)-4,6-diaza-spiro[2.4]heptane-5,7-dione
- la 4-[2-(2-chlorobenzoylamino)-pyridin]-4-ylméthyl-6-(4-trifluorométhanesulfanyl-phényl)-4,6-diaza-spiro[2.4]heptane-5,7-dione
- la 4-[2-(4-fluorobenzoylamino)-pyridin]-4-ylméthyl-6-(4-trifluorométhanesulfanyl-phényl)-4,6-diaza-spiro[2.4]heptane-5,7-dione
- la 4-[2-(4-méthoxybenzoylamino)-pyridin]-4-ylméthyl-6-(4-trifluorométhanesulfanyl-phényl)-4,6-diaza-spiro[2.4]heptane-5,7-dione
- la 4-(2-acétamido-pyridin)-4-ylméthyl-6-(4-trifluorométhanesulfanyl-phényl)-4,6-diaza-spiro[2.4]heptane-5,7-dione
- la 4-(2-phénylacétamido-pyridin)-4-ylméthyl-6-(4-trifluorométhanesulfanyl-phényl)-4,6-diaza-spiro[2.4]heptane-5,7-dione
- la 4-[2-(4-méthylpipérazino)acétamido-pyridin]-4-ylméthyl-6-(4-trifluorométhanesulfanyl-phényl)-4,6-diaza-spiro[2.4]heptane-5,7-dione
- la 4-(2-cyclohéxanylcarboxamido-pyridin)-4-ylméthyl-6-(4-trifluorométhanesulfanyl-phényl)-4,6-diaza-spiro[2.4]heptane-5,7-dione
- la 4-[2-(1-méthyl-4-pipéridinyl)carbonylamino-pyridin]-4-ylméthyl-6-(4-trifluorométhanesulfanyl-phényl)-4,6-diaza-spiro[2.4]heptane-5,7-dione
- la 4-[2-(2-furanylcarbonyl)amino-pyridin]-4-ylméthyl-6-(4-trifluoromêthanesulfanyl-phényl)-4,6-diaza-spiro[2.4]heptane-5,7-dione
- la 4-(2-éthoxycarbonylamino-pyridin)-4-ylméthyl-6-(4-trifluorométhanesulfansl-phényl)-4,6-diaza-spiro[2.4]heptane-5,7-dione
- la 4-(2-isopropoxycarbonylamino-pyridin)-4-ylméthyl-6-(4-trifluorométhanesulfanyl-phényl) -4,6-diaza-spiro[2.4]heptane-5,7-dione
- la 4-(2-butoxycarbonylamino-pyridin)-4-ylméthyl-6-(4-trifluorométhanesulfanyl-phényl)-4,6-diaza-spiro[2.4]heptane-5,7-dione
- la 4-[2-(2-diéthylaminoéthoxycarbonylamino)-pyridin]-4-ylméthyl-6-(4-trifluorométhanesulfanyl-phényl)-4,6-diaza-spiro[2.4]heptane-5,7-dione
- la 4-(2-phénoxycarbonylamino-pyridin)-4-ylméthyl-6-(4-trifluorométhanesulfanyl-phényl)-4,6-diaza-spiro[2.4]heptane-5,7-dione
- la 4-[2-(4-chloroanilino)carbonylamino-pyridin]-4-ylméthyl)-6-(4-trifluorométhanesulfanyl-phényl)-4,6-diaza-spiro[2.4]heptane-5,7-dione
- la 4-(2-anilinocarbonylamino-pyridin)-4-ylméthyl-6-(4-trifluorométhanesulfanyl-phényl)-4,6-diaza-spiro[2.4]heptane-5,7-dione
- la 4-(2-benzylaminocarbonylamino-pyridin)-4-ylméthyl-6-(4-trifluorométhanesulfanyl-phényl)-4,6-diaza-spiro[2.4]heptane-5,7-dione
- la 4-[2-(2-pyridylamino)carbonylamino-pyridin]-4-ylméthyl-6-(4-trifluorométhanesulfanyl-phényl)-4,6-diaza-spiro[2.4]heptane-5,7-dione
- la 4-(2-uréido-pyridin)-4-ylméthyl-6-(4-trifluorométhanesulfanyl-phényl)-4,6-diaza-spiro[2.4]heptane-5,7-dione
- la 4-(2-benzylaminosulfonylamino-pyridin)-4-ylméthyl-6-(4-trifluorométhanesulfanyl-phényl)-4,6-diaza-spiro[2.4]heptane-5,7-dione
- la 4-(2-méthylaminosulfonylamino-pyridin)-4-ylméthyl-6-(4-trifluorométhanesulfanyl-phényl)-4,6-diaza-spiro[2.4]heptane-5,7-dione
- la 4-(2-aminosulfonylamino-pyridin)-4-ylméthyl-6-(4-trifluorométhanesulfanyl-phényl)-4,6-diaza-spiro[2.4]heptane-5,7-dione
- la 4-(2-morpholinosulfonylamino-pyridin)-4-ylméthyl-6-(4-trifluorométhanesulfanyl-phényl)-4,6-diaza-spiro[2.4]heptane-5,7-dione
- la 4-(2-diméthylaminosulfonylamino-pyridin)-4-ylmêthyl-6-(4-trifluorométhanesulfanyl-phényl)-4,6-diaza-spiro[2.4]heptane-5,7-dione
- la 4-(2-anilinosulfonylamino-pyridin)-4-ylméthyl-6-(4-trifluorométhanesulfanyl-phényl)-4,6-diaza-spiro[2.4]heptane-5,7-dione
- la 4-(2-phênylsulfonylamino-pyridin)-4-ylméthyl-6-(4-trifluorométhanesulfanyl-phényl)-4,6-diaza-spiro[2.4]heptane-5,7-dione
- la 4-[2-(2-thiényl)sulfonylamino-pyridin]-4-ylméthyl-6-(4-trifluorométhanesulfanyl-phényl)-4,6-diaza-spiro[2.4]heptane-5,7-dione
- la 4-(2-méthylsulfonylamino-pyridin)-4-ylméthyl-6-(4-trifluorométhanesulfanyl-phényl)-4,6-diaza-spiro[2.4]heptane-5,7-dione
- la 4-[2-(4-chlorophényl)sulfonylamino-pyridin]-4-ylméthyl-6-(4-trifluorométhanesulfanyl-phényl)-4,6-diaza-spiro[2.4]heptane-5,7-dione
- la 4-(2-benzylsulfonylamino-pyridin)-4-ylméthyl-6-(4-trifluorométhanesulfanyl-phényl)-4,6-diaza-aspiro[2.4]heptane-5,7-dione
- la 4-(2-hydroxycarbonyl-pyridin)-4-ylméthyl-6-(4-trifluorométhanesulfanyl-phényl)-4,6-diaza-spiro[2.4]heptane-5,7-dione
- la 4-(2-éthoxycarbonyl-pyridin)-4-ylmé-thyl-6-(4-trifluorométhanesulfanyl-phényl)-4,6-diaza-spiro[2.4]heptane-5,7-dione
- la 4-(2-ter-butoxycarbonyl-pyridin)-4-ylméthyl-6-(4-trifluorométhanesulfanyl-phényl)-4,6-diaza-spiro[2.4]heptane-5,7-dione
- la 4-(2-phénoxycarbonyl-pyridin)-4-ylméthyl-6-(4-trifluorométhanesulfanyl-phényl)-4,6-diaza-spiro[2.4]heptane-5,7-dione
- la 4-[2-(4-méthylpipérazinocarbonyl)-pyridin]-4-ylméthyl-6-(4-trifluorométhanesulfanyl-phényl)-4,6-dia2a-spiro[2.4]heptane-5,7-dione
- la 4-[2-(méthylamino)carbonyl-pyridin]-4-ylméthyl-6-(4-trifluorométhanesulfanyl-phényl)-4,6-diaza-spiro[2.4]heptane-5,7-dione
- la 4-[2-(diméthylamino)carbonyl-pyridin]-4-ylméthyl-6-(4-trifluorométhanesulfanyl-phényl)-4,6-diaza-spiro[2.4]heptane-5,7-dione
- la 4-(2-pipéridinocarbonyl-pyridin)-4-ylméthyl-6-(4-trifluorométhanesulfanyl-phényl)-4,6-diaza-spiro[2.4]heptane-5,7-dione .
- la 4-(2-morpholinocarbonyl-pyridin)-4-ylméthyl-6-(4-trifluorométhanesulfanyl-phényl)-4,6-diaza-spiro[2.4]heptane-5,7-dione
- la 4-[2-(4-phénylpipérazinocarbonyl)-pyridin]-4-ylméthyl-6-(4-trifluorométhanesulfanyl-phényl)-4,6-diaza-spiro[2.4]heptane-5,7-dione
- la 4-(2-phénylaminocarbonyl-pyridin)'-4-ylméthyl-6-(4-trifluorométhanesulfanyl-phényl)-4,6-diaza-spiro[2.4]heptane-5,7-dione
- la 4-[2-(méthylphénylamino)carbonyl-pyridin]-4-ylméthyl-6-(4-trifluorométhanesulfanyl-phényl)-4,6-diaza-spiro[2.4]heptane-5,7-dione
- la 4-[2-(4-méthoxyphényl)méthylaminocarbonyl-pyridin]-4-ylméthyl-6-(4-trifluorométhanesulfanyl-phényl)-4,6-diaza-spiro[2.4]heptane-5,7-dione
- la 4-[2-(4-chlorophényl)méthylaminocarbonyl-pyridin]-4-ylméthyl-6-(4-trifluorométhanesulfanyl-phényl)-4,6-diaza-spiro[2.4]heptane-5;7₋dione
- la 4-[2-(4-morpholinylamino)carbonyl-pyridin]-4-ylméthyl-6-(4-trifluorométhanesulfanyl-phényl)-4,6-diaza-spiro[2.4]heptane-5,7-dione
- la 4-(2-aminocarbonyl-pyridin)-4-ylméthyl-6-(4-trifluorométhanesulfanyl-phényl)-4,6-diaza-spiro[2.4]heptane-5,7-dione
- la 4-(2-phénoxy-pyridin)-4-ylméthyl-6-(4-trifluorométhanesulfanyl-phényl)-4,6-diaza-spiro[2.4]heptane-5,7-dione
- la 4-(2-méthoxy-pyridin)-4-ylméthyl-6-(4-trifluorométhanesulfanyl-phényl)-4,6-diaza-spiro[2.4]heptane-5,7-dione
- la 4-(2-éthoxy-pyridin)-4-ylméthyl-6-(4-trifluorométhanesulfanyl-phényl)-4,6-diaza-spiro[2.4]heptane-5,7-dione

- la 4-[2-(1-pyrrolidinyl)éthoxy-pyridin]-4-ylméthyl-6-(4-trifluorométhanesulfanyl-phényl)-4,6-diaza-spiro[2.4]heptane-5,7-dione
- la 4-(2-benzyloxy-pyridin)-4-ylméthyl-6-(4-trifluorométhanesulfanyl-phényl)-4,6-diaza-spiro[2.4]heptane-5,7-dione
- la 4-(2-bénzoyloxy-pyridin)-4-ylméthyl-6-(4-trifluorométhanesulfanyl-phényl)-4,6-diaza-spiro[2.4]heptane-5,7-dione
- la 4-[2-(4,5-dihydro-1H-imidazol-2-yl)-pyridin]-4-ylméthyl-6-(4-trifluorométhanesulfanyl-phényl)-4,6-diaza-spiro[2.4]heptane-5,7-dione
- la 4-[2-(1H-imidazol-2-yl)-pyridin]-4-ylméthyl-6-(4-trifluorométhanesulfanyl-phényl)-4,6-diaza-spiro[2.4]heptane-5,7-dione
- la 4-[2-(1H-tétrazol-5-yl)-pyridin]-4-ylméthyl-6-(4-trifluorométhanesulfanyl-phényl)-4,6-diaza-spiro[2.4]heptane-5,7-dione
- la 4-(2-amidino-pyridin)-4-ylméthyl-6-(4-trifluorométhanesulfanyl-phényl)-4,6-diaza-spiro[2.4]heptane-5;7-dione
- la 4-(2-diméthylaminométhyl-pyridin)-4-ylméthyl-6-(4-trifluorométhanesulfanyl-phényl)-4,6-diaza-spiro[2.4]heptane-5,7-dione
- la 4-(2-aminométhyl-pyridin)-4-ylméthyl-6-(4-trifluorométhanesulfanyl-phényl)-4,6-diaza-spiro[2.4]heptane-5,7-dione
- la 4-(3-méthyl-pyridin)-4-ylméthyl-6-(4-trifluorométhanesulfanyl-phényl)-4,6-diaza-spiro[2.4]heptane-5,7-dione
- la 4-(3-éthyl-pyridin)-4-ylméthyl-6-(4-trifluorométhanesulfanyl-phényl)-4,6-diaza-spiro[2.4]heptane-5,7-dione
- la 4-(3-isopropyl-pyridin)-4-ylméthyl-6-(4-trifluorométhanesulfanyl-phényl)-4,6-diaza-spiro[2.4]heptane-5,7-dione
- la 4-(3-ter-butyl-pyridin)-4-ylméthyl-6-(4-trifluorométhanesulfanyl-phényl)-4,6-diaza-spiro[2.4]heptane-5,7-dione
- la 4-(3-isobutyl-pyridin)-4-ylméthyl-6-(4-trifluorométhanesulfanyl-phényl)-4,6-diaza-spiro[2.4]heptane-5,7-dione
- la 4-(3-cyclohéxyl-pyridin)-4-ylméthyl-6-(4-trifluorométhanesulfanyl-phényl)-4,6-diaza-spiro[2.4]heptane-5,7-dione
- la 4-(3-phényl-pyridin)-4-ylméthyl-6-(4-trifluorométhanesulfanyl-phényl)-4,6-diaza-spiro[2.4]heptane-5,7-dione
- la 4-[3-(4-chlorophényl)-pyridin]-4-ylméthyl-6-(4-trifluorométhanesulfanyl-phényl)-4,6-diaza-spiro[2.4]heptane-5,7-dione
- la 4-[3-(3-chlorophényl)-pyridin]-4-ylméthyl-6-(4-trifluorométhanesulfanyl-phényl)-4,6-diaza-spiro[2.4]heptane-5,7-dione
- la 4-[3-(2-chlorophény)-pyridin]-4-ylméthyl-6-(4-trifluorométhanesulfanyl-phényl)-4,6-diaza-spiro[2.4]heptane-5,7-dione
- la 4-[3-(4-fluorophényl)-pyridin]-4-ylméthyl-6-(4-trifluorométhanesulfanyl-phényl)-4,6-diaza-spiro[2.4]heptane-5,7-dione
- la 4-[3-(4-méthoxyphényl)-pyridin]-4-ylméthyl-6-(4-trifluorométhanesulfanyl-phényl)-4,6-diaza-spiro[2.4]heptane-5,7-dione
- la 4-(3-benzyl-pyridin)-4-ylméthyl-6-(4-trifluorométhanesulfanyl-phényl)-4,6-diaza-spiro[2.4]heptane-5,7-dione
- la 4-(3,2'bipyridin)-4-ylméthyl-6-(4-trifluorométhanesulfanyl-phényl)-4,6-diaza-spiro[2.4]heptane-5,7-dione
- la 4-(3-hydroxyméthyl-pyridin)-4-ylméthyl-6-(4-trifluoromé-thanesulfanyl-phényl)-4,6-diaza-spiro[2.4]heptane-5,7-dione
- la 4-(3-chloro-pyridin)-4-ylméthyl-6-(4-trifluorométhanesulfanyl-phényl)-4,6-diaza-spiro[2.4]heptane-5,7-dione
- la 4-(3-fluoro-pyridin)-4-ylméthyl-6-(4-trifluorométhanesulfanyl-phényl)-4,6-diaza-spiro[2.4]heptane-5,7-dione
- la 4-(3-bromo-pyridin)-4-ylmëthyl-6-(4-trifluorométhanesulfanyl-phényl)-4,6-diaza-spiro[2.4]heptane-5,7-dione
- la 4-(3-amino-pyridin)-4-ylméthyl-6-(4-trifluorométhanesulfanyl-phényl)-4,6-diaza₋spiro[2.4]heptane-5,7-dione
- la 4-(3-méthylamino-pyridin)-4-ylmé-thyl-6-(4-trifluorométhanesulfanyl-phényl)-4,6-diaza-spiro[2.4]heptane-5,7-dione
- la 4-(3-éthylamino-pyridin)-4-ylméthyl-6-(4-trifluorométhanesulfanyl-phényl)-4,6-diaza-spiro[2.4]heptane-5,7-dione
- la 4-(3-isopropylamino-pyridin)-4-ylméthyl-6-(4-trifluorométhanesulfanyl-phényl)-4,6-diaza-spiro[2.4]heptane-5,7-dione
- la 4-(3-phénylamino-pyridin)-4-ylméthyl-6-(4-trifluorométhanesulfanyl-phényl)-4,6-diaza-spiro[2.4]heptane-5,7-dione
- la 4-(3-cyclohéxylamino-pyridin)-4-ylméthyl-6-(4-trifluorométhanesulfanyl-phényl)-4,6-diaza-spiro[2.4]heptane-5,7-dione
- la 4-(3-diéthylamino-pyridin)-4-ylméthy-1-6-(4-trifluorométhanesulfanyl-phényl)-4,6-diaza-spiro[2.4]heptane-5,7-dione
- la 4-(3-N-cyclohéxyl-N-méthylamino-pyridin)-4-ylméthyl-6-(4-trifluorométhanesulfanyl-phényl)-4,6-diaza-spiro[2.4]heptane-5,7-dione
- la 4-(3-pipéridino-pyridin)-4-ylmêthyl-6-(4-trifluorométhanesulfanyl-phényl)-4,6-diaza-spiro[2.4]heptane-5,7-dione
- la 4-(3-morpholino-pyridin)-4-ylméthyl-6-(4-trifluorométhanesulfanyl-phényl)-4,6-diaza-spiro[2.4]heptane-5,7-dione
- la 4-[3-(4-méthylpipérazino)-pyridin]-4-ylméthyl-6-(4-trifluorométhanesulfanyl-phényl)-4,6-diaza-spiro[2.4]heptane-5,7-dione
- la 4-[3-(1H-pyrazol-1yl)-pyridin]-4-ylméthyl-6-(4-trifluorométhanesulfanyl-phényl)-4,6-diaza-spiro[2.4]heptane-5,7-dione
- la 4-[3-(2-benzoylhydrazino)-pyridin]-4-ylméthyl-6-(4-trifluorométhanesulfanyl-phényl)-4,6-diaza-spiro[2.4]heptane-5,7-dione
- la 4-(3-benzoylamino-pyridin)-4-ylméthyl-6-(4-trifluorométhanesulfanyl-phényl)-4,6-diaza-spiro[2.4]heptane-5,7-dione
- la 4-[3-(4-chlorobenzoylamino)-pyridin]-4-ylméthyl-6-(4-trifluorométhanesulfanyl-phényl)-4,6-diaza-spiro[2.4]heptane-5,7-dione
- la 4-[3-(3-chlorobenzoylamino)-pyridin]-4-ylméthyl-6-(4-trifluorométhanesulfanyl-phényl)-4,6-diaza-spiro[2.4]heptane-5,7-dione
- la 4-[3-(2-chlorobenzoylamino)-pyridin]-4-ylméthyl-6-(4-trifluorométhanesulfanyl-phényl)-4,6-diaza-spiro[2.4]heptane-5,7-dione
- la 4-[3-(4-fluorobenzoylamino)-pyridin]-4-ylméthyl-6-(4-trifluorométhanesulfanyl-phényl)-4,6-diaza-spiro[2.4]heptane-5,7-dione
- la 4-[3-(4-méthoxybenzoylamino)-pyridin]-4-ylméthyl-6-(4-trifluorométhanesulfanyl-phényl)-4,6-diaza-spiro[2.4]heptane-5,7-dione
- la 4-(3-acétamido-pyridin)-4-ylméthyl-6-(4-trifluorométhanesulfanyl-phényl)-4,6-diaza-spiro[2.4]heptane-5,7-dione
- la 4-(3-phénylacétamido-pyridin)-4-ylméthyl-6-(4-trifluorométhanesulfanyl-phényl)-4,6-diaza-spiro[2.4]heptane-5,7-dione
- la 4-[3-(4-méthylpipérazino)acétamido-pyridinl]-4-ylméthyl-6-(4-trifluorométhanesulfanyl-phényl)-4,6-diaza-spiro[2.4]heptane-5,7-dione
- la 4-(3-cyclohéxanylcarboxamido-pyridin)-4-ylméthyl-6-(4-trifluorométhanesulfanyl-phényl) -4,6-diaza-spiro[2.4]heptane-5,7-dione
- la 4-[3-(1-méthyl-4-pipéridinyl)carbonylamino-pyridin]-4-ylméthyl-6-(4-trifluorométhanesulfanyl-phényl)-4,6-diaza-spiro[2.4]heptane-5,7-dione
- la 4-[3-(2-furanylcarbonyl)amino-pyridin]-4-ylméthyl-6-(4-trifluorométhanesulfanyl-phényl)-4,6-diaza-spiro[2.4]heptane-5,7-dione
- la 4-(3-éthoxycarbonylamino-pyridin)-4-ylméthyl-6-(4-trifluorométhanesulfanyl-phényl)-4,6-diaza-spiro[2.4]heptane-5,7-dione
- la 4-(3-isopropoxycarbonylamino-pyridin)-4-ylméthyl-6-(4-trifluorométhanesulfanyl-phényl)-4,6-diaza-spiro[2.4]heptane-5,7-dione
- la 4-(3-butoxycarbonylamino-pyridin)-4-ylméthyl-6-(4-trifluorométhanesulfanyl-phényl)-4,6-diaza-spiro[2.4]heptane-5,7-dione
- la 4-[3-(2-diéthylaminoéthoxycarbonylamino)-pyridin]-4-ylméthyl-6-(4-trifluorométhanesulfanyl-phényl)-4,6-diaza-spiro[2.4]heptane-5,7-dione
- la 4-(3-phénoxycarbonylamino-pyridin)-4-ylméthyl-6-(4-trifluorométhanesulfanyl-phényl)-4,6-diaza-spiro[2.4]heptane-5,7-dione
- la 4-[3-(4-chloroanilino)carbonylamino-pyridin]-4-ylméthyl)-6-(4-trifluorométhanesulfanyl-phényl)-4,6-diaza-spiro[2.4]heptane-5,7-dione
- la 4-(3-anilinocarbonylamino-pyridin)-4-ylméthyl-6-(4-trifluorométhanesulfanyl-phényl)-4,6-diaza-spiro[2.4]heptane-5,7-dione
- la 4-(3-benzylaminocarbonylamino-pyridin)-4-ylméthyl-6-(4-trifluorométhanesulfanyl-phényl)-4,6-diaza-spiro[2.4]heptane-5,7-dione
- la 4-[3-(2-pyridylamino)carbonylamino-pyridin]-4-ylméthyl-6-(4-trifluorométhanesulfanyl-phényl)-4,6-diaza-spiro[2.4]heptane-5,7-dione
- la 4-(3-uréido-pyridin)-4-ylméthyl-6-(4-trifluorométhanesulfanyl-phényl)-4,6-diaza-spiro[2.4]heptane-5,7-dione
- la 4-(3-benzylaminosulfonylamino-pyridin)-4-ylméthyl-6-(4-trifluorométhanesulfanyl-phényl)-4,6-diaza-spiro[2.4]heptane-5,7-dione
- la 4-(3-méthylaminosulfonylamino-pyridin)-4-ylméthyl-6-(4-trifluorométhanesulfanyl-phényl)-4,6-diaza-spiro[2.4]heptane-5,7-dione
- la 4-(3-aminosulfonylamino-pyridin)-4-ylméthyl-6-(4-trifluorométhanesulfanyl-phényl)-4,6-diaza-spiro[2.4]heptane-5,7-dione
- la 4-(3-morpholinosulfonylamino-pyridin)-4-ylméthyl-6-(4-trifluorométhanesulfanyl-phényl)-4,6-diaza-spiro[2.4]heptane-5,7-dione
- la 4-(3-diméthylaminosulfonylamino-pyridin)-4-ylméthyl-6-(4-trifluorométhanesulfanyl-phényl)-4,6-diaza-spiro[2.4]heptane-5,7-dione
- la 4-(3-anilinosulfonylamino-pyridin)-4-ylméthyl-6-(4-trifluorométhanesulfanyl-phényl)-4,6-diaza-spiro[2.4]heptane-5,7-dione
- la 4-(3-phénylsulfonylamino-pyridin)-4-ylméthyl-6-(4-trifluorométhanesulfanyl-phényl)-4,6-diaza-spiro[2.4]heptane-5,7-dione
- la 4-[3-(2-thiényl)sulfonylemino-pyridin]-4-ylméthyl-6-(4-trifluorométhanesulfanyl-phényl)-4,6-diaza-spiro[2.4]heptane-5,7-dione
- la 4-(3-mét-hylsulfonylamino-pyridin)-4-ylméthyl-6-(4-trifluorométhanesulfanyl-phényl)-4,6-diaza-spiro[2.4]heptane-5,7-dione
- la 4-[3-(4-chlorophényl)sulfonylamino-pyridin]-4-ylméthyl-6-(4-trifluorométhanesulfanyl-phényl)-4,6-diaza-spiro[2.4]heptane-5,7-dione
- la 4-(3-benzylsulfonylamino-pyridin)-4-ylméthyl-6-(4-trifluorométhanesulfanyl-phényl)-4,6-diaza-spiro[2.4]heptane-5,7-dione
- la 4-(3-hydroxycarbonyl-pyridin)-4-ylméthyl-6-(4-trifluorométhanesulfanyl-phényl)-4,6-diaza-spiro[2.4]heptane-5,7-dione
- la 4-(3-éthoxycarbonyl-pyridin)-4-ylméthyl-6-(4-trifluorométhanésulfanyl-phényl)-4,6-diaza-spiro[2.4]heptane-5,7-dione
- la 4-(3-ter-butoxycarbonyl-pyridin)-4-ylméthyl-6-(4-trifluorométhanesulfanyl-phényl)-4,6-diaza-spiro[2.4]heptane-5,7-dione
- la 4-(3-phénoxycarbonyl-pyridin)-4-ylméthyl-6-(4-trifluorométhanesulfanyl-phényi)-4,6-diaza-spiro[2.4]heptane-5,7-dione
- la 4-[3-(4-méthylpipérazinocarbonyl)-pyridin]-4-ylméthyl-6-(4-trifluorométhanesulfanyl-phényl)-4,6-diaza-spiro[2.4]heptane-5,7-dione
- la 4-[3-(méthylamino)carbonyl-pyridin]-4-ylméthyl-6-(4-trifluorométhanesulfanyl-phényl)-4,6-diaza-spiro[2.4]heptane-5,7-dione
- la 4-[3-(diméthylamino)carbonyl-pyridin]-4-ylméthyl-6-(4-trifluorométhanesulfanyl-phényl)-4,6-diaza-spiro[2.4]heptane-5,7-dione
- la 4-(3-pipéridinocarbonyl-pyridin)-4-ylméthyl-6-(4-trifluorométhanesulfanyl-phényl)-4,6-diaza-spiro[2.4]heptane-5,7-dione
- la 4-(3-morpholinocarbonyl-pyridin)-4-ylméthyl-6-(4-trifluorométhanesulfanyl-phényl)-4,6-diaza-spiro[2.4]heptane-5,7-dione
- la 4-[3-(4-phénylpipérazinocarbonyl)-pyridin]-4-ylméthyl-6-(4-trifluorométhanesulfanyl-phényl)-4,6-diaza-spiro[2.4]heptane-5,7-dione
- la 4-(3-phénylaminocarbonyl-pyridin)-4-ylméthyl-6-(4-trifluorométhanesulfanyl-phényl)-4,6-diaza-spiro[2.4]heptane-5,7-dione
- la 4-[3-(méthylphénylamino)carbonyl-pyridin]-4-ylméthyl-6-(4-trifluorométhanesulfanyl-phényl)-4,6-diaza-spiro[2.4]heptane-5,7-dione
- la 4-[3-(4-méthoxyphényl)méthylaminocarbonyl-pyridin]-4-ylméthyl-6-(4-trifluorométhanesulfanyl-phényl)-4,6-diaza-spiro[2.4]heptane-5,7-dione
- la 4-[3-(4-chlorophényl)méthylaminocarbonyl-pyridin]-4-ylméthyl-6-(4-trifluorométhanesulfanyl-phényl)-4,6-diaza-spiro[2.4]heptane-5,7-dione
- la 4-[3-(4-morpholinylamino)carbonyl-pyridin]-4-ylméthyl-6-(4-trifluorométhanesulfanyl-phényl)-4,6-diaza-spiro[2.4]heptane-5,7-dione
- la 4-(3-aminocarbonyl-pyridin)-4-ylméthyl-6-(4-trifluorométhanesulfanyl-phényi)-4,6-diaza-spiro[2.4]heptane-5,7-dione
- la 4-(3-phénox-y-pyridin)-4-ylméthyl-6-(4-trifluorométhanesulfanyl-phényl)-4,6-diaza-spiro[2.4]heptane-5,7-dione
- la 4-(3-méthoxy-pyridin)-4-ylméthyl-6-(4-trifluorométhanesulfanyl-phényl)-4,6-diaza-spiro[2.4]heptane-5,7-dione
- la 4-(3-éthoxy-pyridin)-4-ylméthyl-6-(4-trifluorométhanesulfanyl-phényl)-4,6-diaza-spiro[2.4]heptane-5,7-dione
- la 4-[3-(1-pyrrolidinyl)éthoxy-pyridin]-4-ylméthyl-6-(4-trifluorométhanesuilfanyl-phényl)-4,6-diaza-spiro[2.4]heptane-5,7-dione
- la 4-(3-benzyloxy-pyridin)-4-ylméthyl-6-(4-trifluorométhanesulfanyl-phényl)-4,6-diaza-spiro[2.4]heptane-5,7-dione
- la 4-(3-benzoyloxy-pyridin)-4-ylméthyl-6-(4-trifluorométhanesulfanyl-phényl)-4,6-diaza-spiro[2.4]heptane-5,7-, dione
- la 4-[3-(4,5-dihydro-1H-imidazol-2-yl)-pyridin]-4-ylméthyl-6-(4-trifluorométhanesulfanyl-phényl)-4,6-diaza-spiro[2.4]heptane-5,7-dione
- la 4-[3-(1H-imidazol-2-yl)-pyridin]-4-ylméthyl-6-(4-trifluorométhanesulfanyl-phényl)-4,6-diaza-spiro[2.4]heptane-5,7-dione
- la 4-[3-(1H-tétrazol-5-yl)-pyridin]-4-ylméthyl-6-(4-trifluorométhanesulfanyl-phényl)-4,6-diaza-spiro[2.4]heptane-5,7-dione
- la 4-(3-amidino-pyridin)-4-ylméthyl-6-(4-trifluorométhanesulfanyl-phényl)-4,6-diaza-spiro[2.4]heptane-5,7-dione
- la 4-(3-diméthylaminométhyl-pyridin)-4-ylméthyl-6-(4-trifluorométhanesulfanyl-phényl)-4,6-diaza-spiro[2.4]heptane-5,7-dione
- la 4-(3-aminométhyl-pyridin)-4-ylméthyl-6-(4-trifluorométhanesulfanyl-phényl)-4,6-diaza-spiro[2.4]heptane-5,7-dione
- la 5-méthyl-1-(2-méthyl-pyridin-4-ylméthyl)-3-(4-trifluorométhanesulfanyl-phényl)-imidazolidine-2,4-dione
- la 5-méthyl-1-(2-éthyl-pyridin-4-ylméthyl)-3-(4-trifluorométhanesulfanyl-phényl)-imidazolidine-2,4-dione
- la 5-méthyl-1-(2-isopropyl-pyridin-4-ylméthyl)-3-(4-trifluorométhanesulfanyl-phényi)-imidazolidine-2,4-dione
- la 5-méthyl-1- (2-ter-butyl-pyridin-4-ylméthyl)-3-(4-trifluorométhanesulfanyl-phényl) -imidazolidiné-2,4-dione
- la 5-méthyl-1-(2-isobutyl-pyridin-4-ylméthyl)-3-(4-trifluorométhanesulfanyl-phényl)-imidazolidine-2,4-dione
- la 5-méthyl-1- (2-cyrclohéityl-pyridin-4-ylméthyl)-3-(4-trifluorométhanesulfan-yl-phényl)-imidazolidine-2,4-dione
- la 5-méthyl-1-(2-phényl-pyridin-4-ylméthyl)-3-(4-trifluorométhanesulfanyl-phényl)-imidazolidine-2,4-dione
- la 5-méthyl-1-[2-(4-chlorophényl)-pyridin-4-ylméthyl]-3-(4-trifluorométhanesulfanyl-phényl)-imidazolidine-2,4-dione
- la 5-méthyl-1-[2-(3-chlorophényl)-pyridin-4-ylméthyl]-3-(4-trifluorométhanesulfanyl-phényl)-imidazolidine-2,4-dione
- la 5-méthyl-1-[2-(2-chlorophényl)-pyridin-4-ylméthyl]-3-(4-trifluorométhanesulfanyl-phényl)-imidazolidine-2,4-dione
- la 5-méthyl-1-[2-(4-fluorophényl)-pyridin-4-ylméthyl]-3-(4-trifluorométhanesulfanyl-phényl)-imidazolidine-2,4-dione
- la 5-méthyl-1-[2-(4-méthoxyphényl)-pyridin-4-ylméthyl]-3-(4-trifluorométhanesulfanyl-phényl)-imidazolidine-2,4-dione
- la 5-méthyl-1-(2-benzyl-pyridin-4-ylméthyl)-3-(4-trifluorométhanesulfanyl-phényl)-imidazolidine-2,4-dione
- la 5-méthyl-1-(2,2'bipyridin-4-ylméthyl)-3-(4-trifluorométhanesulfanyl-phényl)-imidazolidine-2,4-dione
- la 5-méthyl-1-(2-hydroxyméthyl-pyridin-4-ylméthyl)-3-(4-trifluorométhanesulfanyl-phényl)-imidazolidine-2,4-dione
- la 5-méthyl-1-(2-chloro-pyridin-4-ylméthyl)-3-(4-trifluorométhanesulfanyl-phényl)-imidazolidine-2,4-dione
- la 5-méthyl-1-(2-fluoro-pyridin-4-ylméthyl)-3-(4-trifluorométhanesulfanyl-phényl)-imidazolidine-2,4-dione
- la 5-méthyl-1-(2-bromo-pyridin-4-ylméthyl)-3-(4-trifluorométhanesulfanyl-phényl) -imidazolidine-2,4-dione
- la 5-méthyl-1-(2-amino-pyridin-4-ylméthyl)-3-(4-trifluorométhanesulfanyl-phényl)-imidazolidine-2,4-dione
- la 5-méthyl-1-(2-méthylamino-pyridin-4-ylméthyl)-3-(4-trifluorométhanesulfanyl-phényl)-imidazolidine-2,4-dione
- la 5-méthyl-1-(2-éthylamino-pyridin-4-ylméthyl)-3-(4-trifluorométhanesulfanyl-phényl) -imidazolidine-2,4-dione
- la 5-méthyl-1-(2-isopropylamino-pyridin-4-ylméthyl)-3-(4-trifluorométhanesulfanyl-phényl)-imidazolidine-2,4-dione
- la 5-méthyl-1-(2-phénylamino-pyridin-4-ylméthyl)-3-(4-trifluorométhanesulfanyl-phényl)-imidazolidine-2,4-dione
- la 5-méthyl-1-(2-cyclohéxylamino-pyridin-4-ylméthyl)-3-(4-trifluorométhanesulfanyl-phényl)-imidazolidine-2,4-dione
- la 5-méthyl-1-(2-diéthylamino-pyridin-4-ylméthyl)-3-(4-trifluorométhanesulfanyl-phényl).-imidazolidine-2,4-dione
- la 5-méthyl-1-(2-N-cyclohéxyl-N-méthylamino-pyridin-4-ylméthyl)-3-(4-trifluorométhanesulfanyl-phényl)-imidazolidine-2,4-dione
- la 5-méthyl-1-(2-pipéridino-pyridin-4-ylméthyl)-3-(4-trifluorométhanesulfanyl-phényl)-imidazolidine-2,4-dione
- la 5-méthyl-1-(2-morpholino-pyridin-4-ylméthyl)-3-(4-trifluorométhanesulfanyl-phényl)-imidazolidine-2,4-dione
- la 5-méthyl-1-[2-(4-méthylpipérazino)-pyridin-4-ylméthyl]-3-(4-trifluorométhanesulanyl-phényl)-imidazolidine-2,4-dione
- la 5-méthyl-1-[2-(1H-pyrazol-1yl)-pyridin-4-ylméthyl]-3-(4-trifluorométhanesulfanyl-phényl)-imidazolidine-2,4-dione
- la 5-méthyl-1-[2-(2-benzoylhydrazino)-pyridin-4-ylméthyl]-3-(4-trifluorométhanesulfanyl-phényl)-imidazolidine-2,4-dione
- la 5-méthyl-1-(2-benzoylamino-pyridin-4-ylméthyl)-3-(4-trifluorométhanesulfanyl-phényl)-imidazolidine-2,4-dione
- la 5-méthyl-1-[2-(4-chlorobenzoylamino)-pyridin-4-ylméthyl]-3-(4-trifluorométhanesulfanyl-phényl)-imidazolidine-2,4-dione
- la 5-méthyl-1-[2-(3-chlorobenzoylamino)-pyridin-4-ylméthyl]-3-(4-trifluorométhanesulfanyl-phényl)-imidazolidine-2,4-dione
- la 5-méthyl-1-[2-(2-chlorobenzoylamino)-pyridin-4-ylméthyl]-3-(4-trifluorométhanesulfanyl-phényl)-imidazolidine-2,4-dione
- la 5-méthyl-1-[2-(4-fluorobenzoylamino)-pyridin-4-ylméthyl]-3-(4-trifluorométhanesulfanyl-phényl)-imidazolidine-2,4-dione
- la 5-méthyl-1-[2-(4-méthoxybenzoylàmino)-pyridin-4-ylméthyl]-3-(4-trifluoraméthanesulfanyl-phényl)-imidazolidine-2,4-dione
- la 5-méthyl-1-(2-acétamido-pyridin-4-ylméthyl)-3-(4-trifluorométhanesulfanyl-phényl)-imidazolidine-2,4-dione
- la 5-méthyl-1-(2-phénylacétamido-pyridin-4-ylméthyl)-3-(4-trifluorométhanesulfanyl-phé-nyl)-imidazolidine-2,4-dione
- la 5-méthyl-1-[2-(4-méthylpipérazino)acétamido-pyridin-4-ylméthyl]-3-(4-trifluorométhanesulfanyl-phényl)-imidazolidine-2,4-dione
- la 5-méthyl-1-(2-cyclohéxanylcarboxamido-pyridin-4-ylméthyl)-3-(4-trifluorométhanesulfanyl-phényl)-imidazolidine-2,4-dione
- la 5-méthyl-1-[2-(1-méthyl-4-pipéridinyl)carbonylamino-pyridin-4-ylméthyl]-3-(4-trifluorométhanesulfanyl-phényl)-imidazolidine-2,4-dione
- la 5-méthyl-1-[2-(2-furanylcarbonyl)amino-pyridin-4-ylméthyl]-3-(4-trifluorométhanesulfanyl-phényl)-imidazolidine-2,4-dione
- la 5-méthyl-1-(2-éthoxycarbonylamino-pyridin-4-ylméthyl)-3-(4-trifluorométhanesulfanyl-phényl)-imidazolidine-2,4-dione
- la 5-méthyl-1-(2-isopropoxycarbonylamino-pyridin-4-ylméthyl)-3-(4-trifluorométhanesulfanyl-phényl)-imidazolidine-2,4-dione
- la 5-méthyl-1-(2-butoxycarbonylamino-pyridin-4-ylméthyl)-3-(4-trifluorométhanesulfanyl-phényl)-imidazolidine-2,4-dione
- la 5-méthyl-1-[2-(2-diéthylaminoéthoxycarbonylamino)-pyridin-4-ylméthyl]-3-(4-trifluorométhanesulfanyl-phényl)-imidazolidine-2,4-dione
- la 5-méthyl-1-(2-phénoxycarbonylamino-pyridin-4-ylméthyl)-3-(4-trifluorométhanesulfanyl-phényl)-imidazolidine-2,4-dione
- la 5-méthyl-1-[2-(4-chloroanilino)carbonylamino-pyridin-4-ylméthyl]-3-(4-trifluorométhanesulfanyl-phényl)-imidazolidine-2,4-dione
- la 5-méthyl-1-(2-anilinocarbonylamino-pyridin-4-ylméthyl)-3-(4-trifluorométhanesulfanyl-phényl)-imidazolidine-2,4-dione
- la 5-méthyl-1-(2-benzylaminocarbonylamino-pyridin-4-ylméthyl)-3-(4-trifluorométhanesulfanyl-phényl)-imidazolidine-2,4-dione
- la 5-méthyl-1-[2-(2-pyridylamino)carbonylamino-pyridin-4-ylméthyl]-3-(4-trifluorométhanesulfanyl-phényl)-imidazolidine-2,4-dione
- la 5-méthyl-1-(2-uréido-pyridin-4-ylméthyl)-3-(4-trifluorométhanesulfanyl-phényl)-imidazolidine-2,4-dione
- la 5-méthyl-1-(2-benzylaminosulfonylamino-pyridin-4-ylméthyl)-3-(4-trifluorométhanesulfanyl-phényl)-imidazolidine-2,4-dione
- la 5-méthyl-1-(2-méthylaminosulfonylamino-pyridin-4-ylméthyl)-3-(4-trifluorométhanesulfanyl-phényl)-imidazolidine-2,4-dione
- la 5-méthyl-1-(2-aminosulfonylamino-pyridin-4-ylméthyl)-3-(4-trifluorométhanesulfanyl-phényl)-imidazolidine-2,4-dione
- la 5-méthyl-1-(2-morpholinosulfonylamino-pyridin-4-ylméthyl)-3-(4-trifluorométhanesulfanyl-phényl)-imidazolidine-2,4-dione
- la 5-méthyl-1-(2-diméthylaminosulfonylamino-pyridin-4-ylméthyl)-3-(4-trifluorométhanesulfanyl-phényl)-imidazolidine-2,4-dione
- la 5-méthyl-1-(2-anilinosulfonylamino-pyridin-4-ylméthyl)-3-(4-trifluorométhanesulfanyl-phényl)-imidazolidine-2,4-dione
- la 5-méthyl-1-(2-phénylsulfonylamino-pyridin-4-y-lméthyl)-3-(4-trifluorométhanesulfanyl-phényl)-imidazolidine-2,4-dione
- la 5-méthyl-1-[2-(2-thiényl)sulfonylamino-pyridin-4-ylméthyl]-3-(4-trifluorométhanesulfanyl-phényl)-imidazolidine-2,4-dione
- la 5-méthyl-1-(2-méthylsulfonylamino-pyridin-4-ylméthyl)-3-(4-trifluorométhanesulfanyl-phényl)-imidazolidine-2,4-dione
- la 5-méthyl-1-[2-(4-chlorophényl)sulfonylamino-pyridin-4-ylméthyl]-3-(4-trifluorométhanesulfanyl-phényl)-imidazolidine-2,4-dione
- la 5-méthyl-1-(2-benzylsulfonylamino-pyridin-4-ylméthyl)-3-(4-trifluorométhanesulfanyl-phényl)-imidazolidine-2,4-dione
- la 5-méthyl-1-(2-hydroxycarbonyl-pyridin-4-ylméthyl)-3-(4-trifluorométhanesulfanyl-phényl)-imidazolidine-2,4-dione
- la 5-méthyl-1-(2-éthoxycarbonyl-pyridin-4-ylméthyl)-3-(4-trifluorométhanesulfanyl-phényl)-imidazolidine-2,4-dione
- la 5-méthyl-1-(2-ter-butoxycarbonyl-pyridin-4-ylméthyl)-3-(4-trifluorométhanesulfanyl-phényl)-imidazolidine-2,4-dione
- la 5-méthyl-1-(2-phénoxycarbonyl-pyridin-4-ylméthyl)-3-(4-trifluorométhanesulfanyl-phényl)-imidazolidine-2,4-dione
- la 5-méthyl-1-[2-(4-méthylpipérazinocarbonyl)-pyridin-4-ylméthyl]-3-(4-trifluorométhanesulfanyl-phényl)-imidazolidine-2,4-dione
- la 5-méthyl-1-[2-(méthylamino)carbonyl-pyridin-4-ylméthyl]-3-(4-trifluorométhanesulfanyl-phényl)-imidazolidine-2,4-dione
- la 5-méthyl-1-[2-(diméthylamino)carbonyl-pyridin-4-ylméthyl]-3-(4-trifluorométhanesulfanyl-phényl)-imidazolidine-2,4-dione
- la 5-méthyl-1-(2-pipéridinocarbonyl-pyridin-4-ylméthyl)-3-(4-trifluorométhanesulfany-1-phényl)-imidazolidine-2,4-dione
- la 5-méthyl-1-(2-morpholinocarbonyl-pyridin-4-ylméthyl)-3-(4-trifluorométhanesulfanyl-phényl)-imidazolidine-2,4-dione
- la 5-méthyl-1-[2-(4-phénylpipérazinocarbonyl)-pyridin-4-ylméthyl]-3-(4-trifluorométhanesulfanyl-phényl)-imidazolidine-2,4-dione
- la 5-méthyl-1-(2-phénylaminocarbonyl-pyridin-4-ylméthyl)-3-(4-trifluorométhanesulfanyl-phényl)-imidazolidine-2,4-dione
- la 5-méthyl-1-[2-(méthylphénylamino)carbonyl-pyridin-4-ylméthyl]-3-(4-trifluorométhanesulfanyl-phényl)-imidazolidine-2,4-dione
- la 5-méthyl-1-[2-(4-méthoxyphényl)méthylaminocarbonyl-pyridin-4-ylméthyl]-3-(4-trifluorométhanesulfanyl-phényl)-imidazolidine-2,4-dione
- la 5-méthyl-1-[2-(4-chlorophényl)méthylaminocarbonyl-pyridin-4-ylméthyl]-3-(4-trifluorométhanesulfanyl-phényl)-imidazolidine-2,4-dione
- la 5-méthyl-1-[2-(4-morpholinylamino)carbonyl-pyridin-4-ylméthyl]-3-(4-trifluorométhanesulfanyl-phényl)-imidazolidine-2,4-dione
- la 5-méthyl-1-(2-aminocarbonyl-pyridin-4-ylméthyl)-3-(4-trifluorométhanesulfanyl-phényl)-imidazolidine-2,4-dione
- la 5-méthyl-1-(2-phénoxy-pyridin-4-ylméthyl)-3-(4-trifluorométhanesulfanyl-phényl)-imidazolidine-2,4-dione
- la 5-méthyl-1-(2-méthoxy-pyridin-4-ylméthyl)-3-(4-trifluorométhanesulfanyl-phényl)-imidazolidine-2,4-dione
- la 5-méthyl-1-(2-éthoxy-pyridin-4-ylméthyl)-3-(4-trifluorométhanesulfanyl-phényl)-imidazolidine-2,4-dione
- la 5-méthyl-1-[2-(1-pyrrolidinyl)éthoxy-pyridin-4-ylméthyl]-3-(4-trifluorométhanesulfanyl-phényl)-imidazolidine-2,4-dione
- la 5-méthyl-1-(2-benzyloxy-pyridin-4-ylméthyl)-3-(4-trifluorométhanesulfanyl-phényl)-imidazolidine-2,4-dione
- la 5-méthyl-1-(2-benzoyloxy-pyridin-4-y-lméthyl)-3-(4-trifluorométhanesulfanyl-phényl)-imidazolidine-2,4-dione
- la 5-méthyl-1-[2-(4,5-dihydro-1H-imidazol-2-yl)-pyridin-4-ylméthyl]-3-(4-trifluorométhanesulfanyl-phényl)-imidazolidine-2,4-dione
- la 5-méthyl-1-[2-(1H-imidazol-2-yl)-pyridin-4-ylméthyl]-3-(4-trifluorométhanesulfanyl-phényl)-imidazolidine-2,4-dione
- la 5-méthyl-1-[2-(1H-tétrazol-5-yl)-pyridin-4-ylméthyl]-3-(4-trifluorométhanesulfanyl-phényl)-imidazolidine-2,4-dione
- la 5-méthyl-1-(2-amidino-pyridin-4-ylméthyl)-3-(4-trifluorométhanesulfanyl-phényl)-imidazolidine-2,4-dione
- la 5-méthyl-1-(2-diméthylaminométhyl-pyridin-4-ylméthyl)-3-(4-trifluorométhanésulfanyl-phényl)-imidazolidine-2,4-dione
- la 5-méthyl-1-(2-aminométhyl-pyridin-4-ylméthyl)-3-(4-trifluorométhanesulfanyl-phényl)-imidazolidine-2,4-dione
- la 5-méthyl-1-(3-méthyl-pyridin-4-ylméthyl)-3-(4-trifluorométhanesulfanyl-phényl)-imidazolidine-2,4-dione
- la 5-méthyl-1-(3-éthyl-pyridin-4-ylméthyl)-3-(4-trifluorométhanesulfanyl-phényl)-imidazolidine-2,4-dione
- la 5-méthyl-1-(3-isopropyl-pyridin-4-ylméthyl)-3-(4-trifluorométhanesulfanyl-phényl)-imidazolidine-2,4-dione
- la 5-méthyl-1-(3-ter-butyl-pyridin-4-ylméthyl)-3-(4-trifluorométhanesulfanyl-phényl)-imidazolidine-2,4-dione
- la 5-méthyl-1-(3-isobutyl-pyridin-4-ylméthyl)-3-(4-trifluorométhanesulfanyl-phényl)-imidazolidine-2,4-dione
- la 5-méthyl-1-(3-cyclohéxyl-pyridin-4-ylméthyl)-3-(4-trifluorométhanesulfanyl-phényl)-imidazolidine-2,4-dione
- la 5-méthyl-1-(3-phényl-pyridin-4-ylméthyl)-3-(4-trifluorométhanesulfanyl-phényl)-imidazolidine-2,4-dione
- la 5-méthyl-1-[3-(4-chlorophényl)-pyridin-4-ylméthyl]-3-(4-trifluorométhanesulfanyl-phényl)-imidazolidine-2,4-dione
- la 5-méthyl-1-[3-(3-chlorophényl)-pjrridin-4-ylméthyl] - 3-(4-trifluorométhanesulfanyl-phényl)-imidazolidine-2,4-dione
- la 5-méthyl-1-[3-(2-chlorophényl)-pyridin-4-ylméthyl]-3-(4-trifluorométhanesulfanyl-phényl)-imidazolidine-2,4-dione
- la 5-méthyl-1-[3-(4-fluorophényl)-pyridin-4-ylméthyl]-3-(4-trifluorométhanesulfanyl-phényl)-imidazolidine-2,4-dione
- la 5-méthyl-1-[3-(4-méthoxyphényl)-pyridin-4-ylméthyl]-3-(4-trifluorométhanesulfanyl-phényl)-imidazolidine-2,4-dione
- la 5-méthyl-1-(3-benzyl-pyridin-4-ylméthyl)-3-(4-trifluorométhanesulfanyl-phényl)-imidazolidine-2,4-dione
- la 5-méthyl-1-(3,2'bipyridin-4-ylméthyl)-3-(4-trifluorométhanesulfanyl-phényl)-imidazolidine-2,4-dione
- la 5-méthyl-1-(3-hydroxyméthyl-pyridin-4-ylméthyl)-3-(4-trifluorométhanesulfanyl-phényl)-imidazolidine-2,4-dione
- la 5-méthyl-1-(3-chloro-pyridin-4-ylméthyl)-3-(4-trifluorométhanesulfanyl-phényl)-imidazolidine-2,4-dione
- la 5-méthyl-1-(3-fluoro-pyridin-4-ylméthyl)-3-(4-trifluorométhanesulfanyl-phényl)-imidazolidine-2,4-dione
- la 5-méthyl-1-(3-bromo-pyridin-4-ylméthyl)-3-(4-trifluorométhanesulfanyl-phényl)-imidazolidine-2,4-dione
- la 5-méthyl-1-(3-amino-pyridin-4-ylméthyl)-3-(4-trifluorométhanesulfanyl-phényl)-imidazolidine-2,4-dione
- la 5-méthyl-1-(3-méthylamino-pyridin-4-ylméthyl)-3-(4-trifluorométhanesulfanyl-phényl)-imidazolidine-2,4-dione
- la 5-méthyl-1-(3-éthylamino-pyridin-4-ylméthyl)-3-(4-trifluorométhanesulfanyl-phényl)-imidazolidine-2,4-dione
- la 5-méthyl-1-(3-isopropylamino-pyridin-4-ylméthyl)-3-(4-trifluorométhanesulfanyl-phényl)-imidazolidine-2,4-dione
- la 5-méthyl-1-(3-phénylamino-pyridin-4-ylméthyl)-3-(4-trifluorométhanesulfanyl-phényl)-imidazolidine-2,4-dione
- la 5-méthyl-1-(3-cjrclohéxylamino-pyrid,in-4-ylméthy-1)-3-(4-trifluorométhanesulfanyl-phényl)-imidazolidine-2,4-dione
- la 5-méthyl-1-(3-diéthylamino-pyridin-4-ylméthyl)-3-(4-trifluorométhanesulfanyl-phényl)-imidazolidine-2,4-dione
- la 5-méthyl-1-(3-N-cyclohéxyl-N-méthylamino-pyridin-4-ylméthyl)-3-(4-trifluorométhanesulfanyl-phényl)-imidazolidine-2,4-dione
- la 5-méthyl-1-(3-pipéridino-pyridin-4-ylméthyl)-3-(4-trifluorométhanesulfanyl-phényl)-imidazolidine-2,4-dione
- la 5-méthyl-1-(3-morpholino-pyridin-4-ylméthyl)-3-(4-trifluorométhanesulfanyl-phényl)-imidazolidine-2,4-dione
- la 5-méthyl-1-[3-(4-méthylpipérazino)-pyridin-4-ylméthyl]-3-(4-trifluorométhanesulfanyl-phényl)-imidazolidine-2,4-dione
- la 5-méthyl-1-[3-(1H-pyrazol-1yl)-pyridin-4-ylméthyl]-3-(4-trifluorométhanesulfanyl-phényl)-imidazolidine-2,4-dione
- la 5-méthyl-1-[3-(2-benzoylhydrazino)-pyridin-4-ylméthyl]-3-(4-trifluorométhanesulfanyl-phényl)-imidazolidine-2,4-dione
- la 5-méthyl-1-(3-benzoylamino-pyridin-4-ylméthyl)-3-(4-trifluorométhanesulfanyl-phényl)-imidazolidine-2,4-dione
- la 5-méthyl-1-[3-(4-chlorobenzoylamino)-pyridin-4-ylméthyl]-3-(4-trifluorométhanesulfanyl-phényl)-imidazolidine-2,4-dione
- la 5-méthyl-1-[3-(3-chlorobenzoylamino)-pyridin-4-ylméthyl]-3-(4-trifluorométhanesulfanyl-phényl)-imidazolidine-2,4-dione
- la 5-méthyl-1-[3-(2-chlorobenzoylamino)-pyridin-4-ylméthyl]-3-(4-trifluorométhanésulfanyl-phényl)-imidazolidiné-2,4-dione
- la 5-méthyl-1-[3-(4-fluorobenzoylamino)-pyridin-4-ylméthyl]-3-(4-trifluorométhanesulfanyl-phényl)-imidazolidine-2,4-dione
- la 5-méthyl-1-[3-(4-méthoxybenzoylamino)-pyridin-4-ylméthyl]-3-(4-trifluorométhanesulfanyl-phényl)-imidazolidine-2,4-dione
- la 5-méthyl-1-(3-acétamido-pyridin-4-ylméthyl)-3-(4-trifluorométhanesulfanyl-phényl)-imidazolidine-2,4-dione
- la 5-méthyl-1-(3-phénylacétamido-pyridin-4-ylméthyl)-3-(4-trifluorométhanesulfanyl-phényl)-imidazolidine-2,4-dione
- la 5-méthyl-1-[3-(4-méthylpipérazino)acétamido-pyridin-4-ylméthyl]-3-(4-trifluorométhanesulfanyl-phényl)-imidazolidine-2,4-dione
- la 5-méthyl-1-(3-cyclohéxanylcarboxamido-pyridin-4-ylméthyl)-3-(4-trifluorométhanesulfanyl-phényl)-imidazolidine-2,4-dione
- la 5-méthyl-1-[3-(1-méthyl-4-pipéridinyl)carbonylamino-pyridin-4-ylméthyl]-3-(4-trifluorométhanesulfanyl-phényl)-imidazolidine-2,4-dione
- la 5-méthyl-1-[3-(2-furanylcarbonyl)amino-pyridin-4-ylméthyl]-3-(4-trifluorométhanesulfanyl-phényl)-imidazolidine-2,4-dione
- la 5-méthyl-1-(3-éthoxycarbonylamino-pyridin-4-ylméthyl)-3-(4-trifluorométhanesulfanyl-phényl)-imidazolidine-2,4-dione
- la 5-méthyl-1-(3-isopropoxycarbonylamino-pyridin-4-ylméthyl)-3-(4-trifluorométhanesulfanyl-phényl)-imidazolidine-2,4-dione
- la 5-méthyl-1-(3-butoxycarbonylamino-pyridin-4-ylméthyl)-3-(4-trifluorométhanesulfanyl-phényl)-imidazolidine-2,4-dione
- la 5-méthyl-1-[3-(2-diéthylaminoéthoxycarbonylamino)-pyridin-4-ylméthyl]-3-(4-trifluorométhanesulfanyl-phényl)-imidazolidine-2,4-dione
- la 5-méthyl-1-(3-phénoxycarbonylamino-pyridin-4-ylméthyl)-3-(4-trifluorométhanesulfanyl-phényl)-imidazolidine-2,4-dione
- la 5-méthyl-1-[3-(4-chloroanilino)carbonylamino-pyridin-4-ylméthyl]-3-(4-trifluorométhanesulfanyl-phényl)-imidazolidine-2,4-dione
- la 5-méthyl-1-(3-anilinocarbonylamino-pyridin-4-ylméthyl)-3-(4-trifluorométhanesulfanyl-phényl)-imidazolidine-2,4-dione
- la 5-méthyl-1-(3-benzylaminocarbonylamino-pyridin-4-ylméthyl)-3-(4-trifluorométhanesulfanyl-phényl)-imidazolidine-2,4-dione
- la 5-méthyl-1-[3-(2-pyridylamino)carbonylamino-pyridin-4-ylméthyl]-3-(4-trifluorométhanesulfanyl-phényl)-imidazolidine-2,4-dione
- la 5-méthyl-1-(3-uréido-pyridin-4-ylméthyl)-3-(4-trifluorométhanesulfanyl-phényl)-imidazolidine-2,4-dione
- la 5-méthyl-1-(3-benzylaminosulfonylamino-pyridin-4-ylméthyl)-3-(4-trifluorométhanesulfanyl-phényl)-imidazolidine-2,4-dione
- la 5-méthyl-1-(3-méthylaminosulfonylamino-pyridin-4-ylméthyl)-3-(4-trifluorométhanesulfanyl-phényl)-imidazolidine-2,4-dione
- la 5-méthyl-1-(3-aminosulfonylamino-pyridin-4-ylméthyl)-3-(4-trifluorométhanesulfanyl-phényl)-imidazolidine-2,4-dione
- la 5-méthyl-1-(3-morpholinosulfonylamino-pyridin-4-ylméthyl)-3-(4-trifluorométhanesulfanyl-phényl)-imidazolidine-2,4-dione
- la 5-méthyl-1-(3-diméthylaminosulfonylamino-pyridin-4-ylméthyl)-3-(4-trifluorométhanesulfanyl-phényl)-imidazolidine-2,4-dione
- la 5-méthyl-1-(3-anilinosulfonylamino-pyridin-4-ylméthyl)-3-(4-trifluorométhanesulfanyl-phényl)-imidazolidine-2,4-dione
- la 5-méthyl-1-(3-phénylsulfonylamino-pyridin-4-ylméthyl)-3-(4-trifluorométhanesulfanyl-phényl)-imidazolidine-2,4-dione
- la 5-méthyl-1-[3-(2-thiényl)sulfonylamino-pyridin-4-ylméthyl]-3-(4-trifluorométhanesulfanyl-phényl)-imidazolidine-2,4-dione
- la 5-méthyl-1-(3-méthylsulfonylamino-pyridin-4-ylméthyl)-3-(4-trifluorométhanesulfanyl-phényl)-imidazolidine-2,4-dione
- la 5-méthyl-1-[3-(4-chlorophényl)sulfonylamino-pyridin-4-ylméthyl]-3-(4-trifluorométhanesulfanyl-phényl)-imidazolidine-2,4-dione
- la 5-méthyl-1-(3-benzylsulfonylamino-pyridin-4-ylméthyl)-3-(4-trifluorométhanesulfanyl-phényl)-imidazolidine-2,4-dione
- la 5-méthyl-1-(3-hydroxycarbonyl-pyridin-4-ylméthyl)-3-(4-trifluorométhanesulfanyl-phényl)-imidazolidine-2,4-dione
- la 5-méthyl-1-(3-éthoxycarbonyl-pyridin-4-ylméthyl)-3-(4-trifluorométhanesulfanyl-phényl)-imidazolidine-2,4-dione
- la 5-méthyl-1-(3-ter-butoxycarbonyl-pyridin-4-ylméthyl)-3-(4-trifluorométhanesulfanyl-phényl)-imidazolidine-2,4-dione
- la 5-méthyl-1-(3-phénoxycarbonyl-pyridin-4-ylméthyl)-3-(4-trifluorométhanesulfanyl-phényl)-imidazolidine-2,4-dione
- la 5-méthyl-1-[3-(4-méthylpipérazinocarbonyl)-pyridin-4-ylméthyl]-3-(4-trifluorométhanesulfanyl-phényl)-imidazolidine-2,4-dione
- la 5-méthyl-1-[3-(méthylamino)carbonyl-pyridin-4-ylméthyl]-3-(4-trifluorométhanesulfanyl-phényl)-imidazolidine-2,4-dione
- la 5-méthyl-1-[3-(diméthylamino)carbonyl-pyridin-4-ylméthyl]-3-(4-trifluorométhanesulfanyl-phényl)-imidazolidine-2,4-dione
- la 5-méthyl-1-(3-pipéridinocarbonyl-pyridin-4-ylméthyl)-3-(4-trifluorométhanesulfanyl-phényl)-imidazolidine-2,4-dione
- la 5-méthyl-1-(3-morpholinocarbonyl-pyridin-4-ylméthyl)-3-(4-trifluorométhanesulfanyl-phényl)-imidazolidine-2,4-dione
- la 5-méthyl-1-[3-(4-phénylpipérazinocarbonyl)-pyridin-4-ylméthyl]-3-(4-trifluorométhanesulfanyl-phényl)-imidazolidine-2,4-dione
- la 5-méthyl-1-(3-phénylaminocarbonyl-pyridin-4-ylméthyl)-3-(4-trifluorométhanesulfanyl-phényl)-imidazolidine-2,4-dione
- la 5-méthyl-1-[3-(méthylphénylamino)carbonyl-pyridin-4-ylméthyl]-3-(4-trifluorométhanesulfanyl-phényl)-imidazolidine-2,4-dione
- la 5-méthyl-1-[3-(4-méthoxyphényl)méthylaminocarbonyl-pyridin-4-ylméthyl]-3-(4-trifluorométhanesulfanyl-phényl)-imidazolidine-2,4-dione
- la 5-méthyl-1-[3-(4-chlorophényl)méthylaminocarbonyl-pyridin-4-ylméthyl]-3-(4-trifluorométhanesulfanyl-phényl)-imidazolidine-2,4-dione
- la 5-méthyl-1-[3-(4-morpholinylamino)carbonyl-pyridin-4-ylméthyl]-3-(4-trifluorométhanesulfanyl-phényl)-imidazolidine-2,4-dione
- la 5-méthyl-1-(3-aminocarbonyl-pyridin-4-ylméthyl)-3-(4-trifluorométhanesulfanyl-phényl)-imidazolidine-2,4-dione
- la 5-méthyl-1-(3-phénoxy-pyridin-4-ylméthyl)-3-(4-trifluorométhanesulfanyl-phényl)-imidazolidine-2,4-dione
- la 5-méthyl-1-(3-méthoxy-pyridin-4-ylméthyl)-3-(4-trifluorométhanesulfanyl-phényl)-imidazolidine-2,4-dione
- la 5-méthyl-1-(3-éthoxy-pyridin-4-ylméthyl)-3-(4-trifluorométhanesulfanyl-phényl)-imidazolidine-2,4-dione
- la 5-méthyl-1-[3-(1-pyrrolidinyl)éthoxy-pyridin-4-ylméthyl]-3-(4-trifluorométhanesulfanyl-phényl)-imidazolidine-2,4-dione
- la 5-méthyl-1-(3-benzyloxy-pyridin-4-ylméthyl)-3-(4-trifluorométhanesulfanyl-phényl)-imidazolidine-2,4-dione
- la 5-méthyl-1-(3-benzoyloxy-pyridin-4-ylméthyl)-3-(4-trifluorométhanesulfanyl-phényl)-imidazolidine-2,4-dione
- la 5-méthyl-1-[3-(4,5-dihsrdro-1H-imidazol-2-yl)-pyridin-4-ylméthyl]-3-(4-trifluorométhanesulfanyl-phényl)-imidazolidine-2,4-dione
- la 5-méthyl-1-[3-(1H-imidazol-2-yl)-pyridin-4-ylméthyl]-3-(4-trifluorométhanesulfanyl-phényl)-imidazolidine-2,4-dione
- la 5-méthyl-1-[3-(1H-tétrazol-5-yl)-pyridin-4-ylméthyl]-3-(4-trifluorométhanesulfanyl-phényl)-imidazolidine-2,4-dione
- la 5-méthyl-1-(3-amidino-pyridin-4-ylméthyl)-3-(4-trifluorométhanesulfanyl-phényl)-imidazolidine-2,4-dione
- la 5-méthyl-1-(3-diméthylaminométhyl-pyridin-4-ylméthyl)-3-(4-trifluorométhanesulfanyl-phényl)-imidazolidine-2,4-dione
- la 5-méthyl-1-(3-aminométhyl-pyridin-4-ylméthyl)-3-(4-trifluorométhanesulfanyl-phényl)-imidazolidine-2,4-dione
- la 1-(2-méthyl-pyridin-4-ylméthyl)-3-(4-trifluorométhanesulfanyl-phényl)-imidazolidine-2,4-dione
- la 1-(2-éthyl-pyridin-4-ylméthyl)-3-(4-trifluorométhanesulfanyl-phényl)-imidazolidine-2,4-dione
- la 1-(2-isopropyl-pyridin-4-ylméthyl)-3-(4-trifluorométhanesulfanyl-phényl)-imidazolidine-2,4-dione
- la 1-(2-ter-butyl-pyridin-4-ylméthyl)-3-(4-trifluorométhanesulfanyl-phényl)-imidazolidine-2,4-dione
- la 1-(2-isobutyl-pyridin-4-ylméthyl)-3-(4-trifluorométhanesulfanyl-phényl)-imidazolidine-2,4-dione
- la 1-(2-cyclohéxyl-pyridin-4-ylméthyl)-3-(4-trifluorométhanesulfanyl-phényl)-imidazolidine-2,4-dione
- la 1-(2-phényl-pyridin-4-ylméthyl)-3-(4-trifluorométhanesulfanyl-phényl)-imidazolidine-2,4-dione
- la 1-[2-(4-chlorophényl)-pyridin-4-ylméthyl]-3-(4-trifluorométhanesulfanyl-phényl)-imidazolidine-2,4-dione
- la 1-[2-(3-chlorophényl)-pyridin-4-ylméthyl]-3-(4-trifluorométhanesulfanyl-phényl)-imidazolidine-2,4-dione
- la 1-[2-(2-chlorophényl)-pyridin-4-ylméthyl]-3-(4-trifluorométhanesulfanyl-phényl)-imidazolidine-2,4-dione
- la 1-[2-(4-fluorophényl)-pyridin-4-ylméthyl]-3-(4-trifluorométhanesulfanyl-phényl)-imidazolidine-2,4-dione
- la 1-[2-(4-méthoxyphényl)-pyridin-4-ylméthyl]-3-(4-trifluorométhanesulfanyl-phényl)-imidazolidine-2,4-dione
- la 1-(2-benzyl-pyridin-4-ylméthyl)-3-(4-trifluorométhanesulfanyl-phényl)-imidazolidine-2,4-dione
- la 1-(2,2'bipyridin-4-ylméthyl)-3-(4-trifluorométhanesulfanyl-phényl)-imidazolidine-2,4-dione
- la 1-(2-hydroxyméthyl-pyridin-4-ylméthyl)-3-(4-trifluorométhanesulfanyl-phényl)-imidazolidine-2,4-dione
- la 1-(2-chloro-pyridin-4-ylméthyl)-3-(4-trifluorométhanesulfanyl-phényl)-imidazolidine-2,4-dione
- la 1-(2-fluoro-pyridin-4-ylméthyl)-3-(4-trifluorométhanesulfanyl-phényl)-imidazolidine-2,4-dione
- la 1-(2-bromo-pyridin-4-ylméthyl)-3-(4-trifluorométhanesulfanyl-phényl)-imidazolidine-2,4-dione
- la 1-(2-amino-pyridin-4-ylméthyl)-3-(4-trifluorométhanesulfanyl-phényl)-imidazolidine-2,4-dione
- la 1-(2-méthylamino-pyridin-4-ylméthyl)-3-(4-trifluorométhanesulfanyl-phényl)-imidazolidine-2,4-dione
- la 1-(2-éthylamino-pyridin-4-ylméthyl)-3-(4-trifluorométhanesulfanyl-phényl)-imidazolidine-2,4-dione
- la 1-(2-isopropylamino-pyridin-4-ylméthyl)-3-(4-trifluorométhanesulfanyl-phényl)-imidazolidine-2,4-dione
- la 1-(2-phénylamino-pyridin-4-ylméthyl)-3-(4-trifluorométhanesulfanyl-phényl)-imidazolidine-2,4-dione
- la 1-(2-cyclohéxylamino-pyridin-4-ylméthyl)-3-(4-trifluorométhanesulfanyl-phényl)-imidazolidine-2,4-dione
- la 1-(2-diéthylamino-pyridin-4-ylméthyl)-3-(4-trifluorométhanesulfanyl-phényl)-imidazolidine-2,4-dione
- la 1-(2-N-cyclohéxyl-N-méthylamino-pyridin-4-ylméthyl)-3-(4-trifluorométhanesulfanyl-phényl)-imidazolidine-2,4-dione
- la 1-(2-pipéridino-pyridin-4-ylméthyl)-3-(4-trifluorométhanesulfanyl-phényl)-imidazolidine-2,4-dione
- la 1-(2-morpholino-pyridin-4-ylméthyl)-3-(4-trifluorométhanesulfanyl-phényl)-imidazolidine-2,4-dione
- la 1-[2-(4-méthylpipérazino)-pyridin-4-ylméthyl]-3-(4-trifluorométhanesulfanyl-phényl)-imidazolidine-2,4-dione
- la 1-[2-(1H-pyrazol-lyl)-pyridin-4-ylméthyl]-3-(4-trifluorométhanesulfanyl-phényl)-imidazolidine-2,4-dione
- la 1-[2-(2-benzoylhydrazino)-pyridin-4-ylméthyl]-3-(4-trifluorométhanesulfanyl-phényl)-imidazolidine-2,4-dione
- la l-(2-benzoylamino-pyridin-4-ylméthyl)-3-(4-trifluorométhanesulfanyl-phényl)-imidazolidine-2,4-dione
- la 1-[2-(4-chlorobenzoylamino)-pyridin-4-ylméthyl]-3-(4-trifluorométhanesulfanyl-phényl)-imidazolidine-2,4-dione
- la 1-[2-(3-chlorobenzoylamino)-pyridin-4-ylméthyl]-3-(4-trifluorométhanesulfanyl-phényl)-imidazolidine-2,4-dione
- la 1-[2-(2-chlorobenzoylamino)-pyridin-4-ylméthyl]-3-(4-trifluorométhanesulfanyl-phényl)-imidazolidine-2,4-dione
- la 1-[2-(4-fluorobenzoylamino)-pyridin-4-ylméthyl]-3-(4-trifluorométhanesulfanyl-phényl)-imidazolidine-2,4-dione
- la 1-[2-(4-méthoxybenzoylamino)-pyridin-4-ylméthyl]-3-(4-trifluorométhanesulfanyl-phényl)-imidazolidine-2,4-dione
- la 1-(2-acétamido-pyridin-4-ylméthyl)-3-(4-trifluorométhanesulfanyl-phényl)-imidazolidine-2,4-dione
- la 1-(2-phénylacétamido-pyridin-4-ylméthyl)-3-(4-trifluorométhanesulfanyl-phényl)-imidazolidine-2,4-dione
- la 1-[2-(4-méthylpipérazino)acétamido-pyridin-4-ylméthyl]-3-(4-trifluorométhanesulfanyl-phényl)-imidazolidine-2,4-dione
- la 1-(2-cyclohéxanylcarboxamido-pyridin-4-ylméthyl)-3-(4-trifluorométhanesulfanyl-phényl)-imidazolidine-2,4-dione
- la 1-[2-(1-méthyl-4-pipéridinyl)carbonylamino-pyridin-4-ylméthyl]-3-(4-trifluorométhanesulfanyl-phényl)-imidazolidine-2,4-dione
- la 1-[2-(2-furanylcarbonyl)amino-pyridin-4-ylméthyl]-3-(4-trifluorométhanesulfanyl-phényl)-imidazolidine-2,4-dione
- la 1-(2-éthoxycarbonylamino-pyridin-4-ylméthyl)-3-(4-trifluorométhanesulfanyl-phényl)-imidazolidine-2,4-dione
- la 1-(2-isopropoxycarbonylamino-pyridin-4-ylméthyl)-3-(4-trifluorométhanesulfanyl-phényl)-imidazolidine-2,4-dione
- la 1-(2-butoxycarbonylamino-pyridin-4-ylméthyl)-3-(4-trifluorométhanesulfanyl-phényl)-imidazolidine-2,4-dione
- la 1-[2-(2-diéthylaminoéthoxycarbonylamino)-pyridin-4-ylméthyl]-3-(4-trifluorométhanesulfanyl-phényl)-imidazolidine-2,4-dione
- la 1-(2-phénoxycarbonylamino-pyridin-4-ylméthyl)-3-(4-trifluorométhanesulfanyl-phényl)-imidazolidine-2,4-dione
- la 1-[2-(4-chloroanilino)carbonylamino-pyridin-4-ylméthyl]-3-(4-trifluorométhanesulfanyl-phényl)-imidazolidine-2,4-dione
- la 1-(2-anilinocarbonylamino-pyridin-4-ylméthyl)-3-(4-trifluorométhanesulfanyl-phényl)-imidazolidine-2,4-dione
- la 1-(2-benzylaminocarbonylamino-pyridin-4-ylméthyl)-3-(4-trifluorométhanesulfanyl-phényl)-imidazolidine-2,4-dione
- la 1-[2-(2-pyridylamino)carbonylamino-pyridin-4-ylméthyl]-3-(4-trifluorométhanesulfanyl-phényl)-imidazolidine-2,4-dione
- la 1-(2-uréido-pyridin-4-ylméthyl)-3-(4-trifluorométhanesulfanyl-phényl)-imidazolidine-2,4-dione
- la 1-(2-benzylaminosulfonylamino-pyridin-4-ylméthyl)-3-(4-trifluorométhanesulfanyl-phényl)-imidazolidine-2,4-dione
- la 1-(2-méthylaminosulfonylamino-pyridin-4-ylméthyl)-3-(4-trifluorométhanesulfanyl-phényl)-imidazolidine-2,4-dione
- la 1-(2-aminosulfonylamino-pyridin-4-ylméthyl)-3-(4-trifluorométhanesulfanyl-phényl)-imidazolidine-2,4-dione
- la 1-(2-morpholinosulfonnylamino-pyridin-4-ylméthyl)-3-(4-trifluorométhanesulfan-yl-phényl)-imidazolidine-2,4-dione
- la 1-(2-diméthylaminosulfonylamino-pyridin-4-ylméthyl)-3-(4-trifluorométhanesulfanyl-phényl)-imidazolidine-2,4-dione
- la 1-(2-anilinosulfonylamino-pyridin-4-ylméthyl)-3-(4-trifluorométhanesulfanyl-phényl)-imidazolidine-2,4-dione
- la 1-(2-phénylsulfonylamino-pyridin-4-ylméthyl)-3-(4-trifluorométhanesulfanyl-phényl)-imidazolidine-2,4-dione

- la 1-[2-(2-thiényl)sulfonylamino-pyridin-4-ylméthyl]-3-(4-trifluorométhanesulfanyl-phényl)-imidazolidine-2,4-dione
- la 1-(2-méthylsulfonylamino-pyridin-4-ylméthyl)-3-(4-trifluorométhanesulfanyl-phényl)-imidazolidine-2,4-dione
- la 1-[2-(4-chlorophényl)sulfonylamino-pyridin-4-ylméthyl]-3-(4-trifluorométhanesulfanyl-phényl)-imidazolidine-2,4-dione
- la 1-(2-benzylsulfonylamino-pyridin-4-ylméthyl)-3-(4-trifluorométhanesulfanyl-phényl)-imidazolidine-2,4-dione
- la 1-(2-hydroxycarbonyl-pyridin-4-ylméthyl)-3-(4-trifluorométhanesulfanyl-phényl)-imidazolidine-2,4-dione
- la 1-(2-éthoxycarbonyl-pyridin-4-ylméthyl)-3-(4-trifluorométhanesulfanyl-phényl)-imidazolidine-2,4-dione
- la 1-(2-ter-butoxycarbonyl-pyridin-4-ylméthyl)-3-(4-trifluorométhanesulfanyl-phényl)-imidazolidine-2,4-dione
- la 1-(2-phénoxycarbonyl-pyridin-4-ylméthyl)-3-(4-trifluorométhanesulfanyl-phényl)-imidazolidine-2,4-dione
- la 1-[2-(4-méthylpipérazinocarbonyl)-pyridin-4-ylméthyl]-3-(4-trifluorométhanesulfanyl-phényl)-imidazolidine-2,4-dione
- la 1-[2-(méthylamino)carbonyl-pyridin-4-ylméthyl]-3-(4-trifluorométhanesulfanyl-phényl)-imidazolidine-2,4-dione
- la 1-[2-(diméthylamino)carbonyl-pyridin-4-ylméthyl]-3-(4-trifluorométhanesulfanyl-phényl)-imidazolidine-2,4-dione
- la 1-(2-pipéridinocarbonyl-pyridin-4-ylméthyl)-3-(4-trifluorométhanesulfanyl-phényl)-imidazolidine-2,4-dione
- la 1-(2-morpholinocarbonyl-pyridin-4-ylméthyl)-3-(4-trifluorométhanesulfanyl-phényl)-imidazolidine-2,4-dione
- la 1-[2-(4-phénylpipérazinocarbonyl)-pyridin-4-ylméthyl]-3-(4-trifluorométhanesulfanyl-phényl)-imidazolidine-2,4-dione
- la 1-(2-phénylaminocarbonyl-pyridin-4-ylméthyl)-3-(4-trifluorométhanesulfanyl-phényl)-imidazolidine-2,4-dione
- la 1-[2-(méthylphénylamino)carbonyl-pyridin-4-ylméthyl]-3-(4-trifluorométhanesulfanyl-phényl)-imidazolidine-2,4-dione
- la 1-[2-(4-méthoxyphényl)méthylaminocarbonyl-pyridin-4-ylméthyl]-3-(4-trifluorométhanesulfanyl-phényl)-imidazolidine-2,4-dione
- la 1-[2-(4-chlorophényl)méthylaminocarbonyl-pyridin-4-ylméthyl]-3-(4-trifluorométhanesulfanyl-phényl)-imidazolidine-2,4-dione
- la 1-[2-(4-morpholinylamino)carbonyl-pyridin-4-ylméthyl]-3-(4-trifluorométhanesulfanyl-phényl)-imidazolidine-2,4-dione
- la 1-(2-aminocarbonyl-pyridin-4-ylméthyl)-3-(4-trifluorométhanesulfanyl-phényl)-imidazolidine-2,4-dione
- la 1-(2-phénoxy-pyridin-4-ylméthyl)-3-(4-trifluorométhanesulfanyl-phényl)-imidazolidine-2,4-dione
- la 1-(2-méthoxy-pyridin-4-ylméthyl)-3-(4-trifluorométhanesulfanyl-phényl)-imidazolidine-2,4-dione
- la 1-(2-éthoxy-pyridin-4-ylméthyl)-3-(4-trifluorométhanesulfanyl-phényl)-imidazolidine-2,4-dione
- la 1-[2-(1-pyrrolidinyl)éthoxy-pyridin-4-ylméthyl]-3-(4-trifluorométhanesulfanyl-phényl)-imidazolidine-2,4-dione
- la 1-(2-benzyloxy-pyridin-4-ylméthyl)-3-(4-trifluoro-méthanesulfanyl-phényl)-imidazolidine-2,4-dione
- la 1-(2-benzoyloxy-pyridin-4-ylméthyl)-3-(4-trifluorométhanesulfanyl-phényl)-imidazolidine-2,4-dione
- la 1-[2-(4,5-dihydro-1H-imidazol-2-yl)-pyridin-4-ylméthyl]-3-(4-trifluorométhanesulfanyl-phényl)-imidazolidine-2,4-dione
- la 1-[2-(1H-imidazol-2-yl)-pyridin-4-ylméthyl]-3-(4-trifluorométhanesulfanyl-phényl)-imidazolidine-2,4-dione
- la 1-[2-(1H-tétrazol-5-yl)-pyridin-4-ylméthyl]-3-(4-trifluorométhanesulfanyl-phényl)-imidazolidine-2,4-dione
- la 1-(2-amidino-pyridin-4-ylméthyl)-3-(4-trifluorométhanesulfanyl-phényl)-imidazolidine-2,4-dione
- la 1-(2-diméthylaminométhyl-pyridin-4-ylméthyl)-3-(4-trifluorométhanesulfanyl-phényl)-imidazolidine-2,4-dione
- la 1-(2-aminométhyl-pyridin-4-ylméthyl)-3-(4-trifluorométhanesulfanyl-phényl)-imidazolidine-2,4-dione
- la 1-(3-méthyl-pyridin-4-ylméthyl)-3-(4-trifluorométhanesulfanyl-phényl)-imidazolidine-2,4-dione
- la 1-(3-éthyl-pyridin-4-ylméthyl)-3-(4-trifluorométhanesulfanyl-phényl)-imidazolidine-2,4-dione
- la 1-(3-isopropyl-pyridin-4-ylméthyl)-3-(4-trifluorométhanesulfanyl-phényl)-imidazolidine-2,4-dione
- la 1-(3-ter-butyl-pyridin-4-ylméthyl)-3-(4-trifluorométhanesulfanyl-phényl)-imidazolidine-2,4-dione
- la 1-(3-isobutyl-pyridin-4-ylméthyl)-3-(4-trifluorométhanesulfanyl-phényl)-imidazolidine-2,4-dione
- la 1-(3-cyclohéxyl-pyridin-4-ylméthyl)-3-(4-trifluorométhanesulfanyl-phényl)-imidazolidine-2,4-dione
- la 1-(3-phényl-pyridin-4-ylméthyl)-3-(4-trifluorométhanesulfanyl-phényl)-imidazolidine-2,4-dione
- la 1-[3-(4-chlorophényl)-pyridin-4-ylméthyl]-3-(4-trifluorométhanesulfanyl-phényl)-imidazolidine-2,4-dione
- la 1-[3-(3-chlorophényl)-pyridin-4-ylméthyl]-3-(4-trifluorométhanesulfanyl-phényl)-imidazolidine-2,4-dione
- la 1-[3-(2-chlorophényl)-pyridin-4-ylméthyl]-3-(4-trifluorométhanesulfanyl-phényl)-imidazolidine-2,4-dione
- la 1-[3-(4-fluorophényl)-pyridin-4-ylméthyl]-3-(4-trifluorométhanesulfanyl-phényl)-imidazolidine-2,4-dione
- la 1-[3-(4-méthoxyphényl)-pyridin-4-ylméthyl]-3-(4-trifluorométhanesulfanyl-phényl)-imidazolidine-2,4-dione
- la 1-(3-benzyl-pyridin-4-ylméthyl)-3-(4-trifluorométhanesulfanyl-phényl)-imidazolidine-2,4-dione
- la 1-(3,2'bipyridin-4-ylméthyl)-3-(4-trifluorométhanesulfanyl-phényl)-imidazolidine-2,4-dione
- la 1-(3-hydroxyméthyl-pyridin-4-ylméthyl)-3-(4-trifluorométhanesulfanyl-phényl)-imidazolidine-2,4-dione
- la 1-(3-chloro-pyridin-4-ylméthyl)-3-(4-trifluorométhanesulfanyl-phényl)-imidazolidine-2,4-dione
- la 1-(3-fluoro-pyridin-4-ylméthyl)-3-(4-trifluorométhanesulfanyl-phényl)-imidazolidine-2,4-dione
- la 1-(3-bromo-pyridin-4-ylméthyl)-3-(4-trifluorométhanesulfanyl-phényl)-imidazolidine-2,4-dione
- la 1-(3-amino-pyridin-4-ylméthyl)-3-(4-trifluorométhanesulfanyl-phényl)-imidazolidine-2,4-dione
- la 1-(3-méthylamino-pyridin-4-ylméthyl)-3-(4-trifluorométhanesulfanyl-phényl)-imidazolidine-2,4-dione
- la 1-(3-éthylamino-pyridin-4-ylméthyl)-3-(4-trifluorométhanesulfanyl-phényl)-imidazolidine-2,4-dione
- la 1-(3-isopropylamino-pyridin-4-ylméthyl)-3-(4-trifluorométhanesulfanyl-phényl)-imidazolidine-2,4-dione
- la 1-(3-phénylamino-pyridin-4-ylméthyl)-3-(4-trifluorométhanesulfanyl-phényl)-imidazolidine-2,4-dione
- la 1-(3-cyclohéxylamino-pyridin-4-ylméthyl)-3-(4-trifluorométhanesulfanyl-phényl)-imidazolidine-2,4-dione
- la 1-(3-diéthylamino-pyridin-4-ylméthyl)-3-(4-trifluorométhanesulfanyl-phényl)-imidazolidine-2,4-dione
- la 1-(3-N-cyclohéxyl-N-méthylamino-pyridin-4-ylméthyl)-3-(4-trifluorométhanesulfanyl-phényl)-imidazolidine-2,4-dione
- la 1-(3-pipéridino-pyridin-4-ylméthyl)-3-(4-trifluorométhanesulfanyl-phényl)-imidazolidine-2,4-dione
- là 1-(3-morpholino-pyridin-4-ylméthyl)-3-(4-trifluorométhanesulfanyl-phényl)-imidazolidine-2,4-dione
- la 1-[3-(4-méthylpipérazino)-pyridin-4-ylméthyl]-3-(4-trifluorométhanesulfanyl-phényl)-imidazolidine-2,4-dione
- la 1-[3-(1H-pyrazol-lyl)-pyridin-4-ylméthyl]-3-(4-trifluorométhanesulfanyl-phényl)-imidazolidine-2,4-dione
- la 1-[3-(2-benzoylhydrazino)-pyridin-4-ylméthyl]-3-(4-trifluorométhanesulfanyl-phényl)-imidazolidine-2,4-dione
- la 1-(3-benzoylamino-pyridin-4-ylméthyl)-3-(4-trifluorométhanesulfanyl-phényl)-imidazolidine-2,4-dione
- la 1-[3-(4-chlorobenzoylamino)-pyridin-4-ylméthyl]-3-(4-trifluorométhanesulfanyl-phényl)-imidazolidine-2,4-dione
- la 1-[3-(3-chlorobenzoylamino)-pyridin-4-ylméthyl]-3-(4-trifluorométhanesulfanyl-phényl)-imidazolidine-2,4-dione
- la 1-[3-(2-chlorobenzoylamino)-pyridin-4-ylméthyl]-3-(4-trifluorométhanesulfanyl-phényl)-imidazolidine-2,4-dione
- la 1-[3-(4-fluorobenzoylamino)-pyridin-4-ylméthyl]-3-(4-trifluorométhanesulfanyl-phényl)-imidazolidine-2,4-dione
- la 1-[3-(4-méthoxybenzoylamino)-pyridin-4-ylméthyl]-3-(4-trifluorométhanesulfanyl-phényl)-imidazolidine-2,4-dione
- la 1-(3-acétamido-pyridin-4-ylméthyl)-3-(4-trifluorométhanesulfanyl-phényl)-imidazolidine-2,4-dione
- la 1-(3-phénylacétamido-pyridin-4-ylméthyl)-3-(4-trifluorométhanesulfanyl-phényl)-imidazolidine-2,4-dione
- la 1-[3-(4-méthylpipérazino)acétamido-pyridin-4-ylméthyl]-3-(4-trifluorométhanesulfanyl-phényl)-imidazolidine-2,4-dione
- la 1-(3-cyclohéxanylcarboxamido-pyridin-4-ylméthyl)-3-(4-trifluorométhanesulfanyl-phényl)-imidazolidine-2,4-dione
- la 1-[3-(1-méthyl-4-pipéridinyl)carbonylamino-pyridin-4-ylméthyl]-3-(4-trifluorométhanesulfanyl-phényl)-imidazolidine-2,4-dione
- la 1-[3-(2-furanylcarbonyl)amino-pyridin-4-ylméthyl]-3-(4-trifluorométhanesulfanyl-phényl)-imidazolidine-2,4-dione
- la 1-(3-éthoxycarbonylamino-pyridin-4-ylméthyl)-3-(4-trifluorométhanesulfanyl-phényl)-imidazolidine-2,4-dione
- la 1-(3-isopropoxycarbonylamino-pyridin-4-ylméthyl)-3-(4-trifluorométhanesulfanyl-phényl)-imidazolidine-2,4-dione
- la 1-(3-butoxycarbonylamino-pyridin-4-ylméthyl)-3-(4-trifluorométhanesulfanyl-phényl)-imidazolidine-2,4-dione
- la 1-[3-(2-diéthylaminoéthoxycarbonylamino)-pyridin-4-ylméthyl]-3-(4-trifluorométhanesulfanyl-phényl)-imidazolidine-2,4-dione
- la 1-(3-phénoxycarbonylamino-pyridin-4-ylméthyl)-3-(4-trifluorométhanesulfanyl-phényl)-imidazolidine-2,4-dione
- la 1-[3-(4-chloroanilino)carbonylamino-pyridin-4-ylméthyl]-3-(4-trifluorométhanesulfanyl-phényl)-imidazolidine-2,4-dione
- la 1-(3-anilinocarbonylamino-pyridin-4-ylméthyl)-3-(4-trifluorométhanesulfanyl-phényl)-imidazolidine-2,4-dione
- la 1-(3-benzylaminocarbonylamino-pyridin-4-ylméthyl)-3-(4-trifluorométhanesulfanyl-phényl)-imidazolidine-2,4-dione
- la 1-[3-(2-pyridylamino)carbonylamino-pyridin-4-ylméthyl]-3-(4-trifluorométhanesulfanyl-phényl)-imidazolidine-2,4-dione
- la 1-(3-uréido-pyridin-4-ylméthyl)-3-(4-trifluorométhanesulfanyl-phényl)-imidazolidine-2,4-dione la 1-(3-benzylaminosulfonylamino-pyridin-4-ylméthyl)-3-(4-trifluorométhanesulfanyl-phényl)-imidazolidine-2,4-dione
- la 1-(3-méthylaminosulfonylamino-pyridin-4-ylméthyl)-3-(4-trifluorométhanesulfanyl-phényl)-imidazolidine-2,4-dione
- la 1-(3-aminosulfonylamino-pyridin-4-ylméthyl)-3-(4-trifluorométhanesulfanyl-phényl)-imidazolidine-2,4-dione
- la 1-(3-morpholinosulfonylamino-pyridin-4-ylméthyl)-3-(4-trifluorométhanesulfanyl-phényl)-imidazolidine-2,4-dione
- la 1-(3-diméthylaminosulfonylamino-pyridin-4-ylméthyl)-3-(4-trifluorométhanesulfanyl-phényl)-imidazolidine-2,4-dione
- la 1-(3-anilinosulfonylamino-pyridin-4-ylméthyl)-3-(4-trifluorométhanesulfanyl-phényl)-imidazolidine-2,4-dione
- la 1-(3-phénylsulfonylamino-pyridin-4-ylméthyl)-3-(4-trifluorométhanesulfanyl-phényl)-imidazolidine-2,4-dione
- la 1-[3-(2-thiényl)sulfonylamino-pyridin-4-ylméthyl]-3-(4-trifluorométhanesulfanyl-phényl)-imidazolidine-2,4-dione
- la 1-(3-méthylsulfonylamino-pyridin-4-ylméthyl)-3-(4-trifluorométhanesulfanyl-phényl)-imidazolidine-2,4-dione
- la 1-[3-(4-chlorophényl)sulfonylamino-pyridin-4-ylméthyl]-3-(4-trifluorométhanesulfanyl-phényl)-imidazolidine-2,4-dione
- la 1-(3-benzylsulfonylamino-pyridin-4-ylméthyl)-3-(4-trifluorométhanesulfanyl-phényl)-imidazolidine-2,4-dione
- la 1-(3-hydroxycarbonyl-pyridin-4-ylméthyl)-3-(4-trifluorométhanesulfanyl-phényl)-imidazolidine-2,4-dione
- la 1-(3-éthoxycarbonyl-pyridin-4-ylméthyl)-3-(4-trifluorométhanesulfanyl-phényl)-imidazolidine-2,4-dione
- la 1-(3-ter-butoxycarbonyl-pyridin-4-ylméthyl)-3-(4-trifluorométhanesulfanyl-phényl)-imidazolidine-2,4-dione
- la 1-(3-phénoxycarbonyl-pyridin-4-ylméthyl)-3-(4-trifluorométhanesulfanyl-phényl)-imidazolidine-2,4-dione
- la 1-[3-(4-méthylpipérazinocarbonyl)-pyridin-4-ylméthyl]-3-(4-trifluorométhanesulfanyl-phényl)-imidazolidine-2,4-dione
- la 1-[3-(méthylamino)carbonyl-pyridin-4-ylméthyl]-3-(4-trifluorométhanesulfanyl-phényl)-imidazolidine-2,4-dione
- la 1-[3-(diméthylamino)carbonyl-pyridin-4-ylméthyl]-3-(4-trifluorométhanesulfanyl-phényl)-imidazolidine-2,4-dione
- la 1-(3-pipéridinocarbonyl-pyridin-4-ylméthyl)-3-(4-trifluorométhanesulfanyl-phényl)-imidazolidine-2,4-dione
- la 1-(3-morpholinocarbonyl-pyridin-4-ylméthyl)-3-(4-trifluorométhanesulfany-1-phényl)-imidazolidine-2,4-dione
- la 1-[3-(4-phénylpipérazinocarbonyl)-pyridin-4-ylméthyl]-3-(4-trifluorométhanesulfanyl-phényl)-imidazolidine-2,4-dione
- la 1-(3-phénylaminocarbonyl-pyridin-4-ylméthyl)-3-(4-trifluorométhanesulfanyl-phényl)-imidazolidine-2,4-dione
- la 1-[3-(méthylphénylamino)carbonyl-pyridin-4-ylméthyl]-3-(4-trifluorométhanesulfanyl-phényl)-imidazolidine-2,4-dione
- la 1-[3-(4-méthoxyphényl)méthylaminocarbonyl-pyridin-4-ylméthyl]-3-(4-trifluorométhanesulfanyl-phényl)-imidazolidine-2,4-dione
- la 1-[3-(4-chlorophényl)méthylaminocarbonyl-pyridin-4-ylméthyl]-3-(4-trifluorométhanesulfanyl-phényl)-imidazolidine-2,4-dione
- la 1-[3-(4-morpholinylamino)carbonyl-pyridin-4-ylméthyl]-3-(4-trifluorométhanesulfanyl-phényl)-imidazolidine-2,4-dione
- la 1-(3-aminocarbonyl-pyridin-4-ylméthyl)-3-(4-trifluorométhanesulfanyl-phényl)-imidazolidine-2,4-dione
- la 1-(3-phénoxy-pyridin-4-ylméthyl)-3-(4-trifluorométhanesulfanyl-phényl)-imidazolidine-2,4-dione
- la 1-(3-méthoxy-pyridin-4-ylméthyl)-3-(4-trifluorométhanesulfanyl-phényl)-imidazolidine-2,4-dione
- la 1-(3-éthoxy-pyridin-4-ylméthyl)-3-(4-trifluorométhanesulfanyl-phényl)-imidazolidine-2,4-dione
- la 1-[3-(1-pyrrolidinyl)éthoxy-pyridin-4-ylméthyl]-3-(4-trifluorométhanesulfanyl-phényl)-imidazolidine-2,4-dione
- la 1-(3-benzyloxy-pyridin-4-ylméthyl)-3-(4-trifluorométhanesulfanyl-phényl)-imidazolidine-2,4-dione
- la 1-(3-benzoyloxy-pyridin-4-ylméthyl)-3-(4-trifluorométhanesulfanyl-phényl)-imidazolidine-2,4-dione
- la 1-[3-(4,5-dihydro-1H-imidazol-2-yl)-pyridin-4-ylméthyl]-3-(4-trifluorométhanesulfanyl-phényl)-imidazolidine-2,4-dione
- la 1-[3-(1H-imidazol-2-yl)-pyridin-4-ylméthyl]-3-(4-trifluorométhanesulfanyl-phényl)-imidazolidine-2,4-dione
- la 1-[3-(1H-tétrazol-5-yl)-pyridin-4-ylméthyl]-3-(4-trifluorométhanesulfanyl-phényl)-imidazolidine-2,4-dione
- la 1-(3-amidino-pyridin-4-ylméthyl)-3-(4-trifluorométhanesulfanyl-phényl)-imidazolidine-2,4-dione
- la 1-(3-diméthylaminométhyl-pyridin)-4-ylméthyl]-3-(4-trifluorométhanesulfanyl-phényl)-imidazolidine-2,4-dione
- la 1-(3-aminométhyl-pyridin-4-ylméthyl)-3-(4-trifluorométhanesulfanyl-phényl)-imidazolidine-2,4-dione
- la 5,5-diméthyl-1-(2-méthyl-pyrzdin-4-ylméthyl)-3-(4-trifluorométhanesulfonyl-phényl)-imidazolidine-2,4-dione
- la 5,5-diméthyl-1-(2-éthyl-pyridin-4-ylméthyl)-3-(4-trifluorométhanesulfonyl-phényl)-imidazolidine-2,4-dione
- la 5,5-diméthyl-1-(2-isopropyl-pyridin-4-ylméthyl)-3-(4-trifluorométhanesulfonyl-phényl)-imidazolidine-2,4-dione
- la 5,5-diméthyl-1-(2-ter-butyl-pyridin-4-ylméthyl)-3-(4-trifluorométhanésulfonyl-phényl)-imidazolidine-2,4-dione
- la 5,5-diméthyl-1-(2-isobutyl-pyridin-4-ylméthyl)-3-(4-trifluorométhanesulfonyl-phényl)-imidazolidine-2,4-dione
- la 5,5-diméthyl-1-(2-cyclohéxyl-pyridin-4-ylméthyl)-3-(4-trifluorométhanésulfonyl-phényl)-imidazolidine-2,4-dione
- la 5,5-diméthyl-1-(2-phényl-pyridin-4-ylméthyl)-3-(4-trifluorométhanesulfonyl-phényl)-imidazolidine--2,4-dione
- la 5,5-diméthyl-1-[2-(4-chlorophényl)-pyridin-4-ylméthyl]-3-(4-trifluorométhanesulfonyl-phényl)-imidazolidine-2,4-dione
- la 5,5-diméthyl-1-[2-(3-chlorophényl)-pyridin-4-ylméthyl]-3-(4-trifluorométhanesulfonyl-phényl)-imidazolidine-2,4-dione
- la 5,5-diméthyl-1-[2-(2-chlorophényl)-pyridin-4-ylméthyl]-3-(4-trifluorométhanesulfonyl-phényl)-imidazolidine-2,4-dione
- la 5,5-diméthyl-1-[2-(4-fluorophényl)-pyridin-4-ylméthyl]-3-(4-trifluorométhanesulfonyl-phényl)-imidazolidine-2,4-dione
- la 5,5-diméthyl-1-[2-(4-méthoxyphényl)-pyridin-4-ylméthyl]-3-(4-trifluorométhanesulfonyl-phényl)-imidazolidine-2,4-dione
- la 5,5-diméthyl-1-(2-benzyl-pyridin-4-ylméthyl)-3-(4-trifluorométhanesulfonyl-phényl)-imidazolidine-2,4-dione
- la 5,5-diméthyl-1-(2,2'bipyridin-4-ylméthyl)-3-(4-trifluorométhanesulfonyl-phényl)-imidazolidine-2,4-dione
- la 5,5-diméthyl-1-(2-hydroxyméthyl-pyridin-4-ylméthyl)-3-(4-trifluorométhanesulfonyl-phényl)-imidazolidine-2,4-dione
- la 5,5-diméthyl-1-(2-chloro-pyridin-4-ylméthyl)-3-(4-trifluorométhanesulfonyl-phényl)-imidazolidine-2,4-dione
- la 5,5-diméthyl-1-(2-fluoro-pyridin-4-ylméthyl)-3-(4-trifluorométhanesulfonyl-phényl)-imidazolidine-2,4-dione
- la 5,5-diméthyl-1-(2-bromo-pyridin-4-ylméthyl)-3-(4-trifluorométhanesulfonyl-phényl)-imidazolidine-2,4-dione
- la 5,5-diméthyl-1-(2-amino-pyridin-4-ylméthyl)-3-(4-trifluorométhanesulfonyl-phényl)-imidazolidine-2,4-dione
- la 5,5-diméthyl-1-(2-méthylamino-pyridin-4-ylméthyl)-3-(4-trifluorométhanesulfonyl-phényl)-imidazolidine-2,4-diode
- la 5,5-diméthyl-1-(2-éthylamino-pyridin-4-ylméthyl)-3-(4-trifluorométhanesulfonyl-phényl)-imidazolidine-2,4-dione
- la 5,5-diméthyl-1-(2-isopropylamino-pyridin-4-ylméthyl)-3-(4-trifluorométhanesulfonyl-phényl)-imidazolidine-2,4-dione
- la 5,5-diméthyl-1-(2-phénylamino-pyridin-4-ylméthyl)-3-(4-trifluorométhanesulfonyl-phényl)-imidazolidine-2,4-dione
- la 5,5-diméthyl-1-(2-cyclohéxylamino-pyridin-4-ylméthyl)-3-(4-trifluorométhanesulfonyl-phényl)-imidazolidine-2,4-dione
- la 5,5-diméthyl-1-(2-diéthylamino-pyridin-4-ylméthyl)-3-(4-trifluo-rométhanesulfonyl-phényl)-imidazolidine-2,4-dione
- la 5,5-diméthyl-1-(2-N-cyclohéxyl-N-méthylamino-pyridin-4-ylméthyl)-3-(4-trifluorométhanesulfonyl-phényl)-imidazolidine-2,4-dione
- la 5,5-diméthyl-1-(2-pipéridino-pyridin-4-ylméthyl)-3-(4-trifluorométhanesulfonyl-phényl)-imidazolidine-2,4-dione
- la 5,5-diméthyl-1-(2-morpholino-pyridin-4-ylméthyl)-3-(4-trifluorométhanesulfonyl-phényl)-imidazolidine-2,4-dione
- la 5,5-diméthyl-1-[2-(4-méthylpipérazino)diéthylamino-pyridin-4-ylméthyl]-3-(4-trifluorométhanesulfonyl-phényl)-imidazolidine-2,4-dione
- la 5,5-diméthyl-1-[2-(1H-pyrazol-1yl)-pyridin-4-ylméthyl]-3-(4-trifluorométhanesulfonyl-phényl)-imidazolidine-2,4-dione
- la 5,5-diméthyl-1-[2-(2-benzoylhydrazino)-pyridin-4-ylméthyl]-3-(4-trifluorométhanesulfonyl-phényl)-imidazolidine-2,4-dione
- la 5,5-diméthyl-1-(2-benzoylamino-pyridin-4-ylméthyl)-3-(4-trifluorométhanesulfonyl-phényl)-imidazolidine-2,4-dione
- la 5,5-diméthyl-1-[2-(4-chlorobenzoylamino)-pyridin-4-ylméthyl]-3-(4-trifluorométhanesulfonyl-phényl)-imidazolidine-2,4-dione
- la 5,5-diméthyl-1-[2-(3-chlorobenzoylamino)-pyridin-4-ylméthyl]-3-(4-trifluorométhanesulfonyl-phényl)-imidazolidine-2,4-dione
- la 5,5-diméthyl-1-[2-(2-chlorobenzoylamino)-pyridin-4-ylméthyl]-3-(4-trifluorométhanesulfonyl-phényl)-imidazolidine-2,4-dione
- la 5,5-diméthyl-1-[2-(4-fluorobenzoylamino)-pyridin-4-ylméthyl]-3-(4-trifluorométhanesulfonyl-phényl)-imidazolidine-2,4-dione
- la 5,5-diméthyl-1-[2-(4-méthoxybenzoylamino)-pyridin-4-ylméthyl]-3-(4-trifluorométhanesulfonyl-phényl)-imidazolidine-2,4-dione
- la 5,5-diméthyl-1-(2-acétamido-pyridin-4-ylméthyl)-3-(4-trifluorométhanesulfonyl-phényl)-imidazolidine-2,4-dione
- la 5,5-diméthyl-1-(2-phénylacétamido-pyridin-4-ylméthyl)-3-(4-trifluorométhanesulfonyl-phényl)-imidazolidine-2,4-dione
- la 5,5-diméthyl-1-[2-(4-méthylpipérazino)acétamido-pyridin-4-ylméthyl]-3-(4-trifluorométhanesulfonyl-phényl)-imidazolidine-2,4-dione
- la 5,5-diméthyl-1-(2-cyclohéxanylcarboxamido-pyridin-4-ylméthyl)-3-(4-trifluorométhanesulfonyl-phényl)-imidazolidine-2,4-dione
- la 5,5-diméthyl-1-[2-(1-méthyl-4-pipéridinyl)carbonylamino-pyridin-4-ylméthyl]-3-(4-trifluorométhanesulfonyl-phényl)-imidazolidine-2,4-dione
- la 5,5-diméthyl-1-[2-(2-furanylcarbonyl)amino-pyridin-4-ylméthyl]-3-(4-trifluorométhanesulfonyl-phényl)-imidazolidine-2,4-dione
- la 5,5-diméthyl-1-(2-éthoxycarbonylamino-pyridin-4-ylméthyl)-3-(4-trifluorométhanesulfonyl-phényl)-imidazolidine-2,4-dione
- la 5,5-diméthyl-1-(2-isopropoxycarbonylamino-pyridin-4-ylméthyl)-3-(4-trifluorométhanesulfonyl-phényl)-imidazolidine-2,4-dione
- la 5,5-diméthyl-1-(2-butoxycarbonylamino-pyridin-4-ylméthyl)-3-(4-trifluorométhanesulfonyl-phényl)-imidazolidine-2,4-dione
- la 5,5-diméthyl-1-[2-(2-diéthylaminoéthoxycarbonylamino)-pyridin-4-ylméthyl]-3-(4-trifluorométhanesulfonyl-phényl)-imidazolidine-2,4-dione
- la 5,5-diméthyl-1-(2-phénoxycarbonylamino-pyridin-4-ylméthyl)-3-(4-trifluorométhanesulfonyl-phényl)-imidazolidine-2,4-dione
- la 5,5-diméthyl-1-[2-(4-chloroanilino)carbonylamino-pyridin-4-ylméthyl)]-3-(4-trifluorométhanesulfonyl-phényl)-imidazolidine-2,4-dione
- la 5,5-diméthyl-1-(2-anilinocarbonylamino-pyridin-4-ylméthyl)-3-(4-trifluorométhanesulfonyl-phényl)-imidazolidine-2,4-divne
- la 5,5-diméthyl-1-(2-benzylaminocarbonylamino-pyridin-4-ylméthyl)-3-(4-trifluorométhanesulfonyl-phényl)-imidazolidine-2,4-dione
- la 5,5-diméthyl-1-[2-(2-pyridylamino)carbonylamino-pyridin-4-ylméthyl)]-3-(4-trifluorométhanesulfonyl-phényl)-imidazolidine-2,4-dione
- la 5,5-diméthyl-1-(2-uréido-pyridin-4-ylméthyl)-3-(4-trifluorométhanesulfonyl-phényl)-imidazolidine-2,4-dione
- la 5,5-diméthyl-1-(2-benzylaminosulfonylamino-pyridin-4-ylméthyl)-3-(4-trifluorométhanesulfonyl-phényl)-imidazolidine-2,4-dione
- la 5,5-diméthyl-1-(2-méthylaminosulfonylamino-pyridin-4-ylméthyl)-3-(4-trifluorométhanesulfonyl-phényl)-imidazolidine-2,4-dione
- la 5,5-diméthyl-1-(2-aminosulfonylamino-pyridin-4-ylméthyl)-3-(4-trifluorométhanesulfonyl-phényl)-imidazolidine-2,4-dione
- la 5,5-diméthyl-1-(2-morpholinosulfonylamino-pyridin-4-ylméthyl)-3-(4-trifluorométhanesulfonyl-phényl)-imidazolidine-2,4-dione
- la 5,5-diméthyl-1-(2-diméthylaminosulfonylamino-pyridin-4-ylméthyl)-3-(4-trifluorométhanesulfonyl-phényl)-imidazolidine-2,4-dione
- la 5,5-diméthyl-1-(2-anilinosulfonylamino-pyridin-4-ylméthyl)-3-(4-trifluorométhanesulfonyl-phényl)-imidazolidine-2,4-dione
- la 5,5-diméthyl-1-(2-phénylsulfonylamino-pyridin-4-ylméthyl)-3-(4-trifluorométhanesulfonyl-phényl)-imidazolidine-2,4-dione
- la 5,5-diméthyl-1-[2-(2-thiényl)sulfonylamino-pyridin-4-ylméthyl]-3-(4-trifluorométhanesulfonyl-phényl)-imidazolidine-2,4-dione
- la 5,5-diméthyl-1-(2-méthylsulfonylamino-pyridin-4-ylméthyl)-3-(4-trifluorométhanesulfonyl-phényl)-imidazolidine-2,4-dione
- la 5,5-diméthyl-1-[2-(4-chlorophényl)sulfonylamino-pyridin-4-ylméthyl]-3-(4-trifluorométhanesulfonyl-phényl)-imidazolidine-2,4-dione
- la 5,5-diméthyl-1-(2-benzylsulfonylamino-pyridin-4-ylméthyl)-3-(4-trifluorométhanesulfonyl-phényl)-imidazolidine-2,4-dione
- la 5,5-diméthyl-1-(2-hydroxycarbonyl-pyridin-4-ylméthyl)-3-(4-trifluorométhanesulfonyl-phényl)-imidazolidine-2,4-dione
- la 5,5-diméthyl-1-(2-éthoxycarbonyl-pyridin-4-ylméthyl)-3-(4-trifluorométhanesulfonyl-phényl)-imidazolidine-2,4-dione
- la 5,5-diméthyl-1-(2-ter-butoxycarbonyl-pyridin-4-ylméthyl)-3-(4-trifluorométhanesulfonyl-phényl)-imidazolidine-2,4-dione
- la 5,5-diméthyl-1-(2-phénoxycarbonyl-pyridin-4-ylméthyl)-3-(4-trifluorométhanesulfonyl-phényl)-imidazolidine-2,4-dione
- la 5,5-diméthyl-1-[2-(4-méthylpipérazinocarbonyl)-pyridin-4-ylméthyl]-3-(4-trifluorométhanesulfonyl-phényl)-imidazolidine-2,4-dione
- la 5,5-diméthyl-1-[2-(méthylamino)carbonyl-pyridin-4-ylméthyl]-3-(4-trifluorométhanesulfonyl-phényl)-imidazolidine-2,4-dione
- la 5,5-diméthyl-1-[2-(diméthylamino)carbonyl-pyridin-4-ylméthyl] -3-(4-trifluorométhanesulfonyl-phényl)-imidazolidine-2,4-dione
- la 5,5-diméthyl-1-(2-pipéridinocarbonyl-pyridin-4-ylméthyl)-3-(4-trifluorométhanesulfonyl-phényl)-imidazolidine-2,4-dione
- la 5,5-diméthyl-1-(2-morpholinocarbonyl-pyridin-4-ylméthyl)-3-(4-trifluorométhanesulfonyl-phényl)-imidazolidine-2,4-dione
- la 5,5-diméthyl-1-[2-(4-phénylpipérazinocarbonyl)-pyridin-4-ylméthyl]-3-(4-trifluorométhanesulfonyl-phényl)-imidazolidine-2,4-dione
- la 5,5-diméthyl-1-(2-phénylaminocarbonyl-pyridin-4-ylméthyl)-3-(4-trifluorométhanesulfonyl-phényl)-imidazolidine-2,4-dione
- la 5,5-diméthyl-1-[2-(méthylphénylamino)carbonyl-pyridin-4-ylméthyl]-3-(4-trifluorométhanesulfonyl-phényl)-imidazolidine-2,4-dione
- la 5,5-diméthyl-1-[2-(4-méthoxyphényl)méthylaminocarbonyl-pyridin-4-ylméthyl]-3-(4-trifluorométhanesulfonyl-phényl)-imidazolidine-2,4-dione
- la 5,5-diméthyl-1-[2-(4-chlorophényl)méthylaminocarbonyl-pyridin-4-ylméthyl]-3-(4-trifluorométhanesulfonyl-phényl)-imidazolidine-2,4-dione
- la 5,5-diméthyl-1-[2-(4-morpholinylamino)carbonyl-pyridin-4-ylméthyl]-3-(4-trifluorométhanesulfonyl-phényl)-imidazolidine-2,4-dione
- la 5,5-diméthyl-1-(2-aminocarbonyl-pyridin-4-ylméthyl)-3-(4-trifluorométhanesulfonyl-phényl)-imidazolidine-2,4-dione
- la 5,5-diméthyl-1-(2-phénoxy-pyridin-4-ylméthyl)-3-(4-trifluorométhanesulfonyl-phényl)-imidazolidine-2,4-dione
- la 5,5-diméthyl-1-(2-méthoxy-pyridin-4-ylméthyl)-3-(4-trifluorométhanesulfonyl-phényl)-imidazolidine-2,4-dione
- la 5,5-diméthyl-1-(2-éthoxy-pyridin-4-ylméthyl)-3-(4-trifluorométhanesulfonyl-phényl)-imidazolidine-2,4-dione
- la 5,5-diméthyl-1-[2-(1-pyrrolidinyl)éthoxy-pyridin-4-ylméthyl]-3-(4-trifluorométhanesulfonyl-phényl)-imidazolidine-2,4-dione
- la 5,5-diméthyl-1-(2-benzyloxy-pyridin-4-ylméthyl)-3-(4-trifluorométhanesulfonyl-phényl)-imidazolidine-2,4-dione
- la 5,5-diméthyl-1-(2-benzoyloxy-pyridin-4-ylméthyl)-3-(4-trifluorométhanesulfonyl-phényl)-imidazolidine-2,4-dione
- la 5,5-diméthyl-1-[2-(4,5-dihydro-1H-imidazol-2-yl)-pyridin-4-ylméthyl]-3-(4-trifluorométhanesulfonyl-phényl)-imidazolidine-2,4-dione
- la 5,5-diméthyl-1-[2-(1H-imidazol-2-yl)-pyridin-4-ylméthyl]-3-(4-trifluorométhanesulfonyl-phényl)-imidazolidine-2,4-dione
- la 5,5-diméthyl-1-[2-(1H-tétrazol-5-yl)-pyridin-4-ylméthyl]-3-(4-trifluorométhanesulfonyl-phényl)-imidazolidine-2,4-dione
- la 5,5-diméthyl-1-(2-amidino-pyridin-4-ylméthyl)-3-(4-trifluorométhanesulfonyl-phényl)-imidazolidine-2,4-dione
- la 5,5-diméthyl-1-(2-diméthylaminométhyl-pyridin-4-ylméthyl)-3-(4-trifluorométhanesulfonyl-phényl)-imidazolidine-2,4-dione
- la 5,5-diméthyl-1-(2-aminoméhyl-pyridin-4-ylméthyl)-3-(4-trifluorométhanesulfonyl-phényl)-imidazolidine-2,4-dione
- la 5,5-diméthyl-1-(3-méthyl-pyridin-4-ylméthyl)-3-(4-trifluorométhanesulfonyl-phényl)-imidazolidine-2,4-dione
- la 5,5-diméthyl-1-(3-éthyl-pyridin-4-ylméthyl)-3-(4-trifluorométhanesulfonyl-phényl)-imidazolidine-2,4-dione
- la 5,5-diméthyl-1-(3-isopropyl-pyridin-4-ylméthyl)-3-(4-trifluorométhanesulfonyl-phényl)-imidazolidine-2,4-dione
- la 5,5-diméthyl-1-(3-ter-butyl-pyridin-4-ylméthyl)-3-(4-trifluorométhanesulfonyl-phényl)-imidazolidine-2,4-dione
- la 5,5-diméthyl-1-(3-isobutyl-pyridin-4-ylméthyl)-3-(4-trifluorométhanesulfonyl-phényl)-imidazolidine-2,4-dione
- la 5,5-diméthyl-1-(3-cyclohéxyl-pyridin-4-ylméthyl)-3-(4-trifluorométhanesulfonyl-phényl)-imidazolidine-2,4-dione
- la 5,5-diméthyl-1-(3-phényl-pyridin-4-ylméthyl)-3-(4-trifluorométhanesulfonyl-phényl)-imidazolidine-2,4-dione
- la 5,5-diméthyl-1-[3-(4-chlorophényl)-pyridin-4-ylméthyl]-3-(4-trifluorométhanesulfonyl-phényl)-imidazolidine-2,4-dione
- la 5,5-diméthyl-1-[3-(3-chlorophényl)-pyridin-4-ylméthyl]-3-(4-trifluorométhanesulfonyl-phényl)-imidazolidine-2,4-dione
- la 5,5-diméthyl-1-[3-(2-chlorophényl)-pyridin-4-ylméthyl]-3-(4-trifluorométhanesulfonyl-phényl)-imidazolidine-2,4-dione
- la 5,5-diméthyl-1-[3-(4-fluorophényl)-pyridin-4-ylméthyl]-3-(4-trifluorométhanesulfonyl-phényl)-imidazolidine-2,4-dione
- la 5,5-diméthyl-1-[3-(4-méthoxyphényl)-pyridin-4-ylméthyl]-3-(4-trifluorométhanesulfonyl-phényl)-imidazolidine-2,4-dione
- la 5,5-diméthyl-1-(3-benzyl-pyridin-4-ylméthyl)-3-(4-trifluorométhanesulfonyl-phényl)-imidazolidine-2,4-dione
- la 5,5-diméthyl-1-(3,2'bipyridin-4-ylméthyl)-3-(4-trifluorométhanesulfonyl-phényl)-imidazolidine-2,4-dione
- la 5,5-diméthyl-1-(3-hydroxyméthyl-pyridin-4-ylméthyl)-3-(4-trifluorométhanesulfonyl-phényl)-imidazolidine-2,4-dione
- la 5,5-diméthyl-1-(3-chloro-pyridin-4-ylméthyl)-3-(4-trifluorométhanesulfonyl-phényl)-imidazolidine-2,4-dione
- la 5,5-diméthyl-1-(3-fluoro-pyridin-4-ylméthyl)-3-(4-trifluorométhanesulfonyl-phényl)-imidazolidine-2,4-dione
- la 5,5-diméthyl-1-(3-bromo-pyridin-4-ylméthyl)-3-(4-trifluorométhanesulfonyl-phényl)-imidazolidine-2,4-dione
- la 5,5-diméthyl-1-(3-amino-pyridin-4-ylméthyl)-3-(4-trifluorométhanesulfonyl-phényl)-imidazolidine-2,4-dione
- la 5,5-diméthyl-1-(3-méthylamino-pyridin-4-ylméthyl)-3-(4-trifluorométhanesulfonyl-phényl)-imidazolidine-2,4-dione
- la 5,5-diméthyl-1-(3-éthylamino-pyridin-4-ylméthyl)-3-(4-trifluorométhanesulfonyl-phényl)-imidazolidine-2,4-dione
- la 5,5-diméthyl-1-(3-isopropylamino-pyridin-4-ylméthyl)-3-(4-trifluorométhanesulfonyl-phényl)-imidazolidine-2,4-dione
- la 5,5-diméthyl-1-(3-phénylamino-pyridin-4-ylméthyl)-3-(4-trifluorométhanesulfonyl-phényl)-imidazolidine-2,4-dione
- la 5,5-diméthyl-1-(3-cyclohéxylamino-pyridin-4-ylméthyl)-3-(4-trifluorométhanesulfonyl-phényl)-imidazolidine-2,4-dione
- la 5,5-diméthyl-1-(3-diéthylamino-pyridin-4-ylméthyl)-3-(4-trifluorométhanesulfonyl-phényl)-imidazolidine-2,4-dione
- la 5,5-diméthyl-1-(3-N-cyclohéxyl-N-méthylamino-pyridin-4-ylméthyl)-3-(4-trifluorométhanesulfonyl-phényl)-imidazolidine-2,4-dione
- la 5,5-diméthyl-1-(3-pipéridino-pyridin-4-ylméthyl)-3-(4-trifluorométhanesulfonyl-phényl)-imidazolidine-2,4-dione
- la 5,5-diméthyl-1-(3-morpholino-pyridin-4-ylméthyl)-3-(4-trifluorométhanesulfonyl-phényl)-imidazolidine-2,4-dione
- la 5,5-diméthyl-1-[3-(4-méthylpipérazino)diéthylamino-pyridin-4-ylméthyl]-3-(4-trifluorométhanesulfonyl-phényl)-imidazolidine-2,4-dione
- la 5,5-diméthyl-1-[3-(1H-pyrazol-1yl)-pyridin-4-ylméthyl]-3-(4-trifluorométhanesulfonyl-phényl)-imidazolidine-2,4-dione
- la 5,5-diméthyl-1-[3-(2-benzoylhydrazino)-pyridin-4-ylméthyl]-3-(4-trifluorométhanesulfonyl-phényl)-imidazolidine-2,4-dione
- la 5,5-diméthyl-1-(3-benzoylamino-pyridin-4-ylméthyl)-3-(4-trifluorométhanesulfonyl-phényl)-imidazolidine-2,4-dione
- la 5,5-diméthyl-1-[3-(4-chlorobenzoylamino)-pyridin-4-ylméthyl]-3-(4-trifluorométhanesulfony-1-phényl)-imidazolidine-2,4-dione
- la 5,5-diméthyl-1-[3-(3-chlorobenzoylamino)-pyridin-4-ylméthyl]-3-(4-trifluorométhanesulfonyl-phényl)-imidazolidine-2,4-dione
- la 5,5-diméthyl-1-[3-(2-chlorobenzoylamino)-pyridin-4-ylméthyl]-3-(4-trifluorométhanesulfonyl-phényl)-imidazolidine-2,4-dione
- la 5,5-diméthyl-1-[3-(4-fluorobenzoylamino)-pyridin-4-ylméthyl]-3-(4-trifluorométhanesulfonyl-phényl)-imidazolidine-2,4-dione
- la 5,5-diméthyl-1-[3-(4-méthoxybenzoylamino)-pyridin-4-ylméthyl]-3-(4-trifluorométhanesulfonyl-phényl)-imidazolidine-2,4-dione
- la 5,5-diméthyl-1-(3-acétamido-pyridin-4-ylméthyl)-3-(4-trifluorométhanesulfonyl-phényl)-imidazolidine-2,4-dione
- la 5,5-diméthyl-1-(3-phénylacétamido-pyridin-4-ylméthyl)-3-(4-trifluorométhanesulfonyl-phényl)-imidazolidine-2,4-dione
- la 5,5-diméthyl-1-[3-(4-méthylpipérazino)acétamido-pyridin-4-ylméthyl]-3-(4-trifluorométhanesulfonyl-phényl)-imidazolidine-2,4-dione
- la 5,5-diméthyl-1-(3-cyclohexanylcarboxamido-pyridin-4-ylméthyl)-3-(4-trifluorométhanesulfonyl-phényl)-imidazolidine-2,4-dione
- la 5,5-diméthyl-1-[3-(1-méthyl-4-pipéridinyl)carbonyl-amino-pyridin-4-ylméthyl]-3-(4-trifluorométhanesulfonyl-phényl)-imidazolidine-2,4-dione
- la 5,5-diméthyl-1-[3-(2-furanylcarbonyl)amino-pyridin-4-ylméthyl]-3-(4-trifluorométhanesulfonyl-phényl)-imidazolidine-2,4-dione
- la 5,5-diméthyl-1-(3-éthoxycarbonylamino-pyridin-4-ylméthyl)-3-(4-trifluorométhanesulfonyl-phényl)-imidazolidine-2,4-dione
- la 5,5-diméthy-1-1-(3-isopropoxycarbonylamino-pyridin-4-ylméthyl)-3-(4-trifluorométhanesulfonyl-phényl)-imidazolidine-2,4-dione
- la 5,5-diméthyl-1-(3-butoxycarbonylamino-pyridin-4-ylméthyl)-3-(4-trifluorométhanesulfonyl-phényl)-imidazolidine-2,4-dione
- la 5,5-diméthyl-1-[3-(2-diéthylaminoéthoxy-carbonylamino)-pyridin-4-ylméthyl]-3-(4-trifluoro-(4-trifluorométhanesulfonyl-phényl)-imidazolidine-2,4-dione
- la 5,5-diméthyl-1-(3-phénoxycarbonylamino-pyridin-4-ylméthyl)-3-(4-trifluorométhanesulfonyl-phényl)-imidazolidine-2,4-dione
- la 5,5-diméthyl-1-[3-(4-chloroanilino)carbonylamino-pyridin-4-ylméthyl)]-3-(4-trifluorométhanesulfonyl-phényl)-imidazolidine-2,4-dione
- la 5,5-diméthyl-1-(3-anilinocarbonylamino-pyridin-4-ylméthyl)-3-(4-trifluorométhanesulfonyl-phényl)-imidazolidine-2,4-dione
- la 5,5-diméthyl-1-(3-benzylaminocarbonylamino-pyridin-4-ylméthyl)-3-(4-trifluorométhanesulfonyl-phényl)-imidazolidine-2,4-dione
- la 5,5-diméthyl-1-[3-(2-pyridylamino)carbonylamino-pyridin-4-ylméthyl)]-3-(4-trifluorométhanesulfonyl-phényl)-imidazolidine-2,4-dione
- la 5,5-diméthyl-1-(3-uréido-pyridin-4-ylméthyl)-3-(4-trifluorométhanesulfonyl-phényl)-imidazolidine-2,4-dione
- la 5,5-diméthyl-1-(3-benzylaminosulfonylamino-pyridin-4-ylméthyl)-3-(4-trifluorométhanesulfonyl-phényl)-imidazolidine-2,4-dione
- la 5,5-diméthyl-1-(3-méthylaminosulfonylamino-pyridin-4-ylméthyl)-3-(4-trifluorométhanesulfonyl-phényl)-imidazolidine-2,4-dione
- la 5,5-diméthyl-1-(3-aminosulfonylamino-pyridin-4-ylméthyl)-3-(4-tiifluoromêthanesulfonyl-phényl)-imidazolidine-2,4-dione
- la 5,5-diméthyl-1-(3-morpholinosulfonylamino-pyridin-4-ylméthyl)-3-(4-trifluorométhanesulfonyl-phényl)-imidazolidine-2,4-dione
- la 5,5-diméthyl-1-(3-diméthylaminosulfonylamino-pyridin-4-ylméthyl)-3-(4-trifluorométhanesulfonyl-phényl)-imidazolidine-2,4-dione
- la 5,5-diméthyl-1-(3-anilinosulfonylamino-pyridin-4-ylméthyl)-3-(4-trifluorométhanesulfonyl-phényl)-
- la 5,5-diméthyl-1-(3-phénylsulfonylamino-pyridin-4-ylméthyl)-3-(4-trifluorométhanesulfonyl-phényl)-imidazolidine-2,4-dione
- la 5,5-diméthyl-1-[3-(2-thiényl)sulfonylamino-pyridin-4-ylméthyl]-3-(4-trifluorométhanesulfonyl-phényl)-imidazolidine-2,4-dione
- la 5,5-diméthyl-1-(3-méthylsulfonylamino-pyridin-4-ylméthyl)-3-(4-trifluorométhanesulfonyl-phényl)-imidazolidine-2,4-dione
- la 5,5-diméthyl-1-[3-(4-chlorophényl)sulfonylamino-pyridin-4-ylméthyl]-3-(4-trifluorométhanesulfonyl-phényl)-imidazolidine-2,4-dione
- la 5,5-diméthyl-1-(3-benzylsulfonylamino-pyridin-4-ylméthyl)-3-(4-trifluorométhanesulfonyl-phényl)-imidazolidine-2,4-dione
- la 5,5-diméthyl-1-(3-hydroxycarbonyl-pyridin-4-ylméthyl)-3-(4-trifluorométhanesulfonyl-phényl)-imidazolidine-2,4-dione
- la 5,5-diméthyl-1-(3-éthoxycarbonyl-pyridin-4-ylméthyl)-3-(4-trifluorométhanesulfonyl-phényl)-imidazolidine-2,4-dione
- la 5,5-diméthyl-1-(3-ter-butoxycarbonyl-pyridin-4-ylméthyl)-3-(4-trifluorométhanesulfonyl-phényl)-imidazolidine-2,4-dione
- la 5,5-diméthyl-1-(3-phénoxycarbonyl-pyridin-4-ylméthyl)-3-(4-trifluorométhanesulfonyl-phényl)-imidazolidine-2,4-dione
- la 5,5-diméthyl-1-[3-(4-méthylpipérazinocarbonyl)-pyridin-4-ylméthyl]-3-(4-trifluorométhanesulfonyl-phényl)-imidazolidine-2,4-dione
- la 5,5-diméthyl-1-[3-(méthylamino)carbonyl-pyridin-4-ylméthyl]-3-(4-trifluorométhanesulfonyl-phényl)-imidazolidine-2,4-dione
- la 5,5-diméthyl-1-[3-(diméthylamino)carbonyl-pyridin-4-ylméthyl]-3-(4-trifluorométhanesulfonyl-phényl)-imidazolidine-2,4-dione
- la 5,5-diméthyl-1-(3-pipéridinocarbonyl-pyridin-4-ylméthyl)-3-(4-trifluorométhanesulfonyl-phényl)-imidazolidine-2,4-dione
- la 5,5-diméthyl-1-(3-morpholinocarbonyl-pyridin-4-ylméthyl)-3-(4-trifluorométhanesulfonyl-phényl)-imidazolidine-2,4-dione
- la 5,5-diméthyl-1-[3-(4-phénylpipérazinocarbonyl)-pyridin-4-ylméthyl]-3-(4-trifluorométhanesulfonyl-phényl)-imidazolidine-2,4-dione
- la 5,5-diméthyl-1-(3-phénylaminocarbonyl-pyridin-4-ylméthyl)-3-(4-trifluorométhanesulfonyl-phényl)-imidazolidine-2,4-dione
- la 5,5-diméthyl-1-[3-(méthylphénylamino)carbonyl-pyridin-4-ylméthyl]-3-(4-trifluorométhanesulfonyl-phényl)-imidazolidine-2,4-dione
- la 5,5-diméthyl-1-[3-(4-méthoxyphényl)méthylaminocarbonyl-pyridin-4-ylméthyl]-3-(4-trifluorométhanesulfonyl-phényl)-imidazolidine-2,4-dione
- la 5,5-diméthyl-1-[3-(4-chlorophényl)méthylaminocarbonyl-pyridin-4-ylméthyl]-3-(4-trifluorométhanesulfonyl-phényl)-imidazolidine-2,4-dione
- la 5,5-diméthyl-1-[3-(4-morpholinylamino)carbonyl-pyridin-4-ylméthyl]-3-(4-trifluorométhanesulfonyl-phényl)-imidazolidine-2,4-dione
- la 5,5-diméthyl-1-(3-aminocarbonyl-pyridin-4-ylméthyl)-3-(4-trifluorométhanesulfonyl-phényl)-imidazolidine-2,4-dione
- la 5,5-diméthyl-1-(3-phénoxy-pyridin-4-ylméthyl)-3-(4-trifluorométhanesulfonyl-phényl)-imidazolidine-2,4-dione
- la 5,5-diméthyl-1-(3-méthoxy-pyridin-4-ylméthyl)-3-(4-trifluorométhanesulfonyl-phényl)-imidazolidine-2,4-dione
- la 5,5-diméthyl-1-(3-éthoxy-pyridin-4-ylméthyl)-3-(4-trifluorométhanesulfonyl-phényl)-imidazolidine-2,4-dione
- la 5,5-diméthyl-1-[3-(1-pyrrolidinyl)éthoxy-pyridin-4-ylméthyl]-3-(4-trifluorométhanesulfonyl-phényl)-imidazolidine-2,4-dione
- la 5,5-diméthyl-1-(3-benzyloxy-pyridin-4-ylméthyl)-3-(4-trifluorométhanesulfonyl-phényl)-imidazolidine-2,4-dione
- la 5,5-diméthyl-1-(3-benzoyloxy-pyridin-4-ylméthyl)-3-(4-trifluorométhanesulfonyl-phényl)-imidazolidine-2,4-dione
- la 5,5-diméthyl-1-[3-(4,5-dihydro-1H-imidazol-2-yl)-pyridin-4-ylméthyl]-3-(4-trifluorométhanesulfonyl-phényl)-imidazolidine-2,4-dione
- la 5,5-diméthyl-1-[3-(1H-imidazol-2-yl)-pyridin-4-ylméthyl]-3-(4-trifluorométhanesulfonyl-phényl)-imidazolidine-2,4-dione
- la 5,5-diméthyl-1-[3-(1H-tétrazol-5-yl)-pyridin-4-ylméthyl]-3-(4-trifluorométhanesulfonyl-phényl)-imidazolidine-2,4-dione
- la 5,5-diméthyl-1-(3-amidino-pyridin-4-ylméthyl)-3-(4-trifluorométhanesulfonyl-phényl)-imidazolidine-2,4-dione
- la 5,5-diméthyl-1-(3-diméthylaminométhyl-pyridin-4-ylméthyl)3-(4-trifluorométhanesulfonyl-phényl)-imidazolidine-2,4-dione
- la 5,5-diméthyl-1-(3-aminométhyl-pyridin-4-ylméthyl)-3-(4-trifluorométhanesulfonyl-phényl)-imidazolidine-2,4-dione
- la 4-(2-méthyl-pyridin)-4-ylméthyl-6-(4-trifluorométhanesulfonyl-phényl)-4,6-diaza-spiro[2.4]heptane-5,7-dione
- la 4-(2-éthyl-pyridin)-4-ylméthyl-6-(4-trifluorométhanesulfonyl-phényl)-4,6-diaza-spiro[2.4]heptane-5,7-dione
- la 4-(2-isopropyl-pyridin)-4-ylméthyl-6-(4-trifluorométhanesulfonyl-phényl)-4,6-diaza-spiro[2.4]heptane-5,7-dione
- la 4-(2-ter-butyl-pyridin)-4-ylméthyl-6-(4-trifluorométhanesulfonyl-phényl)-4,6-diaza-spiro[2.4]heptane-5,7-dione
- la 4-(2-isobutyl-pyridin)-4-ylméthyl-6-(4-trifluorométhanesulfonyl-phényl)-4,6-diaza-spiro[2.4]heptane-5,7-dione
- la 4-(2-cyclohéxyl-pyridin)-4-ylméthyl-6-(4-trifluorométhanesulfonyl-phényl)-4,6-diaza-spiro[2.4]heptane-5,7-dione
- la 4-(2-phényl-pyridin)-4-ylméthyl-6-(4-trifluorométhanesulfonyl-phényl)-4,6-diaza-spiro[2.4]heptane-5,7-dione
- la 4-[2-(4-chlorophényl)-pyridin]-4-ylméthyl-6-(4-trifluorométhanesulfonyl-phényl)-4,6-diaza-spiro[2.4]heptane-5,7-dione
- la 4-[2-(3-chlorophényl)-pyridin]-4-ylméthyl-6-(4-trifluorométhanesulfonyl-phényl)-4,6-diaza-spiro[2.4]heptane-5,7-dione
- la 4-[2-(2-chlorophényl)-pyridin]-4-ylméthyl-6-(4-trifluorométhanesulfonyl-phényl)-4,6-diaza-spiro[2.4]heptane-5,7-dione
- la 4-[2-(4-fluorophényl)-pyridin]-4-ylméthyl-6-(4-trifluorométhanesulfonyl-phényl)-4,6-diaza-spiro[2.4]heptane-5,7-dione
- la 4-[2-(4-méthoxyphényl)-pyridin]-4-ylméthyl-6-(4-trifluorométhanesulfonyl-phényl)-4,6-diaza-spiro[2.4]heptane-5,7-dione
- la 4-(2-benzyl-pyridin)-4-ylméthyl-6-(4-trifluorométhanesulfonyl-phényl)-4,6-diaza-spiro[2.4]heptane-5,7-dione
- la 4-(2,2'bipyridin-4-ylméthyl-6-(4-trifluorométhanesulfonyl-phényl)-4,6-diaza-spiro[2.4]heptane-5,7-dione
- la 4-(2-hydroxyméthyl-pyridin)-4-ylméthyl-6-(4-trifluorométhanesulfonyl-phényl)-4,6-diaza-spiro[2.4]heptane-5,7-dione
- la 4-(2-chloro-pyridin-4-ylméthyl-6-(4-trifluoro-méthanesulfonyl-phényl)-4,6-diaza-spiro[2.4]heptane-5,7-dione

- la 4-(2-fluoro-pyridin-4-ylméthyl-6-(4-trifluorométhanesulfonyl-phényl)-4,6-diaza-spiro[2.4]heptane-5,7-dione
- la 4-(2-bromo-pyridin-4-ylméthyl-6-(4-trifluorométhanesulfonyl-phényl)-4,6-diaza-spiro[2.4]heptane-5,7-dione
- la 4-(2-amino-pyridin-4-ylméthyl-6-(4-trifluorométhanesulfonyl-phényl)-4,6-diaza-spiro[2.4]heptane-5,7-dione
- la 4-(2-méthylamino-pyridin-4-ylméthyl-6-(4-trifluorométhanesulfonyl-phényl)-4,6-diaza-spiro[2.4]heptane-5,7-dione
- la 4-(2-éthylamino-pyridin-4-ylméthyl-6-(4-trifluorométhanesulfonyl-phényl)-4,6-diaza-spiro[2.4]heptane-5,7-dione
- la 4-(2-isopropylamino-pyridin-4-ylméthyl-6-(4-trifluorométhanesulfonyl-phényl)-4,6-diaza-spiro[2.4]heptane-5,7-dione
- la 4-(2-phénylamino-pyridin-4-ylméthyl-6-(4-trifluorométhanesulfonyl-phényl)-4,6-diaza-spiro[2.4]heptane-5,7-dione
- la 4-(2-cyclohéxylamino-pyridin-4-ylméthyl-6-(4-trifluorométhanesulfonyl-phényl)-4,6-diaza-spiro[2.4]heptane-5,7-dione
- la 4-(2-diéthylamino-pyridin-4-ylméthyl-6-(4-trifluorométhanesulfonyl-phényl)-4,6-diaza-spiro[2.4]heptane-5,7-dione
- la 4-(2-N-cyclohéxyl-N-méthylamino-pyridin-4-ylméthyl-6-(4-trifluorométhanesulfonyl-phényl)-4,6-diaza-spiro[2.4]heptane-5,7-dione
- la 4-(2-pipéridino-pyridin-4-ylméthyl-6-(4-trifluorométhanesulfonyl-phényl)-4,6-diaza-spiro[2.4]heptane-5,7-dione
- la 4-(2-morpholino-pyridin-4-ylméthyl-6-(4-trifluorométhanesulfonyl-phényl)-4,6-diaza-spiro[2.4]heptane-5,7-dione
- la 4-[2-(4-méthylpipérazino)diéthylamino-pyridin-4-ylméthyl-6-(4-trifluorométhanesulfonyl-phényl)-4,6-diaza-spiro[2.4]heptane-5,7-dione
- la 4-[2-(1H-pyrazol-1yl)-pyridin]-4-ylméthyl-6-(4-trifluorométhanesulfonyl-phényl)-4,6-diaza-spiro[2.4]heptane-5,7-dione
- la 4-[2-(2-benzoylhydrazino)-pyridin-4-ylméthyl-6-(4-trifluorométhanesulfonyl-phényl)-4,6-diaza-spiro[2.4]heptane-5,7-dione
- la 4-(2-benzoylamino-pyridin-4-ylméthyl-6-(4-trifluorométhanesulfonyl-phényl)-4,6-diaza-spiro[2.4]heptane-5,7-dione
- la 4-[2-(4-chlorobenzoylamino)-pyridin-4-ylméthyl-6-(4-trifluorométhanesulfonyl-phényl)-4,6-diaza-spiro[2.4]heptane-5,7-dione
- la 4-[2-(3-chlorobenzoylamino)-pyridin-4-ylméthyl-6-(4-trifluorométhanesulfonyl-phényl)-4,6-diaza-spiro[2.4]heptane-5,7-dione
- la 4-[2-(2-chlorobenzoylamino)-pyridin-4-ylméthyl-6-(4-trifluorométhanesulfonyl-phényl)-4,6-diaza-spiro[2.4]heptane-5,7-dione
- la 4-[2-(4-fluorobenzoylamino)-pyridin-4-ylméthyl-6-(4-trifluorométhanesulfonyl-phényl)-4,6-diaza-spiro[2.4]heptane-5,7-dione
- la 4-[2-(4-méthoxybenzoylamino)-pyridin-4-ylméthyl-6-(4-trifluorométhanesulfonyl-phényl)-4,6-diaza-spiro[2.4]heptane-5,7-dione
- la 4-(2-acétamido-pyridin-4-ylméthyl-6-(4-trifluorométhanesulfonyl-phényl)-4,6-diaza-spiro[2.4]heptane-5,7-dione
- la 4-(2-phénylacétamido-pyridin-4-ylméthyl-6-(4-trifluorométhanesulfonyl-phényl)-4,6-diaza-spiro[2.4]heptane-5,7-dione
- la 4-[2-(4-méthylpipérazino)acétamido-pyridin-4-ylméthyl-6-(4-trifluorométhanesulfonyl-phényl)-4,6-diaza-spiro[2.4]heptane-5,7-dione
- la 4-(2-cyclohéxanylcarboxamido-pyridin-4-ylméthyl-6-(4-trifluorométhanesulfonyl-phényl)-4,6-diaza-spiro[2.4]heptane-5,7-dione
- la 4-[2-(1-méthyl-4-pipéridinyl)carbonylamino-pyridin-4-ylméthyl-6-(4-trifluorométhanesulfonyl-phényl)-4,6-diaza-spiro[2.4]heptane-5,7-dione
- la 4-[2-(2-furanylcarbonyl)amino-pyridin-4-ylméthyl-6-(4-trifluorométhanesulfonyl-phényl)-4,6-diaza-spiro[2.4]heptane-5,7-dione
- la 4-(2-éthoxycarbonylamino-pyridin-4-ylméthyl-6-(4-trifluorométhanesulfonyl-phényl)-4,6-diaza-spiro[2.4]heptane-5,7-dione
- la 4-(2-isopropoxycarbonylamino-pyridin-4-ylméthyl-6-(4-trifluorométhanesulfonyl-phényl)-4,6-diaza-spiro[2.4]heptane-5,7-dione
- la 4-(2-butoxycarbonylamino-pyridin-4-ylméthyl-6-(4-trifluorométhanesulfonyl-phényl)-4,6-diaza-spiro[2.4]heptane-5,7-dione
- la 4-[2-(2-diéthylaminoéthoxycarbonylamino)-pyridin-4-ylméthyl-6-(4-trifluorométhanesulfonyl-phényl)-4,6-diaza-spiro[2.4]heptane-5,7-dione
- la 4-(2-phénoxycarbonylamino-pyridin-4-ylméthyl-6-(4-trifluorométhanesulfonyl-phényl)-4,6-diaza-spiro[2.4]heptane-5,7-dione
- la 4-[2-(4-chloroanilino)carbonylamino-pyridin-4-ylméthyl)-6-(4-trifluorométhanesulfonyl-phényl)-4,6-diaza-spiro[2.4]heptane-5,7-dione
- la 4-(2-anilinoca-rbonylamino-pyridin-4-ylméthyl-6-(4-trifluorométhanesulfonyl-phényl)-4,6-diaza-spiro[2.4]heptane-5,7-dione
- la 4-(2-benzylaminocarbonylamino-pyridin-4-ylméthyl-6-(4-trifluorométhanesulfonyl-phényl)-4,6-diaza-spiro[2.4]heptane-5,7-dione
- la 4-[2-(2-pyridylamino)carbonylamino-pyridin-4-ylméthyl)-6-(4-trifluorométhanesulfonyl-phényl)-4,6-diaza-spiro[2.4]heptane-5,7-dione
- la 4-(2-uréido-pyridin-4-ylméthyl-6-(4-trifluorométhanesulfonyl-phényl)-4,6-diaza-spiro[2.4]heptane-5,7-dione
- la 4-(2-benzylaminosulfonylamino-pyridin-4-ylméthyl-6-(4-trifluorométhanesulfonyl-phényl)-4,6-diaza-spiro[2.4]heptane-5,7-dione
- la 4-(2-méthylaminosulfonylamino-pyridin-4-ylméthyl-6-(4-trifluorométhanesulfonyl-phényl)-4,6-diaza-spiro[2.4]heptane-5,7-dione
- la 4-(2-aminosulfonylamino-pyridin-4-ylméthyl-6-(4-trifluorométhanesulfonyl-phényl)-4,6-diaza-spiro[2.4]heptane-5,7-dione
- la 4-(2-morpholinosulfonylamino-pyridin-4-ylméthyl-6-(4-trifluorométhanesulfonyl-phényl)-4,6-diaza-spiro[2.4]heptane-5,7-dione
- la 4-(2-diméthylaminosulfonylamino-pyridin-4-ylméthyl-6-(4-trifluorométhanesulfonyl-phényl)-4,6-diaza-spiro[2.4]heptane-5,7-dione
- la 4-(2-anilinosulfonylamino-pyridin-4-ylméthyl-6-(4-trifluorométhanesulfonyl-phényl)-4,6-diaza-spiro[2.4]heptane-5,7-dione
- la 4-(2-phénylsulfonylamino-pyridin-4-ylméthyl-6-(4-trifluorométhanesulfonyl-phényl)-4,6-diaza-spiro[2.4]heptane-5,7-dione
- la 4-[2-(2-thiényl)sulfonylamino-pyridin-4-ylméthyl-6-(4-trifluorométhanesulfonyl-phényl)-4,6-diaza-spiro[2.4]heptane-5,7-dione
- la 4-(2-méthylsulfonylamino-pyridin-4-ylméthyl-6-(4-trifluorométhanesulfonyl-phényl)-4,6-diaza-spiro[2.4]heptane-5,7-dione
- la 4-[2-(4-chlorophényl)sulfonylamino-pyridin-4-ylméthyl-6-(4-trifluorométhanesulfonyl-phényl)-4,6-diaza-spiro[2.4]heptane-5,7-dione
- la 4-(2-benzylsulfonylamino-pyridin-4-ylméthyl-6-(4-trifluorométhanesulfonyl-phényl)-4,6-diaza-spiro[2.4]heptane-5,7-dione
- la 4-(2-hydroxycarbonyl-pyridin)-4-ylméthyl-6-(4-trifluorométhanesulfonyl-phényl)-4,6-diaza-spiro[2.4]heptane-5,7-dione
- la 4-(2-éthoxycarbonyl-pyridin)-4-ylméthyl-6-(4-trifluorométhanesulfonyl-phényl)-4,6-diaza-spiro[2.4]heptane-5,7-dione
- la 4-(2-ter-butoxycarbonyl-pyridin)-4-ylméthyl-6-(4-trifluorométhanesulfonyl-phényl)-4,6-diaza-spiro[2.4]heptane-5,7-dione
- la 4-(2-phénoxycarbonyl-pyridin)-4-ylméthyl-6-(4-trifluorométhanesulfonyl-phényl)-4,6-diaza-spiro[2.4]heptane-5,7-dione
- la 4-[2-(4-méthylpipérazinocarbonyl)-pyridin]-4-ylméthyl-6-(4-trifluorométhanesulfonyl-phényl)-4,6-diaza-spiro[2.4]heptane-5,7-dione
- la 4-[2-(méthylamino)carbonyl-pyridin)-4-ylméthyl-6-(4-trifluorométhanesulfonyl-phényl)-4,6-diaza-spiro[2.4]heptane-5,7-dione
- la 4-[2-(diméthylamino)carbonyl-pyridin)-4-ylméthyl-6-(4-trifluorométhanesulfonyl-phényl)-4,6-diaza-spiro[2.4]heptane-5,7-dione
- la 4-(2-pipéridinocarbonyl-pyridin)-4-ylméthyl-6-(4-trifluorométhanesulfonyl-phényl)-4,6-diaza-spiro[2.4]heptane-5,7-dione
- la 4-(2-morpholinocarbonyl-pyridin)-4-ylméthy-1-6-(4-trifluorométhanesulfonyl-phényl)-4,6-diaza-spiro[2.4]heptane-5,7-dione
- la 4-[2-(4-phénylpipérazinocarbonyl)-pyridin]-4-ylméthyl-6-(4-trifluorométhanesulfonyl-phényl)-4,6-diaza-spiro[2.4]heptane-5,7-dione
- la 4-(2-phénylaminocarbonyl-pyridin)-4-ylméthyl-6-(4-trifluorométhanesulfonyl-phényl)-4,6-diaza-spiro[2.4]heptane-5,7-dione
- la 4-[2-(méthylphénylamino)carbonyl-pyridin)-4-ylméthyl-6-(4-trifluorométhanesulfonyl-phényl)-4,6-diaza-spiro[2.4]heptane-5,7-dione
- la 4-[2-(4-méthoxyphényl)méthylaminocarbonyl-pyridin)-4-ylméthyl-6-(4-trifluorométhanesulfonyl-phényl)-4,6-diaza-spiro[2.4]heptane-5,7-dione
- la 4-[2-(4-chlorophényl)méthylaminocarbonyl-pyridin)-4-ylméthyl-6-(4-trifluorométhanesulfonyl-phényl)-4,6-diaza-spiro[2.4]heptane-5,7-dione
- la 4-[2-(4-morpholinylamino)carbonyl-pyridin)-4-ylméthyl-6-(4-trifluorométhanesulfonyl-phényl)-4,6-diaza-spiro[2.4]heptane-5,7-dione
- la 4-(2-aminocarbonyl-pyridin)-4-ylméthyl-6-(4-trifluorométhanesulfonyl-phényl)-4,6-diaza-spiro[2.4]heptane-5,7-dione
- la 4-(2-phénoxy-pyridin-4-ylméthyl-6-(4-trifluoro-méthanesulfonyl-phényl)-4,6-diaza-spiro[2.4]heptane-5,7-dione
- la 4-(2-méthoxy-pyridin-4-ylméthyl-6-(4-trifluorométhanesulfonyl-phényl)-4,6-diaza-spiro[2.4]heptane-5,7-dione
- la 4-(2-éthoxy-pyridin-4-ylméthyl-6-(4-trifluorométhanesulfonyl-phényl)-4,6-diaza-spiro[2.4]heptane-5,7-dione
- la 4-[2-(1-pyrrolidinyl)éthoxy-pyridin-4-ylméthyl-6-(4-trifluorométhanesulfonyl-phényl)-4,6-diaza-spiro[2.4]heptane-5,7-dione
- la 4-(2-benzyloxy-pyridin-4-ylméthyl-6-(4-trifluorométhanesulfonyl-phényl)-4,6-diaza-spiro[2.4]heptane-5,7-dione
- la 4-(2-benzoyloxy-pyridin-4-ylméthyl-6-(4-trifluorométhanesulfonyl-phényl)-4,6-diaza-spiro[2.4]heptane-5,7-dione
- la 4-[2-(4,5-dihydro-1H-imidazol-2-yl)-pyridin]-4-ylméthyl-6-(4-trifluorométhanesulfonyl-phényl)-4,6-diaza-spiro[2.4]heptane-5,7-dione
- la 4-[2-(1H-imidazol-2-yl)-pyridin]-4-ylméthyl-6-(4-trifluorométhanesulfonyl-phényl)-4,6-diaza-spiro[2.4]heptane-5,7-dione
- la 4-[2-(1H-tétrazol-5-yl)-pyridin]-4-ylméthyl-6-(4-trifluorométhanesulfonyl-phényl)-4,6-diaza-spiro[2.4]heptane-5,7-dione
- la 4-(2-amidino-pyridin-4-ylméthyl-6-(4-trifluorométhanesulfonyl-phényl)-4,6-diaza-spiro[2,4]heptane-5,7-dione
- la 4-(2-diméthylaminométhyl-pyridin)-4-ylméthyl-6-(4-trifluorométhanesulfonyl-phényl)-4,6-diaza-spiro[2.4]heptane-5,7-dione
- la 4-(2-aminométhyl-pyridin)-4-ylméthyl-6-(4-trifluorométhanesulfonyl-phényl)-4,6-diaza-spiro[2.4]heptane-5,7-dione
- la 4-(3-méthyl-pyridin)-4-ylméthyl-6-(4-trifluorométhanesulfonyl-phényl)-4,6-diaza-spiro[2.4]heptane-5,7-dione
- la 4-(3-éthyl-pyridin)-4-ylméthyl-6-(4-trifluorométhanesulfonyl-phényl)-4,6-diaza-spiro[2.4]heptane-5,7-dione
- la 4-(3-isopropyl-pyridin)-4-ylméthyl-6-(4-trifluorométhanesulfonyl-phényl)-4,6-diaza-spiro[2.4]heptane-5,7-dione
- la 4-(3-ter-butyl-pyridin)-4-ylméthyl-6-(4-trifluorométhanesulfonyl-phényl)-4,6-diaza-spiro[2.4]heptane-5,7-dione
- la 4-(3-isobutyl-pyridin)-4-ylméthyl-6-(4-trifluorométhanesulfonyl-phényl)-4,6-diaza-spiro[2.4]heptane-5,7-dione
- la 4-(3-cyclohéxyl-pyridin)-4-ylméthyl-6-(4-trifluorométhanesulfonyl-phényl)-4,6-diaza-spiro[2.4]heptane-5,7-dione
- la 4-(3-phényl-pyridin)-4-ylméthyl-6-(4-trifluorométhanesulfonyl-phényl)-4,6-diaza-spiro[2.4]heptane-5,7-dione
- la 4-[3-(4-chlorophényl)-pyridin]-4-ylméthyl-6-(4-trifluorométhanesulfonyl-phényl)-4,6-diaza-spiro[2.4]heptane-5,7-dione
- la 4-[3-(3-chlorophényl)-pyridin]-4-ylméthyl-6-(4-trifluorométhanesulfonyl-phényl)-4,6-diaza-spiro[2.4]heptane-5,7-dione
- la 4-[3-(2-chlorophényl)-pyridin]-4-ylméthyl-6-(4-trifluorométhanesulfonyl-phényl)-4,6-diaza-spiro[2.4]heptane-5,7-dione
- la 4-[3-(4-fluorophényl)-pyridin]-4-ylméthyl-6-(4-trifluorométhanesulfonyl-phényl)-4,6-diaza-spiro[2.4]heptane-5,7-dione
- la 4-[3-(4-méthoxyphényl)-pyridin]-4-ylméthyl-6-(4-trifluorométhanesulfonyl-phényl)-4,6-diaza-spiro[2.4]heptane-5,7-dione
- la 4-(3-benzyl-pyridin)-4-ylméthyl-6-(4-trifluorométhanesulfonyl-phényl)-4,6-diaza-spiro[2.4]heptane-5,7-dione
- la 4-(3,2'bipyridin-4-ylméthyl-6-(4-trifluorométhanesulfonyl-phényl)-4,6-diaza-spiro[2.4]heptane-5,7-dione
- la 4-(3-hydroxyméthyl-pyridin)-4-ylméthyl-6-(4-trifluorométhanesulfonyl-phényl)-4,6-diaza-spiro[2.4]heptane-5,7-dione
- la 4-(3-chloro-pyridin-4-ylméthyl-6-(4-trifluorométhanesulfonyl-phényl)-4,6-diaza-spiro[2.4]heptane-5,7-dione
- la 4-(3-fluoro-pyridin-4-ylméthyl-6-(4-trifluorométhanesulfonyl-phényl)-4,6-diaza-spiro[2.4]heptane-5,7-dione
- la 4-(3-bromo-pyridin-4-ylméthyl-6-(4-trifluorométhanesulfonyl-phényl)-4,6-diaza-spiro[2.4]heptane-5,7-dione
- la 4-(3-amino-pyridin-4-ylméthyl-6-(4-trifluorométhanesulfonyl-phényl)-4,6-diaza-spiro[2.4]heptane-5,7-dione
- la 4-(3-méthylamino-pyridin-4-ylméthyl-6-(4-trifluorométhanesulfonyl-phényl)-4,6-diaza-spiro[2.4]heptane-5,7-dione
- la 4-(3-éthylamino-pyridin-4-ylméthyl-6-(4-trifluorométhanesulfonyl-phényl)-4,6-diaza-spiro[2.4]heptane-5,7-dione
- la 4-(3-isopropylamino-pyridin-4-ylméthyl-6-(4-trifluorométhanesulfonyl-phényl)-4,6-diaza-spiro[2.4]heptane-5,7-dione
- la 4-(3-phénylamino-pyridin-4-ylméthyl-6-(4-trifluorométhanesulfonyl-phényl)-4,6-diaza-spiro[2.4]heptane-5,7-dione
- la 4-(3-cyclohéxylamino-pyridin-4-ylméthyl-6-(4-trifluorométhanesulfonyl-phényl)-4,6-diaza-spiro[2.4]heptane-5,7-dione
- la 4-(3-diéthylamino-pyridin-4-ylméthyl-6-(4-trifluorométhanesulfonyl-phényl)-4,6-diaza-spiro[2.4]heptane-5,7-dione
- la 4-(3-N-cyclohéxyl-N-méthylamino-pyridin-4-ylméthyl-6-(4-trifluorométhanesulfonyl-phényl)-4,6-diaza-spiro[2.4]heptane-5,7-dione
- la 4-(3-pipéridino-pyridin-4-ylméthyl-6-(4-trifluorométhanesulfonyl-phényl)-4,6-diaza-spiro[2.4]heptane-5,7-dione
- la 4-(3-morpholino-pyridin-4-ylméthyl-6-(4-trifluorométhanesulfonyl-phényl)-4,6-diaza-spiro[2.4]heptane-5,7-dione
- la 4-[3-(4-méthylpipérazino)diéthylamino-pyridin-4-ylméthyl-6-(4-trifluorométhanesulfonyl-phényl)-4,6-diaza-spiro[2.4]heptane-5,7-dione
- la 4-[3-(1H-pyrazol-1yl)-pyridin]-4-ylméthyl-6-(4-trifluorométhanesulfonyl-phényl)-4,6-diaza-spiro[2.4]heptane-5,7-dione
- la 4-[3-(2-benzoylhydrazino)-pyridin-4-ylméthyl-6-(4-trifluorométhanesulfonyl-phényl)-4,6-diaza-spiro[2.4]heptane-5,7-dione
- la 4-(3-benzoylamino-pyridin-4-ylméthyl-6-(4-trifluorométhanesulfonyl-phényl)-4,6-diaza-spiro[2.4]heptane-5,7-dione
- la 4-[3-(4-chlorobenzoylamino)-pyridin-4-ylméthyl-6-(4-trifluorométhanesulfonyl-phényl)-4,6-diaza-spiro[2.4]heptane-5,7-dione
- la 4-[3-(3-chlorobenzoylamino)-pyridin-4-ylméthyl-6-(4-trifluorométhanesulfonyl-phényl)-4,6-diaza-spiro[2.4]heptane-5,7-dione
- la 4-[3-(2-chlorobenzoylamino)-pyridin-4-ylméthyl-6-(4-trifluorométhanesulfonyl-phényl)-4,6-diaza-spiro[2.4]heptane-5,7-dione
- la 4-[3-(4-fluorobenzoylamino)-pyridin-4-ylméthyl-6-(4-trifluorométhanesulfonyl-phényl)-4,6-diaza-spiro[2.4]heptane-5,7-dione
- la 4-[3-(4-méthoxybenzoylamino)-pyridin-4-ylméthyl-6-(4-trifluorométhanesulfonyl-phényl)-4,6-diaza-spiro[2.4]heptane-5,7-dione
- la 4-(3-acétamido-pyridin-4-ylméthyl-6-(4-trifluorométhanesulfonyl-phényl)-4,6-diaza-spiro[2.4]heptane-5,7-dione
- la 4-(3-phénylacétamido-pyridin-4-ylméthyl-6-(4-trifluorométhanesulfonyl-phényl)-4,6-diaza-spiro[2.4]heptane-5,7-dione
- la 4-[3-(4-méthylpipérazino)acétamido-pyridin-4-ylméthyl-6-(4-trifluorométhanesulfonyl-phényl)-4,6-diaza-spiro[2.4]heptane-5,7-dione
- la 4-(3-cyclohéxanylcarboxamido-pyridin-4-ylméthyl-6-(4-trifluorométhanesulfonyl-phényl)-4,6-diaza-spiro[2.4]heptane-5,7-dione
- la 4-[3-(1-méthyl-4-pipéridinyl)carbonylamino-pyridin-4-ylméthyl-6-(4-trifluorométhanesulfonyl-phényl)-4,6-diaza-spiro[2.4]heptane-5,7-dione
- la 4-[3-(2-furanylcarbonyl)amino-pyridin-4-ylméthyl-6-(4-trifluorométhanesulfonyl-phényl)-4,6-diaza-spiro[2.4]heptane-5,7-dione
- la 4-(3-éthoxycarbonylamino-pyridin-4-ylméthyl-6-(4-trifluorométhanesulfonyl-phényl)-4,6-diaza-spiro[2.4]heptane-5,7-dione
- la 4-(3-isopropoxycarbonylamino-pyridin-4-ylméthyl-6-(4-trifluorométhanesulfonyl-phényl)-4,6-diaza-spiro[2.4]heptane-5,7-dione
- la 4-(3-butoxycarbonylamino-pyridin-4-ylméthyl-6-(4-trifluorométhanesulfonyl-phényl)-4,6-diaza-spiro[2.4]heptane-5,7-dione
- la 4-[3-(2-diéthylaminoéthoxycarbonylamino)-pyridin-4-ylméthyl-6-(4-trifluorométhanesulfonyl-phényl)-4,6-diaza-spiro[2.4]heptane-5,7-dione
- la 4-(3-phénoxycarbonylamino-pyridin-4-ylméthyl-6-(4-trifluorométhanesulfonyl-phényl)-4,6-diaza-spiro[2.4]heptane-5,7-dione
- la 4-[3-(4-chloroanilino)carbonylamino-pyridin-4-ylméthyl)-6-(4-trifluorométhanesulfonyl-phényl)-4,6-diaza-spiro[2.4]heptane-5,7-dione
- la 4-(3-anilinocarbonylamino-pyridin-4-ylméthyl-6-(4-trifluorométhanesulfonyl-phényl)-4,6-diaza-spiro[2.4]heptane-5,7-dione
- la 4-(3-benzylaminocarbonylamino-pyridin-4-ylméthyl-6-(4-trifluorométhanesulfonyl-phényl)-4,6-diaza-spiro[2.4]heptane-5,7-dione
- la 4-[3-(2-pyridylamino)carbonylamino-pyridin-4-ylméthyl)-6-(4-trifluorométhanesulfonyl-phényl)-4,6-diaza-spiro[2.4]heptane-5,7-dione
- la 4-(3-uréido-pyridin-4-ylméthyl-6-(4-trifluorométhanesulfonyl-phényl)-4,6-diaza-spiro[2.4]heptane-5,7-dione
- la 4-(3-benzylaminosulfonylamino-pyridin-4-ylméthyl-6-(4-trifluorométhanesulfonyl-phényl)-4,6-diaza-spiro[2.4]heptane-5,7-dione
- la 4-(3-méthylaminosulfonylamino-pyridin-4-ylméthyl-6-(4-trifluorométhanesulfonyl-phényl)-4,6-diaza-spiro[2.4]heptane-5,7-dione
- la 4-(3-aminosulfonylamino-pyridin-4-ylméthyl-6-(4-trifluorométhanesulfonyl-phényl)-4,6-diaza-spiro[2.4]heptane-5,7-dione
- la 4-(3-morpholinosulfonylamino-pyridin-4-ylméthyl-6-(4-trifluorométhanesulfonyl-phényl)-4,6-diaza-spiro[2.4]heptane-5,7-dione
- la 4-(3-diméthylaminosulfonylamino-pyridin-4-ylméthyl-6-(4-trifluorométhanesulfonyl-phényl)-4,6-diaza-spiro[2.4]heptane-5,7-dione
- la 4-(3-anilinosulfonylamino-pyridin-4-ylméthyl-6-(4-trifluorométhanesulfonyl-phényl)-4,6-diaza-spiro[2.4]heptane-5,7-dione
- la 4-(3-phénylsulfonylamino-pyridin-4-ylméthyl-6-(4-trifluorométhanesulfonyl-phényl)-4,6-diaza-spiro[2.4]heptane-5,7-dione
- la 4-[3-(2-thiényl)sulfonylamino-pyridin-4-ylméthyl-6-(4-trifluorométhanesulfonyl-phényl)-4,6-diaza-spiro[2.4]heptane-5,7-dione
- la 4-(3-méthylsulfonylamino-pyridin-4-ylméthyl-6-(4-trifluorométhanesulfonyl-phényl)-4,6-diaza-spiro[2.4]heptane-5,7-dione
- la 4-[3-(4-chlorophényl)sulfonylamino-pyridin-4-ylméthyl-6-(4-trifluorométhanesulfonyl-phényl)-4,6-diaza-spiro[2.4]heptane-5,7-dione
- la 4-(3-benzylsulfonylamino-pyridin-4-ylméthyl-6-(4-trifluorométhanesulfonyl-phényl)-4,6-diaza-spiro[2,4]heptane-5,7-dione
- la 4-(3-hydroxycarbonyl-pyridin)-4-ylméthyl-6-(4-trifluorométhanesulfonyl-phényl)-4,6-diaza-spiro[2.4]heptane-5,7-dione
- la 4-(3-éthoxycarbonyl-pyridin)-4-ylméthyl-6-(4-trifluorométhanesulfonyl-phényl)-4,6-diaza-spiro[2.4]heptane-5,7-dione
- la 4-(3-ter-butoxycarbonyl-pyridin)-4-ylméthyl-6-(4-trifluorométhanesulfonyl-phényl)-4,6-diaza-spiro[2.4]heptane-5,7-dione
- la 4-(3-phénoxycarbonyl-pyridin)-4-ylméthyl-6-(4-trifluorométhanesulfonyl-phényl)-4,6-diaza-spiro[2.4]heptane-5,7-dione
- la 4-[3-(4-méthylpipérazinocarbonyl)-pyridin]-4-ylméthyl-6-(4-trifluorométhanesulfonyl-phényl)-4,6-diaza-spiro[2.4]heptane-5,7-dione
- la 4-[3-(méthylamino)carbonyl-pyridin)-4-ylméthyl-6-(4-trifluorométhanesulfonyl-phényl)-4,6-diaza-spiro[2.4]heptane-5,7-dione
- la 4-[3-(diméthylamino)carbonyl-pyridin)-4-ylméthyl-6-(4-trifluorométhanesulfonyl-phényl)-4,6-diaza-spiro[2.4]heptane-5,7-dione
- la 4-(3-pipéridinocarbonyl-pyridin)-4-ylméthyl-6-(4-trifluorométhanesulfonyl-phényl)-4,6-diaza-spiro[2.4]heptane-5,7-dione
- la 4-(3-morpholinocarbonyl-pyridin)-4-ylméthyl-6-(4-trifluorométhanesulfonyl-phényl)-4,6-diaza-spiro[2.4]heptane-5,7-dione
- la 4-[3-(4-phénylpipérazinocarbonyl)-pyridin]-4-ylméthyl-6-(4-trifluorométhanesulfonyl-phényl)-4,6-diaza-spiro[2.4]heptane-5,7-dione
- la 4-(3-phénylaminocarbonyl-pyridin)-4-ylméthyl-6-(4-trifluorométhanesulfonyl-phényl)-4,6-diaza-spiro[2.4]heptane-5,7-dione
- la 4-[3-(méthylphénylamino)carbonyl-pyridin)-4-ylméthyl-6-(4-trifluorométhanesulfonyl-phényl)-4,6-diaza-spiro[2.4]heptane-5,7-dione
- la 4-[3-(4-méthoryphényl)méthylaminocarbonyl-pyridin)-4-ylméthyl-6-(4-trifluorométhanesulfonyl-phényl)-4,6-diaza-spiro[2.4]heptane-5,7-dione
- la 4-[3-(4-chlorophényl)méthylaminocarbonyl-pyridin)-4-ylméthyl-6-(4-trifluorométhanesulfonyl-phényl)-4,6-diaza-spiro[2.4]heptane-5,7-dione
- la 4-[3-(4-morpholinylamino)carbonyl-pyridin)-4-ylméthyl-6-(4-trifluorométhanesulfonyl-phényl)-4,6-diaza-spiro[2.4]heptane-5,7-dione
- la 4-(3-aminocarbonyl-pyridin)-4-ylméthyl-6-(4-trifluorométhanesulfonyl-phényl)-4,6-diaza-spiro[2.4]heptane-5,7-dione
- la 4-(3-phénoxy-pyridin-4-ylméthyl-6-(4-trifluorométhanesulfonyl-phényl)-4,6-diaza-spiro[2.4]heptane-5,7-dione
- la 4-(3-méthoxy-pyridin-4-ylméthyl-6-(4-trifluorométhanesulfonyl-phényl)-4,6-diaza-spiro[2.4]heptane-5,7-dione
- la 4-(3-éthoxy-pyridin-4-ylméthyl-6-(4-trifluorométhanesulfonyl-phényl)-4,6-diaza-spiro[2.4]heptahe-5,7-dione
- la 4-[3-(1-pyrrolidinyl)éthoxy-pyridin-4-ylméthyl-6-(4-trifluorométhanesulfonyl-phényl)-4,6-diaza-spiro[2.4]heptane-5,7-dione
- la 4-(3-benzyloxy-pyridin-4-ylméthyl-6-(4-trifluorométhanesulfonyl-phényl)-4,6-diaza-spiro[2.4]heptane-5,7-dione
- la 4-(3-benzoyloxy-pyridin-4-ylméthyl-6-(4-trifluorométhanesulfonyl-phényl)-4,6-diaza-spiro[2.4]heptane-5,7-dione
- la 4-[3-(4,5-dihydro-1H-imidazol-2-yl)-pyridin]-4-ylméthyl-6-(4-trifluorométhanesulfonyl-phényl)-4,6-diaza-spiro[2.4]heptane-5,7-dione
- la 4-[3-(1H-imidazol-2-yl)-pyridin]-4-ylméthyl-6-(4-trifluorométhanesulfonyl-phényl)-4,6-diaza-spiro[2.4]heptane-5,7-dione
- la 4-[3-(1H-tétrazol-5-yl)-pyridinl-4-ylméthyl-6-(4-trifluorométhanesulfonyl-phényl)-4,6-diaza-spiro[2.4]heptane-5,7-dione
- la 4-(3-amidino-pyridin-4-ylméthyl-6-(4-trifluorométhanesulfonyl-phényl)-4,6-diaza-spiro[2.4]heptane-5,7-dione
- la 4-(3-diméthylaminométhyl-pyridin)-4-ylméthyl-6-(4-trifluorométhanesulfonyl-phényl)-4,6-diaza-spiro[2.4]heptane-5,7-dione
- la 4-(3-aminométhyl-pyridin)-4-ylméthyl-6-(4-trifluorométhanesulfonyl-phényl)-4,6-diaza-spiro[2.4]heptane-5,7-dione
- la 5-méthyl-1-(2-méthyl-pyridin-4-ylméthyl)-3-(4-trifluorométhanesulfonyl-phényl)-imidazolidine-2,4-dione
- la 5-méthyl-1-(2-éthyl-pyridin-4-ylméthyl)-3-(4-trifluorométhanesulfonyl-phényl)-imidazolidine-2,4-dione
- la 5-méthyl-1-(2-isopropyl-pyridin-4-ylméthyl)-3-(4-trifluorométhanesulfonyl-phényl)-imidazolidine-2,4-dione
- la 5-méthyl-1-(2-ter-butyl-pyridin-4-ylméthyl)-3-(4'-trifluorométhanesulfonyl-phényl)-imidazolidine-2,4-dione
- la 5-méthyl-1-(2-isobutyl-pyridin-4-ylméthyl)-3-(4-trifluorométhanesulfonyl-phényl)-imidazolidine-2,4-dione
- la 5-méthyl-1-(2-cyclohéxyl-pyridin-4-ylméthyl)-3-(4-trifluorométhanesulfonyl-phényl)-imidazolidine-2,4-dione
- la 5-méthyl-1-(2-phényl-pyridin-4-ylméthyl)-3-(4-trifluorométhanesulfonyl-phényl)-imidazolidine-2,4-dione
- la 5-méthyl-1-[2-(4-chlorophényl)-pyridin-4-ylméthyl]-3-(4-trifluorométhanesulfonyl-phényl)-imidazolidine-2,4-dione
- la 5-méthyl-1-[2-(3-chlorophényl)-pyridin-4-ylméthyl]-3-(4-trifluorométhanesulfonyl-phényl)-imidazolidine-2,4-dione
- la 5-méthyl-1-[2-(2-chlorophényl)-pyridin-4-ylméthyl]-3-(4-trifluorométhanesulfonyl-phényl)-imidazolidine-2,4-dione
- la 5-méthyl-1-[2-(4-fluorophényl)-pyridin-4-ylméthyl]-3-(4-trifluorométhanesulfonyl-phényl)-imidazolidine-2,4-dione
- la 5-méthyl-1-[2-(4-méthoxyphényl)-pyridin-4-ylméthyl] - 3-(4-trifluorométhanesulfonyl-phényl)-imidazolidine-2,4-dione
- la 5-méthyl-1-(2-benzyl-pyridin-4-ylméthyl)-3-(4-trifluorométhanesulfonyl-phényl)-imidazolidine-2,4-dione
- la 5-méthyl-1-(2,2'bipyridin-4-ylméthyl)-3-(4-trifluorométhanesulfonyl-phényl)-imidazolidine-2,4-dione
- la 5-méthyl-1-(2-hydroxyméthyl-pyridin-4-ylméthyl)-3-(4-trifluorométhanesulfonyl-phényl)-imidazolidine-2,4-dione
- la 5-méthyl-1-(2-chloro-pyridin-4-ylméthyl)-3-(4-trifluorométhanesulfonyl-phényl)-imidazolidine-2,4-dione
- la 5-méthyl-1-(2-fluoro-pyridin-4-ylméthyl)-3-(4-trifluorométhanesulfonyl-phényl)-imidazolidine-2,4-dione
- la 5-méthyl-1-(2-bromo-pyridin-4-ylméthyl)-3-(4-trifluorométhanesulfonyl-phényl)-imidazolidine-2,4-dione
- la 5-méthyl-1-(2-amino-pyridin-4-ylméthyl)-3-(4-trifluorométhanesulfonyl-phényl)-imidazolidine-2,4-dione
- la 5-méthyl-1-(2-méthylamino-pyridin-4-ylméthyl)-3-(4-trifluorométhanesulfonyl-phényl)-imidazolidine-2,4-dione
- la 5-méthyl-1-(2-éthylamino-pyridin-4-ylméthyl)-3-(4-trifluorométhanesulfonyl-phényl)-imidazolidine-2,4-dione
- la 5-méthyl-1-(2-isopropylamino-pyridin-4-ylméthyl)-3-(4-trifluorométhanesulfonyl-phényl)-imidazolidine-2,4-dione
- la 5-méthyl-1-(2-phénylamino-pyridin-4-ylméthyl)-3-(4-trifluorométhanesulfonyl-phényl)-imidazolidine-2,4-dione
- la 5-méthyl-1-(2-cyclohéxylamino-pyridin-4-ylméthyl)-3-(4-trifluorométhanesulfonyl-phényl)-imidazolidine-2,4-dione
- la 5-méthyl-1-(2-diéthylamino-pyridin-4-ylméthyl)-3-(4-trifluorométhanesulfonyl-phényl)-imidazolidine-2,4-dione
- la 5-méthyl-1-(2-N-cyclohéxyl-N-méthylamino-pyridin-4-ylméthyl)-3-(4-trifluorométhanesulfonyl-phényl)-imidazolidine-2,4-dione
- la 5-méthyl-1-(2-pipéridino-pyridin-4-ylméthyl)-3-(4-trifluorométhanesulfonyl-phényl)-imidazolidine-2,4-dione
- la 5-méthyl-1-(2-morpholino-pyridin-4-ylméthyl)-3-(4-trifluorométhanesulfonyl-phényl)-imidazolidine-2,4-dione
- la 5-méthyl-1-[2-(4-méthylpipérazino)diéthylamino-pyridin-4-ylméthyl]-3-(4-trifluorométhanesulfonyl-phényl)-imidazolidine-2,4-dione
- la 5-méthyl-1-[2-(1H-pyrazol-1yl)-pyridin-4-ylméthyl]-3-(4-trifluorométhanesulfonyl-phényl)-imidazolidine-2,4-dione
- la 5-méthyl-1-[2-(2-benzoylhydrazino)-pyridin-4-ylméthyl]-3-(4-trifluorométhanesulfonyl-phényl)-imidazolidine-2,4-dione
- la 5-méthyl-1-(2-benzoylamino-pyridin-4-ylméthyl)-3-(4-trifluorométhanesulfonyl-phényl)-imidazolidine-2,4-dione
- la 5-méthyl-1-[2-(4-chlorobenzoylamino)-pyridin-4-ylméthyl]-3-(4-trifluorométhanesulfonyl-phényl)-imidazolidine-2,4-dione
- la 5-méthyl-1-[2-(3-chlorobenzoylamino)-pyridin-4-ylméthyl]-3-(4-trifluorométhanesulfonyl-phényl)-imidazolidine-2,4-dione
- la 5-méthyl-1-[2-(2-chlorobenzoylamino)-pyridin-4-ylméthyl]-3-(4-trifluorométhanesulfonyl-phényl)-imidazolidine-2,4-dione
- la 5-méthyl-1-[2-(4-fluorobenzoylamino)-pyridin-4-ylméthyl]-3-(4-trifluorométhanesulfonyl-phényl)-imidazolidine-2,4-dione
- la 5-méthyl-1-[2-(4-méthoxybenzoylamino)-pyridin-4-ylméthyl]-3-(4-trifluorométhanesulfonyl-phényl)-imidazolidine-2,4-dione
- la 5-méthyl-1-(2-acétamido-pyridin-4-ylméthyl)-3-(4-trifluoraméthanesulfonyl-phényl)-imidazolidine-2,4-dione
- la 5-méthyl-1-(2-phénylacétamido-pyridin-4-ylméthyl)-3-(4-trifluorométhanesulfonyl-phényl)-imidazolidine-2,4-dione
- la 5-méthyl-1-[2-(4-méthylpipérazino)acétamido-pyridin-4-ylméthyl]-3-(4-trifluorométhanesulfonyl-phényl)-imidazolidine-2,4-dione
- la 5-méthyl-1-(2-cyclohéxanylcarboxamido-pyridin-4-ylméthyl)-3-(4-trifluorométhanesulfonyl-phényl)-imidazolidine-2,4-dione
- la 5-méthyl-1-[2-(1-méthyl-4-pipéridinyl)carbonylamino-pyridin-4-ylméthyl]-3-(4-trifluorométhanesulfonyl-phényl)-imidazolidine-2,4-dione
- la 5-méthyl-1-[2-(2-furanylcarbonyl)amino-pyridin-4-ylméthyl]-3-(4-trifluorométhanesulfonyl-phényl)-imidazolidine-2,4-dione
- la 5-méthyl-1-(2-éthoxycarbonylamino-pyridin-4-lméthyl)-3-(4-trifluorométhanesulfonyl-phényl)-imidazolidine-2,4-dione
- la 5-méthyl-1-(2-isopropoxycarbonylamino-pyridin-4-ylméthyl)-3-(4-trifluorométhanesulfonyl-phényl)-imidazolidine-2,4-dione
- la 5-méthyl-1-(2-butoxycarbonylamino-pyridin-4-ylméthyl)-3-(4-trifluorométhanesulfonyl-phényl)-imidazolidine-2,4-dione
- la 5-méthyl-1-[2-(2-diéthylaminoéthoxycarbonylamino)-pyridin-4-ylméthyl]-3-(4-trifluorométhanesulfonyl-phényl)-imidazolidine-2,4-dione
- la 5-méthyl-1-(2-phénoxycarbonylamino-pyridin-4-ylméthyl)-3-(4-trifluorométhanesulfonyl-phényl)-imidazolidine-2,4-dione
- la 5-méthyl-1-[2-(4-chloroanilino)carbonylamino-pyridin-4-ylméthyl)]-3-(4-trifluorométhanesulfonyl-phényl)-imidazolidine-2,4-dione
- la 5-méthyl-1-(2-anilinocarbonylamino-pyridin-4-ylméthyl)-3-(4-trifluorométhanesulfonyl-phényl)-imidazolidine-2,4-dione
- la 5-méthyl-1-(2-benzylaminocarbonylamino-pyridin-4-ylméthyl)-3-(4-trifluorométhanesulfonyl-phényl)-imidazolidine-2,4-dione
- la 5-méthyl-1-[2-(2-pyridylamino)carbonylamino-pyridin-4-ylméthyl)]-3-(4-trifluorométhanesulfonyl-phényl)-imidazolidine-2,4-dione
- la 5-méthyl-1-(2-uréido-pyridin-4-ylméthyl)-3-(4-trifluorométhanesulfonyl-phényl)-imidazolidine-2,4-dione
- la 5-méthyl-1-(2-benzylaminosulfonylamino-pyridin-4-ylméthyl)-3-(4-trifluorométhanesulfonyl-phényl)-imidazolidine-2,4-dione
- la 5-méthyl-1-(2-méthylaminosulfonylamino-pyridin-4-ylméthyl)-3-(4-trifluorométhanesulfonyl-phényl)-imidazolidine-2,4-dione
- la 5-méthyl-1-(2-aminosulfonylamino-pyridin-4-ylméthyl)-3-(4-trifluorométhanesulfonyl-phényl)-imidazolidine-2,4-dione
- la 5-méthyl-1-(2-morpholinosulfonylamino-pyridin-4-ylméthyl)-3-(4-trifluorométhanesulfonyl-phényl)-imidazolidine-2,4-dione
- la 5-méthyl-1-(2-diméthylaminosulfonylamino-pyridin-4-ylméthyl)-3-(4-trifluorométhanesulfonyl-phényl)-imidazolidine-2,4-dione
- la 5-méthyl-1-(2-anilinosulfonylamino-pyridin-4-ylméthyl)-3-(4-trifluorométhanesulfonyl-phényl)-imidazolidine-2,4-dione
- la 5-méthyl-1-(2-phénylsulfonylamino-pyridin-4-ylméthyl)-3-(4-trifluorométhanesulfonyl-phényl)-imidazolidine-2,4-dione
- la 5-méthyl-1-[2-(2-thiényl)sulfonylamino-pyridin-4-ylméthyl]-3-(4-trifluorométhanesulfonyl-phényl)-imidazolidine-2,4-dione
- la 5-méthyl-1-(2-méthylsulfonylamino-pyridin-4-ylméthyl)-3-(4-trifluorométhanesulfonyl-phényl)-imidazolidine-2,4-dione
- la 5-méthyl-1-[2-(4-chlorophényl)sulfonylamino-pyridin-4-ylméthyl]-3-(4-trifluorométhanesulfonyl-phényl)-imidazolidine-2,4-dione
- la 5-méthyl-1-(2-benzylsulfonylamino-pyridin-4-ylméthyl)-3-(4-trifluorométhanesulfonyl-phényl)-imidazolidine-2,4-dione
- la 5-méthyl-1-(2-hydroxycarbonyl-pyridin-4-ylméthyl)-3-(4-trifluorométhanesulfonyl-phényl)-imidazolidine-2,4-dione
- la 5-méthyl-1-(2-éthoxycarbonyl-pyridin-4-ylméthyl)-3-(4-trifluorométhanesulfonyl-phényl)-imidazolidine-2,4-dione
- la 5-méthyl-1-(2-ter-butoxycarbonyl-pyridin-4-ylméthyl)-3-(4-trifluorométhanesulfonyl-phényl)-imidazolidine-2,4-dione
- la 5-méthyl-1-(2-phénoxycarbonyl-pyridin-4-ylméthyl)-3-(4-trifluorométhanesulfonyl-phényl)-imidazolidine-2,4-dione
- la 5-méthyl-1-[2-(4-méthylpipérazinocarbonyl)-pyridin-4-ylméthyl]-3-(4-trifluorométhanesulfonyl-phényl)-imidazolidine-2,4-dione
- la 5-méthyl-1-[2-(méthylamino)carbonyl-pyridin-4-ylméthyl]-3-(4-trifluorométhanesulfonyl-phényl)-imidazolidine-2,4-dione
- la 5-méthyl-1-[2-(diméthylamino)carbonyl-pyridin-4-ylméthyl]-3-(4-trifluorométhanesulfonyl-phényl)-imidazolidine-2,4-dione
- la 5-méthyl-1-(2-pipéridinocarbonyl-pyridin-4-ylméthyl)-3-(4-trifluorométhanesulfonyl-phényl)-imidazolidine-2,4-dione.
- la 5-méthyl-1-(2-morpholinocarbonyl-pyridin-4-ylméthyl)-3-(4-trifluorométhanesulfonyl-phényl)-imidazolidine-2,4-dione
- la 5-méthyl-1-[2-(4-phénylpipérazinocarbonyl)-pyridin-4-ylméthyl]-3-(4-trifluorométhanesulfonyl-phényl)-imidazolidine-2,4-dione
- la 5-méthyl-1-(2-phénylaminocarbonyl-pyridin-4-ylméthyl)-3-(4-trifluorométhanesulfonyl-phényl)-imidazolidine-2,4-dione
- la 5-méthyl-1-[2-(méthylphénylamino)carbonyl-pyridin-4-ylméthyl]-3-(4-trifluorométhanesulfonyl-phényl)-imidazolidine-2,4-dione
- la 5-méthyl-1-[2-(4-méthoxyphényl)méthylaminocarbonyl-pyridin-4-ylméthyl]-3-(4-trifluorométhanesulfonyl-phényl)-imidazolidine-2,4-dione
- la 5-méthyl-1-[2-(4-chlorophényl)méthylaminocarbonyl-pyridin-4-ylméthyl]-3-(4-trifluorométhanesulfonyl-phényl)-imidazolidine-2,4-dione
- la 5-méthyl-1-[2-(4-morpholinylamino)carbonyl-pyridin-4-ylméthyl]-3-(4-trifluorométhanesulfonyl-phényl)-imidazolidine-2,4-dione
- la 5-méthyl-1-(2-aminocarbonyl-pyridin-4-ylméthyl)-3-(4-trifluorométhanesulfonyl-phényl)-imidazolidine-2,4-dione
- la 5-méthyl-1-(2-phénoxy-pyridin-4-ylméthyl)-3-(4-trifluorométhanesulfonyl-phényl)-imidazolidine-2,4-dione
- la 5-méthyl-1-(2-méthoxy-pyridin-4-ylméthyl)-3-(4-trifluorométhanesulfonyl-phényl)-imidazolidine-2,4-dione
- la 5-méthyl-1-(2-éthoxy-pyridin-4-ylméthyl)-3-(4-trifluorométhanesulfonyl-phényl)-imidazolidine-2,4-dione
- la 5-méthyl-1-[2-(1-pyrrolidinyl)éthoxy-pyridin-4-ylméthyl]-3-(4-trifluorométhanesulfonyl-phényl)-imidazolidine-2,4-dione
- la 5-méthyl-1-(2-benzyloxy-pyridin-4-ylméthyl)-3-(4-trifluorométhanesulfonyl-phényl)-imidazolidine-2,4-dione
- la 5-méthyl-1-(2-benzoyloxy-pyridin-4-ylméthyl)-3-(4-trifluorométhanesulfonyl-phényl)-imidazolidine-2,4-dione
- la 5-méthyl-1-[2-(4,5-dihydro-1H-imidazol-2-yl)-pyridin-4-ylméthyl]-3-(4-trifluorométhanesulfonyl-phényl)-imidazolidine-2,4-dione
- la 5-méthyl-1-[2-(1H-imidazol-2-yl)-pyridin-4-ylméthyl]-3-(4-trifluorométhanesulfonyl-phényl)-imidazolidine-2,4-dione
- la 5-méthyl-1-[2-(1H-tétrazol-5-yl)-pyridin-4-ylméthyl]-3-(4-trifluorométhanesulfonyl-phényl)-imidazolidine-2,4-dione
- la 5-méthyl-1-(2-amidino-pyridin-4-ylméthyl)-3-(4-trifluorométhanesulfonyl-phényl)-imidazolidine-2,4-dione
- la 5-méthyl-1-(2-diméthylaminométhyl-pyridin-4-ylméthyl)-3-(4-trifluorométhanesulfonyl-phényl)-imidazolidine-2,4-dione
- la 5-méthyl-1-(2-aminométhyl-pyridin-4-ylméthyl)-3-(4-trifluorométhanesulfonyl-phényl)-imidazolidine-2,4-dione
- la 5-méthyl-1-(3-méthyl-pyridin-4-ylméthyl)-3-(4-trifluorométhanesulfonyl-phényl)-imidazolidine-2,4-dione
- la 5-méthyl-1-(3-éthyl-pyridin-4-ylméthyl)-3-(4-trifluorométhanesulfonyl-phényl)-imidazolidine-2,4-dione
- la 5-méthyl-1-(3-isopropyl-pyridin-4-ylméthyl)-3-(4-trifluorométhanesulfonyl-phényl)-imidazolidine-2,4-dione
- la 5-méthyl-1-(3-ter-butyl-pyridin-4-ylméthyl)-3-(4-trifluorométhanesulfonyl-phényl)-imidazolidine-2,4-dione
- la 5-méthyl-1-(3-isobutyl-pyridin-4-ylméthyl)-3-(4-trifluorométhanesulfonyl-phényl)-imidazolidine-2,4-dione
- la 5-méthyl-1-(3-cyclohéxyl-pyridin-4-ylméthyl)-3-(4-trifluorométhanesulfonyl-phényl)-imidazolidine-2,4-dione
- la 5-méthyl-1-(3-phényl-pyridin-4-ylméthyl)-3-(4-trifluorométhanesulfonyl-phényl)-imidazolidine-2,4-dione
- la 5-méthyl-1-[3-(4-chlorophényl)-pyridin-4-ylméthyl]-3-(4-trifluorométhanesulfonyl-phényl)-imidazolidine-2,4-dione
- la 5-méthyl-1-[3-(3-chlorophényl)-pyridin-4-ylméthyl]-3-(4-trifluorométhanesulfonyl-phényl)-imidazolidine-2,4-dione
- la 5-méthyl-1-[3-(2-chlorophényl)-pyridin-4-ylméthyl]-3-(4-trifluorométhanesulfonyl-phényl)-imidazolidine-2,4-dione
- la 5-méthyl-1-[3-(4-fluorophényl)-pyridin-4-ylméthyl]-3-(4-trifluorométhanesulfonyl-phényl)-imidazolidine-2,4-dione
- la 5-méthyl-1-[3-(4-méthoxyphényl)-pyridin-4-ylméthyl]-3-(4-trifluorométhanesulfonyl-phényl)-imidazolidine-2,4-dione
- la 5-méthyl-1-(3-benzyl-pyridin-4-ylméthyl)-3-(4-trifluorométhanesulfonyl-phényl)-imidazolidine-2,4-dione
- la 5-méthyl-1-(3,2'bipyridin-4-ylméthyl)-3-(4-trifluorométhanesulfonyl-phényl)-imidazolidine-2,4-dione
- la 5-méthyl-1-(3-hydroxyméthyl-pyridin-4-ylméthyl)-3-(4-trifluorométhanesulfonyl-phényl)-imidazolidine-2,4-dione
- la 5-méthyl-1-(3-chloro-pyridin-4-ylméthyl)-3-(4-trifluorométhanesulfonyl-phényl)-imidazolidine-2,4-dione
- la 5-méthyl-1-(3-fluoro-pyridin-4-ylméthyl)-3-(4-trifluorométhanesulfonyl-phényl)-imidazolidiné-2,4-dione
- la 5-méthyl-1-(3-bromo-pyridin-4-ylméthyl)-3-(4-trifluorométhanesulfonyl-phényl)-imidazolidine-2,4-dione
- la 5-méthyl-1-(3-amino-pyridin-4-ylméthyl)-3-(4-trifluorométhanesulfonyl-phényl)-imidazolidine-2,4-dione
- la 5-méthyl-1-(3-méthylamino-pyridin-4-ylméthyl)-3-(4-trifluorométhanesulfonyl-phényl)-imidazolidine-2,4-dione
- la 5-méthyl-1-(3-éthylamino-pyridin-4-ylméthyl)-3-(4-trifluorométhanesulfonyl-phényl)-imidazolidine-2,4-dione
- la 5-méthyl-1-(3-isopropylamino-pyridin-4-ylméthyl)-3-(4-trifluorométhanesulfonyl-phényl)-imidazolidine-2,4-dione
- la 5-méthyl-1-(3-phénylamino-pyridin-4-ylméthyl)-3-(4-trifluorométhanesulfonyl-phényl)-imidazolidine-2,4-dione
- la 5-méthyl-1-(3-cyclohéxylamino-pyridin-4-ylméthyl)-3-(4-trifluorométhanesulfonyl-phényl)-imidazolidine-2,4-dione
- la 5-méthyl-1-(3-diéthylamino-pyridin-4-ylméthyl)-3-(4-trifluorométhanesulfonyl-phényl)-imidazolidine-2,4-dione
- la 5-méthyl-1-(3-N-cyclohéxyl-N-méthylamino-pyridin-4-ylméthyl)-3-(4-trifluorométhanesulfonyl-phényl)-imidazolidine-2,4-dione
- la 5-méthyl-1-(3-pipéridino-pyridin-4-ylméthyl)-3-(4-trifluorométhanesulfonyl-phényl)-imidazolidine-2,4-dione
- la 5-méthyl-1-(3-morpholino-pyridin-4-ylméthyl)-3-(4-trifluorométhanesulfonyl-phényl)-imidazolidine-2,4-dione
- la 5-méthyl-1-[3-(4-méthylpipérazino)diéthylamino-pyridin-4-ylméthyl]-3-(4-trifluorométhanesulfonyl-phényl)-imidazolidine-2,4-dione
- la 5-méthyl-1-[3-(1H-pyrazol-1yl)-pyridin-4-ylméthyl]-3-(4-trifluorométhanesulfonyl-phényl)-imidazolidine-2,4-dione
- la 5-méthyl-1-[3-(2-benzoylhydrazino)-pyridin-4-ylméthyl]-3-(4-trifluorométhanesulfonyl-phényl)-imidazolidine-2,4-dione
- la 5-méthyl-1-(3-benzoylamino-pyridin-4-ylméthyl)-3-(4-trifluorométhanesulfonyl-phényl)-imidazolidine-2,4-dione
- la 5-méthyl-1-[3-(4-chlorobenzoylamino)-pyridin-4-ylméthyl]-3-(4-trifluorométhanesulfonyl-phényl)-imidazolidine-2,4-dione
- la 5-méthyl-1-[3-(3-chlorobenzoylamino)-pyridin-4-ylméthyl]-3-(4-trifluorométhanesulfonyl-phényl)-imidazolidine-2,4-dione
- la 5-méthyl-1-[3-(2-chlorobenzoylamino)-pyridin-4-ylméthyl]-3-(4-trifluorométhanesulfonyl-phényl)-imidazolidine-2,4-dione
- la 5-méthyl-1-[3-(4-fluorobenzoylamino)-pyridin-4-ylméthyl]-3-(4-trifluorométhanesulfonyl-phényl)-imidazolidine-2,4-dione
- la 5-méthyl-1-[3-(4-méthoxybenzoylamino)-pyridin-4-ylméthyl]-3-(4-trifluorométhanesulfonyl-phényl)-imidazolidine-2,4-dione
- la 5-méthyl-1-(3-acétamido-pyridin-4-ylméthyl)-3-(4-trifluorométhanesulfonyl-phényl)-imidazolidine-2,4-dione
- la 5-méthyl-1-(3-phénylacétamido-pyridin-4-ylméthyl)-3-(4-trifluorométhanesulfonyl-phényl)-imidazolidine-2,4-dione
- la 5-méthyl-1-[3-(4-méthylpipérazino)acétamido-pyridin-4-ylméthyl]-3-(4-trifluorométhanesulfonyl-phényl)-imidazolidine-2,4-dione
- la 5-méthyl-1-(3-cyclohéxanylcarboxamido-pyridin-4-ylméthyl)-3-(4-trifluorométhanesulfonyl-phényl)-imidazolidine-2,4-dione
- la 5-méthyl-1-[3-(1-méthyl-4-pipéridinyl)carbonylamino-pyridin-4-ylméthyl]-3-(4-trifluorométhanesulfonyl-phényl)-imidazolidine-2,4-dione
- la 5-méthyl-1-[3-(2-furanylcarbonyl)amino-pyridin-4-ylméthyl]-3-(4-trifluorométhanesulfonyl-phényl)-imidazolidine-2,4-dione
- la 5-méthyl-1-(3-éthoxycarbonylamino-pyridin-4-ylméthyl)-3-(4-trifluorométhanesulfonyl-phényl)-imidazolidine-2,4-dione
- la 5-méthyl-1-(3-isopropoxycarbonylamino-pyridin-4-ylméthyl)-3-(4-trifluorométhanesulfonyl-phényl)-imidazolidine-2,4-dione
- la 5-méthyl-1-(3-butoxycarbonylamino-pyridin-4-ylméthyl)-3-(4-trifluorométhanesulfonyl-phényl)-imidazolidine-2,4-dione
- la 5-méthyl-1-[3-(2-diéthylaminoéthoxycarbonylamino)-pyridin-4-ylméthyl]-3-(4-trifluorométhanesulfonyl-phényl)-imidazolidine-2,4-dione
- la 5-méthyl-1-(3-phénoxycarbonylamino-pyridin-4-ylméthyl)-3-(4-trifluorométhanesulfonyl-phényl)-imidazolidine-2,4-dione
- la 5-méthyl-1-[3-(4-chloroanilino)carbonylamino-pyridin-4-ylméthyl)]-3-(4-trifluorométhanesulfonyl-phényl)-imidazolidine-2,4-dione
- la 5-méthyl-1-(3-anilinocarbonylamino-pyridin-4-ylméthyl)-3-(4-trifluorométhanesulfonyl-phényl)-imidazolidine-2,4-dione
- la 5-méthyl-1-(3-benzylaminocarbonylamino-pyridin-4-ylméthyl)-3-(4-trifluorométhanesulfonyl-phényl)-imidazolidine-2,4-dione
- la 5-méthyl-1-[3-(2-pyridylamino)carbonylamino-pyridin-4-ylméthyl)]-3-(4-trifluorométhanesulfonyl-phényl)-imidazolidine-2,4-dione
- la 5-méthyl-1-(3-uréido-pyridin-4-ylméthyl)-3-(4-trifluorométhanesulfonyl-phényl)-imidazolidine-2,4-dione
- la 5-méthyl-1-(3-benzylaminosulfonylamino-pyridin-4-ylméthyl)-3-(4-trifluorométhanesulfonyl-phényl)-imidazolidine-2,4-dione
- la 5-méthyl-1-(3-méthylaminosulfonylamino-pyridin-4-ylméthyl)-3-(4-trifluorométhanesulfonyl-phényl)-imidazolidine-2,4-dione
- la 5-méthyl-1-(3-aminosulfonylamino-pyridin-4-ylméthyl)-3-(4-trifluorométhanesulfonyl-phényl)-imidazolidine-2,4-dione
- la 5-méthyl-1-(3-morpholinosulfonylamino-pyridin-4-ylméthyl)-3-(4-trifluorométhanesulfonyl-phényl)-imidazolidine-2,4-dione
- la 5-méthyl-1-(3-diméthylaminosulfonylamino-pyridin-4-ylméthyl)-3-(4-trifluorométhanesulfonyl-phényl)-imidazolidine-2,4-dione
- la 5-méthyl-1-(3-anilinosulfonylamino-pyridin-4-ylméthyl)-3-(4-trifluorométhanesulfonyl-phényl)-imidazolidine-2,4-dione
- la 5-méthyl-1-(3-phénylsulfonylamino-pyridin-4-ylméthyl)-3-(4-trifluorométhanesulfonyl-phényl)-imidazolidine-2,4-dione
- la 5-méthyl-1-[3-(2-thiényl)sulfonylamino-pyridin-4-ylméthyl]-3-(4-trifluorométhanesulfonyl-phényl)-imidazolidine-2,4-dione
- la 5-méthyl-1-(3-méthylsulfonylamino-pyridin-4-ylméthyl)-3-(4-trifluorométhanesulfonyl-phényl)-imidazolidine-2,4-dione
- la 5-méthyl-1-[3-(4-chlorophényl)sulfonylamino-pyridin-4-ylméthyl]-3-(4-trifluorométhanesulfonyl-phényl)-imidazolidine-2,4-dione
- la 5-méthyl-1-(3-benzylsulfonylamino-pyridin-4-ylméthyl)-3-(4-trifluarométhanesulfonyl-phényl)-imidazolidine-2,4-dione
- la 5-méthyl-1-(3-hydroxycarbonyl-pyridin-4-ylméthyl)-3-(4-trifluorométhanesulfonyl-phényl)-imidazolidine-2,4-dione
- la 5-méthyl-1-(3-éthoxycarbonyl-pyridin-4-ylméthyl)-3-(4-trifluorométhanesulfonyl-phényl)-imidazolidine-2,4-dione
- la 5-méthyl-1-(3-ter-butoxycarbonyl-pyridin-4-ylméthyl)-3-(4-trifluorométhanesulfonyl-phényl)-imidazolidine-2,4-dione
- la 5-méthyl-1-(3-phénoxycarbonyl-pyridin-4-ylméthyl)-3-(4-trifluorométhanesulfonyl-phényl)-imidazolidine-2,4-dione
- la 5-méthyl-1-[3-(4-méthylpipérazinocarbonyl)-pyridin-4-ylméthyl]-3-(4-trifluorométhanesulfonyl-phényl)-imidazolidine-2,4-dione

- la 5-méthyl-1-[3-(méthylamino)carbonyl-pyridin-4-ylméthyl]-3-(4-trifluorométhanesulfonyl-phényl)-imidazolidine-2,4-dione
- la 5-méthyl-1-[3-(diméthylamino)carbonyl-pyridin-4-ylméthyl]-3-(4-trifluorométhanesulfonyl-phényl)-imidazolidine-2,4-dione
- la 5-méthyl-1-(3-pipéridinocarbonyl-pyridin-4-ylméthyl)-3-(4-trifluorométhanesulfonyl-phényl)-imidazolidine-2,4-dione
- la 5-méthyl-1-(3-morpholinocarbonyl-pyridin-4-ylméthyl)-3-(4-trifluorométhanesulfonyl-phényl)-imidazolidine-2,4-dione
- la 5-méthyl-1-[3-(4-phénylpipérazinocarbonyl)-pyridin-4-ylméthyl]-3-(4-trifluorométhanesulfonyl-phényl)-imidazolidine-2,4-dione
- la 5-méthyl-1-(3-phénylaminocarbonyl-pyridin-4-ylméthyl)-3-(4-trifluorométhanesulfonyl-phényl)-imidazolidine-2,4-dione
- la 5-méthyl-1-[3-(méthylphénylamino)carbonyl-pyridin-4-ylméthyl]-3-(4-trifluorométhanesulfonyl-phényl)-imidazolidine-2,4-dione
- la 5-méthyl-1-[3-(4-méthoxyphényl)méthylaminocarbonyl-pyridin-4-ylméthyl]-3-(4-trifluorométhanesulfonyl-phényl)-imidazolidine-2,4-dione
- la 5-méthyl-1-[3-(4-chlorophényl)méthylaminocarbonyl-pyridin-4-ylméthyl]-3-(4-trifluorométhanesulfonyl-phényl)-imidazolidine-2,4-dione
- la 5-méthyl-1-[3-(4-morpholinylamino)carbonyl-pyridin-4-ylméthyl]-3-(4-trifluorométhanesulfonyl-phényl)-imidazolidine-2,4-dione
- la 5-méthyl-1-(3-aminocarbonyl-pyridin-4-ylméthyl)-3-(4-trifluorométhanesulfonyl-phényl)-imidazolidine-2,4-dione
- la 5-méthyl-1-(3-phénoxy-pyridin-4-ylméthyl)-3-(4-trifluorométhanesulfonyl-phényl)-imidazolidine-2,4-dione
- la 5-méthyl-1-(3-méthoxy-pyridin-4-ylméthyl)-3-(4-trifluorométhanesulfonyl-phényl)-imidazolidine-2,4-dione
- la 5-méthyl-1-(3-éthoxy-pyridin-4-ylméthyl)-3-(4-trifluorométhanesulfonyl-phényl)-imidazolidine-2,4-dione
- la 5-méthyl-1-[3-(1-pyrrolidinyl)éthoxy-pyridin-4-ylméthyl]-3-(4-trifluorométhanesulfonyl-phényl)-imidazolidine-2,4-dione
- la 5-méthyl-1-(3-benzyloxy-pyridin-4-ylméthyl)-3-(4-trifluorométhanesulfonyl-phényl)-imidazolidine-2,4-dione
- la 5-méthyl-1-(3-benzoyloxy-pyridin-4-ylméthyl)-3-(4-trifluorométhanesulfonyl-phényl)-imidazolidine-2,4-dione
- la 5-méthyl-1-[3-(4,5-dihydro-1H-imidazol-2-yl)-pyridin-4-ylméthyl]-3-(4-trifluorométhanesulfonyl-phényl)-imidazolidine-2,4-dione
- la 5-méthyl-1-[3-(1H-imidazol-2-yl)-pyridin-4-ylméthyl]-3-(4-trifluorométhanesulfonyl-phényl)-imidazolidine-2,4-dione
- la 5-méthyl-1-[3-(1H-tétrazol-5-yl)-pyridin-4-ylméthyl]-3-(4-trifluorométhanesulfonyl-phényl)-imidazolidine-2,4-dione
- la 5-méthyl-1-(3-amidino-pyridin-4-ylméthyl)-3-(4-trifluorométhanesulfonyl-phényl)-imidazolidine-2,4-dione
- la 5-méthyl-1-(3-diméthylaminométhyl-pyridin-4-ylméthyl)-3-(4-trifluorométhanesulfonyl-phényl)-imidazolidine-2,4-dione
- la 5-méthyl-1-(3-aminométhyl-pyridin-4-ylméthyl)-3-(4-trifluorométhanesulfonyl-phényl)-imidazolidine-2,4-dione
- la 1-(2-méthyl-pyridin-4-ylméthyl)-3-(4-trifluorométhanesulfonyl-phényl)-imidazolidine-2,4-dione
- la 1-(2-éthyl-pyridin-4-ylméthyl)-3-(4-trifluorométhanesulfonyl-phényl)-imidazolidine-2,4-dione
- la 1-(2-isopropyl-pyridin-4-ylméthyl)-3-(4-trifluorométhanesulfonyl-phényl)-imidazolidine-2,4-dione
- la 1-(2-ter-butyl-pyridin-4-ylméthyl)-3-(4-trifluorométhanesulfonyl-phényl)-imidazolidine-2,4-dione
- la 1-(2-isobutyl-pyridin-4-ylméthyl)-3-(4-trifluorométhanesulfonyl-phényl)-imidazolidine-2,4-dione
- la 1-(2-cyclohéxyl-pyridin-4-ylméthyl)-3-(4-trifluorométhanesulfonyl-phényl)-imidazolidine-2,4-dione
- la 1-(2-phényl-pyridin-4-ylméthyl)-3-(4-trifluorométhanesulfonyl-phényl)-imidazolidine-2,4-dione
- la 1-[2-(4-chlorophényl)-pyridin-4-ylméthyl]-3-(4-trifluorométhanesulfonyl-phényl)-imidazolidine-2,4-dione
- la 1-[2-(3-chlorophényl)-pyridin-4-ylméthyl]-3-(4-trifluorométhanesulfonyl-phényl)-imidazolidine-2,4-dione
- la 1-[2-(2-chlorophényl)-pyridin-4-ylméthyl]-3-(4-trifluorométhanesulfonyl-phényl)-imidazolidine-2,4-dione
- la 1-[2-(4-fluorophényl)-pyridin-4-ylméthyl]-3-(4-trifluorométhanesulfonyl-phényl)-imidazolidine-2,4-dione
- la 1-[2-(4-méthoxyphényl)-pyridin-4-ylméthyl]-3-(4-trifluorométhanesulfonyl-phényl)-imidazolidine-2,4-dione
- la 1-(2-benzyl-pyridin-4-ylméthyl)-3-(4-trifluorométhanesulfonyl-phényl)-imidazolidine-2,4-dione
- la 1-(2,2'bipyridin-4-ylméthyl)-3-(4-trifluorométhanesulfonyl-phényl)-imidazolidine-2,4-dione
- la 1-(2-hydroxyméthyl-pyridin-4-ylméthyl)-3-(4-trifluorométhanesulfonyl-phényl)-imidazolidine-2,4-dione
- la 1-(2-chloro-pyridin-4-ylméthyl)-3-(4-trifluorométhanesulfonyl-phényl)-imidazolidine-2,4-dione
- la 1-(2-fluoro-pyridin-4-ylméthyl)-3-(4-trifluorométhanesulfonyl-phényl)-imidazolidine-2,4-dione
- la 1-(2-bromo-pyridin-4-ylméthyl)-3-(4-trifluorométhanesulfonyl-phényl)-imidazolidine-2,4-dione
- la 1-(2-amino-pyridin-4-ylméthyl)-3-(4-trifluorométhanesulfonyl-phényl)-imidazolidine-2,4-dione
- la 1-(2-méthylamino-pyridin-4-ylméthyl)-3-(4-trifluorométhanesulfonyl-phényl)-imidazolidine-2,4-dione
- la 1-(2-éthylamino-pyridin-4-ylméthyl)-3-(4-trifluorométhanesulfonyl-phényl)-imidazolidine-2,4-dione
- la 1-(2-isopropylamino-pyridin-4-ylméthyl)-3-(4-trifluorométhanesulfonyl-phényl)-imidazolidine-2,4-dione
- la 1-(2-phénylamino-pyridin-4-ylméthyl)-3-(4-trifluorométhanesulfonyl-phényl)-imidazolidine-2,4-dione
- la 1-(2-cyclohéxylamino-pyridin-4-ylméthyl)-3-(4-trifluorométhanesulfonyl-phényl)-imidazolidine-2,4-dione
- la 1-(2-diéthylamino-pyridin-4-ylméthyl)-3-(4-trifluorométhanesulfonyl-phényl)-imidazolidine-2,4-dione
- la 1-(2-N-cyclohéxyl-N-méthylamino-pyridin-4-ylméthyl)-3-(4-trifluorométhanesulfonyl-phényl)-imidazolidine-2,4-dione
- la 1-(2-pipéridino-pyridin-4-ylméthyl)-3-(4-trifluorométhanesulfonyl-phényl)-imidazolidine-2,4-dione
- la 1-(2-morpholino-pyridin-4-ylméthyl)-3-(4-trifluorométhanesulfonyl-phényl)-imidazolidine-2,4-dione
- la 1-[2-(4-méthylpipérazino)diéthylamino-pyridin-4-ylméthyl]-3-(4-trifluorométhanesulfonyl-phényl)-imidazolidine-2,4-dione
- la 1-[2-(1H-pyrazol-1yl)-pyridin-4-ylméthyl]-3-(4-trifluorométhanesulfonyl-phényl)-imidazolidine-2,4-dione
- la 1-[2-(2-benzoylhydrazino)-pyridin-4-ylméthyl]-3-(4-trifluorométhanesulfonyl-phényl)-imidazolidine-2,4-dione
- la 1-(2-benzoylamino-pyridin-4-ylméthyl)-3-(4-trifluorométhanesulfonyl-phényl)-imidazolidine-2,4-dione
- la 1-[2-(4-chlorobenzoylamino)-pyridin-4-ylméthyl]-3-(4-trifluorométhanesulfonyl-phényl)-imidazolidine-2,4-dione
- la 1-[2-(3-chlorobenzoylamino)-pyridin-4-ylméthyl]-3-(4-trifluorométhanesulfonyl-phényl)-imidazolidine-2,4-dione
- la 1-[2-(2-chlorobenzoylamino)-pyridin-4-ylméthyl]-3-(4-trifluorométhanesulfonyl-phényl)-imidazolidine-2,4-dione
- la 1-[2-(4-fluorobenzoylamino)-pyridin-4-ylméthyl]-3-(4-trifluorométhanesulfonyl-phényl)-imidazolidine-2,4-dione
- la 1-[2-(4-méthoxybenzoylamino)-pyridin-4-ylméthyl]-3-(4-trifluorométhanesulfonyl-phényl)-imidazolidine-2,4-dione
- la 1-(2-acétamido-pyridin-4-ylméthyl)-3-(4-trifluorométhanesulfonyl-phényl)-imidazolidine-2,4-dione
- la 1-(2-phénylacétamido-pyridin-4-ylméthyl)-3-(4-trifluorométhanesulfonyl-phényl)-imidazolidine-2,4-dione
- la 1-[2-(4-méthylpipérazino)acétamido-pyridin-4-ylméthyl]-3-(4-trifluorométhanesulfonyl-phényl)-imidazolidine-2,4-dione
- la 1-(2-cyclohéxanylcarboxamido-pyridin-4-ylméthyl)-3-(4-trifluorométhanesulfonyl-phényl)-imidazolidine-2,4-dione
- la 1-[2-(1-méthyl-4-pipéridinyl)carbonylamino-pyridin-4-ylméthyl]-3-(4-trifluorométhanesulfonyl-phényl)-imidazolidine-2,4-dione
- la 1-[2-(2-furanylcarbonyl)amino-pyridin-4-ylméthyl]-3-(4-trifluorométhanesulfonyl-phényl)-imidazolidine-2,4-dione
- la 1-(2-éthoxycarbonylamino-pyridin-4-ylméthyl)-3-(4-trifluorométhanesulfonyl-phényl)-imidazolidine-2,4-dione
- la 1-(2-isopropoxycarbonylamino-pyridin-4-ylméthyl)-3-(4-trifluorométhanesulfonyl-phényl)-imidazolidine-2,4-dione
- la 1-(2-butoxycarbonylamino-pyridin-4-ylméthyl)-3-(4-trifluorométhanesulfonyl-phényl)-imidazolidine-2,4-dione
- la 1-[2-(2-diéthylaminoéthoxycarbonylamino)-pyridin-4-ylméthyl]-3-(4-trifluorométhanesulfonyl-phényl)-imidazolidine-2,4-dione
- la 1-(2-phénoxyca-rbonylamino-pyridin-4-ylméthyl)-3-(4-trifluorométhanesulfonyl-phényl)-imidazolidine-2,4-dione
- la 1-[2-(4-chloroanilino)carbonylamino-pyridin-4-ylméthyl)]-3-(4-trifluorométhanesulfonyl-phényl)-imidazolidine-2,4-dione
- la 1-(2-anilinocarbonylamino-pyridin-4-ylméthyl)-3-(4-trifluorométhanesulfonyl-phényl)-imidazolidine-2,4-dione
- la 1-(2-benzylaminocarbonylamino-pyridin-4-ylméthyl)-3-(4-trifluorométhanesulfonyl-phényl)-imidazolidine-2,4-dione
- la 1-[2-(2-pyridylamino)carbonylamino-pyridin-4-ylméthyl)]-3-(4-trifluorométhanesulfonyl-phényl)-imidazolidine-2,4-dione
- la 1-(2-uréido-pyridin-4-ylméthyl)-3-(4-trifluorométhanesulfonyl-phényl)-imidazolidine-2,4-dione
- la 1-(2-benzylaminosulfonylamino-pyridin-4-ylméthyl)-3-(4-trifluorométhanesulfonyl-phényl)-imidazolidine-2,4-dione
- la 1-(2-méthylaminosulfonylamino-pyridin-4-ylméthyl)-3-(4-trifluorométhanesulfonyl-phényl)-imidazolidine-2,4-dione
- la 1-(2-aminosulfonylamino-pyridin-4-ylméthyl)-3-(4-trifluorométhanesulfonyl-phényl)-imidazolidine-2,4-dione
- la 1-(2-morpholinosulfonylamino-pyridin-4-ylméthyl)-3-(4-trifluorométhanesulfonyl-phényl)-imidazolidine-2,4-dione
- la 1-(2-diméthylaminosulfonylamino-pyridin-4-ylméthyl)-3-(4-trifluorométhanesulfonyl-phényl)-imidazolidine-2,4-dione
- la 1-(2-anilinosulfonylamino-pyridin-4-ylméthyl)-3-(4-trifluorométhanesulfonyl-phényl)-imidazolidine-2,4-dione
- la 1-(2-phénylsulfonylamino-pyridin-4-ylméthyl)-3-(4-trifluorométhanesulfonyl-phényl)-imidazolidine-2,4-dione
- la 1-[2-(2-thiényl)sulfonylamino-pyridin-4-ylméthyl]-3-(4-trifluorométhanesulfonyl-phényl)-imidazolidine-2,4-dione
- la 1-(2-méthylsulfonylamino-pyridin-4-ylméthyl)-3-(4-trifluorométhanesulfonyl-phényl)-imidazolidine-2,4-dione
- la 1-[2-(4-chlorophényl)sulfonylamino-pyridin-4-ylméthyl]-3-(4-trifluorométhanesulfonyl-phényl)-imidazolidine-2,4-dione
- la 1-(2-benzylsulfonylamino-pyridin-4-ylméthyl)-3-(4-trifluorométhanesulfony-1-phényl)-imidazolidine-2,4-dione
- la 1-(2-hydroxycarbonyl-pyridin-4-ylméthyl)-3-(4-trifluorométhanesulfonyl-phényl)-imidazolidine-2,4-dione
- la 1-(2-éthoxycarbonyl-pyridin-4-ylméthyl)-3-(4-trifluorométhanesulfonyl-phényl)-imidazolidine-2,4-dione
- la 1-(2-ter-butoxycarbonyl-pyridin-4-ylméthyl)-3-(4-trifluorométhanesulfonyl-phényl)-imidazolidine-2,4-dione
- la 1-(2-phénoxycarbonyl-pyridin-4-ylméthyl)-3-(4-trifluorométhanesulfonyl-phényl)-imidazolidine-2,4-dione
- la 1-[2-(4-méthylpipérazinocarbonyl)-pyridin-4-ylméthyl]-3-(4-trifluorométhanesulfonyl-phényl)-imidazolidine-2,4-dione
- la 1-[2-(méthylamino)carbonyl-pyridin-4-ylméthyl]-3-(4-trifluorométhanesulfonyl-phényl)-imidazolidine-2,4-dione
- la 1-[2-(diméthylamino)carbonyl-pyridin-4-ylméthyl]-3-(4-trifluorométhanesulfonyl-phényl)-imidazolidine-2,4-dione
- la 1-(2-pipéridinocarbonyl-pyridin-4-ylméthyl)-3-(4-trifluorométhanesulfonyl-phényl)-imidazolidine-2,4-dione
- la 1-(2-morpholinocarbonyl-pyridin-4-ylméthyl)-3-(4-trifluorométhanesulfonyl-phényl)-imidazolidine-2,4-dione
- la 1-[2-(4-phénylpipérazinocarbonyl)-pyridin-4-ylméthyl]-3-(4-trifluorométhanesulfonyl-phényl)-imidazolidine-2,4-dione
- la 1-(2-phénylaminocarbonyl-pyridin-4-ylméthyl)-3-(4-trifluorométhanesulfonyl-phényl)-imidazolidine-2,4-dione
- la 1-[2-(méthylphénylamino)carbonyl-pyridin-4-ylméthyl]-3-(4-trifluorométhanesulfonyl-phényl)-imidazolidine-2,4-dione
- la 1-[2-(4-méthoxyphényl)méthylaminocarbonyl-pyridin-4-ylméthyl]-3-(4-trifluorométhanesulfonyl-phényl)-imidazolidine-2,4-dione
- la 1-[2-(4-chlorophényl)méthylaminocarbonyl-pyridin-4-ylméthyl]-3-(4-trifluorométhanesulfonyl-phényl)-imidazolidine-2,4-dione
- la 1-[2-(4-morpholinylamino)carbonyl-pyridin-4-ylméthyl]-3-(4-trifluorométhanesulfonyl-phényl)-imidazolidine-2,4-dione
- la 1-(2-aminocarbonyl-pyridin-4-ylméthyl)-3-(4-trifluorométhanesulfonyl-phényl)-imidazolidine-2,4-dione
- la 1-(2-phénoxy-pyridin-4-ylméthyl)-3-(4-trifluorométhanesulfonyl-phényl)-imidazolidine-2,4-dione
- la 1-(2-méthoxy-pyridin-4-ylméthyl)-3-(4-trifluorométhanesulfonyl-phényl)-imidazolidine-2,4-dione
- la 1-(2-éthoxy-pyridin-4-ylméthyl)-3-(4-trifluorométhanesulfonyl-phényl)-imidazolidine-2,4-dione
- la 1-[2-(1-pyrrolidinyl)éthoxy-pyridin-4-ylméthyl]-3-(4-trifluorométhanesulfonyl-phényl)-imidazolidine-2,4-dione
- la 1-(2-benzyloxy-pyridin-4-ylméthyl)-3-(4-trifluorométhanesulfonyl-phényl)-imidazolidine-2,4-dione
- la 1-(2-benzoyloxy-pyridin-4-ylméthyl)-3-(4-trifluorométhanesulfonyl-phényl)-imidazolidine-2,4-dione
- la 1-[2-(4,5-dihydro-1H-imidazol-2-yl)-pyridin-4-ylméthyl]-3-(4-trifluorométhanesulfonyl-phényl)-imidazolidine-2,4-dione
- la 1-[2-(1H-imidazol-2-yl)-pyridin-4-ylméthyl]-3-(4-trifluorométhanesulfonyl-phényl)-imidazolidine-2,4-dione
- la 1-[2-(1H-tétrazol-5-yl)-pyridin-4-ylméthyl]-3-(4-trifluorométhanesulfonyl-phényl)-imidazolidine-2,4-dione
- la 1-(2-amidino-pyridin-4-ylméthyl)-3-(4-trifluorométhanesulfonyl-phényl)-imidazolidine-2,4-dione
- la 1-(2-diméthylaminométhyl-pyridin-4-ylméthyl)-3-(4-trifluorométhanesulfonyl-phényl)-imidazolidine-2,4-dione
- la 1-(2-aminométhyl-pyridin-4-ylméthyl)-3-(4-trifluorométhanesulfonyl-phényl)-imidazolidine-2,4-dione
- la 1-(3-méthyl-pyridin-4-ylméthyl)-3-(4-trifluorométhanesulfonyl-phényl)-imidazolidine-2,4-dione
- la 1-(3-éthyl-pyridin-4-ylméthyl)-3-(4-trifluorométhanesulfonyl-phényl)-imidazolidine-2,4-dione
- la 1-(3-isopropyl-pyridin-4-ylméthyl)-3-(4-trifluorométhanesulfonyl-phényl)-imidazolidine-2,4-dione
- la 1-(3-ter-butyl-pyridin-4-ylméthyl)-3-(4-trifluorométhanesulfonyl-phényl)-imidazolidine-2,4-dione
- la 1-(3-isobutyl-pyridin-4-ylméthyl)-3-(4-trifluorométhanesulfonyl-phényl)-imidazolidine-2,4-dione
- la 1-(3-cyclohéxyl-pyridin-4-ylméthyl)-3-(4-trifluorométhanesulfonyl-phényl)-imidazolidine-2,4-dione
- la 1-(3-phényl-pyridin-4-ylméthyl)-3-(4-trifluorométhanesulfonyl-phényl)-imidazolidine-2,4-dione
- la 1-[3-(4-chlorophényl)-pyridin-4-ylméthyl]-3-(4-trifluorométhanesulfonyl-phényl)-imidazolidine-2,4-dione
- la 1-[3-(3-chlorophényl)-pyridin-4-ylméthyl]-3-(4-trifluorométhanesulfonyl-phényl)-imidazolidine-2,4-dione
- la 1-[3-(2-chlorophényl)-pyridin-4-ylméthyl]-3-(4-trifluorométhanesulfonyl-phényl)-imidazolidine-2,4-dione
- la 1-[3-(4-fluorophényl)-pyridin-4-ylméthyl]-3-(4-trifluorométhanesulfonyl-phényl)-imidazolidine-2,4-dione
- la 1-[3-(4-méthoxyphényl)-pyridin-4-ylméthyl]-3-(4-trifluorométhanesulfonyl-phényl)-imidazolidine-2,4-dione
- la 1-(3-benzyl-pyridin-4-ylméthyl)-3-(4-trifluorométhanesulfonyl-phényl)-imidazolidine-2,4-dione 1-(3,2'bipyridin-4-ylméthyl)-3-(4-trifluorométhanesulfonyl-phényl)-imidazolidine-2,4-dione
- la 1-(3-hydroxyméthyl-pyridin-4-ylméthyl)-3-(4-trifluorométhanesulfonyl-phényl)-imidazolidine-2,4-dione
- la 1-(3-chloro-pyridin-4-ylméthyl)-3-(4-trifluorométhanesulfonyl-phényl)-imidazolidine-2,4-dione
- la 1-(3-fluoro-pyridin-4-ylméthyl)-3-(4-trifluorométhanesulfonyl-phényl)-imidazolidine-2,4-dione
- la 1-(3-bromo-pyridin-4-ylméthyl)-3-(4-trifluorométhanesulfonyl-phényl)-imidazolidine-2,4-dione
- la 1-(3-amino-pyridin-4-ylméthyl)-3-(4-trifluorométhanesulfonyl-phényl)-imidazolidine-2,4-dione
- la 1-(3-méthylamino-pyridin-4-ylméthyl)-3-(4-trifluorométhanesulfonyl-phényl)-imidazolidine-2,4-dione
- la 1-(3-éthylamino-pyridin-4-ylméthyl)-3-(4-trifluorométhanesulfonyl-phényl)-imidazolidine-2,4-dione
- la 1-(3-isopropylamino-pyridin-4-ylméthyl)-3-(4-trifluorométhanesulfonyl-phényl)-imidazolidine-2,4-dione
- la 1-(3-phénylamino-pyridin-4-ylméthyl)-3-(4-trifluorométhanesulfonyl-phényl)-imidazolidine-2,4-dione
- la 1-(3-cyclohéxylamino-pyridin-4-ylméthyl)-3-(4-trifluorométhanesulfonyl-phényl)-imidazolidine-2,4-dione
- la 1-(3-diéthylamino-pyridin-4-ylméthyl)-3-(4-trifluorométhanesulfonyl-phényl)-imidazolidine-2,4-dione
- la 1-(3-N-cyclohéxyl-N-méthylamino-pyridin-4-ylméthyl)-3-(4-trifluorométhanesulfonyl-phényl)-imidazolidine-2,4-dione
- la 1-(3-pipéridino-pyridin-4-ylméthyl)-3-(4-trifluorométhanesulfonyl-phényl)-imidazolidine-2,4-dione
- la 1-(3-morpholino-pyridin-4-ylméthyl)-3-(4-trifluorométhanesulfonyl-phényl)-imidazolidine-2,4-dione
- la 1-[3-(4-méthylpipérazino)diéthylamino-pyridin-4-ylméthyl]-3-(4-trifluorométhanesulfonyl-phényl)-imidazolidine-2,4-dione
- la 1-[3-(1H-pyrazol-1yl)-pyridin-4-ylméthyl]-3-(4-trifluorométhanesulfonyl-phényl)-imidazolidine-2,4-dione
- la 1-[3-(2-benzoylhydrazino)-pyridin-4-ylméthyl]-3-(4-trifluorométhanesulfonyl-phényl)-imidazolidine-2,4-dione
- la 1-(3-benzoylamino-pyridin-4-ylméthyl)-3-(4-trifluorométhanesulfonyl-phényl)-imidazolidine-2,4-dione
- la 1-[3-(4-chlorobenzoylamino)-pyridin-4-ylméthyl]-3-(4-trifluorométhanesulfonyl-phényl)-imidazolidine-2,4-dione
- la 1-[3-(3-chlorobenzoylamino)-pyridin-4-ylméthyl]-3-(4-trifluorométhanesulfonyl-phényl)-imidazolidine-2,4-dione
- la 1-[3-(2-chlorobenzoylamino)-pyridin-4-ylméthyl]-3-(4-trifluorométhanesulfonyl-phényl)-imidazolidine-2,4-dione
- la 1-[3-(4-fluorobenzoylamino)-pyridin-4-ylméthyl]-3-(4-trifluorométhanesulfonyl-phényl)-imidazolidine-2,4-dione
- la 1-[3-(4-méthoxybenzoylamino)-pyridin-4-ylméthyl]-3-(4-trifluorométhanesulfonyl-phényl)-imidazolidine-2,4-dione
- la 1-(3-acétamido-pyridin-4-ylméthyl)-3-(4-trifluorométhanesulfonyl-phényl)-imidazolidine-2,4-dione
- la 1-(3-phénylacétamido-pyridin-4-ylméthyl)-3-(4-trifluorométhanesulfonyl-phényl)-imidazolidine-2,4-dione
- la 1-[3-(4-méthylpipérazino)acétamido-pyridin-4-ylméthyl]-3-(4-trifluorométhanesulfonyl-phényl)-imidazolidine-2,4-dione
- la 1-(3-cyclohéxanylcarboxamido-pyridin-4-ylméthyl)-3-(4-trifluorométhanesulfonyl-phényl)-imidazolidine-2,4-dione
- la 1-[3-(1-méthyl-4-pipéridinyl)carbonylamino-pyridin-4-ylméthyl]-3-(4-trifluorométhanesulfonyl-phényl)-imidazolidine-2,4-dione
- la 1-[3-(2-furanylcarbonyl)amino-pyridin-4-ylméthyl]-3-(4-trifluorométhanesulfonyl-phényl)-imidazolidine-2,4-dione
- la 1-(3-éthoxycarbonylamino-pyridin-4-ylméthyl)-3-(4-trifluorométhanesulfonyl-phényl)-imidazolidine-2,4-dione
- la 1-(3-isopropoxycarbonylamino-pyridin-4-ylméthyl)-3-(4-trifluorométhanesulfonyl-phényl)-imidazolidine-2,4-dione
- la 1-(3-butoxycarbonylamino-pyridin-4-ylméthyl)-3-(4-trifluorométhanesulfonyl-phényl)-imidazolidine-2,4-dione
- la 1-[3-(2-diéthylaminoéthoxycarbonylamino)-pyridin-4-ylméthyl]-3-(4-trifluorométhanesulfonyl-phényl)-imidazolidine-2,4-dione
- la 1-(3-phénoxycarbonylamino-pyridin-4-ylméthyl)-3-(4-trifluorométhanesulfonyl-phényl)-imidazolidine-2,4-dione
- la 1-[3-(4-chloroanilino)carbonylamino-pyridin-4-ylméthyl)]-3-(4-trifluorométhanesulfonyl-phényl)-imidazolidine-2,4-dione
- la 1-(3-anilinocarbonylamino-pyridin-4-ylméthyl)-3-(4-trifluorométhanesulfonyl-phényl)-imidazolidine-2,4-dione
- la 1-(3-benzylaminocarbonylamino-pyridin-4-ylméthyl)-3-(4-trifluorométhanesulfonyl-phényl)-imidazolidine-2,4-dione
- la 1-[3-(2-pyridylamino)carbonylamino-pyridin-4-ylméthyl)]-3-(4-trifluorométhanesulfonyl-phényl)-imidazolidine-2,4-dione
- la 1-(3-uréido-pyridin-4-ylméthyl)-3-(4-trifluorométhanesulfonyl-phényl)-imidazolidine-2,4-dione
- la 1-(3-benzylaminosulfonylamino-pyridin-4-ylméthyl)-3-(4-trifluorométhanesulfonyl-phényl)-imidazolidine-2,4-dione
- la 1-(3-méthylaminosulfonylamino-pyridin-4-ylméthyl)-3-(4-trifluorométhanesulfonyl-phényl)-imidazolidine-2,4-dione
- la 1-(3-aminosulfonylamino-pyridin-4-ylméthyl)-3-(4-trifluorométhanesulfonyl-phényl)-imidazolidine-2,4-dione
- la 1-(3-morpholinosulfonylamino-pyridin-4-ylméthyl)-3-(4-trifluorométhanesulfonyl-phényl)-imidazolidine-2,4-dione
- la 1-(3-diméthylaminosulfonylamino-pyridin-4-ylméthyl)-3-(4-trifluorométhanesulfonyl-phényl)-imidazolidine-2,4-dione
- la 1-(3-anilinosulfonylamino-pyridin-4-ylméthyl)-3-(4-trifluorométhanesulfonyl-phényl)-imidazolidine-2,4-dione
- la 1-(3-phénylsulfonylamino-pyridin-4-ylméthyl)-3-(4-trifluorométhanesulfonyl-phényl)-imidazolidine-2,4-dione
- la 1-[3-(2-thiényl)sulfonylamino-pyridin-4-ylméthyl]-3-(4-trifluorométhanesulfonyl-phényl)-imidazolidine-2,4-dione
- la 1-(3-méthylsulfonylamino-pyridin-4-ylméthyl)-3-(4-trifluorométhanesulfonyl-phényl)-imidazolidine-2,4-dione
- la 1-[3-(4-chlorophényl)sulfonylamino-pyridin-4-ylméthyl]-3-(4-trifluorométhanesulfonyl-phényl)-imidazolidine-2,4-dione
- la 1-(3-benzylsulfonylamino-pyridin-4-ylméthyl)-3-(4-trifluorométhanesulfonyl-phényl)-imidazolidine-2,4-dione
- la 1-(3-hydroxycarbonyl-pyridin-4-ylméthyl)-3-(4-trifluorométhanesulfonyl-phényl)-imidazolidine-2,4-dione
- la 1-(3-éthoxycarbonyl-pyridin-4-ylméthyl)-3-(4-trifluorométhanesulfonyl-phényl)-imidazolidine-2,4-dione
- la 1-(3-ter-butoxycarbonyl-pyridin-4-ylméthyl)-3-(4-trifluorométhanesulfonyl-phényl)-imidazolidine-2,4-dione
- la 1-(3-phénoxycarbonyl-pyridin-4-ylméthyl)-3-(4-trifluorométhanesulfonyl-phényl)-imidazolidine-2,4-dione
- la 1-[3-(4-méthylpipérazinocarbonyl)-pyridin-4-ylméthyl]-3-(4-trifluorométhanesulfonyl-phényl)-imidazolidine-2,4-dione
- la 1-[3-(méthylamino)carbonyl-pyridin-4-ylméthyl]-3-(4-trifluorométhanesulfonyl-phényl)-imidazolidine-2,4-dione
- la 1-[3-(diméthylamino)carbonyl-pyridin-4-ylméthyl]-3-(4-trifluorométhanesulfonyl-phényl)-imidazolidine-2,4-dione
- la 1-(3-pipéridinocarbonyl-pyridin-4-ylméthyl)-3-(4-trifluorométhanesulfonyl-phényl)-imidazolidine-2,4-dione
- la 1-(3-morpholinocarbonyl-pyridin-4-ylméthyl)-3-(4-trifluorométhanesulfonyl-phényl)-imidazolidine-2,4-dione
- la 1-[3-(4-phénylpipérazinocarbonyl)-pyridin-4-ylméthyl]-3-(4-trifluorométhanesulfonyl-phényl)-imidazolidine-2,4-dione
- la 1-(3-phénylaminocarbonyl-pyridin-4-ylméthyl)-3-(4-trifluorométhanesulfonyl-phényl)-imidazolidine-2,4-dione
- la 1-[3-(méthylphénylamino)carbonyl-pyridin-4-ylméthyl]-3-(4-trifluorométhanesulfonyl-phényl)-imidazolidine-2,4-dione
- la 1-[3-(4-méthoxyphényl)méthylaminocarbonyl-pyridin-4-ylméthyl]-3-(4-trifluorométhanesulfonyl-phényl)-imidazolidine-2,4-dione
- la 1-[3-(4-chlorophényl)méthylaminocarbonyl-pyridin-4-ylméthyl]-3-(4-trifluorométhanesulfonyl-phényl)-imidazolidine-2,4-dione
- la 1-[3-(4-morpholinylamino)carbonyl-pyridin-4-ylméthyl]-3-(4-trifluorométhanesulfonyl-phényl)-imidazolidine-2,4-dione
- la 1-(3-aminocarbonyl-pyridin-4-ylméthyl)-3-(4-trifluorométhanesulfonyl-phényl)-imidazolidine-2,4-dione
- la 1-(3-phénoxy-pyridin-4-ylméthyl)-3-(4-trifluorométhanesulfonyl-phényl)-imidazolidine-2,4-dione
- la 1-(3-méthoxy-pyridin-4-ylméthyl)-3-(4-trifluorométhanesulfonyl-phényl)-imidazolidine-2,4-dione
- la 1-(3-éthoxy-pyridin-4-ylméthyl)-3-(4-trifluorométhanesulfonyl-phényl)-imidazolidine-2,4-dione
- la 1-[3-(1-pyrrolidinyl)éthoxy-pyridin-4-ylméthyl]-3-(4-trifluorométhanesulfonyl-phényl)-imidazolidiné-2,4-dione
- la 1-(3-benzyloxy-pyridin-4-ylméthyl)-3-(4-trifluoro-méthanesulfonyl-phényl)-imidazolidine-2,4-dione
- la 1-(3-benzoyloxy-pyridin-4-ylméthyl)-3-(4-trifluorométhanesulfonyl-phényl)-imidazolidine-2,4-dione
- la 1-[3-(4,5-dihydro-1H-imidazol-2-yl)-pyridin-4-lméthyl]-3-(4-trifluorométhanesulfonyl-phényl)-imidazolidine-2,4-dione
- la 1-[3-(1H-imidazol-2-yl)-pyridin-4-ylméthyl]-3-(4-trifluorométhanesulfonyl-phényl)-imidazolidine-2,4-dione
- la 1-[3-(1H-tétrazol-5-yl)-pyridin-4-ylméthyl]-3-(4-trifluorométhanesulfonyl-phényl)-imidazolidine-2,4-dione
- la 1-(3-amidino-pyridin-4-ylméthyl)-3-(4-trifluorométhanesulfonyl-phényl)-imidazolidine-2,4-dione
- la 1-(3-diméthylaminométhyl-pyridin)-4-ylméthyl]-3-(4-trifluorométhanesulfonyl-phényl)-imidazolidine-2,4-dione
- la 1-(3-aminométhyl-pyridin-4-ylméthyl)-3-(4-trifluorométhanesulfonyl-phényl)-imidazolidine-2,4-dione
- la 5,5-diméthyl-1-(2-méthyl-quinolin-4-ylméthyl)-3-(4-trifluorométhoxy-phényl)-imidazolidine-2,4-dione
- la 5,5-diméthy-1-1-(2-méthoxy-quinolin-4-ylméthyl)-3-(4-trifluorométhoxy-phényl)-imidazolidine-2,4-dione
- la 5,5-diméthyl-1-(2-chloro-quinolin-4-ylméthyl)-3-(4-trifluorométhoxy-phényl)-imidazolidine-2,4-dione
- la 5,5-diméthyl-1-(2-fluoro-quinolin-4-ylméthyl)-3-(4-trifluorométhoxy-phényl)-imidazolidine-2,4-dione
- la 5,5-diméthyl-1-(2-amino-quinolin-4-ylméthyl)-3-(4-trifluorométhoxy-phényl)-imidazolidine-2,4-dione
- la 5,5-diméthyl-1-(2-méthylamino-quinolin-4-ylméthyl)-3-(4-trifluorométhoxy-phényl)-imidazolidine-2,4-dione
- la 5,5-diméthyl-1-(2-diméthylamino-quinolin-4-ylméthyl)-3-(4-trifluorométhoxy-phényl)-imidazolidine-2,4-dione
- la 5,5-diméthyl-1-(2-pipéridino-quinolin-4-y)méthyl)-3-(4-trifluorométhoxy-phényl)-imidazolidine-2,4-dione
- la 5,5-diméthyl-1-(3-méthyl-quinolin-4-ylméthyl)-3-(4-trifluorométhoxy-phényl)-imidazolidine-2,4-dione
- la 5,5-diméthyl-1-(3-méthoxy-quinolin-4-ylméthyl)-3-(4-trifluorométhoxy-phényl)-imidazolidine-2,4-dione
- la 5,5-diméthyl-1-(3-chloro-quinolin-4-ylméthyl)-3-(4-trifluorométhoxy-phényl)-imidazolidine-2,4-dione
- la 5,5-diméthyl-1-(3-fluoro-quinolin-4-ylméthyl)-3-(4-trifluorométhoxy-phényl)-imidazolidine-2,4-dione
- la 5,5-diméthyl-1-(3-amino-quinolin-4-ylméthyl)-3-(4-trifluorométhoxy-phényl)-imidazolidine-2,4-dione
- la 5,5-diméthyl-1-(3-méthylamino-quinolin-4-ylméthyl)-3-(4-trifluorométhoxy-phényl)-imidazolidine-2,4-dione
- la 5,5-diméthyl-1-(3-diméthylamino-quinolin-4-ylméthyl)-3-(4-trifluorométhoxy-phényl)-imidazolidine-2,4-dione
- la 5,5-diméthyl-1-(3-pipéridino-quinolin-4-ylméthyl)-3-(4-trifluorométhoxy-phényl)-imidazolidine-2,4-dione
- la 5,5-diméthyl-1-(5-méthyl-quinolin-4-ylméthyl)-3-(4-trifluorométhoxy-phényl)-imidazolidine-2,4-dione
- la 5,5-diméthyl-1-(5-méthoxy-quinolin-4-ylméthyl)-3-(4-trifluorométhoxy-phényl)-imidazolidine-2,4-dione
- la 5,5-diméthyl-1-(5-chloro-quinolin-4-ylméthyl)-3-(4-trifluorométhoxy-phényl)-imidazolidine-2,4-dione
- la 5,5-diméthyl-1-(5-fluo-ro-quinolin-4-ylméthyl)-3-(4-trifluorométhoxy-phényl)-imidazolidine-2,4-dione
- la 5,5-diméthyl-1-(5-amino-quinolin-4-ylméthyl),-3-(4-trifluorométhoxy-phényl)-imidazolidine-2,4-dione
- la 5,5-diméthyl-1-(5-méthylamino-quinolin-4-ylméthyl)-3-(4-trifluorométhoxy-phényl)-imidazolidine-2,4-dione
- la 5,5-diméthyl-1-(5-diméthylamino-quinolin-4-ylméthyl)-3-(4-trifluorométhoxy-phényl)-imidazolidine-2,4-dione
- la 5,5-diméthyl-1-(5-pipéridino-quinolin-4-ylméthyl)-3-(4-trifluorométhoxy-phényl)-imidazolidine-2,4-dione
- la 5,5-diméthyl-1-(6-méthyl-quinolin-4-ylméthyl)-3-(4-trifluorométhoxy-phényl)-imidazolidine-2,4-dione
- la 5,5-diméthyl-1-(6-méthoxy-quinolin-4-ylméthyl)-3-(4-trifluorométhoxy-phényl)-imidazolidine-2,4-dione
- la 5,5-diméthyl-1-(6-chloro-quinolin-4-ylméthyl)-3-(4-trifluorométhoxy-phényl)-imidazolidine-2,4-dione
- la 5,5-diméthyl-1-(6-fluoro-quinolin-4-ylméthyl)-3-(4-trifluorométhoxy-phényl)-imidazolidine-2,4-dione
- la 5,5-diméthyl-1-(6-amino-quinolin-4-ylméthyl)-3-(4-trifluorométhoxy-phényl)-imidazolidine-2,4-dione
- la 5,5-diméthyl-1-(6-méthylamino-quinolin-4-ylméthyl)-3-(4-trifluorométhoxy-phényl)-imidazolidine-2,4-dione
- la 5,5-diméthyl-1-(6-diméthylamino-quinolin-4-ylméthyl)-3-(4-trifluorométhoxy-phényl)-imidazolidine-2,4-dione
- la 5,5-diméthyl-1-(6-pipéridino-quinolin-4-ylméthyl)-3-(4-trifluorométhoxy-phényl)-imidazolidine-2,4-dione
- la 5,5-diméthyl-1-(7-méthyl-quinolin-4-ylméthyl)-3-(4-trifluorométhoxy-phényl)-imidazolidine-2,4-dione
- la 5,5-diméthyl-1-(7-méthoxy-quinolin-4-ylméthyl)-3-(4-trifluorométhoxy-phényl)-imidazolidine-2,4-dione
- la 5,5-diméthyl-1-(7-chloro-quinolin-4-ylméthyl)-3-(4-trifluorométhoxy-phényl)-imidazolidine-2,4-dione
- la 5,5-diméthyl-1-(7-fluoro-quinolin-4-ylméthyl)-3-(4-trifluorométhoxy-phényl)-imidazolidine-2,4-dione
- la 5,5-diméthyl-1-(7-amino-quinolin-4-ylméthyl)-3-(4-trifluorométhoxy-phényl)-imidazolidine-2,4-dione
- la 5,5-diméthyl-1-(7-méthylamino-quinolin-4-ylméthyl)-3-(4-trifluorométhoxy-phényl)-imidazolidine-2,4-dione
- la 5,5-diméthyl-1-(7-diméthylamino-quinalin-4-ylméthyl)-3-(4-trifluorométhoxy-phényl)-imidazolidine-2,4-dione
- la 5,5-diméthyl-1-(7-pipéridino-quinolin-4-ylméthyl)-3-(4-trifluorométhoxy-phényl)-imidazolidine-2,4-dione
- la 5,5-diméthyl-1-(8-méthyl-quinolin-4-ylméthyl)-3-(4-trifluorométhoxy-phényl)-imidazolidine-2,4-dione
- la 5,5-diméthyl-1-(8-méthoxy-quinolin-4-ylméthyl)-3-(4-trifluorométhoxy-phényl)-imidazolidine-2,4-dione
- la 5,5-diméthyl-1-(8-chloro-quinolin-4-ylméthyl)-3-(4-trifluorométhoxy-phényl)-imidazolidine-2,4-dione
- la 5,5-diméthyl-1-(8-fluoro-quinolin-4-ylméthyl)-3-(4-trifluorométhoxy-phényl)-imidazolidine-2,4-dione
- la 5,5-diméthyl-1-(8-amino-quinolin-4-ylméthyl)-3-(4-trifluorométhoxy-phényl)-imidazolidine-2,4-dione
- la 5,5-diméthyl-1-(8-méthylamino-quinolin-4-ylméthyl)-3-(4-trifluorométhoxy-phényl)-imidazolidine-2,4-dione
- la 5,5-diméthyl-1-(8-diméthylamino-quinolin-4-ylméthyl)-3-(4-trifluorométhoxy-phényl)-imidazolidine-2,4-dione
- la 5,5-diméthyl-1-(8-pipéridino-quinolin-4-ylméthyl)-3-(4-trifluorométhoxy-phényl)-imidazolidine-2,4-dione
- la 4-(2-méthyl-quinolin)-4-ylméthyl-6-(4-trifluorométhoxy-phényl)-4,6-diaza-spiro[2.4]heptane-5,7-dione
- la 4-(2-méthoxy-quinolin)-4-ylméthyl-6-(4-trifluorométhoxy-phényl)-4,6-diaza-spiro[2.4]heptane-5,7-dione
- la 4-(2-chloro-quinolin)-4-ylméthyl-6-(4-trifluoro-méthoxy-phényl)-4,6-diaza-spiro[2.4]heptane-5,7-dione
- la 4-(2-fluoro-quinolin)-4-ylméthyl-6-(4-trifluorométhoxy-phényl)-4,6-diaza-spiro[2.4]heptane-5,7-dione
- la 4-(2-amino-quinolin)-4-ylméthyl-6-(4-trifluorométhoxy-phényl)-4,6-diaza-spiro[2.4]heptane-5,7-dione
- la 4-(2-méthylamino-quinolin)-4-ylméthyl-6-(4-trifluorométhoxy-phényl)-4,6-diaza-spiro[2.4]heptane-5,7-dione
- la 4-(2-diméthylamino-quinolin)-4-ylméthyl-6-(4-trifluorométhoxy-phényl)-4,6-diaza-spiro[2.4]heptane-5,7-dione
- la 4-(2-pipéridino-quinolin)-4-ylméthyl-6-(4-trifluorométhoxy-phényl)-4,6-diaza-spiro[2.4]heptane-5,7-dione
- la 4-(3-méthyl-quinolin)-4-ylméthyl-6-(4-trifluorométhoxy-phényl)-4,6-diaza-spiro[2.4]heptane-5,7-dione
- la 4-(3-méthoxy-quinolin)-4-ylméthyl-6-(4-trifluorométhoxy-phényl)-4,6-diaza-spiro[2.4]heptane-5,7-dione
- la 4-(3-chloro-quinolin)-4-ylméthyl-6-(4-trifluorométhoxy-phényl)-4,6-diaza-spiro[2.4]heptane-5,7-dione
- la 4-(3-fluoro-quinolin)-4-ylméthyl-6-(4-trifluoro-méthoxy-phényl)-4,6-diaza-spiro[2.4]heptane-5,7-dione
- la 4-(3-amino-quinolin)-4-ylméthyl-6-(4-trifluorométhoxy-phényl)-4,6-diaza-spiro[2.4]heptane-5,7-dione
- la 4-(3-méthylamino-quinolin)-4-ylméthyl-6-(4-trifluorométhoxy-phényl)-4,6-diaza-spiro[2.4]heptane-5,7-dione
- la 4-(3-diméthylamino-quinolin)-4-ylméthyl-6-(4-trifluorométhoxy-phényl)-4,6-diaza-spiro[2.4]heptane-5,7-dione
- la 4-(3-pipéridino-quinolin)-4-ylméthyl-6-(4-trifluorométhoxy-phényl)-4,6-diaza-spiro[2.4]heptane-5,7-dione
- la 4-(5-méthyl-quinolin)-4-ylméthyl-6-(4-trifluorométhoxy-phényl)-4,6-diaza-spiro[2.4]heptane-5,7-dione
- la 4-(5-méthoxy-quinolin)-4-ylméthyl-6-(4-trifluorométhoxy-phényl)-4,6-diaza-spiro[2.4]heptane-5,7-dione
- la 4-(5-chloro-quinolin)-4-ylméthyl-6-(4-trifluorométhoxy-phényl)-4,6-diaza-spiro[2.4]heptane-5,7-dione
- la 4-(5-fluoro-quinolin)-4-ylméthyl-6-(4-trifluorométhoxy-phényl)-4,6-diaza-spiro[2.4]heptane-5,7-dione
- la 4-(5-amino-quinolin)-4-ylméthyl-6-(4-trifluorométhoxy-phényl)-4,6-diaza-spiro[2.4]heptane-5,7-dione
- la 4-(5-méthylamino-quinolin)-4-ylméthyl-6-(4-trifluorométhoxy-phényl)-4,6-diaza-spiro[2.4]heptane-5,7-dione
- la 4-(5-diméthylamino-quinolin)-4-ylméthyl-6-(4-trifluorométhoxy-phényl)-4,6-diaza-spiro[2.4]heptane-5,7-dione
- la 4-(5-pipéridino-quinolin)-4-ylméthyl-6-(4-trifluorométhoxy-phényl)-4,6-diaza-spiro[2.4]heptane-5,7-dione
- la 4-(6-méthyl-quinolin)-4-ylméthyl-6-(4-trifluorométhoxy-phényl)-4,6-diaza-spiro[2.4]heptane-5,7-dione
- la 4-(6-méthoxy-quinolin)-4-ylméthyl-6-(4-trifluorométhoxy-phényl)-4,6-diaza-spiro[2.4]heptane-5,7-dione
- la 4-(6-chloro-quinolin)-4-ylméthyl-6-(4-trifluorométhoxy-phényl)-4,6-diaza-spiro[2.4]heptane-5,7-dione
- la 4-(6-fluoro-quinolin)-4-ylméthyl-6-(4-trifluorométhoxy-phényl)-4,6-diaza-spiro[2.4]heptane-5,7-dione
- la 4-(6-amino-quinolin)-4-ylméthyl-6-(4-trifluorométhoxy-phényl)-4,6-diaza-spiro[2.4]heptane-5,7-dione
- la 4-(6-méthylamino-quinolin)-4-ylméthyl-6-(4-trifluorométhoxy-phényl)-4,6-diaza-spiro[2.4]heptane-5,7-dione
- la 4-(6-diméthylamino-quinolin)-4-ylméthyl-6-(4-trifluorométhoxy-phényl)-4,6-diaza-spiro[2.4]heptané-5,7-dione
- la 4-(6-pipéridino-quinolin)-4-ylméthyl-6-(4-trifluorométhoxy-phényl)-4,6-diaza-spiro[2.4]heptane-5,7-dione
- la 4-(7-méthyl-quinolin)-4-ylméthyl-6-(4-trifluorométhoxy-phényl)-4,6-diaza-spiro[2.4]heptane-5,7-dione
- la 4-(7-méthoxy-quinolin)-4-ylméthyl-6-(4-trifluorométhoxy-phényl)-4,6-diaza-spiro[2.4]heptane-5,7-dione
- la 4-(7-chloro-quinolin)-4-ylméthyl-6-(4-trifluorométhoxy-phényl)-4,6-diaza-spiro[2.4]heptane-5,7-dione
- la 4-(7-fluoro-quinolin)-4-ylméthyl-6-(4-trifluorométhoxy-phényl)-4,6-diaza-spiro[2.4]heptane-5,7-dione
- la 4-(7-amino-quinolin)-4-ylméthyl-6-(4-trifluorométhdxy-phényl)-4,6-diaza-spiro[2.4]heptane-5,7-dione
- la 4-(7-méthylamino-quinolin)-4-ylméthyl-6-(4-trifluorométhoxy-phényl)-4,6-diaza-spiro[2.4]heptane-5,7-dione
- la 4-(7-diméthylamino-quinolin)-4-ylméthyl-6-(4-trifluorométhoxy-phényl)-4,6-diaza-spiro[2.4]heptane-5,7-dione
- la 4-(7-pipéridino-quinolin)-4-ylméthyl-6-(4-trifluorométhoxy-phényl)-4,6-diaza-spiro[2.4]heptane-5,7-dione
- la 4-(8-méthyl-quinolin)-4-ylméthyl-6-(4-trifluorométhoxy-phényl)-4,6-diaza-spiro[2.4]heptane-5,7-dione
- la 4-(8-méthoxy-quinolin)-4-ylméthyl-6-(4-trifluorométhoxy-phényl)-4,6-diaza-spiro[2.4]heptane-5,7-dione
- la 4-(8-chloro-quinolin)-4-ylméthyl-6-(4-trifluorométhoxy-phényl)-4,6-diaza-spiro[2.4]heptane-5,7-dione
- la 4-(8-fluoro-quinolin)-4-ylméthyl-6-(4-trifluorométhoxy-phényl)-4,6-diaza-spiro[2.4]heptane-5,7-dione
- la 4-(8-amino-quinolin)-4-ylméthyl-6-(4-trifluorométhoxy-phényl)-4,6-diaza-spiro[2.4]heptane-5,7-dione
- la 4-(8-méthylamino-quinolin)-4-ylméthyl-6-(4-trifluorométhoxy-phényl)-4,6-diaza-spiro[2.4]heptane-5,7-dione
- la 4-(8-diméthylamino-quinolin)-4-ylméthyl-6-(4-trifluorométhoxy-phényl)-4,6-diaza-spiro[2.4]heptane-5,7-dione
- la 4-(8-pipéridino-quinolin)-4-ylméthyl-6-(4-trifluorométhoxy-phényl)-4,6-diaza-spiro[2.4]heptane-5,7-dione
- la 5-méthyl-1-(2-méthyl-quinolin-4-ylméthyl)-3-(4-trifluorométhoxy-phényl)-imidazolidine-2,4-dione
- la 5-méthyl-1-(2-méthoxy-quinolin-4-ylméthyl)-3-(4-trifluorométhoxy-phényl)-imidazolidine-2,4-dione
- la 5-méthyl-1-(2-chloro-quinolin-4-ylméthyl)-3-(4-trifluorométhoxy-phényl)-imidazolidine-2,4-dione
- la 5-méthyl-1-(2-fluoro-quinolin-4-ylméthyl)-3-(4-trifluorométhoxy-phényl)-imidazolidine-2,4-dione
- la 5-méthyl-1-(2-amino-quinolin-4-ylméthyl)-3-(4-trifluorométhoxy-phényl)-imidazolidine-2,4-dione
- la 5-méthyl-1-(2-méthylamino-quinolin-4-ylméthyl)-3-(4-trifluorométhoxy-phényl)-imidazolidine-2,4-dione
- la 5-méthyl-1-(2-diméthylamino-quinolin-4-ylméthyl)-3-(4-trifluorométhoxy-phényl)-imidazolidine-2,4-dione
- la 5-méthyl-1-(2-pipéridino-quinolin-4-ylméthyl)-3-(4-trifluorométhoxy-phényl)-imidazolidine-2,4-dione

- la 5-méthyl-1-(3-méthyl-quinolin-4-ylméthyl)-3-(4-trifluorométhoxy-phényl)-imidazolidine-2,4-dione
- la 5-méthyl-1-(3-méthoxy-quinolin-4-ylméthyl)-3-(4-trifluorométhoxy-phényl)-imidazolidine-2,4-dione
- la 5-méthyl-1-(3-chloro-quinolin-4-ylméthyl)-3-(4-trifluorométhoxy-phényl)-imidazolidine-2,4-dione
- la 5-méthyl-1-(3-fluoro-quinolin-4-ylméthyl)-3-(4-trifluorométhoxy-phényl)-imidazolidine-2,4-dione
- la 5-méthyl-1-(3-amino-quinolin-4-ylméthyl)-3-(4-trifluorométhoxy-phényl)-imidazolidine-2,4-dione
- la 5-méthyl-1-(3-méthylamino-quinolin-4-ylméthyl)-3-(4-trifluorométhoxy-phényl)-imidazolidine-2,4-dione
- la 5-méthyl-1-(3-diméthylamino-quinolin-4-ylméthyl)-3-(4-trifluorométhoxy-phényl)-imidazolidine-2,4-dione
- la 5-méthyl-1-(3-pipéridino-quinolin-4-ylméthyl)-3-(4-trifluorométhoxy-phényl)-imidazolidine-2,4-dione
- la 5-méthyl-1-(5-méthyl-quinolin-4-ylméthyl)-3-(4-trifluorométhoxy-phényl)-imidazolidine-2,4-dione
- la 5-méthyl-1-(5-méthoxy-quinolin-4-ylméthyl)-3-(4-trifluorométhoxy-phényl)-imidazolidine-2,4-dione
- la 5-méthyl-1-(5-chloro-quinolin-4-ylméthyl)-3-(4-trifluorométhoxy-phényl)-imidazolidine-2,4-dione
- la 5-méthyl-1-(5-fluoro-quinolin-4-ylméthyl)-3-(4-trifluorométhoxy-phényl)-imidazolidine-2,4-dione
- la 5-méthyl-1-(5-amino-quinolin-4-ylméthyl)-3-(4-trifluorométhoxy-phényl)-imidazolidine-2,4-dione
- la 5-méthyl-1-(5-méthylamino-quinolin-4-ylméthyl)-3-(4-trifluorométhoxy-phényl)-imidazolidine-2,4-dione
- la 5-méthyl-1-(5-diméthylamino-quinolin-4-ylméthyl)-3-(4-trifluorométhoxy-phényl)-imidazolidine-2,4-dione
- la 5-méthyl-1-(5-pipéridino-quinolin-4-ylméthyl)-3-(4-trifluorométhoxy-phényl)-imidazolidine-2,4-dione
- la 5-méthyl-1-(6-méthyl-quinolin-4-ylméthyl)-3-(4-trifluorométhoxy-phényl)-imidazolidine-2,4-dione
- la 5-méthyl-1-(6-méthoxy-quinolin-4-ylméthyl)-3-(4-trifluorométhoxy-phényl)-imidazolidine-2,4-dione
- la 5-méthyl-1-(6-chloro-quinolin-4-ylméthyl)-3-(4-trifluorométhoxy-phényl)-imidazolidine-2,4-dione
- la 5-méthyl-1-(6-fluoro-quinolin-4-ylméthyl)-3-(4-trifluorométhoxy-phényl)-imidazolidine-2,4-dione
- la 5-méthyl-1-(6-amino-quinolin-4-ylméthyl)-3-(4-trifluorométhoxy-phényl)-imidazolidine-2,4-dione
- la 5-méthyl-1-(6-méthylamino-quinolin-4-ylméthyl)-3-(4-trifluorométhoxy-phényl)-imidazolidine-2,4-dione
- la 5-méthyl-1-(6-diméthylamino-quinolin-4-ylméthyl)-3-(4-trifluorométhoxy-phényl)-imidazolidine-2,4-dione
- la 5-méthyl-1-(6-pipéridino-quinolin-4-ylméthyl)-3-(4-trifluorométhoxy-phényl)-imidazolidine-2,4-dione
- la 5-méthyl-1-(7-méthyl-quinolin-4-ylméthyl)-3-(4-trifluorométhoxy-phényl)-imidazolidine-2,4-dione
- la 5-méthyl-1-(7-méthoxy-quinolin-4-ylméthyl)-3-(4-trifluorométhoxy-phényl)-imidazolidine-2,4-dione
- la 5-méthyl-1-(7-chloro-quinolin-4-ylméthyl)-3-(4-trifluorométhoxy-phényl)-imidazolidine-2,4-dione
- la 5-méthyl-1-(7-fluoro-quinolin-4-ylméthyl)-3-(4-trifluorométhoxy-phényl)-imidazolidine-2,4-dione
- la 5-méthyl-1-(7-amino-quinolin-4-ylméthyl)-3-(4-trifluorométhoxy-phényl)-imidazolidine-2,4-dione
- la 5-méthyl-1-(7-méthylamino-quinolin-4-ylméthyl)-3-(4-trifluorométhoxy-phényl)-imidazolidine-2,4-dione
- la 5-méthyl-1-(7-diméthylamino-quinolin-4-ylméthyl)-3-(4-trifluorométhoxy-phényl)-imidazolidine-2,4-dione
- la 5-méthyl-1-(7-pipéridino-quinolin-4-ylméthyl)-3-(4-trifluorométhoxy-phény-1)-imidazolidine-2,4-dione
- la 5-méthyl-1-(8-méthyl-quinolin-4-ylméthyl)-3-(4-trifluorométhoxy-phényl)-imidazolidine-2,4-dione
- la 5-méthyl-1-(8-méthoxy-quinolin-4-ylméthyl)-3-(4-trifluorométhoxy-phényl)-imidazolidine-2,4-dione
- la 5-méthyl-1-(8-chloro-quinolin-4-ylméthyl)-3-(4-trifluorométhoxy-phényl)-imidazolidine-2,4-dione
- la 5-méthyl-1-(8-fluoro-quinolin-4-ylméthyl)-3-(4-trifluorométhoxy-phényl)-imidazolidine-2,4-dione
- la 5-méthyl-1-(8-amino-quinolin-4-ylméthyl)-3-(4-trifluorométhoxy-phényl)-imidazolidine-2,4-dione
- la 5-méthyl-1-(8-méthylamino-quinolin-4-ylméthyl)-3-(4-trifluorométhoxy-phényl)-imidazolidine-2,4-dione
- la 5-méthyl-1-(8-diméthylamino-quinolin-4-ylméthyl)-3-(4-trifluorométhoxy-phényl)-imidazolidine-2,4-dione
- la 5-méthyl-1-(8-pipéridino-quinolin-4-ylméthyl)-3-(4-trifluorométhoxy-phényl)-imidazolidine-2,4-dione
- la 1-(2-méthyl-quinolin-4-ylméthyl)-3-(4-trifluorométhoxy-phényl)-imidazolidine-2,4-dione
- la 1-(2-méthoxy-quinolin-4-ylméthyl)-3-(4-trifluorométhoxy-phényl)-imidazolidine-2,4-dione
- la 1-(2-chloro-quinolin-4-ylméthyl)-3-(4-trifluorométhoxy-phényl)-imidazolidine-2,4-dione
- la 1-(2-fluoro-quinolin-4-ylméthyl)-3-(4-trifluorométhoxy-phényl)-imidazolidine-2,4-dione
- la 1-(2-amino-quinolin-4-ylméthyl)-3-(4-trifluorométhoxy-phényl)-imidazolidine-2,4-dione
- la 1-(2-méthylamino-quinolin-4-ylméthyl)-3-(4-trifluorométhoxy-phényl)-imidazolidine-2,4-dione
- la 1-(2-diméthylamino-quinolin-4-ylméthyl)-3-(4-trifluorométhoxy-phényl)-imidazolidine-2,4-dione
- la 1-(2-pipéridino-quinolin-4-ylméthyl)-3-(4-trifluorométhoxy-phényl)-imidazolidine-2,4-dione
- la 1-(3-méthyl-quinolin-4-ylméthyl)-3-(4-trifluorométhoxy-phényl)-imidazolidine-2,4-dione
- la 1-(3-méthoxy-quinolin-4-ylméthyl)-3-(4-trifluorométhoxy-phényl)-imidazolidine-2,4-dione
- la 1-(3-chloro-quinolin-4-ylméthyl)-3-(4-trifluorométhoxy-phényl)-imidazolidine-2,4-dione
- la 1-(3-fluoro-quinolin-4-ylméthyl)-3-(4-trifluorométhoxy-phényl)-imidazolidine-2,4-dione
- la 1-(3-amino-quinolin-4-ylméthyl)-3-(4-trifluorométhoxy-phényl)-imidazolidine-2,4-dione
- la 1-(3-méthylamino-quinolin-4-ylméthyl)-3-(4-trifluorométhoxy-phényl)-imidazolidine-2,4-dione
- la 1-(3-diméthylamino-quinolin-4-ylméthyl)-3-(4-trifluorométhoxy-phényl)-imidazolidine-2,4-dione
- la 1-(3-pipéridino-quinolin-4-ylméthyl)-3-(4-trifluorométhoxy-phényl)-imidazolidine-2,4-dione
- la 1-(5-méthyl-quinolin-4-ylméthyl)-3-(4-trifluorométhoxy-phényl)-imidazolidine-2,4-dione
- la 1-(5-méthoxy-quinolin-4-ylméthyl)-3-(4-trifluorométhoxy-phényl)-imidazolidine-2,4-dione
- la 1-(5-chloro-quinolin-4-ylméthyl)-3-(4-trifluorométhoxy-phényl)-imidazolidine-2,4-dione
- la 1-(5-fluoro-quinolin-4-ylméthyl)-3-(4-trifluorométhoxy-phényl)-imidazolidine-2,4-dione
- la 1-(5-amino-quinolin-4-ylméthyl)-3-(4-trifluorométhoxy-phényl)-imidazolidine-2,4-dione
- la 1-(5-méthylamino-quinolin-4-ylméthyl)-3-(4-trifluorométhoxy-phényl)-imidazolidine-2,4-dione
- la 1-(5-diméthylamino-quinolin-4-ylméthyl)-3-(4-trifluorométhoxy-phényl)-imidazolidine-2,4-dione
- la 1-(5-pipéridino-quinolin-4-ylméthyl)-3-(4-trifluorométhoxy-phényl)-imidazolidine-2,4-dione
- la 1-(6-méthyl-quinolin-4-ylméthyl)-3-(4-trifluorométhoxy-phényl)-imidazolidine-2,4-dione
- la 1-(6-méthoxy-quinolin-4-ylméthyl)-3-(4-trifluorométhoxy-phényl)-imidazolidine-2,4-dione
- la 1-(6-chloro-quinolin-4-ylméthyl)-3-(4-trifluorométhoxy-phényl)-imidazolidine-2,4-dione
- la 1-(6-fluoro-quinolin-4-ylméthyl)-3-(4-trifluorométhoxy-phényl)-imidazolidine-2,4-dione
- la 1-(6-amino-quinolin-4-ylméthyl)-3-(4-trifluorométhoxy-phényl)-imidazolidine-2,4-dione
- la 1-(6-méthylamino-quinolin-4-ylméthyl)-3-(4-trifluorométhoxy-phényl)-imidazolidine-2,4-dione
- la 1-(6-diméthylamino-quinolin-4-ylméthyl)-3-(4-trifluorométhoxy-phényl)-imidazolidine-2,4-dione
- la 1-(6-pipéridino-quinolin-4-ylméthyl)-3-(4-trifluorométhoxy-phényl)-imidazolidine-2,4-dione
- la 1-(7-méthyl-quinolin-4-ylméthyl)-3-(4-trifluorométhoxy-phényl)-imidazolidine-2,4-dione
- la 1-(7-méthoxy-quinolin-4-ylméthyl)-3-(4-trifluorométhoxy-phényl)-imidazolidine-2,4-dione
- la 1-(7-chloro-quinolin-4-ylméthyl)-3-(4-trifluorométhoxy-phényl)-imidazolidine-2,4-dione
- la 1-(7-fluoro-quinolin-4-ylméthyl)-3-(4-trifluorométhoxy-phényl)-imidazolidine-2,4-dione
- la 1-(7-amino-quinolin-4-ylméthyl)-3-(4-trifluorométhoxy-phényl)-imidazolidine-2,4-dione
- la 1-(7-méthylamino-quinolin-4-ylméthyl)-3-(4-trifluorométhoxy-phényl)-imidazolidine-2,4-dione
- la 1-(7-diméthylamino-quinolin-4-ylméthyl)-3-(4-trifluorométhoxy-phényl)-imidazolidine-2,4-dione
- la 1-(7-pipéridino-quinolin-4-ylméthyl)-3-(4-trifluorométhoxy-phényl)-imidazolidine-2,4-dione
- la 1-(8-méthyl-quinolin-4-ylméthyl)-3-(4-trifluorométhoxy-phényl)-imidazolidine-2,4-dione
- la 1-(8-méthoxy-quinolin-4-ylméthyl)-3-(4-trifluorométhoxy-phényl)-imidazolidine-2,4-dione
- la 1-(8-chloro-quinolin-4-ylméthyl)-3-(4-trifluorométhoxy-phényl)-imidazolidine-2,4-dione
- la 1-(8-fluoro-quinolin-4-ylméthyl)-3-(4-trifluorométhoxy-phényl)-imidazolidine-2,4-dione
- la 1-(8-amino-quinolin-4-ylméthyl)-3-(4-trifluorométhoxy-phényl)-imidazolidine-2,4-dione
- la 1-(8-méthylamino-quinolin-4-ylméthyl)-3-(4-trifluorométhoxy-phényl)-imidazolidine-2,4-dione
- la 1-(8-diméthylamino-quinolin-4-ylméthyl)-3-(4-trifluorométhoxy-phényl)-imidazolidine-2,4-dione
- la 1-(8-pipéridino-quinolin-4-ylméthyl)-3-(4-trifluorométhoxy-phényl)-imidazolidine-2,4-dione
- la 5,5-diméthyl-1-(2-méthyl-quinolin-4-ylméthyl)-3-(4-trifluorométhanesulfanyl-phényl)-imidazolidine-2,4-dione
- la 5,5-diméthyl-1-(2-méthoxy-quinolin-4-ylméthyl)-3-(4-trifluorométhanesulfanyl-phényl)-imidazolidine-2,4-dione
- la 5,5-diméthyl-1-(2-chloro-quinolin-4-ylméthyl)-3-(4-trifluorométhanesulfanyl-phényl)-imidazolidine-2,4-dione
- la 5,5-diméthyl-1-(2-fluoro-quinolin-4-ylméthyl)-3-(4-trifluorométhanesulfanyl-phényl)-imidazolidine-2,4-dione
- la 5,5-diméthyl-1-(2-amino-quinolin-4-ylméthyl)-3-(4-trifluorométhanesulfanyl-phényl)-imidazolidine-2,4-dione
- la 5,5-diméthyl-1-(2-méthylamino-quinolin-4-ylméthyl)-3-(4-trifluorométhanesulfanyl-phényl)-imidazolidine-2,4-dione
- la 5,5-diméthyl-1-(2-diméthylamino-quinolin-4-ylméthyl)-3-(4-trifluorométhanesulfanyl-phényl)-imidazolidine-2,4-dione
- la 5,5-diméthyl-1-(2-pipéridino-quinolin-4-ylméthyl)-3-(4-trifluorométhanesulfanyl-phényl)-imidazolidine-2,4-dione
- la 5,5-diméthyl-1-(3-méthyl-quinolin-4-ylméthyl)-3-(4-trifluorométhanesulfanyl-phényl)-imidazolidine-2,4-dione
- la 5,5-diméthyl-1-(3-méthoxy-quinolin-4-ylméthyl)-3-(4-trifluorométhanesulfanyl-phényl)-imidazolidine-2,4-dione
- la 5,5-diméthyl-1-(3-chloro-quinolin-4-ylméthyl)-3-(4-trifluorométhanesulfanyl-phényl)-imidazolidine-2,4-dione
- la 5,5-diméthyl-1-(3-fluoro-quinolin-4-ylméthyl)-3-(4-trifluorométhanesulfanyl-phényl)-imidazolidine-2,4-dione
- la 5,5-diméthyl-1-(3-amino-quinolin-4-ylméthyl)-3-(4-trifluorométhanesulfanyl-phényl)-imidazolidine-2,4-dione
- la 5,5-diméthyl-1-(3-méthylamino-quinolin-4-ylméthyl)-3-(4-trifluorométhanesulfanyl-phényl)-imidazolidine-2,4-dione
- la 5,5-diméthyl-1-(3-diméthylamino-quinolin-4-ylméthyl)-3-(4-trifluorométhanesulfanyl-phényl)-imidazolidine-2,4-dione
- la 5,5-diméthyl-1-(3-pipéridino-quinolin-4-ylméthyl)-3-(4-trifluorométhanesulfanyl-phényl)-imidazolidine-2,4-dione
- la 5,5-diméthyl-1-(5-méthyl-quinolin-4-ylméthyl)-3-(4-trifluorométhanesulfanyl-phényl)-imidazolidine-2,4-dione
- la 5,5-diméthyl-1-(5-méthoxy-quinolin-4-ylméthyl)-3-(4-trifluorométhanesulfanyl-phényl)-imidazolidine-2,4-dione
- la 5,5-diméthyl-1-(5-chloro-quinolin-4-ylméthyl)-3-(4-trifluorométhanesulfanyl-phényl)-imidazolidine-2,4-dione
- la 5,5-diméthyl-1-(5-fluoro-quinolin-4-ylméthyl)-3-(4-trifluorométhanesulfanyl-phényl)-imidazolidine-2,4-dione
- la 5,5-diméthyl-1-(5-amino-quinolin-4-ylméthyl)-3-(4-trifluorométhanesulfanyl-phényl)-imidazolidine-2,4-dione
- la 5,5-diméthyl-1-(5-méthylamino-quinolin-4-ylméthyl)-3-(4-trifluorométhanesulfanyl-phényl)-imidazolidine-2,4-dione
- la 5,5-diméthyl-1-(5-diméthylamino-quinolin-4-ylméthyl)-3-(4-trifluorométhanesulfanyl-phényl)-imidazolidine-2,4-dione
- la 5,5-diméthyl-1-(5-pipéridino-quinolin-4-ylméthyl)-3-(4-trifluorométhanesulfanyl-phényl)-imidazolidine-2,4-dione
- la 5,5-diméthyl-1-(6-méthyl-quinolin-4-ylméthyl)-3-(4-trifluorométhanesulfanyl-phényl)-imidazolidine-2,4-dione
- la 5,5-diméthyl-1-(6-méthoxy-quinolin-4-ylméthyl)-3-(4-trifluorométhanesulfanyl-phényl)-imidazolidine-2,4-dione
- 1a 5,5-diméthyl-1-(6-chloro-quinolin-4-ylméthyl)-3-(4-trifluorométhanésulfanyl-phényl)-imidazolidine-2,4-dione
- la 5,5-diméthyl-1-(6-fluoro-quinolin-4-ylméthyl)-3-(4-trifluorométhanesulfanyl-phényl)-imidazolidine-2,4-dione
- la 5,5-diméthyl-1-(6-amino-quinolin-4-ylméthyl)-3-(4-trifluorométhanesulfanyl-phényl)-imidazolidine-2,4-dione
- la 5,5-diméthyl-1-(6-méthylamino-quinolin-4-ylméthyl)-3-(4-trifluorométhanesulfanyl-phényl)-imidazolidine-2,4-dione
- la 5,5-diméthyl-1-(6-diméthylamino-quinolin-4-ylméthyl)-3-(4-trifluorométhanesulfanyl-phényl)-imidazolidine-2,4-dione
- la 5,5-diméthyl-1-(6-pipéridino-quinolin-4-ylméthyl)-3-(4-trifluorométhanesulfanyl-phényl)-imidazolidine-2,4-dione
- la 5,5-diméthyl-1-(7-méthyl-quinolin-4-ylméthyl)-3-(4-trifluorométhanesulfanyl-phényl)-imidazolidine-2,4-dione
- la 5,5-diméthyl-1-(7-méthoxy-quinolin-4-ylméthyl)-3-(4-trifluorométhanesulfanyl-phényl)-imidazolidine-2,4-dione
- la 5,5-diméthyl-1-(7-chloro-quinolin-4-ylméthyl)-3-(4-trifluorométhanesulfanyl-phényl)-imidazolidine-2,4-dione
- la 5,5-diméthyl-1-(7-fluoro-quinolin-4-ylméthyl)-3-(4-trifluorométhanesulfanyl-phényl)-imidazolidine-2,4-dione
- la 5,5-diméthyl-1-(7-amino-quinolin-4-ylméthyl)-3-(4-trifluorométhanesulfanyl-phényl)-imidazolidine-2,4-dione
- la 5,5-diméthyl-1-(7-méthylamino-quinolin-4-ylméthyl)-3-(4-trifluorométhanesulfanyl-phényl)-imidazolidine-2,4-dione
- la 5,5-diméthyl-1-(7-diméthylamino-quinolin-4-ylméthyl)-3-(4-trifluorométhanesulfanyl-phényl)-imidazolidine-2,4-dione
- la 5,5-diméthyl-1-(7-pipéridino-quinolin-4-ylméthyl)-3-(4-trifluoromé-thanesulfanyl-phényl)-imidazolidine-2,4-dione
- la 5,5-diméthyl-1-(8-méthyl-quinolin-4-ylméthyl)-3-(4-trifluorométhanesulfanyl-phényl)-imidazolidine-2,4-dione
- la 5,5-diméthyl-1-(8-méthoxy-quinolin-4-ylméthyl)-3-(4-trifluorométhanesulfanyl-phényl)-imidazolidine-2,4-dione
- la 5,5-diméthyl-1-(8-chloro-quinolin-4-ylméthyl)-3-(4-trifluorométhanesulfanyl-phényl)-imidazolidine-2,4-dione
- la 5,5-diméthyl-1-(8-fluoro-quinolin-4-ylméthyl)-3-(4-trifluorométhanesulfanyl-phényl)-imidazolidine-2,4-dione
- la 5,5-diméthyl-1-(8-amino-quinolin-4-ylméthyl)-3-(4-trifluorométhanesulfanyl-phényl)-imidazolidine-2,4-dione
- la 5,5-diméthyl-1-(8-méthylamino-quinolin-4-ylméthyl)-3-(4-trifluorométhanesulfanyl-phényl)-imidazolidine-2,4-dione
- la 5,5-diméthyl-1-(8-diméthylamino-quinolin-4-ylméthyl)-3-(4-trifluorométhanesulfanyl-phényl)-imidazolidine-2,4-dione
- la 5,5-diméthyl-1-(8-pipéridino-quinolin-4-ylméthyl)-3-(4-trifluorométhanesulfanyl-phényl)-imidazolidine-2,4-dione
- la 4-(2-méthyl-quinolin)-4-ylméthyl-6-(4-trifluorométhanesulfanyl-phényl)-4,6-diaza-spiro[2.4]heptane-5,7-dione
- la 4-(2-méthoxy-quinolin)-4-ylméthyl-6-(4-trifluorométhanesulfanyl-phényl)-4,6-diaza-spiro[2.4]heptane-5,7-dione
- la 4-(2-chloro-quinolin)-4-ylméthyl-6-(4-trifluorométhanesulfanyl-phényl)-4,6-diaza-spiro[2.4]heptane-5,7-dione
- la 4-(2-fluoro-quinolin)-4-ylméthyl-6-(4-trifluorométhanesulfanyl-phényl)-4,6-diaza-spiro[2.4]heptane-5,7-dione
- la 4-(2-amino-quinolin)-4-ylméthyl-6-(4-trifluorométhanesulfanyl-phényl)-4,6-diaza-spiro[2.4]heptane-5,7-dione
- la 4-(2-méthylamino-quinolin)-4-ylméthyl-6-(4-trifluorométhanesulfanyl-phényl)-4,6-diaza-spiro[2.4]heptane-5,7-dione
- la 4-(2-diméthylamino-quinolin)-4-ylméthyl-6-(4-trifluorométhanésulfanyl-phényl)-4,6-diaza-spiro[2.4]heptane-5,7-dione
- la 4-(2-pipéridino-quinolin)-4-ylméthyl-6-(4-trifluorométhanesulfanyl-phényl)-4,6-diaza-spiro[2.4]heptane-5,7-dione
- la 4-(3-méthyl-quinolin)-4-ylméthyl-6-(4-trifluorométhanesulfanyl-phényl)-4,6-diaza-spiro[2.4]heptane-5,7-dione
- la 4-(3-méthoxy-quinolin)-4-ylméthyl-6-(4-trifluorométhanesulfanyl-phényl)-4,6-diaza-spiro[2.4]heptane-5,7-dione
- la 4-(3-chloro-quinolin)-4-ylméthyl-6-(4-trifluorométhanesulfanyl-phényl)-4,6-diaza-spiro[2.4]heptane-5,7-dione
- la 4-(3-fluoro-quinolin)-4-ylméthyl-6-(4-trifluorométhanesulfanyl-phényl)-4,6-diaza-spiro[2.4]heptane-5,7-dione
- la 4-(3-amino-quinolin)-4-ylméthyl-6-(4-trifluorométhanesulfanyl-phényl)-4,6-diaza-spiro[2.4]heptane-5,7-dione
- la 4-(3-méthylamino-quinolin)-4-ylméthyl-6-(4-trifluorométhanesulfanyl-phényl)-4,6-diaza-spiro[2.4] heptane-5,7-dione
- la 4-(3-diméthylamino-quinolin)-4-ylméthyl-6-(4-trifluorométhanesulfanyl-phényl)-4,6-diaza-spiro[2.4] heptane-5,7-dione
- la 4-(3-pipéridino-quinolin)-4-ylméthyl-6-(4-trifluorométhanesulfanyl-phényl)-4,6-diaza-spiro[2.4]heptane-5,7-dione
- la 4-(5-méthyl-quinolin)-4-ylméthyl-6-(4-trifluorométhanesulfanyl-phényl)-4,6-diaza-spiro[2.4]heptane-5,7-dione
- la 4-(5-méthoxy-quinolin)-4-ylméthyl-6-(4-trifluoro-méthanesulfanyl-phényl)-4,6-diaza-spiro[2.4]heptane-5,7-dione
- la 4-(5-chloro-quinolin)-4-ylméthyl-6-(4-trifluorométhanesulfanyl-phényl)-4,6-diaza-spiro[2.4]heptane-5,7-dione
- la 4-(5-fluoro-quinolin)-4-ylméthyl-6-(4-trifluorométhanesulfanyl-phényl)-4,6-diaza-spiro[2.4]heptane-5,7-dione
- la 4-(5-amino-quinolin)-4-ylméthyl-6-(4-trifluorométhanesulfanyl-phényl)-4,6-diaza-spiro[2.4]heptane-5,7-dione
- la 4-(5-méthylamino-quinolin)-4-ylméthyl-6-(4-trifluorométhanesulfanyl-phényl)-4,6-diaza-spiro[2.4] heptane-5,7-dione
- la 4-(5-diméthylamino-quinolin)-4-ylméthyl-6-(4-trifluorométhanesulfanyl-phényl)-4,6-diaza-spiro[2,4] heptane-5,7-dione
- la 4-(5-pipéridino-quinolin)-4-ylméthyl-6-(4-trifluorométhanesulfanyl-phényl)-4,6-diaza-spiro[2.4]heptane-5,7-dione
- la 4-(6-méthyl-quinolin)-4-ylméthyl-6-(4-trifluorométhanesulfanyl-phényl)-4,6-diaza-spiro[2.4]heptane-5,7-dione
- la 4-(6-méthoxy-quinolin)-4-ylméthyl-6-(4-trifluorométhanesulfanyl-phényl)-4,6-diaza-spiro[2.4]heptane-5,7-dione
- la 4-(6-chloro-quinolin)-4-ylméthyl-6-(4-trifluorométhanesulfanyl-phényl)-4,6-diaza-spiro[2.4]heptane-5,7-dione
- la 4-(6-fluoro-quinolin)-4-ylméthyl-6-(4-trifluorométhanesulfanyl-phényl)-4,6-diaza-spiro[2.4]heptane-5,7-dione
- la 4-(6-amino-quinolin)-4-ylméthyl-6-(4-trifluorométhanesulfanyl-phényl)-4,6-diaza-spiro[2.4]heptane-5,7-dione
- la 4-(6-méthylamino-quinolin)-4-ylméthyl-6-(4-trifluorométhanesulfanyl-phényl)-4,6-diaza-spiro[2.4] heptane-5,7-dione
- la 4-(6-diméthylamino-quinolin)-4-ylméthyl-6-(4-trifluorométhanesulfanyl-phényl)-4,6-diaza-spiro[2.4] heptane-5,7-dione
- la 4-(6-pipéridino-quinolin)-4-ylméthyl-6-(4-trifluorométhanesulfanyl-phényl)-4,6-diaza-spiro[2.4]heptane-5,7-dione
- la 4-(7-méthyl-quinolin)-4-ylméthyl-6-(4-trifluorométhanesulfanyl-phényl)-4,6-diaza-spiro[2.4]heptane-5,7-dione
- la 4-(7-méthoxy-quinolin)-4-ylméthyl-6-(4-trifluorométhanesulfanyl-phényl)-4,6-diaza-spiro[2.4]heptane-5,7-dione
- la 4-(7-chloro-quinolin)-4-ylméthyl-6-(4-trifluorométhanesulfanyl-phényl)-4,6-diaza-spiro[2.4]heptane-5,7-dione
- la 4-(7-fluoro-quinolin)-4-ylméthyl-6-(4-trifluorométhanesulfanyl-phényl)-4,6-diaza-spiro[2.4]heptane-5,7-dione
- la 4-(7-amino-quinolin)-4-ylméthyl-6-(4-trifluorométhanesulfanyl-phényl)-4,6-diaza-spiro[2.4]heptane-5,7-dione
- la 4-(7-méthylamino-quinolin)-4-ylméthyl-6-(4-trifluorométhanesulfanyl-phényl)-4,6-diaza-spiro[2.4] heptane-5,7-dione
- la 4-(7-diméthylamino-quinolin)-4-ylméthyl-6-(4-trifluorométhanesulfanyl-phényl)-4,6-diaza-spiro[2.4] heptane-5,7-dione
- la 4-(7-pipéridino-quinolin)-4-ylméthyl-6-(4-trifluorométhanesulfanyl-phényl)-4,6-diaza-spiro[2.4]heptane-5,7-diode
- la 4-(8-méthyl-quinolin)-4-ylméthyl-6-(4-trifluorométhanesulfanyl-phényl)-4,6-diaza-spiro[2.4]heptane-5,7-dione
- la 4-(8-méthoxy-quinolin)-4-ylméthyl-6-(4-trifluorométhanesulfanyl-phényl)-4,6-diaza-spiro[2.4]heptane-5,7-dione
- la 4-(8-chloro-quinolin)-4-ylméthyl-6-(4-trifluorométhanesulfanyl-phényl)-4,6-diaza-spiro[2.4]heptane-5,7-dione
- la 4-(8-fluoro-quinolin)-4-ylméthyl-6-(4-trifluorométhanesulfanyl-phényl)-4,6-diaza-spiro[2.4]heptane-5,7-dione
- la 4-(8-amino-quinolin)-4-ylméthyl-6-(4-trifluorométhanesulfanyl-phényl)-4,6-diaza-spiro[2.4]heptane-5,7-dione
- la 4-(8-méthylamino-quinolin)-4-ylméthyl-6-(4-trifluorométhanesulfanyl-phényl)-4,6-diaza-spiro[2.4] heptane-5,7-dione
- la 4-(8-diméthylamino-quinolin)-4-ylméthyl-6-(4-trifluorométhanesulfanyl-phényl)-4,6-diaza-spiro[2.4] heptane-5,7-dione
- la 4-(8-pipéridino-quinolin)-4-ylméthyl-6-(4-trifluorométhanesulfanyl-phényl)-4,6-diaza-spiro[2.4]heptane-5,7-dione
- la 5-méthyl-1-(2-méthyl-quinolin-4-ylméthyl)-3-(4-trifluorométhanesulfanyl-phényl)-imidazolidine-2,4-dione
- la 5-méthyl-1-(2-méthoxy-quinolin-4-ylméthyl)-3-(4-trifluorométhanesulfanyl-phényl)-imidazolidine-2,4-dione
- la 5-méthyl-1-(2-chloro-quinolin-4-ylméthyl)-3-(4-trifluorométhanesulfanyl-phényl)-imidazolidine-2,4-dione
- la 5-méthyl-1-(2-fluoro-quinolin-4-ylméthyl)-3-(4-trifluorométhanesulfanyl-phényl)-imidazolidine-2,4-dione
- la 5-méthyl-1-(2-amino-quinolin-4-ylméthyl)-3-(4-trifluorométhanesulfanyl-phényl)-imidazolidine-2,4-dione
- la 5-méthyl-1-(2-méthylamino-quinolin-4-ylméthyl)-3-(4-trifluorométhanesulfanyl-phényl)-imidazolidine-2,4-dione
- la 5-méthyl-1-(2-diméthylamino-quinolin-4-ylméthyl)-3-(4-trifluorométhanesulfanyl-phényl)-imidazolidine-2,4-dione
- la 5-méthyl-1-(2-pipéridino-quinolin-4-ylméthyl)-3-(4-trifluorométhanesulfanyl-phényl)-imidazolidine-2,4-dione
- la 5-méthyl-1-(3-méthyl-quinolin-4-ylméthyl)-3-(4-trifluorométhanesulfanyl-phényl)-imidazolidine-2,4-dione
- la 5-méthyl-1- (3-méthoxy-quinolin-4-ylméthyl)-3-(4-trifluorométhanesulfanyl-phényl)-imidazolidine-2,4-dione
- la 5-méthyl-1-(3-chloro-quinolin-4-ylméthyl)-3-(4-trifluorométhanesulfanyl-phényl)-imidazolidine-2,4-dione
- la 5-méthyl-1-(3-fluoro-quinolin-4-ylméthyl)-3-(4-trifluorométhanesulfanyl-phényl)-imidazolidine-2,4-dione
- la 5-méthyl-1-(3-amino-quinolin-4-ylméthyl)-3-(4-trifluorométhanesulfanyl-phényl)-imidazolidine-2,4-dione
- la 5-méthyl-1-(3-méthylamino-quinolin-4-ylméthyl)-3-(4-trifluorométhanesulfanyl-phényl)-imidazolidine-2,4-dione
- la 5-méthyl-1-(3-diméthylamino-quinolin-4-ylméthyl)-3-(4-trifluorométhanesulfanyl-phényl)-imidazolidine-2,4-dione
- la 5-méthyl-1-(3-pipéridino-quinolin-4-ylméthyl)-3-(4-trifluorométhanesulfanyl-phényl)-imidazolidine-2,4-dione
- la 5-méthyl-1-(5-méthyl-quinolin-4-ylméthyl)-3-(4-trifluorométhanesulfanyl-phényl)-imidazolidine-2,4-dione
- la 5-méthyl-1-(5-méthoxy-quinolin-4-ylméthyl)-3-(4-trifluorométhanesulfanyl-phényl)-imidazolidine-2,4-dione
- la 5-méthyl-1-(5-chloro-quinolin-4-ylméthyl)-3-(4-trifluorométhanesulfanyl-phényl)-imidazolidine-2,4-dione
- la 5-méthyl-1-(5-fluoro-quinolin-4-ylméthyl)-3-(4-trifluorométhanesulfanyl-phényl)-imidazolidine-2,4-dione
- la 5-méthyl-1-(5-amino-quinolin-4-ylméthyl)-3-(4-trifluorométhanesulfanyl-phényl)-imidazolidine-2,4-dione
- la 5-méthyl-1-(5-méthylamino-quinolin-4-ylméthyl)-3-(4-trifluorométhanesulfanyl-phényl)-imidazolidine-2,4-dione
- la 5-méthyl-1-(5-diméthylamino-quinolin-4-ylméthyl)-3-(4-trifluorométhanesulfanyl-phényl)-imidazolidine-2,4-dione
- la 5-méthyl-1-(5-pipéridino-quinolin-4-ylméthyl)-3-(4-trifluorométhanesulfanyl-phényl)-imidazolidine-2,4-dione
- la 5-méthyl-1-(6-méthyl-quinolin-4-ylméthyl)-3-(4-trifluorométhanesulfanyl-phényl)-imidazolidine-2,4-dione
- la 5-méthyl-1-(6-méthoxy-quinolin-4-ylméthyl)-3-(4-trifluorométhanesulfanyl-phényl)-imidazolidine-2,4-dione
- la 5-méthyl-1-(6-chloro-quinolin-4-ylméthyl)-3-(4-trifluorométhanesulfanyl-phényl)-imidazolidine-2,4-dione
- la 5-méthyl-1-(6-fluoro-quinolin-4-ylméthyl)-3-(4-trifluorométhanesulfanyl-phényl)-imidazolidine-2,4-dione
- la 5-méthy-1-1-(6-amino-quinolin-4-ylméthyl)-3-(4-trifluorométhanesulfanyl-phényl)-imidazolidine-2,4-dione
- la 5-méthyl-1-(6-méthylamino-quinolin-4-ylméthyl)-3-(4-trifluorométhanesulfanyl-phényl)-imidazolidine-2,4-dione
- la 5-méthyl-1-(6-diméthylamino-quinolin-4-ylméthyl)-3-(4-trifluorométhanesulfanyl-phényl)-imidazolidine-2,4-dione
- la 5-méthyl-1-(6-pipéridino-quinolin-4-ylméthyl)-3-(4-trifluorométhanesulfanyl-phényl)-imidazolidine-2,4-dione
- la 5-méthyl-1-(7-méthyl-quinolin-4-ylméthyl)-3-(4-trifluorométhanesulfanyl-phényl)-imidazolidine-2,4-dione
- la 5-méthyl-1-(7-méthoxy-quinolin-4-ylméthyl)-3-(4-trifluorométhanesulfanyl-phényl)-imidazolidine-2,4-dione
- la 5-méthyl-1-(7-chloro-quinolin-4-ylméthyl)-3-(4-trifluorométhanesulfanyl-phényl)-imidazolidine-2,4-dione
- la 5-méthyl-1-(7-fluoro-quinolin-4-ylméthyl)-3-(4-trifluorométhanesulfanyl-phényl)-imidazolidine-2,4-dione
- la 5-méthyl-1-(7-amino-quinolin-4-ylméthyl)-3-(4-trifluorométhanesulfanyl-phényl)-imidazolidine-2,4-dione
- la 5-méthyl-1-(7-méthylamino-quinolin-4-ylméthyl)-3-(4-trifluorométhanesulfanyl-phényl)-imidazolidine-2,4-dione
- la 5-méthyl-1-(7-diméthylamino-quinolin-4-ylméthyl)-3-(4-trifluorométhanesulfanyl-phényl)-imidazolidine-2,4-dione
- la 5-méthyl-1-(7-pipéridino-quinolin-4-ylméthyl)-3-(4-trifluorométhanesulfanyl-phényl)-imidazolidine-2,4-dione
- la 5-méthyl-1-(8-méthyl-quinolin-4-ylméthyl)-3-(4-trifluorométhanesulfanyl-phényl)-imidazolidine-2,4-dione
- la 5-méthyl-1-(8-méthoxy-quinolin-4-ylméthyl)-3-(4-trifluorométhanesulfanyl-phényl)-imidazolidine-2,4-dione
- la 5-méthyl-1-(8-chloro-quinolin-4-ylméthyl)-3-(4-trifluorométhanesulfanyl-phényl)-imidazolidine-2,4-dione
- la 5-méthyl-1-(8-fluoro-quinolin-4-ylméthyl)-3-(4-trifluorométhanesulfanyl-phényl)-imidazolidine-2,4-dione
- la 5-méthyl-1-(8-amino-quinolin-4-ylméthyl)-3-(4-trifluorométhanesulfanyl-phényl)-imidazolidine-2,4-dione
- la 5-méthyl-1-(8-méthylamino-quinolin-4-ylméthyl)-3-(4-trifluorométhanesulfany-1-phényl)-imidazolidine-2,4-dione
- la 5-méthyl-1-(8-diméthylamino-quinolin-4-ylméthyl)-3-(4-trifluorométhanesulfanyl-phényl)-imidazolidine-2,4-dione
- la 5-méthyl-1-(8-pipéridino-quinolin-4-ylméthyl)-3-(4-trifluorométhanesulfanyl-phényl)-imidazolidine-2,4-dione
- la 1-(2-méthyl-quinolin-4-ylméthyl)-3-(4-trifluorométhanesulfanyl-phényl)-imidazolidine-2,4-dione
- la 1-(2-méthoxy-quinolin-4-ylméthyl)-3-(4-trifluorométhanesulfanyl-phényl)-imidazolidine-2,4-dione
- la 1-(2-chloro-quinolin-4-ylméthyl)-3-(4-trifluorométhanesulfanyl-phényl)-imidazolidine-2,4-dione
- la 1-(2-fluoro-quinolin-4-ylméthyl)-3-(4-trifluorométhanesulfanyl-phényl)-imidazolidine-2,4-dione
- la 1-(2-amino-quinolin-4-ylméthyl)-3-(4-trifluorométhanesulfanyl-phényl)-imidazolidine-2,4-dione
- la 1-(2-méthylamino-quinolin-4-ylméthyl)-3-(4-trifluorométhanesulfanyl-phényl)-imidazolidine-2,4-dione
- la 1-(2-diméthylamino-quinolin-4-ylméthyl)-3-(4-trifluorométhanesulfanyl-phényl)-imidazolidine-2,4-dione
- la 1-(2-pipéridino-quinolin-4-ylméthyl)-3-(4-trifluorométhanesulfanyl-phényl)-imidazolidine-2,4-dione
- la 1-(3-méthyl-quinolin-4-ylméthyl)-3-(4-trifluorométhanesulfanyl-phényl)-imidazolidine-2,4-dione
- la 1-(3-méthoxy-quinolin-4-ylméthyl)-3-(4-trifluorométhanesulfanyl-phényl)-imidazolidine-2,4-dione
- la 1-(3-chloro-quinolin-4-ylméthyl)-3-(4-trifluorométhanesulfanyl-phényl)-imidazolidine-2,4-dione
- la 1-(3-fluoro-quinolin-4-ylméthyl)-3-(4-trifluorométhanesulfanyl-phényl)-imidazolidine-2,4-dione
- la 1-(3-amino-quinolin-4-ylméthyl)-3-(4-trifluorométhanesulfanyl-phényl)-imidazolidine-2,4-dione
- la 1-(3-méthylamino-quinolin-4-ylméthyl)-3-(4-trifluorométhanesulfanyl-phényl)-imidazolidine-2,4-dione
- la 1-(3-diméthylamino-quinolin-4-ylméthyl)-3-(4-trifluorométhanesulfanyl-phényl)-imidazolidine-2,4-dione
- la 1-(3-pipéridino-quinolin-4-ylméthyl)-3-(4-trifluorométhanesulfanyl-phényl)-imidazolidine-2,4-dione
- la 1-(5-méthyl-quinolin-4-ylméthyl)-3-(4-trifluorométhanesulfanyl-phényl)-imidazolidine-2,4-dione
- la 1-(5-méthoxy-quinolin-4-ylméthyl)-3-(4-trifluorométhanesulfanyl-phényl)-imidazolidine-2,4-dione
- la 1-(5-chloro-quinolin-4-ylméthyl)-3-(4-trifluorométhanésulfanyl-phényl)-imidazolidine-2,4-dione
- la 1-(5-fluoro-quinolin-4-ylméthyl)-3-(4-trifluorométhanesulfanyl-phényl)-imidazolidine-2,4-dione
- la 1-(5-amino-quinolin-4-ylméthyl)-3-(4-trifluorométhanésulfanyl-phényl)-imidazolidine-2,4-dione
- la 1-(5-méthylamino-quinolin-4-ylméthyl)-3-(4-trifluorométhanesulfanyl-phényl)-imidazolidine-2,4-dione
- la 1-(5-diméthylamino-quinolin-4-ylméthyl)-3-(4-trifluorométhanesulfanyl-phényl)-imidazolidine-2,4-dione
- la 1-(5-pipéridino-quinolin-4-ylméthyl)-3-(4-trifluorométhanesulfanyl-phényl)-imidazolidine-2,4-dione
- la 1-(6-méthyl-quinolin-4-ylméthyl)-3-(4-trifluorométhanesulfanyl-phényl)-imidazolidine-2,4-dione
- la 1-(6-méthoxy-quinolin-4-ylméthyl)-3-(4-trifluorométhanesulfanyl-phényl)-imidazolidine-2,4-dione
- la 1-(6-chloro-quinolin-4-ylméthyl)-3-(4-trifluorométhanesulfanyl-phényl)-imidazolidine-2,4-dione
- la 1-(6-fluoro-quinolin-4-ylméthyl)-3-(4-trifluorométhanesulfanyl-phényl)-imidazolidine-2,4-dione
- la 1-(6-amino-quinolin-4-ylméthyl)-3-(4-trifluorométhanesulfanyl-phényl)-imidazolidine-2,4-dione
- la 1-(6-méthylamino-quinolin-4-ylméthyl)-3-(4-trifluorométhanesulfanyl-phényl)-imidazolidine-2,4-dione
- la 1-(6-diméthylamino-quinolin-4-ylméthyl)-3-(4-trifluorométhanesulfanyl-phényl)-imidazolidine-2,4-dione
- la 1-(6-pipéridino-quinolin-4-ylméthyl)-3-(4-trifluorométhanesulfanyl-phényl)-imidazolidine-2,4-dione
- la 1-(7-méthyl-quinolin-4-ylméthyl)-3-(4-trifluorométhanesulfanyl-phényl)-imidazolidine-2,4-dione
- la 1-(7-méthoxy-quinolin-4-ylméthyl)-3-(4-trifluorométhanesulfanyl-phényl)-imidazolidine-2,4-dione
- la 1-(7-chloro-quinolin-4-ylméthyl)-3-(4-trifluorométhanesulfanyl-phényl)-imidazolidine-2,4-dione
- la 1-(7-fluoro-quinolin-4-ylméthyl)-3-(4-trifluorométhanesulfanyl-phényl)-imidazolidine-2,4-dione
- la 1-(7-amino-quinolin-4-ylméthyl)-3-(4-trifluorométhanesulfanyl-phényl)-imidazolidine-2,4-dione
- la 1-(7-méthylamino-quinolin-4-ylméthyl)-3-(4-trifluorométhanesulfanyl-phényl)-imidazolidine-2,4-dione
- la 1-(7-diméthylamino-quinolin-4-ylméthyl)-3-(4-trifluorométhanesulfanyl-phényl)-imidazolidine-2,4-dione
- la 1-(7-pipéridino-quinolin-4-ylméthyl)-3-(4-trifluorométhanesulfanyl-phényl)-imidazolidine-2,4-dione
- la 1-(8-méthyl-quinolin-4-ylméthyl)-3-(4-trifluorométhanesulfanyl-phényl)-imidazolidine-2,4-dione
- la 1-(8-méthoxy-quinolin-4-ylméthyl)-3-(4-trifluorométhanesulfanyl-phényl)-imidazolidine-2,4-dione
- la 1-(8-chloro-quinolin-4-ylméthyl)-3-(4-trifluorométhanesulfanyl-phényl)-imidazolidine-2,4-dione
- la 1-(8-fluoro-quinolin-4-ylméthyl)-3-(4-trifluorométhanesulfanyl-phényl)-imidazolidine-2,4-dione
- la 1-(8-amino-quinolin-4-ylméthyl)-3-(4-trifluorométhanesulfanyl-phényl)-imidazolidine-2,4-dione
- la 1-(8-méthylamino-quinolin-4-ylméthyl)-3-(4-trifluorométhanesulfanyl-phényl)-imidazolidine-2,4-dione
- la 1-(8-diméthylamino-quinolin-4-ylméthyl)-3-(4-trifluorométhanesulfanyl-phényl)-imidazolidine-2,4-dione
- la 1-(8-pipéridino-quinolin-4-ylméthyl)-3-(4-trifluorométhanesulfanyl-phényl)-imidazolidine-2,4-dione.
- la 5,5-diméthyl-1-(2-méthyl-quinolin-4-ylméthyl)-3-(4-trifluorométhanesulfonyl-phényl)-imidazolidine-2,4-dione
- la 5,5-diméthyl-1-(2-méthoxy-quinolin-4-ylméthyl)-3-(4-trifluorométhanesulfonyl-phényl)-imidazolidine-2,4-dione
- la 5,5-diméthyl-1-(2-chloro-quinolin-4-ylméthyl)-3,-(4-trifluorométhanesulfonyl-phényl)-imidazolidine-2,4-dione
- la 5,5-diméthyl-1-(2-fluoro-quinolin-4-ylméthyl)-3-(4-trifluorométhanssulfonyl-phényl) -imidazolidine-2,4-dione
- la 5,5-diméthyl-1-(2-amino-quinolin-4-ylméthyl)-3-(4-trifluorométhanesulfonyl-phényl)-imidazolidine-2,4-dione
- la 5,5-diméthyl-1-(2-méthylamino-quinolin-4-ylméthyl)-3-(4-trifluorométhanesulfonyl-phényl)-imidazolidine-2,4-dione
- la 5,5-diméthyl-1-(2-diméthylamino-quinolin-4-ylméthyl)-3-(4-trifluorométhanesulfonyl-phényl)-imidazolidine-2,4-dione
- la 5,5-diméthyl-1-(2-pipéridino-quinolin-4-ylméthyl)-3-(4-trifluorométhanesulfonyl-phényl)-imidazolidine-2,4-dione
- la 5,5-diméthyl-1-(3-méthyl-quinolin-4-ylméthyl)-3-(4-trifluorométhanesulfonyl-phényl)-imidazolidine-2,4-dione
- la 5,5-diméthyl-1-(3-méthoxy-quinolin-4-ylméthyl)-3-(4-trifluorométhanesulfonyl-phényl)-imidazolidine-2,4-dione
- la 5,5-diméthyl-1-(3-chloro-quinolin-4-ylméthyl)-3-(4-trifluorométhanesulfonyl-phényl)-imidazolidine-2,4-dione
- la 5,5-diméthyl-1-(3-fluoro-quinolin-4-ylméthyl)-3-(4-trifluorométhanesulfonyl-phényl)-imidazolidine-2,4-dione
- la 5,5-diméthyl-1-(3-amino-quinolin-4-ylméthyl)-3-(4-trifluorométhanesulfonyl-phényl)-imidazolidine-2,4-dione
- la 5,5-diméthyl-1-(3-méthylamino-quinolin-4-ylméthyl)-3-(4-trifluorométhanesulfonyl-phényl)-imidazolidine-2,4-dione
- la 5,5-diméthyl-1-(3-diméthylamino-quinolin-4-ylméthyl)-3-(4-trifluorométhanesulfonyl-phényl)-imidazolidine-2,4-dione
- la 5,5-diméthyl-1-(3-pipéridino-quinolin-4-ylméthyl)-3-(4-trifluorométhanesulfonyl-phényl)-imidazolidine-2,4-dione
- la 5,5-diméthyl-1-(5-mêthyl-quinolin-4-ylméthyl)-3-(4-trifluorométhanesulfonyl-phényl)-imidazolidine-2,4-dione
- la 5,5-diméthyl-1-(5-méthoxy-quinolin-4-ylméthyl)-3-(4-trifluorométhanesulfonyl-phényl)-imidazolidine-2,4-dione
- la 5,5-diméthyl-1-(5-chloro-quinolin-4-ylméthyl)-3-(4-trifluorométhanesulfonyl-phényl)-imidazolidine-2,4-dione
- la 5,5-diméthyl-1-(5-fluoro-quinolin-4-ylméthyl)-3-(4-trifluorométhanesulfonyl-phényl)-imidazolidine-2,4-dione
- la 5,5-diméthyl-1-(5-amino-quinolin-4-ylméthyl)-3-(4-trifluorométhanesulfonyl-phényl)-imidazolidine-2,4-dione
- la 5,5-diméthyl-1-(5-méthylamino-quinolin-4-ylméthyl)-3-(4-trifluorométhanesulfonyl-phényl)-imidazolidine-2,4-dione
- la 5,5-diméthyl-1-(5-diméthylamino-quinolin-4-ylméthyl)-3-(4-trifluorométhanesulfonyl-phényl)-imidazolidine-2,4-dione
- la 5,5-diméthyl-1-(5-pipéridino-quinolin-4-ylméthyl)-3-(4-trifluorométhanesulfonyl-phényl)-imidazolidine-2,4-dione
- la 5,5-diméthyl-1-(6-méthyl-quinolin-4-ylméthyl)-3-(4-trifluorométhanesulfonyl-phényl)-imidazolidine-2,4-dione
- la 5,5-diméthyl-1-(6-méthoxy-quinolin-4-ylméthyl)-3-(4-trifluorométhanesulfonyl-phényl)-imidazolidine-2,4-dione
- la 5,5-diméthyl-1-(6-chloro-quinolin-4-ylméthyl)-3-(4-trifluorométhanesulfonyl-phényl)-imidazolidine-2,4-dione
- la 5,5-diméthyl-1-(6-fluoro-quinolin-4-ylméthyl)-3-(4-trifluorométhanesulfonyl-phényl)-imidazolidine-2,4-dione
- la 5,5-diméthyl-1-(6-amino-quinolin-4-ylméthyl)-3-(4-trifluorométhanesulfonyl-phényl)-imidazolidine-2,4-dione
- la 5,5-diméthyl-1-(6-méthylamino-quinolin-4-ylméthyl)-3-(4-trifluorométhanesulfonyl-phényl)-imidazolidine-2,4-dione
- la 5,5-diméthyl-1-(6-diméthylamino-quinolin-4-ylméthyl)-3-(4-trifluorométhanesulfonyl-phényl)-imidazolidine-2,4-dione
- la 5,5-diméthyl-1-(6-pipéridino-quinolin-4-ylméthyl)-3-(4-trifluorométhanesulfonyl-phényl)-imidazolidine-2,4-dione
- la 5,5-diméthyl-1-(7-méthyl-quinolin-4-ylméthyl)-3-(4-trifluorométhanesulfonyl-phényl)-imidazolidine-2,4-dione
- la 5,5-diméthyl-1-(7-méthoxy-quinolin-4-ylméthyl)-3-(4-trifluorométhanesulfonyl-phényl)-imidazolidine-2,4-dione
- la 5,5-diméthyl-1-(7-chloro-quinolin-4-ylméthyl)-3-(4-trifluorométhanesulfonyl-phényl)-imidazolidine-2,4-dione
- la 5,5-diméthyl-1-(7-fluoro-quinolin-4-ylméthyl)-3-(4-trifluorométhanesulfonyl-phényl)-imidazolidine-2,4-dione
- la 5,5-diméthyl-1-(7-amino-quinolin-4-ylméthyl)-3-(4-trifluorométhanesulfonyl-phényl)-imidazolidine-2,4-dione
- la 5,5-diméthyl-1-(7-méthylamino-quinolin-4-ylméthyl)-3-(4-trifluorométhanesulfonyl-phényl)-imidazolidine-2,4-dione
- la 5,5-diméthyl-1-(7-diméthylamino-quinolin-4-ylméthyl)-3-(4-trifluorométhanesulfonyl-phényl)-imidazolidine-2,4-dione
- la 5,5-diméthyl-1-(7-pipéridino-quinolin-4-ylméthyl)-3-(4-trifluorométhanesulfonyl-phényl)-imidazolidine-2,4-dione
- la 5,5-diméthyl-1-(8-méthyl-quinolin-4-ylméthyl)-3-(4-trifluorométhanesulfonyl-phényl)-imidazolidine-2,4-dione
- la 5,5-diméthyl-1-(8-méthoxy-quinolin-4-ylméthyl)-3-(4-trifluorométhanesulfonyl-phényl)-imidazolidine-2,4-dione
- la 5,5-diméthyl-1-(8-chloro-quinolin-4-ylméthyl)-3-(4-trifluorométhanesulfonyl-phényl)-imidazolidine-2,4-dione
- la 5,5-diméthyl-1-(8-fluoro-quinolin-4-ylméthyl)-3-(4-trifluorométhanesulfonyl-phényl)-imidazolidine-2,4-dione
- la 5,5-diméthyl-1-(8-amino-quinolin-4-ylméthyl)-3-(4-trifluorométhanesulfonyl-phényl)-imidazolidine-2,4-dione
- la 5,5-diméthyl-1-(8-méthylamino-quinolin-4-ylméthyl)-3-(4-trifluorométhanesulfonyl-phényl)-imidazolidine-2,4-dione
- la 5,5-diméthyl-1-(8-diméthylamino-quinolin-4-ylméthyl)-3-(4-trifluorométhanesulfonyl-phényl)-imidazolidine-2,4-dione
- la 5,5-diméthyl-1-(8-pipéridino-quinolin-4-ylméthyl)-3-(4-trifluorométhanesulfonyl-phényl)-imidazolidine-2,4-dione
- la 4-(2-méthyl-quinolin)-4-ylméthyl-6-(4-trifluorométhanesulfonyl-phén-yl)-4,6-diaza-spiro[2.4]heptane-5,7-dione
- la 4-(2-méthoxy-quinolin)-4-ylméthyl-6-(4-trifluorométhanesulfonyl-phényl)-4,6-diaza-spiro[2.4]heptane-5,7-dione
- la 4-(2-chloro-quinolin)-4-ylméthyl-6-(4-trifluorométhanesulfonyl-phényl)-4,6-diaza-spiro[2.4]heptane-5,7-dione
- la 4-(2-fluoro-quinolin)-4-ylméthyl-6-(4-trifluorométhanesulfonyl-phényl)-4,6-diaza-spiro[2.4]heptane-5,7-dione
- la 4-(2-amino-quinolin)-4-ylméthyl-6-(4-trifluoro-méthanesulfonyl-phényl)-4,6-diaza-spiro[2.4]heptane-5,7-dione
- la 4-(2-méthylamino-quinolin)-4-ylméthyl-6-(4-trifluorométhanesulfonyl-phényl)-4,6-diaza-spiro[2.4] heptane-5,7-dione
- la 4-(2-diméthylamino-quinolin)-4-ylméthyl-6-(4-trifluorométhanesulfonyl-phényl)-4,6-diaza-spiro[2.4] heptane-5,7-dione
- la 4-(2-pipéridino-quinolin)-4-ylméthyl-6-(4-trifluorométhanesulfonyl-phényl)-4,6-diaza-spiro[2.4]heptane-5,7-dione
- la 4-(3-méthyl-quinolin)-4-ylméthyl-6-(4-trifluorométhanesulfonyl-phényl)-4,6-diaza-spiro[2.4]heptane-5,7-dione
- la 4-(3-méthoxy-quinolin)-4-ylméthyl-6-(4-trifluorométhanesulfonyl-phényl)-4,6-diaza-spiro[2.4]heptane-5,7-dione
- la 4-(3-chloro-quinolin)-4-ylméthyl-6-(4-trifluorométhanesulfonyl-phényl)-4,6-diaza-spiro[2.4]heptane-5,7-dione
- la 4-(3-fluoro-quinolin)-4-ylméthyl-6-(4-trifluorométhanesulfonyl-phényl)-4,6-diaza-spiro[2.4]heptane-5,7-dione
- la 4-(3-amino-quinolin)-4-ylméthyl-6-(4-trifluorométhanesulfonyl-phény-1)-4,6-diaza-spiro[2.4]heptane-5,7-dione
- la 4-(3-méthylamino-quinolin)-4-ylméthyl-6-(4-trifluorométhanesulfonyl-phényl)-4,6-diaza-spiro[2.4] heptane-5,7-dione
- la 4-(3-diméthylamino-quinolin)-4-ylméthyl-6-(4-trifluorométhanesulfonyl-phényl)-4,6-diaza-spiro[2.4] heptane-5,7-dione
- la 4-(3-pipéridino-quinolin)-4-ylméthyl-6-(4-trifluorométhanesulfonyl-phényl)-4,6-diaza-spiro[2.4]heptane-5,7-dione
- la 4-(5-méthyl-quinolin)-4-ylméthyl-6-(4-trifluorométhanesulfonyl-phényl)-4,6-diaza-spiro[2.4]heptane-5,7-dione
- la 4-(5-méthoxy-quinolin)-4-ylméthyl-6-(4-trifluorométhanesulfonyl-phényl)-4,6-diaza-spiro[2.4]heptane-5,7-dione
- la 4-(5-chloro-quinolin)-4-ylméthyl-6-(4-trifluorométhanesulfonyl-phényl)-4,6-diaza-spiro[2.4]heptane-5,7-dione
- la 4-(5-fluoro-quinolin)-4-ylméthyl-6-(4-trifluorométhanesulfonyl-phényl)-4,6-diaza-spiro[2.4]heptane-5,7-diode
- la 4-(5-amino-qµinolin)-4-ylméthyl-6-(4-trifluorométhanesulfonyl-phényl)-4,6-diaza-spiro[2.4]heptane-5,7-dione
- la 4-(5-méthylamino-quinolin)-4-ylméthyl-6-(4-trifluorométhanesulfonyl-phényl)-4,6-diaza-spiro[2.4] heptane-5,7-dione
- la 4-(5-diméthylamino-quinolin)-4-ylméthyl-6-(4-trifluorométhanesulfonyl-phényl)-4,6-diaza-spiro[2.4] heptane-5,7-dione
- la 4-(5-pipéridino-quinolin)-4-ylméthyl-6-(4-trifluorométhanesulfonyl-phényl)-4,6-diaza-spiro[2.4]heptane-5,7-dione
- la 4-(6-méthyl-quinolin)-4-ylméthyl-6-(4-trifluorométhanesulfony-1-phényl)-4,6-diaza-spiro[2.4]heptane-5,7-dione
- la 4-(6-méthoxy-quinolin)-4-ylméthyl-6-(4-trifluorométhanesulfonyl-phényl)-4,6-diaza-spiro[2.4]heptane-5,7-dione
- la 4-(6-chloro-quinolin)-4-ylméthyl-6-(4-trifluorométhanesulfonyl-phényl)-4,6-diaza-spiro[2.4]heptane-5,7-dione
- la 4-(6-fluoro-quinolin)-4-ylméthyl-6-(4-trifluorométhanesulfonyl-phényl)-4,6-diaza-spiro[2.4]heptane-5,7-dione
- la 4-(6-amino-quinolin)-4-ylméthyl-6-(4-trifluorométhanesulfonyl-phényl)-4,6-diaza-spiro[2.4]heptane-5,7-dione
- la 4-(6-méthylamino-quinolin)-4-ylméthyl-6-(4-trifluorométhanesulfonyl-phényl)-4,6-diaza-spiro[2.4] heptane-5,7-dione
- la 4-(6-diméthylamino-quinolin)-4-ylméthyl-6-(4-trifluorométhanesulfonyl-phényl)-4,6-diaza-spiro[2.4] heptane-5,7-dione
- la 4-(6-pipéridino-quinolin)-4-ylméthyl-6-(4-trifluorométhanesulfonyl-phényl)-4,6-diaza-spiro[2.4]heptane-5,7-dione
- la 4-(7-méthyl-quinolin)-4-ylméthyl-6-(4-trifluorométhanesulfonyl-phényl)-4,6-diaza-spiro[2.4]heptane-5,7-dione
- la 4-(7-méthoxy-quinolin)-4-ylméthyl-6-(4-trifluorométhanesulfonyl-phényl)-4,6-diaza-spiro[2.4]heptane-5,7-dione
- la 4-(7-chloro-quinolin)-4-ylméthyl-6-(4-trifluorométhanesulfonyl-phényl)-4,6-diaza-spiro[2.4]heptane-5,7-dione
- la 4-(7-fluoro-quinolin)-4-ylméthyl-6-(4-trifluorométhanesulfonyl-phényl)-4,6-diaza-spiro[2.4]heptane-5,7-dione
- la 4-(7-amino-quinolin)-4-ylméthyl-6-(4-trifluorométhanesulfonyl-phény-1)-4,6-diaza-spiro[2.4]heptane-5,7-dione
- la 4-(7-méthylamino-quinolin)-4-ylméthyl-6-(4-trifluorométhanesulfonyl-phényl)-4,6-diaza-spiro[2.4] heptane-5,7-dione
- la 4-(7-diméthylamino-quinolin)-4-ylméthyl-6-(4-trifluorométhanesulfonyl-phényl)-4,6-diaza-spiro[2.4] heptane-5,7-dione
- la 4-(7-pipéridino-quinolin)-4-ylméthyl-6-(4-trifluorométhanesulfonyl-phényl)-4,6-diaza-spiro[2.4]heptane-5,7-dione
- la 4-(8-méthyl-quinolin)-4-ylméthyl-6-(4-trifluorométhanesulfonyl-phényl)-4,6-diaza-spiro[2.4]heptane-5,7-dione
- la 4-(8-méthoxy-quinolin)-4-ylméthyl-6-(4-trifluorométhanesulfonyl-phényl)-4,6-diaza-spiro[2.4]heptane-5,7-dione
- la 4-(8-chloro-quinolin)-4-ylméthyl-6-(4-trifluorométhanesulfonyl-phényl)-4,6-diaza-spiro[2.4]heptane-5,7-dione
- la 4-(8-fluoro-quinolin)-4-ylméthyl-6-(4-trifluorométhanesulfonyl-phényl)-4,6=diaza-spiro[2.4]heptane-5,7-dione
- la 4-(8-amino-quinolin)-4-ylméthyl-6-(4-trifluorométhanesulfonyl-phényl)-4,6-diaza-spiro[2.4]heptane-5,7-dione
- la 4-(8-méthylamino-quinolin)-4-ylméthyl-6-(4-trifluorométhanesulfonyl-phényl)-4,6-diaza-spiro[2.4] heptane-5,7-dione
- la 4-(8-diméthylamino-quinolin)-4-ylméthyl-6-(4-trifluorométhanesulfonyl-phényl)-4,6-diaza-spiro[2.4]heptane-5,7-dione
- la 4-(8-pipéridino-quinolin)-4-ylméthyl-6-(4-trifluorométhanesulfonyl-phényl)-4,6-diaza-spiro[2.4]heptane-5,7-dione
- la 5-méthul-1-(2-méthyl-quinolin-4-ylméthyl)-3-(4-trifluorométhanesulfonyl-phényl)-imidazolidine-2,4-dione
- la 5-méthyl-1-(2-méthoxy-quinolin-4-ylméthyl)-3-(4-trifluorométhanesulfonyl-phényl)-imidazolidine-2,4-dione
- la 5-méthyl-1-(2-chloro-quinolin-4-ylméthyl)-3-(4-trifluorométhanesulfonyl-phényl)-imidazolidine-2,4-dione
- la 5-méthyl-1-(2-fluoro-quinolin-4-ylméthyl)-3-(4-trifluorométhanesulfonyl-phényl)-imidazolidine-2,4-dione
- la 5-méthyl-1-(2-amino-quinolin-4-ylméthyl)-3-(4-trifluorométhanesulfonyl-phényl)-imidazolidine-2,4-diorie
- la 5-méthyl-1-(2-méthylamino-quinolin-4-ylméthyl)-3-(4-trifluorométhanesulfonyl-phényl)-imidazolidine-2,4-dione
- la 5-méthyl-1-(2-diméthylamino-quinolin-4-ylméthyl)-3-(4-trifluorométhanesulfonyl-phényl)-imidazolidine-2,4-dione
- la 5-méthyl-1-(2-pipéridino-quinolin-4-ylméthyl)-3-(4-trifluorométhanesulfonyl-phényl)-imidazolidine-2,4-dione
- la 5-méthyl-1-(3-méthyl-quinolin-4-ylméthyl)-3-(4-trifluorométhanesulfonyl-phényl)-imidazolidine-2,4-dione
- la 5-méthyl-1-(3-méthoxy-quinolin-4-ylméthyl)-3-(4-trifluorométhanesulfonyl-phényl)-imidazolidine-2,4-dione
- la 5-méthyl-1-(3-chloro-quinolin-4-ylméthyl)-3-(4-trifluorométhanesulfonyl-phényl)-imidazolidine-2,4-dione
- la 5-méthyl-1-(3-fluoro-quinolin-4-ylméthyl)-3-(4-trifluorométhanesulfonyl-phényl)-imidazolidine-2,4-dione
- la 5-méthyl-1-(3-amino-quinolin-4-ylméthyl)-3-(4-trifluorométhanesulfonyl-phényl)-imidazolidine-2,4-dione
- la 5-méthyl-1-(3-méthylamino-quinolin-4-ylméthyl)-3-(4-trifluorométhanesulfonyl-phényl)-imidazolidine-2,4-dione
- la 5-méthyl-1-(3-diméthylamino-quinolin-4-ylméthyl)-3-(4-trifluorométhanesulfonyl-phényl)-imidazolidine-2,4-dione
- la 5-méthyl-1-(3-pipéridino-quinolin-4-ylméthyl)-3-(4-trifluorométhanesulfonyl-phényl)-imidazolidine-2,4-dione
- la 5-méthyl-1-(5-méthyl-quinolin-4-ylméthyl)-3-(4-trifluorométhanesulfonyl-phényl)-imidazolidine-2,4-dione
- la 5-méthyl-1-(5-méthoxy-quinolin-4-ylméthyl)-3-(4-trifluorométhanesulfon-yl-phényl)-imidazolidine-2,4-dione
- la 5-méthyl-1-(5-chloro-quinolin-4-ylméthyl)-3-(4-trifluorométhanesulfonyl-phényl)-imidazolidine-2,4-dione
- la 5-méthyl-1-(5-fluoro-quinolin-4-ylméthyl)-3-(4-trifluorométhanesulfonyl-phényl)-imidazolidine-2,4-dione
- la 5-méthyl-1-(5-amino-quinolin-4-ylméthyl)-3-(4-trifluorométhanesulfonyl-phényl)-imidazolidine-2,4-dione
- la 5-méthyl-1-(5-méthylamino-quinolin-4-ylméthyl)-3-(4-trifluorométhanesulfonyl-phényl)-imidazolidine-2,4-dione
- la 5-méthyl-1-(5-diméthylamino-quinolin-4-ylméthyl)-3-(4-trifluorométhanesulfonyl-phényl)-imidazolidine-2,4-dione
- la 5-méthyl-1-(5-pipéridino-quinolin-4-ylméthyl)-3-(4-trifluorométhanesulfonyl-phényl)-imidazolidine-2,4-dione
- la 5-méthyl-1-(6-méthyl-quinolin-4-ylméthyl)-3-(4-trifluorométhanesulfonyl-phényl)-imidazolidine-2,4-dione
- la 5-méthyl-1-(6-méthoxy-quinolin-4-ylméthyl)-3-(4-trifluorométhanesulfonyl-phényl)-imidazolidine-2,4-dione
- la 5-méthyl-1-(6-chloro-quinolin-4-ylméthyl)-3-(4-trifluorométhanesulfonyl-phényl)-imidazolidine-2,4-dione
- la 5-méthyl-1-(6-fluoro-quinolin-4-ylméthyl)-3-(4-trifluorométhanesulfonyl-phényl)-imidazolidine-2,4-dione
- la 5-méthyl-1-(6-amino-quinolin-4-ylméthyl)-3-(4-trifluorométhanesulfonyl-phényl)-imidazolidine-2,4-dione
- la 5-méthyl-1-(6-méthylamino-quinolin-4-ylméthyl)-3-(4-trifluorométhanesulfonyl-phényl)-imidazolidine-2,4-dione
- la 5-méthyl-1-(6-diméthylamino-quinolin-4-ylméthyl)-3-(4-trifluorométhanesulfonyl-phényl)-imidazolidine-2,4-dione
- la 5-méthyl-1-(6-pipéridino-quiriolin-4-ylméthyl)-3-(4-trifluorométhanesulfonyl-phényl)-imidazolidine-2,4-dione
- la 5-méthyl-1-(7-méthyl-quinolin-4-ylméthyl)-3-(4-trifluorométhanesulfonyl-phényl)-imidazolidine-2,4-dione
- la 5-méthyl-1-(7-méthoxy-guinolin-4-ylméthyl)-3-(4-trifluorométhanesulfonyl-phényl)-imidazolidine-2,4-dione
- la 5-méthyl-1-(7-chloro-quinolin-4-ylméthyl)-3-(4-trifluorométhanesulfonyl-phényl)-imidazolidine-2,4-dione
- la 5-méthyl-1-(7-fluoro-quinolin-4-ylméthyl)-3-(4-trifluorométhanesulfonyl-phényl)-imidazolidine-2,4-dione
- la 5-méthyl-1-(7-amino-quinolin-4-ylméthyl)-3-(4-trifluorométhanesulfonyl-phényl)-imidazolidine-2,4-dione
- la 5-méthyl-1-(7-méthylamino-quinolin-4-ylméthyl)-3-(4-trifluorométhanesulfonyl-phényl)-imidazolidine-2,4-dione
- la 5-méthyl-1-(7-diméthylamino-quinolin-4-ylméthyl)-3-(4-trifluorométhanesulfonyl-phényl)-imidazolidine-2,4-dione
- la 5-méthyl-1-(7-pipéridino-quinolin-4-ylméthyl)-3-(4-trifluorométhanesulfonyl-phényl)-imidazolidine-2,4-dione
- la 5-méthyl-1-(8-méthyl-quinolin-4-ylméthyl)-3-(4-trifluorométhanesulfonyl-phényl)-imidazolidine-2,4-dione
- la 5-méthyl-1-(8-méthoxy-quinolin-4-ylméthyl)-3-(4-trifluorométhanesulfonyl-phényl)-imidazolidine-2,4-dione
- la 5-méthyl-1-(8-chloro-quinolin-4-ylméthyl)-3-(4-trifluorométhanesulfonyl-phényl)-imidazolidine-2,4-dione
- la 5-méthyl-1-(8-fluoro-quinolin-4-ylméthyl)-3-(4-trifluorométhanesulfonyl-phényl)-imidazolidine-2,4-dione
- la 5-méthyl-1-(8-amino-quinolin-4-ylméthyl)-3-(4-trifluorométhanesulfonyl-phényl)-imidazolidine-2,4-dione
- la 5-méthyl-1-(8-méthylamino-quinolin-4-ylméthyl)-3-(4-trifluorométhanesulfonyl-phényl)-imidazolidine-2,4-dione
- la 5-méthyl-1-(8-diméthylamino-quinolin-4-ylméthyl)-3-(4-trifluorométhanesulfonyl-phényl)-imidazolidiné-2,4-dione
- la 5-méthyl-1-(8-pipéridino-quinolin-4-ylméthyl)-3-(4-trifluorométhanesulfonyl-phényl)-imidazolidine-2,4-dione
- la 1-(2-méthyl-quinolin-4-ylméthyl)-3-(4-trifluorométhanesulfonyl-phényl)-imidazolidine-2,4-dione
- la 1-(2-méthoxy-quinolin-4-ylméthyl)-3-(4-trifluorométhanesulfonyl-phényl)-imidazolidine-2,4-dione
- la 1-(2-chloro-quinolin-4-ylméthyl)-3-(4-trifluorométhanesulfonyl-phényl)-imidazolidine-2,4-dione
- la 1-(2-fluoro-quinolin-4-ylméthyl)-3-(4-trifluorométhanesulfonyl-phényl)-imidazolidine-2,4-dione
- la 1-(2-amino-quinolin-4-ylméthyl)-3-(4-trifluorométhanesulfonyl-phényl)-imidazolidine-2,4-dione
- la 1-(2-méthylamino-quinolin-4-ylméthyl)-3-(4-trifluorométhanesulfonyl-phényl)-imidazolidine-2,4-dione
- la 1-(2-diméthylamino-quinolin-4-ylméthyl)-3-(4-trifluorométhanesulfonyl-phényl)-imidazolidine-2,4-dione
- la 1-(2-pipéridino-quinolin-4-ylméthyl)-3-(4-trifluorométhanesulfonyl-phényl)-imidazolidine-2,4-dione
- la 1-(3-méthyl-quinolin-4-ylméthyl)-3-(4-trifluorométhanesulfonyl-phényl)-imidazolidine-2,4-dione
- la 1-(3-méthoxy-quinolin-4-ylméthyl)-3-(4-trifluorométhanesulfonyl-phényl)-imidazolidine-2,4-dione
- la 1-(3-chloro-quinolin-4-ylméthyl)-3-(4-trifluorométhanesulfonyl-phényl)-imidazolidine-2,4-dione
- la 1-(3-fluoro-quinolin-4-ylméthyl)-3-(4-trifluorométhanesulfonyl-phényl)-imidazolidine-2,4-dione
- la 1-(3-amino-quinolin-4-ylméthyl)-3-(4-trifluorométhanesulfonyl-phényl)-imidazolidine-2,4-dione
- la 1-(3-méthylamino-quinolin-4-ylméthyl)-3-(4-trifluorométhanesulfonyl-phényl)-imidazolidine-2,4-dione
- la 1-(3-diméthylamino-quinolin-4-ylméthyl)-3-(4-trifluorométhanesulfonyl-phényl)-imidazolidine-2,4-dione
- la 1-(3-pipéridino-quinolin-4-ylméthyl)-3-(4-trifluorométhanesulfonyl-phényl)-imidazolidine-2,4-dione
- la 1-(5-méthyl-quinolin-4-ylméthyl)-3-(4-trifluorométhanesulfonyl-phényl)-imidazolidine-2,4-dione
- la 1-(5-méthoxy-quinolin-4-ylméthyl)-3-(4-trifluorométhanesulfonyl-phényl)-imidazolidine-2,4-dione
- la 1-(5-chloro-quinolin-4-ylméthyl)-3-(4-trifluorométhanesulfonyl-phényl)-imidazolidine-2,4-dione
- la 1-(5-fluoro-quinolin-4-ylméthyl)-3-(4-trifluorométhanesulfonyl-phényl)-imidazolidine-2,4-dione
- la 1-(5-amino-quinolin-4-ylméthyl)-3-(4-trifluorométhanesulfonyl-phényl)-imidazolidine-2,4-dione
- la 1-(5-méthylamino-quinolin-4-ylméthyl)-3-(4-trifluorométhanesulfonyl-phényl)-imidazolidine-2,4-dione
- la 1-(5-diméthylamino-quinolin-4-ylméthyl)-3-(4-trifluorométhanesulfonyl-phényl)-imidazolidine-2,4-dione
- la 1-(5-pipéridino-quinolin-4-ylméthyl)-3-(4-trifluorométhanesulfonyl-phényl)-imidazolidine-2,4-dione
- la 1-(6-méthyl-quinolin-4-ylméthyl)-3-(4-trifluorométhanesulfonyl-phényl)-imidazolidine-2,4-dione
- la 1-(6-méthoxy-quinolin-4-ylméthyl)-3-(4-trifluorométhanesulfonyl-phényl)-imidazolidine-2,4-dione
- la 1-(6-chloro-quinolin-4-ylméthyl)-3-(4-trifluorométhanesulfonyl-phényl)-imidazolidine-2,4-dione
- la 1-(6-fluoro-quinolin-4-ylméthyl)-3-(4-trifluorométhanesulfonyl-phényl)-imidazolidine-2,4-dione
- la 1-(6-amino-quinolin-4-ylméthyl)-3-(4-trifluorométhansulfonyl-phényl)-imidazolidine-2,4-dione
- la 1-(6-méthylamino-quinolin-4-ylméthyl)-3-(4-trifluorométhanesulfonyl-phényl)-imidazolidine-2,4-dione
- la 1-(6-diméthylamino-quinolin-4-ylméthyl)-3-(4-trifluorométhanesulfonyl-phényl)-imidazolidine-2,4-dione
- la 1-(6-pipéridino-quinolin-4-ylméthyl)-3-(4-trifluorométhanesulfonyl-phényl)-imidazolidine-2,4-dione
- la 1-(7-méthyl-quinolin-4-ylméthyl)-3-(4-trifluorométhanesulfonyl-phényl)-imidazolidine-2,4-dione
- la 1-(7-méthoxy-quinolin-4-ylméthyl)-3-(4-trifluorométhanesulfonyl-phényl)-imidazolidine-2,4-dione
- la 1-(7-chloro-quinolin-4-ylméthyl)-3-(4-trifluorométhanesulfonyl-phényl)-imidazolidine-2,4-dione
- la 1-(7-fluoro-quinolin-4-ylméthyl)-3-(4-trifluorométhanesulfonyl-phényl)-imidazolidine-2,4-dione
- la 1-(7-amino-quinolin-4-ylméthyl)-3-(4-trifluorométhanesulfonyl-phényl)-imidazolidine-2,4-dione
- la 1-(7-méthylamino-quinolin-4-ylméthyl)-3-(4-trifluorométhanesulfonyl-phényl)-imidazolidine-2,4-dione
- la 1-(7-diméthylamino-quinolin-4-ylméthyl)-3-(4-trifluorométhanesulfonyl-phényl)-imidazolidine-2,4-dione
- la 1-(7-pipéridino-quinolin-4-ylméthyl)-3-(4-trifluorométhanesulfonyl-phényl) imidazolidine-2,4-dione
- la 1-(8-méthyl-quinolin-4-ylméthyl)-3-(4-trifluorométhanesulfonyl-phényl)-imidazolidine-2,4-dione
- la 1-(8-méthoxy-quinolin-4-ylméthyl)-3-(4-trifluorométhanesulfonyl-phényl)-imidazolidine-2,4-dione
- la 1-(8-chloro-quinolin-4-ylméthyl)-3-(4-trifluorométhanesulfonyl-phényl)-imidazolidine-2,4-dione
- la 1-(8-fluoro-quinolin-4-ylméthyl)-3-(4-trifluorométhanesulfonyl-phényl)-imidazolidine-2,4-dione
- la 1-(8-amino-quinolin-4-ylméthyl)-3-(4-trifluorométhanesulfonyl-phényl)-imidazolidine-2,4-dione
- la 1-(8-méthylamino-quinolin-4-ylméthyl)-3-(4-trifluorométhanesulfonyl-phényl)-imidazolidine-2,4-dione
- la 1-(8-diméthylamino-quinolin-4-ylméthyl)-3-(4-trifluorométhanesulfonyl-phényl)-imidazolidine-2,4-dione
- la 1-(8-pipéridino-quinolin-4-ylméthyl)-3-(4-trifluorométhanesulfonyl-phényl)-imidazolidine-2,4-dione

| Exemples | Structure |
|---|---|
| 264 | 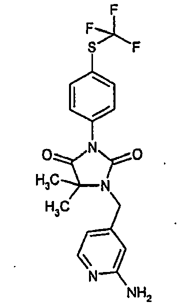 |
| 265 | 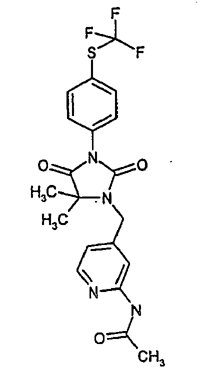 |
| 266 | 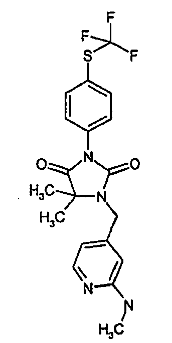 |
| 267 | 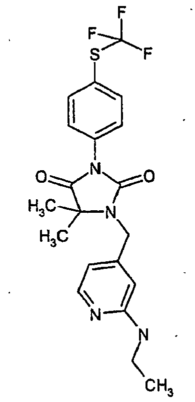 |
| 268 | 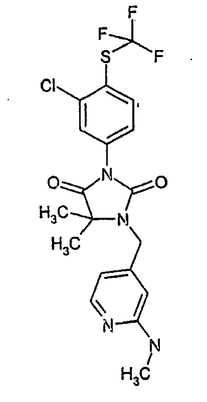 |
| 269 | 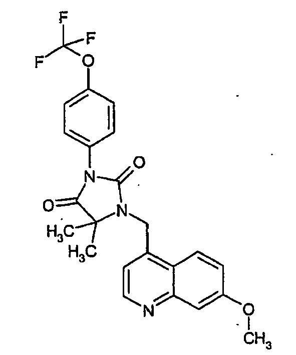 |
| 270 | 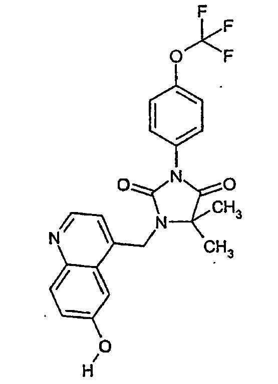 |
| 271 | 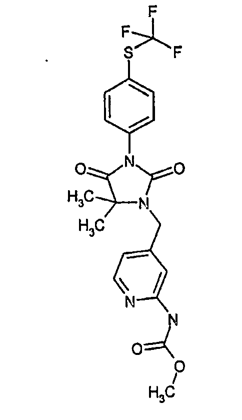 |
| 272 | 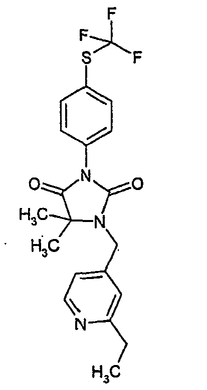 |
| 273 | 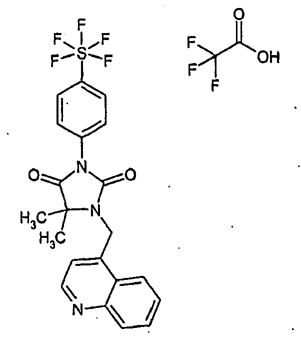 |
| 274 | 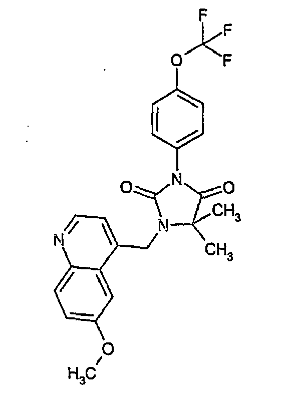 |
| 275 | 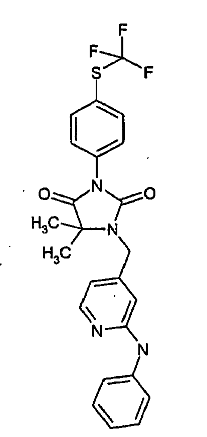 |
| 276 | 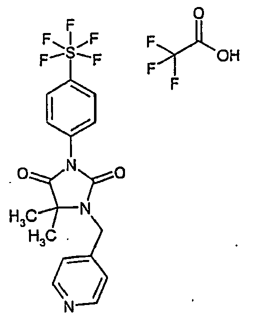 |
| 277 | 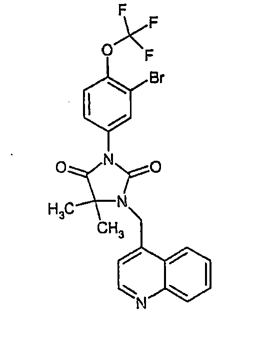 |
| 278 | 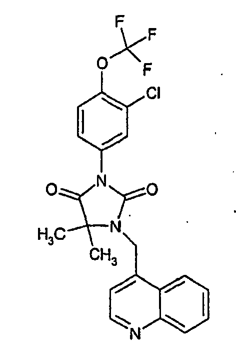 |
| 279 | 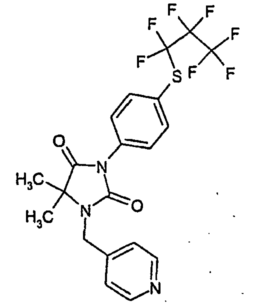 |
| 280 | 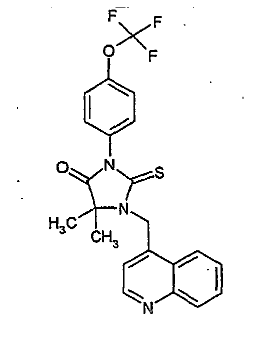 |
| 281 | 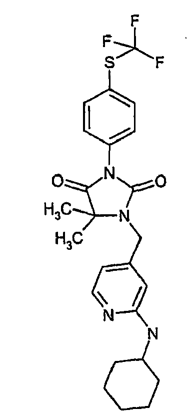 |
| 282 | 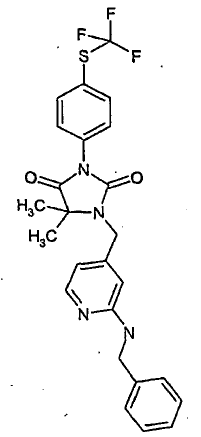 |
| 283 | 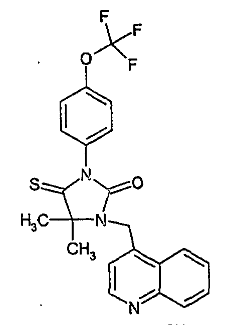 |
| 284 | 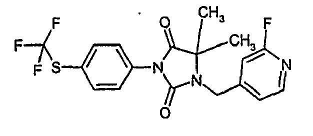 |
| 285 | 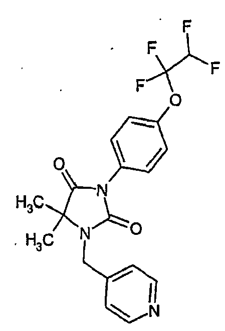 |
| 286 | 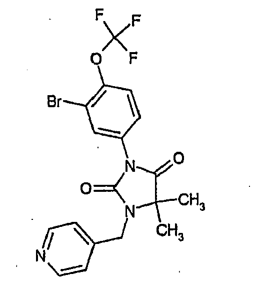 |
| 287 | 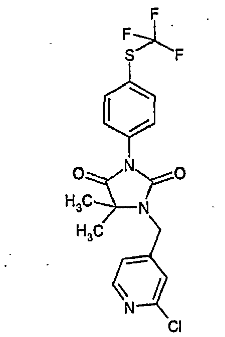 |
| 288 | 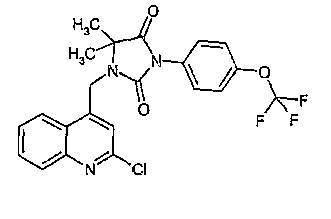 |
| 289 | 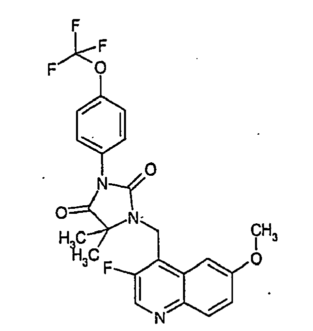 |
| 290 | 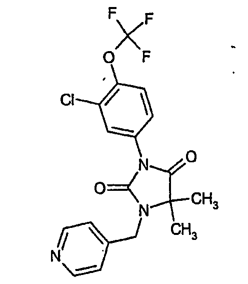 |
| 291 | 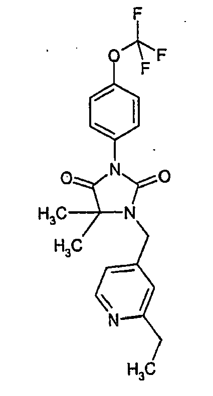 |
| 292 | 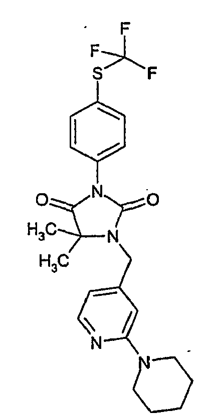 |
| 293 | 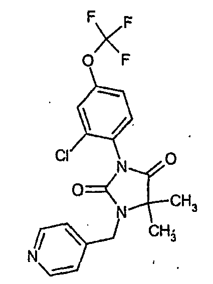 |
| 294 | 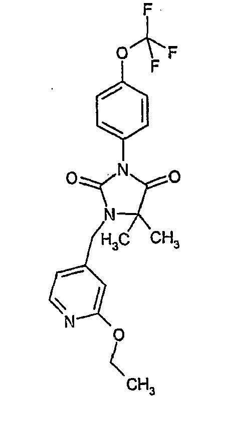 |
| 295 | 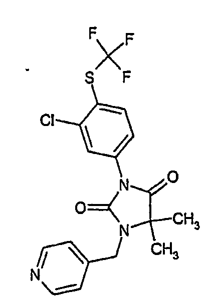 |
| 296 | 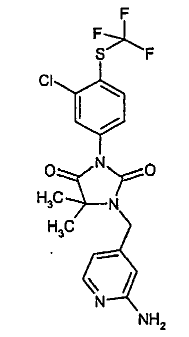 |
| 297 | 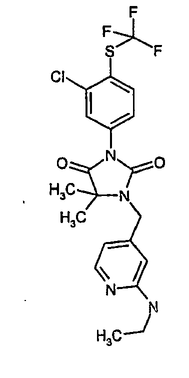 |
| 298 | 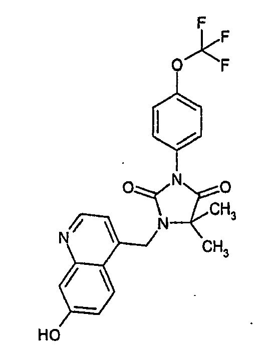 |
| 299 | 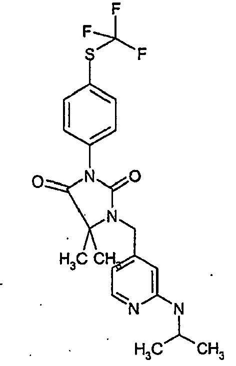 |
| 300 | 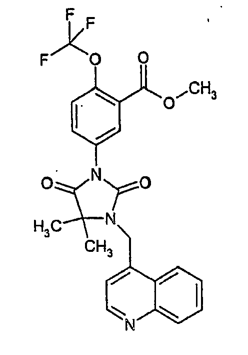 |
| 301 | 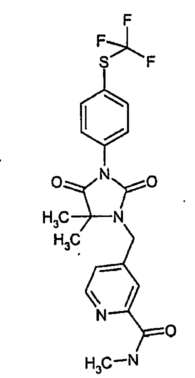 |
| 302 | 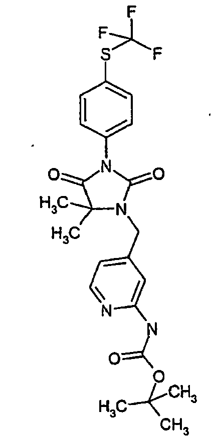 |
| 303 | 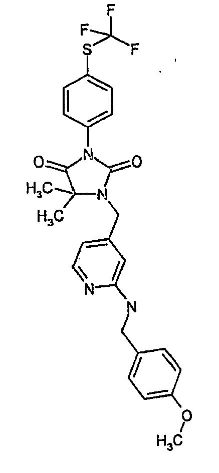 |
| 304 | 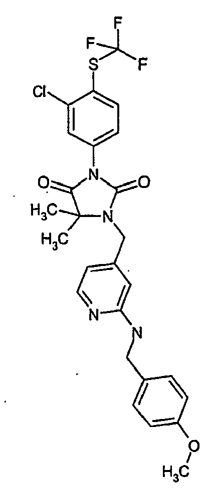 |
| 305 | 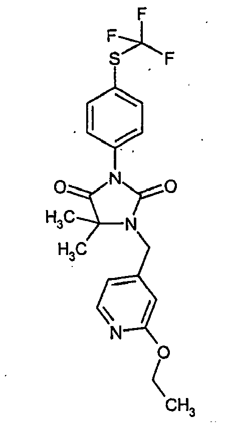 |
| 306 | 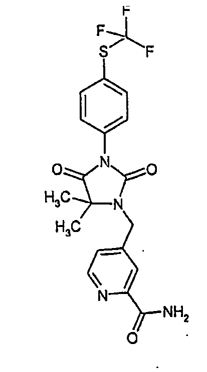 |
| 307 | 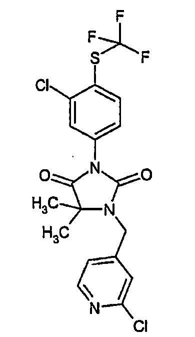 |
| 308 | 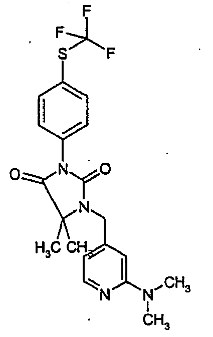 |
| 309 | 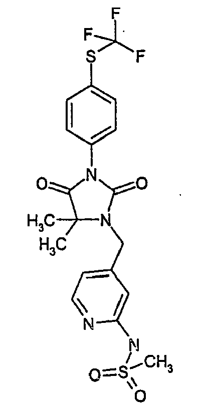 |
| 310 | 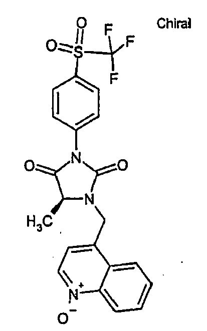 |
| 311 | 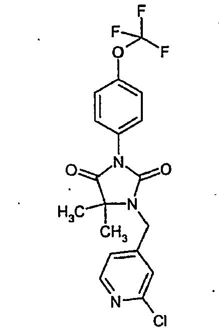 |
| 312 | 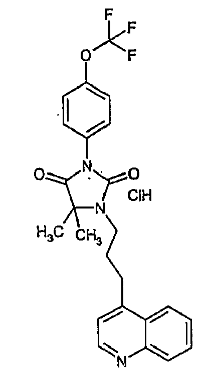 |
| 313 | 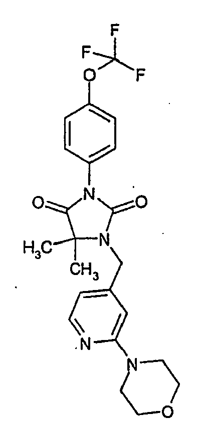 |
| 314 | 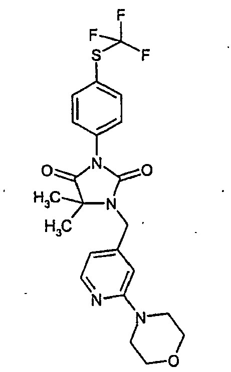 |
| 315 | 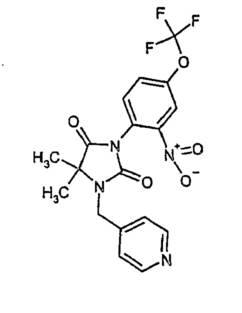 |
| 316 | 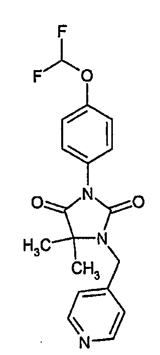 |
| 317 | 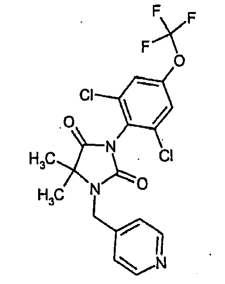 |
| 318 | 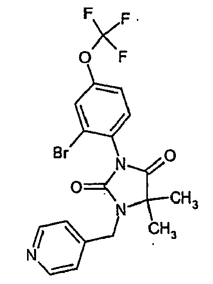 |
| 319 | 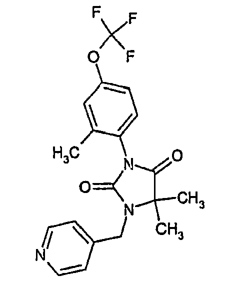 |
| 320 | 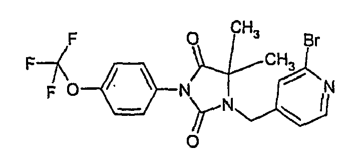 |
| 321 | 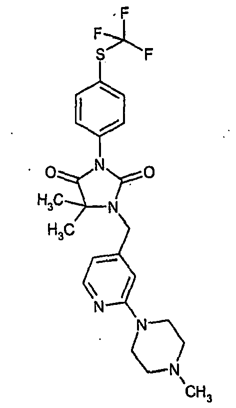 |
| 322 | 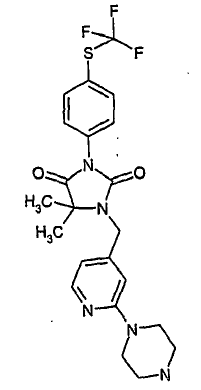 |
| 323 | 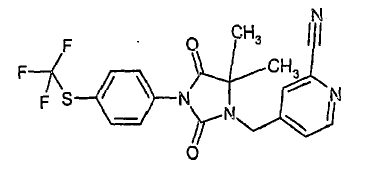 |
| 324 | 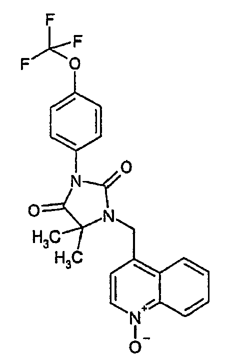 |
| 325 | 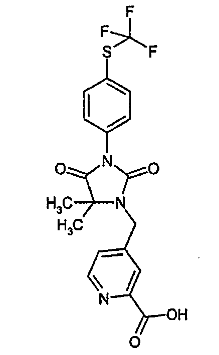 |
| 326 | 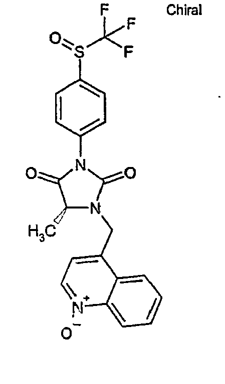 |
| 327 | 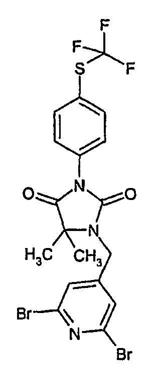 |
| 328 | 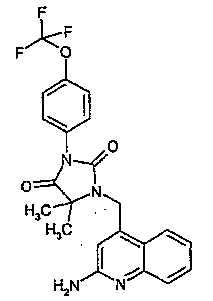 |
| 329 | 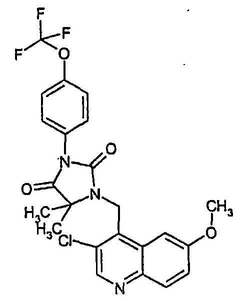 |
| 330 | 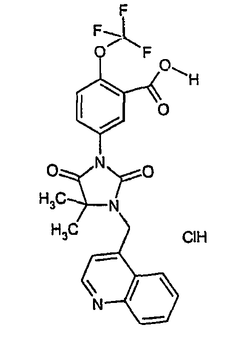 |
| 331 | 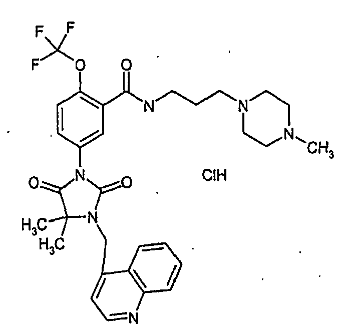 |
| 332 | 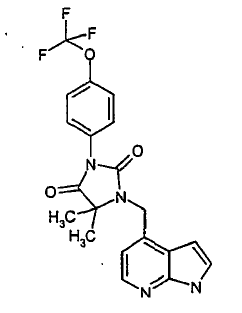 |

Tableau 1 (ex 264 à 332)

## Revendications

1. Produits de formule (I): 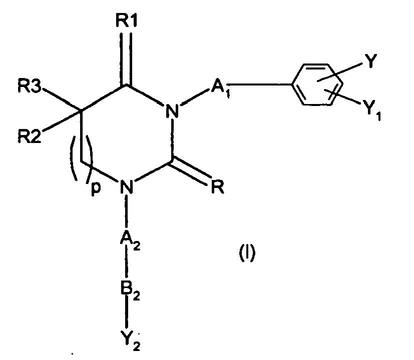 dans laquelle p représente un entier de 0 à 2,
R et R1 identiques ou différents représentent O ou NH,
R2 et R3, identiques ou différents représentent hydrogène, alkyle, alkényle, alkynyle, cycloalkyle, aryle et hétéroaryle éventuellement substitués ou bien R2 et R3 forment ensemble avec l'atome de carbone auxquels ils sont liés un radical carbocyclique ou hétérocyclique, ces radicaux étant constitués de 3 à 10 chaînons et le radical hétérocyclique renfermant un ou plusieurs hétéroatomes choisis parmi O, S, N et NR7, tous ces radicaux étant éventuellement substitués,
A1 représente une simple liaison, un radical alkyle, un radical alkényle ou alkynyle,
Y et Y1 identiques ou différents sont tels que l'un de Y et Y1 est choisi parmi OCF3, -O-CF2-CHF2, -O-CHF2, -O-CH2-CF3,_S(O)nCF3, -S-CF2-CF2-CF3, -S(O)n-Alk, -S-Alk-O-Alk, -S-Alk-OH, -S-Alk-CN, -S-Alk-hétérocycloalkyle (description : morpholino, pyrrolidinyle, pipérazinyle éventuellement substitué par alk), -S02CHF2, -SO2CF2CF3, -SO2NR5R6 et -SF5, avec Alk représente un radical alkyle renfermant de 1 à 4 atomes de carbone, et l'autre de Y et Y1 est choisi parmi les valeurs suivantes : hydrogène, halogène, nitro, NR5R6, carboxy libre ou estérifié et CONR5R6,
ou bien le radical phényle forme avec ses substituants Y et Y1 l'un des radicaux suivants : 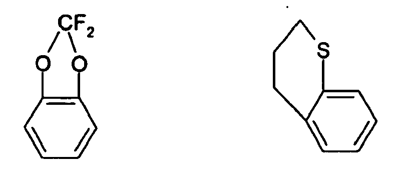 le radical ainsi formé étant éventuellement substitué par un ou plusieurs radicaux alkyle eux-mêmes éventuellement substitués,
R5 et R6 identiques ou différents sont choisis parmi hydrogène, alkyle, alkényle, alkynyle, cycloalkyle, cycloalkényle, hétérocycloalkyle, aryle et hétéroaryle éventuellement substitués ou bien R5 et R6 forment avec l'atome d'azote auxquels ils sont liés un radical hétérocyclique renfermant 3 à 10 chaînons renfermant un ou plusieurs hétéroatomes choisis parmi O, S, N et NR7 éventuellement substitué,
A2, identique ou différent de A1, représente les valeurs de A1 et CO et SO2,
B2 représente un radical hétérocyclique monocyclique ou bicyclique saturé ou insaturé renfermant 1 à plusieurs hétéroatomes identiques ou différents choisis parmi O, S, N et NR7, éventuellement substitués par un ou plusieurs substituants identiques ou différents choisis parmi les valeurs de Y2,
R7 représente un atome d'hydrogène, un radical alkyle, cycloalkyle, phényle, acyle, S(O)2Alk, S(O)2Aryle, S (O) 2hétéroaryle et S (O) 2NR5R6,
Y2 représente hydrogène, halogène, hydroxyle, cyano, alkyle, alcoxy, cycloalkyle, hétérocycloalkyle, aryle, hétéroaryle, -O-alkényle, -O-alkynyle, -O-cycloalkyle, -S(O)n-alkyle, -S(O)n-alkényle, -S(O)n-alkynyle, S(O)n-cycloalkyle, COOR13, -OCOR13, NR5R6, CONR5R6, S(O)n-NR5R6, -NR10-CO-R13,-NR10-SO2-R13, NH-SO2-NR5R6, -NR10-CO-NR5R6, -NR10-CS-NR5R6, -NR10-COOR13, tous ces radicaux étant éventuellement substitués,
tous les radicaux ci-dessus alkyle, alkényle, alkynyle, alcoxy, étant linéaires ou ramifiés et renfermant au plus 6 atomes de carbone,
tous les radicaux ci-dessus cycloalkyle, hétérocycloalkyle renfermant au plus 7 atomes de carbone, tous les radicaux ci-dessus aryle et hétéroaryle renfermant au plus 10 atomes de carbone,
tous les radicaux ci-dessus alkyle, alkényle, alkynyle, alcoxy, cycloalkyle, hétérocycloalkyle, aryle et hétéroaryle, carbocyclique et hétérocycliques ainsi que le cycle formé par R5 et R6 avec l'atome auxquels ils sont liés étant éventuellement substitués par un ou plusieurs radicaux identiques ou différents choisis parmi les atomes d'halogène et les radicaux cyano, hydroxy, alcoxy, CF3, nitro, aryle, hétéroaryle, -C(=O)-OR9, -C(=O)-R8, -NR11R12, -C(=O)-NR11R12, -N(R10)-C(=O)-R8, -N(R10)-C(=O)-OR9, N(R10)-C(=O)-NR11R12, -N(R10)-S(O)n-R8, -S(O)n-R8, -N(R10)-S(O)n-NR11R12 ou -S(O)n-NR11R12, tous les radicaux aryle et hétéroaryle ci-dessus étant de plus éventuellement substitués par un ou plusieurs radicaux choisis parmi les radicaux alkyle, alcoxy et alkylènedioxy,
tous les radicaux cycliques ci-dessus ainsi que le cycle formé par R5 et R6 avec l'atome auxquels ils sont liés étant de plus éventuellement substitués par un ou plusieurs radicaux choisis parmi oxo et thioxo,
n représente un entier de 0 à 2,
R8 représente alkyle, alkényle, cycloalkyle, cycloalkylalkyle, hétérocycloalkyle, hétérocycloalkylalkyle, aryle, arylalkyle, hétéroaryle et hétéroarylalkyle,
R9 représente les valeurs de R8 et hydrogène,
R10 représente hydrogène ou alkyle,
R11 et R12, identiques ou différents, représentent hydrogène, C3-C6 cycloalkyle, C1-C4 alkyle et phényle éventuellement substitués par un ou plusieurs radicaux identiques ou différents choisis parmi les atomes d'halogène
et les radicaux cyano, hydroxy, alcoxy, CF3, nitro, phényle
et carboxy libre, salifié, estérifié ou amidifié,
ou bien R11 et R12 forment avec l'atome d'azote auxquels ils
sont liés un radical cyclique renfermant 5 à 7 chaînons renfermant un ou plusieurs hétéroatomes choisis parmi O, S, N et NR7 et de préférence une amine cyclique, R13, identique ou différent de R5 ou R6, étant choisi parmi les valeurs de R5 ou R6,
étant entendu que les produits de formule (I) sont tels que définis ci-après de a) à d):
a) lorsque p représente l'entier 0, R représente Oxygène, R1 représente Oxygène, A1 représente une simple liaison ou alkyle, Y et Y1 identiques ou différents sont tels que l'un au moins représente OCF3 ou Salk, A2 représente simple liaison ou alkyle et B2 représente un radical hétérocycle éventuellement substitué, alors R2 et R3 ne représentent pas l'un hydrogène et l'autre imidazolylalkyle
b) lorsque p représente l'entier 0, R et R1 représentent Oxygène, A1 représente une simple liaison ou alkyle, Y et Y1 identiques ou différents sont tels que l'un au moins représente OCF3, SOAlk, S(O)2alk ou S02NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
c) lorsque p représente l'entier 0, R et R1 représentent Oxygène, A1 représente une simple liaison ou alkyle, Y et Y1 identiques ou différents sont tels que l'un au moins représente S(O)nAlk, A2 représente une simple liaison et B2 représente un radical hétérocycle aromatique renfermant 5 ou 6 chaînons éventuellement substitué, alors R2 et R3 ne sont pas choisis parmi hydrogène, alkyle, arylalkyle, aryle et hétéroaryle,
d) lorsque p représente un entier de 0 à 2, R et R1 représentent Oxygène, A1 représente une simple liaison, Y et Y1 identiques ou différents sont tels que l'un représente S02Alk ou SO2NH2 et l'autre représente NR5R6, A2 représente une simple liaison ou alkylène et B2 représente un radical hétérocycle renfermant 5 à 10 chaînons éventuellement substitué, alors R2 et R3 ne représentent pas tous deux hydrogène,
étant entendu que les radicaux ci-dessus ont les significations suivantes :
- le terme radical alkyle, alk, Alk ou ALK, désigne un radical linéaire ou ramifié renfermant au plus 12 atomes de carbone choisi parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés,
- le terme radical alkényle désigne un radical linéaire ou ramifié renfermant au plus 12 atomes de carbone et préférentiellement 4 atomes de carbone choisi par exemple parmi les valeurs suivantes: éthényle ou vinyle, propényl ou allyle, 1-propényle, n-butényle, i-butényle, 3-méthylbut-2-ényle, n-pentényle, hexényle, heptényle, octényle, cyclohexylbutényle et décényle ainsi que leurs isomères de position linéaires ou ramifiés,
- le terme radical alkynyle désigne un radical linéaire ou ramifié renfermant au plus 12 atomes de carbone et préférentiellement 4 atomes de carbone choisi par exemple parmi les valeurs suivantes: éthynyle, propynyle ou propargyle, butynyle, n-butynyle, i-butynyle, 3-méthylbut-2-ynyle, pentynyle ou hexynyle ainsi que leurs isomères de position linéaires ou ramifiés,
- le terme radical cycloalkyle désigne un radical carbocyclique monocyclique ou bicyclique renfermant de 3 à 10 chaînons et désigne notamment les radicaux cyclopropyle, cyclobutyle, cyclopentyle et cyclohexyle,
- le terme radical aryle désigne les radicaux insaturés, monocycliques ou constitués de cycles condensés, carbocycliques. Comme exemples de tel radical aryle, on peut citer les radicaux phényle ou naphtyle,
- le terme radical hétérocyclique désigne un radical carbocylique saturé (hétérocycloalkyle) ou insaturé (hétéroaryle) constitué au plus de 6 chaînons interrompus par un ou plusieurs hétéroatomes, identiques ou différents, choisis parmi les atomes d'oxygène, d'azote ou de soufre,
comme radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridinyle, pyrrolidinyle, imidazolidinyle, pyrazolidinyle, morpholinyle ou encore tétrahydrofuryle, tétrahydrothiényle, chromanyle, dihydrobenzofuranyle, indolinyle, pipéridinyle, perhydropyranyle, pyrindolinyle, tétrahydroquinoléinyle, tétrahydroisoquinoléinyle ou thioazolidinyle, tous ces radicaux étant éventuellement substitués,
- parmi les radicaux hétéroaryles à 5 chaînons on peut citer les radicaux furyle tel que 2-furyle, thiényle tel que 2-thiényle et 3-thiényle, pyrrolyle, diazolyle, thiazolyle, thiadiazolyle, thiatriazolyle, isothiazolyle, oxazolyle oxadiazolyle, 3- ou 4-isoxazolyle, imidazolyle, pyrazolyle, isoxazolyle,
Parmi les radicaux hétéroaryles à 6 chaînons on peut citer notamment les radicaux pyridyle tel que 2-pyridyle, 3-pyridyle et 4-pyridyle, pyrimidyle, pyrimidinyle, pyridazinyle, pyrazinyle et tétrazolyle,
- Comme radicaux hétéroaryles condensés contenant au moins un hétéroatome choisi parmi le soufre, l'azote et l'oxygène, on peut citer par exemple benzothiényle tel que 3-benzothiényle, benzofuryle, benzofurannyle, benzopyrrolyle, benzimidazolyle, benzoxazolyle, thionaphtyle, indolyle, purinyle, quinoléinyle, isoquinoléinyle et naphtyridinyle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

2. Produits de formule (I) telle que définis à **la** revendication **1** dans laquelle l'un de Y et Y1 représente un atome d'hydrogène et l'autre est choisi parmi OCF3, S(O)nCF3, S(O)nAlk, S02CHF2, S02CF2CF3 et SO2NR5R6,
les autres substituants desdits produits de formule (I) étant choisis parmi les valeurs définies à la revendication 1 et étant entendu que :
a) lorsque p représente l'entier 0, R représente Oxygène, R1 représente Oxygène, A1 représente une simple liaison ou alkyle, Y et Y1 identiques ou différents sont tels que l'un représente hydrogène et l'autre représente OCF3 ou Salk, A2 représente simple liaison ou alkyle et B2 représente un radical hétérocycle éventuellement substitué, alors R2 et R3 ne représentent pas l'un hydrogène et l'autre imidazolylalkyle,
b) lorsque p représente l'entier 0, R et R1 représentent Oxygène, A1 représente une simple liaison ou alkyle, Y et Y1 identiques ou différents sont tels que l'un représente hydrogène et l'autre représente OCF3, SOAlk, S(O)2alk ou S02NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
c) lorsque p représente l'entier 0, R et R1 représentent Oxygène, A1 représente une simple liaison ou alkyle, Y et Y1 identiques ou différents sont tels que l'un représente hydrogène et l'autre représente S(O)nAlk, A2 représente une simple liaison et B2 représente un radical hétérocycle aromatique renfermant 5 ou 6 chaînons éventuellement substitué, alors R2 et R3 ne sont pas choisis parmi hydrogène, alkyle, arylalkyle, aryle et hétéroaryle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

3. Produits de formule (I) telle que définie à l'une quelconque des revendications dans laquelle l'un de Y et Y1 représente un atome d'hydrogène et l'autre est choisi parmi S(O)nCF3, SOAlk, S(O)2Alk, SO2CHF2, SO2CF2CF3 et SO2NR5R6,
les autres substituants desdits produits de formule (I) étant choisis parmi les valeurs définies à la revendication 1 et étant entendu que les produits de formule (I) sont tels que définis ci-après en a) et b):
a) lorsque p représente l'entier 0, R et R1 représentent Oxygène, A1 représente une simple liaison ou alkyle, Y et Y1 identiques ou différents sont tels que l'un représente hydrogène et l'autre représente SOAlk, S(O)2alk ou S02NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
b) lorsque p représente l'entier 0, R et R1 représentent Oxygène, A1 représente une simple liaison ou alkyle, Y et Y1 identiques ou différents sont tels que l'un représente hydrogène et l'autre représente SOAlk ou S(0)2Alk, A2 représente une simple liaison et B2 représente un radical hétérocycle aromatique renfermant 5 ou 6 chaînons éventuellement substitué, alors R2 et R3 ne sont pas choisis parmi hydrogène, alkyle, arylalkyle, aryle et hétéroaryle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

4. Produits de formule (I) telle que définie à l'une quelconque des revendications dans laquelle l'un de Y et Y1 représente un atome d'hydrogène et l'autre est choisi parmi S(O)nCF3, S02CHF2, SO2CF2CF3 et SO2NR5R6 ,
les autres substituants desdits produits de formule (I) étant choisis parmi les valeurs définies à la revendication 1 et étant entendu que lorsque p représente l'entier 0, R et R1 représentent Oxygène, A1 représente une simple liaison ou alkyle, Y et Y1 identiques ou différents sont tels que l'un représente hydrogène et l'autre représente SO2NH2, A2 représente CH2 et B2 représente un radical hétérocycle éventuellement substitué, alors R2 et R3 ne représentent pas l'un hydrogène et l'autre une chaîne alkyle éventuellement interrompue par O, S, Nalk toujours substituée par un hydroxamate -CO-NHOH
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

5. Produits de formule (I) telle que définie à l'une quelconque des revendications dans laquelle l'un de Y et Y1 représente un atome d'hydrogène et l'autre est choisi parmi S(O)nCF3, S02CHF2 et SO2CF2CF3,
les autres substituants desdits produits de formule (I) étant choisis parmi les valeurs définies à la revendication 1,
lesdits produits de formule (I) étant sous toutes les formes isomères possibles racémiques, énantiomères et diastéréo-isomères, ainsi que les sels d'addition avec les acides minéraux et organiques ou avec lés bases minérales et organiques desdits produits de formule (I).

6. Produits de formule (I) telle que définie à l'une quelconque des revendications telle que tous les radicaux alkyle, alkényle, alkynyle, cycloalkyle, hétérocycloalkyle, aryle ou hétéroaryle définis à la revendication 1 sont éventuellement substitués par un ou plusieurs radicaux identiques ou différents choisis halogène, cyano, hydroxy, alcoxy, CF3, nitro, phényle, carboxy libre, salifié, estérifié par un radical alkyle ou amidifié par un radical NR11aR12a, -C(=O)-R9a, -NR11aR12a, -C(=O)-NR11aR12a, -N(R10a)-C(=O)-R9a, -N(R10a)-C(=O)-OR8a, N(R10a)-C(=O)-NR11aR12a, -N(R10a)-S(O)n-R9a, -S(O)n-R9a, -N(R10a)-S(O)n-NR11aR12a ou -S(O)n-NR11aR12a,
tous les radicaux aryle et hétéroaryle ci-dessus étant de plus éventuellement substitués par un radical éthylènedioxy,
R8a représente hydrogène, alkyle, alkényle, phényle, phénylalkyle, hétéroaryle ou hétéroarylalkyle,
R9a représente alkyle, cycloalkyle, cycloalkylalkyle, hétérocycloalkyle, hétérocycloalkylalkyle, phényle, phénylalkyle, hétéroaryle ou hétéroarylalkyle,
R10a représente hydrogène ou alkyle,
R11a et R12a, identiques ou différents, représentent hydrogène, alkyle, cycloalkyle, cycloalkylalkyle, phényle, phénylalkyle éventuellement substitués par un ou plusieurs substituants identiques ou différents choisis parmi halogène, hydroxy, C1-C4alkyl ou C1-C4alkoxy ou bien R11a et R12a forment avec l'atome d'azote auxquels ils sont liés un radical cyclique choisi parmi pyrrolidinyle, pipéridinyle, pipérazinyle, morpholinyle, indolinyle, pyrindolinyle, tétrahydroquinoléinyle, thiazolidinyle et naphtyridyle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

7. Produits de formule (I) telle que définie à l'une quelconque des revendications telle que p représente l'entier 0, les autres substituants desdits produits de formule (I) ayant les valeurs définies à l'une quelconque des revendications.

8. Produits de formule (I) telle que définie à l'une quelconque des revendications telle que p représente l'entier 1, les autres substituants desdits produits de formule (I) ayant les valeurs définies à l'une quelconque des revendications.

9. Produits de formule (I) telle que définie à l'une quelconque des revendications telle que p représente l'entier 2, les autres substituants desdits produits de formule (I) ayant les valeurs définies à l'une quelconque des revendications.

10. Produits de formule (I) telle que définie à l'une quelconque des revendications telle que R1 représente 0, les autres substituants desdits produits de formule (I) ayant les valeurs définies à l'une quelconque des revendications.

11. Produits de formule (I) telle que définie à l'une quelconque des revendications telle que R représente 0, les autres substituants desdits produits de formule (I) ayant les valeurs définies à l'une quelconque des revendications.

12. Produits de formule (I) telle que définie à l'une quelconque des revendications telle que R2 et R3, identiques ou différents représentent hydrogène, alkyle, alkényle, cycloalkyle, cycloalkylalkyle, phényle, phénylalkyle, hétérocycloalkyle, hétérocycloalkylalkyle, hétéroaryle et hétéroarylalkyle, éventuellement substitués ou bien R2 et R3 forment ensemble avec l'atome de carbone auxquels ils sont liés un radical carbocyclique ou hétérocyclique, ces radicaux étant constitués de 3 à 10 chaînons et le radical hétérocyclique renfermant un ou plusieurs hétéroatomes choisis parmi O, S, N et NR7b, tous ces radicaux étant éventuellement substitués,
tous les radicaux ci-dessus étant éventuellement substitués par un ou plusieurs radicaux choisis parmi halogène, cyano, hydroxy, alkyle et alcoxy renfermant 1 à 4 atomes de carbone, CF3, nitro, phényle, carboxy libre, salifié, estérifié par un radical alkyle ou amidifié par un radical NR11bR12b, -C(=O)-R9b, -NR11bR12b et -C(=O)-NR11bR12b,
R7b représente un atome d'hydrogène, un radical alkyle ou un radical phényle,
R9 représente hydrogène, alkyle, cycloalkyle, cycloalkylalkyle et phényle,
R11b et R12b, identiques ou différents, représentent hydrogène, alkyle, cycloalkyle et phényle ou bien R11b et R12b forment avec l'atome d'azote auxquels ils sont liés un radical pipérazinyle éventuellement substitué,
les autres substituants desdits produits de formule (I) ayant les valeurs définies à l'une quelconque des revendications.

13. **P**roduits de formule (I) telle que définie à l'une quelconque des revendications telle que R2 et R3 identiques ou différents sont choisis parmi hydrogène, alkyle, phénylalkyle, pyridylalkyle et thiénylbenzothienylalkyle, éventuellement substitués par un ou plusieurs radicaux choisis parmi les atomes d'halogène et les radicaux hydroxy, alkyle et alcoxy renfermant un à 4 atomes de carbone ou bien R2 et R3 forment ensemble avec l'atome de carbone auxquels ils sont liés un radical cycloalkyle ou hétérocycloalkyle de 3 à 6 chaînons renfermant un atome d'azote, les autres substituants desdits produits de formule (I) ayant les valeurs définies à l'une quelconque des revendications.

14. Produits de formule (I) telle que définie à l'une quelconque des revendications telle que R2 et R3 identiques ou différents sont choisis parmi hydrogène, alkyle, hydroxyalkyle, phénylalkyle, hydroxyphénylalkyle, pyridylalkyle, thiénylbenzothienylalkyle ou bien R2 et R3 forment ensemble avec l'atome de carbone auxquels ils sont liés un radical cycloalkyle renfermant de 3 à 6 atomes de carbone ou un radical azétidinyle, pyrrolidinyle et pipéridinyle,
les autres substituants desdits produits de formule (I) ayant les valeurs définies à l'une quelconque des revendications.

15. Produits de formule (I) telle que définie à l'une quelconque des revendications telle que R2 et R3 identiques ou différents sont choisis parmi hydrogène, alkyle, hydroxyalkyle, phénylalkyle et hydroxyphénylalkyle, ou bien R2 et R3 forment ensemble avec l'atome de carbone auxquels ils sont liés un radical cycloalkyle renfermant de 3 à 6 atomes de carbone.

16. Produits de formule (I) telle que définie à l'une quelconque des revendications telle que l'un de R2 et R3 est choisi parmi hydrogène et alkyle, et l'autre de R2 et R3 est choisi parmi toutes les valeurs de R2 et R3 ou bien R2 et R3 forment ensemble avec l'atome de carbone auxquels ils sont liés un radical cycloalkyle renfermant de 3 à 6 atomes de carbone,
les autres substituants desdits produits de formule (I) ayant les valeurs définies à l'une quelconque des revendications.

17. Produits de formule (I) telle que définie à l'une quelconque des revendications telle que R2 et R3 identiques ou différents représentent hydrogène et alkyle, ou bien R2 et R3 forment ensemble avec l'atome de carbone auxquels ils sont liés un radical cycloalkyle renfermant de 3 à 6 atomes de carbone,
les autres substituants desdits produits de formule (I) ayant les valeurs définies à l'une quelconque des revendications.

18. Produits de formule (I) telle que définie à l'une quelconque des revendications telle que R2 et R3 identiques ou différents représentent hydrogène et CH3, ou bien R2 et R3 forment ensemble avec l'atome de carbone auxquels ils sont liés un radical cyclopropyle, les autres substituants desdits produits de formule (I) ayant les valeurs définies à l'une quelconque des revendications.

19. Produits de formule (I) telle que définie à l'une quelconque des revendications telle que A1 représente une simple liaison et A2 est choisi parmi une simple liaison, un radical alkyle linéaire ou ramifié renfermant au plus 6 atomes de carbone et les radicaux allyle, propynyle, C=O et SO2, les autres substituants desdits produits de formule (I) ayant les valeurs définies à l'une quelconque des revendications.

20. Produits de formule (I) telle que définie à l'une quelconque des revendications telle que A1 représente une simple liaison et A2 est choisi parmi une simple liaison, les radicaux alkyle, allyle, propynyle, C=O et SO2, les autres substituants desdits produits de formule (I) ayant les valeurs définies à l'une quelconque des revendications.

21. Produits de formule (I) telle que définie à l'une quelconque des revendications telle que A1 représente une simple liaison et A2 représente un radical alkyle ou C=O, les autres substituants desdits produits de formule (I) ayant les valeurs définies à l'une quelconque des revendications.

22. Produits de formule (I) telle que définie à l'une quelconque des revendications telle que A1 représente une simple liaison et A2 représente C=O, -CH2-CH2- ou -CH2, les autres substituants desdits produits de formule (I) ayant les valeurs définies à l'une quelconque des revendications.

23. Produits de formule (I) telle que définie à l'une quelconque des revendications telle que A1 représente une simple liaison et A2 représente -CH2, les autres substituants desdits produits de formule (I) ayant les valeurs définies à l'une quelconque des revendications.

24. Produits de formule (I) telle que définie à l'une quelconque des revendications dans laquelle Y et Y1 sont tels que l'un représente un atome d'hydrogène, un atome d'halogène ou un radical amino et l'autre est choisi parmi -OCF3, -O-CF2-CHF2, -O-CHF2, -O-CH2-CF3, -SF5, -S(O)n-CF3, -S(O)n-Alk, -SO2CHF2, S02CF2CF3,-SO2NH2, -S-CF2-CF2-CF3, -S-Alk-O-Alk, -S-Alk-OH, -S-Alk-CN, -S-Alk-morpholino, -S-Alk-pyrrolidinyle et -S-Alk-pipérazinyle, les radicaux morpholino, pyrrolidinyle et pipérazinyle étant éventuellement substitué par Alk, avec Alk représente un radical alkyle renfermant de 1 à 4 atomes de carbone,
les autres substituants desdits produits de formule (I) ayant les valeurs définies à l'une quelconque des revendications.

25. Produits de formule (I) telle que définie à l'une quelconque des revendications telle que Y représente un atome d'hydrogène et Y1 est choisi parmi -OCF3, S(O)n-CF3, S(O)n-CH3, SO2CHF2 et SO2-N(alk)2 les autres substituants desdits produits de formule (I) ayant les valeurs définies à l'une quelconque des revendications.

26. Produits de formule (I) telle que définie à l'une quelconque des revendications telle que Y représente un atome d'hydrogène et Y1 est choisi parmi -OCF3, S(O)n-CF3 et S02CHF2,
les autres substituants desdits produits de formule (I) ayant les valeurs définies à l'une quelconque des revendications.

27. Produits de formule (I) telle que définie à l'une quelconque des revendications telle que Y représente un atome d'hydrogène et Y1 est choisi parmi -OCF3 et S(O)n-CF3,
les autres substituants desdits produits de formule (I) ayant les valeurs définies à l'une quelconque des revendications.

28. Produits de formule (I) telle que définie à l'une quelconque des revendications telle que Y représente un atome d'hydrogène et Y1 est choisi parmi -OCF3, S-CF3 et S(O)2-CF3,
les autres substituants desdits produits de formule (I) ayant les valeurs définies à l'une quelconque des revendications.

29. Produits de formule (I) telle que définie à l'une quelconque des revendications telle que B2 représente un radical hétéroaryle monocyclique ou bicyclique choisi parmi les radicaux pyridyle, pyrimidinyle, quinolyle, azaindolyle, 1H-pyrrolo[2,3-b]pyridinyle, quinazolyle, thiazolyle, imidazolyle, pyrazolyle, furazanyle, isoxazolyle, morpholinyle, pyrrolidinyle, furyle, pipéridyle, thiényle, chroményle, oxochroményle, indolyle, pyrrolyle, purinyle, benzoxazinyle, benzimidazolyle, benzofurannyle ces radicaux étant éventuellement substitués par un ou plusieurs radicaux choisis parmi les valeurs de Y2,
les autres substituants desdits produits de formule (I) ayant les valeurs définies à l'une quelconque des revendications.

30. Produits de formule (I) telle que définie à l'une quelconque des revendications telle que B2 représente un radical hétéroaryle choisi parmi les radicaux 3- ou 4-pyridyle, pyrimidinyle, 3- ou 4- quinolyle, azaindolyle, quinazolyle, thiazolyle, imidazolyle, pyrazolyle, furazanyle et isoxazolyle,
ces radicaux étant éventuellement substitués par un ou plusieurs radicaux choisis parmi les valeurs de Y2,
les autres substituants desdits produits de formule (I) ayant les valeurs définies à l'une quelconque des revendications.

31. Produits de formule (I) telle que définie à l'une quelconque des revendications telle que B2 représente un radical hétéroaryle choisi parmi les radicaux 3- ou 4-pyridyle, pyrimidinyle, 3- ou 4- quinolyle, azaindolyle et quinazolyle, ces radicaux étant éventuellement substitués par un ou plusieurs radicaux choisis parmi les valeurs de Y2,
les autres substituants desdits produits de formule (I) ayant les valeurs définies à l'une quelconque des revendications.

32. Produits de formule (I) telle que définie à l'une quelconque des revendications telle que B2 représente les radicaux 4- pyridyle et 4- quinolyle, 1H-Pyrrolo[2,3-b]pyridine-4-yl éventuellement substitués par un ou plusieurs radicaux choisis parmi les valeurs de Y2 définies à l'une quelconque des revendications,
les autres substituants desdits produits de formule (I) ayant les valeurs définies à l'une quelconque des revendications.

33. Produits de formule (I) telle que définie à l'une quelconque des revendications telle que Y2 représente hydrogène, halogène, hydroxyle, cyano, alkyle, alcoxy, phényle, COOH, COOAlk, CONR5R6, NR5R6, -NR10-COOR6, -NR10-CO-R6, -NR10-CS-NR5R6, -NR10-CO-NR5R6 ou -NR10-SO2-R6, tous ces radicaux étant éventuellement substitués,
R5 et R6 identiques ou différents sont choisis parmi hydrogène, alkyle, cycloalkyle, phényle et lesradicaux hétéroaryles renfermant 5 à 6 chaînons et contenant 1 à 3 hétéroatomes choisis parmi O, N et S, tous ces radicaux étant éventuellement substitués, ou bien R5 et R6 forment avec l'atome d'azote auxquels ils sont liés un radical pyrrolidinyle, pipéridyle, pipérazinyle, morpholinyle ou quinazolinyle éventuellement substitués
R10 représente hydrogène ou alkyle,
tous les radicaux ci-dessus alkyle, alcoxy, cycloalkyle et phényle ainsi que le cycle formé par R5 et R6 avec l'atome auxquels ils sont liés étant éventuellement substitués par un ou plusieurs radicaux identiques ou différents choisis parmi les atomes d'halogène et les radicaux cyano ; hydroxyle ; alkyle ; alcoxy ; OCF3 ; CF3 ; S(O)n-CF3 nitro ; oxo ; thioxo ; OCOAlk ; phényle lui-même éventuellement substitué par un ou plusieurs radicaux choisis parmi les atomes d'halogène et les radicaux alkyle et alcoxy ; -OCOAlk ; ; NH2, NHAlk, N(Alk)2, N(alk)(phénylalkyle), N(Alk) (aminoalkyle) N(Alk)(alkylaminoalkyle)
N(Alk)(dialkylaminoalkyle ; carboxy libre ou estérifié par un radical alkyle
tous les radicaux ci-dessus phényle étant de plus éventuellement substitués par un radical alkylènedioxy,
tous les radicaux ci-dessus alkyle étant de plus éventuellement substitués par un ou plusieurs radicaux hétérocycliques saturés ou partiellement insaturés renfermant 4 à 7 chaînons et contenant au moins un atome d'azote N et en plus 0 à 2 autres hétéroatomes choisi(s) parmi O, N et S,
tous les radicaux pyrrolidinyle et quinazolinyle ci-dessus étant de plus éventuellement substitués par oxo ou thioxo,
tous les radicaux ci-dessus alkyle et alcoxy, étant linéaires ou ramifiés et renfermant au plus 6 atomes de carbone,
tous les radicaux ci-dessus cycloalkyle renfermant au plus 7 atomes de carbone,
les autres substituants desdits produits de formule (I) ayant les valeurs définies à l'une quelconque des revendications.

34. Produits de formule (I) telle que définie à l'une quelconque des revendications telle que Y2 représente hydrogène, halogène, hydroxyle, cyano, alkyle, alcoxy, phényle, CONR5R6, NR5R6, -NR10-COOH, -NR10-COOAlk, -NR10-CO-R6, -NR10-CS-NR5R6, -NR10-CO-NR5R6 ou -NR10-S02-R6,
R5 et R6 identiques ou différents sont choisis parmi hydrogène : alkyle ; cycloalkyle ; phényle ; pyrimidinyle ; thiényle ; pyridyle, quinolyle ; thiazolyle éventuellement substitué par un ou deux atomes d'halogène ; pyranne éventuellement substitué par un ou plusieurs OCOAlk ; phényle substitué par un ou plusieurs radicaux choisis parmi les atomes d'halogène et les radicaux alkyle, alcoxy , amino, alkylamino, dialkylamino et carboxy libre ou estérifié par un radical alkyle : alkyle substitué par phényle lui-même éventuellement substitué par un ou plusieurs radicaux choisis parmi les atomes d'halogène, alkyle, alcoxy, amino, alkylamino, dialkylamino, carboxy libre ou estérifié par un radical alkyle alkyle substitué par pipérazinyle lui-même éventuellement substitué par un ou plusieurs radicaux choisis parmi Alk, Alk-OH et pyridyle ; alkyle substitué par imidazolyle ; alkyle substitué par un ou plusieurs radicaux choisis parmi NH2, NHAlk, N(Alk)2, N(alk)(phénylalkyle), N(Alk)(aminoalkyle) N(Alk) (alkylaminoalkyle)et N(Alk)(dialkylaminoalkyle) ; alkyle substitué par morpholinyle éventuellement substitué par un ou deux Alk ; alkyle substitué par pyrrolidinyle ; alkyle substitué par pipéridyle lui-même éventuellement substitué par un ou deux Alk ; alkyle substitué par thiomorpholinyle ; alkyle substitué par azetidinyle ; alkyle substitué par azépanyle éventuellement substitué par oxo,
ou bien R5 et R6 forment avec l'atome d'azote auxquels ils sont liés un radical pyrrolidinyle ; pipéridyle ; pipérazinyle ; morpholinyle ; ou quinazolinyle, tous ces radicaux étant éventuellement substitués par un ou plusieurs radicaux identiques ou différents choisis parmi les atomes d'halogène et les radicaux alkyle, hydroxyle, alcoxy et phényle lui-même éventuellemnt substitué par un ou plusieurs radicaux choisis parmi les atomes d'halogène et les radicaux alkyle et alcoxy,
les radicaux pyrrolidinyle et quinazolinyle étant de plus éventuellement substitué par oxo ou thioxo,
le radical pipérazinyle lui-même éventuellement substitué par un ou plusieurs radicaux choisis parmi Alk, Alk-OH et pyridyle,
R10 représente hydrogène ou alkyle,
tous les radicaux ci-dessus alkyle ou Alk et alcoxy, étant linéaires ou ramifiés et renfermant au plus 6 atomes de carbone,
tous les radicaux ci-dessus cycloalkyle renfermant au plus 7 atomes de carbone,
tous les radicaux phényle étant de plus éventuellement substitués par un radical choisi parmi CF3, -OCF3, nitro et alkylènedioxy,
les autres substituants desdits produits de formule (I) ayant les valeurs définies à l'une quelconque des revendications.

35. Produits de formule (I) telle que définie à l'une quelconque des revendications telle que Y2 représente V1, halogène, hydroxyle, -C(=NH)NH2, OV1, O-CO-V1, COOV1, COV1, CO-NVlV2, -NV1V2, -NH-CO-V1, -NH-COO-V1, -NH-NH-CO-V1, -NV1-CO-NV1V2, -NVl-CO-NHV1, -NH-CO-NHV1, -NH-SO2-NHV1 et -NH-SO2-V1,
dans lesquels V1 et V2 identiques ou différents représentent un atome d'hydrogène, un radical alkyle, cycloalkyle ou phényle ou un radical hétérocyclique tel que pyridinyle, pyrazolyle, imidazolyle, dihydroimidazolyle, tétrazolyle, morpholinyle, pipérazinyle, pipérazinylalkyle, alkylpipérazinyle, phénylpipérazinyle, thiényle, furannyle, pipéridinyle, méthylpipéridinyle, pyridyle, pyrrolidinyle et pyrrolidinylalkyle,
tous les radicaux alkyle, phényle et hétérocyclique étant éventuellement substitués par un ou plusieurs radicaux choisis parmi les atomes d'halogène, les radicaux hydroxyle, alkyle, alcoxy, CF3, NH2, NHalk, N(alk)2 et phényle lui-même éventuellement substitué par un ou plusieurs substituants choisis parmi les atomes d'halogène, les radicaux hydroxy et alcoxy,
tous les radicaux phényle et hétérocyclique ci-dessus étant de plus éventuellement substitués par un ou plusieurs radicaux alkyle,
les radicaux phényle étant de plus éventuellement substitués par NR5R6 avec R5 et R6 tels que définis à la revendication 1,
les autres substituants desdits produits de formule (I) ayant les valeurs définies à l'une quelconque des revendications.

36. Produits de formule (I) telle que définie à l'une quelconque des revendications telle que Y2 représente hydrogène, halogène, alkyle, cycloalkyle, hydroxyle, alcoxy, carboxy libre ou estérifié par un radical alkyle ou phényle, NH2, NHalk, N(alk)2 et phényle,
tous les radicaux alkyle, alcoxy et phényle étant éventuellement substitués par un ou plusieurs radicaux choisis parmi les atomes d'halogène, les radicaux hydroxyle, C1-C4alkyle, C1-C4alcoxy, CF3, NH2, NHalk, N(alk)2 et phényle lui-même éventuellement substitué par un ou plusieurs substituants choisis parmi les atomes d'halogène, les radicaux hydroxy et alcoxy,
tous les radicaux phényle étant de plus éventuellement substitués par un ou plusieurs radicaux Cl-C4alkyle et éventuellement substitués par NR5R6 avec R5 et R6 tels que définis à la revendication 1,
les autres substituants desdits produits de formule (I) ayant les valeurs définies à l'une quelconque des revendications.

37. Produits de formule (I) telle que définie à l'une quelconque des revendications telle que Y2 représente hydrogène, F, Cl, CH3, CH2CH3, OH, OCH3, NH2, NHAlk et phényle éventuellement substitué par NR5R6 avec R5 et R6 tels que définis à la revendication 1,
les autres substituants desdits produits de formule (I) ayant les valeurs définies à l'une quelconque des revendications.

38. Produits de formule (I) telle que définie à l'une quelconque des revendications telle que B2 représente les radicaux 4- pyridyl et 4- quinolyl substitués par un ou deux radicaux choisis parmi F, Cl, OH et OCH3,
les autres substituants desdits produits de formule (I) ayant les valeurs définies à l'une quelconque des revendications.

39. Produits de formule (I) telle que définie à l'une quelconque des revendications répondant à la formule (IC) : 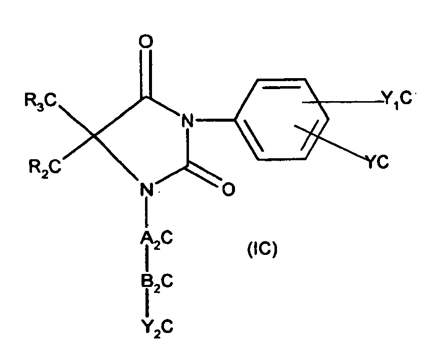 dans laquelle YC et Y1C sont tels que l'un représente un atome d'hydrogène, un atome d'halogène ou un radical amino et l'autre est choisi parmi -OCF3, -O-CF2-CHF2, -O-CHF2, -O-CH2-CF3, -SFS, -S(O)n-CF3, -S(O)n-Alk, -S02CHF2, SO2CF2CF3,-SO2NH2. -S-CF2-CF2-CF3, -S-Alk-O-Alk, -S-Alk-OH, -S-Alk-CN, -S-Alk-morpholino, -S-Alk-pyrrolidinyle et -S-Alk-pipérazinyle, les radicaux morpholino, pyrrolidinyle et pipérazinyle étant éventuellement substitué par Alk, avec Alk représente un radical alkyle renfermant de 1 à 4 atomes de carbone,
ou bien le radical phényle forme avec ses substituants YC et Y1C l'un des deux radicaux suivants : 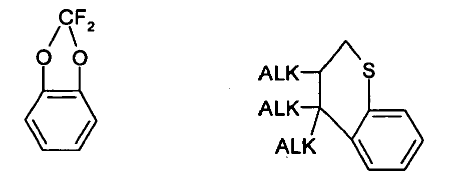 avec ALK représente un radical alkyle renfermant de 1 à 4 atomes de carbone,
R2C et R3C identiques ou différents représentent hydrogène ou alkyle éventuellement substitué ou bien R2C R2C et R3C identiques ou différents représentent hydrogène ou alkyle éventuellement substitué ou bien R2C et R3C forment ensemble avec l'atome de carbone auxquels ils sont liés un radical C3-C10 cycloalkyle ou hétérocycloalkyle,
A2C représente une simple liaison ou CH2,
B2C représente les radicaux pyridyle, pyrimidinyle, quinolyle, azaindolyle, quinazolyle, thiazolyle, imidazolyle, pyrazolyle, furazannyle, isoxazolyle, morpholinyle, pyrrolidinyle, furyle, pipéridyle, chroményle, oxochroményle, quinazolyle, thiényle, indolyle, pyrrolyle, purinyle, benzoxazinyle, benzimidazolyle, benzofurannyle éventuellement substitués par un ou plusieurs radicaux choisis parmi les valeurs de Y2A,
Y2CA représente hydrogène, halogène, hydroxyle, cyano, alkyle, alcoxy, phényle, COOH, COOAlk, CONR5R6, NR5R6, -NR10-COOH, -NR10-COOAlk, -NR10-CO-R6, -NR10-CS-NR5R6, -NR10-CO-NR5R6 ou -NR10-SO2-R6, tous ces radicaux étant éventuellement substitués,
R5 et R6 identiques ou différents sont choisis parmi hydrogène, alkyle, cycloalkyle, phényle, pyrimidinyle, thiényle, pyridyle, quinolyle, thiazolyle et pyranne, tous ces radicaux étant éventuellement substitués, ou bien R5 et R6 forment avec l'atome d'azote auxquels ils sont liés un radical pyrrolidinyle, pipéridyle, pipérazinyle, morpholinyle ou quinazolinyle éventuellement substitués,
R10 représente hydrogène ou alkyle,
tous les radicaux ci-dessus alkyle, alcoxy, cycloalkyle et phényle ainsi que le cycle formé par R5 et R6 avec l'atome auxquels ils sont liés étant éventuellement substitués par un ou plusieurs radicaux identiques ou différents choisis parmi les atomes d'halogène et les radicaux cyano ; hydroxyle ; alkyle ; alcoxy ; OCF3 ; CF3 ; S(O)n-CF3 : nitro ; oxo ; thioxo ; OCOAlk ; phényle lui-même éventuellement substitué par un ou plusieurs radicaux choisis parmi les atomes d'halogène et les radicaux alkyle et alcoxy ; -OCOAlk ; NH2, NHAlk, N(Alk)2, N(alk)(phénylalkyle), N(Alk)(aminoalkyle) N(Alk) (alkylaminoalkyle) N(Alk)(dialkylaminoalkyle ; carboxy libre ou estérifié par un radical alkyle
tous les radicaux ci-dessus phényle étant de plus éventuellement substitués par un radical alkylènedioxy, tous les radicaux ci-dessus alkyle étant de plus éventuellement substitués par un ou plusieurs radicaux choisis parmi les radicaux pipérazinyle lui-même éventuellement substitué par Alk, Alk-OH et pyridyle ; imidazolyle ; morpholinyle ; pyrrolidinyle ; pipéridyle lui-même éventuellement substitué par un ou deux alk ; azépanyle éventuellement substitué par oxo,
tous les radicaux pyrrolidinyle et quinazolinyle ci-dessus étant de plus éventuellement substitués par oxo ou thioxo,
tous les radicaux ci-dessus alkyle et alcoxy, étant linéaires ou ramifiés et renfermant au plus 6 atomes de carbone,
tous les radicaux ci-dessus cycloalkyle renfermant au plus 7 atomes de carbone,
n représente un entier de 0 à 2,
lesdits produits de formule (IC) étant sous toutes les formes isomères possibles racémiques, énantiomères et diastéréo-isomères, ainsi que les sels d'addition avec les acides minéraux et organiques ou avec les bases minérales et organiques desdits produits de formule (IC).

40. Produits de formule (I) telle que définie à l'une quelconque des revendications répondant à la formule (IA) : 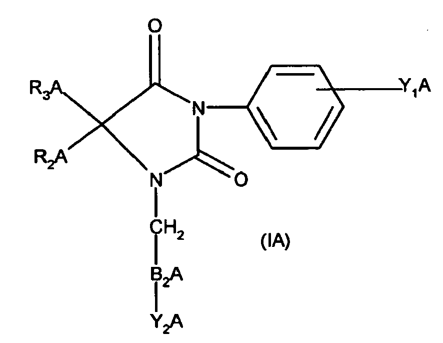 dans laquelle :
Y1A représente -OCF3, S(O)n-CF3 et SO2CHF2,
B2A représente les radicaux 4-quinolyl et 4-pyridyl éventuellement substitués par un ou plusieurs radicaux choisis parmi les valeurs de Y2A,
Y2A a la signification indiquée à l'une quelconque des revendications pour Y2,
R2A et R3A identiques ou différents représentent hydrogène ou alkyle éventuellement substitué ou bien R2A et R3A forment ensemble avec l'atome de carbone auxquels ils sont liés un radical C3-C10 cycloalkyle ou hétérocycloalkyle,
tous les radicaux alkyle et phényle étant éventuellement substitués par un ou plusieurs radicaux choisis parmi Halogène, OH, alk, Oalk, OCF3, S(O)n-CF3, CF3, NH2, NHAlk et N(alk)2,
n représente un entier de 0 à 2,
lesdits produits de formule (IA) étant sous toutes les formes isomères possibles racémiques, énantiomères et
diastéréo-isomères, ainsi que les sels d'addition avec les acides minéraux et organiques ou avec les bases minérales et organiques desdits produits de formule (IA).

41. Produits de formule (IA) telle que définie à la revendication précédente dans laquelle Y1A, B2a, R2A et R3A ont les significations indiquées précédemment et Y2A représente les radicaux halogène, -OH, -alk, Oalk, -Oacyl, -NR5AR6A, -CO2H, -CO2alk, -CO-NR5AR6A, -S(O)n-CF3, -NH-S(O)n-CF3 ou phényle, alk représentant un radical alkyle linéaire ou ramifié renfermant au plus 6 atomes de carbone, tous les radicaux alkyle, alcoxy et phényle étant éventuellement substitués,
R5A et R6A identiques ou différents représentent hydrogène, alkyle, cycloalkyle, phényle, les radicaux alkyle et phényle étant éventuellement substitués ou bien R5A et R6A forment avec l'atome d'azote auxquels ils sont liés un radical cyclique choisi parmi les radicaux pyrrolidinyle, pipéridinyle, pipérazinyle, morpholinyle, pipérazinyle, indolinyle, pyrindolinyle, tétrahydroquinoline et azétidine,
tous les radicaux alkyle, alcoxy et phényle étant éventuellement substitués par un ou plusieurs radicaux choisis parmi Halogène, OH, alk, Oalk, OCF3, S(O)n-CF3, CF3, NH2, NHAlk et N(alk)2,
n représente un entier de 0 à 2,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

42. Produits de formule (I) telle que définie à l'une quelconque des revendications répondant à la formule (IA) dans laquelle :
Y1A représente -OCF3, SCF3 ou S(O)2-CF3,
B2a représente un radical 4-quinolyle ou 4-pyridyle éventuellement substitués par un ou deux radicaux choisis parmi halogène, -OH, alk, -Oalk, -CO2H, -CO2alk, -NR5AR6A, -CF3, -OCF3 et phényle éventuellement substitué,
R5A et R6A identiques ou différents représentent hydrogène, alkyle, cycloalkyle, phényle, les radicaux alkyle et phényle étant éventuellement substitués,
ou bien R5A et R6A forment avec l'atome d'azote auxquels ils sont liés un radical cyclique choisi parmi les radicaux pyrrolidinyle, pipéridinyle, pipérazinyle, morpholinyle, pipérazinyle et azétidine,
R2A et R3A identiques ou différents représentent hydrogène ou alkyle éventuellement substitué ou bien R2A et R3A forment ensemble avec l'atome de carbone auxquels ils sont liés un radical C3-C6 cycloalkyle ou hétérocycloalkyle,
tous les radicaux alkyle et phényle étant éventuellement substitués par un ou plusieurs radicaux choisis parmi Halogène, OH, alk, Oalk, OCF3, S(O)n-CF3, CF3, NH2, NHalk et N(alk)2,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

43. Produits de formule (I) telle que définie à l'une quelconque des revendications répondant à la formule (IA) dans laquelle :
Y1A représente -OCF3, SCF3 ou S(O)2-CF3,
B2a représente un radical 4-quinolyle ou 4-pyridyle éventuellement substitués par un ou deux radicaux choisis parmi halogène, -OH, alk et -Oalk,
R2A et R3A identiques ou différents représentent hydrogène et alkyle linéaire ou ramifié renfermant au plus 4 atome de carbone éventuellement substitué par un radical hydroxyle ou bien R2A et R3A forment ensemble avec l'atome de carbone auxquels ils sont liés un radical C3-C6 cycloalkyle
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

44. Produits de formule (I) telle que définie à l'une quelconque des revendications répondant à la formule (IA) dans laquelle Y1a représente OCF3, SCF3 ou S(O)2CF3 et R2A et R3A identiques ou différents représentent hydrogène et CH3 ou bien R2A et R3A forment ensemble avec l'atome de carbone auxquels ils sont liés un radical cyclopropyle,
les autres substituants ayant les valeurs indiquées à l'une quelconque des revendications,
lesdits produits de formule (IA) étant sous toutes les formes isomères possibles racémiques, énantiomères et diastéréo-isomères, ainsi que les sels d'addition avec les acides minéraux et organiques ou avec les bases minérales et organiques desdits produits de formule (IA).

45. Produits de formule (I) telle que définie à l'une quelconque des revendications répondant à la formule (IB): 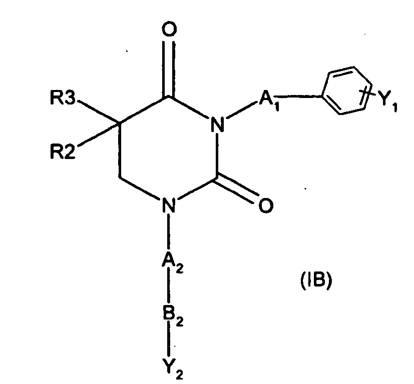 dans laquelle R2, R3, A1, Y, Y1, A2, B2 et Y2 ont les significations indiquées à l'une quelconque des revendications,
lesdits produits de formule (IB) étant sous toutes les formes isomères possibles racémiques, énantiomères et diastéréo-isomères, ainsi que les sels d'addition avec les acides minéraux et organiques ou avec les bases minérales et organiques desdits produits de formule (IB).

46. Produits de formule (IB) telle que définie à la revendication précédente dans laquelle Y1 représente OCF3, SCF3 ou S(O)2CF3 et R2 et R3 identiques ou différents représentent hydrogène et CH3 ou bien R2 et R3 forment ensemble avec l'atome de carbone auxquels ils sont liés un radical cyclopropyle,
les autres substituants ayant les valeurs indiquées à l'une quelconque des revendications,
lesdits produits de formule (IB) étant sous toutes les formes isomères possibles racémiques, énantiomères et diastéréo-isomères, ainsi que les sels d'addition avec les acides minéraux et organiques ou avec les bases minérales et organiques desdits produits de formule (IB).

47. Produits de formule (I) telle que définie à l'une quelconque des revendications dont les noms suivent :
- le trifluoroacétate de (S)-5-méthyl-1-quinolin-4-ylméthyl-3-(4-trifluorométhanesulfonyl-phényl)-imidazolidine-2,4-dione
- le trifluoroacétate de (S)-5-méthyl-1-pyridin-4-ylméthyl-3-(4-trifluorométhanesulfonyl-phényl)-imidazolidine-2,4-dione
- le trifluoroacétate de (S)-5-méthyl-1-quinolin-4-ylméthyl-3-(4-trifluorométhylsulfanyl-phényl)-imidazolidine-2,4-dione
- le trifluoroacétate de 5,5-diméthyl-1-quinolin-4-ylméthyl-3-(4-trifluorométhanesulfonyl-phényl)-imidazolidine-2,4-dione
- le trifluoroacétate de (R)-5-méthyl-1-quinolin-4-ylméthyl-3-(4-trifluorométhanesulfonyl-phényl)-imidazolidine-2,4-dione
- le trifluoroacétate de (R)-5-méthyl-1-quinolin-4-ylméthyl-3-(4-trifluorométhylsulfanyl-phényl)-imidazolidine-2,4-dione
- le trifluoroacétate de (R)-5-méthyl-1-pyridin-4-ylméthyl-3-(4-trifluorométhanesulfonyl-phényl)-imidazolidine-2,4-dione
- le trifluoroacétate de (R)-5-méthyl-1-(3-méthyl-pyridin-4-ylméthyl)-3-(4-trifluorométhanesulfonyl-phényl)-imidazolidine-2,4-dione
- le trifluoroacétate de (R)-4-méthyl-3-quinolin-4-ylméthyl-5-thioxo-1-(4-trifluorométhylsulfanyl-phényl)-imidazolidin-2-one
- le trifluoroacétate de (R)-5-isopropyl-1-quinolin-4-ylméthyl-3-(4-trifluorométhylsulfanyl-phényl)-imidazolidine-2,4-dione
- le trifluoroacétate de (R)-5-isopropyl-1-quinolin-4-ylméthyl-3-(4-trifluorométhanesulfonyl-phényl)-imidazolidine-2,4-dione
- le trifluoroacétate de (R)-5-(4-hydroxy-benzyl)-1-quinolin-4-ylméthyl-3-(4-trifluorométhanesulfonyl-phényl)-imidazolidine-2,4-dione
- le trifluoroacétate de (R)-5-(9-hydroxy-benzyl)-1-pyridin-4-ylméthyl-3-(4-trifluorométhanesulfonyl-phényl)-imidazolidine-2,4-dione
- le trifluoroacétate de (R)-5-(1-hydroxy-éthyl)-1-quinolin-4-ylméthyl-3-(4-trifluorométhanesulfonyl-phényl)-imidazolidine-2,4-dione
- le trifluoroacétate de, 4-quinolin-4-ylméthyl-6-(4-trifluorométhylsulfanyl-phényl)-4,6-diaza-spiro[2.4] heptane-5,7-dione
- le trifluoroacétate de 4-quinolin-4-ylméthyl-6-(4-trifluorométhanesulfonyl-phényl)-4,6-diaza-spiro[2.4] heptane-5,7-dione, trifluoroacétate
- le trifluoroacétate de 4-pyridin-4-ylméthyl-6-(4-trifluorométhylsulfanyl-phényl)-4,6-diaza-spiro[2.4] heptane-5,7-dione
- le trifluoroacétate de 4-pyridin-4-ylméthyl-6-(4-trifluorométhanesulfonyl-phényl)-4,6-diaza-spiro[2.4] heptane-5,7-dione
- le trifluoroacétate de (R)-1-(3-hydroxy-pyridin-4-ylméthyl)-5-méthyl-3-(4-trifluorométhylsulfanyl-phényl)-imidazolidine-2,4-dione
- le trifluoroacétate de 5,5-diméthyl-1-quinolin-4-ylméthyl-3-(4-trifluorométhoxy-phényl)-imidazolidine-2,4-dione
- le trifluoroacétate de 5,5-diméthyl-1-quinolin-4-ylméthyl-3-(4-trifluorométhylsulfanyl-phényl)-imidazolidine-2,4-dione
- le trifluoroacétate de 5,5-diméthyl-1-(3-méthyl-pyridin-4-ylméthyl)-3-(4-trifluorométhoxy-phényl)-imidazolidine-2,4-dione
- le trifluoroacétate de 5,5-diméthyl-1-(3-méthyl-pyridin-4-ylméthyl)-3-(4-trifluorométhylsulfanyl-phényl)-imidazolidine-2,4-dione
- le trifluoroacétate de 5,5-diméthyl-1-(3-méthyl-pyridin-4-ylméthyl)-3-(4-trifluorométhanesulfonyl-phényl)-imidazolidine-2,4-dione
- le trifluoroacétate de 1-(3-hydroxy-pyridin-4-ylméthyl)-5,5-diméthyl-3-(4-trifluorométhoxy-phényl)-imidazolidine-2,4-dione
- le trifluoroacétate de 1-(3-hydroxy-pyridin-4-ylméthyl)-5,5-diméthyl-3-(4-trifluorométhylsulfanyl-phényl)-imidazolidine-2,4-dione
- le trifluoroacétate de 1-(3-hydroxy-pyridin-4-ylméthyl)-5,5-diméthyl-3-(4-trifluorométhanesulfonyl-phényl)-imidazolidine-2,4-dione
- le trifluoroacétate de 4-quinolin-4-ylméthyl-6-(4-trifluorométhoxy-phényl)-4,6-diaza-spiro[2.4]heptane-5,7-dione
- le trifluoroacétate de 4-(3-méthyl-pyridin-4-ylméthyl)-6-(4-trifluorométhylsulfanylphényl)-4,6-diaza-spiro[2.4] heptane-5,7-dione
- le trifluoroacétate de 4-(3-hydroxy-pyridin-4-ylméthyl)-6-(4-trifluorométhoxy-phényl)-4,6-diaza-spiro[2.4]heptane-5,7-dione
- le trifluoroacétate de 4-(3-Hydroxy-pyridin-4-ylméthyl)-6-(4-trifluorométhylsulfanyl-phényl)-4,6-diaza-spiro[2.4]heptane-5,7-dione
- le trifluoroacétate de 4-(3-hydroxy-pyridin-4-ylméthyl)-6-(4-trifluorométhanesulfonyl-phényl)-4,6-diaza-spiro[2.4]heptane-5,7-dione
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

48. Produits de formule (I) telle que définie à l'une quelconque des revendications dont les noms suivent :
- le trifluoroacétate de la {4-[5,5-diméthyl-2,4-dioxo-3-(4-trifluorométhylsulfanyl-phényl)-imidazolidin-1-ylméthyl]-pyridin-2-yl}-amide de l'acide cyclopropanecarboxylique ;
- le 5,5-diméthyl-1-[2-(pyridin-2-ylamino)-pyridin-4-yl-méthyl]-3-(4-trifluorométhylsulfanyl-phényl)-imidazolidine-2,4-dione ; composé avec de l'acide trifluoroacétique ;
- le N-{4-[5,5-diméthyl-2,4-dioxo-3-(4-trifluorométhylsulfanyl-phényl)-imidazolidin-1-ylméthyl]-pyridin-2-yl}-isobutyramide ; composé avec de l'acide trifluoroacétique ;
- le N-{4-[5,5-diméthyl-2,4-dioxo-3-(4-trifluorométhylsulfanyl-phényl)-imidazolidin-1-ylméthyl]-pyridin-2-yl}-propionamide ; composé avec de l'acide trifluoroacétique ;
- le chlorhydrate de 1-(2-amino-pyridin-4-ylméthyl)-5,5-diméthyl-3-(4-trifluorométhylsulfanyl-phényl)-imidazolidine-2,4-dione ;
- le trifluoroacétate de la {4-[5,5-diméthyl-2,4-dioxo-3-(4-trifluorométhylsulfanyl-phényl)-imidazolidin-1-ylméthyl]-pyridin-2-yl}-amide de l'acide pyridine-2-carboxylique ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-piperidin-1-yl-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-[4-(2-hydroxy-éthyl)-piperazin-1-yl]-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-morpholin-4-yl-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-pyrrolidin-l-yl-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-(4-méthyl-piperazin-1-yl)-propionamide ;
- le 1-{4-[5,5-diméthyl-2,4-dioxo-3-(4-trifluorométhylsulfanyl-phényl)-imidazolidin-1-ylméthyl]-pyridin-2-yl}-3-phényl-urée ;
- le 1-[2-(6-éthyl-pyridin-2-ylamino)-pyridin-4-ylméthyl]-5,5-diméthyl-3-(4-trifluorométhylsulfanyl-phényl)-imidazolidine-2,4-dione ;
- le 5,5-diméthyl-1-[2-(4-méthyl-pyridin-2-ylamino)-pyridin-4-ylméthyl]-3-(4-trifluorométhylsulfanyl-phényl)-imidazolidine-2,4-dione ;
- le 5,5-diméthyl-1-[2-(6-méthyl-pyridin-2-ylamino)-pyridin-4-ylméthyl]-3-(4-trifluorométhylsulfanyl-phényl)-imi-dazolidine-2,4-dione ;
- le 1-[2-(4,6-diméthyl-pyridin-2-ylamino)-pyridin-4-yl-méthyl]-5,5-diméthyl-3-(4-trifluorométhylsulfanyl-phényl)-imidazolidine-2,4-dione ;
- le 1-[2-(3,5-dichloro-pyridin-2-ylamino)-pyridin-4-yl-méthyl]-5,5-diméthyl-3-(4-trifluorométhylsulfanyl-phényl)-imidazolidine-2,4-dione :
- le 5,5-diméthyl-1-[2-(pyridin-4-ylamino)-pyridin-4-yl-méthyl]-3-(4-trifluorométhylsulfanyl-phényl)-imidazolidine-2,4-dione ;
- le 5,5-diméthyl-1-[2-(pyridin-3-ylamino)-pyridin-4-yl-méthyl]-3-(4-trifluorométhylsulfanyl-phényl)-imidazolidine-2,4-dione ;
- le N-{4-[5,5-diméthyl-2,4-dioxo-3-(4-trifluorométhylsulfanyl-phényl)-imidazolidin-1-ylméthyl]-pyridin-2-yl}-3-(2-oxo-azépan-1-yl)-propionamide ;
- le 3-(benzyl-méthyl-amino)-N-{4-[5,5-diméthyl-2,4-dioxo-3-(4-trifluorométhylsulfanyl-phényl)-imidazolidin-1-ylméthyl]-pyridin-2-yl}-propionamide ;
- l'acide 4,5-diacétoxy-6-acétoxyméthyl-2-(3-(4-[5,5-diméthyl-2,4-dioxo-3-(4-trifluorométhylsulfanyl-phényl)-imidazolidin-1-ylméthyl]-pyridin-2-yl}-thiouréido acétique ;
- le 5,5-diméthyl-1-[2-(5-méthyl-pyridin-2-ylamino)-pyridin-4-ylméthyl]-3-(4-trifluorométhylsulfanyl-phényl)-imidazolidine-2,4-dione ;
- le trifluoroacétate de N-{4-[5,5-diméthyl-2,4-dioxo-3-(4-trifluorométhyl-sulfanyl-phényl)-imidazolidin-1-ylméthyl]-pyridin-2-yl}-3,5-diméthoxy-benzamide ;
- le trifluoroacétate de 5,5-diméthyl-1-[2-(pyrazin-2-yl-amino)-pyridin-4-ylméthyl]-3-(4-trifluorométhylsulfanyl-phényl)-imidazolidine-2,4-dione ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-(3-méthyl-piperidin-1-yl)-propionamide ;
- le trifluoroacétate de N-{4-[5,5-diméthyl-2,4-dioxo-3-(4-trifluorométhylsulfanyl-phényl)-imidazolidin-1-ylméthyl]-pyridin-2-yl}-3-(3,5-diméthyl-piperidin-1-yl)-propionamide ;
- le trifluoroacétate de N-{9-[5,5-diméthyl-2,4-dioxo-3-(4-trifluorométhylsulfanyl-phényl)-imidazolidin-1-ylméthyl]-pyridin-2-yl}-3-méthoxy-benzamide ;
- le trifluoroacétate de la {4-[5,5-diméthyl-2,4-dioxo-3-(4-trifluorométhylsulfanyl-phényl)-imidazolidin-1-ylméthyl]-pyridin-2-yl}-amide de l'acide pyrazine-2-carboxylique :
- le trifluoroacétate de la {4-[5,5-diméthyl-2,4-dioxo-3-(4-trifluorométhylsulfanyl-phényl)-imidazolidin-1-ylméthyl]-pyridin-2-yl}-amide de l'acide thiophène-2-carboxylique ;
- le N-{4-[5,5-diméthyl-2,4-dioxo-3-(4-trifluorométhylsulfanyl-phényl)-imidazolidin-1-ylméthyl]-pyridin-2-yl}-4-méthyl-benzamide ; composé avec de l'acide trifluoroacétique ;
- le 1-isoquinolin-5-yl-5,5-diméthyl-3-(4-trifluorométhylsulfanyl-phényl)-imidazolidine-2,4-dione ;
- le 3-(4-acétyl-piperazin-1-yl)-N-{4-[5,5-diméthyl-2,4-dioxo-3-(4-trifluorométhylsulfanyl- phényl)-imidazolidin-1-ylméthyl]-pyridin-2-yl}-propionamide ;
- le 3-[4-(2-diéthylamino-éthyl)-piperazin-1-yl]-N-{4-[5,5-diméthyl-2,4-dioxo-3-(4-trifluorométhylsulfanyl-phényl)-imidazolidin-1-ylméthyl]-pyridin-2-yl}-propionamide ;
- le N-{4-[5,5-diméthyl-2,4-dioxo-3-(4-trifluorométhylsulfanyl-phényl)-imidazolidin-1-ylméthyl]-pyridin-2-yl}-3-(2,6-diméthyl-morpholin-4-yl)-propionamide ;
- le N-{4-[5,5-diméthyl-2,4-dioxo-3-(4-trifluorométhylsulfanyl-phényl)-imidazolidin-1-ylméthyl]-pyridin-2-yl}-3-(4-pyrrolidin-1-yl-piperidin-1-yl)-propionamide ;
- le N-{4-[5,5-diméthyl-2,4-dioxo-3-(4-trifluorométhylsulfanyl-phényl)-imidazolidin-1-ylméthyl]-pyridin-2-yl}-2-(4-pyrrolidin-1-yl-piperidin-1-yl)-acétamide ;
- le 5,5-diméthyl-1-[2-(4-méthyl-pyridin-3-ylamino)-pyridin-4-ylméthyl]-3-(4-trifluorométhylsulfanyl-phényl)-imidazolidine-2,4-dione ;
- le 5,5-diméthyl-1-[2-(6-morpholin-4-yl-pyridin-3-yl-amino)-pyridin-4-ylméthyl]-3-(4-trifluorométhylsulfanyl-phényl)-imidazolidine-2,4-dione ;
- le 1-[2-(2,6-diméthyl-pyridin-3-ylamino)-pyridin-4-yl-méthyl]-5,5-diméthyl-3-(4-trifluorométhylsulfanyl-phényl)-imidazolidine-2,4-dione ;
- l'ester méthylique de l'acide 5-{4-[5,5-diméthyl-2,4-dioxo-3-(4-trifluorométhyl-sulfanyl-phényl)-imidazolidin-1-ylméthyl]-pyridin-2-ylamino}-pyridine-2-carboxylique ;
- le 1-[2-(2,6-diméthoxy-pyridin-3-ylamino)-pyridin-4-ylméthyl]-5,5-diméthyl-3-(4-trifluorométhylsulfanyl-phényl)-imidazolidine-2,4-dione ;
- le 1-[2-(6-fluoro-pyridin-3-ylamino)-pyridin-4-ylméthyl]-5,5-diméthyl-3-(4-trifluorométhylsulfanyl-phényl)-imidazolidine-2,4-dione ;
- le 1-[2-(6-méthoxy-pyridin-3-ylamino)-pyridin-4-ylméthyl]-5,5-diméthyl-3-(4-trifluorométhylsulfanyl-phényl)-imidazolidine-2,4-dione,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

49. A titre de médicaments, les produits de formule (I) telle que définie aux revendications 1 à **48,** lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

50. A titre de médicaments, les produits de formule (IC) telle que définie aux revendications précédentes, lesdits produits de formule (IC) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (IC).

51. A titre de médicaments, les produits de formule (IA) telle que définie aux revendications précédentes, lesdits produits de formule (IA) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (IA).

52. A titre de médicaments, les produits telle que définie à la revendication **47** ou **48** ainsi que les sels d'addition avec les acides minéraux et organiques ou avec les bases minérales et organiques pharmaceutiquement acceptables de ces produits.

53. Les compositions pharmaceutiques contenant à titre de principe actif, l'un au moins des médicaments tels que définis aux revendications **49** à **52.**

54. Les compositions pharmaceutiques contenant à titre de principe actif, l'un au moins des médicaments tels que définis à la revendication **47** ou **48.**

55. Compositions pharmaceutiques telles que définies aux revendications contenant en plus, des principes actifs d'autres médicaments de chimiothérapie contre le cancer.

56. Compositions pharmaceutiques selon l'une quelconque des revendications **caractérisées en ce qu'**elles sont utilisées comme médicaments, en particulier pour la chimiothérapie de cancers.

57. Utilisation de produits de formule (I) tels que définis à l'une quelconque des revendications, ou de sels pharmaceutiquement acceptables desdits produits de formule (I) pour la préparation de médicaments destinés à inhiber l'activité de protéines kinases et notamment d'une protéine kinase.

58. Utilisation de produits de formule (I) telle que définie à la revendication précédente ou de sels pharmaceutiquement acceptables desdits produits de formule (I) dans laquelle la protéine kinase est une protéine tyrosine kinase.

59. Utilisation de produits de formule (I) telle que définie à l'une quelconque des revendications, ou de sels pharmaceutiquement acceptables desdits produits de formule (I) dans laquelle la protéine kinase est choisie dans le groupe suivant: EGFR, Fak, FLK-1, FGFR1, FGFR2, FGFR3, FGFR4, FGFR5, flt-1, IGF-1R, KDR, PLK, PDGFR, tie2, VEGFR, AKT, Raf.

60. Utilisation de produits de formule (I) telle que définie à l'une quelconque des revendications, ou de sels pharmaceutiquement acceptables desdits produits de formule (I) dans laquelle la protéine kinase est IGF1R.

61. Utilisation de produits de formule (I) telle que définie à l'une quelconque des revendications, ou de sels pharmaceutiquement acceptables desdits produits de formule (I) dans laquelle la protéine kinase est FAK.

62. Utilisation de produits de formule (I) telle que définie à l'une quelconque des revendications, ou de sels pharmaceutiquement acceptables desdits produits de formule (I) dans laquelle la protéine kinase est AKT.

63. Utilisation de produits de formule (I) telle que définie à l'une quelconque des revendications, ou de sels pharmaceutiquement acceptables desdits produits de formule (I) dans laquelle la protéine kinase est dans une culture cellulaire.

64. Utilisation de produits de formule (I) telle que définie à l'une quelconque des revendications, ou de sels pharmaceutiquement acceptables desdits produits de formule (I) dans laquelle la protéine kinase est dans un mammifère.

65. Utilisation de produits de formule (I) telle que définie à l'une quelconque des revendications ou de sels pharmaceutiquement acceptables desdits produits de formule (I) pour la préparation d'un médicament destiné à prévenir ou traiter une maladie **caractérisée par** le dérèglement de l'activité d'une protéine kinase.

66. Utilisation de produits de formule (I) selon la revendication précédente dans laquelle la maladie à prévenir ou traiter est chez un mammifère.

67. Utilisation de produits de formule (I) telle que définie à l'une quelconque des revendications ou de sels pharmaceutiquement acceptables desdits produits de formule (I) pour la préparation d'un médicament destiné à prévenir ou traiter une maladie appartenant au groupe suivant: désordres de la prolifération de vaisseaux sanguins, désordres fibrotiques, désordres de la prolifération de cellules mésangiales, désordres métaboliques, allergies, asthme, thromboses, maladies du système nerveux, rétinopathies, psoriasis, arthrite rhumatoïde, diabètes, dégénération musculaire, maladies en oncologie, cancers.

68. Utilisation de produits de formule (I) telle que définie à l'une quelconque des revendications ou de sels pharmaceutiquement acceptables desdits produits de formule (I) pour la préparation d'un médicament destiné à traiter des maladies en oncologie.

69. Utilisation de produits de formule (I) telle que définie à l'une quelconque des revendications ou de sels pharmaceutiquement acceptables desdits produits de formule (I) pour la préparation d'un médicament destiné à traiter des cancers.

70. Utilisation de produits de formule (I) selon la revendication précédente dans laquelle la maladie à traiter est un cancer de tumeurs solides.

71. Utilisation de produits de formule (I) selon la revendication précédente dans laquelle la maladie à traiter est un cancer résistant aux agents cytotoxiques.

72. Utilisation de produits de formule (I) telle que définie à l'une quelconque des revendications ou de sels pharmaceutiquement acceptables desdits produits de formule (I) pour la préparation d'un médicament destiné à traiter des cancers parmi lesquels les cancers du sein, de l'estomac, du colon, des poumons, des ovaires, de l'utérus, du cerveau, du rein, du larynx, du système lymphatique, de la thyroïde, du tractus uro-génital, du tractus incluant vésicule et prostate, du cancer des os, du pancréas, les mélanomes.

73. Utilisation de produits de formule (I) selon la revendication précédente dans laquelle la maladie à traiter est un cancer du sein, du colon ou des poumons.

74. Utilisation de produits de formule (I) telle que définie à l'une quelconque des revendications ou de sels pharmaceutiquement acceptables desdits produits de formule (I) pour la préparation d'un médicament destiné à la chimiothérapie de cancers.

75. Utilisation de produits de formule (I) telle que définie à l'une quelconque des revendications ou de sels pharmaceutiquement acceptables desdits produits de formule (I) pour la préparation de médicaments destinés à la chimiothérapie de cancers utilisés seuls ou en association.

76. Utilisation de produits de formule (I) telle que définie à l'une quelconque des revendications ou de sels pharmaceutiquement acceptables desdits produits de formule (I) pour la préparation de médicaments destinés à être utilisés seuls ou en association avec chimiothérapie ou radiothérapie ou alternativement en association avec d'autres agents thérapeutiques.

77. Utilisation de produits de formule (I) selon la revendication précédente dans laquelle les agents thérapeutiques peuvent être des agents anti-tumoraux utilisés communément.

78. Produits de formule (I) tels que définis à l'une quelconque des revendications comme inhibiteurs de protéines kinases, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I) ainsi que leurs prodrugs.

79. Produits de formule (I) tels que définis à l'une quelconque des revendications comme inhibiteurs de IGF1R.

80. Produits de formule (IA) tels que définis aux revendications comme inhibiteurs de IGF1R.

## Claims

1. Products of formula (I): 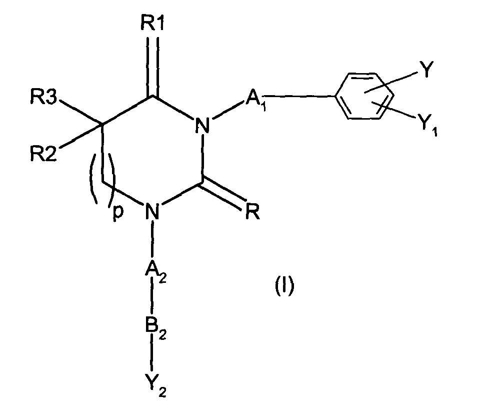 in which p represents an integer from 0 to 2,
R and R1, which may be identical or different, represent O or NH,
R2 and R3, which may be identical or different, represent hydrogen, alkyl, alkenyl, alkynyl, cycloalkyl, aryl and heteroaryl which are optionally substituted, or alternatively R2 and R3 form, together with the carbon atom to which they are attached, a carbocyclic or heterocyclic radical, these radicals being 3- to 10-membered and the heterocyclic radical containing one or more hetero atoms chosen from O, S, N and NR7, all these radicals optionally being substituted,
A1 represents a single bond, an alkyl radical or an alkenyl or alkynyl radical,
Y and Y1, which may be identical or different, are such that one from among Y and Y1 is chosen from OCF3, -O-CF2-CHF2, -O-CHF2, -O-CH2-CF3, -S(O)nCF3, -S-CF2-CF2-CF3, S(O)n-Alk, -S-Alk-O-Alk, -S-Alk-OH, -S-Alk-CN, S-Alk-heterocycloalkyl (description: morpholino, pyrrolidinyl, piperazinyl, optionally substituted with alk), -S02CHF2, -SO2CF2CF3, -SO2NR5R6 and -SF5, with Alk representing an alkyl radical containing from 1 to 4 carbon atoms, and the other from among Y and Y1 is chosen from the following values: hydrogen, halogen, nitro, NR5R6, free or esterified carboxyl and CONR5R6
or alternatively the phenyl radical forms with its substituents Y and Y1 one of the following radicals: 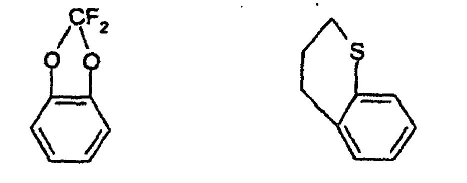 the radical thus formed being optionally substituted with one or more alkyl radicals that are themselves optionally substituted,
R5 and R6, which may be identical or different, are chosen from hydrogen, alkyl, alkenyl, alkynyl, cycloalkyl, cycloalkenyl, heterocycloalkyl, aryl and heteroaryl, which are optionally substituted, or alternatively R5 and R6 form, with the nitrogen atom to which they are attached, a 3- to 10-membered heterocyclic radical containing one or more hetero atoms chosen from O, S, N and NR7, which is optionally substituted, A2, which may be identical to or different from A1, represents the values of A1 and CO and SO2,
B2 represents a saturated or unsaturated monocyclic or bicyclic heterocyclic radical containing 1 or more identical or different hetero atoms chosen from O, S and NR7, optionally substituted with one or more identical or different substitutents chosen from the values of Y2,
R7 represents a hydrogen atom or an alkyl, cycloalkyl, phenyl, acyl, S(O)2Alk, S(O)2Aryl, S(O)2heteroaryl or S(O)2NR5R6 radical,
Y2 represents hydrogen, halogen, hydroxyl, cyano, alkyl, alkoxy, cycloalkyl, heterocycloalkyl, aryl, heteroaryl, O-alkenyl, O-alkynyl, -O-cycloalkyl, -S(O)n-alkyl, -S(O)n-alkenyl, -S(O)n-alkynyl, S(O)n-cycloalkyl, COOR13, -OCOR13, NR5R6, CONR5R6, S(O)n-R5R6, -NR10-CO-Rl3, -NR10-SO2-R13, NH-SO2-NR5R6, -NR10-CO-NR5R6, -NR10-CS-NR5R6, -NR10-COOR13, all these radicals optionally being substituted,
all the alkyl, alkenyl, alkynyl and alkoxy radicals above being linear or branched and containing not more than 6 carbon atoms,
all the cycloalkyl and heterocycloalkyl radicals above containing not more than 7 carbon atoms,
all the aryl and heteroaryl radicals above containing not more than 10 carbon atoms,
all the carbocyclic and heterocyclic alkyl, alkenyl, alkynyl, alkoxy, cycloalkyl, heterocycloalkyl, aryl and heteroaryl radicals above and also the ring formed by R5 and R6 with the atom to which they are attached optionally being substituted with one or more radicals, which may be identical or different, chosen from halogen atoms and cyano, hydroxyl, alkoxy, CF3, nitro, aryl, heteroaryl, -C(=O)-OR9, -C(=O)-R8, -NR11R12, -C(=O)-NR11R12, -N(RlO)-C(=O)-R8, -N(R10)-C(=O)-OR9, N(R10)-C(=O)-NR11R12, -N(R10)-S(O)n-R8, -S(O)n-R8, -N(R10)-S(O)n-NR11R12 or -S(O)n-NR11R12 radicals,
all the aryl and heteroaryl radicals above furthermore being optionally substituted with one or more radicals chosen from alkyl, alkoxy and alkylenedioxy radicals,
all the above cyclic radicals and also the ring formed by R5 and R6 with the atom to which they are attached moreover being optionally substituted with one or more radicals chosen from oxo and thioxo;
n represents an integer from 0 to 2,
R8 represents alkyl, alkenyl, cycloalkyl, cycloalkylalkyl, heterocycloalkyl, heterocycloalkylalkyl, aryl, arylalkyl, heteroaryl and heteroarylalkyl,
R9 represents the values of R8 and hydrogen,
R10 represents hydrogen or alkyl,
R11 and R12, which may be identical or different, represent hydrogen, C3-C6 cycloalkyl, C1-C4 alkyl and phenyl, optionally substituted with one or more radicals, which may be identical or different, chosen from halogen atoms and cyano, hydroxyl, alkoxy, CF3, nitro, phenyl and free, salified, esterified or amidated carboxyl radicals, or alternatively R11 and R12 form, with the nitrogen atom to which they are attached, a 5- to 7-membered cyclic radical containing one or more hetero atoms chosen from O, S, N and NR7 and preferably a cyclic amine,
R13, which may be identical to or different from R5 or R6, being chosen from the values of R5 or R6,
it being understood that the products of formula (I) are as defined below from a) to d) :
a) when p represents the integer 0, R represents oxygen, R1 represents oxygen, A1 represents a single bond or alkyl, Y and Y1, which may be identical or different, are such that at least one represents OCF3 or Salk, A2 represents a single bond or alkyl and B2 represents an optionally substituted heterocyclic radical, then R2 and R3 do not represent one hydrogen and the other imidazolylalkyl,
b) when p represents the integer 0, R and R1 represent oxygen, A1 represents a single bond or alkyl, Y and Y1, which may be identical or different, are such that at least one represents OCF3, SOAlk, S(O)2alk or SO2NH2, A2 represents CH2 and B2 represents an optionally substituted heterocyclic radical, then R2 and R3 do not represent one hydrogen and the other an alkyl chain optionally interrupted with O, S or Nalk always substituted with a hydroxamate -CO-NHOH
c) when p represents the integer 0, R and R1 represent oxygen, A1 represents a single bond or alkyl, Y and Y1, which may be identical or different, are such that at least one represents S(O)nAlk, A2 represents a single bond and B2 represents an optionally substituted 5- or 6-membered aromatic heterocyclic radical, then R2 and R3 are not chosen from hydrogen, alkyl, arylalkyl, aryl and heteroaryl,
d) when p represents an integer from 0 to 2, R and R1 represent oxygen, A1 represents a single bond, Y and Y1, which may be identical or different, are such that one represents SO2Alk or SO2NH2 and the other represents NR5R6, A2 represents a single bond or alkylene and B2 represents an optionally substituted 5- to 10-membered heterocyclic radical, then R2 and R3 do not both represent hydrogen,
it being understood that the above radicals have the following meanings:
- the term alkyl radical, alk, Alk or ALK denotes a linear or branched radical containing not more than 12 carbon atoms, chosen from methyl, ethyl, propyl, isopropyl, butyl, isobutyl, sec-butyl, tert-butyl, pentyl, isopentyl, sec-pentyl, tert-pentyl, neopentyl, hexyl, isohexyl, sec-hexyl, tert-hexyl, and also heptyl, octyl, nonyl, decyl, undecyl and dodecyl radicals, and also the linear or branched positional isomers thereof,
- the term alkenyl radical denotes a linear or branched radical containing not more than 12 carbon atoms and preferentially 4 carbon atoms, chosen, for example, from the following values: ethenyl or vinyl, propenyl or allyl, 1-propenyl, n-butenyl, i-butenyl, 3-methyl-2-butenyl, n-pentenyl, hexenyl, heptenyl, octenyl, cyclohexylbutenyl and decenyl, and also the linear or branched positional isomers thereof,
- the term alkynyl radical denotes a linear or branched radical containing not more than 12 carbon atoms and preferentially 4 carbon atoms, chosen, for example, from the following values: ethynyl, propynyl or propargyl, butynyl, n-butynyl, i-butynyl, 3-methyl-2-butynyl, pentynyl or hexynyl, and also the linear or branched positional isomers thereof,
- the term cycloalkyl radical denotes a monocyclic or bicyclic carbocyclic radical containing from 3 to 10 members and especially denotes cyclopropyl, cyclobutyl, cyclopentyl and cyclohexyl radicals,
- the term aryl radical denotes unsaturated radicals, which are monocyclic or which are formed from condensed carbocyclic rings. Examples of such aryl radicals that may be mentioned include phenyl or naphthyl radicals,
- the term heterocyclic radical denotes a saturated carbocyclic radical (heterocycloalkyl) or unsaturated carbocyclic radical (heteroaryl) formed from not more than 6 members interrupted with one or more heteroatoms, which may be identical or different, chosen from oxygen, nitrogen or sulfur atoms,
heterocycloalkyl radicals that may especially be mentioned include dioxolane, dioxane, dithiolane, thiooxolane, thiooxane, oxiranyl, oxolanyl, dioxolanyl, piperazinyl, piperidyl, pyrrolidinyl, imidazolidinyl, pyrazolidinyl, morpholinyl or alternatively tetrahydrofuryl, tetrahydrothienyl, chromanyl, dihydrobenzofuryl, indolinyl, piperidyl, perhydropyranyl, pyrindolinyl, tetrahydroquinolyl, tetrahydroisoquinolyl or thioazolidinyl radicals, all these radicals being optionally substituted,
- among the 5-membered heteroaryl radicals that may be mentioned are furyl radicals such as 2-furyl, thienyl radicals such as 2-thienyl and 3-thienyl, pyrrolyl, diazolyl, thiazolyl, thiadiazolyl, thiatriazolyl, isothiazolyl, oxazolyl, oxadiazolyl, 3- or 4-isoxazolyl, imidazolyl, pyrazolyl or isoxazolyl radicals,
among the 6-membered heteroaryl radicals that may especially be mentioned are pyridyl radicals such as 2-pyridyl, 3-pyridyl and 4-pyridyl, pyrimidyl, pyrimidinyl, pyridazinyl, pyrazinyl and tetrazolyl,
- as fused heteroaryl radicals containing at least one heteroatom chosen from sulfur, nitrogen and oxygen, examples that may be mentioned include benzothienyl such as 3-benzothienyl, benzofuryl, benzofuranyl, benzopyrrolyl, benzimidazolyl, benzoxazolyl, thionaphthyl, indolyl, purinyl, quinolyl, isoquinolyl and naphthyridinyl,
said products of formula (I) being in any possible racemic, enantiomeric or diastereoisomeric isomer form, and also the addition salts with mineral and organic acids or with mineral and organic bases of said products of formula (I).

2. Products of formula (I) as defined in Claim 1, in which one from among Y and Y1 represent a hydrogen atom and the other is chosen from OCF3, S(O)nCF3, S(O)nAlk, SO2CHF2, SO2CF2CF3 and SO2NR5R6,
the other substituents of the said products of formula (I) being chosen from the values defined in Claim 1 and it being understood that:
a) when p represents the integer 0, R represents oxygen, R1 represents oxygen, A1 represents a single bond or alkyl, Y and Y1, which may be identical or different, are such that one represents hydrogen and the other represents OCF3 or Salk, A2 represents a single bond or alkyl and B2 represents an optionally substituted heterocyclic radical, then R2 and R3 do not represent one hydrogen and the other imidazolylalkyl,
b) when p represents the integer 0, R and R1 represent oxygen, A1 represents a single bond or alkyl, Y and Y1, which may be identical or different, are such that one represents hydrogen and the other represents OCF3, SOAlk, S(O)2alk or SO2NH2, A2 represents CH2 and B2 represents an optionally substituted heterocyclic radical, then R2 and R3 do not represent one hydrogen and the other an alkyl chain optionally interrupted with O, S or Nalk always substituted with a hydroxamate -CO-NHOH
c) when p represents the integer 0, R and R1 represent oxygen, A1 represents a single bond or alkyl, Y and Y1, which may be identical or different, are such that one represents hydrogen and the other represents S(O)nAlk, A2 represents a single bond and B2 represents an optionally substituted 5- or 6-membered aromatic heterocyclic radical, then R2 and R3 are not chosen from hydrogen,
alkyl, arylalkyl, aryl and heteroaryl,
said products of formula (I) being in any possible racemic, enantiomeric or diastereoisomeric isomer form, and also the addition salts with mineral and organic acids or with mineral and organic bases of said products of formula (I).

3. Products of formula (I) as defined in any one of the claims, in which one from among Y and Y1 represents a hydrogen atom and the other is chosen from S(O)nCF3, SOAlk, S(O)2Alk, SO2CHF2, SO2CF2CF3 and SO2NR5R6,
the other substituents of said products of formula (I) being chosen from the values defined in Claim 1 and it being understood that the products of formula (I) are as defined below in a) and b):
a) when p represents the integer 0, R and R1 represent oxygen, A1 represents a single bond or alkyl, Y and Y1, which may be identical or different, are such that one represents hydrogen and the other represents SOAlk, S(O)2alk or SO2NH2, A2 represents CH2 and B2 represents an optionally substituted heterocyclic radical, then R2 and R3 do not represent one hydrogen and the other an alkyl chain optionally interrupted with O, S or Nalk always substituted with a hydroxamate -CO-NHOH
b) when p represents the integer 0, R and R1 represent oxygen, A1 represents a single bond or alkyl, Y and Y1, which may be identical or different, are such that one represents hydrogen and the other represents SoAlk or S(O)2Alk, A2 represents a single bond and B2 represents an optionally substituted 5- or 6-membered aromatic heterocyclic radical, then R2 and R3 are not chosen from hydrogen, alkyl, arylalkyl, aryl and heteroaryl,
said products of formula (I) being in any possible racemic, enantiomeric or diastereoisomeric isomer form, and also the addition salts with mineral and organic acids or with mineral and organic bases of said products of formula (I).

4. Products of formula (I) as defined in any one of the claims, in which one from among Y and Y1 represents a hydrogen atom and the other is chosen from S(O)nCF3, S02CHF2, SO2CF2CF3 and SO2NR5R6,
the other substituents of said product of formula (I) being chosen from the values defined in claim 1 and it being understood that when p represents the integer 0, R and R1 represent oxygen, A1 represents a single bond or alkyl, Y and Y1, which may be identical or different, are such that one represents hydrogen and the other represents SO2NH2, A2 represents CH2 and B2 represents an optionally substituted heterocyclic radical, then R2 and R3 do not represent one hydrogen and the other an alkyl chain optionally interrupted with O, S or Nalk, always substituted with a hydroxamate -CO-NHOH
said products of formula (I) being in any possible racemic, enantiomeric or diastereoisomeric isomer form, and also the addition salts with mineral and organic acids or with mineral and organic bases of said products of formula (I).

5. Products of formula (I) as defined in any one of the claims, in which one from among Y and Y1 represents a hydrogen atom and the other is chosen from S(O)nCF3, S02CHF2 and SO2CF2CF3,
the other substituents of said product of formula (I) being chosen from the values defined in claim 1,
said products of formula (I) being in any possible racemic, enantiomeric or diastereoisomeric isomer form, and also the addition salts with mineral and organic acids or with mineral and organic bases of said products of formula (I).

6. Products of formula (I) as defined in any one of the claims, such that all the alkyl, alkenyl, alkynyl, cycloalkyl, heterocycloalkyl, aryl or heteroaryl radicals defined in claim 1 are optionally substituted with one or more radicals, which may be identical or different, chosen from halogen, cyano, hydroxyl, alkoxy, CF3, nitro, phenyl, carboxyl which is free, salified, esterified with an alkyl radical or amidated with a radical NR11aR12a, -C(=O)-R9a, -NR11aRl2a, -C(=O)-NR11aR12a, -N(R10a)-C((=O)-R9a, -N(R10a)-C(=O)-OR8a, N(R10a)-C(=O)-NR11aR12a, - N(R10a)-S(O)n-R9a, -S(O)n-R9a, -N(R10a)-S(O)n-NRllaRl2a or -(O)n-NR11aR12a,
all the aryl and heteroaryl radicals above furthermore being optionally substituted with an ethylenedioxy radical,
R8a represents hydrogen, alkyl, alkenyl, phenyl, phenylalkyl, heteroaryl or heteroarylalkyl,
R9a represents alkyl, cycloalkyl, cycloalkylalkyl, heterocycloalkyl, heterocycloalkylalkyl, phenyl, phenylalkyl, heteroaryl or heteroarylalkyl,
R10a represents hydrogen or alkyl,
R11a and R12a, which may be identical or different represent
hydrogen, alkyl, cycloalkyl, cycloalkylalkyl, phenyl, phenylalkyl, optionally substituted with one or more substituents, which may be identical or different, chosen from halogen, hydroxyl, C1-C4 alkyl or C1-C4 alkoxy, or alternatively R11a and R12a form, with the nitrogen atom to which they are attached, a cyclic radical chosen from pyrrolidyl, piperidyl, piperazinyl, morpholinyl, indolinyl, pyrindolinyl, tetrahydroquinolyl, thiazolidinyl and naphthyridyl,
said products of formula (I) being in any possible racemic, enantiomeric or diastereoisomeric isomer form, and also the addition salts with mineral and organic acids or with mineral and organic bases of said products of formula (I).

7. Products of formula (I) as defined in any one of the claims, such that p represents the integer 0, the other substituents of said products of formula (I) having the values defined in any one of the claims.

8. Products of formula (I) as defined in any one of the claims, such that p represents the integer 1, the other substituents of said products of formula (I) having the values defined in any one of the claims.

9. Products of formula (I) as defined in any one of the claims, such that p represents the integer 2, the other substituents of said products of formula (I) having the values defined in any one of the claims.

10. Products of formula (I) as defined in any one of the claims, such that R1 represents 0, the other substituents of said products of formula (I) having the values defined in any one of the claims.

11. Products of formula (I) as defined in any one of the claims, such that R represents 0, the other substituents of said products of formula (I) having the values defined in any one of the claims.

12. Products of formula (I) as defined in any one of the claims, such that R2 and R3, which may be identical or different, represent hydrogen, alkyl, alkenyl, cycloalkyl, cycloalkylalkyl, phenyl, phenylalkyl, heterocycloalkyl, heterocycloalkylalkyl, heteroaryl and heteroarylalkyl, which are optionally substituted, or alternatively R2 and R3 form, together with the carbon atom to which they are attached, a carbocyclic or heterocyclic radical, these radicals being 3- to 10-membered and the heterocyclic radical containing one or more hetero atoms chosen from O, S, N and NR7b, all these radicals being optionally substituted,
all the above radicals being optionally substituted with one or more radicals chosen from halogen, cyano, hydroxyl, alkyl and alkoxy containing 1 to 4 carbon atoms, CF3, nitro, phenyl, carboxyl which is free, salified, esterified with an alkyl radical or amidated with a radical NR11bRl2b, -C(=O)-R9b, -NR11bR12b and -C(=O)-NR11bR12b,
R7b represents a hydrogen atom, an alkyl radical or a phenyl radical,
R9 represents hydrogen, alkyl, cycloalkyl, cycloalkylalkyl or phenyl,
R11b and R12b, which may be identical or different, represent hydrogen, alkyl, cycloalkyl or phenyl, or alternatively R11b and R12b form, with the nitrogen atom to which they are attached, an optionally substituted piperazinyl radical,
the other substituents of said products of formula (I) having the values defined in any one of the claims.

13. Products of formula (I) as defined in any one of the claims, such that R2 and R3, which may be identical or different, are chosen from hydrogen, alkyl, phenylalkyl, pyridylalkyl and thienylbenzothienylalkyl, which are optionally substituted with one or more radicals chosen from halogen atoms and hydroxyl, alkyl and alkoxy radicals containing from one to 4 carbon atoms, or alternatively R2 and R3 form, together with the carbon atom to which they are attached, a 3- to 6-membered cycloalkyl or heterocycloalkyl radical containing a nitrogen atom, the other substituents of said products of formula (I) having the values defined in any one of the claims.

14. Products of formula (I) as defined in any one of the claims, such that R2 and R3, which may be identical or different, are chosen from hydrogen, alkyl, hydroxyalkyl, phenylalkyl, hydroxyphenylalkyl, pyridylalkyl or thienylbenzothienylalkyl, or alternatively R2 and R3 form, together with the carbon atom to which they are attached, a cycloalkyl radical containing from 3 to 6 carbon atoms or an azetidinyl, pyrrolidyl or piperidyl radical,
the other substituents of said products of formula (I) having the values defined in any one of the claims.

15. Products of formula (I) as defined in any one of the claims, such that R2 and R3, which may be identical or different, are chosen from hydrogen, alkyl, hydroxyalkyl, phenylalkyl and hydroxyphenylalkyl, or alternatively R2 and R3 form, together with the carbon atom to which they are attached, a cycloalkyl radical containing from 3 to 6 carbon atoms.

16. Products of formula (I) as defined in any one of the claims, such that one from among R2 and R3 is chosen from hydrogen and alkyl, and the other from among R2 and R3 is chosen from all the values of R2 and R3, or alternatively R2 and R3 form, together with the carbon atom to which they are attached, a cycloalkyl radical containing from 3 to 6 carbon atoms,
the other substituents of said products of formula (I) having the values defined in any one of the claims.

17. Products of formula (I) as defined in any one of the claims, such that R2 and R3, which may be identical or different, represent hydrogen and alkyl, or alternatively R2 and R3 form, together with the carbon atom to which they are attached, a cycloalkyl radical containing from 3 to 6 carbon atoms,
the other substituents of said products of formula (I) having the values defined in any one of the claims.

18. Products of formula (I) as defined in any one of the claims, such that R2 and R3, which may be identical or different, represent hydrogen and CH3, or alternatively R2 and R3 form, together with the carbon atom to which they are attached, a cyclopropyl radical, the other substituents of said products of formula (I) having the values defined in any one of the claims.

19. Products of formula (I) as defined in any one of the claims, such that A1 represents a single bond and A2 is chosen from a single bond, a linear or branched alkyl radical containing not more than 6 carbon atoms and allyl, propynyl, C=O and SO2 radicals, the other substituents of said products of formula (I) having the values defined in any one of the claims.

20. Products of formula (I) as defined in any one of the claims, such that A1 represents a single bond and A2 is chosen from a single bond or alkyl, allyl, propynyl, C=O and SO2 radicals, the other substituents of said products of formula (I) having the values defined in any one of the claims.

21. Products of formula (I) as defined in any one of the claims, such that A1 represents a single bond and A2 represents an alkyl or C=O radical, the other substituents of said products of formula (I) having the values defined in any one of the claims.

22. Products of formula (I) as defined in any one of the claims, such that A1 represents a single bond and A2 represents C=O, -CH2-CH2- or -CH2, the other substituents of said products of formula (I) having the values defined in any one of the claims.

23. Products of formula (I) as defined in any one of the claims, such that A1 represents a single bond and A2 represents -CH2, the other substituents of said products of formula (I) having the values defined in any one of the claims.

24. Products of formula (I) as defined in any one of the claims, in which Y and Y1 are such that one represents a hydrogen atom, a halogen atom or an amino radical and the other is chosen from -OCF3, -O-CF2-CHF2, -O-CHF2, -O-CH2-CF3, -SF5, S(O)n-CF3, -S(O)n-Alk, -S02CHF2, S02CF2CF3, -S02NH2, -S-CF2-CF2-CF3, -S-Alk-O-Alk, -S-Alk-OH, -S-Alk-CN, -S-Alk-morpholino, -S-Alk-pyrrolidinyl and -S-Alk-piperazinyl, the morpholino, pyrrolidinyl and piperazinyl radicals being optionally substituted with Alk, with Alk representing an alkyl radical containing from 1 to 4 carbon atoms,
the other substituents of the said products of formula (I) having the values defined in any of the claims.

25. Products of formula (I) as defined in any one of the claims, such that Y represents a hydrogen atom and Y1 is chosen from -OCF3, S(O)n-CF3, S(O)n-CH3, SO2CHF2 and SO2-N(alk)2, the other substituents of said products of formula (I) having the values defined in any one of the claims.

26. Products of formula (I) as defined in any one of the claims, such that Y represents a hydrogen atom and Y1 is chosen from -OCF3, S(O)n-CF3 and SO2CHF2,
the other substituents of said products of formula (I) having the values defined in any one of the claims.

27. Products of formula (I) as defined in any one of the claims, such that Y represents a hydrogen atom and Y1 is chosen from -OCF3 and S(O)n-CF3,
the other substituents of said products of formula (I) having the values defined in any one of the claims.

28. Products of formula (I) as defined in any one of the claims, such that Y represents a hydrogen atom and Y1 is chosen from -OCF3, S-CF3 and S(O)2-CF3,
the other substituents of said products of formula (I) having the values defined in any one of the claims.

29. Products of formula (I) as defined in any one of the claims, such that B2 represents a monocyclic or bicyclic heteroaryl radical chosen from pyridyl, pyrimidinyl, quinolyl, azaindolyl, 1H-pyrrolo[2,3-b]pyridyl, quinazolyl, thiazolyl, imidazolyl, pyrazolyl, furazanyl, isoxazolyl, morpholinyl, pyrrolidinyl, furyl, piperidyl, thienyl, chromenyl, oxochromenyl, indolyl, pyrrolyl, purinyl, benzoxazinyl, benzimidazolyl and benzofuranyl radicals, these radicals being optionally substituted with one or more radicals chosen from the values of Y2, the other substituents of the said products of formula (I) having the values defined in any one of the claims.

30. Products of formula (I) as defined in any one of the claims, such that B2 represents a heteroaryl radical chosen from 3- or 4-pyridyl, pyrimidinyl, 3- or 4-quinolyl, azaindolyl, quinazolyl, thiazolyl, imidazolyl, pyrazolyl, furazanyl and isoxazolyl radicals,
these radicals being optionally substituted with one or more radicals chosen from the values of Y2,
the other substituents of said products of formula (I) having the values defined in any one of the claims.

31. Products of formula (I) as defined in any one of the claims, such that B2 represents a heteroaryl radical chosen from 3- or 4-pyridyl, pyrimidinyl, 3- or 4-quinolyl, azaindolyl and quinazolyl radicals, these radicals being optionally substituted with one or more radicals chosen from the values of Y2,
the other substituents of said products of formula (I) having the values defined in any one of the claims.

32. Products of formula (I) as defined in any one of the claims, such that B2 represents the 4-pyridyl, 4-quinolyl and 1H-pyrrolo[2,3-b]pyridin-4-yl radicals, optionally substituted with one or more radicals chosen from the values of Y2 defined in any one of the claims,
the other substituents of said products of formula (I) having the values defined in any one of the claims.

33. Products of formula (I) as defined in any one of the claims, such that Y2 represents hydrogen, halogen,
hydroxyl, cyano, alkyl, alkoxy, phenyl, COOH, COOAlk, CONR5R6, NR5R6, -NR10-COOR6, -NR10-CO-R6, -NR10-CS-NR5R6, -NR10-CO-NR5R6 or NR10-S02-R6, all these radicals being optionally substituted,
R5 and R6, which may be identical or different, are chosen from hydrogen, alkyl, cycloalkyl, phenyl and 5- to 6-membered heteroaryl radicals containing 1 to 3 heteroatoms chosen from O, N and S, all these radicals being optionally substituted, or alternatively R5 and R6 form with the nitrogen atom to which they are attached an optionally substituted pyrrolidinyl, piperidyl, piperazinyl, morpholinyl or quinazolinyl radical,
R10 represents hydrogen or alkyl,
all the above alkyl, alkoxy, cycloalkyl and phenyl radicals and also the ring formed by R5 and R6 with the atom to which they are attached being optionally substituted with one or more radicals, which may be identical or different, chosen from halogen atoms and cyano; hydroxyl; alkyl; alkoxy; OCF3; CF3; S(O)n-CF3; nitro; oxo; thioxo; OCOAlk and phenyl radicals, which phenyl radical is itself optionally substituted with one or more radicals chosen from halogen atoms and alkyl and alkoxy radicals; -OCOAlk, NH2, NHAlk, N(Alk)2, N(Alk) (phenylalkyl), N(Alk) (aminoalkyl), N(Alk) (alkylaminoalkyl), N(Alk) (dialkylaminoalkyl), carboxyl free or esterified with an alkyl radical,
all the above phenyl radicals moreover being optionally substituted with an alkylenedioxy radical,
all the above alkyl radicals moreover being optionally substituted with one or more saturated or partially unsaturated 4- to 7-membered heterocyclic radicals containing at least one nitrogen atom N and in addition 0 to 2 other heteroatoms chosen from O, N and S,
all the above pyrrolidinyl and quinazolinyl radicals moreover being optionally substituted with oxo or thioxo, all the above alkyl and alkoxy radicals being linear or branched and containing not more than 6 carbon atoms,
all the above cycloalkyl radicals containing not more than 7 carbon atoms,
the other substituents of the said products of formula (I) having the values defined in any one of the claims.

34. Products of formula (I) as defined in any one of the claims, such that Y2 represents hydrogen, halogen, hydroxyl, cyano, alkyl, alkoxy, phenyl, CONR5R6, NR5R6, -NR10-COOH, -NR10-COOAlk, -NR10-CO-R6, -NR10-CS-NR5R6, -NR10-CO-NR5R6 or -NR10-SO2-R6,
R5 and R6, which may be identical or different, are chosen from hydrogen,; alkyl; cycloalkyl; phenyl; pyrimidinyl; thienyl; pyridyl; quinolyl; thiazolyl optionally substituted with one or two halogen atoms; pyran optionally substituted with one or more OCOAlk; phenyl substituted with one or more radicals chosen from halogen atoms and alkyl, alkoxy, amino, alkylamino, dialkylamino and carboxyl radicals, which carboxyl radical is free or esterified with an alkyl radical; alkyl substituted with phenyl which is itself optionally substituted with one or more radicals chosen from halogen atoms, alkyl, alkoxy, amino, alkylamino, dialkylamino, carboxyl free or esterified with an alkyl radical; alkyl substituted with piperazinyl which is itself optionally substituted with one or more radicals chosen from Alk, Alk-OH and pyridyl; alkyl substituted with imidazolyl; alkyl substituted with one or more radicals chosen from NH2, NHAlk, N(Alk)2, N(Alk) (phenylalkyl), N(alk)(aminoalkyl), N(Alk) (alkylaminoalkyl) and N(Alk)(dialkylaminoalkyl); alkyl substituted with morpholinyl optionally substituted with one or two Alk; alkyl substituted with pyrrolidinyl; alkyl substituted with piperidyl which is itself optionally substituted with one or two Alk; alkyl substituted with thiomorpholinyl; alkyl substituted with azetidinyl; alkyl substituted with azepanyl optionally substituted with oxo,
or alternatively R5 and R6 form with the nitrogen atom to which they are attached a pyrrolidinyl; piperidyl; piperazinyl; morpholinyl; or quinazolinyl radical, all these radicals being optionally substituted with one or more radicals, which may be identical or different, chosen from halogen atoms and alkyl, hydroxyl and alkoxy radicals and phenyl itself optionally substituted with one or more radicals chosen from halogen atoms and alkyl and alkoxy radicals,
the pyrrolidinyl and quinazolinyl radicals moreover being optionally substituted with oxo or thioxo,
the piperazinyl radical itself optionally substituted with one or more radicals chosen from Alk, Alk-OH and pyridyl,
R10 represents hydrogen or alkyl,
a11 the above alkyl or Alk and alkoxy radicals being linear or branched and containing not more than 6 carbon atoms,
all the above cycloalkyl radicals containing not more than 7 carbon atoms,
all the phenyl radicals moreover being optionally substituted with a radical chosen from CF3, -OCF3, nitro and alkylenedioxy,
the other substituents of the said products of formula (I) having the values defined in any one of the claims.

35. Products of formula (I) as defined in any one of the claims, such that Y2 represents V1, halogen, hydroxyl, -C(=NH)NH2, OV1, O-CO-V1, COOV1, COV1, CO-NV1V2, -NV1V2, -NH-CO-V1, -NH-COO-V1, -NH-NH-CO-V1, -NV1-CO-NV1V2, -NV1-CO-NHV1, -NH-CO-NHV1, -NH-SO2-NHV1 and -NH-SO2-V1,
in which V1 and V2, which may be identical or different, represent a hydrogen atom, an alkyl, cycloalkyl or phenyl radical or a heterocyclic radical such as pyridinyl,
pyrazolyl, imidazolyl, dihydroimidazolyl, tetrazolyl, morpholinyl, piperazinyl, piperazinylalkyl, alkylpiperazinyl, phenylpiperazinyl, thienyle, furanyl, piperidyl, methylpiperidyl, pyridyl, pyrrolidyl and pyrrolidylalkyl,
all the alkyl, phenyl and heterocyclic radicals being optionally substituted with one or more radicals chosen from halogen atoms and hydroxyl, alkyl, alkoxy, CF3, NH2, NHalk, N(alk)2 radicals and a phenyl radical, itself optionally substituted with one or more substituents chosen from halogen atoms and hydroxyl or alkoxy radicals,
all the phenyl and heterocyclic radicals above furthermore being optionally substituted with one or more alkyl radicals,
the phenyl radicals furthermore being optionally substituted with NR5R6 in which R5 and R6 are as defined in claim 1,
the other substituents of said products of formula (I) having the values defined in any one of the claims.

36. Products of formula (I) as defined in any one of the claims, such that Y2 represents hydrogen, halogen, alkyl, cycloalkyl, hydroxyl, alkoxy, carboxyl which is free or esterified with an alkyl or phenyl radical, NH2, NHalk, N(alk)2 and phenyl,
all the alkyl, alkoxy and phenyl radicals being optionally substituted with one or more radicals chosen from halogen atoms and hydroxyl, C1-C4 alkyl, C1-C4 alkoxy, CF3, NH2, NHalk and N(alk)2 radicals and a phenyl radical, which is itself optionally substituted with one or more substituents chosen from halogen atoms and hydroxyl or alkoxy radicals,
all the phenyl radicals furthermore being optionally substituted with one or more C1-C4 alkyl radicals and optionally substituted with NR5R6 in which R5 and R6 are as defined above,
the other substituents of said products of formula (I) having the values defined in any one of the claims.

37. Products of formula (I) as defined in any one of the claims, such that Y2 represents hydrogen, F, Cl, CH3, CH2CH3, OH, OCH3, NH2, NHAlk and phenyl optionally substituted with NR5R6 in which R5 and R6 are as defined in claim 1,
the other substituents of said products of formula (I) having the values defined in any one of the claims.

38. Products of formula (I) as defined in any one of the preceding claims, such that B2 represents 4-pyridyl and 4-quinolyl radicals substituted with one or more radicals chosen from F, Cl, OH and OCH3,
the other substituents of said products of formula (I) having the values defined in any one of the claims.

39. Products of formula (I) as defined in any one of the claims, corresonding to formula (IC): 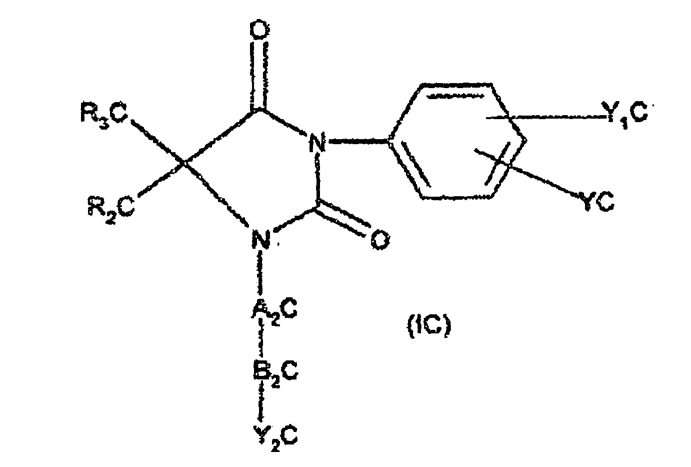 in which YC and Y1C are such that one represents a hydrogen atom, a halogen atom or an amino radical and the other is chosen from -OCF3, -O-CF2-CHF2, -O-CHF2, -O-CH2-CF3, -SF5, -S(O)n-CF3, -S(O)n-Alk, -S02CHF2, SO2CF2CF3, -SO2NH2, -S-CF2-CF2-CF3, -S-Alk-O-Alk, -S-Alk-OH, -S-Alk-CN, -S-Alk-morpholino, -S-Alk-pyrrolidinyl and -S-Alk-piperazinyl, the morpholino, pyrrolidinyl and piperazinyl radicals being optionally substituted with Alk, with Alk representing an alkyl radical containing from 1 to 4 carbon atoms,
or alternatively the phenyl radical forms with its substituents YC and Y1C one of the following two radicals: 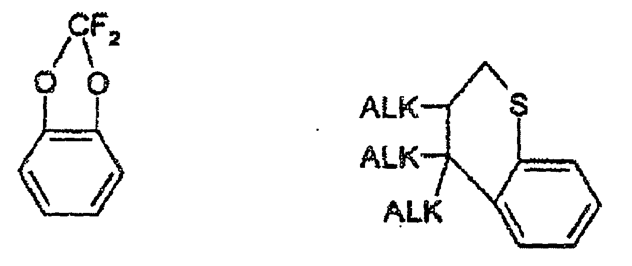 with ALK representing an alkyl radical containing from 1 to 4 carbon atoms,
R2C and R3C, which may be identical or different, represent hydrogen or optionally substituted alkyl or alternatively R2C,
R2C and R3C, which may be identical or different, represent hydrogen or optionally substituted alkyl or alternatively R2C and R3C form, together with the carbon atom to which they are attached, a C3-C10 cycloalkyl or heterocycloalkyl radical,
A2C represents a single bond or CH2,
B2C represents pyridyl, pyrimidinyl, quinolyl, azaindolyl, quinazolyl, thiazolyl, imidazolyl, pyrazolyl, furazanyl, isoxazolyl, morpholinyl, pyrrolidinyl, furyl, piperidyl, chromenyl, oxochromenyl, quinazolyl, thienyl, indolyl, pyrrolyl, purinyl, benzoxazinyl, benzimidazolyl or benzofuranyl radicals, optionally substituted with one or more radicals chosen from the values of Y2A,
Y2CA represents hydrogen, halogen, hydroxyl, cyano, alkyl, alkoxy, phenyl, COOH, COOAlk, CONR5R6, NR5R6, -NR10-COOH, -NR10-COOAlk, -NR10-CO-R6, -NR10-CS-NR5R6, -NR10-CO-NR5R6 or -NR10-S02-R6, all these radicals being optionally substituted,
R5 and R6, which may be identical or different, are chosen from hydrogen, alkyl, cycloalkyl, phenyl, pyrimidinyl, thienyl, pyridyl, quinolyl, thiazolyl and pyran,
all these radicals being optionally substituted, or alternatively R5 and R6 form, with the nitrogen atom to which they are attached, an optionally substituted pyrrolidinyl, piperidyl, piperazinyl, morpholinyl or quinazolinyl radical,
R10 represents hydrogen or alkyl,
all the above alkyl, alkoxy, cycloalkyl and phenyl radicals and also the ring formed by R5 and R6 with the atom to which they are attached being optionally substituted with one or more radicals, which may be identical or different, chosen from halogen atoms and cyano; hydroxyl; alkyl; alkoxy; OCF3; CF3; S(O)n-CF3; nitro; oxo; thioxo; OCOAlk and phenyl radicals, which phenyl radical is itself optionally substituted with one or more radicals chosen from halogen atoms and alkyl and alkoxy radicals; -OCOAlk; NH2, NHAlk, N(Alk)2, N(alk)(phenylalkyl), N(Alk)(aminoalkyl), N(Alk)(alkylaminoalkyl), N(Alk)(dialkylaminoalkyl); carboxyl free or esterified with an alkyl radical,
all the above phenyl radicals moreover being optionally substituted with an alkylenedioxy radical,
all the above alkyl radicals moreover being optionally substituted with one or more radicals chosen from a piperazinyl radical, itself optionally substituted with Alk, Alk-OH and pyridyl; and imidazolyl radical; a morpholinyl radical; a pyrrolidinyl radical, a piperidyl radical, itself optionally substituted with one or two alk; an an azepanyl radical optionally substituted with oxo,
all the above pyrrolidinyl and quinazolinyl radicals moreover being optionally substituted with oxo or thioxo, all the above alkyl and alkoxy radicals being linear or branched and containing not more than 6 carbon atoms,
all the above cycloalkyl radicals containing not more than 7 carbon atoms,
n represents an integer from 0 to 2,
the said products of formula (IC) being in any possible racemic, enantiomeric or diastereoisomeric isomer form, and also the addition salts with mineral and organic acids or with mineral and organic bases of the said products of formula (IC).

40. Products of formula (I) as defined in any one of the claims, corresponding to formula (IA): 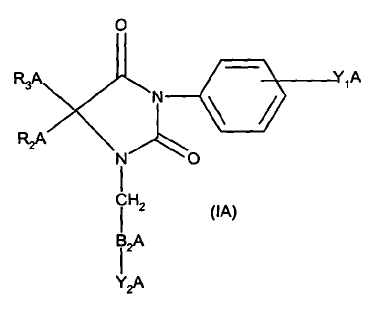 in which:
Y1A represents -OCF3, S(O)n-CF3 and SO2CHF2,
B2a represents 4-quinolyl and 4-pyridyl radicals optionally substituted with one or more radicals chosen from the values of Y2A,
Y2A has the meaning given in any one of the claims for Y2,
R2A and R3A, which may be identical or different,
represent hydrogen or optionally substituted alkyl, or
alternatively R2A and R3A form, together with the carbon atom to which they are attached, a C3-C10 cycloalkyl or heterocycloalkyl radical,
all the alkyl and phenyl radicals being optionally substituted with one or more radicals chosen from halogen, OH, alk, Oalk, OCF3, S(O)n-CF3, CF3, NH2, NHAlk and N(alk)2,
n represents an integer fom 0 to 2,
said products of formula (IA) being in any possible racemic, enantiomeric or diastereoisomeric isomer form, and also the addition salts with mineral and organic acids or with mineral and organic bases of said products of formula (IA).

41. Products of formula (IA) as defined in the preceding claim, in which Y1A, B2a, R2A and R3A have the meanings given above and Y2A represents halogen, -OH, -alk, Oalk, -Oacyl, -NR5AR6A, -CO2H, -CO2alk, -CO-NR5AR6A, -S(O)n-CF3, -NH-S(O)n-CF3 or phenyl radicals, alk representing a linear or branched alkyl radical containing not more than 6 carbon atoms, all the alkyl, alkoxy and phenyl radicals being optionally substituted, R5A and R6A, which may be identical or different, represent hydrogen, alkyl, cycloalkyl or phenyl, the alkyl and phenyl radicals being optionally substituted, or alternatively R5A and R6A form, with the nitrogen atom to which they are attached, a cyclic radical chosen from pyrrolidyl, piperidyl, piperazinyl, morpholinyl, piperazinyl, indolinyl, pyrindolinyl, tetrahydroquinolyl and azetidinyl radicals,
all the alkyl, alkoxy and phenyl radicals being optionally substituted with one or more radicals chosen from halogen, OH, alk, Oalk, OCF3, S(O)n-CF3, CF3, NH2, NHAlk and N(alk)2,
n represents an integer from 0 to 2,
said products of formula (IA) being in any possible racemic, enantiomeric or diastereoisomeric isomer form, and also the addition salts with mineral and organic acids or with mineral and organic bases of said products of formula (IA).

42. Products of formula (I) as defined in any one of the claims, corresponding to formula (IA) in which:
Y1A represents -OCF3, SCF3 or S(O)2-CF3,
B2a represents a 4-quinolyl or 4-pyridyl radical optionally substituted with one or two radicals chosen from halogen, -OH, alk, -Oalk, -CO2H, -CO2alk, -NR5AR6A, -CF3, -OCF3 and optionally substituted phenyl,
R5A and R6A, which may be identical or different, represent hydrogen, alkyl, cycloalkyl or phenyl, the alkyl and phenyl radicals being optionally substituted,
or alternatively R5A and R6A form, with the nitrogen atom to which they are attached, a cyclic radical chosen from pyrrolidyl, piperidyl, piperazinyl, morpholinyl, piperazinyl and azetidinyl radicals,
R2A and R3A, which may be identical or different, represent hydrogen or optionally substituted alkyl, or alternatively R2A and R3A form, together with the carbon atom to which they are attached, a C3-C6 cycloalkyl or heterocycloalkyl radical, all the alkyl and phenyl radicals being optionally substituted with one or more radicals chosen from halogen, OH, alk, Oalk, OCF3, S(O)n-CF3, CF3, NH2, NHalk and N(alk)2,
said products of formula (IA) being in any possible, racemic, enantiomeric or diastereoisomeric isomer form, and also the addition salts with mineral and organic acids or with mineral and organic bases of said products of formula (IA).

43. Products of formula (I) as defined in any one of the claims, corresponding to formula (IA) in which:
Y1A represents -OCF3, SCF3 or S(O)2-CF3,
B2a represents a 4-quinolyl or 4-pyridyl radical optionally substituted with one or two radicals chosen from halogen, -OH, alk and -Oalk,
R2A and R3A, which may be identical or different, represent hydrogen and linear or branched alkyl containing not more than 4 carbon atoms optionally substituted with a hydroxyl radical, or alternatively R2A and R3A form, together with the carbon atom to which they are attached, a C3-C6 cycloalkyl radical,
said products of formula (IA) being in any possible racemic, enantiomeric or diastereoisomeric isomer form, and also the addition salts with mineral and organic acids or with mineral and organic bases of said products of formula (IA).

44. Products of formula (I) as defined in any one of the claims, corresponding to formula (IA) in which Y1a represents OCF3, SCF3 or S(O)2CF3 and R2A and R3A, which may be identical or different, represent hydrogen and CH3, or alternatively R2A and R3A form, together with the carbon atom to which they are attached, a cyclopropyl radical,
the other substituents having the values given in any one of the claims,
said products of formula (IA) being in any possible racemic, enantiomeric or diastereoisomeric isomer form, and also the addition salts with mineral and organic acids or with mineral and organic bases of said products of formula (IA).

45. Products of formula (I) as defined in any one of the claims, corresponding to formula (IB): 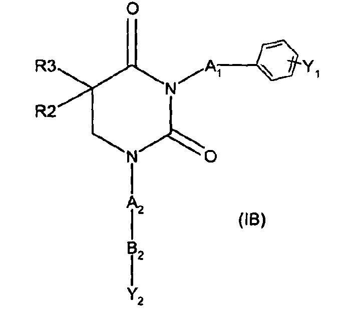 in which R2, R3, A1, Y, Y1, A2, B2 and Y2 have the meanings given in any one of the claims
said products of formula (IB) being in any possible racemic, enantiomeric or diastereoisomeric isomer form, and also the addition salts with mineral and organic acids or with mineral and organic bases of said products of formula (IB).

46. Products of formula (IB) as defined in the preceding claim, in which Y1 represents OCF3, SCF3 or S(O)2CF3 and R2 and R3, which may be identical or different, represent hydrogen and CH3 or, alternatively R2 and R3 form, together with the carbon atom to which they are attached, a cyclopropyl radical,
the other substituents having values given in any one of the claims,
said products of formula (IB) being in any possible racemic, enantiomeric or diastereoisomeric isomer form, and also the addition salts with mineral and organic acids or with mineral and organic bases of said products of formula (IB).

47. Products of formula (I) as defined in any one of the claims, the names of which are below:
- (S)-5-methyl-1-quinol-4-ylmethyl-3-(4-trifluoromethane-sulfonylphenyl)imidazolidine-2,4-dione trifluoroacetate
- (S)-5-methyl-1-pyrid-4-ylmethyl-3-(4-trifluoromethane-sulfonylphenyl)imidazolidine-2,4-dione trifluoroacetate
- (S)-5-methyl-1-quinol-4-ylmethyl-3-(4-trifluoromethyl-sulfanylphenyl)imidazolidine-2,4-dione trifluoroacetate
- 5,5-dimethyl-1-quinol-4-ylmethyl-3-(4-trifluoromethane-sulfonylphenyl)imidazolidine-2,4-dione trifluoroacetate
- (R)-5-methyl-1-quinol-4-ylmethyl-3-(4-trifluoromethane-sulfonylphenyl)imidazolidine-2,4-dione trifluoroacetate
- (R)-5-methyl-1-quinol-4-ylmethyl-3-(4-trifluoromethyl-sulfanylphenyl)imidazolidine-2,4-dione trifluoroacetate
- (R)-5-methyl-1-pyrid-4-ylmethyl-3-(4-trifluoromethane-sulfonylphenyl)imidazolidine-2,4-dione trifluoroacetate
- (R)-5-methyl-1-(3-methylpyrid-4-ylmethyl)-3-(4-tri-fluoromethanesulfonylphenyl)imidazolidine-2,4-dione trifluoroacetate
- (R)-4-methyl-3-quinol-4-ylmethyl-5-thioxo-1-(4-tri-fluoromethylsulfanylphenyl)imidazolidin-2-one trifluoroacetate
- (R)-5-isopropyl-1-quinol-4-ylmethyl-3-(4-trifluoro-methylsulfanylphenyl)imidazolidine-2,4-dione trifluoroacetate
- (R)-5-isopropyl-1-quinol-4-ylmethyl-3-(4-trifluoro-methanesulfonylphenyl)imidazolidine-2,4-dione trifluoroacetate
- (R)-5-(4-hydroxy-benzyl)-1-quinol-4-ylmethyl-3-(4-tri-fluoromethanesulfonylphenyl)imidazolidine-2,4-dione trifluoroacetate
- (R)-5-(4-hydroxy-benzyl)-1-pyrid-4-ylmethyl-3-(4-tri-fluoromethanesulfonylphenyl)imidazolidine-2,4-dione trifluoroacetate
- (R)-5-(1-hydroxy-ethyl)-1-quinol-4-ylmethyl-3-(4-tri-fluoromethanesulfonylphenyl)imidazolidine-2,4-dione trifluoroacetate
- 4-quinol-4-ylmethyl-6-(4-trifluoromethylsulfanyl-phenyl)-4,6-diazaspiro[2.4]heptane-5,7-dione trifluoroacetate
- 4-quinol-4-ylmethyl-6-(4-trifluoromethanesulfonyl-phenyl)-4,6-diazaspiro[2.4]heptane-5,7-dione trifluoroacetate
- 4-pyrid-4-ylmethyl-6-(4-trifluoromethylsulfanylphenyl)-4,6-diazaspiro[2.4]heptane-5,7-dione trifluoroacetate
- 4-pyrid-4-ylmethyl-6-(4-trifluoromethanesulfonyl-phenyl)-4,6-diazaspiro[2.4]heptane-5,7-dione trifluoroacetate
- (R)-1-(3-hydroxypyrid-4-ylmethyl)-5-methyl-3-(4-tri-fluoromethylsulfanylphenyl)imidazolidine-2,4-dione trifluoroacetate
- 5,5-dimethyl-1-quinol-4-ylmethyl-3-(4-trifluoromethoxyphenyl)imidazolidine-2,4-dione trifluoroacetate
- 5,5-dimethyl-1-quinol-4-ylmethyl-3-(4-trifluoromethylsulfanylphenyl)imidazolidine-2,4-dione trifluoroacetate
- 5,5-dimethyl-1-(3-methylpyrid-4-ylmethyl)-3-(4-trifluoromethoxyphenyl)imidazolidine-2,4-dione trifluoroacetate
- 5,5-dimethyl-1-(3-methylpyrid-4-ylmethyl)-3-(4-trifluoromethylsulfanylphenyl)imidazolidine-2,4-dione trifluoroacetate
- 5,5-dimethyl-1-(3-methylpyrid-4-ylmethyl)-3-(4-trifluoromethanesulfonylphenyl)imidazolidine-2,4-dione trifluoroacetate
- 1-(3-hydroxypyrid-4-ylmethyl)-5,5-dimethyl-3-(4-trifluoromethoxyphenyl)imidazolidine-2,4-dione trifluoroacetate
- 1-(3-hydroxypyrid-4-ylmethyl)-5,5-dimethyl-3-(4-trifluoromethylsulfanylphenyl)imidazolidine-2,4-dione trifluoroacetate
- 1-(3-hydroxypyrid-4-ylmethyl)-5,5-dimethyl-3-(4-trifluoromethanesulfonylphenyl)imidazolidine-2,4-dione trifluoroacetate
- 4-quinol-4-ylmethyl-6-(4-trifluoromethoxyphenyl)-4,6-diazaspiro[2.4]heptane-5,7-dione trifluoroacetate
- 4-(3-methylpyrid-4-ylmethyl)-6-(4-trifluoromethylsulfanylphenyl)-4,6-diazaspiro[2.4]heptane-5,7-dione trifluoroacetate
- 4-(3-hydroxypyrid-4-ylmethyl)-6-(4-trifluoromethoxyphenyl)-4,6-diazaspiro[2.4]heptane-5,7-dione trifluoroacetate,
- 4-(3-hydroxypyrid-4-ylmethyl)-6-(4-trifluoromethylsulfanylphenyl)-4,6-diazaspiro[2.4]heptane-5,7-dione trifluoroacetate
- 4-(3-hydroxypyrid-4-ylmethyl)-6-(4-trifluoromethanesulfonylphenyl)-4,6-diazaspiro[2.4]heptane-5,7-dione trifluoroacetate,
said products of formula (I) being in any possible racemic, enantiomeric or diastereoisomeric isomer form, and also the addition salts with mineral and organic acids or with mineral and organic bases of said products of formula (I).

48. Products of formula (I) as defined in any one of the claims, the names of which are as follows:
- cyclopropanecarboxylic acid {4-[5,5-dimethyl-2,4-dioxo-3-(4-trifluoromethylsulfanylphenyl)imidazolidin-1-ylmethyl]pyridin-2-yl}amide trifluoroacetate;
- 5,5-dimethyl-1-[2-(pyridin-2-ylamino)pyridin-4-yl-methyl]-3-(4-trifluoromethylsulfanylphenyl)imidazolidine-2,4-dione; compound with trifluoroacetic acid;
- N-{4-[5,5-dimethyl-2,4-dioxo-3-(4-trifluoromethylsulfanylphenyl)imidazolidin-1-ylmethyl]pyridin-2-yl}isobutyramide; compound with trifluoroacetic acid;
- N-{4-[5,5-dimethyl-2,4-dioxo-3-(4-trifluoromethylsulfanylphenyl)imidazolidin-1-ylmethyl]pyridin-2-yl}propionamide; compound with trifluoroacetic acid;
- 1-(2-aminopyridin-4-ylmethyl)-5,5-dimethyl-3-(4-trifluoromethylsulfanylphenyl)imidazolidine-2,4-dione hydrochloride;
- pyridine-2-carboxylic acid {4-[5,5-dimethyl-2,4-dioxo-3-(4-trifluoromethylsulfanylphenyl)imidazolidin-1-ylmethyl]pyridin-2-yl}amide trifluoroacetate;
- N-{4-[5,5-dimethyl-2,4-dioxo-3-(4-trifluoromethylsulfanylphenyl)imidazolidin-1-ylmethyl]pyridin-2-yl}-3-piperidin-1-yl-propionamide trifluoroacetate;
- N-{4-[5,5-dimethyl-2,4-dioxo-3-(4-trifluoromethylsulfanylphenyl)imidazolidin-1-ylmethyl]pyridin-2-yl}-3-[4-(2-hydroxyethyl)piperazin-1-yl]propionamide trifluoroacetate;
- N-{4-[5,5-dimethyl-2,4-dioxo-3-(4-trifluoromethylsulfanylphenyl)imidazolidin-1-ylmethyl]pyridin-2-yl}-3-morpholin-4-yl-propionamide trifluoroacetate;
- N-{4-[5,5-dimethyl-2,4-dioxo-3-(4-trifluoromethylsulfanylphenyl)imidazolidin-l-ylmethyl]pyridin-2-yl}-3-pyrrolidin-1-yl-propionamide trifluoroacetate;
- N-{4-[5,5-dimethyl-2,4-dioxo-3-(4-trifluoromethylsulfanylphenyl)imidazolidin-l-ylmethyl]pyridin-2-yl}-3-(4-methylpiperazin-1-yl)propionamide trifluoroacetate;
- 1-{4-[5,5-dimethyl-2,4-dioxo-3-(4-trifluoromethylsulfanylphenyl)imidazolidin-1-ylmethyl]pyridin-2-yl}-3-phenylurea;
- 1-[2-(6-ethylpyridin-2-ylamino)pyridin-4-ylmethyl]-5,5-dimethyl-3-(4-trifluoromethylsulfanylphenyl)imidazolidine-2,4-dione;
- 5,5-dimethyl-1-[2-(4-methylpyridin-2-ylamino)pyridin-4-ylmethyl]-3-(4-trifluoromethylsulfanylphenyl)imidazolidine-2,4-dione;
- 5,5-dimethyl-1-[2-(6-methylpyridin-2-ylamino)pyridin-4-ylmethyl]-3-(4-trifluoromethylsulfanylphenyl)imidazolidine-2,4-dione;
- 1-[2-(4,6-dimethylpyridin-2-ylamino)pyridin-4-yl-methyl]-5,5-dimethyl-3-(4-trifluoromethylsulfanylphenyl)imidazolidine-2,4-dione;
- 1-[2-(3,5-dichloropyridin-2-ylamino)pyridin-4-yl-methyl]-5,5-dimethyl-3-(4-trifluoromethylsulfanylphenyl)imidazolidine-2,4-dione;
- 5,5-dimethyl-1-[2-(pyridin-4-ylamino)pyridin-4-yl-methyl]-3-(4-trifluoromethylsulfanylphenyl)imidazolidine-2,4-dione;
- 5,5-dimethyl-1-[2-(pyridin-3-ylamino)pyridin-4-yl-methyl]-3-(4-trifluoromethylsulfanylphenyl)imidazolidine-2,4-dione;
- N-{4-[5,5-dimethyl-2,4-dioxo-3-(4-trifluoromethylsulfanylphenyl)imidazolidin-1-yl-methyl]pyridin-2-yl}-3-(2-oxoazepan-1-yl)propionamide;
- 3-(benzylmethylamino)-N-{4-[5,5-dimethyl-2,4-dioxo-3-(4-trifluoromethylsulfanylphenyl)imidazolidin-1-yl-methyl]pyridin-2-yl}propionamide;
- 4,5-diacetoxy-6-acetoxymethyl-2-(3-{4-[5,5-dimethyl-2,4-dioxo-3-(4-trifluoromethylsulfanylphenyl)imidazolidin-1-ylmethyl]pyridin-2-yl}thioureidoacetic acid;
- 5,5-dimethyl-1-[2-(5-methylpyridin-2-ylamino)pyridin-4-ylmethyl]-3-(4-trifluoromethylsulfanylphenyl)imidazolidine-2,4-dione;
- N-(4-[5,5-dimethyl-2,4-dioxo-3-(4-trifluoromethylsulfanylphenyl)imidazolidin-1-ylmethyl]pyridin-2-yl}-3,5-dimethoxy-benzamide trifluoroacetate;
- 5,5-dimethyl-1-[2-(pyrazin-2-ylamino)pyridin-4-ylmethyl]-3-(4-trifluoromethylsulfanylphenyl)imidazolidine-2,4-dione trifluoroacetate;
- N-{4-[5,5-dimethyl-2,4-dioxo-3-(4-trifluoromethylsulfanylphenyl)imidazolidin-1-ylmethyl]pyridin-2-yl}-3-(3-methylpiperidin-1-yl)propionamide trifluoroacetate;
- N-{4-[5,5-dimethyl-2,4-dioxo-3-(4-trifluoromethylsulfanylphenyl)imidazolidin-1-ylmethyl]pyridin-2-yl}-3-(3,5-dimethylpiperidin-1-yl)propionamide trifluoroacetate;
- N-{4-[5,5-dimethyl-2,4-dioxo-3-(4-trifluoromethylsulfanylphenyl)imidazolidin-1-ylmethyl]pyridin-2-yl}-3-methoxybenzamide trifluoroacetate;
- pyrazine-2-carboxylic acid {4-[5,5-dimethyl-2,4-dioxo-3-(4-trifluoromethylsulfanylphenyl)imidazolidin-1-ylmethyl]pyridin-2-yl}amide trifluoroacetate;
- thiophene-2-carboxylic acid {4-[5,5-dimethyl-2,4-dioxo-3-(4-trifluoromethylsulfanylphenyl)imidazolidin-1-ylmethyl]pyridin-2-yl}amide trifluoracetate;
- N-{4-[5,5-dimethyl-2,4-dioxo-3-(4-trifluoromethylsulfanylphenyl)imidazolidin-1-ylmethyl]pyridin-2-yl}-4-methylbenzamide; compound with trifluoroacetic acid;
- 1-isoquinolin-5-yl-5,5-dimethyl-3-(4-trifluoromethylsulfanylphenyl)imidazolidine-2,4-dione;
- 3-(4-acetylpiperazin-1-yl)-N-{4-[5,5-dimethyl-2,4-dioxo-3-(4-trifluoromethylsulfanylphenyl)imidazolidin-1-ylmethyl]pyridin-2-yl}propionamide;
- 3-(4-(2-diethylaminoethyl)piperazin-1-yl)-N-{4-[5,5-dimethyl-2,4-dioxo-3-(4-trifluoromethylsulfanylphenyl)imidazolidin-1-ylmethyl]pyridin-2-yl}propionamide;
- N-{4-[5,5-dimethyl-2,4-dioxo-3-(4-trifluoromethylsulfanylphenyl)imidazolidin-1-ylmethyl]pyridin-2-yl}-3-(2,6-dimethylmorpholin-4-yl)propionamide;
- N-{4-[5,5-dimethyl-2,4-dioxo-3-(4-trifluoromethylsulfanylphenyl)imidazolidin-1-ylmethyl]pyridin-2-yl}-3-(4-pyrrolidin-1-ylpiperidin-1-yl)propionamide;
- N-(4-[5,5-dimethyl-2,4-dioxo-3-(4-trifluoromethylsulfanylphenyl)imidazolidin-1-ylmethyl]pyridin-2-yl)-2-(4-pyrrolidin-1-ylpiperidin-1-yl)acetamide;
- 5,5-dimethyl-1-[2-(4-methylpyridin-3-ylamino)pyridin-4-ylmethyl]-3-(4-trifluoromethylsulfanylphenyl)imidazolidine-2,4-dione;
- 5,5-dimethyl-1-[2-(6-morpholin-4-ylpyridin-3-yl-amino)pyridin-4-ylmethyl]-3-(4-trifluoromethylsulfanylphenyl)imidazolidine-2,4-dione;
- 1-[2-(2,6-dimethylpyridin-3-ylamino)pyridin-4-yl-methyl]-5,5-dimethyl-3-(4-trifluoromethylsulfanylphenyl)imidazolidine-2,4-dione;
- the methyl ester of 5-{4-[5,5-dimethyl-2,4-dioxo-3-(4-trifluoromethylsulfanylphenyl)imidazolidin-1-ylmethyl]pyridin-2-ylamino}pyridine-2-carboxylic acid;
- 1-[2-(2,6-dimethoxypyridin-3-ylamino)pyridin-4-ylmethyl]-5,5-dimethyl-3-(4-trifluoromethylsulfanylphenyl)imidazolidine-2,4-dione;
- 1-[2-(6-fluoropyridin-3-ylamino)pyridin-4-ylmethyl]-5,5-dimethyl-3-(4-trifluoromethylsulfanylphenyl)imidazolidine-2,4-dione;
- 1-[2-(6-methoxypyridin-3-ylamino)pyridin-4-ylmethyl]-5,5-dimethyl-3-(4-trifluoromethylsulfanylphenyl)imidazolidine-2,4-dione; the said products of formula (I) being in any possible racemic, enantiomeric or diastereoisomeric isomer form, and also the addition salts with mineral and organic acids or with mineral and organic bases of the said products of formula (I).

49. As medicinal products, the products of formula (I) as defined in claims 1 to 48, the said products of formula (I) being in any possible racemic, enantiomeric or diastereoisomeric isomer form, and also the addition salts with pharmaceutically acceptable mineral and organic acids or with pharmaceutically acceptable mineral and organic bases of said products of formula (I).

50. As medicinal products, the products of formula (IC) as defined in the preceding claims, the said products of formula (IC) being in any possible racemic, enantiomeric or diastereoisomeric isomer form, and also the addition salts with pharmaceutically acceptable mineral and organic acids or with pharmaceutically acceptable mineral and organic bases of said products of formula (IC).

51. As medicinal products, the products of formula (IA) as defined in the preceding claims, the said products of formula (IA) being in any possible racemic, enantiomeric or diastereoisomeric isomer form, and also the addition salts with pharmaceutically acceptable mineral and organic acids or with pharmaceutically acceptable mineral and organic bases of said products of formula (IA).

52. As medicinal products, the products of formula (I) as defined in claim 47 or 48, and the addition salts with pharmaceutically acceptable mineral and organic acids or with pharmaceutically acceptable mineral and organic bases of these products.

53. Pharmaceutical compositions containing, as active principle, at least one of the medicinal products as defined in claims 49 to 52.

54. Pharmaceutical compositions containing, as active principle, at least one of the medicinal products as defined in claim 47 or 48.

55. Pharmaceutical compositions as defined in the claims, also containing active principles of other chemotherapy medicinal products for combating cancer.

56. Pharmaceutical compositions according to any one of the claims, **characterized in that** they are used as medicinal products, in particular for cancer chemotherapy.

57. Use of products of formula (I) as defined in any one of the claims or of pharmaceutically acceptable salts of said products of formula (I) for the preparation of medicinal products for inhibiting the activity of protein kinases and especially of a protein kinase.

58. Use of products of formula (I) as defined in the preceding claim or of pharmaceutically acceptable salts of said products of formula (I) in which the protein kinase is a protein tyrosine kinase.

59. Use of products of formula (I) as defined in any one of the claims or of pharmaceutically acceptable salts of said products of formula (I), in which the protein kinase is chosen from the following group: EGFR, Fak, FLK-1, FGFR1, FGFR2, FGFR3, FGFR4, FGFR5, flt-1, IGF-1R, KDR, PLK, PDGFR, tie2, VEGFR, AKT, Raf.

60. Use of products of formula (I) as defined in any one of the claims or of pharmaceutically acceptable salts of said products of formula (I) in which the protein kinase is IGF1R.

61. Use of products of formula (I) as defined in any one of the claims or of pharmaceutically acceptable salts of said products of formula (I) in which the protein kinase is FAK.

62. Use of products of formula (I) as defined in any one of the claims or of pharmaceutically acceptable salts of said products of formula (I) in which the protein kinase is AKT.

63. Use of products of formula (I) as defined in any one of the claims or of pharmaceutically acceptable salts of said products of formula (I) in which the protein kinase is in a cell culture.

64. Use of products of formula (I) as defined in any one of the claims or of pharmaceutically acceptable salts of said products of formula (I) in which the protein kinase is in a mammal.

65. Use of products of formula (I) as defined in any one of the claims or of pharmaceutically acceptable salts of said products of formula (I), for the preparation of a medicinal product for preventing or treating a disease **characterized by** the deregulation of the activity of a protein kinase.

66. Use of products of formula (I) according to the preceding claim, in which the disease to be prevented or treated is in a mammal.

67. Use of products of formula (I) as defined in any one of the claims or of pharmaceutically acceptable salts of said products of formula (I), for the preparation of a medicinal product for preventing or treating a disease belonging to the following group: disorders of blood vessel proliferation, fibrotic disorders, disorders of mesangial cell proliferation, metabolic disorders, allergies, asthma, thrombosis, diseases of the nervous system, retinopathy, psoriasis, rheumatoid arthritis, diabetes, muscle degeneration, oncology diseases and cancers.

68. Use of products of formula (I) as defined in any one of the claims or of pharmaceutically acceptable salts of said products of formula (I), for the preparation of a medicinal product for treating oncology diseases.

69. Use of products of formula (I) as defined in any one of the preceding claims or of pharmaceutically acceptable salts of said products of formula (I) for the preparation of a medicinal product for treating cancers.

70. Use of products of formula (I) according to the preceding claim, in which the disease to be treated is a cancer of solid tumors.

71. Use of products of formula (I) according to the preceding claim, in which the disease to be treated is a cancer that is resistant to cytotoxic agents.

72. Use of products of formula (I) as defined in any one of the claims or of pharmaceutically acceptable salts of said products of formula (I) for the preparation of a medicinal product for treating cancers including breast cancer, stomach cancer, cancer of the colon, lung cancer, cancer of the ovaries, cancer of the uterus, brain cancer, cancer of the kidney, cancer of the larynx, cancer of the lymphatic system, cancer of the thyroid, cancer of the urogenital tract, cancer of the tract including the seminal vesicle and prostate, bone cancer, cancer of the pancreas and melanomas.

73. Use of products of formula (I) according to the preceding claim, in which the disease to be treated is a breast cancer, cancer of the colon or lung cancer.

74. Use of products of formula (I) as defined in any one of the claims or of pharmaceutically acceptable salts of said products of formula (I) for the preparation of a medicinal product for cancer chemotherapy.

75. Use of products of formula (I) as defined in any one of the claims or of pharmaceutically acceptable salts of said products of formula (I) for the preparation of medicinal products for cancer chemotherapy, used alone or in combination.

76. Use of products of formula (I) as defined in any one of the claims or of pharmaceutically acceptable salts of said products of formula (I) for the preparation of medicinal products to be used alone or in combination with a chemotherapy or radiotherapy, or alternatively in combination with other therapeutic agents.

77. Use of products of formula (I) according to the preceding claim, in which the therapeutic agents may be commonly used antitumor agents.

78. Products of formula (I) as defined in any one of the claims, as protein kinase inhibitors, said products of formula (I) being in any possible racemic, enantiomeric or diastereoisomeric isomer form, and also the addition salts with pharmaceutically acceptable mineral and organic acids or with pharmaceutically acceptable mineral and organic bases of said products of formula (I), and also prodrugs thereof.

79. Products of formula (I) as defined in any one of the claims, as IGF1R inhibitors.

80. Products of formula (IA) as defined in the claims, as IGF1R inhibitors.

## Patentansprüche

1. Produkte mit der Formel (I): 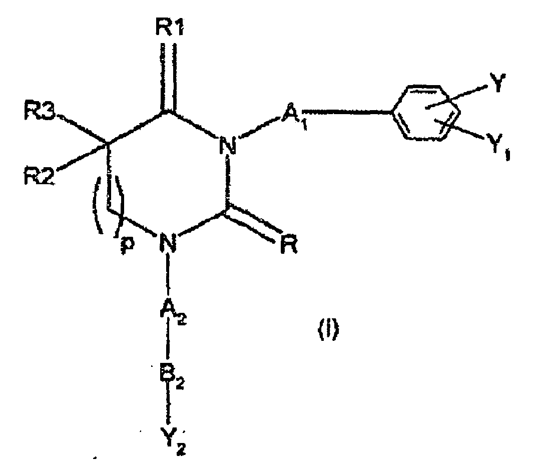 worin p für eine ganze Zahl von 0 bis 2 steht,
R und R1, gleich oder verschieden, für O oder NH stehen,
R2 und R3, gleich oder verschieden, für Wasserstoff, Alkyl, Alkenyl, Alkynyl, Cycloalkyl, Aryl und Heteroaryl stehen, gegebenenfalls substituiert, oder R2 und R3 zusammen mit dem Kohlenstoffatom, an das sie gebunden sind, einen carbocyclischen oder heterocyclischen Rest bilden, wobei diese Reste aus 3 bis 10 Gliedern bestehen und der heterocyclische Rest ein oder mehrere Heteroatome, ausgewählt aus O, S, N und NR7, umfasst, wobei alle diese Reste gegebenenfalls substituiert sind,
A1 für eine einfache Bindung, einen Alkylrest, einen Alkenyl- oder Alkynylrest steht,
Y und Y1, gleich oder verschieden, so sind, dass einer von Y und Y1 ausgewählt ist aus OCF3, -O-CF2-CHF2, -O-CHF2, -O-CH2-CF3, -S(O)nCF3, -S-CF2-CF2-CF3, -S(O)n-Alk, -S-Alk-O-Alk, -S-Alk-OH, -S-Alk-CN, -S-Alk-Heterocycloalkyl (Beschreibung: Morpholino, Pyrrolidinyl, Piperazinyl, gegebenenfalls substituiert mit alk), -SO2CHF2, -SO2CF2CF3, -SO2NR5R6 und -SF5, wobei Alk für einen Alkylrest steht, der 1 bis 4 Kohlenstoffatome umfasst, und der andere von Y und Y1 ausgewählt ist aus den folgenden Werten: Wasserstoff, Halogen, Nitro, NR5R6, freies oder verestertes Carboxy und CONR5R6,
oder der Phenylrest mit seinen Substituenten Y und Y1 einen der folgenden Reste bildet: 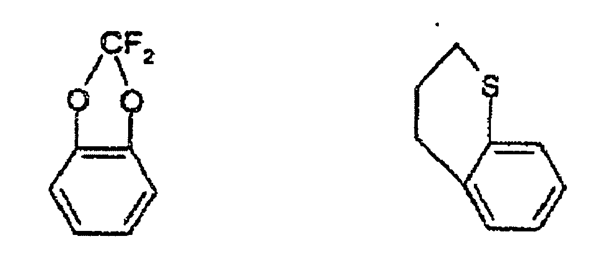 wobei der so gebildete Rest gegebenenfalls mit einem oder mehreren Alkylresten substituiert ist, die ihrerseits gegebenenfalls substituiert sind,
R5 und R6, gleich oder verschieden, ausgewählt sind aus Wasserstoff, Alkyl, Alkenyl, Alkynyl, Cycloalkyl, Cycloalkenyl, Heterocycloalkyl, Aryl und Heteroaryl, gegebenenfalls substituiert, oder R5 und R6 mit dem Stickstoffatom, an das sie gebunden sind, einen heterocyclischen Rest bilden, der 3 bis 10 Glieder umfasst, umfassend ein oder mehrere Heteroatome, ausgewählt aus O, S, N und NR7, gegebenenfalls substituiert,
A2, gleich oder verschieden von A1, für die Werte von A1 und CO und SO2 steht,
B2 für einen gesättigten oder ungesättigten, monocyclischen oder bicyclischen, heterocyclischen Rest steht, der 1 bis mehrere Heteroatome umfasst, gleich oder verschieden, ausgewählt aus O, S, N und NR7, gegebenenfalls substituiert mit einem oder mehreren Substituenten, gleich oder verschieden, ausgewählt aus den Werten für Y2,
R7 für ein Wasserstoffatom, einen Rest Alkyl, Cycloalkyl, Phenyl, Acyl, S(O)2Alk, S(O)2-Aryl, S(O)2-Heteroaryl und S(O)2NR5R6 steht,
Y2 für Wasserstoff, Halogen, Hydroxyl, Cyano, Alkyl, Alcoxy, Cycloalkyl, Heterocycloalkyl, Aryl, Heteroaryl, -O-Alkenyl, -O-Alkynyl, -O-Cycloalkyl, -S(O)n-Alkyl, -S (O) n-Alkenyl, -S(O)n-Alkynyl, S(O)n-Cycloalkyl, COOR13, -OCOR13, NR5R6, CONR5R6, S(O)n-NR5R6, -NR10-CO-R13, -NR10-SO2-R13, NH-SO2-NR5R6, -NR10-CO-NR5R6, -NR10-CS-NR5R6, -NR10-COOR13 steht, wobei alle diese Reste gegebenenfalls substituiert sind,
wobei alle oben genannten Alkyl-, Alkenyl-, Alkynyl-, Alcoxyreste linear oder verzweigt sind und höchstens 6 Kohlenstoffatome umfassen,
wobei alle oben genannten Cycloalkyl-, Heterocycloalkylreste höchstens 7 Kohlenstoffatome umfassen, wobei alle oben genannten Aryl- und Heteroarylreste höchstens 10 Kohlenstoffatomen umfassen,
wobei alle oben genannten carbocyclischen und heterocyclischen Alkyl-, Alkenyl-, Alkynyl-, Alcoxy-, Cycloalkyl-, Heterocycloalkyl-, Aryl- und Heteroarylreste sowie der Ring, der aus R5 und R6 mit dem Atom gebildet wird, an das sie gebunden sind, gegebenenfalls mit einem oder mehreren Resten substituiert sind, gleich oder verschieden, ausgewählt aus den Halogenatomen und den Resten Cyano, Hydroxy, Alcoxy, CF3, Nitro, Aryl, Heteroaryl, -C(=O)-OR9, -C(=O)-R8, -NR11R12, -C(=O)-NR11R12, -N(R10)-C(=O)-R8, -N(R10)-C(=O)-OR9, N(R10)-C(=O)-NR11R12, -N(R10)-S(O)n-R8, -S(O)n-R8, -N(R10)-S(O)n-NR11R12 oder -S(O)n-NR11R12,
wobei alle oben genannten Aryl- und Heteroarylreste ferner gegebenenfalls mit einem oder mehreren Resten, ausgewählt aus den Resten Alkyl, Alcoxy und Alkylendioxy, substituiert sind,
wobei alle oben genannten cyclischen Reste sowie der Ring, der aus R5 und R6 mit dem Atom gebildet wird, an das sie gebunden sind, ferner gegebenenfalls mit einem oder mehreren Resten, ausgewählt aus Oxo und Thioxo, substituiert sind,
n für eine ganze Zahl von 0 bis 2 steht,
R8 für Alkyl, Alkenyl, Cycloalkyl, Cycloalkylalkyl, Heterocycloalkyl, Heterocycloalkylalkyl, Aryl, Arylalkyl, Heteroaryl und Heteroarylalkyl steht,
R9 für die Werte von R8 und Wasserstoff steht,
R10 für Wasserstoff oder Alkyl steht,
R11 und R12, gleich oder verschieden, für Wasserstoff, C3-C6-Cycloalkyl, C1-C4-Alkyl und Phenyl steht, gegebenenfalls substituiert mit einem oder mehreren Resten, gleich oder verschieden, ausgewählt aus den Halogenatomen
und den Resten Cyano, Hydroxy, Alcoxy, CF3, Nitro, Phenyl
und Carboxy, das frei, in ein Salz überführt, verestert oder amidiert ist,
oder R11 und R12 mit dem Stickstoffatom, an das sie gebunden sind, einen cyclischen Rest bilden, der 5 bis 7 Glieder umfasst, der ein oder mehrere Heteroatome, ausgewählt aus O, S, N und NR7, umfasst, und bevorzugt ein cyclisches Amin,
wobei R13, gleich oder verschieden von R5 oder R6, ausgewählt ist aus den Werten für R5 oder R6,
wobei es sich versteht, dass die Produkte mit der Formel (I) so sind, wie nachfolgend von a) bis d) definiert:
a) wenn p für die ganze Zahl 0 steht, R für Sauerstoff steht, R1 für Sauerstoff steht, A1 für eine einfache Bindung oder Alkyl steht, Y und Y1, gleich oder verschieden, so sind, dass mindestens einer für OCF3 oder Salk steht, A2 für eine einfache Bindung oder Alkyl steht und B2 für einen heterocyclischen Rest steht, der gegebenenfalls substituiert ist, dann steht einer von R2 und R3 nicht für Wasserstoff und der andere nicht für Imidazolylalkyl,
b) wenn p für die ganze Zahl 0 steht, R und R1 für Sauerstoff stehen, A1 für eine einfache Bindung oder Alkyl steht, Y und Y1, gleich oder verschieden, so sind, dass mindestens einer für OCF3, SOAlk, S(O)2alk oder SO2NH2 steht, A2 für CH2 steht und B2 für einen heterocyclischen Rest steht, der gegebenenfalls substituiert ist, dann steht einer von R2 und R3 nicht für Wasserstoff und der andere nicht für eine Alkylkette, die gegebenenfalls durch O, S, Nalk unterbrochen ist, immer substituiert mit einem Hydroxamat -CO-NHOH,
c) wenn p für die ganze Zahl 0 steht, R und R1 für Sauerstoff stehen, A1 für eine einfache Bindung oder Alkyl steht, Y und Y1, gleich oder verschieden, so sind, dass mindestens einer für S(O)nAlk steht, A2 für eine einfache Bindung steht und B2 für einen aromatischen heterocyclischen Rest steht, der 5 oder 6 Glieder umfasst, gegebenenfalls substituiert, dann sind R2 und R3 nicht aus Wasserstoff, Alkyl, Arylalkyl, Aryl und Heteroaryl ausgewählt,
d) wenn p für eine ganze Zahl von 0 bis 2 steht, R und R1 für Sauerstoff stehen, A1 für eine einfache Bindung steht, Y und Y1, gleich oder verschieden, so sind, dass einer für SO2Alk oder SO2NH2 steht und der andere für NR5R6 steht, A2 für eine einfache Bindung oder Alkylen steht und B2 für einen heterocyclischen Rest steht, der 5 bis 10 Glieder umfasst, gegebenenfalls substituiert, dann stehen R2 und R3 nicht beide für Wasserstoff,
wobei es sich versteht, dass die oben genannten Reste die folgenden Bedeutungen haben:
- der Begriff Alkyl-, alk-, Alk- oder ALK-Rest bezeichnet einen linearen oder verzweigten Rest, der höchstens 12 Kohlenstoffatome umfasst, ausgewählt aus den Resten Methyl, Ethyl, Propyl, Isopropyl, Butyl, Isobutyl, sec-Butyl, tert-Butyl, Pentyl, Isopentyl, sec-Pentyl, tert-Pentyl, neo-Pentyl, Hexyl, Isohexyl, sec-Hexyl, tert-Hexyl und auch Heptyl, Octyl, Nonyl, Decyl, Undecyl und Dodecyl sowie deren linearen und verzweigten Stellungsisomeren,
- der Begriff Alkenylrest bezeichnet einen linearen oder verzweigten Rest, der höchstens 12 Kohlenstoffatome und vorzugsweise 4 Kohlenstoffatome umfasst, zum Beispiel ausgewählt aus den folgenden Werten: Ethenyl oder Vinyl, Propenyl oder Allyl, 1-Propenyl, n-Butenyl, i-Butenyl, 3-Methylbut-2-enyl, n-Pentenyl, Hexenyl, Heptenyl, Octenyl, Cyclohexylbutenyl und Decenyl sowie deren linearen und verzweigten Stellungsisomeren,
- der Begriff Alkynylrest bezeichnet einen linearen oder verzweigten Rest, der höchstens 12 Kohlenstoffatome und vorzugsweise 4 Kohlenstoffatome umfasst, zum Beispiel ausgewählt aus den folgenden Werten: Ethynyl, Propynyl oder Propargyl, Butynyl, n-Butynyl, i-Butynyl, 3-Methylbut-2-ynyl, Pentynyl oder Hexynyl sowie deren linearen und verzweigten Stellungsisomeren,
- der Begriff Cycloalkylrest bezeichnet einen monocyclischen oder bicyclischen carbocyclischen Rest, der 3 bis 10 Glieder umfasst, und bezeichnet insbesondere die Reste Cyclopropyl, Cyclobutyl, Cyclopentyl und Cyclohexyl,
- der Begriff Arylrest bezeichnet die ungesättigten, monocyclischen oder aus anellierten Ringen bestehenden, carbocyclischen Reste. Als Beispiele für einen solchen Arylrest können die Reste Phenyl oder Naphtyl zitiert werden,
- der Begriff heterocyclischer Rest bezeichnet einen carbocyclischen, gesättigten (Heterocycloalkyl) oder ungesättigten (Heteroaryl) Rest, der aus höchstens 6 Glieder besteht, die durch ein oder mehrere Heteroatome, gleich oder verschieden, ausgewählt aus den Sauerstoff-, Stickstoff- oder Schwefelatomen, unterbrochen sind,
als Heterocycloalkylreste können insbesondere die Reste Dioxolan, Dioxan, Dithiolan, Thiooxolan, Thiooxan, Oxiranyl, Oxolanyl, Dioxolanyl, Piperazinyl, Piperidinyl, Pyrrolidinyl, Imidazolidinyl, Pyrazolidinyl, Morpholinyl oder auch Tetrahydrofuryl, Tetrahydrothienyl, Chromanyl, Dihydrobenzofuranyl, Indolinyl, Piperidinyl, Perhydropyranyl, Pyrindolinyl, Tetrahydrochinolinyl, Tetrahydroisochinolinyl oder Thioazolidinyl zitiert werden, wobei alle diese Reste gegebenenfalls substituiert sind,
- aus den Heteroarylresten mit 5 Gliedern können die Furylreste, wie etwa 2-Furyl, die Thienylreste, wie etwa 2-Thienyl und 3-Thienyl, die Reste Pyrrolyl, Diazolyl, Thiazolyl, Thiadiazolyl, Thiatriazolyl, Isothiazolyl, Oxazolyl, Oxadiazolyl, 3-oder 4-Isoxazolyl, Imidazolyl, Pyrazolyl, Isoxazolyl zitiert werden,
aus den Heteroarylresten mit 6 Gliedern können insbesondere die Pyridylreste, wie etwa 2-Pyridyl, 3-Pyridyl, und 4-Pyridyl, die Reste Pyrimidyl, Pyrimidinyl, Pyridazinyl, Pyrazinyl und Tetrazolyl zitiert werden,
- als anellierte Heteroarylreste, die mindestens ein Heteroatom enthalten, ausgewählt aus Schwefel, Stickstoff und Sauerstoff, können zum Beispiel Benzothienylreste, wie etwa 3-Benzothienyl, die Reste Benzofuryl, Benzofuranyl, Benzopyrrolyl, Benzimidazolyl, Benzoxazolyl, Thionaphtyl, Indolyl, Purinyl, Chinolinyl, Isochinolinyl und Naphtyridinyl zitiert werden,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

2. Produkte mit der Formel (I), wie in Anspruch 1 definiert, worin einer von Y und Y1 für ein Wasserstoffatom steht und der andere ausgewählt ist aus OCF3, S(O)nCF3, S(O)nAlk, SO2CHF2, SO2CF2CF3 und SO2NR5R6,
wobei die anderen Substituenten der Produkte mit der Formel (I) aus den Werten ausgewählt sind, die in Anspruch 1 definiert sind, und es sich versteht, dass:
a) wenn p für die ganze Zahl 0 steht, R für Sauerstoff steht, R1 für Sauerstoff steht, A1 für eine einfache Bindung oder Alkyl steht, Y und Y1, gleich oder verschieden, so sind, dass einer für Wasserstoff steht und der andere für OCF3 oder Salk steht, A2 für eine einfache Bindung oder Alkyl steht und B2 für einen heterocyclischen Rest steht, der gegebenenfalls substituiert ist, dann einer von R2 und R3 nicht für Wasserstoff und der andere nicht für Imidazolylalkyl steht,
b) wenn p für die ganze Zahl 0 steht, R und R1 für Sauerstoff stehen, A1 für eine einfache Bindung oder Alkyl steht, Y und Y1, gleich oder verschieden, so sind, dass einer für Wasserstoff steht und der andere für OCF3, SOAlk, S(O)2alk oder SO2NH2 steht, A2 für CH2 steht und B2 für einen heterocyclischen Rest steht, der gegebenenfalls substituiert ist, dann einer von R2 und R3 nicht für Wasserstoff und der andere nicht für eine Alkylkette steht, die gegebenenfalls durch O, S, Nalk unterbrochen ist, immer substituiert mit einem Hydroxamat -CO-NHOH,
c) wenn p für die ganze Zahl 0 steht, R und R1 für Sauerstoff stehen, A1 für eine einfache Bindung oder Alkyl steht, Y und Y1, gleich oder verschieden, so sind, dass einer für Wasserstoff steht und der andere für S(O)nAlk steht, A2 für eine einfache Bindung steht und B2 für einen aromatischen heterocyclischen Rest steht, der 5 oder 6 Glieder umfasst, gegebenenfalls substituiert, dann R2 und R3 nicht aus Wasserstoff, Alkyl, Arylalkyl, Aryl und Heteroaryl ausgewählt sind,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

3. Produkte mit der Formel (I), wie in einem der Ansprüche definiert, worin einer von Y und Y1 für ein Wasserstoffatom steht und der andere ausgewählt ist aus S(O)nCF3, SOAlk, S(O)2Alk, SO2CHF2, SO2CF2CF3 und SO2NR5R6,
wobei die anderen Substituenten der Produkte mit der Formel (I) aus den Werten ausgewählt sind, die in Anspruch 1 definiert sind, und es sich versteht, dass die Produkte mit der Formel (I) so sind, wie nachfolgend in a) und b) definiert:
a) wenn p für die ganze Zahl 0 steht, R und R1 für Sauerstoff stehen, A1 für eine einfache Bindung oder Alkyl steht, Y und Y1, gleich oder verschieden, so sind, dass einer für Wasserstoff steht und der andere für SOAlk, S(O)2alk oder SO2NH2 steht, A2 für CH2 steht und B2 für einen heterocyclischen Rest steht, der gegebenenfalls substituiert ist, dann steht einer von R2 und R3 nicht für Wasserstoff und der andere nicht für eine Alkylkette, die gegebenenfalls durch O, S, Nalk unterbrochen ist, immer substituiert mit einem Hydroxamat -CO-NHOH,
b) wenn p für die ganze Zahl 0 steht, R und R1 für Sauerstoff stehen, A1 für eine einfache Bindung oder Alkyl steht, Y und Y1, gleich oder verschieden, so sind, dass einer für Wasserstoff steht und der andere für SOAlk oder S(O)2Alk steht, A2 für eine einfache Bindung steht und B2 für einen aromatischen heterocyclischen Rest steht, der 5 oder 6 Glieder umfasst, gegebenenfalls substituiert, dann sind R2 und R3 nicht aus Wasserstoff, Alkyl, Arylalkyl, Aryl und Heteroaryl ausgewählt,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

4. Produkte mit der Formel (I), wie in einem der Ansprüche definiert, worin einer von Y und Y1 für ein Wasserstoffatom steht und der andere ausgewählt ist aus S(O)nCF3, SO2CHF2, SO2CF2CF3 und SO2NR5R6,
wobei die anderen Substituenten der Produkte mit der Formel (I) ausgewählt sind aus den Werten, die in Anspruch 1 definiert sind und es sich versteht, dass, wenn p für die ganze Zahl 0 steht, R und R1 für Sauerstoff stehen, A1 für eine einfache Bindung oder Alkyl steht, Y und Y1, gleich oder verschieden, so sind, dass einer für Wasserstoff steht und der andere für SO2NH2 steht, A2 für CH2 steht und B2 für einen heterocyclischen Rest steht, der gegebenenfalls substituiert ist, dann einer von R2 und R3 nicht für Wasserstoff und der andere nicht für eine Alkylkette steht, die gegebenenfalls durch O, S, Nalk unterbrochen ist, immer substituiert mit einem Hydroxamat -CO-NHOH,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

5. Produkte mit der Formel (I), wie in einem der Ansprüche definiert, worin einer von Y und Y1 für ein Wasserstoffatom steht und der andere ausgewählt ist aus S(O)nCF3, SO2CHF2 und SO2CF2CF3,
wobei die anderen Substituenten der Produkte mit der Formel (I) aus den Werten ausgewählt sind, die in Anspruch 1 definiert sind,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

6. Produkte mit der Formel (I), wie in einem der Ansprüche definiert, so dass alle Alkyl-, Alkenyl-, Alkynyl-, Cycloalkyl-, Heterocycloalkyl-, Aryl- oder Heteroarylreste, die in Anspruch 1 definiert sind, gegebenenfalls mit einem oder mehreren Resten substituiert sind, gleich oder verschieden, ausgewählt aus Halogen, Cyano, Hydroxy, Alcoxy, CF3, Nitro, Phenyl, Carboxy, das frei, in ein Salz überführt, mit einem Alkylrest verestert oder amidiert ist mit einem Rest NR11aR12a, -C(=O)-R9a, -NR11aR12a, -C(=O)-NR11aR12a, -N(R10a)-C(=O)-R9a, -N(R10a)-C(=O)-OR8a, N(R10a)-C(=O)-NR11aR12a, -N(R10a)-S(O)n-R9a, -S(O)n-R9a, -N(R10a)-S(O)n-NR11aR12a oder -S(O)n-NR11aR12a,
wobei alle oben genannten Aryl- und Heteroarylreste ferner gegebenenfalls mit einem Ethylendioxyrest substituiert sind,
R8a für Wasserstoff, Alkyl, Alkenyl, Phenyl, Phenylalkyl, Heteroaryl oder Heteroarylalkyl steht,
R9a für Alkyl, Cycloalkyl, Cycloalkylalkyl, Heterocycloalkyl, Heterocycloalkylalkyl, Phenyl, Phenylalkyl, Heteroaryl oder Heteroarylalkyl steht,
R10a für Wasserstoff oder Alkyl steht,
R11a und R12a, gleich oder verschieden, für Wasserstoff, Alkyl, Cycloalkyl, Cycloalkylalkyl, Phenyl, Phenylalkyl stehen, gegebenenfalls substituiert mit einem oder mehreren Substituenten, gleich oder verschieden, ausgewählt aus Halogen, Hydroxy, C1-C4-Alkyl oder C1-C4-Alkoxy, oder R11a und R12a mit dem Stickstoffatom, an das sie gebunden sind, einen cyclischen Rest bilden, ausgewählt aus Pyrrolidinyl, Piperidinyl, Piperazinyl, Morpholinyl, Indolinyl, Pyrindolinyl, Tetrahydrochinolinyl, Thiazolidinyl und Naphtyridyl,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

7. Produkte mit der Formel (I), wie in einem der Ansprüche definiert, so dass p für die ganze Zahl 0 steht, wobei die anderen Substituenten der Produkte mit der Formel (I) die Werte haben, die in einem der Ansprüche definiert sind.

8. Produkte mit der Formel (I), wie in einem der Ansprüche definiert, so dass p für die ganze Zahl 1 steht, wobei die anderen Substituenten der Produkte mit der Formel (I) die Werte haben, die in einem der Ansprüche definiert sind.

9. Produkte mit der Formel (I), wie in einem der Ansprüche definiert, so dass p für die ganze Zahl 2 steht, wobei die anderen Substituenten der Produkte mit der Formel (I) die Werte haben, die in einem der Ansprüche definiert sind.

10. Produkte mit der Formel (I), wie in einem der Ansprüche definiert, so dass R1 für 0 steht, wobei die anderen Substituenten der Produkte mit der Formel (I) die Werte haben, die in einem der Ansprüche definiert sind.

11. Produkte mit der Formel (I), wie in einem der Ansprüche definiert, so dass R für 0 steht, wobei die anderen Substituenten der Produkte mit der Formel (I) die Werte haben, die in einem der Ansprüche definiert sind.

12. Produkte mit der Formel (I), wie in einem der Ansprüche definiert, so dass R2 und R3, gleich oder verschieden, für Wasserstoff, Alkyl, Alkenyl, Cycloalkyl, Cycloalkylalkyl, Phenyl, Phenylalkyl, Heterocycloalkyl, Heterocycloalkylalkyl, Heteroaryl und Heteroarylalkyl stehen, gegebenenfalls substituiert, oder R2 und R3 zusammen mit dem Kohlenstoffatom, an das sie gebunden sind, einen carbocyclischen oder heterocyclischen Rest bilden, wobei diese Reste aus 3 bis 10 Gliedern bestehen und der heterocyclische Rest ein oder mehrere Heteroatome, ausgewählt aus O, S, N und NR7b, umfasst, wobei alle diese Reste gegebenenfalls substituiert sind,
wobei alle oben genannten Reste gegebenenfalls mit einem oder mehreren Resten substituiert sind, ausgewählt aus Halogen, Cyano, Hydroxy, Alkyl und Alcoxyl, umfassend 1 bis 4 Kohlenstoffatome, CF3, Nitro, Phenyl, Carboxy, das frei, in ein Salz überführt, mit einem Alkylrest verestert oder amidiert ist mit einem Rest NR11bR12b, -C(=O)-R9b, -NR11bR12b und -C(=O)-NR11bR12b,
R7b für ein Wasserstoffatom, einen Alkylrest oder einen Phenylrest steht,
R9 für Wasserstoff, Alkyl, Cycloalkyl, Cycloalkylalkyl und Phenyl steht,
R11b und R12b, gleich oder verschieden, für Wasserstoff, Alkyl, Cycloalkyl und Phenyl stehen oder R11b und R12b mit dem Stickstoffatom, an das sie gebunden sind, einen Piperazinylrest bilden, der gegebenenfalls substituiert ist,
wobei die anderen Substituenten der Produkte mit der Formel (I) die Werte haben, die in einem der Ansprüche definiert sind.

13. Produkte mit der Formel (I), wie in einem der Ansprüche definiert, so dass R2 und R3, gleich oder verschieden, ausgewählt sind aus Wasserstoff, Alkyl, Phenylalkyl, Pyridylalkyl und Thienylbenzothienylalkyl, gegebenenfalls substituiert mit einem oder mehreren Resten, ausgewählt aus den Halogenatomen und den Resten Hydroxy, Alkyl und Alcoxy, die ein bis 4 Kohlenstoffatome umfassen, oder R2 und R3 zusammen mit dem Kohlenstoffatom, an das sie gebunden sind, einen Cycloalkyl- oder Heterocycloalkylrest mit 3 bis 6 Gliedern bilden, der ein Stickstoffatom umfasst, wobei die anderen Substituenten der Produkte mit der Formel (I) die Werte haben, die in einem der Ansprüche definiert sind.

14. Produkte mit der Formel (I), wie in einem der Ansprüche definiert, so dass R2 und R3, gleich oder verschieden, ausgewählt sind aus Wasserstoff, Alkyl, Hydroxyalkyl, Phenylalkyl, Hydroxyphenylalkyl, Pyridylalkyl, Thienylbenzothienylalkyl, oder R2 und R3 zusammen mit dem Kohlenstoffatom, an das sie gebunden sind, einen Cycloalkylrest, der 3 bis 6 Kohlenstoffatome umfasst oder einen Azetidinyl-, Pyrrolidinyl- und Piperidinylrest bilden,
wobei die anderen Substituenten der Produkte mit der Formel (I) die Werte haben, die in einem der Ansprüche definiert sind.

15. Produkte mit der Formel (I), wie in einem der Ansprüche definiert, so dass R2 und R3, gleich oder verschieden, ausgewählt sind aus Wasserstoff, Alkyl, Hydroxyalkyl, Phenylalkyl und Hydroxyphenylalkyl, oder R2 und R3 zusammen mit dem Kohlenstoffatom, an das sie gebunden sind, einen Cycloalkylrest bilden, der 3 bis 6 Kohlenstoffatome umfasst.

16. Produkte mit der Formel (I), wie in einem der Ansprüche definiert, so dass einer von R2 und R3 ausgewählt ist aus Wasserstoff und Alkyl und der andere von R2 und R3 ausgewählt ist aus allen Werten für R2 und R3, oder R2 und R3 zusammen mit dem Kohlenstoffatom, an das sie gebunden sind, einen Cycloalkylrest bilden, der 3 bis 6 Kohlenstoffatome umfasst,
wobei die anderen Substituenten der Produkte mit der Formel (I) die Werte haben, die in einem der Ansprüche definiert sind.

17. Produkte mit der Formel (I), wie in einem der Ansprüche definiert, so dass R2 und R3, gleich oder verschieden, für Wasserstoff und Alkyl stehen, oder R2 und R3 zusammen mit dem Kohlenstoffatom, an das sie gebunden sind, einen Cycloalkylrest bilden, der 3 bis 6 Kohlenstoffatome umfasst,
wobei die anderen Substituenten der Produkte mit der Formel (I) die Werte haben, die in einem der Ansprüche definiert sind.

18. Produkte mit der Formel (I), wie in einem der Ansprüche definiert, so dass R2 und R3, gleich oder verschieden, für Wasserstoff und CH3 stehen, oder R2 und R3 zusammen mit dem Kohlenstoffatom, an das sie gebunden sind, einen Cyclopropylrest bilden, wobei die anderen Substituenten der Produkte mit der Formel (I) die Werte haben, die in einem der Ansprüche definiert sind.

19. Produkte mit der Formel (I), wie in einem der Ansprüche definiert, so dass A1 für eine einfache Bindung steht und A2 ausgewählt ist aus einer einfachen Bindung, einem linearen oder verzweigten Alkylrest, der höchstens 6 Kohlenstoffatome umfasst, und den Resten Allyl, Propynyl, C=O und S02, wobei die anderen Substituenten der Produkte mit der Formel (I) die Werte haben, die in einem der Ansprüche definiert sind.

20. Produkte mit der Formel (I), wie in einem der Ansprüche definiert, so dass A1 für eine einfache Bindung steht und A2 ausgewählt ist aus einer einfachen Bindung, den Resten Alkyl, Allyl, Propynyl, C=O und SO2, wobei die anderen Substituenten der Produkte mit der Formel (I) die Werte haben, die in einem der Ansprüche definiert sind.

21. Produkte mit der Formel (I), wie in einem der Ansprüche definiert, so dass A1 für eine einfache Bindung steht und A2 für einen Alkylrest oder C=O steht, wobei die anderen Substituenten der Produkte mit der Formel (I) die Werte haben, die in einem der Ansprüche definiert sind.

22. Produkte mit der Formel (I), wie in einem der Ansprüche definiert, so dass A1 für eine einfache Bindung steht und A2 für C=O, -CH2-CH2- oder -CH2 steht, wobei die anderen Substituenten der Produkte mit der Formel (I) die Werte haben, die in einem der Ansprüche definiert sind.

23. Produkte mit der Formel (I), wie in einem der Ansprüche definiert, so dass A1 für eine einfache Bindung steht und A2 für -CH2 steht, wobei die anderen Substituenten der Produkte mit der Formel (I) die Werte haben, die in einem der Ansprüche definiert sind.

24. Produkte mit der Formel (I), wie in einem der Ansprüche definiert, worin Y und Y1 so sind, dass einer für ein Wasserstoffatom, ein Halogenatom oder einen Aminorest steht, und der andere ausgewählt ist aus -OCF3, -O-CF2-CHF2, -O-CHF2, -O-CH2-CF3, -SF5, -S(O)nCF3, -S(O)n-Alk, -SO2CHF2, SO2CF2CF3, -S02NH2, -S-CF2-CF2-CF3, -S-Alk-O-Alk, -S-Alk-OH, -S-Alk-CN, -S-Alk-Morpholino, -S-Alk-Pyrrolidinyl und -S-Alk-Piperazinyl, wobei die Morpholino-, Pyrrolidinyl- und Piperazinylreste gegebenenfalls mit Alk substituiert sind, wobei Alk für einen Alkylrest steht, der 1 bis 4 Kohlenstoffatome umfasst,
wobei die anderen Substituenten der Produkte mit der Formel (I) die Werte haben, die in einem der Ansprüche definiert sind.

25. Produkte mit der Formel (I), wie in einem der Ansprüche definiert, so dass Y für ein Wasserstoffatom steht und Y1 ausgewählt ist aus -OCF3, S(O)n-CF3, S(O)n-CH3, SO2CHF2 und SO2-N(alk)2, wobei die anderen Substituenten der Produkte mit der Formel (I) die Werte haben, die in einem der Ansprüche definiert sind.

26. Produkte mit der Formel (I), wie in einem der Ansprüche definiert, so dass Y für ein Wasserstoffatom steht und Y1 ausgewählt ist aus -OCF3, S(O)n-CF3 und SO2CHF2,
wobei die anderen Substituenten der Produkte mit der Formel (I) die Werte haben, die in einem der Ansprüche definiert sind.

27. Produkte mit der Formel (I), wie in einem der Ansprüche definiert, so dass Y für ein Wasserstoffatom steht und Y1 ausgewählt ist aus -OCF3 und S(O)n-CF3,
wobei die anderen Substituenten der Produkte mit der Formel (I) die Werte haben, die in einem der Ansprüche definiert sind.

28. Produkte mit der Formel (I), wie in einem der Ansprüche definiert, so dass Y für ein Wasserstoffatom steht und Y1 ausgewählt ist aus -OCF3, S-CF3 und S(O)2-CF3,
wobei die anderen Substituenten der Produkte mit der Formel (I) die Werte haben, die in einem der Ansprüche definiert sind.

29. Produkte mit der Formel (I), wie in einem der Ansprüche definiert, so dass B2 für einen monocyclischen oder bicyclischen Heteroarylrest steht, ausgewählt aus den Resten Pyridyl, Pyrimidinyl, Chinolyl, Azaindolyl, 1H-Pyrrolo[2,3-b]pyridinyl, Chinazolyl, Thiazolyl, Imidazolyl, Pyrazolyl, Furazanyl, Isoxazolyl, Morpholinyl, Pyrrolidinyl, Furyl, Piperidyl, Thienyl, Chromenyl, Oxochromenyl, Indolyl, Pyrrolyl, Purinyl, Benzoxazinyl, Benzimidazolyl, Benzofuranyl, wobei diese Reste gegebenenfalls mit einem oder mehreren Resten, ausgewählt aus den Werten für Y2, substituiert sind,
wobei die anderen Substituenten der Produkte mit der Formel (I) die Werte haben, die in einem der Ansprüche definiert sind.

30. Produkte mit der Formel (I), wie in einem der Ansprüche definiert, so dass B2 für einen Heteroarylrest steht, ausgewählt aus den Resten 3- oder 4-Pyridyl, Pyrimidinyl, 3- oder 4-Chinolyl, Azaindolyl, Chinazolyl, Thiazolyl, Imidazolyl, Pyrazolyl, Furazanyl und Isoxazolyl,
wobei diese Reste gegebenenfalls mit einem oder mehreren Resten, ausgewählt aus den Werten für Y2, substituiert sind,
wobei die anderen Substituenten der Produkte mit der Formel (I) die Werte haben, die in einem der Ansprüche definiert sind.

31. Produkte mit der Formel (I), wie in einem der Ansprüche definiert, so dass B2 für einen Heteroarylrest steht, ausgewählt aus den Resten 3- oder 4-Pyridyl, Pyrimidinyl, 3- oder 4-Chinolyl, Azaindolyl und Chinazolyl, wobei diese Reste gegebenenfalls mit einem oder mehreren Resten, ausgewählt aus den Werten für Y2, substituiert sind,
wobei die anderen Substituenten der Produkte mit der Formel (I) die Werte haben, die in einem der Ansprüche definiert sind.

32. Produkte mit der Formel (I), wie in einem der Ansprüche definiert, so dass B2 für die Reste 4-Pyridyl und 4-Chinolyl, 1H-Pyrrolo[2,3-b]pyridin-4-yl steht, gegebenenfalls substituiert mit einem oder mehreren Resten, ausgewählt aus den Werten für Y2, die in einem der Ansprüche definiert sind,
wobei die anderen Substituenten der Produkte mit der Formel (I) die Werte haben, die in einem der Ansprüche definiert sind.

33. Produkte mit der Formel (I), wie in einem der Ansprüche definiert, so dass Y2 für Wasserstoff, Halogen, Hydroxyl, Cyano, Alkyl, Alcoxy, Phenyl, COOH, COOAlk, CONR5R6, NR5R6, -NR10-COOR6, -NR10-CO-R6, -NR10-CS-NR5R6, -NR10-CO-NR5R6 oder -NR10-SO2-R6 steht, wobei alle diese Reste gegebenenfalls substituiert sind,
R5 und R6, gleich oder verschieden, ausgewählt sind aus Wasserstoff, Alkyl, Cycloalkyl, Phenyl und die Heteroarylreste, die 5 bis 6 Glieder umfassen und 1 bis 3 Heteroatome enthalten, ausgewählt aus O, N und S, wobei alle diese Reste gegebenenfalls substituiert sind, oder R5 und R6 mit dem Stickstoffatom, an das sie gebunden sind, einen Pyrrolidinyl-, Piperidyl-, Piperazinyl-, Morpholinyl- oder Chinazolinylrest bilden, gegebenenfalls substituiert,
R10 für Wasserstoff oder Alkyl steht,
wobei alle oben genannten Alkyl-, Alcoxy-, Cycloalkyl- und Phenylreste sowie der Ring, der aus R5 und R6 mit dem Atom, an das sie gebunden sind, gebildet wurde, gegebenenfalls mit einem oder mehreren Resten substituiert sind, gleich oder verschieden, ausgewählt aus den Halogenatomen und den Resten Cyano, Hydroxyl, Alkyl, Alcoxy, OCF3, CF3, S(O)n-CF3, Nitro, Oxo, Thioxo, OCOAlk, wobei Phenyl seinerseits gegebenenfalls mit einem oder mehreren Resten substituiert ist, ausgewählt aus den Halogenatomen und den Resten Alkyl und Alcoxy, -OCOAlk, NH2, NHAlk, N(Alk)2, N(alk)(phenylalkyl), N(Alk)(aminoalkyl) N(alk)(alkylaminoalkyl) N(Alk)(dialkylaminoalkyl, freiem oder mit einem Alkylrest verestertem Carboxy,
wobei alle oben genannten Phenylreste ferner gegebenenfalls mit einem Alkylendioxyrest substituiert sind,
wobei alle oben genannten Alkylreste ferner gegebenenfalls mit einem oder mehreren gesättigten oder teilweise ungesättigten heterocyclischen Resten substituiert sind, die 4 bis 7 Glieder umfassen und mindestens ein Stickstoffatom N und ferner 0 bis 2 andere Heteroatome umfassen, ausgewählt aus O, N und S,
wobei alle oben genannten Pyrrolidinyl- und Chinazolinylreste ferner gegebenenfalls mit Oxo oder Thioxo substituiert sind,
wobei alle oben genannten Alkyl- und Alcoxyreste linear oder verzweigt sind und höchstens 6 Kohlenstoffatome umfassen,
wobei alle oben genannten Cycloalkylreste höchstens 7 Kohlenstoffatome umfassen,
wobei die anderen Substituenten der Produkte mit der Formel (I) die Werte haben, die in einem der Ansprüche definiert sind.

34. Produkte mit der Formel (I), wie in einem der Ansprüche definiert, so dass Y2 für Wasserstoff, Halogen, Hydroxyl, Cyano, Alkyl, Alcoxy, Phenyl, CONR5R6, NR5R6, -NR10-COOH, -NR10-COOAlk, -NR10-CO-R6, -NR10-CS-NR5R6, -NR10-CO-NR5R6 oder -NR10-SO2-R6 steht,
R5 und R6, gleich oder verschieden, ausgewählt sind aus Wasserstoff; Alkyl; Cycloalkyl; Phenyl; Pyrimidinyl; Thienyl; Pyridyl; Chinolyl; Thiazolyl gegebenenfalls substituiert mit einem oder zwei Halogenatomen; Pyran, gegebenenfalls substituiert mit einem oder mehreren OCOAlk; Phenyl, substituiert mit einem oder mehreren Resten, ausgewählt aus den Halogenatomen und den Resten Alkyl, Alcoxy, Amino, Alkylamino, Dialkylamino und freiem oder mit einem Alkylrest verestertem Carboxy; Alkyl, substituiert mit Phenyl, seinerseits gegebenenfalls mit einem oder mehreren Resten substituiert, ausgewählt aus den Halogenatomen, Alkyl, Alcoxy, Amino, Alkylamino, Dialkylamino, freiem oder mit einem Alkylrest verestertem Carboxy; Alkyl, substituiert mit Piperazinyl, seinerseits gegebenenfalls mit einem oder mehreren Resten substituiert, ausgewählt aus Alk, Alk-OH und Pyridyl; Alkyl, substituiert mit Imidazolyl; Alkyl, substituiert mit einem oder mehreren Resten, ausgewählt aus NH2, NHAlk, N(Alk)2, N(alk)(phenylalkyl), N(Alk)(aminoalkyl) N(Alk)(alkylaminoalkyl) und N(Alk)(dialkylaminoalkyl); Alkyl, substituiert mit Morpholinyl, gegebenenfalls substituiert mit einem oder zwei Alk; Alkyl, substituiert mit Pyrrolidinyl; Alkyl, substituiert mit Piperidyl, seinerseits gegebenenfalls mit einem oder zwei Alk substituiert; Alkyl, substituiert mit Thiomorpholinyl; Alkyl, substituiert mit Azetidinyl; Alkyl, substituiert mit Azepanyl, gegebenenfalls substituiert mit Oxo,
oder R5 und R6 mit dem Stickstoffatom, an das sie gebunden sind, einen Pyrrolidinyl-, Piperidyl-, Piperazinyl-, Morpholinyl- oder Chinazolinylrest bilden, wobei alle diese Reste gegebenenfalls mit einem oder mehreren Resten substituiert sind, gleich oder verschieden, ausgewählt aus den Halogenatomen und den Resten Alkyl, Hydroxyl, Alcoxy und Phenyl, seinerseits gegebenenfalls mit einem oder mehreren Resten substituiert, ausgewählt aus den Halogenatomen und den Alkyl- und Alcoxyresten,
wobei die Pyrrolidinyl- und Chinazolinylreste ferner gegebenenfalls mit Oxo oder Thioxo substituiert sind,
wobei der Piperazinylrest seinerseits gegebenenfalls mit einem oder mehreren Resten, ausgewählt aus Alk, Alk-OH und Pyridyl, substituiert ist,
R10 für Wasserstoff oder Alkyl steht,
wobei alle oben genannten Alkyl- oder Alk- und Alcoxyreste linear oder verzweigt sind und höchstens 6 Kohlenstoffatome umfassen,
wobei alle oben genannten Cycloalkylreste höchstens 7 Kohlenstoffatome umfassen,
wobei die Phenylreste ferner gegebenenfalls mit einem Rest, ausgewählt aus CF3, -OCF3, Nitro und Alkylendioxy, substituiert sind,
wobei die anderen Substituenten der Produkte mit der Formel (I) die Werte haben, die in einem der Ansprüche definiert sind.

35. Produkte mit der Formel (I), wie in einem der Ansprüche definiert, so dass Y2 für V1, Halogen, Hydroxyl, -C(=NH)NH2, OV1, O-CO-V1, COOV1, COV1, CO-NV1V2, -NV1V2, -NH-CO-V1, -NH-COO-Vl, -NH-NH-CO-V1, - NV1-CO-NV1V2, -NV1-CO-NHV1, -NH-CO-NHVl, -NH-SO2-NHV1 und -NH-SO2-V1 steht,
worin V1 und V2, gleich oder verschieden, für ein Wasserstoffatom, einen Alkyl-, Cycloalkyl- oder Phenylrest oder einen heterocyclischen Rest stehen, wie etwa Pyridinyl, Pyrazolyl, Imidazolyl, Dihydroimidazolyl, Tetrazolyl, Morpholinyl, Piperazinyl, Piperazinylalkyl, Alkylpiperazinyl, Phenylpiperazinyl, Thienyl, Furanyl, Piperidinyl, Methylpiperidinyl, Pyridyl, Pyrrolidinyl und Pyrrolidinylalkyl,
wobei alle Alkylreste, Phenylreste und heterocyclischen Reste gegebenenfalls mit einem oder mehreren Resten substituiert sind, ausgewählt aus den Halogenatomen, den Resten Hydroxyl, Alkyl, Alcoxy, CF3, NH2, NHalk, N(alk)2 und Phenyl, seinerseits gegebenenfalls mit einem oder mehreren Substituenten substituiert, ausgewählt aus den Halogenatomen, den Hydroxy- und Alcoxyresten,
wobei alle oben genannten Phenylreste und heterocyclischen Reste ferner gegebenenfalls mit Alkylresten substituiert sind,
wobei die Phenylreste ferner gegebenenfalls mit NR5R6 substituiert sind, wobei R5 und R6 sind, wie in Anspruch 1 definiert,
wobei die anderen Substituenten der Produkte mit der Formel (I) die Werte haben, die in einem der Ansprüche definiert sind.

36. Produkte mit der Formel (I), wie in einem der Ansprüche definiert, so dass Y2 für Wasserstoff, Halogen, Alkyl, Cycloalkyl, Hydroxyl, Alcoxy, freies Carboxy oder mit einem Alkyl- oder Phenylrest verestertes Carboxy, NH2, NHalk, N(alk)2 und Phenyl steht,
wobei alle Alkyl-, Alcoxy- und Phenylreste gegebenenfalls mit einem oder mehreren Resten substituiert sind, ausgewählt aus den Halogenatomen, den Resten Hydroxyl, Cl-C4-Alkyl, C1-C4-Alcoxy, CF3, NH2, NHalk, N(alk)2 und Phenyl, seinerseits gegebenenfalls mit einem oder mehreren Substituenten substituiert, ausgewählt aus den Halogenatomen, den Hydroxy- und Alcoxyresten,
wobei alle Phenylreste ferner gegebenenfalls mit einem oder mehreren C1-C4-Alkylresten substituiert sind und gegebenenfalls mit NR5R6 substituiert sind, wobei R5 und R6 sind, wie in Anspruch 1 definiert,
wobei die anderen Substituenten der Produkte mit der Formel (I) die Werte haben, die in einem der Ansprüche definiert sind.

37. Produkte mit der Formel (I), wie in einem der Ansprüche definiert, so dass Y2 für Wasserstoff, F, Cl, CH3, CH2CH3, OH, OCH3, NH2, NHAlk und Phenyl steht, gegebenenfalls substituiert mit NR5R6, wobei R5 und R6 sind, wie in Anspruch 1 definiert,
wobei die anderen Substituenten der Produkte mit der Formel (I) die Werte haben, die in einem der Ansprüche definiert sind.

38. Produkte mit der Formel (I), wie in einem der Ansprüche definiert, so dass B2 für die Reste 4-Pyridyl und 4-Chinolyl steht, substituiert mit einem oder zwei Resten, ausgewählt aus F, Cl, OH und OCH3,
wobei die anderen Substituenten der Produkte mit der Formel (I) die Werte haben, die in einem der Ansprüche definiert sind.

39. Produkte mit der Formel (I), wie in einem der Ansprüche definiert, die der Formel (IC) entsprechen: 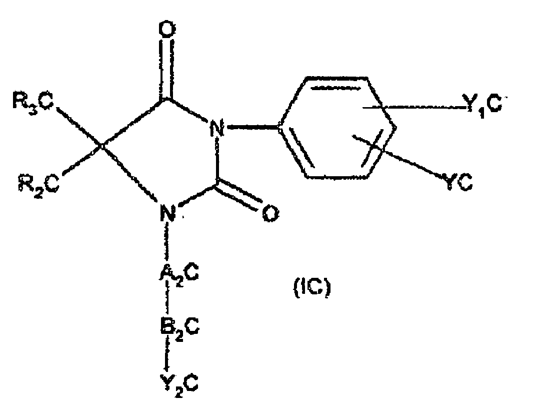 worin YC und Y1C so sind, dass einer für ein Wasserstoffatom, ein Halogenatom oder einen Aminorest steht, und der andere ausgewählt ist aus -OCF3, -O-CF2-CHF2, -O-CHF2, -O-CH2-CF3, -SF5, -S(O)n-CF3, -S(O)n-Alk, -SO2CHF2, S02CF2CF3, -SO2NH2, -S-CF2-CF2-CF3, -S-Alk-O-Alk, -S-Alk-OH, -S-Alk-CN, -S-Alk-Morpholino, -S-Alk-Pyrrolidinyl und -S-Alk-Piperazinyl, wobei die Morpholino-, Pyrrolidinyl- und Piperazinylreste gegebenenfalls mit Alk substituiert sind, wobei Alk für einen Alkylrest steht, der 1 bis 4 Kohlenstoffatome umfasst,
oder der Phenylrest mit seinen Substituenten YC und Y1C einen der beiden folgenden Reste bildet: 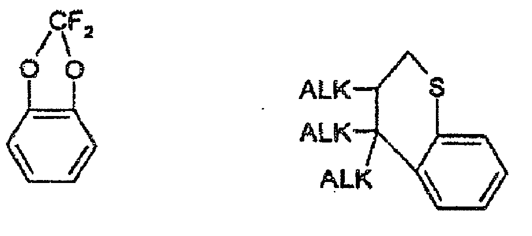 wobei ALK für einen Alkylrest steht, der 1 bis 4 Kohlenstoffatome umfasst,
R2C und R3C, gleich oder verschieden, für Wasserstoff oder Alkyl stehen, gegebenenfalls substituiert, oder R2C
R2C und R3C, gleich oder verschieden, für Wasserstoff oder Alkyl stehen, gegebenenfalls substituiert, oder R2C und R3C zusammen mit dem Kohlenstoffatom, an das sie gebunden sind, einen C3-C10-Cycloalkyl- oder C3-C10-Heterocycloalkylrest bilden,
A2C für eine einfache Bindung oder CH2 steht,
B2C für die Reste Pyridyl, Pyrimidinyl, Chinolyl,
Azaindolyl, Chinazolyl, Thiazolyl, Imidazolyl, Pyrazolyl, Furazanyl, Isoxazolyl, Morpholinyl, Pyrrolidinyl, Furyl, Piperidyl, Chromenyl, Oxochromenyl, Chinazolyl, Thienyl, Indolyl, Pyrrolyl, Purinyl, Benzoxazinyl, Benzimidazolyl, Benzofuranyl steht, gegebenenfalls substituiert mit einem oder mehreren Resten, ausgewählt aus den Werten für Y2A,
Y2CA für Wasserstoff, Halogen, Hydroxyl, Cyano, Alkyl, Alcoxy, Phenyl, COOH, COOAlk, CONR5R6, NR5R6, - NR10-COOH, -NR10-COOAlk, -NR10-CO-R6, -NR10-CS-NR5R6, - NR10-CO-NR5R6 oder -NR10-SO2-R6 steht, wobei alle diese Reste gegebenenfalls substituiert sind,
R5 und R6, gleich oder verschieden, ausgewählt sind aus Wasserstoff, Alkyl, Cycloalkyl, Phenyl, Pyrimidinyl, Thienyl, Pyridyl, Chinolyl, Thiazolyl und Pyran, wobei alle diese Reste gegebenenfalls substituiert sind, oder R5 und R6 mit dem Stickstoffatom, an das sie gebunden sind, einen Pyrrolidinyl-, Piperidyl-, Piperazinyl-, Morpholinyl-oder Chinazolinylrest bilden, gegebenenfalls substituiert,
R10 für Wasserstoff oder Alkyl steht,
wobei alle oben genannten Alkyl-, Alcoxy-, Cycloalkyl- und Phenylreste sowie der Ring, gebildet aus R5 und R6 mit dem Atom, an das sie gebunden sind, gegebenenfalls mit einem oder mehreren Resten substituiert sind, gleich oder verschieden, ausgewählt aus den Halogenatomen und den Resten Cyano; Hydroxyl; Alkyl; Alcoxy; OCF3; CF3; S(O)n-CF3; Nitro; Oxo; Thioxo; OCOAlk; wobei Phenyl seinerseits gegebenenfalls mit einem oder mehreren Resten substituiert ist, ausgewählt aus den Halogenatomen und den Resten Alkyl und Alcoxy; -OCOAlk; NH2, NHAlk, N(Alk)2, N(Alk)(phenylalkyl), N(Alk)(aminoalkyl) N(Alk)(alkylaminoalkyl), N(Alk)(dialkylaminoalkyl; freiem oder mit einem Alkylrest verestertem Carboxy,
wobei alle oben genannten Phenylreste ferner gegebenenfalls mit einem Alkylendioxyrest substituiert sind,
wobei alle oben genannten Alkylreste ferner gegebenenfalls mit einem oder mehreren Resten substituiert sind, ausgewählt aus den Piperazinylresten, seinerseits gegebenenfalls substituiert mit Alk, Alk-OH und Pyridyl; Imidazolyl; Morpholinyl; Pyrrolidinyl; Piperidyl, seinerseits gegebenenfalls substituiert mit einem oder zwei alk; Azepanyl, gegebenenfalls substituiert mit Oxo,
wobei alle oben genannten Pyrrolidinyl- und Chinazolinylreste ferner gegebenenfalls mit Oxo oder Thioxo substituiert sind,
wobei alle oben genannten Alkyl- und Alcoxyreste linear oder verzweigt sind und höchstens 6 Kohlenstoffatome umfassen,
wobei alle oben genannten Cycloalkylreste höchstens 7 Kohlenstoffatome umfassen,
n für eine ganze Zahl von 0 bis 2 steht,
wobei die Produkte mit der Formel (IC) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IC) vorliegen.

40. Produkte mit der Formel (I), wie in einem der Ansprüche definiert, die der Formel (IA) entsprechen: 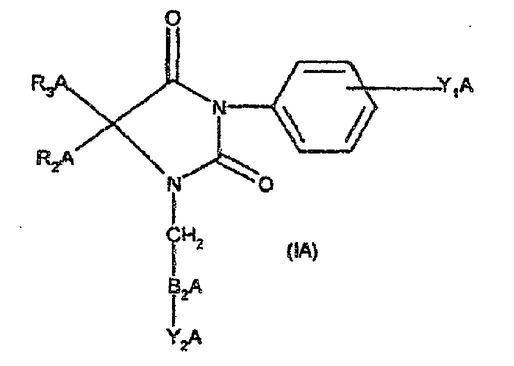 worin:
Y1A für -OCF3, S(O)n-CF3 und S02CHF2 steht,
B2A für die Reste 4-Chinolyl und 4-Pyridyl steht, gegebenenfalls substituiert mit einem oder mehreren Resten, ausgewählt aus den Werten für Y2A,
Y2A die Bedeutung hat, die in einem der vorhergehenden Ansprüche für Y2 angegeben ist,
R2A und R3A, gleich oder verschieden, für Wasserstoff oder Alkyl stehen, gegebenenfalls substituiert, oder R2A und R3A zusammen mit dem Kohlenstoffatom, an das sie gebunden sind, einen C3-C10-Cycloalkyl- oder C3-C10-Heterocycloalkylrest bilden,
wobei alle Alkyl- und Phenylreste gegebenenfalls mit einem oder mehreren Resten substituiert sind, ausgewählt aus Halogen, OH, alk, Oalk, OCF3, S(O)n-CF3, CF3, NH2, NHAlk und N(alk)2,
n für eine ganze Zahl von 0 bis 2 steht,
wobei die Produkte mit der Formel (IA) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IA) vorliegen.

41. Produkte mit der Formel (IA), wie im vorhergehenden Anspruch definiert, worin Y1A, B2a, R2A und R3A die zuvor angegebenen Bedeutungen haben, und
Y2A für die Reste Halogen, -OH, -alk, Oalk, Oacyl, -NR5AR6A, -CO2H, -CO2alk, -CO-NR5AR6A, -S(O)nCF3, -NH-S(O)n-CF3 oder Phenyl steht, wobei alk für einen linearen oder verzweigten Alkylrest steht, der höchstens 6 Kohlenstoffatome umfasst, wobei alle Alkyl-, Alcoxy- und Phenylreste gegebenenfalls substituiert sind,
R5A und R6A, gleich oder verschieden, für Wasserstoff, Alkyl, Cycloalkyl, Phenyl stehen, wobei die Alkyl- und Phenylreste gegebenenfalls substituiert sind, oder R5A und R6A mit dem Stickstoffatom, an das sie gebunden sind, einen cyclischen Rest bilden, ausgewählt aus den Resten Pyrrolidinyl, Piperidinyl, Piperazinyl, Morpholinyl, Piperazinyl, Indolinyl, Pyrindolinyl, Tetrahydrochinolin und Azetidin,
wobei alle Alkyl-, Alcoxy- und Phenylreste gegebenenfalls mit einem oder mehreren Resten substituiert sind, ausgewählt aus Halogen, OH, alk, Oalk, OCF3, S(O)n-CF3, CF3, NH2, NHAlk und N(alk)2,
n für eine ganze Zahl von 0 bis 2 steht,
wobei die Produkte mit der Formel (IA) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IA) vorliegen.

42. Produkte mit der Formel (I), wie in einem der Ansprüche definiert, die der Formel (IA) entsprechen, worin:
Y1A für -OCF3, SCF3 oder S(O)2-CF3 steht,
B2a für einen 4-Chinolyl- oder 4-Pyridylrest steht, gegebenenfalls substituiert mit einem oder zwei Resten, ausgewählt aus Halogen, -OH, alk, -Oalk, -CO2H, -CO2alk, -NR5AR6A, -CF3, -OCF3 und Phenyl, gegebenenfalls substituiert,
R5A und R6A, gleich oder verschieden, für Wasserstoff, Alkyl, Cycloalkyl, Phenyl stehen, wobei die Alkyl- und Phenylreste gegebenenfalls substituiert sind,
oder R5A und R6A mit dem Stickstoffatom, an das sie gebunden sind, einen cyclischen Rest bilden, ausgewählt aus den Resten Pyrrolidinyl, Piperidinyl, Piperazinyl, Morpholinyl, Piperazinyl und Azetidin,
R2A und R3A, gleich oder verschieden, für Wasserstoff oder Alkyl stehen, gegebenenfalls substituiert, oder R2A und R3A zusammen mit dem Kohlenstoffatom, an das sie gebunden sind, einen C3-C6-Cycloalkyl- oder C3-C6-Heterocycloalkylrest bilden,
wobei alle Alkyl- und Phenylreste gegebenenfalls mit einem oder mehreren Resten substituiert sind, ausgewählt aus Halogen, OH, alk, Oalk, OCF3, S(O)n-CF3, CF3, NH2, NHalk und N(alk)2,
wobei die Produkte mit der Formel (IA) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IA) vorliegen.

43. Produkte mit der Formel (I), wie in einem der Ansprüche definiert, die der Formel (IA) entsprechen, worin:
Y1A für -OCF3, SCF3 oder S(O)2-CF3 steht,
B2a für einen 4-Chinolyl- oder 4-Pyridylrest steht, gegebenenfalls substituiert mit einem oder zwei Resten, ausgewählt aus Halogen, -OH, alk und -Oalk,
R2A und R3A, gleich oder verschieden, für Wasserstoff und lineares oder verzweigtes Alkyl stehen, das höchstens 4 Kohlenstoffatome umfasst, gegebenenfalls substituiert mit einem Hydroxylrest, oder R2A und R3A zusammen mit dem Kohlenstoffatom, an das sie gebunden sind, einen C3-C6-Cycloalkylrest bilden,
wobei die Produkte mit der Formel (IA) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IA) vorliegen.

44. Produkte mit der Formel (I), wie in einem der Ansprüche definiert, die der Formel (IA) entsprechen, worin Y1a für OCF3, SCF3 oder S(O)2CF3 steht, und R2A und R3A, gleich oder verschieden, für Wasserstoff und CH3 stehen, oder R2A und R3A zusammen mit dem Kohlenstoffatom, an das sie gebunden sind, einen Cyclopropylrest bilden,
wobei die anderen Substituenten die Werte haben, die in einem der Ansprüche angegeben sind,
wobei die Produkte mit der Formel (IA) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IA) vorliegen.

45. Produkte mit der Formel (I), wie in einem der Ansprüche definiert, die der Formel (IB) entsprechen: 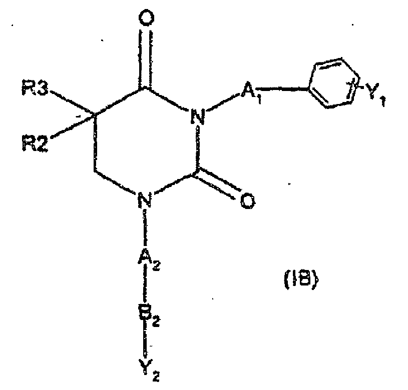 worin R2, R3, A1, Y, Y1, A2, B2 und Y2 die Bedeutungen haben, die in einem der Ansprüche angegeben sind,
wobei die Produkte mit der Formel (IB) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IB) vorliegen.

46. Produkte mit der Formel (IB), wie im vorhergehenden Anspruch definiert, worin Y1 für OCF3, SCF3 oder S(O)2CF3 steht, und R2 und R3, gleich oder verschieden, für Wasserstoff und CH3 stehen, oder R2 und R3 zusammen mit dem Kohlenstoffatom, an das sie gebunden sind, einen Cyclopropylrest bilden,
wobei die anderen Substituenten die Werte haben, die in einem der Ansprüche angegeben sind,
wobei die Produkte mit der Formel (IB) in allen möglichen razemischen, enantiomeren und diastereoisomeren Isomerformen sowie als Additionssalze mit den mineralischen und organischen Säuren oder mit den mineralischen und organischen Basen der Produkte mit der Formel (IB) vorliegen.

47. Produkte mit der Formel (I), wie in einem der Ansprüche definiert, die folgende Namen aufweisen:
- (S)-5-Methyl-1-chinolin-4-ylmethyl-3-(4-trifluormethansulfonylphenyl)imidazolidin-2,4-dion-trifluoracetat
- (S)-5-Methyl-1-pyridin-4-ylmethyl-3-(4-trifluormethansulfonylphenyl)imidazolidin-2,4-dion-trifluoracetat
- (S)-5-Methyl-1-quinolin-4-ylmethyl-3-(4-trifluormethylsulfanylphenyl)imidazolidin-2,4-dion-trifluoracetat
- 5,5-Dimethyl-1-quinolin-4-ylmethyl-3-(4-trifluormethansulfonylphenyl)imidazolidin-2,4-dion-trifluoracetat
- (R)-5-Methyl-1-quinolin-4-ylmethyl-3-(4-trifluormethansulfonylphenyl)imidazolidin-2,4-dion-trifluoracetat
- (R)-5-Methyl-1-quinolin-4-ylmethyl-3-(4-trifluormethylsulfanylphenyl)imidazolidin-2,4-dion-trifluoracetat
- (R)-5-Methyl-1-pyridin-4-ylmethyl-3-(4-trifluormethansulfonylphenyl)imidazolidin-2,4-dion-trifluoracetat
- (R)-5-Methyl-1-(3-methylpyridin-4-ylmethyl)-3-(4-trifluormethansulfonylphenyl)imidazolidin-2,4-dion-trifluoracetat
- (R)-4-Methyl-3-quinolin-4-ylmethyl-5-thioxo-1-(4-trifluormethylsulfanylphenyl)imidazolidin-2-on-trifluoracetat
- (R)-5-Isopropyl-1-quinolin-4-ylmethyl-3-(4-trifluormethylsulfanylphenyl)imidazolidin-2,4-dion-trifluoracetat
- (R)-5-isopropyl-1-quinolin-4-ylmethyl-3-(4-trifluormethansulfonylphenyl)imidazolidin-2,4-dion-trifluoracetat
- (R)-5-(4-Hydroxybenzyl)-1-quinolin-4-ylmethyl-3- (4-trifluormethansulfonylphenyl)imidazolidin-2,4-dion-trifluoracetat
- (R)-5-(4-Hydroxybenzyl)-1-pyridin-4-ylmethyl-3-(4-trifluormethansulfonylphenyl)imidazolidin-2,4-dion-trifluoracetat
- (R)-5-(1-Hydroxyethyl)-1-quinolin-4-ylmethyl-3-(4-trifluormethansulfonylphenyl)imidazolidin-2,4-dion-trifluoracetat
- 4-Chinolin-4-ylmethyl-6-(4-trifluormethylsulfanylphenyl)-4,6-diaza-spiro[2.4]heptan-5,7-dion-trifluoracetat
- 4-Chinolin-4-ylmethyl-6-(4-trifluormethansulfonylphenyl)-4,6-diaza-spiro[2.4]heptan-5,7-dion-trifluoracetat
- 4-Pyridin-4-ylmethyl-6-(4-trifluormethylsulfanylphenyl)-4,6-diaza-spiro[2.4]heptan-5,7-dion-trifluoracetat
- 4-Pyridin-4-ylmethyl-6-(4-trifluormethansulfonylphenyl)-4,6-diaza-spiro[2.4]heptan-5,7-dion-trifluoracetat
- (R)-1-(3-Hydroxy-pyridin-4-ylmethyl)-5-methyl-3-(4-trifluormethylsulfanylphenyl)imidazolidin-2,4-dion-trifluoracetat
- 5,5-Dimethyl-1-chinolin-4-ylmethyl-3-(4-trifluormethoxyphenyl)imidazolidin-2,4-dion-trifluoracetat
- 5,5-Dimethyl-1-chinolin-4-ylmethyl-3-(4-trifluormethylsulfanylphenyl)imidazolidin-2,4-dion-trifluoracetat
- 5,5-Dimethyl-1-(3-methylpyridin-4-ylmethyl)-3-(4-trifluormethoxyphenyl)imidazolidin-2,4-dion-trifluoracetat
- 5,5-Dimethyl-1-(3-methylpyridin-4-ylmethyl)-3-(4-trifluormethylsulfanylphenyl)imidazolidin-2,4-dion-trifluoracetat
- 5,5-Dimethyl-1-(3-methylpyridin-4-ylmethyl)-3-(4-trifluormethansulfonylphenyl)imidazolidin-2,4-dion-trifluoracetat
- 1-(3-Hydroxypyridin-4-ylmethyl)-5,5-dimethyl-3-(4-trifluormethoxyphenyl)imidazolidin-2,4-dion-trifluoracetat
- 1-(3-Hydroxypyridin-4-ylmethyl)-5,5-dimethyl-3-(4-trifluormethylsulfanylphenyl)imidazolidin-2,4-dion-trifluoracetat
- 1-(3-Hydroxypyridin-4-ylmethyl)-5,5-dimethyl-3-(4-trifluormethansulfonylphenyl)imidazolidin-2,4-dion-trifluoracetat
- 4-Chinolin-4-ylmethyl-6-(4-trifluormethoxyphenyl)-4,6-diaza-spiro[2.4]heptan-5,7-dion-trifluoracetat
- 4-(3-Methylpyridin-4-ylmethyl)-6-(4-trifluormethylsulfanylphenyl)-4,6-diaza-spiro[2.4]heptan-5,7-dion-trifluoracetat
- 4-(3-Hydroxypyridin-4-ylmethyl)-6-(4-trifluormethoxyphenyl)-4,6-diaza-spiro[2.4]heptan-5,7-dion-trifluoracetat
- 4-(3-Hydroxypyridin-4-ylmethyl)-6-(4-trifluormethylsulfanylphenyl)-4,6-diaza-spiro[2.4]heptan-5,7-dion-trifluoracetat
- 4-(3-Hydroxypyridin-4-ylmethyl)-6-(4-trifluormethansulfonylphenyl)-4,6-diaza-spiro[2.4]heptan-5,7-dion-trifluoracetat,
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

48. Produkte mit der Formel (I), wie in einem der Ansprüche definiert, die folgende Namen aufweisen:
- {4-[5,5-Dimethyl-2,4-dioxo-3-(4-trifluormethylsulfanylphenyl)imidazolidin-1-ylmethyl]pyridin-2-yl}amidtrifluoracetat der Cyclopropancarbonsäure;
- 5,5-Dimethyl-1-[2-(pyridin-2-ylamino)pyridin-4-yl-methyl]-3-(4-trifluormethylsulfanylphenyl)imidazolidin-2,4-dion; Verbindung mit Trifluoressigsäure;
- N-{4-[5,5-Dimethyl-2,4-dioxo-3-(4-trifluormethylsulfanylphenyl)imidazolidin-1-ylmethyl]pyridin-2-yl}isobutyramid; Verbindung mit Trifluoressigsäure;
- N-{4-[5,5-Dimethyl-2,4-dioxo-3-(4-trifluormethylsulfanylphenyl)imidazolidin-1-ylmethyl]pyridin-2-yl}propionamid; Verbindung mit Trifluoressigsäure;
- 1-(2-Amino-pyridin-4-ylmethyl)-5,5-dimethyl-3-(4-trifluormethylsulfanylphenyl)imidazolidin-2,4-dion-hydrochlorid;
- {4-[5,5-Dimethyl-2,4-dioxo-3-(4-trifluormethylsulfanylphenyl)imidazolidin-1-ylmethyl]pyridin-2-yl}amidtrifluoracetat der Pyridin-2-carbonsäure;
- N-{4-[5,5-Dimethyl-2,4-dioxo-3-(4-trifluormethylsulfanylphenyl)imidazolidin-1-ylmethyl]pyridin-2-yl}-3-piperidin-1-yl-propionamidtrifluoracetat;
- N-{4-[5,5-Dimethyl-2,4-dioxo-3-(4-trifluormethylsulfanylphenyl)imidazolidin-1-ylmethyl]pyridin-2-yl}-3-[4-(2-hydroxyethyl)piperazin-1-yl]propionamidtrifluoracetat;
- N-{4-[5,5-Dimethyl-2,4-dioxo-3-(4-trifluormethylsulfanylphenyl)imidazolidin-1-ylmethyl]pyridin-2-yl}-3-morpholin-4-yl-propionamidtrifluoracetat;
- N-{4-[5,5-Dimethyl-2,4-dioxo-3-(4-trifluormethylsulfanylphenyl)imidazolidin-1-ylmethyl]pyridin-2-yl}-3-pyrrolidin-1-yl-propionamidtrifluoracetat;
- N-{4-[5,5-Dimethyl-2,4-dioxo-3-(4-trifluormethylsulfanylphenyl)imidazolidin-1-ylmethyl]pyridin-2-yl}-3-(4-methylpiperazin-1-yl)propionamidtrifluoracetat;
- 1-{4-[5,5-Dimethyl-2,4-dioxo-3-(4-trifluormethylsulfanylphenyl)imidazolidin-1-ylmethyl]pyridin-2-yl}-3-phenylharnstoff;
- 1-[2-(6-Ethylpyridin-2-ylamino)pyridin-4-ylmethyl]-5,5-dimethyl-3-(4-trifluormethylsulfanylphenyl)imidazolidin-2,4-dion;
- 5,5-Dimethyl-1-[2-(4-methylpyridin-2-ylamino)pyridin-4-ylmethyl]-3-(4-trifluormethylsulfanylphenyl)imidazolidin-2,4-dion;
- 5,5-Dimethyl-1-[2-(6-methylpyridin-2-ylamino)pyridin-4-ylmethyl]-3-(4-trifluormethylsulfanylphenyl)imidazolidin-2,4-dion;
- 1-[2-(4,6-Dimethylpyridin-2-ylamino)pyridin-4-yl-methyl]-5,5-dimethyl-3-(4-trifluormethylsulfanylphenyl)imidazolidin-2,4-dion;
- 1-[2-(3,5-Dichlorpyridin-2-ylamino)pyridin-4-yl-methyl]-5,5-dimethyl-3-(4-trifluormethylsulfanylphenyl)imidazolidin-2,4-dion;
- 5,5-Dimethyl-1-[2-(pyridin-4-ylamino)pyridin-4-yl-methyl]-3-(4-trifluormethylsulfanylphenyl)imidazolidin-2,4-dion;
- 5,5-Dimethyl-1-[2-(pyridin-3-ylamino)pyridin-4-yl-methyl]-3-(4-trifluormethylsulfanylphenyl)imidazolidin-2,4-dion;
- N-{4-[5,5-dimethyl-2,4-dioxo-3-(4-trifluormethylsulfanylphenyl)imidazolidin-1-yl-methyl]pyridin-2-yl}-3-(2-oxo-azepan-1-yl)propionamid;
- 3-(Benzylmethylamino)-N-{4-[5,5-dimethyl-2,4-dioxo-3-(4-trifluormethylsulfanylphenyl)imidazolidin-1-yl-methyl]pyridin-2-yl}propionamid;
- 4,5-Diacetoxy-6-acetoxymethyl-2-(3-{4-[5,5-dimethyl-2,4-dioxo-3-(4-trifluormethylsulfanylphenyl)imidazolidin-1-ylmethyl]pyridin-2-yl}thioureido-essigsäure;
- 5,5-Dimethyl-1-[2-(5-methylpyridin-2-ylamino)pyridin-4-ylmethyl]-3-(4-trifluormethylsulfanylphenyl)imidazolidin-2,4-dion;
- N-{4-[5,5-Dimethyl-2,4-dioxo-3-(4-trifluormethylsulfanylphenyl)imidazolidin-1-ylmethyl]pyridin-2-yl}-3,5-dimethoxy-benzamidtrifluoracetat;
- 5,5-Dimethyl-1-[2-(pyrazin-2-yl-amino)pyridin-4-ylmethyl]-3-(4-trifluormethylsulfanylphenyl)imidazolidin-2,4-dion-trifluoracetat;
- N-{4-[5,5-Dimethyl-2,4-dioxo-3-(4-trifluormethylsulfanylphenyl)imidazolidin-1-ylmethyl]pyridin-2-yl}-3-(3-methylpiperidin-1-yl)propionamidtrifluoracetat;
- N-{4-[5,5-Dimethyl-2,4-dioxo-3-(4-trifluormethylsulfanylphenyl)imidazolidin-1-ylmethyl]pyridin-2-yl}-3-(3,5-dimethyl-piperidin-1-yl)propionamidtrifluoracetat;
- N-{4-[5,5-Dimethyl-2,4-dioxo-3-(4-trifluormethylsulfanylphenyl)imidazolidin-1-ylmethyl]pyridin-2-yl}-3-methoxy-benzamidtrifluoracetat;
- {4-[5,5-Dimethyl-2,4-dioxo-3-(4-trifluormethylsulfanylphenyl)imidazolidin-1-ylmethyl]pyridin-2-yl}-amidtrifluoracetat der Pyrazin-2-carbonsäure;
- {4-[5,5-Dimethyl-2,4-dioxo-3-(4-trifluormethylsulfanylphenyl)imidazolidin-1-ylmethyl]pyridin-2-yl}amidtrifluoracetat der Thiophen-2-carbonsäure;
- N-{4-[5,5-Dimethyl-2,4-dioxo-3-(4-trifluormethylsulfanylphenyl)imidazolidin-1-ylmethyl]pyridin-2-yl}-4-methylbenzamid; Verbindung mit Trifluoressigsäure;
- 1-Isochinolin-5-yl-5,5-dimethyl-3-(4-trifluormethylsulfanylphenyl)imidazolidin-2,4-dion;
- 3-(4-Acetylpiperazin-1-yl)-N-{4-[5,5-dimethyl-2,4-dioxo-3-(4-trifluormethylsulfanylphenyl)imidazolidin-1-ylmethyl]pyridin-2-yl}propionamid;
- 3-[4-(2-Diethylaminoethyl)piperazin-1-yl]-N-{4-[5,5-dimethyl-2,4-dioxo-3-(4-trifluormethylsulfanylphenyl)imidazolidin-1-ylmethyl]pyridin-2-yl}propionamid;
- N-{4-[5,5-Dimethyl-2,4-dioxo-3-(4-trifluormethylsulfanylphenyl)imidazolidin-1-ylmethyl]pyridin-2-yl}-3-(2,6-dimethylmorpholin-4-yl)propionamid;
- N-{4-[5,5-Dimethyl-2,4-dioxo-3-(4-trifluormethylsulfanylphenyl)imidazolidin-1-ylmethyl]pyridin-2-yl}-3-(4-pyrrolidin-1-yl-piperidin-1-yl)propionamid;
- N-{4-[5,5-Dimethyl-2,4-dioxo-3-(4-trifluormethylsulfanylphenyl)imidazolidin-1-ylmethyl]pyridin-2-yl}-2-(4-pyrrolidin-1-yl-piperidin-1-yl)-acetamid;
- 5,5-Dimethyl-1-[2-(4-methyl-pyridin-3-ylamino)pyridin-4-ylmethyl]-3-(4-trifluormethylsulfanylphenyl)imidazolidin-2,4-dion;
- 5,5-Dimethyl-1-[2-(6-morpholin-4-yl-pyridin-3-yl-amino)pyridin-4-ylmethyl]-3-(4-trifluormethylsulfanylphenyl)imidazolidin-2,4-dion;
- 1-[2-(2,6-Dimethylpyridin-3-ylamino)pyridin-4-yl-methyl]-5,5-dimethyl-3-(4-trifluormethylsulfanylphenyl)imidazolidin-2,4-dion;
- 5-{4-[5,5-Dimethyl-2,4-dioxo-3-(4-trifluormethylsulfanylphenyl)imidazolidin-1-ylmethyl]pyridin-2-ylamino}pyridin-2-carbonsäuremethylester;
- 1-[2-(2,6-Dimethoxypyridin-3-ylamino)pyridin-4-ylmethyl]-5,5-dimethyl-3-(4-trifluormethylsulfanylphenyl)imidazolidin-2,4-dion;
- 1-[2-(6-fluorpyridin-3-ylamino)pyridin-4-ylmethyl]-5,5-dimethyl-3-(4-trifluormethylsulfanylphenyl)imidazolidin-2,4-dion;
- 1-[2-(6-Methoxypyridin-3-ylamino)pyridin-4-ylmethyl]-5,5-dimethyl-3-(4-trifluormethylsulfanylphenyl)imidazolidin-2,4-dion;
wobei die Produkte mit der Formel (I) in allen möglichen razemischen, enantiomeren und diastereoisomeren Isomerformen sowie als Additionssalze mit den mineralischen und organischen Säuren oder mit den mineralischen und organischen Basen der Produkte mit der Formel (I) vorliegen.

49. Die Produkte mit der Formel (I), wie in den Ansprüchen 1 bis 48 definiert, als Medikamente, wobei die Produkte mit der Formel (I) in allen möglichen razemischen, enantiomeren und diastereoisomeren Isomerformen sowie als pharmazeutisch akzeptable Additionssalze mit den mineralischen und organischen Säuren oder mit den mineralischen und organischen Basen der Produkte mit der Formel (I) vorliegen.

50. Die Produkte mit der Formel (IC), wie in den vorhergehenden Ansprüchen definiert, als Medikamente, wobei die Produkte mit der Formel (IC) in allen möglichen razemischen, enantiomeren und diastereoisomeren Isomerformen sowie als pharmazeutisch akzeptable Additionssalze mit den mineralischen und organischen Säuren oder mit den mineralischen und organischen Basen der Produkte mit der Formel (IC) vorliegen.

51. Die Produkte mit der Formel (IA), wie in den vorhergehenden Ansprüchen definiert, als Medikamente, wobei die Produkte mit der Formel (IA) in allen möglichen razemischen, enantiomeren und diastereoisomeren Isomerformen sowie als pharmazeutisch akzeptable Additionssalze mit den mineralischen und organischen Säuren oder mit den mineralischen und organischen Basen der Produkte mit der Formel (IA) vorliegen.

52. Die Produkte mit der Formel (1), wie in Anspruch 47 oder 48 definiert, als Medikamente, sowie die pharmazeutisch akzeptablen Additionssalze mit den mineralischen und organischen Säuren oder mit den mineralischen und organischen Basen dieses Produkts.

53. Pharmazeutische Zusammensetzungen, die als Wirkstoff mindestens eines der Medikamente enthalten, wie in den Ansprüchen 49 bis 52 definiert.

54. Pharmazeutische Zusammensetzungen, die als Wirkstoff mindestens eines der Medikamente enthalten, wie in Anspruch 47 oder 48 definiert.

55. Pharmazeutische Zusammensetzungen, wie in den Ansprüchen definiert, die ferner Wirkstoffe anderer Medikamente zur Chemotherapie gegen Krebs enthalten.

56. Pharmazeutische Zusammensetzungen nach einem der Ansprüche, **dadurch gekennzeichnet, dass** sie als Medikamente, vor allem zur Chemotherapie von Krebs verwendet werden.

57. Verwendung von Produkten mit der Formel (I), wie in einem der Ansprüche definiert, oder von pharmazeutisch akzeptablen Salzen der Produkte mit der Formel (I) zur Herstellung von Medikamenten, die dazu bestimmt sind, die Aktivität von Proteinkinasen und insbesondere einer Proteinkinase zu inhibieren.

58. Verwendung von Produkten mit der Formel (I), wie im vorhergehenden Anspruch definiert, oder von pharmazeutisch akzeptablen Salzen der Produkte mit der Formel (I), wobei die Proteinkinase eine Tyrosinproteinkinase ist.

59. Verwendung von Produkten mit der Formel (I), wie im einem der Ansprüche definiert, oder von pharmazeutisch akzeptablen Salzen der Produkte mit der Formel (I), wobei die Proteinkinase aus der folgenden Gruppe ausgewählt ist: EGFR, Fak, FLK-1, FGFR1, FGFR2, FGFR3, FGFR4, FGFR5, flt-1, IGF-1R, KDR, PLK, PDGFR, tie2, VEGFR, AKT, Raf.

60. Verwendung von Produkten mit der Formel (I), wie im einem der Ansprüche definiert, oder von pharmazeutisch akzeptablen Salzen der Produkte mit der Formel (I), wobei die Proteinkinase IGF1R ist.

61. Verwendung von Produkten mit der Formel (I), wie im einem der Ansprüche definiert, oder von pharmazeutisch akzeptablen Salzen der Produkte mit der Formel (I), wobei die Proteinkinase FAK ist.

62. Verwendung von Produkten mit der Formel (I), wie im einem der Ansprüche definiert, oder von pharmazeutisch akzeptablen Salzen der Produkte mit der Formel (I), wobei die Proteinkinase AKT ist.

63. Verwendung von Produkten mit der Formel (I), wie im einem der Ansprüche definiert, oder von pharmazeutisch akzeptablen Salzen der Produkte mit der Formel (I), wobei die Proteinkinase in einer Zellkultur vorliegt.

64. Verwendung von Produkten mit der Formel (I), wie im einem der Ansprüche definiert, oder von pharmazeutisch akzeptablen Salzen der Produkte mit der Formel (I), wobei die Proteinkinase in einem Säugetier vorliegt.

65. Verwendung von Produkten mit der Formel (I), wie in einem der Ansprüche definiert, oder von pharmazeutisch akzeptablen Salzen der Produkte mit der Formel (I) zur Herstellung eines Medikaments, das zur Vorbeugung oder Behandlung einer Krankheit bestimmt ist, die durch die Störung der Aktivität einer Proteinkinase **gekennzeichnet** ist.

66. Verwendung von Produkten mit der Formel (I) nach dem vorhergehenden Anspruch, wobei die Krankheit, der vorzubeugen ist oder die zu behandeln ist, bei einem Säugetier auftritt.

67. Verwendung von Produkten mit der Formel (I), wie in einem der Ansprüche definiert, oder von pharmazeutisch akzeptablen Salzen der Produkte mit der Formel (I) zur Herstellung eines Medikaments, das zur Vorbeugung oder Behandlung einer Krankheit bestimmt ist, die zur folgenden Gruppe gehört: Störungen der Blutgefäßproliferation, fibrotische Störungen, Störungen der Mesangialzellproliferation, Stoffwechselstörungen, Allergien, Asthma, Thrombosen, Krankheiten des Nervensystems, Retinopathien, Psoriasis, rheumatoide Arthritis, Diabetes, Muskeldegeneration, onkologische Krankheiten, Krebs.

68. Verwendung von Produkten mit der Formel (I), wie in einem der Ansprüche definiert, oder von pharmazeutisch akzeptablen Salzen der Produkte mit der Formel (I) zur Herstellung eines Medikaments, das zur Behandlung onkologischer Krankheiten bestimmt ist.

69. Verwendung von Produkten mit der Formel (I), wie in einem der Ansprüche definiert, oder von pharmazeutisch akzeptablen Salzen der Produkte mit der Formel (I) zur Herstellung eines Medikaments, das zur Krebsbehandlung bestimmt ist.

70. Verwendung von Produkten mit der Formel (I) nach dem vorhergehenden Anspruch, wobei die zu behandelnde Krankheit ein Krebs in Form solider Tumore ist.

71. Verwendung von Produkten mit der Formel (I) nach dem vorhergehenden Anspruch, wobei die zu behandelnde Krankheit ein Krebs ist, der gegen zytotoxische Mittel resistent ist.

72. Verwendung von Produkten mit der Formel (I), wie in einem der Ansprüche definiert, oder von pharmazeutisch akzeptablen Salzen der Produkte mit der Formel (I) zur Herstellung eines Medikaments, das zur Krebsbehandlung bestimmt ist, darunter Brustkrebs, Magenkrebs, Kolonkrebs, Lungenkrebs, Eierstockkrebs, Gebärmutterkrebs, Gehirnkrebs, Nierenkrebs, Kehlkopfkrebs, Lymphsystemkrebs, Schilddrüsenkrebs, Krebs des Urogenitalapparats, Krebs des Apparats, der Samenblase und Prostata beinhaltet, Knochenkrebs, Bauchspeicheldrüsenkrebs, Melanome.

73. Verwendung von Produkten mit der Formel (I) nach dem vorhergehenden Anspruch, wobei die zu behandelnde Krankheit ein Brustkrebs, Kolonkrebs oder Lungenkrebs ist.

74. Verwendung von Produkten mit der Formel (I), wie in einem der Ansprüche definiert, oder von pharmazeutisch akzeptablen Salzen der Produkte mit der Formel (I) zur Herstellung eines Medikaments, das zur Chemotherapie von Krebs bestimmt ist.

75. Verwendung von Produkten mit der Formel (I), wie in einem der Ansprüche definiert, oder von pharmazeutisch akzeptablen Salzen der Produkte mit der Formel (I) zur Herstellung von Medikamenten, die zur Chemotherapie von Krebs bestimmt sind, die allein oder in Kombination verwendet werden.

76. Verwendung von Produkten mit der Formel (I), wie in einem der Ansprüche definiert, oder von pharmazeutisch akzeptablen Salzen der Produkte mit der Formel (I) zur Herstellung von Medikamenten, die zur Verwendung, allein oder in Kombination mit Chemotherapie oder Radiotherapie oder abwechselnd in Kombination mit anderen Therapeutika bestimmt sind.

77. Verwendung von Produkten mit der Formel (I) nach dem vorhergehenden Anspruch, wobei die Therapeutika üblicherweise verwendete Antitumormittel sein können.

78. Produkte mit der Formel (I), wie in einem der Ansprüche definiert, als Proteinkinaseinhibitoren, wobei die Produkte mit der Formel (I) in allen möglichen razemischen, enantiomeren und diastereoisomeren Isomerformen sowie als pharmazeutisch akzeptable Additionssalze mit den mineralischen und organischen Säuren oder mit den mineralischen und organischen Basen der Produkte mit der Formel (I) vorliegen, sowie deren Prodrugs.

79. Produkte mit der Formel (I), wie in einem der Ansprüche definiert, als IGFlR-Inhibitoren

80. Produkte mit der Formel (IA), wie in den Ansprüchen definiert, als IGFlR-Inhibitoren.
